(19) Europäisches Patentamt
European Patent Office
Office européen des brevets

(11) EP 2 193 203 B1

# (12) EUROPEAN PATENT SPECIFICATION

(45) Date of publication and mention of the grant of the patent:
**25.07.2012 Bulletin 2012/30**

(21) Application number: **08804474.8**

(22) Date of filing: **19.09.2008**

(51) Int Cl.:
*C12N 15/82* (2006.01) *A01H 5/00* (2006.01)
*C12N 15/29* (2006.01)

(86) International application number:
**PCT/EP2008/062540**

(87) International publication number:
**WO 2009/037338 (26.03.2009 Gazette 2009/13)**

## (54) PLANTS HAVING INCREASED YIELD-RELATED TRAITS AND A METHOD FOR MAKING THE SAME

PFLANZEN MIT VERBESSERTEN ERTRAGSEIGENSCHAFTEN UND VERFAHREN ZU IHRER HERSTELLUNG

PLANTES AYANT DES CARACTÈRES SE RAPPORTANT À UN RENDEMENT ÉLEVÉ ET LEUR PROCÉDÉ D'OBTENTION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.09.2007 EP 07116988**
**28.09.2007 US 975882 P**

(43) Date of publication of application:
**09.06.2010 Bulletin 2010/23**

(73) Proprietor: **BASF Plant Science GmbH**
**67056 Ludwigshafen (DE)**

(72) Inventors:
- **FRANKARD, Valerie**
  **B-1410 Waterloo (BE)**
- **REUZEAU, Christophe**
  **F-24350 Tocan Saint Apre (FR)**

(74) Representative: **Mistry, Meeta et al**
**Greaves Brewster LLP**
**Copa House**
**Station Road**
**Cheddar, BS27 3AH (GB)**

(56) References cited:
**WO-A-2006/079655**

- **HORIGUCHI GOROU ET AL: "The transcription factor AtGRF5 and the transcription coactivator AN3 regulate cell proliferation in leaf primordia of Arabidopsis thaliana" PLANT JOURNAL, BLACKWELL SCIENTIFIC PUBLICATIONS, OXFORD, GB, vol. 43, no. 1, 1 July 2005 (2005-07-01), pages 68-78, XP002410132 ISSN: 0960-7412 cited in the application**
- **KIM JEONG HOE ET AL: "A transcriptional coactivator, AtGIF1, is involved in regulating leaf growth and morphology in Arabidopsis" PROCEEDINGS OF THE NATIONAL ACADEMY OF SCIENCES OF USA, NATIONAL ACADEMY OF SCIENCE, WASHINGTON, DC.; US, vol. 101, no. 36, 7 September 2004 (2004-09-07), pages 13374-13379, XP002362467 ISSN: 0027-8424**
- **GOROU HORIGUCHI ET AL: "Coordination of cell proliferation and cell expansion in the control of leaf size in Arabidopsis thaliana" JOURNAL OF PLANT RESEARCH, SPRINGER-VERLAG, TO, vol. 119, no. 1, 1 January 2006 (2006-01-01), pages 37-42, XP019375380 ISSN: 1618-0860 cited in the application**

EP 2 193 203 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**SEQUENCE LISTING**

<110> BASF Plant Science GmbH

<120> Plants having increased yield-related traits and a method for making the same

<130> PF60170

<150> EP 07116988.2
<151> 2007-09-21

<150> US 60/975,882
<151> 2007-09-28

<160> 270

<170> PatentIn version 3.3

<210> 1
<211> 1194
<212> DNA
<213> Arabidopsis thaliana

<400> 1

atgatgagtc taagtggaag tagcgggaga acaataggaa ggcctccatt tacaccaaca 60
caatgggaag aactggaaca tcaagcccta atctacaagt acatggtctc tggtgttcct 120
gtcccacctg agctcatctt ctccattaga agaagcttgg acacttcctt ggtctctaga 180
ctccttcctc accaatccct tggatggggg tgttaccaga tgggatttgg gagaaaacca 240
gatccagagc caggaagatg cagaagaaca gatggtaaga aatggagatg ctcaagagaa 300
gcttacccag attcgaagta ctgtgaaaaa cacatgcaca gaggaagaaa ccgtgccaga 360
aaatctcttg atcagaatca gacaacaaca actcctttaa catcaccatc tctctcattc 420
accaacaaca acaacccaag tcccaccttg tcttcttctt cttcctctaa ttcctcttct 480
actacttatt ctgcttcttc ttcttcaatg gatgcctaca gtaacagtaa taggtttggg 540
cttggtggaa gtagtagtaa cactagaggt tatttcaaca gccattctct tgattatcct 600
tatccttcta cttcacccaa acaacaacaa caaactcttc atcatgcttc cgctttgtca 660
cttcatcaaa atactaattc tacttctcag ttcaatgtct tagcctctgc tactgaccac 720
aaagacttca ggtactttca agggattggg gagagagttg gaggagttgg ggagagaacg 780
ttctttccag aagcatctag aagctttcaa gattctccat accatcatca ccaacaaccg 840
ttagcaacag tgatgaatga tccgtaccac cactgtagta ctgatcataa taagattgat 900
catcatcaca catactcatc ctcatcatca tctcaacatc ttcatcatga tcatgatcat 960
agacagcaac agtgttttgt tttgggcgcc gacatgttca acaaacctac aagaagtgtc 1020
cttgcaaact catcaagaca agatcaaaat caagaagaag atgagaaaga ttcatcagag 1080
tcgtccaaga agtctctaca tcacttcttt ggtgaggact gggcacagaa caagaacagt 1140
tcagattctt ggcttgacct ttcttcccac tcaagactcg acactggtag ctaa 1194

<210> 2
<211> 397
<212> PRT
<213> Arabidopsis thaliana

<400> 2

```
Met Met Ser Leu Ser Gly Ser Ser Gly Arg Thr Ile Gly Arg Pro Pro
1               5                   10                  15

Phe Thr Pro Thr Gln Trp Glu Glu Leu Glu His Gln Ala Leu Ile Tyr
            20                  25                  30

Lys Tyr Met Val Ser Gly Val Pro Val Pro Pro Glu Leu Ile Phe Ser
        35                  40                  45

Ile Arg Arg Ser Leu Asp Thr Ser Leu Val Ser Arg Leu Leu Pro His
    50                  55                  60

Gln Ser Leu Gly Trp Gly Cys Tyr Gln Met Gly Phe Gly Arg Lys Pro
65                  70                  75                  80

Asp Pro Glu Pro Gly Arg Cys Arg Arg Thr Asp Gly Lys Lys Trp Arg
                85                  90                  95

Cys Ser Arg Glu Ala Tyr Pro Asp Ser Lys Tyr Cys Glu Lys His Met
            100                 105                 110

His Arg Gly Arg Asn Arg Ala Arg Lys Ser Leu Asp Gln Asn Gln Thr
        115                 120                 125

Thr Thr Thr Pro Leu Thr Ser Pro Ser Leu Ser Phe Thr Asn Asn Asn
    130                 135                 140

Asn Pro Ser Pro Thr Leu Ser Ser Ser Ser Ser Ser Asn Ser Ser Ser
145                 150                 155                 160

Thr Thr Tyr Ser Ala Ser Ser Ser Ser Met Asp Ala Tyr Ser Asn Ser
                165                 170                 175

Asn Arg Phe Gly Leu Gly Gly Ser Ser Ser Asn Thr Arg Gly Tyr Phe
            180                 185                 190

Asn Ser His Ser Leu Asp Tyr Pro Tyr Pro Ser Thr Ser Pro Lys Gln
        195                 200                 205

Gln Gln Gln Thr Leu His His Ala Ser Ala Leu Ser Leu His Gln Asn
    210                 215                 220

Thr Asn Ser Thr Ser Gln Phe Asn Val Leu Ala Ser Ala Thr Asp His
225                 230                 235                 240

Lys Asp Phe Arg Tyr Phe Gln Gly Ile Gly Glu Arg Val Gly Gly Val
                245                 250                 255

Gly Glu Arg Thr Phe Phe Pro Glu Ala Ser Arg Ser Phe Gln Asp Ser
```

```
                260                 265                 270

Pro Tyr His His His Gln Gln Pro Leu Ala Thr Val Met Asn Asp Pro
        275                 280                 285

Tyr His His Cys Ser Thr Asp His Asn Lys Ile Asp His His His Thr
    290                 295                 300

Tyr Ser Ser Ser Ser Ser Ser Gln His Leu His His Asp His Asp His
305                 310                 315                 320

Arg Gln Gln Gln Cys Phe Val Leu Gly Ala Asp Met Phe Asn Lys Pro
                325                 330                 335

Thr Arg Ser Val Leu Ala Asn Ser Ser Arg Gln Asp Gln Asn Gln Glu
            340                 345                 350

Glu Asp Glu Lys Asp Ser Ser Glu Ser Ser Lys Lys Ser Leu His His
        355                 360                 365

Phe Phe Gly Glu Asp Trp Ala Gln Asn Lys Asn Ser Ser Asp Ser Trp
    370                 375                 380

Leu Asp Leu Ser Ser His Ser Arg Leu Asp Thr Gly Ser
385                 390                 395
```

<210> 3
<211> 735
<212> DNA
<213> Arabidopsis thaliana

<400> 3

```
atggctacaa ggattccatt cacagaatca caatgggaag aacttgaaaa ccaagctctt      60
gtgttcaagt acttagctgc aaatatgcct gttccacctc atcttctctt cctcatcaaa     120
agaccctttc tcttctcttc ttcttcttct tcatcttctt cttcaagctt cttctctccc     180
actctttctc cacactttgg gtggaatgtg tatgagatgg gaatgggaag aaagatagat     240
gcagagccag gaagatgtag aagaactgat ggcaagaaat ggagatgctc taaagaagct     300
taccctgact ctaagtactg tgagagacat atgcatagag gcaagaaccg ttcttcctca     360
agaaagcctc ctcctactca attcactcca aatctctttc tcgactcttc ttccagaaga     420
agaagaagtg gatacatgga tgatttcttc tccatagaac cttccgggtc aatcaaaagc     480
tgctctggct cagcaatgga agataatgat gatggctcat gtagaggcat caacaacgag     540
gagaagcagc cggatcgaca ttgcttcatc cttggtactg acttgaggac acgtgagagg     600
ccattgatgt tagaggagaa gctgaaacaa agagatcatg ataatgaaga agagcaagga     660
agcaagaggt tttataggtt tcttgatgaa tggccttctt ctaaatcttc tgtttctact     720
tcactcttca tttga                                                      735
```

<210> 4
<211> 244
<212> PRT
<213> Arabidopsis thaliana

<400> 4

```
Met Ala Thr Arg Ile Pro Phe Thr Glu Ser Gln Trp Glu Glu Leu Glu
1               5                   10                  15

Asn Gln Ala Leu Val Phe Lys Tyr Leu Ala Ala Asn Met Pro Val Pro
            20                  25                  30

Pro His Leu Leu Phe Leu Ile Lys Arg Pro Phe Leu Phe Ser Ser Ser
        35                  40                  45

Ser Ser Ser Ser Ser Ser Ser Ser Phe Phe Ser Pro Thr Leu Ser Pro
    50                  55                  60

His Phe Gly Trp Asn Val Tyr Glu Met Gly Met Gly Arg Lys Ile Asp
65                  70                  75                  80

Ala Glu Pro Gly Arg Cys Arg Arg Thr Asp Gly Lys Lys Trp Arg Cys
                85                  90                  95

Ser Lys Glu Ala Tyr Pro Asp Ser Lys Tyr Cys Glu Arg His Met His
            100                 105                 110

Arg Gly Lys Asn Arg Ser Ser Ser Arg Lys Pro Pro Pro Thr Gln Phe
        115                 120                 125

Thr Pro Asn Leu Phe Leu Asp Ser Ser Ser Arg Arg Arg Arg Ser Gly
    130                 135                 140

Tyr Met Asp Asp Phe Phe Ser Ile Glu Pro Ser Gly Ser Ile Lys Ser
145                 150                 155                 160

Cys Ser Gly Ser Ala Met Glu Asp Asn Asp Asp Gly Ser Cys Arg Gly
                165                 170                 175

Ile Asn Asn Glu Glu Lys Gln Pro Asp Arg His Cys Phe Ile Leu Gly
            180                 185                 190

Thr Asp Leu Arg Thr Arg Glu Arg Pro Leu Met Leu Glu Glu Lys Leu
        195                 200                 205

Lys Gln Arg Asp His Asp Asn Glu Glu Glu Gln Gly Ser Lys Arg Phe
    210                 215                 220

Tyr Arg Phe Leu Asp Glu Trp Pro Ser Ser Lys Ser Ser Val Ser Thr
225                 230                 235                 240

Ser Leu Phe Ile
```

<210> 5
<211> 1593
<212> DNA
<213> Arabidopsis thaliana

<400> 5

```
atggatcttg gagttcgtgt ttctggtcat gaaaccgttt cttctccggg tcaaactgaa      60
ctcggatctg gtttcagtaa caagcaagaa agatccggtt tcgatggtga agattgctgg     120
agaagttcaa agctctcacg aacatcaact gatggattct cttcttcccc tgcctctgct     180
aaaacgctgt cgtttcatca aggcatccct ttactgagat ctaccactat taatgatcct     240
cgtaaaggac aagaacacat gcttagcttc tcttctgctt caggcaaatc agatgtctca     300
ccttatcttc agtactgtag aaactcagga tatggtttag gaggaatgat gaacacaagc     360
aacatgcatg gaaacttgtt gacaggagta aaaggacctt tttcattgac tcagtgggca     420
gagctagagc aacaggcgtt gatctataag tatatcacag ccaatgtccc tgttccatct     480
agtttacttc tctctctcaa gaaatctttt ttcccttatg gttccttgcc tcctaattct     540
tttggatggg gctcttttca tctgggcttt tccggtggta acatggatcc cgagccaggg     600
agatgtcgcc ggacagatgg aaagaaatgg cggtgctcga gggacgctgt tcccgatcaa     660
aagtactgtg aacgacatat taacagaggc cgccatcgtt caagaaagcc tgtggaaggc     720
caaaatggcc acaatactaa tgctgccgcc gctgcttctg ctgctgccgc ttctaccgct     780
gctgctgtgt ccaaagcggc agcggggact tcagctgttg cgatgcgtgg atcagataat     840
aacaatagcc ttgccgctgc tgttggaaca caacatcata ccaataatca atctacagat     900
tctttggcta acagagttca aaattctcga ggggcttcgg tttttcctgc cacgatgaac     960
ttacagtcga aggaaactca tccgaaacaa agcaataatc cctttgaatt cggactcatc    1020
tcttctgatt cgttacttaa tccgtcgcat aaacaagcct cgtatgcaac ctcttccaaa    1080
ggctttggat cgtatcttga cttcggcaac caagccaagc acgcggggaa tcacaacaat    1140
gtcgattctt ggcccgaaga gctgaaatcg gattggactc agctctcaat gtcaatccct    1200
atggctccat cttcccctgt tcaagataaa cttgcactct cacctttaag gttatcgcgt    1260
gagtttgacc ccgcgatcca catgggatta ggcgtcaaca ccgagtttct tgaccccggg    1320
aaaaagacga ataactggat accaatctcc tggggtaata acaactccat gggaggtcca    1380
ctcggcgagg tactaaacag cacgaccaat agtcccaagt ttggttcctc tccaacaggc    1440
gtcttgcaaa agtcgacatt tggttctctt tctaacagca gctcggcaag cagcaccatc    1500
attggcgata acaacaataa gaacggtgat ggaaaagatc cgcttggccc gaccacgctg    1560
atgaatactt ctgctactgc tccttctctg tga                                 1593
```

<210> 6
<211> 530
<212> PRT
<213> Arabidopsis thaliana

<400> 6

```
Met Asp Leu Gly Val Arg Val Ser Gly His Glu Thr Val Ser Ser Pro
1               5                   10                  15

Gly Gln Thr Glu Leu Gly Ser Gly Phe Ser Asn Lys Gln Glu Arg Ser
            20                  25                  30

Gly Phe Asp Gly Glu Asp Cys Trp Arg Ser Ser Lys Leu Ser Arg Thr
        35                  40                  45

Ser Thr Asp Gly Phe Ser Ser Ser Pro Ala Ser Ala Lys Thr Leu Ser
    50                  55                  60

Phe His Gln Gly Ile Pro Leu Leu Arg Ser Thr Thr Ile Asn Asp Pro
65                  70                  75                  80

Arg Lys Gly Gln Glu His Met Leu Ser Phe Ser Ser Ala Ser Gly Lys
                85                  90                  95

Ser Asp Val Ser Pro Tyr Leu Gln Tyr Cys Arg Asn Ser Gly Tyr Gly
            100                 105                 110

Leu Gly Gly Met Met Asn Thr Ser Asn Met His Gly Asn Leu Leu Thr
        115                 120                 125

Gly Val Lys Gly Pro Phe Ser Leu Thr Gln Trp Ala Glu Leu Glu Gln
    130                 135                 140

Gln Ala Leu Ile Tyr Lys Tyr Ile Thr Ala Asn Val Pro Val Pro Ser
145                 150                 155                 160

Ser Leu Leu Leu Ser Leu Lys Lys Ser Phe Phe Pro Tyr Gly Ser Leu
                165                 170                 175

Pro Pro Asn Ser Phe Gly Trp Gly Ser Phe His Leu Gly Phe Ser Gly
            180                 185                 190

Gly Asn Met Asp Pro Glu Pro Gly Arg Cys Arg Arg Thr Asp Gly Lys
        195                 200                 205

Lys Trp Arg Cys Ser Arg Asp Ala Val Pro Asp Gln Lys Tyr Cys Glu
    210                 215                 220

Arg His Ile Asn Arg Gly Arg His Arg Ser Arg Lys Pro Val Glu Gly
225                 230                 235                 240

Gln Asn Gly His Asn Thr Asn Ala Ala Ala Ala Ala Ser Ala Ala Ala
                245                 250                 255

Ala Ser Thr Ala Ala Ala Val Ser Lys Ala Ala Ala Gly Thr Ser Ala
            260                 265                 270

Val Ala Met Arg Gly Ser Asp Asn Asn Asn Ser Leu Ala Ala Ala Val
```

```
            275                     280                     285

Gly Thr Gln His His Thr Asn Asn Gln Ser Thr Asp Ser Leu Ala Asn
    290                     295                     300

Arg Val Gln Asn Ser Arg Gly Ala Ser Val Phe Pro Ala Thr Met Asn
305                     310                     315                     320

Leu Gln Ser Lys Glu Thr His Pro Lys Gln Ser Asn Asn Pro Phe Glu
                325                     330                     335

Phe Gly Leu Ile Ser Ser Asp Ser Leu Leu Asn Pro Ser His Lys Gln
            340                     345                     350

Ala Ser Tyr Ala Thr Ser Ser Lys Gly Phe Gly Ser Tyr Leu Asp Phe
        355                     360                     365

Gly Asn Gln Ala Lys His Ala Gly Asn His Asn Asn Val Asp Ser Trp
    370                     375                     380

Pro Glu Glu Leu Lys Ser Asp Trp Thr Gln Leu Ser Met Ser Ile Pro
385                     390                     395                     400

Met Ala Pro Ser Ser Pro Val Gln Asp Lys Leu Ala Leu Ser Pro Leu
                405                     410                     415

Arg Leu Ser Arg Glu Phe Asp Pro Ala Ile His Met Gly Leu Gly Val
            420                     425                     430

Asn Thr Glu Phe Leu Asp Pro Gly Lys Lys Thr Asn Asn Trp Ile Pro
        435                     440                     445

Ile Ser Trp Gly Asn Asn Asn Ser Met Gly Gly Pro Leu Gly Glu Val
    450                     455                     460

Leu Asn Ser Thr Thr Asn Ser Pro Lys Phe Gly Ser Ser Pro Thr Gly
465                     470                     475                     480

Val Leu Gln Lys Ser Thr Phe Gly Ser Leu Ser Asn Ser Ser Ser Ala
                485                     490                     495

Ser Ser Thr Ile Ile Gly Asp Asn Asn Asn Lys Asn Gly Asp Gly Lys
            500                     505                     510

Asp Pro Leu Gly Pro Thr Thr Leu Met Asn Thr Ser Ala Thr Ala Pro
        515                     520                     525

Ser Leu
    530
```

<210> 7
<211> 1197
<212> DNA

<213> Arabidopsis thaliana

<400> 7

atggatttgc aactgaaaca atggagaagc cagcagcagc aacaacatca gacagagtca 60
gaagaacaac cttctgcagc taagatacca aaacatgtct ttgaccagat tcattctcac 120
actgcaactt ctactgctct tcctctcttt acccctgagc ctacttcttc taaactctcc 180
tctttgtctc ctgattcttc ctccaggttc cccaagatgg ggagcttctt tagctgggca 240
cagtggcaag aacttgaact acaagctctg atctacaggt acatgttggc tggtgctgct 300
gttcctcagg agctcctttt accaatcaag aaaagccttc tccatctatc tccttcctac 360
tttcttcacc atcctcttca acacctacct cattaccaac ctgcttggta tttgggaagg 420
gcagcgatgg atcctgagcc aggcagatgc aggagaacgg atggtaagaa gtggagatgt 480
tcaagagacg tcttcgctgg ccacaagtat tgcgagcgcc acatgcaccg tggccgcaac 540
cgttcaagaa agcctgtgga aactccaacc accgtcaatg caactgccac gtccatggct 600
tcatcagtag cagccgcagc caccactaca acagcaacaa caacatctac gtttgctttt 660
ggtggtggtg gtggtagtga ggaagtggtt ggtcaaggag gatctttctt cttctctggc 720
tcttctaact cttcatctga acttctccac cttagtcaaa gttgttcgga gatgaagcaa 780
gaaagcaaca acatgaacaa caagaggcca tacgagtccc acatcggatt cagtaacaac 840
agatcagatg gaggacacat cctgaggccc ttctttgacg attggcctcg ttcttcgctc 900
caagaagctg acaatagttc aagccccatg agctcagcca cttgtctctc catctccatg 960
cccgggaact cttcctcaga cgtctctctg aagctgtcca caggcaacga agagggagcc 1020
cggagcaaca acaatgggag agatcagcaa aacatgagct ggtggagcgg tggaggttcc 1080
aaccaccatc atcacaacat gggcggacca ttggccgaag ccctgagatc ttcttcctca 1140
tcttccccaa ccagtgttct ccatcagctt ggtgtctcga cacaagcctt tcattga 1197

<210> 8
<211> 398
<212> PRT
<213> Arabidopsis thaliana

<400> 8

```
Met Asp Leu Gln Leu Lys Gln Trp Arg Ser Gln Gln Gln Gln Gln His
1               5                   10                  15

Gln Thr Glu Ser Glu Glu Gln Pro Ser Ala Ala Lys Ile Pro Lys His
            20                  25                  30

Val Phe Asp Gln Ile His Ser His Thr Ala Thr Ser Thr Ala Leu Pro
        35                  40                  45

Leu Phe Thr Pro Glu Pro Thr Ser Ser Lys Leu Ser Ser Leu Ser Pro
    50                  55                  60

Asp Ser Ser Ser Arg Phe Pro Lys Met Gly Ser Phe Phe Ser Trp Ala
```

65 70 75 80

Gln Trp Gln Glu Leu Glu Leu Gln Ala Leu Ile Tyr Arg Tyr Met Leu
85 90 95

Ala Gly Ala Ala Val Pro Gln Glu Leu Leu Leu Pro Ile Lys Lys Ser
100 105 110

Leu Leu His Leu Ser Pro Ser Tyr Phe Leu His His Pro Leu Gln His
115 120 125

Leu Pro His Tyr Gln Pro Ala Trp Tyr Leu Gly Arg Ala Ala Met Asp
130 135 140

Pro Glu Pro Gly Arg Cys Arg Arg Thr Asp Gly Lys Lys Trp Arg Cys
145 150 155 160

Ser Arg Asp Val Phe Ala Gly His Lys Tyr Cys Glu Arg His Met His
165 170 175

Arg Gly Arg Asn Arg Ser Arg Lys Pro Val Glu Thr Pro Thr Thr Val
180 185 190

Asn Ala Thr Ala Thr Ser Met Ala Ser Ser Val Ala Ala Ala Ala Thr
195 200 205

Thr Thr Thr Ala Thr Thr Thr Ser Thr Phe Ala Phe Gly Gly Gly Gly
210 215 220

Gly Ser Glu Glu Val Val Gly Gln Gly Gly Ser Phe Phe Phe Ser Gly
225 230 235 240

Ser Ser Asn Ser Ser Ser Glu Leu Leu His Leu Ser Gln Ser Cys Ser
245 250 255

Glu Met Lys Gln Glu Ser Asn Asn Met Asn Asn Lys Arg Pro Tyr Glu
260 265 270

Ser His Ile Gly Phe Ser Asn Asn Arg Ser Asp Gly Gly His Ile Leu
275 280 285

Arg Pro Phe Phe Asp Asp Trp Pro Arg Ser Ser Leu Gln Glu Ala Asp
290 295 300

Asn Ser Ser Ser Pro Met Ser Ser Ala Thr Cys Leu Ser Ile Ser Met
305 310 315 320

Pro Gly Asn Ser Ser Ser Asp Val Ser Leu Lys Leu Ser Thr Gly Asn
325 330 335

Glu Glu Gly Ala Arg Ser Asn Asn Asn Gly Arg Asp Gln Gln Asn Met
340 345 350

Ser Trp Trp Ser Gly Gly Gly Ser Asn His His His His Asn Met Gly
355 360 365

Gly Pro Leu Ala Glu Ala Leu Arg Ser Ser Ser Ser Ser Ser Pro Thr
370 375 380

Ser Val Leu His Gln Leu Gly Val Ser Thr Gln Ala Phe His
385 390 395

<210> 9
<211> 1290
<212> DNA
<213> Arabidopsis thaliana

<400> 9

atgcagagcc ctaaaatgga gcaggaggag gttgaggagg agaggatgag gaataagtgg 60
ccgtggatga aggcggcgca gttaatggag tttcggatgc aagctttggt gtatagatac 120
atagaggctg gtctccgtgt gcctcatcat ctcgtggtgc ctatttggaa cagtcttgct 180
ctctcttctt cctccaatta caactatcac tcttcttctc tgttgagtaa caagggagta 240
acccatatcg acacgttgga aactgaacca actaggtgca ggagaacaga tgggaagaaa 300
tggcgctgta gcaacacggt ccttctattc gagaagtact gtgaacggca catgcataga 360
ggtcgtaaac gttcaagaaa gcttgtggaa tcttcttctg aggttgcttc atcatcaacc 420
aaatacgaca acacttatgg tttggatagg tataacgaga gtcagagtca tcttcatggg 480
acaatctcgg gttctagtaa tgcgcaggta gttaccattg cttcactgcc tagtgccaga 540
tcctgtgaaa atgtcattcg tccgtcttta gtgatctctg aattcacaaa caaaagtgtg 600
agtcacggca gaaagaacat ggagatgagt tatgatgact ttattaatga aaaagaggcg 660
agtatgtgtg ttggagttgt tcctcttcaa ggtgatgaga gcaaaccttc ggttcaaaag 720
ttcttccctg aggtatctga taaatgctta gaagctgcaa aattctcaag caacaggaag 780
aatgatataa ttgcaagaag cagagaatgg aagaatatga atgttaatgg tggtttgttt 840
catggtatcc actttctcc agacactgtt cttcaagaac gtggttgttt tcgtttacaa 900
ggagttgaaa cagacaatga accaggaagg tgccgaagaa cagatgggaa gaagtggaga 960
tgcagcaaag atgttttgtc tggtcagaag tactgcgata agcacatgca tagaggtatg 1020
aagaagaagc atccagttga tactactaac tcacatgaga atgccgggtt tagcccgtta 1080
accgtggaaa cagctgttag atcggttgtg ccttgcaaag atggagatga ccagaagcat 1140
tctgtttcag tcatgggaat tacactgccc cgagtttctg atgagaagag cactagcagt 1200
tgcagtaccg acactaccat tactgacaca gctttaaggg gtgaagacga cgatgaggag 1260
tacttgtctt tgttttcacc aggtgtttag 1290

<210> 10
<211> 429
<212> PRT
<213> Arabidopsis thaliana

<400> 10

Met Gln Ser Pro Lys Met Glu Gln Glu Glu Val Glu Glu Glu Arg Met
1               5                   10                  15

Arg Asn Lys Trp Pro Trp Met Lys Ala Ala Gln Leu Met Glu Phe Arg
            20                  25                  30

Met Gln Ala Leu Val Tyr Arg Tyr Ile Glu Ala Gly Leu Arg Val Pro
        35                  40                  45

His His Leu Val Val Pro Ile Trp Asn Ser Leu Ala Leu Ser Ser Ser
    50                  55                  60

Ser Asn Tyr Asn Tyr His Ser Ser Ser Leu Leu Ser Asn Lys Gly Val
65                  70                  75                  80

Thr His Ile Asp Thr Leu Glu Thr Glu Pro Thr Arg Cys Arg Arg Thr
                85                  90                  95

Asp Gly Lys Lys Trp Arg Cys Ser Asn Thr Val Leu Leu Phe Glu Lys
            100                 105                 110

Tyr Cys Glu Arg His Met His Arg Gly Arg Lys Arg Ser Arg Lys Leu
        115                 120                 125

Val Glu Ser Ser Ser Glu Val Ala Ser Ser Ser Thr Lys Tyr Asp Asn
    130                 135                 140

Thr Tyr Gly Leu Asp Arg Tyr Asn Glu Ser Gln Ser His Leu His Gly
145                 150                 155                 160

Thr Ile Ser Gly Ser Ser Asn Ala Gln Val Val Thr Ile Ala Ser Leu
                165                 170                 175

Pro Ser Ala Arg Ser Cys Glu Asn Val Ile Arg Pro Ser Leu Val Ile
            180                 185                 190

Ser Glu Phe Thr Asn Lys Ser Val Ser His Gly Arg Lys Asn Met Glu
        195                 200                 205

Met Ser Tyr Asp Asp Phe Ile Asn Glu Lys Glu Ala Ser Met Cys Val
    210                 215                 220

Gly Val Val Pro Leu Gln Gly Asp Glu Ser Lys Pro Ser Val Gln Lys
225                 230                 235                 240

Phe Phe Pro Glu Val Ser Asp Lys Cys Leu Glu Ala Ala Lys Phe Ser
                245                 250                 255

Ser Asn Arg Lys Asn Asp Ile Ile Ala Arg Ser Arg Glu Trp Lys Asn
            260                 265                 270

Met Asn Val Asn Gly Gly Leu Phe His Gly Ile His Phe Ser Pro Asp
275 280 285

Thr Val Leu Gln Glu Arg Gly Cys Phe Arg Leu Gln Gly Val Glu Thr
290 295 300

Asp Asn Glu Pro Gly Arg Cys Arg Arg Thr Asp Gly Lys Lys Trp Arg
305 310 315 320

Cys Ser Lys Asp Val Leu Ser Gly Gln Lys Tyr Cys Asp Lys His Met
325 330 335

His Arg Gly Met Lys Lys Lys His Pro Val Asp Thr Thr Asn Ser His
340 345 350

Glu Asn Ala Gly Phe Ser Pro Leu Thr Val Glu Thr Ala Val Arg Ser
355 360 365

Val Val Pro Cys Lys Asp Gly Asp Asp Gln Lys His Ser Val Ser Val
370 375 380

Met Gly Ile Thr Leu Pro Arg Val Ser Asp Glu Lys Ser Thr Ser Ser
385 390 395 400

Cys Ser Thr Asp Thr Thr Ile Thr Asp Thr Ala Leu Arg Gly Glu Asp
405 410 415

Asp Asp Glu Glu Tyr Leu Ser Leu Phe Ser Pro Gly Val
420 425

<210> 11
<211> 1647
<212> DNA
<213> Arabidopsis thaliana

<400> 11

atggacttgc aactgaaaca atggagaagt cagcagcaga atgagtcaga agaacaaggc 60
tctgctgcaa ctaagatatc aaactttttc tttgatcaga ttcagtccca aactgctact 120
tctgctgctg cggctcctct tcctctcttt gtccctgaac ccacttcttc ctcttctttc 180
tcttgcttct ctcctgactc ttctaattct tcttcttctt ccaggttcct caagatggga 240
aacttcttca gctgggcaca gtggcaagaa cttgagctac aagcactgat ctatagatac 300
atgttggctg gtgcttctgt tcctcaagag cttctcttac ctattaagaa aagtctcctc 360
catcaatctc ctatgcattt ccttcaccat cctcttcaac atagttttcc tcatcaccaa 420
ccttcttggt attggggaag aggagcaatg gatcctgagc cagggaggtg taagagaact 480
gacggcaaga aatggagatg ttcaagggat gttgtagcgg gccacaagta ttgtgaccgc 540
cacattcacc gtggaagaaa ccgttcaaga aagcctgtgg aaaccgccac aaccaccatc 600
acaacgacag ccacaacaac cgcatcttct tttgtcttag gtgaggagct tggtcatgga 660

```
ccaaacaaca accacttctt ctcctctggt tcatctcaac ctctccacct tagtcatcaa      720
caaagttgtt cttcagagat gaaacaagaa agcaacaaca acaagaggcc atatgaagct      780
aacagtggat tcagcaatgg aagatcagac gatggtcaca tcttgaggca tttctttgac      840
gattggccac gatcatcaga ctctacctcc agtccaatga gctcatccac ttgtcatctt      900
tcaatctcca tgcccggtaa caacacgtcc tcagatgttt ctctaaaact ttccacaggc      960
aatgaagaag aagaagagaa catgagaaat aacaacaatg agagggagca aatgaattgg     1020
tggagcaatg gagggaatca ccacaacaat atgggaggac cattagctga ggctttgagg     1080
tcagcttctt cgacgtcaag tgttcttcat cagatgggaa tctctactca agaaatgaag     1140
tatgtgaagc cattgagctt attgggtaat gcgctgaaga ccaaagtgtc agtccctggt     1200
cggtttctgg gtttagatgt tggtgataag tatgttggat tagctatctc agatccttca     1260
aatatggttg cttctccatt gagtgttttg ctcagaaaga aatcaaacat tgacctgatg     1320
gctacagatt tccagaacct ggtcaaagca ttttctgtgt cgggattagt cgttggttat     1380
ccatttggca aactgaacaa tgtagaggat gttgtcactg tgaatctttt cattgaggaa     1440
cttcgtaaga ccgaaaaact caaggatgtg aaatacacat attgggacga gcgattatca     1500
tcaaagaccg ttgaactgat gttgaagccc ttgaatttgc atcctgttca agagaagaca     1560
atgttggaca agttagccgc agtagttata cttcaggagt atttagatta cgcgaacagg     1620
tatgtaaaca ctgagccagc agagtaa                                         1647
```

<210> 12
<211> 548
<212> PRT
<213> Arabidopsis thaliana

<400> 12

```
Met Asp Leu Gln Leu Lys Gln Trp Arg Ser Gln Gln Gln Asn Glu Ser
1               5                   10                  15

Glu Glu Gln Gly Ser Ala Ala Thr Lys Ile Ser Asn Phe Phe Phe Asp
            20                  25                  30

Gln Ile Gln Ser Gln Thr Ala Thr Ser Ala Ala Ala Ala Pro Leu Pro
        35                  40                  45

Leu Phe Val Pro Glu Pro Thr Ser Ser Ser Ser Phe Ser Cys Phe Ser
    50                  55                  60

Pro Asp Ser Ser Asn Ser Ser Ser Ser Ser Arg Phe Leu Lys Met Gly
65                  70                  75                  80

Asn Phe Phe Ser Trp Ala Gln Trp Gln Glu Leu Glu Leu Gln Ala Leu
                85                  90                  95

Ile Tyr Arg Tyr Met Leu Ala Gly Ala Ser Val Pro Gln Glu Leu Leu
            100                 105                 110
```

Leu Pro Ile Lys Lys Ser Leu Leu His Gln Ser Pro Met His Phe Leu
115 120 125

His His Pro Leu Gln His Ser Phe Pro His His Gln Pro Ser Trp Tyr
130 135 140

Trp Gly Arg Gly Ala Met Asp Pro Glu Pro Gly Arg Cys Lys Arg Thr
145 150 155 160

Asp Gly Lys Lys Trp Arg Cys Ser Arg Asp Val Val Ala Gly His Lys
165 170 175

Tyr Cys Asp Arg His Ile His Arg Gly Arg Asn Arg Ser Arg Lys Pro
180 185 190

Val Glu Thr Ala Thr Thr Thr Ile Thr Thr Thr Ala Thr Thr Thr Ala
195 200 205

Ser Ser Phe Val Leu Gly Glu Glu Leu Gly His Gly Pro Asn Asn Asn
210 215 220

His Phe Phe Ser Ser Gly Ser Ser Gln Pro Leu His Leu Ser His Gln
225 230 235 240

Gln Ser Cys Ser Ser Glu Met Lys Gln Glu Ser Asn Asn Asn Lys Arg
245 250 255

Pro Tyr Glu Ala Asn Ser Gly Phe Ser Asn Gly Arg Ser Asp Asp Gly
260 265 270

His Ile Leu Arg His Phe Phe Asp Asp Trp Pro Arg Ser Ser Asp Ser
275 280 285

Thr Ser Ser Pro Met Ser Ser Ser Thr Cys His Leu Ser Ile Ser Met
290 295 300

Pro Gly Asn Asn Thr Ser Ser Asp Val Ser Leu Lys Leu Ser Thr Gly
305 310 315 320

Asn Glu Glu Glu Glu Glu Asn Met Arg Asn Asn Asn Asn Glu Arg Glu
325 330 335

Gln Met Asn Trp Trp Ser Asn Gly Gly Asn His His Asn Asn Met Gly
340 345 350

Gly Pro Leu Ala Glu Ala Leu Arg Ser Ala Ser Ser Thr Ser Ser Val
355 360 365

Leu His Gln Met Gly Ile Ser Thr Gln Glu Met Lys Tyr Val Lys Pro
370 375 380

Leu Ser Leu Leu Gly Asn Ala Leu Lys Thr Lys Val Ser Val Pro Gly
385 390 395 400

Arg Phe Leu Gly Leu Asp Val Gly Asp Lys Tyr Val Gly Leu Ala Ile
405 410 415

Ser Asp Pro Ser Asn Met Val Ala Ser Pro Leu Ser Val Leu Leu Arg
420 425 430

Lys Lys Ser Asn Ile Asp Leu Met Ala Thr Asp Phe Gln Asn Leu Val
435 440 445

Lys Ala Phe Ser Val Ser Gly Leu Val Val Gly Tyr Pro Phe Gly Lys
450 455 460

Leu Asn Asn Val Glu Asp Val Val Thr Val Asn Leu Phe Ile Glu Glu
465 470 475 480

Leu Arg Lys Thr Glu Lys Leu Lys Asp Val Lys Tyr Thr Tyr Trp Asp
485 490 495

Glu Arg Leu Ser Ser Lys Thr Val Glu Leu Met Leu Lys Pro Leu Asn
500 505 510

Leu His Pro Val Gln Glu Lys Thr Met Leu Asp Lys Leu Ala Ala Val
515 520 525

Val Ile Leu Gln Glu Tyr Leu Asp Tyr Ala Asn Arg Tyr Val Asn Thr
530 535 540

Glu Pro Ala Glu
545

<210> 13
<211> 1482
<212> DNA
<213> Arabidopsis thaliana

<400> 13

atgaggatgc ttcttgggat tccttacgta gacaagtcgg ttctttccaa ctctgttctt 60
gagagaggca agcaggataa aagcaaacta ttgttagtcg acaaatgcca ttatgagctt 120
gatgttgaag aacgcaagga agattttgtt ggtgggtttg gatttggtgt tgtagaaaat 180
tcgcataaag acgttatggt gctacctcat catcactatt atccatcata ttcatcacct 240
tcctcttctt ctttgtgtta ctgttctgct ggtgttagcg atcccatgtt ctctgtttct 300
agcaatcagg cttacacttc ttctcacagt ggtatgttca cacccgccgg ttctggttct 360
gctgctgtga ctgtagcaga tcctttttc tccttgagct cttcagggga aatgagaaga 420
agtatgaacg aagatgctgg tgcagctttc agcgaagctc aatggcatga gcttgagagg 480
cagaggaata tatacaagta catgatggct tctgttcctg ttcctccaga gcttctcaca 540

```
cccttccca agaaccacca atcaaacact aacccggatg tggatacata taggagtgga 600
atgtttagta tttatgctga ttacaagaat ctgccgttgt ctatgtggat gacagtaact 660
gtggcagtgg cgacaggagg ctcattgcag ctggggattg cttcaagcgc aagcaataac 720
acggctgatc tggagccatg gaggtgcaag agaacagatg ggaagaaatg gaggtgctct 780
agaaacgtga ttcctgatca gaaatactgt gagagacaca cacacaagag ccgtcctcgt 840
tcaagaaagc atgtggaatc atctcaccaa tcatctcacc acaatgacat tcgtacggct 900
aagaatgata ctagccagct tgtgagaact tatcctcagt tttacggaca acctataagc 960
cagatccctg tgctttctac tcttccgtct gcctcctctc catatgatca ccacagagga 1020
ctgaggtggt ttacgaaaga agatgatgcc attggaacct taaacccgga gactcaagaa 1080
gctgtccagc tgaaagttgg atcaagcaga gagctcaaac ggggattcga ttatgatctg 1140
aatttcaggc agaaagagcc aatagtagac cagagctttg gagcattgca gggtctatta 1200
agtctaaacc agacaccaca acataaccaa gaaacaagac agtttgttgt agaaggaaag 1260
caagatgaag cgatgggaag ctctctgaca ctctcaatgg ctggaggagg catggaggaa 1320
acagagggaa caaaccagca tcagtgggtt agccatgaag gtccatcatg gctctattca 1380
acaacaccag gtggaccatt ggctgaagca ctgtgtctcg gtgtctccaa caacccaagt 1440
tctagtacta ctactagtag ctgcagcaga agctcaagct aa 1482
```

<210> 14
<211> 493
<212> PRT
<213> Arabidopsis thaliana

<400> 14

Met Arg Met Leu Leu Gly Ile Pro Tyr Val Asp Lys Ser Val Leu Ser
1               5                   10                  15

Asn Ser Val Leu Glu Arg Gly Lys Gln Asp Lys Ser Lys Leu Leu Leu
            20                  25                  30

Val Asp Lys Cys His Tyr Glu Leu Asp Val Glu Glu Arg Lys Glu Asp
        35                  40                  45

Phe Val Gly Gly Phe Gly Phe Gly Val Val Glu Asn Ser His Lys Asp
    50                  55                  60

Val Met Val Leu Pro His His His Tyr Tyr Pro Ser Tyr Ser Ser Pro
65                  70                  75                  80

Ser Ser Ser Ser Leu Cys Tyr Cys Ser Ala Gly Val Ser Asp Pro Met
                85                  90                  95

Phe Ser Val Ser Ser Asn Gln Ala Tyr Thr Ser Ser His Ser Gly Met
            100                 105                 110

Phe Thr Pro Ala Gly Ser Gly Ser Ala Ala Val Thr Val Ala Asp Pro

115 120 125

Phe Phe Ser Leu Ser Ser Ser Gly Glu Met Arg Arg Ser Met Asn Glu
130 135 140

Asp Ala Gly Ala Ala Phe Ser Glu Ala Gln Trp His Glu Leu Glu Arg
145 150 155 160

Gln Arg Asn Ile Tyr Lys Tyr Met Met Ala Ser Val Pro Val Pro Pro
165 170 175

Glu Leu Leu Thr Pro Phe Pro Lys Asn His Gln Ser Asn Thr Asn Pro
180 185 190

Asp Val Asp Thr Tyr Arg Ser Gly Met Phe Ser Ile Tyr Ala Asp Tyr
195 200 205

Lys Asn Leu Pro Leu Ser Met Trp Met Thr Val Thr Val Ala Val Ala
210 215 220

Thr Gly Gly Ser Leu Gln Leu Gly Ile Ala Ser Ser Ala Ser Asn Asn
225 230 235 240

Thr Ala Asp Leu Glu Pro Trp Arg Cys Lys Arg Thr Asp Gly Lys Lys
245 250 255

Trp Arg Cys Ser Arg Asn Val Ile Pro Asp Gln Lys Tyr Cys Glu Arg
260 265 270

His Thr His Lys Ser Arg Pro Arg Ser Arg Lys His Val Glu Ser Ser
275 280 285

His Gln Ser Ser His His Asn Asp Ile Arg Thr Ala Lys Asn Asp Thr
290 295 300

Ser Gln Leu Val Arg Thr Tyr Pro Gln Phe Tyr Gly Gln Pro Ile Ser
305 310 315 320

Gln Ile Pro Val Leu Ser Thr Leu Pro Ser Ala Ser Ser Pro Tyr Asp
325 330 335

His His Arg Gly Leu Arg Trp Phe Thr Lys Glu Asp Asp Ala Ile Gly
340 345 350

Thr Leu Asn Pro Glu Thr Gln Glu Ala Val Gln Leu Lys Val Gly Ser
355 360 365

Ser Arg Glu Leu Lys Arg Gly Phe Asp Tyr Asp Leu Asn Phe Arg Gln
370 375 380

Lys Glu Pro Ile Val Asp Gln Ser Phe Gly Ala Leu Gln Gly Leu Leu
385 390 395 400

Ser Leu Asn Gln Thr Pro Gln His Asn Gln Glu Thr Arg Gln Phe Val
405 410 415

Val Glu Gly Lys Gln Asp Glu Ala Met Gly Ser Ser Leu Thr Leu Ser
420 425 430

Met Ala Gly Gly Gly Met Glu Glu Thr Glu Gly Thr Asn Gln His Gln
435 440 445

Trp Val Ser His Glu Gly Pro Ser Trp Leu Tyr Ser Thr Thr Pro Gly
450 455 460

Gly Pro Leu Ala Glu Ala Leu Cys Leu Gly Val Ser Asn Asn Pro Ser
465 470 475 480

Ser Ser Thr Thr Thr Ser Ser Cys Ser Arg Ser Ser Ser
485 490

<210> 15
<211> 1608
<212> DNA
<213> Arabidopsis thaliana

<400> 15

atggatattg gtgttcatgt tcttgggtcg gttactagta atgaaaatga gtcacttggt 60
ctaaaagagc ttataggaac taaacaagat agatccggat tcatcggtga ggattgcttg 120
caacgaagct tgaagctagc aagaacgaca actagagcgg aagaagaaga aaacttgtct 180
tcttctgttg cagctgctta ttgcaaaacg atgtcgtttc accaaggcat tcctctcatg 240
agatctgctt ctcctctttc ctctgattct cgccgtcaag aacaaatgct tagcttctca 300
gataaaccag acgctcttga tttcagtaaa tatgtcggtt tggataatag cagtaataac 360
aagaactctc tctcgccgtt tcttcaccag attcctccac cttcttactt tagaagctca 420
ggaggatatg gttctggtgg aatgatgatg aacatgagca tgcaagggaa cttcacaggt 480
gttaaaggac cttttacatt gactcaatgg gctgagttag agcaacaggc gttgatctat 540
aagtacatca cagccaatgt ccctgttcct tctagtttgc tcatctctat caagaagtct 600
ttttatcctt acggatcttt gcctcctagt tccttcggat ggggaacttt ccatctcggt 660
ttcgcaggcg gtaacatgga ccctgagcca gggagatgcc gcagaacaga tgggaagaaa 720
tggcggtgct caagagacgc cgttcctgat cagaaatact gtgaaagaca catcaacaga 780
ggccgtcatc gttcaagaaa gcctgtggaa gtccaatctg gccaaaacca aaccgccgct 840
gctgcatcca aagcggttac tacaccacaa cagcctgttg tcgctggtaa tactaacaga 900
agcaatgccc gtgcatcaag caaccgcagc ctcgccattg gaagtcaata tatcaatcct 960
tctacagaat ctttacctaa caacagagga gtttcgatat atccttccac cgtcaactta 1020
caacccaagg aatctccggt tattcatcag aaacacagaa acaacaacaa cccttttgag 1080
tttggacaca tatcctctga ttcgttactc aacccgaata ccgcaaagac ctatggatca 1140

```
tcgttcttgg atttcagcag caaccaagag aagcattcag ggaatcacaa tcacaattct   1200
tggcctgaag agctgacatc agattggaca cagctctcaa tgtcaattcc aatagcatca   1260
tcatcccctt cctccacaca caacaacaac aatgctcaag aaaaaacaac actctcgcct   1320
ctcaggctat cccgcgagct tgacctatcg atccaaaccg atgaaacaac aatcgagcct   1380
actgtgaaaa aggtgaatac ttggatacca atctcatggg gaaactcctt aggaggtcct   1440
ctaggtgaag tactaaacag tacaacgaat agtccaacat ttggatcttc tcctacaggg   1500
gttttgcaaa agtccacatt ttgttcactc tctaacaaca gctccgtgag cagccccatt   1560
gcagagaaca acagacacaa tggcgattac tttcattaca caacctga                1608
```

<210> 16
<211> 535
<212> PRT
<213> Arabidopsis thaliana

<400> 16

```
Met Asp Ile Gly Val His Val Leu Gly Ser Val Thr Ser Asn Glu Asn
1               5                   10                  15

Glu Ser Leu Gly Leu Lys Glu Leu Ile Gly Thr Lys Gln Asp Arg Ser
            20                  25                  30

Gly Phe Ile Gly Glu Asp Cys Leu Gln Arg Ser Leu Lys Leu Ala Arg
        35                  40                  45

Thr Thr Thr Arg Ala Glu Glu Glu Glu Asn Leu Ser Ser Ser Val Ala
    50                  55                  60

Ala Ala Tyr Cys Lys Thr Met Ser Phe His Gln Gly Ile Pro Leu Met
65                  70                  75                  80

Arg Ser Ala Ser Pro Leu Ser Ser Asp Ser Arg Arg Gln Glu Gln Met
                85                  90                  95

Leu Ser Phe Ser Asp Lys Pro Asp Ala Leu Asp Phe Ser Lys Tyr Val
            100                 105                 110

Gly Leu Asp Asn Ser Ser Asn Asn Lys Asn Ser Leu Ser Pro Phe Leu
        115                 120                 125

His Gln Ile Pro Pro Pro Ser Tyr Phe Arg Ser Ser Gly Gly Tyr Gly
    130                 135                 140

Ser Gly Gly Met Met Met Asn Met Ser Met Gln Gly Asn Phe Thr Gly
145                 150                 155                 160

Val Lys Gly Pro Phe Thr Leu Thr Gln Trp Ala Glu Leu Glu Gln Gln
                165                 170                 175
```

Ala Leu Ile Tyr Lys Tyr Ile Thr Ala Asn Val Pro Val Pro Ser Ser
180 185 190

Leu Leu Ile Ser Ile Lys Lys Ser Phe Tyr Pro Tyr Gly Ser Leu Pro
195 200 205

Pro Ser Ser Phe Gly Trp Gly Thr Phe His Leu Gly Phe Ala Gly Gly
210 215 220

Asn Met Asp Pro Glu Pro Gly Arg Cys Arg Arg Thr Asp Gly Lys Lys
225 230 235 240

Trp Arg Cys Ser Arg Asp Ala Val Pro Asp Gln Lys Tyr Cys Glu Arg
245 250 255

His Ile Asn Arg Gly Arg His Arg Ser Arg Lys Pro Val Glu Val Gln
260 265 270

Ser Gly Gln Asn Gln Thr Ala Ala Ala Ala Ser Lys Ala Val Thr Thr
275 280 285

Pro Gln Gln Pro Val Val Ala Gly Asn Thr Asn Arg Ser Asn Ala Arg
290 295 300

Ala Ser Ser Asn Arg Ser Leu Ala Ile Gly Ser Gln Tyr Ile Asn Pro
305 310 315 320

Ser Thr Glu Ser Leu Pro Asn Asn Arg Gly Val Ser Ile Tyr Pro Ser
325 330 335

Thr Val Asn Leu Gln Pro Lys Glu Ser Pro Val Ile His Gln Lys His
340 345 350

Arg Asn Asn Asn Asn Pro Phe Glu Phe Gly His Ile Ser Ser Asp Ser
355 360 365

Leu Leu Asn Pro Asn Thr Ala Lys Thr Tyr Gly Ser Ser Phe Leu Asp
370 375 380

Phe Ser Ser Asn Gln Glu Lys His Ser Gly Asn His Asn His Asn Ser
385 390 395 400

Trp Pro Glu Glu Leu Thr Ser Asp Trp Thr Gln Leu Ser Met Ser Ile
405 410 415

Pro Ile Ala Ser Ser Ser Pro Ser Ser Thr His Asn Asn Asn Asn Ala
420 425 430

Gln Glu Lys Thr Thr Leu Ser Pro Leu Arg Leu Ser Arg Glu Leu Asp
435 440 445

Leu Ser Ile Gln Thr Asp Glu Thr Thr Ile Glu Pro Thr Val Lys Lys

450 455 460

Val Asn Thr Trp Ile Pro Ile Ser Trp Gly Asn Ser Leu Gly Gly Pro
465 470 475 480

Leu Gly Glu Val Leu Asn Ser Thr Thr Asn Ser Pro Thr Phe Gly Ser
485 490 495

Ser Pro Thr Gly Val Leu Gln Lys Ser Thr Phe Cys Ser Leu Ser Asn
500 505 510

Asn Ser Ser Val Ser Ser Pro Ile Ala Glu Asn Asn Arg His Asn Gly
515 520 525

Asp Tyr Phe His Tyr Thr Thr
530 535

<210> 17
<211> 1098
<212> DNA
<213> Arabidopsis thaliana

<400> 17

```
atggactttc tcaaagtttc agacaagaca acaattccat atagaagtga ttctttgttt     60
agtttgaatc agcaacaata caaagagtct tcttttggat tcagagacat ggagattcat    120
ccgcatccta ctccatatgc aggaaatgga cttttgggtt gttattacta ttaccctttc    180
acaaacgcac aattgaagga gcttgagaga caagcaatga tctacaagta catgatcgca    240
tctattcctg ttcctttcga tctacttgtt tcttcaccat cctctgcctc tccttgtaac    300
aataaaaaca tcgccggaga tttagagccg ggaagatgcc ggagaacaga cggaaagaaa    360
tggagatgcg cgaaagaagt cgtctctaat cacaaatact gtgagaaaca cttacacaga    420
ggtcgtcctc gttcaagaaa gcatgtggaa cctccttatt ctcgccctaa caacaatggt    480
ggttctgtga aaaacagaga tctcaaaaag cttcctcaaa agttatctag tagttccatc    540
aaagacaaaa cacttgagcc aatggaggtt tcatcatcaa tctcaaacta tagagactcc    600
agaggaagtg agaaatttac tgtattggca acaacagagc aagagaacaa gtatctgaat    660
ttcatagatg tatggtccga tggagtaaga tcatctgaaa aacagagtac aacttcaaca    720
cctgtttctt cttccaatgg caatctctct ctttactcgc ttgatctctc aatgggagga    780
aacaacttaa tgggccaaga cgaaatgggc ctgatacaaa tgggcttagg tgtaatcggg    840
tcgggtagtg aggatcatca cgggtatggt ccttatggtg tgacttcttc actagaggag    900
atgtcaagct ggcttgctcc gatgtctacc acacctggtg gaccattagc ggagatactg    960
aggccgagta cgaatttggc gatctctggt gatatcgaat cgtatagctt gatggagact   1020
cccactccaa gctcgtcccc gtctagagtg atgaagaaga tgactagttc agtgtccgac   1080
gaaagcagcc aggtttag                                                 1098
```

<210> 18
<211> 365
<212> PRT
<213> Arabidopsis thaliana

<400> 18

```
Met Asp Phe Leu Lys Val Ser Asp Lys Thr Thr Ile Pro Tyr Arg Ser
1               5                   10                  15

Asp Ser Leu Phe Ser Leu Asn Gln Gln Gln Tyr Lys Glu Ser Ser Phe
            20                  25                  30

Gly Phe Arg Asp Met Glu Ile His Pro His Pro Thr Pro Tyr Ala Gly
        35                  40                  45

Asn Gly Leu Leu Gly Cys Tyr Tyr Tyr Tyr Pro Phe Thr Asn Ala Gln
    50                  55                  60

Leu Lys Glu Leu Glu Arg Gln Ala Met Ile Tyr Lys Tyr Met Ile Ala
65                  70                  75                  80

Ser Ile Pro Val Pro Phe Asp Leu Leu Val Ser Ser Pro Ser Ser Ala
                85                  90                  95

Ser Pro Cys Asn Asn Lys Asn Ile Ala Gly Asp Leu Glu Pro Gly Arg
            100                 105                 110

Cys Arg Arg Thr Asp Gly Lys Lys Trp Arg Cys Ala Lys Glu Val Val
        115                 120                 125

Ser Asn His Lys Tyr Cys Glu Lys His Leu His Arg Gly Arg Pro Arg
    130                 135                 140

Ser Arg Lys His Val Glu Pro Pro Tyr Ser Arg Pro Asn Asn Asn Gly
145                 150                 155                 160

Gly Ser Val Lys Asn Arg Asp Leu Lys Lys Leu Pro Gln Lys Leu Ser
                165                 170                 175

Ser Ser Ser Ile Lys Asp Lys Thr Leu Glu Pro Met Glu Val Ser Ser
            180                 185                 190

Ser Ile Ser Asn Tyr Arg Asp Ser Arg Gly Ser Glu Lys Phe Thr Val
        195                 200                 205

Leu Ala Thr Thr Glu Gln Glu Asn Lys Tyr Leu Asn Phe Ile Asp Val
    210                 215                 220

Trp Ser Asp Gly Val Arg Ser Ser Glu Lys Gln Ser Thr Thr Ser Thr
225                 230                 235                 240

Pro Val Ser Ser Ser Asn Gly Asn Leu Ser Leu Tyr Ser Leu Asp Leu
                245                 250                 255
```

Ser Met Gly Gly Asn Asn Leu Met Gly Gln Asp Glu Met Gly Leu Ile
260 265 270

Gln Met Gly Leu Gly Val Ile Gly Ser Gly Ser Glu Asp His His Gly
275 280 285

Tyr Gly Pro Tyr Gly Val Thr Ser Ser Leu Glu Glu Met Ser Ser Trp
290 295 300

Leu Ala Pro Met Ser Thr Thr Pro Gly Gly Pro Leu Ala Glu Ile Leu
305 310 315 320

Arg Pro Ser Thr Asn Leu Ala Ile Ser Gly Asp Ile Glu Ser Tyr Ser
325 330 335

Leu Met Glu Thr Pro Thr Pro Ser Ser Ser Pro Ser Arg Val Met Lys
340 345 350

Lys Met Thr Ser Ser Val Ser Asp Glu Ser Ser Gln Val
355 360 365

<210> 19
<211> 1658
<212> DNA
<213> Aquilegia formosa x Aquilegia pubescens

<400> 19

acttaaaaga ccagtcttag ctttcttcat taattcctac tactgttctc agtgttgctc 60
tttgagttta tagatatttt tcttacaatg atgatgagtg ctagaaacag aaatcctttc 120
actgtaactc aatggcaaga acttgaacat caagctctca tttataagta tatggcttca 180
ggaatgccta taccacctga tctcatcttc cctattaaga gaagtcttga ttcttcaaga 240
ttctttcctc atcaaccaat ggattggggt tgttttcaga tgggttatgg caggaaagtt 300
gatccagaac ctggaaggtg cagaagaaca gatggaaaga agtggagatg ctcaaaggaa 360
gcatacccag actcaaagta ctgtgagaga cacatgcaca gaggcagaaa ccgttcaaga 420
aagcctgtgg aagttaatac tacatcaaat tcctcattac cactttcatc ttttacctct 480
agaactcctt ctagtaccat tacttcaaat accaaccctt cttcttattc cctttcttca 540
tctctaacat ctgacaaatc tcagcaagaa catcatcacc cttatcataa cacccctctt 600
cattcctttc tcaatcctag tagaacttct tgttcttctc ctagaactca taatattgat 660
ttctcacctc atagcaataa caatgccaat ttggtattag actctgggtc ttactctaac 720
tcttatgaag atcacagaaa caggtatgtt catggtctaa aagaagaggt agatgaaaga 780
gctttctttt cagaagcatc aggaacatta agaagtgtac cagaatcaac tttgaaagat 840
ccatggcgtt taacaccatt aagaatgagt tcttcaactc ataaccaacc aaaagatgga 900
aatttttctg atttacaaag agggtattct cagtttcaac tccaacataa acaacaacaa 960
cagcaacaag aagaagaaca gcattgtttt attttaggta ctgatttcaa atctgacagg 1020

Page 23

tttatgaaaa ctagtactac tactactgag aaagaagaat cacaacaacc acttcgccat 1080
ttctttgatg aatggccacc taagagtaaa gattcttggt tgggtttaga agaagataga 1140
tcagatcaag gttcacattc aacaactcaa ctttcaatat ctattcctat gtcttctcat 1200
gagttctcag tttcaaattc cagaacctaa caataagatg atggttgatt tacttaaagt 1260
gggattatta tggaaagatt aatgacaaca aggagttgat tcaaggttgg gttcagtgtc 1320
tttgtacttg tattgtcttt atttaattga tgatgagaag tttaggtaga gagtgctatg 1380
tgttattttt tttttgtta tgtgtgtgga gtgattgaaa agtgtgtctt taaacagtaa 1440
gattcctgtc ttgtgttttc ttgatagctg ttagaacttt gtttgaatga ctgatgaaca 1500
aatatttggg atttggggat ttgtttgtat caatattagg tgttttttct gtctttttgg 1560
cttcttccat gattgccaaa gaccatttgt tcaacctaaa aatgataatg aagggggggc 1620
caatttgata tcatgagctt ggttgtcagt taggaaag 1658

<210> 20
<211> 377
<212> PRT
<213> Aquilegia formosa x Aquilegia pubescens

<400> 20

```
Met Met Met Ser Ala Arg Asn Arg Asn Pro Phe Thr Val Thr Gln Trp
1               5                   10                  15

Gln Glu Leu Glu His Gln Ala Leu Ile Tyr Lys Tyr Met Ala Ser Gly
            20                  25                  30

Met Pro Ile Pro Pro Asp Leu Ile Phe Pro Ile Lys Arg Ser Leu Asp
        35                  40                  45

Ser Ser Arg Phe Phe Pro His Gln Pro Met Asp Trp Gly Cys Phe Gln
    50                  55                  60

Met Gly Tyr Gly Arg Lys Val Asp Pro Glu Pro Gly Arg Cys Arg Arg
65                  70                  75                  80

Thr Asp Gly Lys Lys Trp Arg Cys Ser Lys Glu Ala Tyr Pro Asp Ser
                85                  90                  95

Lys Tyr Cys Glu Arg His Met His Arg Gly Arg Asn Arg Ser Arg Lys
            100                 105                 110

Pro Val Glu Val Asn Thr Thr Ser Asn Ser Ser Leu Pro Leu Ser Ser
        115                 120                 125

Phe Thr Ser Arg Thr Pro Ser Ser Thr Ile Thr Ser Asn Thr Asn Pro
    130                 135                 140

Ser Ser Tyr Ser Leu Ser Ser Ser Leu Thr Ser Asp Lys Ser Gln Gln
145                 150                 155                 160
```

Glu His His His Pro Tyr His Asn Thr Pro Leu His Ser Phe Leu Asn
165 170 175

Pro Ser Arg Thr Ser Cys Ser Ser Pro Arg Thr His Asn Ile Asp Phe
180 185 190

Ser Pro His Ser Asn Asn Asn Ala Asn Leu Val Leu Asp Ser Gly Ser
195 200 205

Tyr Ser Asn Ser Tyr Glu Asp His Arg Asn Arg Tyr Val His Gly Leu
210 215 220

Lys Glu Glu Val Asp Glu Arg Ala Phe Phe Ser Glu Ala Ser Gly Thr
225 230 235 240

Leu Arg Ser Val Pro Glu Ser Thr Leu Lys Asp Pro Trp Arg Leu Thr
245 250 255

Pro Leu Arg Met Ser Ser Ser Thr His Asn Gln Pro Lys Asp Gly Asn
260 265 270

Phe Ser Asp Leu Gln Arg Gly Tyr Ser Gln Phe Gln Leu Gln His Lys
275 280 285

Gln Gln Gln Gln Gln Gln Glu Glu Glu Gln His Cys Phe Ile Leu Gly
290 295 300

Thr Asp Phe Lys Ser Asp Arg Phe Met Lys Thr Ser Thr Thr Thr Thr
305 310 315 320

Glu Lys Glu Glu Ser Gln Gln Pro Leu Arg His Phe Phe Asp Glu Trp
325 330 335

Pro Pro Lys Ser Lys Asp Ser Trp Leu Gly Leu Glu Glu Asp Arg Ser
340 345 350

Asp Gln Gly Ser His Ser Thr Thr Gln Leu Ser Ile Ser Ile Pro Met
355 360 365

Ser Ser His Glu Phe Ser Val Ser Asn
370 375

<210> 21
<211> 1286
<212> DNA
<213> Brassica napus

<400> 21

gaagaaagat gatgggtcta agtggaaatg gtgggagaac aatagagagg cctccattta 60
caccaacaca atggcaagaa ctggagaatc aagccctaat ttacaagtac atggtctcag 120
gagttcctgt cccacctgag ctcatcttct ccattagaag aagcttggac tcttccttgg 180

```
tctctagact cctccctcac caatccattg ggtggggatg ctatcagatg gggtttggta     240
gaaaaccaga tccagaacca ggaaggtgca gaagaacaga tggtaagaaa tggagatgct     300
caagagaagc ataccctgat tcaaagtact gtgaaaaaca catgcacaga ggaaggaacc     360
gtgccagaaa atctattgat cagaatcaga caactgctcc tttaacatca ccatctctct     420
ctttccccaa caacaacaac ccaagcccta ccttgtcttc ttcctcctct acttattcag     480
ctgcttcttc atctccttcc attgatgctt acagtaatat caataggctt ggtgttggta     540
gtagtaacag tagaggttac ttcaacaacc attcccttga ctatccttat cctttgtcct     600
cacctaaaca gcaacaacaa cagcaacaaa ctcttagtca tgtttctgct ttgtcacttc     660
atcaaaacac atctacacct cagctcaatg tctttgcctc tgcaactgac cacaaagact     720
tcagatattt tcaagggatt ggggagagag ttggagttgg ggaaagaact tttttccag      780
aagcttctag aagctttcaa gattctccat accatcacca acaaccgtta gcaacggtag     840
tggataatcc gtacgactgt actactgatc ataagtttga tcatcatcat acatactcat     900
catcatctca acatcatcat catgaccaag atcatcgaca acaacaacaa tgttttgttt     960
tgggcgccga catgttcaac aaacccacaa gaactatctt ggaaaacaca tcgagacaag    1020
attatcttaa tcaagaagag gaagagaaag attcatcgga cacgaagaag tcccttcatc    1080
atttctttgg tgaagagtgg acacagaaca agaacagttc agattcttgg cttgaccttt    1140
cttcccagtc aagactcgac actggtagct gattgatgag gccagatagc atcagtgatg    1200
ggtctgcacc aacacacaca caaacacgtt tgaagggtca catttcacat ctatttccgt    1260
ggaacattga gacagacaag acactg                                         1286
```

<210> 22
<211> 387
<212> PRT
<213> Brassica napus

<400> 22

```
Met Met Gly Leu Ser Gly Asn Gly Gly Arg Thr Ile Glu Arg Pro Pro
1               5                   10                  15

Phe Thr Pro Thr Gln Trp Gln Glu Leu Glu Asn Gln Ala Leu Ile Tyr
            20                  25                  30

Lys Tyr Met Val Ser Gly Val Pro Val Pro Pro Glu Leu Ile Phe Ser
        35                  40                  45

Ile Arg Arg Ser Leu Asp Ser Ser Leu Val Ser Arg Leu Leu Pro His
    50                  55                  60

Gln Ser Ile Gly Trp Gly Cys Tyr Gln Met Gly Phe Gly Arg Lys Pro
65                  70                  75                  80

Asp Pro Glu Pro Gly Arg Cys Arg Arg Thr Asp Gly Lys Lys Trp Arg
                85                  90                  95
```

Cys Ser Arg Glu Ala Tyr Pro Asp Ser Lys Tyr Cys Glu Lys His Met
100 105 110

His Arg Gly Arg Asn Arg Ala Arg Lys Ser Ile Asp Gln Asn Gln Thr
115 120 125

Thr Ala Pro Leu Thr Ser Pro Ser Leu Ser Phe Pro Asn Asn Asn Asn
130 135 140

Pro Ser Pro Thr Leu Ser Ser Ser Ser Ser Thr Tyr Ser Ala Ala Ser
145 150 155 160

Ser Ser Pro Ser Ile Asp Ala Tyr Ser Asn Ile Asn Arg Leu Gly Val
165 170 175

Gly Ser Ser Asn Ser Arg Gly Tyr Phe Asn Asn His Ser Leu Asp Tyr
180 185 190

Pro Tyr Pro Leu Ser Ser Pro Lys Gln Gln Gln Gln Gln Gln Gln Thr
195 200 205

Leu Ser His Val Ser Ala Leu Ser Leu His Gln Asn Thr Ser Thr Pro
210 215 220

Gln Leu Asn Val Phe Ala Ser Ala Thr Asp His Lys Asp Phe Arg Tyr
225 230 235 240

Phe Gln Gly Ile Gly Glu Arg Val Gly Val Gly Glu Arg Thr Phe Phe
245 250 255

Pro Glu Ala Ser Arg Ser Phe Gln Asp Ser Pro Tyr His His Gln Gln
260 265 270

Pro Leu Ala Thr Val Val Asp Asn Pro Tyr Asp Cys Thr Thr Asp His
275 280 285

Lys Phe Asp His His His Thr Tyr Ser Ser Ser Ser Gln His His His
290 295 300

His Asp Gln Asp His Arg Gln Gln Gln Gln Cys Phe Val Leu Gly Ala
305 310 315 320

Asp Met Phe Asn Lys Pro Thr Arg Thr Ile Leu Glu Asn Thr Ser Arg
325 330 335

Gln Asp Tyr Leu Asn Gln Glu Glu Glu Glu Lys Asp Ser Ser Asp Thr
340 345 350

Lys Lys Ser Leu His His Phe Phe Gly Glu Glu Trp Thr Gln Asn Lys
355 360 365

Asn Ser Ser Asp Ser Trp Leu Asp Leu Ser Ser Gln Ser Arg Leu Asp
370 375 380

Thr Gly Ser
385

<210> 23
<211> 1559
<212> DNA
<213> Hordeum vulgare

<400> 23

```
gggcagccgc agccgcagcc gcagcagagg agagagagag ggagggagaa gcatatatgg     60
cgatgccctt tgcctccctg tcgccggcag ccgaccacca ccgctcctcc cccatcttcc    120
ccttctgccg ctcctcccct ctctactcgg taggggagga ggcggcgcat cagcatcctc    180
atcctcagca gcagcagcag cagcacgcga tgagcggcgc gcggtgggcg gcgaggccgg    240
cgcccttcac ggcggcgcag tacgaggagc tggagcagca ggcgctcatc tacaagtacc    300
tcgtcgccgg cgtccccgtc ccgcaggacc tcctcctccc catccgccgc ggcttcgaga    360
ccctcgcctc gcgcttctac caccaccacg cccttgggta cgggtcctac ttcgggaaga    420
agctggatcc ggagccgggg cggtgccggc ggacggacgg caagaagtgg cggtgctcca    480
aggaggccgc tcaggactcc aagtactgcg agcgccacat gcaccgcggc cgcaaccgtt    540
caagaaagcc tgtggaaacg cagctcgtcg ccagctccca ctcccagtcc cagcagcacg    600
ccaccgccgc cttccacaac cactcgccgt atccggcgat cgccactggc ggtggctcct    660
tcgccctggg gtctgctcag ctgcacatgg acactgctgc gccttacgcg acgaccgccg    720
gtgctgccgg aaacaaagat ttcaggtatt ctgcctatgg agtgaggacg tcggcgatcg    780
aggagcacaa ccagttcatc accgcggcca tggacaccgc catggacaac tactcgtggc    840
gcctgatgcc gtcccaggcc tcggcattct cgctctccag ctaccccatg ctgggcacgc    900
tgagcgacct ggaccagagc gcgatctgct cgctggccaa gactgagagg gagccactgt    960
ccttcttcgg cggcggcggc gacttcgacg acgactcggc tgcggtgaag caggagaacc   1020
agacgctgcg gcccttcttc gacgagtggc ccaaggacag ggactcgtgg ccggagctgc   1080
aagaccacga cgccaacaac aacagcaacg ccttctcagc caccaagctg tccatctcca   1140
tgccggtcac cagctccgac ttctctggca ccaccgccgg ctcccgctcg cccaacggta   1200
tatactcccg gtgaacggcg tcggccggcc tgatctctgc tgatttgccg tggtcacgac   1260
gggcgtcctc aaatcatcac agatgagcga accggccgac ccgatcgaat gtgtctgtga   1320
gccgactgca gcttgcttgc tcattttgta tggatcgtcg tgcagcagga acgaaacact   1380
actcctttaa tttcctttct ttaatttcac aacgtttttt ctgggttttg ccgtgtatcg   1440
gccggaactg tactaccaag ttttctatag cctcgatggt catgcacgac atcgttgact   1500
gtttcccgcg cacttactgt tgaaataatc ttccattttt ggcaaaaaaa aaaaaaaaa    1559
```

<210> 24

<211> 385
<212> PRT
<213> Hordeum vulgare

<400> 24

```
Met Ala Met Pro Phe Ala Ser Leu Ser Pro Ala Ala Asp His His Arg
1               5                   10                  15

Ser Ser Pro Ile Phe Pro Phe Cys Arg Ser Ser Pro Leu Tyr Ser Val
            20                  25                  30

Gly Glu Glu Ala Ala His Gln His Pro His Pro Gln Gln Gln Gln Gln
        35                  40                  45

Gln His Ala Met Ser Gly Ala Arg Trp Ala Ala Arg Pro Ala Pro Phe
    50                  55                  60

Thr Ala Ala Gln Tyr Glu Glu Leu Glu Gln Gln Ala Leu Ile Tyr Lys
65                  70                  75                  80

Tyr Leu Val Ala Gly Val Pro Val Pro Gln Asp Leu Leu Leu Pro Ile
                85                  90                  95

Arg Arg Gly Phe Glu Thr Leu Ala Ser Arg Phe Tyr His His His Ala
            100                 105                 110

Leu Gly Tyr Gly Ser Tyr Phe Gly Lys Lys Leu Asp Pro Glu Pro Gly
        115                 120                 125

Arg Cys Arg Arg Thr Asp Gly Lys Lys Trp Arg Cys Ser Lys Glu Ala
    130                 135                 140

Ala Gln Asp Ser Lys Tyr Cys Glu Arg His Met His Arg Gly Arg Asn
145                 150                 155                 160

Arg Ser Arg Lys Pro Val Glu Thr Gln Leu Val Ala Ser Ser His Ser
                165                 170                 175

Gln Ser Gln Gln His Ala Thr Ala Ala Phe His Asn His Ser Pro Tyr
            180                 185                 190

Pro Ala Ile Ala Thr Gly Gly Gly Ser Phe Ala Leu Gly Ser Ala Gln
        195                 200                 205

Leu His Met Asp Thr Ala Ala Pro Tyr Ala Thr Thr Ala Gly Ala Ala
    210                 215                 220

Gly Asn Lys Asp Phe Arg Tyr Ser Ala Tyr Gly Val Arg Thr Ser Ala
225                 230                 235                 240

Ile Glu Glu His Asn Gln Phe Ile Thr Ala Ala Met Asp Thr Ala Met
                245                 250                 255
```

Asp Asn Tyr Ser Trp Arg Leu Met Pro Ser Gln Ala Ser Ala Phe Ser
260 265 270

Leu Ser Ser Tyr Pro Met Leu Gly Thr Leu Ser Asp Leu Asp Gln Ser
275 280 285

Ala Ile Cys Ser Leu Ala Lys Thr Glu Arg Glu Pro Leu Ser Phe Phe
290 295 300

Gly Gly Gly Gly Asp Phe Asp Asp Asp Ser Ala Ala Val Lys Gln Glu
305 310 315 320

Asn Gln Thr Leu Arg Pro Phe Phe Asp Glu Trp Pro Lys Asp Arg Asp
325 330 335

Ser Trp Pro Glu Leu Gln Asp His Asp Ala Asn Asn Asn Ser Asn Ala
340 345 350

Phe Ser Ala Thr Lys Leu Ser Ile Ser Met Pro Val Thr Ser Ser Asp
355 360 365

Phe Ser Gly Thr Thr Ala Gly Ser Arg Ser Pro Asn Gly Ile Tyr Ser
370 375 380

Arg
385

<210> 25
<211> 1521
<212> DNA
<213> Lycopersicon esculentum

<400> 25

```
gatgataaga aacacacaaa tgacttaact ttgcaggttt caccgcactc gacactgcaa      60
aaaagataca tataaaaaaa aaggtccact caactctctg caaaaataaa aaaaattaaa     120
aacttttgtc caagacttaa ctttctcttc agaaataaat ttgccttcac attaatattt     180
tgttgttagt aacaaaaatc attctcaatc gaaacatgga cttcaatatg aagcaatgga     240
gtaatcaaca tgagtcagaa aatcaagaat caccaacaaa gttaccaaga cttcttcttg     300
acttccactc tgtttcttct gattctgctt ctgctgctgc tctaccattg tttgtatctg     360
aaccaacaac atcaacaaca acttgtacca aattaatgtc agattcagca accactgtca     420
ccaccaaatt tccaaggatt ggaagtggtg gtggttactt cagcttggct caatggcaag     480
aacttgaact acacagtttg atttttaggc attttgtagc tggtgcccct gttccttctg     540
aactacttca tcttgttaag aaaagtatta ttgcttctcc tcctcctcct ccttcatatt     600
actttgctca tccatatcaa cagtatcctc attatcaaca agctttgatg cagtcagggt     660
actggggtag agccgccatg gatccagaac caggaaggtg taggaggact gatggcaaga     720
aatggaggtg ctcaagggat gtagtggctg gccagaaata ctgcgagcgc cacgttcatc     780
gtggccgcag ccgttcaaga aagcctgtgg aaattcccac acctgccaac aatggcagta     840

aaaacaacaa cactgtttct catcatcaag cctttggaaa aatgactgga catgctcatg     900
ctggtggtgg tgctcctcag ttttctcttt cgggacattc accttccact aatgcgcctt     960
ttcatctcaa tcaaaggcca attaagggtc caccacaaga agtacttcaa aaagatgtat    1020
ctattggtga tggtaaatca tctagtggcc aaatcctacg ccatttcttc gacgattggc    1080
ctagacaaca acttcaagaa ggcgacaatg ctgcaaccag cctgtccatt tcgatgcccg    1140
gtgtaggggg taaccccctcg tcagacttct cgttgaagct ttcaactggg aattactatg    1200
attcaggtac tcaagttagt aatgttgaac ggtctacatg gggacgagt caccaccacg      1260
tagcctcaat gggtggtcca cttgccgagg ccttaaggtc atcaacaact aactcgtccc    1320
ctactagcgt gttgcatcaa ttggcacgag gtagcgcgtc cgaggccagc tatattagca    1380
cttgatttct gcaagtgttc ttgttaaatg ttttttcttt ttggacttta ttgtttttta    1440
acttggttgt gttgttgttc attgttcttt attggtattg atatacctaa ctgtcacctg    1500
tacaaaaaaa aaaaaaaaaa a                                              1521
```

<210> 26
<211> 389
<212> PRT
<213> Lycopersicon esculentum

<400> 26

```
Met Asp Phe Asn Met Lys Gln Trp Ser Asn Gln His Glu Ser Glu Asn
1               5                   10                  15

Gln Glu Ser Pro Thr Lys Leu Pro Arg Leu Leu Leu Asp Phe His Ser
            20                  25                  30

Val Ser Ser Asp Ser Ala Ser Ala Ala Ala Leu Pro Leu Phe Val Ser
        35                  40                  45

Glu Pro Thr Thr Ser Thr Thr Thr Cys Thr Lys Leu Met Ser Asp Ser
    50                  55                  60

Ala Thr Thr Val Thr Thr Lys Phe Pro Arg Ile Gly Ser Gly Gly Gly
65                  70                  75                  80

Tyr Phe Ser Leu Ala Gln Trp Gln Glu Leu Glu Leu His Ser Leu Ile
                85                  90                  95

Phe Arg His Phe Val Ala Gly Ala Pro Val Pro Ser Glu Leu Leu His
            100                 105                 110

Leu Val Lys Lys Ser Ile Ile Ala Ser Pro Pro Pro Pro Pro Ser Tyr
        115                 120                 125

Tyr Phe Ala His Pro Tyr Gln Gln Tyr Pro His Tyr Gln Gln Ala Leu
    130                 135                 140
```

Met Gln Ser Gly Tyr Trp Gly Arg Ala Ala Met Asp Pro Glu Pro Gly
145 150 155 160

Arg Cys Arg Arg Thr Asp Gly Lys Lys Trp Arg Cys Ser Arg Asp Val
165 170 175

Val Ala Gly Gln Lys Tyr Cys Glu Arg His Val His Arg Gly Arg Ser
180 185 190

Arg Ser Arg Lys Pro Val Glu Ile Pro Thr Pro Ala Asn Asn Gly Ser
195 200 205

Lys Asn Asn Asn Thr Val Ser His His Gln Ala Phe Gly Lys Met Thr
210 215 220

Gly His Ala His Ala Gly Gly Gly Ala Pro Gln Phe Ser Leu Ser Gly
225 230 235 240

His Ser Pro Ser Thr Asn Ala Pro Phe His Leu Asn Gln Arg Pro Ile
245 250 255

Lys Gly Pro Pro Gln Glu Val Leu Gln Lys Asp Val Ser Ile Gly Asp
260 265 270

Gly Lys Ser Ser Ser Gly Gln Ile Leu Arg His Phe Phe Asp Asp Trp
275 280 285

Pro Arg Gln Gln Leu Gln Glu Gly Asp Asn Ala Ala Thr Ser Leu Ser
290 295 300

Ile Ser Met Pro Gly Val Gly Gly Asn Pro Ser Ser Asp Phe Ser Leu
305 310 315 320

Lys Leu Ser Thr Gly Asn Tyr Tyr Asp Ser Gly Thr Gln Val Ser Asn
325 330 335

Val Glu Arg Ser Thr Trp Gly Thr Ser His His His Val Ala Ser Met
340 345 350

Gly Gly Pro Leu Ala Glu Ala Leu Arg Ser Ser Thr Thr Asn Ser Ser
355 360 365

Pro Thr Ser Val Leu His Gln Leu Ala Arg Gly Ser Ala Ser Glu Ala
370 375 380

Ser Tyr Ile Ser Thr
385

<210> 27
<211> 1100
<212> DNA
<213> Medicago truncatula

<400> 27

```
atgatgagtg caagttcaag aaataggtca cttttcacac caaatcaatg gcaagaactt      60
gaacaacaag ccctagtttt taaatacatg gttactggaa cacctattcc accagatctc     120
atatactcta ttaagagaag tttagacact tcaatatctt caagaatctt tcctcatcca     180
ccaattgggt ggggatgttt tgaaatggga tttggcagaa aagtagaccc agagccaggg     240
aggtgcagaa gaacagatgg caagaaatgg agatgctcaa aggaagcata tccagactca     300
aagtactgtg aaagacacat gcacagaggt agaaaccgtt caagaaagcc tgtggaacta     360
gtagtttctt cttcaacaac aacaccaaca aataacacaa acacagcatc ttcttacagc     420
aacagaaaca tctccttgaa caacaacagc agcagcataa actcaccttc ttctttccct     480
ttctctactt catccatggc ttgtcatgat cagtcacaat ctttttcaca atcctaccaa     540
aactcttctt taaaccctta ctattactct caatcaatta cctctactaa cccacttgat     600
cattctcatt ttcaaactca agatgctact actcatcacc tctttttgga ctcaacatct     660
tattctcagg atgacaagga ctttaggtat gtacaagttc aaggaataag agatggtact     720
gtggatgaga gaactttctt tccagaagct acaggttcat ctaggagctg ttatcatgat     780
tcatatcaac aacaactatc aatgaatccc tttaagtctt actcaagctc acagtttcag     840
aatatcaatg atgataattc aagacaacaa caagaacaac actgttttgt tttaggcact     900
gacatcaagt caacaagaac aacaaacaag gacaaagaaa gtgagacaac tcagaaacca     960
cttcatcatt tctttggtga gtggacacca aagaacacag attcctggct agatcttgct    1020
tctaactcca gaattccaac aggttgatta tcatttatca tcattcctat gtttttgttt    1080
tttttttgtt attattaata                                               1100
```

<210> 28
<211> 348
<212> PRT
<213> Medicago truncatula

<400> 28

```
Met Met Ser Ala Ser Ser Arg Asn Arg Ser Leu Phe Thr Pro Asn Gln
1               5                   10                  15

Trp Gln Glu Leu Glu Gln Gln Ala Leu Val Phe Lys Tyr Met Val Thr
            20                  25                  30

Gly Thr Pro Ile Pro Pro Asp Leu Ile Tyr Ser Ile Lys Arg Ser Leu
        35                  40                  45

Asp Thr Ser Ile Ser Ser Arg Ile Phe Pro His Pro Pro Ile Gly Trp
    50                  55                  60

Gly Cys Phe Glu Met Gly Phe Gly Arg Lys Val Asp Pro Glu Pro Gly
65                  70                  75                  80

Arg Cys Arg Arg Thr Asp Gly Lys Lys Trp Arg Cys Ser Lys Glu Ala
                85                  90                  95
```

Tyr Pro Asp Ser Lys Tyr Cys Glu Arg His Met His Arg Gly Arg Asn
100 105 110

Arg Ser Arg Lys Pro Val Glu Leu Val Val Ser Ser Ser Thr Thr Thr
115 120 125

Pro Thr Asn Asn Thr Asn Thr Ala Ser Ser Tyr Ser Asn Arg Asn Ile
130 135 140

Ser Leu Asn Asn Asn Ser Ser Ser Ile Asn Ser Pro Ser Ser Phe Pro
145 150 155 160

Phe Ser Thr Ser Ser Met Ala Cys His Asp Gln Ser Gln Ser Phe Ser
165 170 175

Gln Ser Tyr Gln Asn Ser Ser Leu Asn Pro Tyr Tyr Tyr Ser Gln Ser
180 185 190

Ile Thr Ser Thr Asn Pro Leu Asp His Ser His Phe Gln Thr Gln Asp
195 200 205

Ala Thr Thr His His Leu Phe Leu Asp Ser Thr Ser Tyr Ser Gln Asp
210 215 220

Asp Lys Asp Phe Arg Tyr Val Gln Val Gln Gly Ile Arg Asp Gly Thr
225 230 235 240

Val Asp Glu Arg Thr Phe Phe Pro Glu Ala Thr Gly Ser Ser Arg Ser
245 250 255

Cys Tyr His Asp Ser Tyr Gln Gln Gln Leu Ser Met Asn Pro Phe Lys
260 265 270

Ser Tyr Ser Ser Ser Gln Phe Gln Asn Ile Asn Asp Asp Asn Ser Arg
275 280 285

Gln Gln Gln Glu Gln His Cys Phe Val Leu Gly Thr Asp Ile Lys Ser
290 295 300

Thr Arg Thr Thr Asn Lys Asp Lys Glu Ser Glu Thr Thr Gln Lys Pro
305 310 315 320

Leu His His Phe Phe Gly Glu Trp Thr Pro Lys Asn Thr Asp Ser Trp
325 330 335

Leu Asp Leu Ala Ser Asn Ser Arg Ile Pro Thr Gly
340 345

<210> 29
<211> 1302
<212> DNA
<213> Medicago truncatula

<400> 29

```
atgcatatgt tgacaatgga agctaaacct cttcaacttg ttccctcttc acacaacagc      60
acaactggtg gtggacccca gatgaagatt gagaatggtg aagttgatga agagaaaagg     120
gttgttgttg gagtgaagga agatatagaa aacaagcctt tgatcacaga agctcaaagg     180
cgtgaacttg atcatcaagt tttatttt aatcattttg cttataatct tcctcttcct      240
tattaccttt tgcaatttcc aagtaatatg tcagagtaca gtcgtcgtgg gtctgattat     300
gtgactatgg tggatcaaga accacatagg tgtagaagaa ctgacggaaa gaaatggagg     360
tgcggcaagg acacagtacc taatcagaag tattgtgaac gtcacatgca cagaggtcga     420
aatcgttcaa gaaagcttgt ggaaacatct caacttaact ctcctttgaa aacaaatcct     480
agtggtggtg gcaagtcaca tgcaaaacta gtcccaaaca ttaaatcttc agtttcaaat     540
ccaaaccctt tgattattca tcacaatggc acattctcat acaatccgag gaccttctgc     600
gttgtagata cttcttctgt ttgtgatcgg tcgagacatg tcatagatta tggtgccact     660
gcagtgacaa cttcgggaag cacgacatcc gtttctttgg ataacagagt ttgtcctaac     720
gtatgcaagc aagatgagca gatcaagagg tgtatcaccg acaacgtggg tattaaaagt     780
ggtcggaaag gaagcatatc ttgtgaaagt attggcatct ctactggaat aggcttttcc     840
ccaaagagtg ttcttccagt ttctggttgc aatgattcat acctcaacaa cagaaacaat     900
atattagaac ctgaacccgg tagatgccga agaacagatg gtaagaagtg gcgatgcaag     960
agtgcggttc ttccaggtca gaagtattgt gcaacacata tgcatagagg tgctaaaagg    1020
cgttttacaa acctcgaatc tcctcctcct gccaccactg ttattcctaa aactactgat    1080
attagttcag ctgttaccat tgctcagttg cccgaccctt cggctccaat cgacatccag    1140
aaagcgaatt gttggtctcc gagcactaag ctttcaatgt cggttcaaga aagtgcgccc    1200
tttgttgatt gtaatgagaa aagtgttagc agcggtgaca cggatggtac tagtaccacc    1260
atcactgaca ccatgaatga gtgtagctat ctttctttct aa                       1302
```

<210> 30
<211> 433
<212> PRT
<213> Medicago truncatula

<400> 30

```
Met His Met Leu Thr Met Glu Ala Lys Pro Leu Gln Leu Val Pro Ser
1               5                   10                  15

Ser His Asn Ser Thr Thr Gly Gly Gly Pro Gln Met Lys Ile Glu Asn
            20                  25                  30

Gly Glu Val Asp Glu Glu Lys Arg Val Val Val Gly Val Lys Glu Asp
        35                  40                  45

Ile Glu Asn Lys Pro Leu Ile Thr Glu Ala Gln Arg Arg Glu Leu Asp
    50                  55                  60
```

```
His Gln Val Phe Ile Phe Asn His Phe Ala Tyr Asn Leu Pro Leu Pro
65                  70                  75                  80

Tyr Tyr Leu Leu Gln Phe Pro Ser Asn Met Ser Glu Tyr Ser Arg Arg
                85                  90                  95

Gly Ser Asp Tyr Val Thr Met Val Asp Gln Glu Pro His Arg Cys Arg
            100                 105                 110

Arg Thr Asp Gly Lys Lys Trp Arg Cys Gly Lys Asp Thr Val Pro Asn
        115                 120                 125

Gln Lys Tyr Cys Glu Arg His Met His Arg Gly Arg Asn Arg Ser Arg
    130                 135                 140

Lys Leu Val Glu Thr Ser Gln Leu Asn Ser Pro Leu Lys Thr Asn Pro
145                 150                 155                 160

Ser Gly Gly Gly Lys Ser His Ala Lys Leu Val Pro Asn Ile Lys Ser
                165                 170                 175

Ser Val Ser Asn Pro Asn Pro Leu Ile Ile His His Asn Gly Thr Phe
            180                 185                 190

Ser Tyr Asn Pro Arg Thr Phe Cys Val Val Asp Thr Ser Ser Val Cys
        195                 200                 205

Asp Arg Ser Arg His Val Ile Asp Tyr Gly Ala Thr Ala Val Thr Thr
    210                 215                 220

Ser Gly Ser Thr Thr Ser Val Ser Leu Asp Asn Arg Val Cys Pro Asn
225                 230                 235                 240

Val Cys Lys Gln Asp Glu Gln Ile Lys Arg Cys Ile Thr Asp Asn Val
                245                 250                 255

Gly Ile Lys Ser Gly Arg Lys Gly Ser Ile Ser Cys Glu Ser Ile Gly
            260                 265                 270

Ile Ser Thr Gly Ile Gly Phe Ser Pro Lys Ser Val Leu Pro Val Ser
        275                 280                 285

Gly Cys Asn Asp Ser Tyr Leu Asn Asn Arg Asn Asn Ile Leu Glu Pro
    290                 295                 300

Glu Pro Gly Arg Cys Arg Arg Thr Asp Gly Lys Lys Trp Arg Cys Lys
305                 310                 315                 320

Ser Ala Val Leu Pro Gly Gln Lys Tyr Cys Ala Thr His Met His Arg
                325                 330                 335
```

```
Gly Ala Lys Arg Arg Phe Thr Asn Leu Glu Ser Pro Pro Pro Ala Thr
            340                 345                 350

Thr Val Ile Pro Lys Thr Thr Asp Ile Ser Ser Ala Val Thr Ile Ala
        355                 360                 365

Gln Leu Pro Asp Pro Ser Ala Pro Ile Asp Ile Gln Lys Ala Asn Cys
    370                 375                 380

Trp Ser Pro Ser Thr Lys Leu Ser Met Ser Val Gln Glu Ser Ala Pro
385                 390                 395                 400

Phe Val Asp Cys Asn Glu Lys Ser Val Ser Ser Gly Asp Thr Asp Gly
                405                 410                 415

Thr Ser Thr Thr Ile Thr Asp Thr Met Asn Glu Cys Ser Tyr Leu Ser
            420                 425                 430

Phe
```

<210> 31
<211> 1185
<212> DNA
<213> Oryza sativa

<400> 31

```
atggcgatgc cgtatgcctc cctgtctccg gcggtggccg accaccgctc gtccccggca      60
gccgcgaccg cctccctcct ccccttctgc cgctccaccc cgctctccgc gggcggtggt     120
ggcgtcgcga tgggggagga cgcgccgatg accgcgaggt ggccgccggc ggcggcggcg     180
aggctgccgc cgttcaccgc ggcgcagtac gaggagctgg agcagcaggc gctcatatac     240
aagtacctgg tggcaggcgt gcccgtcccg ccggatctcg tgctccccat ccgccgcgga     300
ctcgactccc tcgccgcccg cttctacaac catcccgccc ttggatatgg tccgtacttc     360
ggcaagaagc tggacccaga gccagggcgg tgccggcgta cggacggcaa gaaatggcgg     420
tgctcgaagg aggccgcgcc ggattccaag tactgcgagc gccacatgca ccgcggccgc     480
aaccgttcaa gaaagcctgt ggaaacgcag ctggtcgccc agtcccaacc gccctcatct     540
gttgtcggtt ctgcggcggc gccccttgct gctgcctcca atggcagcag cttccaaaac     600
cactctcttt accctgctat tgccggcagc aatggcgggg gcggggggag gaacatgccc     660
agctcatttg gctcggcgtt gggttctcag ctgcacatgg ataatgctgc cccttatgca     720
gctgttggtg gtggaacagg caaagatctc aggtatactg cttatggcac aagatctttg     780
gcggatgagc agagtcaact cattactgaa gctatcaaca catctattga aaatccatgg     840
cggctgctgc catctcagaa ctcgccattt cccctttcaa gctattctca gctgggggca     900
ctaagtgacc ttggtcagaa cacccccagc tcactttcaa aggttcagag gcagccactt     960
tcgttctttg ggaacgacta tgcggctgtc gattctgtga agcaagagaa ccagacgctg    1020
```

```
cgtcccttct ttgatgagtg gccaaaggga agggattcat ggtcagacct cgctgatgag   1080
aatgctaatc tttcgtcatt ctcaggcacc caactgtcga tctccatacc aatggcatcc   1140
tctgacttct cggcggccag ttctcgatca actaatggtg actga                   1185
```

<210> 32
<211> 394
<212> PRT
<213> oryza sativa

<400> 32

```
Met Ala Met Pro Tyr Ala Ser Leu Ser Pro Ala Val Ala Asp His Arg
1               5                   10                  15

Ser Ser Pro Ala Ala Ala Thr Ala Ser Leu Leu Pro Phe Cys Arg Ser
            20                  25                  30

Thr Pro Leu Ser Ala Gly Gly Gly Gly Val Ala Met Gly Glu Asp Ala
        35                  40                  45

Pro Met Thr Ala Arg Trp Pro Pro Ala Ala Ala Ala Arg Leu Pro Pro
    50                  55                  60

Phe Thr Ala Ala Gln Tyr Glu Glu Leu Glu Gln Gln Ala Leu Ile Tyr
65                  70                  75                  80

Lys Tyr Leu Val Ala Gly Val Pro Val Pro Pro Asp Leu Val Leu Pro
                85                  90                  95

Ile Arg Arg Gly Leu Asp Ser Leu Ala Ala Arg Phe Tyr Asn His Pro
            100                 105                 110

Ala Leu Gly Tyr Gly Pro Tyr Phe Gly Lys Lys Leu Asp Pro Glu Pro
        115                 120                 125

Gly Arg Cys Arg Arg Thr Asp Gly Lys Lys Trp Arg Cys Ser Lys Glu
    130                 135                 140

Ala Ala Pro Asp Ser Lys Tyr Cys Glu Arg His Met His Arg Gly Arg
145                 150                 155                 160

Asn Arg Ser Arg Lys Pro Val Glu Thr Gln Leu Val Ala Gln Ser Gln
                165                 170                 175

Pro Pro Ser Ser Val Val Gly Ser Ala Ala Ala Pro Leu Ala Ala Ala
            180                 185                 190

Ser Asn Gly Ser Ser Phe Gln Asn His Ser Leu Tyr Pro Ala Ile Ala
        195                 200                 205

Gly Ser Asn Gly Gly Gly Gly Gly Arg Asn Met Pro Ser Ser Phe Gly
    210                 215                 220
```

Ser Ala Leu Gly Ser Gln Leu His Met Asp Asn Ala Ala Pro Tyr Ala
225 230 235 240

Ala Val Gly Gly Gly Thr Gly Lys Asp Leu Arg Tyr Thr Ala Tyr Gly
245 250 255

Thr Arg Ser Leu Ala Asp Glu Gln Ser Gln Leu Ile Thr Glu Ala Ile
260 265 270

Asn Thr Ser Ile Glu Asn Pro Trp Arg Leu Leu Pro Ser Gln Asn Ser
275 280 285

Pro Phe Pro Leu Ser Ser Tyr Ser Gln Leu Gly Ala Leu Ser Asp Leu
290 295 300

Gly Gln Asn Thr Pro Ser Ser Leu Ser Lys Val Gln Arg Gln Pro Leu
305 310 315 320

Ser Phe Phe Gly Asn Asp Tyr Ala Ala Val Asp Ser Val Lys Gln Glu
325 330 335

Asn Gln Thr Leu Arg Pro Phe Phe Asp Glu Trp Pro Lys Gly Arg Asp
340 345 350

Ser Trp Ser Asp Leu Ala Asp Glu Asn Ala Asn Leu Ser Ser Phe Ser
355 360 365

Gly Thr Gln Leu Ser Ile Ser Ile Pro Met Ala Ser Ser Asp Phe Ser
370 375 380

Ala Ala Ser Ser Arg Ser Thr Asn Gly Asp
385 390

<210> 33
<211> 1194
<212> DNA
<213> Oryza sativa

<400> 33

atgatgatga tgagcggtcg cccgagcggc ggcgccggcg gaggtcggta cccgttcacg 60
gcgtcgcagt ggcaggagct ggagcaccag gcgctcatct acaagtacat ggcgtccggg 120
actcccatcc cctccgacct catcctcccc ctccgccgca gcttcctcct cgactccgcc 180
ctcgccacct ccccttccct cgccttccct ccccaacctt cactggggtg gggttgcttt 240
ggcatggggt ttgggcggaa ggcggaggac ccggagccag ggcgatgccg gcgtacggac 300
ggcaagaagt ggcggtgctc caaggaggcg tacccggact ccaagtactg cgagaagcac 360
atgcaccgtg gcaagaaccg ttcaagaaag cctgtggaaa tgtccttggc cacgccgccg 420
ccgccgtcct cctccgccac ctccgccgcg tcgaacacct ccgccggcgt cgcccccacc 480
accaccacca cctcctcccc ggcgccctcc tacagccgcc cggcgccgca cgacgcggcg 540

```
ccgtaccagg cgctctacgg cgggccctac gccgcggcca ccgcgcgcac ccccgccgcc    600
gcggcgtacc acgcgcaggt gagcccgttc cacctccagc tcgacaccac ccacccgcac    660
ccgccgccgt cctactactc catggaccac aaggagtacg cgtacgggca cgccaccaag    720
gaggtgcacg gcgagcacgc cttcttctcc gatggcaccg agagggagca ccaccacgcc    780
gccgccgggc acggccagtg gcagttcaag cagctcggca tggagcccaa gcagagcacc    840
acgcctctct tcccgggcgc cggctacggc cacaccgcgg cgtcgccgta cgccattgat    900
ctttcaaaag aggacgacga tgagaaagag aggcggcaac agcagcagca gcagcagcag    960
cagcactgct tcctcctggg cgccgacctc cgtctggaga agccggcggg ccacgaccac   1020
gcggcggcgg cgcagaaacc tctccgccac ttcttcgacg agtggccgca tgagaagaac   1080
agcaagggct cctggatggg gctcgaaggc gagacgcagc tgtccatgtc catccccatg   1140
gccgccaacg acctcccgat caccaccacc tcccgctacc acaatgatga ttaa         1194
```

<210> 34
<211> 397
<212> PRT
<213> Oryza sativa

<400> 34

```
Met Met Met Met Ser Gly Arg Pro Ser Gly Gly Ala Gly Gly Gly Arg
1               5                   10                  15

Tyr Pro Phe Thr Ala Ser Gln Trp Gln Glu Leu Glu His Gln Ala Leu
            20                  25                  30

Ile Tyr Lys Tyr Met Ala Ser Gly Thr Pro Ile Pro Ser Asp Leu Ile
        35                  40                  45

Leu Pro Leu Arg Arg Ser Phe Leu Leu Asp Ser Ala Leu Ala Thr Ser
    50                  55                  60

Pro Ser Leu Ala Phe Pro Pro Gln Pro Ser Leu Gly Trp Gly Cys Phe
65                  70                  75                  80

Gly Met Gly Phe Gly Arg Lys Ala Glu Asp Pro Glu Pro Gly Arg Cys
                85                  90                  95

Arg Arg Thr Asp Gly Lys Lys Trp Arg Cys Ser Lys Glu Ala Tyr Pro
            100                 105                 110

Asp Ser Lys Tyr Cys Glu Lys His Met His Arg Gly Lys Asn Arg Ser
        115                 120                 125

Arg Lys Pro Val Glu Met Ser Leu Ala Thr Pro Pro Pro Pro Ser Ser
    130                 135                 140

Ser Ala Thr Ser Ala Ala Ser Asn Thr Ser Ala Gly Val Ala Pro Thr
145                 150                 155                 160
```

Thr Thr Thr Thr Ser Ser Pro Ala Pro Ser Tyr Ser Arg Pro Ala Pro
165 170 175

His Asp Ala Ala Pro Tyr Gln Ala Leu Tyr Gly Gly Pro Tyr Ala Ala
180 185 190

Ala Thr Ala Arg Thr Pro Ala Ala Ala Ala Tyr His Ala Gln Val Ser
195 200 205

Pro Phe His Leu Gln Leu Asp Thr Thr His Pro His Pro Pro Pro Ser
210 215 220

Tyr Tyr Ser Met Asp His Lys Glu Tyr Ala Tyr Gly His Ala Thr Lys
225 230 235 240

Glu Val His Gly Glu His Ala Phe Phe Ser Asp Gly Thr Glu Arg Glu
245 250 255

His His His Ala Ala Ala Gly His Gly Gln Trp Gln Phe Lys Gln Leu
260 265 270

Gly Met Glu Pro Lys Gln Ser Thr Thr Pro Leu Phe Pro Gly Ala Gly
275 280 285

Tyr Gly His Thr Ala Ala Ser Pro Tyr Ala Ile Asp Leu Ser Lys Glu
290 295 300

Asp Asp Asp Glu Lys Glu Arg Arg Gln Gln Gln Gln Gln Gln Gln Gln
305 310 315 320

Gln His Cys Phe Leu Leu Gly Ala Asp Leu Arg Leu Glu Lys Pro Ala
325 330 335

Gly His Asp His Ala Ala Ala Ala Gln Lys Pro Leu Arg His Phe Phe
340 345 350

Asp Glu Trp Pro His Glu Lys Asn Ser Lys Gly Ser Trp Met Gly Leu
355 360 365

Glu Gly Glu Thr Gln Leu Ser Met Ser Ile Pro Met Ala Ala Asn Asp
370 375 380

Leu Pro Ile Thr Thr Thr Ser Arg Tyr His Asn Asp Asp
385 390 395

<210> 35
<211> 1371
<212> DNA
<213> Oryza sativa

<400> 35

atgcagggtg caatggccag ggtgaggggt cccttcacgc cgtctcagtg gatcgagctg 60

gagcaccagg cgctgatata caagtacttg gctgcgaata gccctgtacc acacagcctc 120
ctcatcccca tcaggaggag cctcacatcg ccctactcac ctgcctactt tggctcaagc 180
acattgggat ggggatcttt ccagctgggc tactccggca gcgcggatcc ggagcccggc 240
cggtgccgcc ggacggacgg caagaaatgg cggtgctcga gggatgcggt cgccgaccag 300
aagtactgtg agcgacacat gaaccgggga cgccaccgtt caagaaagca tgtggaaggc 360
cagcctggcc atgccgcgaa agcgatgccc gcggcggtgg cagcagccgc tgcctctgct 420
acccagccta gtgctccggc cgcccacagt ggcggagctg ttgctggcct cgctatcaac 480
catcagcacc agcaaatgaa gaactacgct gccaacactg ccaatccttg ctctctgcaa 540
tatagcaggg atctggcaaa caagcataat gagagtgaac aagtgcaaga ctcagacagt 600
ctctcgatgc tgacttccat tagcacgaga aatacgggca gcctgtttcc gttctcaaaa 660
caacataatc cttttgaagt gtccaactca aggccagatt ttggcctagt atcacctgat 720
tcactgatga gttctcctca tagctccttg gagaacgtca atttgctcac ttcgcagagt 780
ctgaatgaac aacagagttc agtttccctt caacactttg tggactggcc aaggacacct 840
gcacaaggag ctctcgcatg gcctgatgct gaagacatgc aagctcagag aagccagctc 900
tcaatatctg ctccaatggc gtcttctgac ctgtcatcag cctcaacatc tcccatccat 960
gagaagctga tgttgtcacc acttaaactg agccgtgaat atagtcctat tggtctcggt 1020
tttgcagcaa atagagatga ggttaaccag ggagaagcaa actggatgcc tatgttccgt 1080
gattctttga tgggcggacc attgggagag gttttaacca agaataacaa catggaagca 1140
aggaattgcc tatcggagtc tctgaatctt ttaaatgatg gctgggattc aagctcaggg 1200
tttgattcat ccccagttgg tgttctgcag aagaccacct ttggatcagt atccagtagc 1260
accggaagca gtcctagact ggagaatcat agtgtttatg atggcaacag taacctgcgg 1320
gatgatctcg gttcagttgt tgtaaatcat ccgagcatcc gcctggtgtg a 1371

<210> 36
<211> 456
<212> PRT
<213> Oryza sativa

<400> 36

Met Gln Gly Ala Met Ala Arg Val Arg Gly Pro Phe Thr Pro Ser Gln
1               5                   10                  15

Trp Ile Glu Leu Glu His Gln Ala Leu Ile Tyr Lys Tyr Leu Ala Ala
            20                  25                  30

Asn Ser Pro Val Pro His Ser Leu Leu Ile Pro Ile Arg Arg Ser Leu
        35                  40                  45

Thr Ser Pro Tyr Ser Pro Ala Tyr Phe Gly Ser Ser Thr Leu Gly Trp
    50                  55                  60

Gly Ser Phe Gln Leu Gly Tyr Ser Gly Ser Ala Asp Pro Glu Pro Gly

65 70 75 80

Arg Cys Arg Arg Thr Asp Gly Lys Lys Trp Arg Cys Ser Arg Asp Ala
85 90 95

Val Ala Asp Gln Lys Tyr Cys Glu Arg His Met Asn Arg Gly Arg His
100 105 110

Arg Ser Arg Lys His Val Glu Gly Gln Pro Gly His Ala Ala Lys Ala
115 120 125

Met Pro Ala Ala Val Ala Ala Ala Ala Ala Ser Ala Thr Gln Pro Ser
130 135 140

Ala Pro Ala Ala His Ser Gly Gly Ala Val Ala Gly Leu Ala Ile Asn
145 150 155 160

His Gln His Gln Gln Met Lys Asn Tyr Ala Ala Asn Thr Ala Asn Pro
165 170 175

Cys Ser Leu Gln Tyr Ser Arg Asp Leu Ala Asn Lys His Asn Glu Ser
180 185 190

Glu Gln Val Gln Asp Ser Asp Ser Leu Ser Met Leu Thr Ser Ile Ser
195 200 205

Thr Arg Asn Thr Gly Ser Leu Phe Pro Phe Ser Lys Gln His Asn Pro
210 215 220

Phe Glu Val Ser Asn Ser Arg Pro Asp Phe Gly Leu Val Ser Pro Asp
225 230 235 240

Ser Leu Met Ser Ser Pro His Ser Ser Leu Glu Asn Val Asn Leu Leu
245 250 255

Thr Ser Gln Ser Leu Asn Glu Gln Gln Ser Ser Val Ser Leu Gln His
260 265 270

Phe Val Asp Trp Pro Arg Thr Pro Ala Gln Gly Ala Leu Ala Trp Pro
275 280 285

Asp Ala Glu Asp Met Gln Ala Gln Arg Ser Gln Leu Ser Ile Ser Ala
290 295 300

Pro Met Ala Ser Ser Asp Leu Ser Ser Ala Ser Thr Ser Pro Ile His
305 310 315 320

Glu Lys Leu Met Leu Ser Pro Leu Lys Leu Ser Arg Glu Tyr Ser Pro
325 330 335

Ile Gly Leu Gly Phe Ala Ala Asn Arg Asp Glu Val Asn Gln Gly Glu
340 345 350

```
Ala Asn Trp Met Pro Met Phe Arg Asp Ser Leu Met Gly Gly Pro Leu
        355                 360                 365

Gly Glu Val Leu Thr Lys Asn Asn Asn Met Glu Ala Arg Asn Cys Leu
    370                 375                 380

Ser Glu Ser Leu Asn Leu Leu Asn Asp Gly Trp Asp Ser Ser Ser Gly
385                 390                 395                 400

Phe Asp Ser Ser Pro Val Gly Val Leu Gln Lys Thr Thr Phe Gly Ser
                405                 410                 415

Val Ser Ser Ser Thr Gly Ser Ser Pro Arg Leu Glu Asn His Ser Val
            420                 425                 430

Tyr Asp Gly Asn Ser Asn Leu Arg Asp Asp Leu Gly Ser Val Val Val
        435                 440                 445

Asn His Pro Ser Ile Arg Leu Val
    450                 455
```

<210> 37
<211> 711
<212> DNA
<213> oryza sativa

<400> 37

```
atgttggccg agggaaggca agtctacttg ccgccgccgc cgccgtccaa gcttcctcgt      60
ctctccggca ccgatccaac cgacggcgtg gtgacgatgg cagcgccgtc gccgctggtt     120
cttgggctgg gtctcggtct gggcggcagc ggcagcgaca gcagtgggag cgacgcggaa     180
gcgtctgcgg ccaccgtgcg ggaggcgcgg ccgccgtcgg cgctgacgtt catgcagcgg     240
caggagctgg agcagcaggt gctcatctac cgctacttcg ccgccggcgc gcctgtgccg     300
gttcacctcg tgctgcccat atggaagagc atcgccgccg cctcctcgtt cggcccgcaa     360
agctttccct ccctgacggg cctggggagc ctgtgcttcg actacaggag cagcatggag     420
ccggagccgg ggcggtgccg gcgcacggac ggcaagaagt ggcggtgctc gcgcgacgtg     480
gtgccggggc acaagtattg cgagcggcac gtccaccgtg gccgcggccg ttcaagaaag     540
cctatggaag cctctgcagc agtcgctccc acatatctcc cggtccggcc ggcactccac     600
accgtcgcca ccctcgccac cagcgcgcca tcgctgtcgc acctcggttt ctcctccgcc     660
agcaaagtgc tcctcgccca caccaccacc ggcaccacgc gcgctacttg a              711
```

<210> 38
<211> 236
<212> PRT
<213> oryza sativa

<400> 38

Met Leu Ala Glu Gly Arg Gln Val Tyr Leu Pro Pro Pro Pro Pro Ser
1               5                   10                  15

Lys Leu Pro Arg Leu Ser Gly Thr Asp Pro Thr Asp Gly Val Val Thr
            20                  25                  30

Met Ala Ala Pro Ser Pro Leu Val Leu Gly Leu Gly Leu Gly Leu Gly
        35                  40                  45

Gly Ser Gly Ser Asp Ser Ser Gly Ser Asp Ala Glu Ala Ser Ala Ala
    50                  55                  60

Thr Val Arg Glu Ala Arg Pro Pro Ser Ala Leu Thr Phe Met Gln Arg
65                  70                  75                  80

Gln Glu Leu Glu Gln Gln Val Leu Ile Tyr Arg Tyr Phe Ala Ala Gly
                85                  90                  95

Ala Pro Val Pro Val His Leu Val Leu Pro Ile Trp Lys Ser Ile Ala
            100                 105                 110

Ala Ala Ser Ser Phe Gly Pro Gln Ser Phe Pro Ser Leu Thr Gly Leu
        115                 120                 125

Gly Ser Leu Cys Phe Asp Tyr Arg Ser Ser Met Glu Pro Glu Pro Gly
    130                 135                 140

Arg Cys Arg Arg Thr Asp Gly Lys Lys Trp Arg Cys Ser Arg Asp Val
145                 150                 155                 160

Val Pro Gly His Lys Tyr Cys Glu Arg His Val His Arg Gly Arg Gly
                165                 170                 175

Arg Ser Arg Lys Pro Met Glu Ala Ser Ala Ala Val Ala Pro Thr Tyr
            180                 185                 190

Leu Pro Val Arg Pro Ala Leu His Thr Val Ala Thr Leu Ala Thr Ser
        195                 200                 205

Ala Pro Ser Leu Ser His Leu Gly Phe Ser Ser Ala Ser Lys Val Leu
    210                 215                 220

Leu Ala His Thr Thr Thr Gly Thr Thr Arg Ala Thr
225                 230                 235

<210> 39
<211> 1164
<212> DNA
<213> Oryza sativa

<400> 39

```
atggcgatgc cctttgcctc cctgtcgccg gcagccgacc accggccctc cttcatcttc      60
cccttctgcc gctcctcccc tctctccgcg gtcggggagg aggcgcagca gcacatgatg     120
ggcgcgaggt gggcggcggc ggtggccagg ccgccgccct tcacggcggc gcagtacgag     180
gagctggagc agcaggcgct catatacaag tacctcgtcg ccggcgtgcc cgtcccggcg     240
gatctcctcc tccccatccg ccgtggcctc gactcactcg cctcgcgctt ctaccaccac     300
cctgtccttg gatacggttc ctacttcggc aagaagctgg acccggagcc cggacggtgc     360
cggcgtacgg acggcaagaa gtggcggtgc tccaaggagg ccgcgccgga ctccaagtac     420
tgtgagcgac acatgcaccg cggccgcaac cgttcaagaa agcctgtgga agcgcagctc     480
gtcgcccccc actcgcagcc ccccgccacg gcgccggccg ccgccgtcac ctccaccgcc     540
ttccagaacc actcgctgta cccggcgatt gctaatggcg gcggcgccaa cggaggcggt     600
ggtggtggtg gcggtggcgg cagcgcgcct ggctcgttcg ccttggggtc taatactcag     660
ctgcacatgg acaatgctgc gtcttactcg actgttgctg ctggtgccgg aaacaaagat     720
ttcaggtatt ctgcttatgg agtgagacca ttggcagatg agcacagccc actcatcact     780
ggagctatgg atacctctat tgacaattcg tggtgcttgc tgccttctca gacctccaca     840
ttttcagttt cgagctaccc tatgcttgga aatctgagtg agctggacca gaacaccatc     900
tgctcgctgc cgaaggtgga gagggagcca ttgtcattct tcgggagcga ctatgtgacc     960
gtcgactccg ggaagcagga gaaccagacg ctgcgcccct ttttcgacga gtggccaaag    1020
gcaagggact cctggcctga tctagctgat gacaacagcc ttgccacctt ctctgccact    1080
cagctctcga tctccattcc aatggcaacc tctgacttct cgaccaccag ctcacgatca    1140
cacaacggta tatactcccg atga                                           1164
```

<210> 40
<211> 387
<212> PRT
<213> oryza sativa

<400> 40

Met Ala Met Pro Phe Ala Ser Leu Ser Pro Ala Ala Asp His Arg Pro
1               5                   10                  15

Ser Phe Ile Phe Pro Phe Cys Arg Ser Ser Pro Leu Ser Ala Val Gly
            20                  25                  30

Glu Glu Ala Gln Gln His Met Met Gly Ala Arg Trp Ala Ala Ala Val
        35                  40                  45

Ala Arg Pro Pro Pro Phe Thr Ala Ala Gln Tyr Glu Glu Leu Glu Gln
    50                  55                  60

Gln Ala Leu Ile Tyr Lys Tyr Leu Val Ala Gly Val Pro Val Pro Ala
65                  70                  75                  80

Asp Leu Leu Leu Pro Ile Arg Arg Gly Leu Asp Ser Leu Ala Ser Arg
                85                  90                  95

Phe Tyr His His Pro Val Leu Gly Tyr Gly Ser Tyr Phe Gly Lys Lys

100 105 110

Leu Asp Pro Glu Pro Gly Arg Cys Arg Arg Thr Asp Gly Lys Lys Trp
115 120 125

Arg Cys Ser Lys Glu Ala Ala Pro Asp Ser Lys Tyr Cys Glu Arg His
130 135 140

Met His Arg Gly Arg Asn Arg Ser Arg Lys Pro Val Glu Ala Gln Leu
145 150 155 160

Val Ala Pro His Ser Gln Pro Pro Ala Thr Ala Pro Ala Ala Ala Val
165 170 175

Thr Ser Thr Ala Phe Gln Asn His Ser Leu Tyr Pro Ala Ile Ala Asn
180 185 190

Gly Gly Gly Ala Asn Gly Gly Gly Gly Gly Gly Gly Gly Gly Gly Ser
195 200 205

Ala Pro Gly Ser Phe Ala Leu Gly Ser Asn Thr Gln Leu His Met Asp
210 215 220

Asn Ala Ala Ser Tyr Ser Thr Val Ala Ala Gly Ala Gly Asn Lys Asp
225 230 235 240

Phe Arg Tyr Ser Ala Tyr Gly Val Arg Pro Leu Ala Asp Glu His Ser
245 250 255

Pro Leu Ile Thr Gly Ala Met Asp Thr Ser Ile Asp Asn Ser Trp Cys
260 265 270

Leu Leu Pro Ser Gln Thr Ser Thr Phe Ser Val Ser Ser Tyr Pro Met
275 280 285

Leu Gly Asn Leu Ser Glu Leu Asp Gln Asn Thr Ile Cys Ser Leu Pro
290 295 300

Lys Val Glu Arg Glu Pro Leu Ser Phe Phe Gly Ser Asp Tyr Val Thr
305 310 315 320

Val Asp Ser Gly Lys Gln Glu Asn Gln Thr Leu Arg Pro Phe Phe Asp
325 330 335

Glu Trp Pro Lys Ala Arg Asp Ser Trp Pro Asp Leu Ala Asp Asp Asn
340 345 350

Ser Leu Ala Thr Phe Ser Ala Thr Gln Leu Ser Ile Ser Ile Pro Met
355 360 365

Ala Thr Ser Asp Phe Ser Thr Thr Ser Ser Arg Ser His Asn Gly Ile
370 375 380

Tyr Ser Arg
385

<210> 41
<211> 1071
<212> DNA
<213> oryza sativa

<400> 41

```
atgctgagct cgtcgccctc ggcggcggcg ccggggatag gagggtacca gccgcagcgc      60
ggggcggcgg tcttcacggc ggcgcagtgg gcggagctgg agcagcaggc gctcatttac     120
aagtacctcg tcgccggtgt ccccgtcccg ggcgatctcc tcctcccaat ccgcccccac     180
tcctccgccg ccgccaccta ctccttcgcc aaccccgccg ccgcgccctt ctaccaccac     240
caccaccacc cctctctgag ctattatgcc tactatggca agaagcttga ccctgagccg     300
tggcgttgcc gccgcaccga cggcaagaag tggcggtgct ccaaggaggc gcaccccgac     360
tccaagtact gcgagcgcca catgcaccgt ggccgcaacc gttcaagaaa gcctgtggaa     420
tccaagaccg ctgcccctgc gccccagtcg cagccccagc tgtccaatgt cacgaccgcg     480
actcacgaca ccgatgcgcc tctcccgtca ctcactgtgg gtgctaaaac ccacggtctg     540
tcccttggtg gtgctggctc gtcgcagttc catgtcgacg caccatcgta cggcagcaag     600
tactctcttg gagctaaagc tgatgtgggt gaactgagct tcttctcagg agcatcagga     660
aacaccaggg gcttcaccat tgattctcca acagatagct catggcattc actgccttcc     720
agtgtacccc catacccgat gtcaaagcca agggactctg gcctcctacc aggtgcctac     780
tcctactccc accttgaacc ttcacaggaa cttggccagg tcaccatcgc ctcgctgtcc     840
caagagcagg agcgccgctc ttttggtggt ggagcggggg ggatgctagg aaatgtgaag     900
cacgagaacc agccgctgag gcctttcttc gatgagtggc ctgggaggcg agactcgtgg     960
tcggagatgg atgaggagag gtccaaccag acctccttct cgacaaccca gctctcgatc    1020
tccatcccga tgcccagatg tgggtcccct atcggtccgc gtctaccttg a             1071
```

<210> 42
<211> 356
<212> PRT
<213> Oryza sativa

<400> 42

Met Leu Ser Ser Ser Pro Ser Ala Ala Ala Pro Gly Ile Gly Gly Tyr
1               5                   10                  15

Gln Pro Gln Arg Gly Ala Ala Val Phe Thr Ala Ala Gln Trp Ala Glu
            20                  25                  30

Leu Glu Gln Gln Ala Leu Ile Tyr Lys Tyr Leu Val Ala Gly Val Pro
        35                  40                  45

Val Pro Gly Asp Leu Leu Leu Pro Ile Arg Pro His Ser Ser Ala Ala

50 55 60

Ala Thr Tyr Ser Phe Ala Asn Pro Ala Ala Ala Pro Phe Tyr His His
65 70 75 80

His His His Pro Ser Leu Ser Tyr Tyr Ala Tyr Tyr Gly Lys Lys Leu
85 90 95

Asp Pro Glu Pro Trp Arg Cys Arg Arg Thr Asp Gly Lys Lys Trp Arg
100 105 110

Cys Ser Lys Glu Ala His Pro Asp Ser Lys Tyr Cys Glu Arg His Met
115 120 125

His Arg Gly Arg Asn Arg Ser Arg Lys Pro Val Glu Ser Lys Thr Ala
130 135 140

Ala Pro Ala Pro Gln Ser Gln Pro Gln Leu Ser Asn Val Thr Thr Ala
145 150 155 160

Thr His Asp Thr Asp Ala Pro Leu Pro Ser Leu Thr Val Gly Ala Lys
165 170 175

Thr His Gly Leu Ser Leu Gly Gly Ala Gly Ser Ser Gln Phe His Val
180 185 190

Asp Ala Pro Ser Tyr Gly Ser Lys Tyr Ser Leu Gly Ala Lys Ala Asp
195 200 205

Val Gly Glu Leu Ser Phe Phe Ser Gly Ala Ser Gly Asn Thr Arg Gly
210 215 220

Phe Thr Ile Asp Ser Pro Thr Asp Ser Ser Trp His Ser Leu Pro Ser
225 230 235 240

Ser Val Pro Pro Tyr Pro Met Ser Lys Pro Arg Asp Ser Gly Leu Leu
245 250 255

Pro Gly Ala Tyr Ser Tyr Ser His Leu Glu Pro Ser Gln Glu Leu Gly
260 265 270

Gln Val Thr Ile Ala Ser Leu Ser Gln Glu Gln Glu Arg Arg Ser Phe
275 280 285

Gly Gly Gly Ala Gly Gly Met Leu Gly Asn Val Lys His Glu Asn Gln
290 295 300

Pro Leu Arg Pro Phe Phe Asp Glu Trp Pro Gly Arg Arg Asp Ser Trp
305 310 315 320

Ser Glu Met Asp Glu Glu Arg Ser Asn Gln Thr Ser Phe Ser Thr Thr
325 330 335

Gln Leu Ser Ile Ser Ile Pro Met Pro Arg Cys Gly Ser Pro Ile Gly
340 345 350

Pro Arg Leu Pro
355

<210> 43
<211> 1230
<212> DNA
<213> oryza sativa

<400> 43

```
atgctgagct cttgtggtgg ccatggccat ggaaatccaa gaagcttgca agaagaacac      60
catggcagat gtggtgagca gcaaggtgga ggaggaggag gagggcaaga gcaagagcaa     120
gatgggttct tggtgagaga ggcaagggca tccccaccat ctccatcttc ttcatcattt     180
cttggatcca caagctcttc ttgttctgga ggaggaggag gagggcagat gttgagcttc     240
tcctccccca atggaacagc agggttgggc ttgagctcag gaggaagcat gcagggggtc     300
ttggcaaggg tcagggggcc gttcacccca acacagtgga tggagctgga gcaccaggca     360
ctgatctaca agcacattgc tgcaaatgtt tctgtccctt ccagcttgct cctccccatc     420
aggagaagcc tccatccatg gggatgggga tcattccctc ctggctgtgc tgatgtagaa     480
cccagaagat gccgccgcac agacggcaag aagtggcggt gctccagaga tgctgttggg     540
gatcagaagt attgtgagcg acacataaac cgtggtcgcc atcgttcaag aaagcatgtg     600
gaaggccgaa aggcgacact caccattgca gaaccatcca cggttattgc tgctggtgta     660
tcatctcgcg gccacactgt ggctcggcag aagcaggtga aaggctcagc tgctactgtc     720
tctgatcctt tctcgagaca atccaacagg aaatttctgg agaaacagaa cgttgtcgac     780
caattgtctc ccatggattc atttgatttc tcatccacac aatcttctcc aaactatgac     840
aatgtagcat tgtcaccact gaagttgcac catgatcatg atgaatctta catcgggcat     900
ggagcaggca gttcatcaga aaaaggcagt atgatgtacg aaagtcggtt aacagtctct     960
aaggaaacac ttgatgatgg acctttaggt gaagttttca aaagaaagaa ttgccaatca    1020
gcttctacag aaatcttaac tgaaaaatgg actgagaacc ccaacttaca ttgcccatct    1080
ggaatcctac aaatggctac taagttcaat tcaatttcca gcggcaacac agtaaatagt    1140
ggtggcaccg cagtggagaa tcttatcact gataatggat atcttactgc aagaatgatg    1200
aatcctcata ttgtcccaac acttctctaa                                     1230
```

<210> 44
<211> 409
<212> PRT
<213> oryza sativa

<400> 44

Met Leu Ser Ser Cys Gly Gly His Gly His Gly Asn Pro Arg Ser Leu
1 5 10 15

Gln Glu Glu His His Gly Arg Cys Gly Glu Gln Gln Gly Gly Gly Gly
20 25 30

Gly Gly Gly Gln Glu Gln Glu Gln Asp Gly Phe Leu Val Arg Glu Ala
35 40 45

Arg Ala Ser Pro Pro Ser Pro Ser Ser Ser Ser Phe Leu Gly Ser Thr
50 55 60

Ser Ser Ser Cys Ser Gly Gly Gly Gly Gly Gly Gln Met Leu Ser Phe
65 70 75 80

Ser Ser Pro Asn Gly Thr Ala Gly Leu Gly Leu Ser Ser Gly Gly Ser
85 90 95

Met Gln Gly Val Leu Ala Arg Val Arg Gly Pro Phe Thr Pro Thr Gln
100 105 110

Trp Met Glu Leu Glu His Gln Ala Leu Ile Tyr Lys His Ile Ala Ala
115 120 125

Asn Val Ser Val Pro Ser Ser Leu Leu Leu Pro Ile Arg Arg Ser Leu
130 135 140

His Pro Trp Gly Trp Gly Ser Phe Pro Pro Gly Cys Ala Asp Val Glu
145 150 155 160

Pro Arg Arg Cys Arg Arg Thr Asp Gly Lys Lys Trp Arg Cys Ser Arg
165 170 175

Asp Ala Val Gly Asp Gln Lys Tyr Cys Glu Arg His Ile Asn Arg Gly
180 185 190

Arg His Arg Ser Arg Lys His Val Glu Gly Arg Lys Ala Thr Leu Thr
195 200 205

Ile Ala Glu Pro Ser Thr Val Ile Ala Ala Gly Val Ser Ser Arg Gly
210 215 220

His Thr Val Ala Arg Gln Lys Gln Val Lys Gly Ser Ala Ala Thr Val
225 230 235 240

Ser Asp Pro Phe Ser Arg Gln Ser Asn Arg Lys Phe Leu Glu Lys Gln
245 250 255

Asn Val Val Asp Gln Leu Ser Pro Met Asp Ser Phe Asp Phe Ser Ser
260 265 270

Thr Gln Ser Ser Pro Asn Tyr Asp Asn Val Ala Leu Ser Pro Leu Lys
275 280 285

Leu His His Asp His Asp Glu Ser Tyr Ile Gly His Gly Ala Gly Ser
290 295 300

Ser Ser Glu Lys Gly Ser Met Met Tyr Glu Ser Arg Leu Thr Val Ser
305 310 315 320

Lys Glu Thr Leu Asp Asp Gly Pro Leu Gly Glu Val Phe Lys Arg Lys
325 330 335

Asn Cys Gln Ser Ala Ser Thr Glu Ile Leu Thr Glu Lys Trp Thr Glu
340 345 350

Asn Pro Asn Leu His Cys Pro Ser Gly Ile Leu Gln Met Ala Thr Lys
355 360 365

Phe Asn Ser Ile Ser Ser Gly Asn Thr Val Asn Ser Gly Gly Thr Ala
370 375 380

Val Glu Asn Leu Ile Thr Asp Asn Gly Tyr Leu Thr Ala Arg Met Met
385 390 395 400

Asn Pro His Ile Val Pro Thr Leu Leu
405

<210> 45
<211> 1293
<212> DNA
<213> oryza sativa

<400> 45

atggcaatgg cgacccctac gaccaacggc agcttccttc ttggatcagg tggctatccc 60
ggtgcccaga ttctaagctt ctcctcctca ggtcacagcg gcaatgggtt ggattgtgga 120
agctcagatg tggcaagaat gcagggggtt ttagcaaggg ttagggggcc attcacacca 180
acacaatgga tggagctgga gcaccaggct ctgatctaca agcacattgt ggcgaatgcg 240
ccggtaccgg ccggcttgct cctccccatc aggagaagcc tccatccacc agtgttccca 300
cacttctcct ctggtggcat tcttggctcc agctccttgg gatgggggtc atttcagctg 360
ggctattctg ggagtgctga ctccgagccc gggagatgcc gtcgaaccga tggcaagaaa 420
tggcggtgct cgagagacgc agttgtcgac caaaagtact gcgagcggca cataaaccgg 480
ggtcgccacc gttcaagaaa gcatgtggaa ggccaatcta gccatgccgc aaaagcaacg 540
gttcccgcca tagcacaacc acccattggt gcatctaatg gcaaattgtc aggcagccat 600
ggtgtgtcaa atgagctcac gaaaaccttg gctactaaca ggatgatgtt ggataaagca 660
aatcttattg aacgctccca ggactacact aatcagcaac acaacatcct acagaacaac 720
acaaaaggtg ataattggtc tgaagagatg tcctcacaag cagactatgc agtaatccct 780
gctggctctc tcatgaacac accgcaatcg gcgaatttaa atccaattcc ccagcaacaa 840
cgctgtaagc agtcactctt tggcaaaggg atacagcatg atgacattca gctgtcgata 900
tccattcccg tggataactc cgacttaccc actaactaca acaaggctca aatggaccat 960

gtagtaggcg gttcatcgaa tggcggaaac aacacgcgag caagttggat accgggctcc 1020
tgggaagcgt ccataggtgg acctctgggt gagttcttca ccaacaccag cagcgcatca 1080
gacgacaaag gcaaaagccg ccacccgcca tctttgaacc tcttagctga tggacatact 1140
acaagtccac agctgcaatc gcccaccgga gtcctgcaga tgactagctt cagttcagtg 1200
cccagcagca ctgttagtag tcctgcaggc agcctctgca atggcttgct cacttcaggc 1260
ctggtgaatg cccagactgt ccaaacactg tga 1293

<210> 46
<211> 430
<212> PRT
<213> oryza sativa

<400> 46

Met Ala Met Ala Thr Pro Thr Thr Asn Gly Ser Phe Leu Leu Gly Ser
1               5                   10                  15

Gly Gly Tyr Pro Gly Ala Gln Ile Leu Ser Phe Ser Ser Ser Gly His
            20                  25                  30

Ser Gly Asn Gly Leu Asp Cys Gly Ser Ser Asp Val Ala Arg Met Gln
        35                  40                  45

Gly Val Leu Ala Arg Val Arg Gly Pro Phe Thr Pro Thr Gln Trp Met
    50                  55                  60

Glu Leu Glu His Gln Ala Leu Ile Tyr Lys His Ile Val Ala Asn Ala
65                  70                  75                  80

Pro Val Pro Ala Gly Leu Leu Leu Pro Ile Arg Arg Ser Leu His Pro
                85                  90                  95

Pro Val Phe Pro His Phe Ser Ser Gly Gly Ile Leu Gly Ser Ser Ser
            100                 105                 110

Leu Gly Trp Gly Ser Phe Gln Leu Gly Tyr Ser Gly Ser Ala Asp Ser
        115                 120                 125

Glu Pro Gly Arg Cys Arg Arg Thr Asp Gly Lys Lys Trp Arg Cys Ser
    130                 135                 140

Arg Asp Ala Val Val Asp Gln Lys Tyr Cys Glu Arg His Ile Asn Arg
145                 150                 155                 160

Gly Arg His Arg Ser Arg Lys His Val Glu Gly Gln Ser Ser His Ala
                165                 170                 175

Ala Lys Ala Thr Val Pro Ala Ile Ala Gln Pro Pro Ile Gly Ala Ser
            180                 185                 190

Asn Gly Lys Leu Ser Gly Ser His Gly Val Ser Asn Glu Leu Thr Lys
195 200 205

Thr Leu Ala Thr Asn Arg Met Met Leu Asp Lys Ala Asn Leu Ile Glu
210 215 220

Arg Ser Gln Asp Tyr Thr Asn Gln Gln His Asn Ile Leu Gln Asn Asn
225 230 235 240

Thr Lys Gly Asp Asn Trp Ser Glu Glu Met Ser Ser Gln Ala Asp Tyr
245 250 255

Ala Val Ile Pro Ala Gly Ser Leu Met Asn Thr Pro Gln Ser Ala Asn
260 265 270

Leu Asn Pro Ile Pro Gln Gln Gln Arg Cys Lys Gln Ser Leu Phe Gly
275 280 285

Lys Gly Ile Gln His Asp Asp Ile Gln Leu Ser Ile Ser Ile Pro Val
290 295 300

Asp Asn Ser Asp Leu Pro Thr Asn Tyr Asn Lys Ala Gln Met Asp His
305 310 315 320

Val Val Gly Gly Ser Ser Asn Gly Gly Asn Asn Thr Arg Ala Ser Trp
325 330 335

Ile Pro Gly Ser Trp Glu Ala Ser Ile Gly Gly Pro Leu Gly Glu Phe
340 345 350

Phe Thr Asn Thr Ser Ser Ala Ser Asp Asp Lys Gly Lys Ser Arg His
355 360 365

Pro Pro Ser Leu Asn Leu Leu Ala Asp Gly His Thr Thr Ser Pro Gln
370 375 380

Leu Gln Ser Pro Thr Gly Val Leu Gln Met Thr Ser Phe Ser Ser Val
385 390 395 400

Pro Ser Ser Thr Val Ser Ser Pro Ala Gly Ser Leu Cys Asn Gly Leu
405 410 415

Leu Thr Ser Gly Leu Val Asn Ala Gln Thr Val Gln Thr Leu
420 425 430

<210> 47
<211> 1281
<212> DNA
<213> Oryza sativa

<400> 47

atgtttgctg acttctctgc tgctgccatg gagcttggag aggtgttggg cttgcaagga 60
ctcacagtgc catccaccaa ggagggtgat ctgagcctca tcaagagagc tgctgctggt 120
agcttcaccc aggctgctgc tgcatcatac ccttccccct ttcttgatga acagaagatg 180
ctcagattcg ccaaggctgc tcacacattg ccatcaggtt tggattttgg gagggaaaat 240
gagcagaggt tcttgttgtc taggaccaag aggcctttca ctccctcaca gtggatggag 300
ctggagcacc aggctctcat ttacaagtat ctcaatgcaa aggcccctat accttccagc 360
ctgctcattt caatcagcaa aagcttcaga tcatcagcta acagaatgag ctggaggcct 420
ctctatcaag gcttcccaaa tgcagactct gacccagaac ctggaagatg ccgtcgaaca 480
gatggcaaga aatggcggtg ttcaaaggag gccatggccg accacaagta ttgtgagagg 540
cacatcaaca gaaaccgcca ccgttcaaga aagcctgtgg aaaaccaaag tagaaagact 600
gtgaaagaga caccgtgtgc tggctcattg ccatcttctg tcgggcaggg cagcttcaag 660
aaggcaaaag ttaatgaaat gaagccacgc agtatcagct attggacaga tagtttgaac 720
aggacaatgg cgaacaaaga gaaaggaaac aaagctgctg aagaaaacaa tggcccactg 780
ctaaatttaa cgaatcaaca gccaacattg tccctgttct ctcagttgaa gcaacagaac 840
aaaccggaga agttcaatac agcaggagac agtgaatcga tttcttcaaa taccatgttg 900
aagccttggg agagcagcaa ccagcagaac aacaaaagca ttcctttcac caagatgcat 960
gatcgtggat gccttcagtc agtccttcag aatttcagct tgcctaagga cgagaaaatg 1020
gagtttcaga aaagcaaaga ttccaatgtc atgacagttc catcaacttt ctattcctcg 1080
ccagaggacc cacgcgtcag ctgccatgca cctaatatgg cacaaatgca agaggatagc 1140
atctcaagtt cttgggagat gcctcaaggt ggacctctag gtgagatctt gacaaactcc 1200
aaaaatcctg acgattcaat catgaaacca gaagcaaggc catatggttg gttactgaac 1260
ctcgaggatc atgcaatgtg a 1281

<210> 48
<211> 426
<212> PRT
<213> Oryza sativa

<400> 48

Met Phe Ala Asp Phe Ser Ala Ala Ala Met Glu Leu Gly Glu Val Leu
1 5 10 15

Gly Leu Gln Gly Leu Thr Val Pro Ser Thr Lys Glu Gly Asp Leu Ser
20 25 30

Leu Ile Lys Arg Ala Ala Ala Gly Ser Phe Thr Gln Ala Ala Ala Ala
35 40 45

Ser Tyr Pro Ser Pro Phe Leu Asp Glu Gln Lys Met Leu Arg Phe Ala
50 55 60

Lys Ala Ala His Thr Leu Pro Ser Gly Leu Asp Phe Gly Arg Glu Asn
65 70 75 80

Glu Gln Arg Phe Leu Leu Ser Arg Thr Lys Arg Pro Phe Thr Pro Ser
85 90 95

Gln Trp Met Glu Leu Glu His Gln Ala Leu Ile Tyr Lys Tyr Leu Asn
100 105 110

Ala Lys Ala Pro Ile Pro Ser Ser Leu Leu Ile Ser Ile Ser Lys Ser
115 120 125

Phe Arg Ser Ser Ala Asn Arg Met Ser Trp Arg Pro Leu Tyr Gln Gly
130 135 140

Phe Pro Asn Ala Asp Ser Asp Pro Glu Pro Gly Arg Cys Arg Arg Thr
145 150 155 160

Asp Gly Lys Lys Trp Arg Cys Ser Lys Glu Ala Met Ala Asp His Lys
165 170 175

Tyr Cys Glu Arg His Ile Asn Arg Asn Arg His Arg Ser Arg Lys Pro
180 185 190

Val Glu Asn Gln Ser Arg Lys Thr Val Lys Glu Thr Pro Cys Ala Gly
195 200 205

Ser Leu Pro Ser Ser Val Gly Gln Gly Ser Phe Lys Lys Ala Lys Val
210 215 220

Asn Glu Met Lys Pro Arg Ser Ile Ser Tyr Trp Thr Asp Ser Leu Asn
225 230 235 240

Arg Thr Met Ala Asn Lys Glu Lys Gly Asn Lys Ala Ala Glu Glu Asn
245 250 255

Asn Gly Pro Leu Leu Asn Leu Thr Asn Gln Gln Pro Thr Leu Ser Leu
260 265 270

Phe Ser Gln Leu Lys Gln Gln Asn Lys Pro Glu Lys Phe Asn Thr Ala
275 280 285

Gly Asp Ser Glu Ser Ile Ser Ser Asn Thr Met Leu Lys Pro Trp Glu
290 295 300

Ser Ser Asn Gln Gln Asn Asn Lys Ser Ile Pro Phe Thr Lys Met His
305 310 315 320

Asp Arg Gly Cys Leu Gln Ser Val Leu Gln Asn Phe Ser Leu Pro Lys
325 330 335

Asp Glu Lys Met Glu Phe Gln Lys Ser Lys Asp Ser Asn Val Met Thr
340 345 350

Val Pro Ser Thr Phe Tyr Ser Ser Pro Glu Asp Pro Arg Val Ser Cys

355 360 365

His Ala Pro Asn Met Ala Gln Met Gln Glu Asp Ser Ile Ser Ser Ser
370 375 380

Trp Glu Met Pro Gln Gly Gly Pro Leu Gly Glu Ile Leu Thr Asn Ser
385 390 395 400

Lys Asn Pro Asp Asp Ser Ile Met Lys Pro Glu Ala Arg Pro Tyr Gly
405 410 415

Trp Leu Leu Asn Leu Glu Asp His Ala Met
420 425

<210> 49
<211> 894
<212> DNA
<213> oryza sativa

<400> 49

```
gacaactcac tgccccccat cttctttctt cattcctctt cccaccaaga accccaaacc      60
ttacctccat tgagttcgaa accgaggagc gaggagttac aagccgagtt gtcagaatgg     120
atgaggagaa ggaagccgac tcgccgcagc caccgtccaa gctgcctcgc ctctccggcg     180
ctgacccgaa tgccggagtg gtgaccatgg cagcaccccc gccgccggtg ggtcttgggc     240
tggggcttgg actcggcggc gacagccgcg gcgagcgtga cgtggaagcg tcggcggcgg     300
cggcgcacaa ggcgacggcg ctgacgttca tgcagcagca ggagctggag caccaggtgc     360
tcatctaccg ctacttcgcc gcgggcgcgc ccgtgccggt gcacctcgtg ctccccatct     420
ggaagagcgt cgcgtcctcc tccttcggcc cgcaccgctt cccttccctg gcagtgatgg     480
ggttggggaa cctgtgcttc gactaccgga gcagcatgga gccggaccca gggcggtgca     540
ggcgcacgga cggcaagaag tggcggtgct cgcgcgacgt ggtgccgggg cacaagtact     600
gcgagcggca cgtccaccgc ggacgcggcc gttcaagaaa gcctgtggaa gcctccgcgg     660
ccgccacccc ggcgaacaac ggcggcggcg gtggcatcgt cttctccccc accagcgtcc     720
tcctcgccca cggcaccgcg cgcgccacct gaccagtgac cagaccggcg cccgtttgtt     780
tgtttgtctc ggcgcatggg aaaacccaaa tccgcaggga ttatgtcatg tcctgtaact     840
ctttttttcc tcgcaacttt tgaagccaaa acaattctca ccgtgttcga tggc           894
```

<210> 50
<211> 211
<212> PRT
<213> oryza sativa

<400> 50

```
Met Asp Glu Glu Lys Glu Ala Asp Ser Pro Gln Pro Pro Ser Lys Leu
1               5                   10                  15

Pro Arg Leu Ser Gly Ala Asp Pro Asn Ala Gly Val Val Thr Met Ala
            20                  25                  30

Ala Pro Pro Pro Pro Val Gly Leu Gly Leu Gly Leu Gly Leu Gly Gly
        35                  40                  45

Asp Ser Arg Gly Glu Arg Asp Val Glu Ala Ser Ala Ala Ala Ala His
    50                  55                  60

Lys Ala Thr Ala Leu Thr Phe Met Gln Gln Gln Glu Leu Glu His Gln
65                  70                  75                  80

Val Leu Ile Tyr Arg Tyr Phe Ala Ala Gly Ala Pro Val Pro Val His
                85                  90                  95

Leu Val Leu Pro Ile Trp Lys Ser Val Ala Ser Ser Ser Phe Gly Pro
            100                 105                 110

His Arg Phe Pro Ser Leu Ala Val Met Gly Leu Gly Asn Leu Cys Phe
        115                 120                 125

Asp Tyr Arg Ser Ser Met Glu Pro Asp Pro Gly Arg Cys Arg Arg Thr
    130                 135                 140

Asp Gly Lys Lys Trp Arg Cys Ser Arg Asp Val Val Pro Gly His Lys
145                 150                 155                 160

Tyr Cys Glu Arg His Val His Arg Gly Arg Gly Arg Ser Arg Lys Pro
                165                 170                 175

Val Glu Ala Ser Ala Ala Ala Thr Pro Ala Asn Asn Gly Gly Gly Gly
            180                 185                 190

Gly Ile Val Phe Ser Pro Thr Ser Val Leu Leu Ala His Gly Thr Ala
        195                 200                 205

Arg Ala Thr
    210
```

<210> 51
<211> 1149
<212> DNA
<213> oryza sativa

<400> 51

atgccctttg cctccctgtc gccggcagcc gaccaccggc cctccttcat cttccccttc 60
tgccgctcct cccctctctc cgcggtcggg gaggaggcgc agcagcacat gatgggcgcg 120
aggtgggcgg cggcggtggc caggccgccg cccttcacgg cggcgcagta cgaggagctg 180
gagcagcagg cgctcatata caagtacctc gtcgccggcg tgcccgtccc ggcggatctc 240
ctcctcccca tccgccgtgg cctcgactca ctcgcctcgc gcttctacca ccaccctgtc 300
cttggatacg gttcctactt cggcaagaag ctggacccgg agcccggacg gtgccggcgt 360
acggacggca agaagtggcg gtgctccaag gaggccgcgc cggactccaa gtactgtgag 420

cgacacatgc accgcggccg caaccgttca agaaagcctg tggaagcgca gctcgtcgcc 480
ccccactcgc agcccccgc cacggcgccg gccgccgccg tcacctccac cgccttccag 540
aaccactcgc tgtacccggc gattgctaat ggcggcggcg ccaacggagg cggtggtggt 600
ggtggcggtg gcggcagcgc gcctggctcg ttcgccttgg ggtctaatac tcagctgcac 660
atggacaatg ctgcgtctta ctcgactgtt gctgctggtg ccggaaacaa agatttcagg 720
tattctgctt atggagtgag accattggca gatgagcaca gcccactcat cactggagct 780
atggatacct ctattgacaa ttcgtggtgc ttgctgcctt ctcagacctc cacattttca 840
gtttcgagct accctatgct tggaaatctg agtgagctgg accagaacac catctgctcg 900
ctgccgaagg tggagaggga gccattgtca ttcttcggga gcgactatgt gaccgtcgac 960
tccgggaagc aggagaacca gacgctgcgc cccttttcg acgagtggcc aaaggcaagg 1020
gactcctggc ctgatctagc tgatgacaac agccttgcca ccttctctgc cactcagctc 1080
tcgatctcca ttccaatggc aacctctgac ttctcgacca ccagctcacg atcacacaac 1140
gatgagtga 1149

<210> 52
<211> 382
<212> PRT
<213> oryza sativa

<400> 52

```
Met Pro Phe Ala Ser Leu Ser Pro Ala Ala Asp His Arg Pro Ser Phe
1               5                   10                  15

Ile Phe Pro Phe Cys Arg Ser Ser Pro Leu Ser Ala Val Gly Glu Glu
            20                  25                  30

Ala Gln Gln His Met Met Gly Ala Arg Trp Ala Ala Ala Val Ala Arg
        35                  40                  45

Pro Pro Pro Phe Thr Ala Ala Gln Tyr Glu Glu Leu Glu Gln Gln Ala
    50                  55                  60

Leu Ile Tyr Lys Tyr Leu Val Ala Gly Val Pro Val Pro Ala Asp Leu
65                  70                  75                  80

Leu Leu Pro Ile Arg Arg Gly Leu Asp Ser Leu Ala Ser Arg Phe Tyr
                85                  90                  95

His His Pro Val Leu Gly Tyr Gly Ser Tyr Phe Gly Lys Lys Leu Asp
            100                 105                 110

Pro Glu Pro Gly Arg Cys Arg Arg Thr Asp Gly Lys Lys Trp Arg Cys
        115                 120                 125

Ser Lys Glu Ala Ala Pro Asp Ser Lys Tyr Cys Glu Arg His Met His
    130                 135                 140
```

Arg Gly Arg Asn Arg Ser Arg Lys Pro Val Glu Ala Gln Leu Val Ala
145 150 155 160

Pro His Ser Gln Pro Pro Ala Thr Ala Pro Ala Ala Ala Val Thr Ser
165 170 175

Thr Ala Phe Gln Asn His Ser Leu Tyr Pro Ala Ile Ala Asn Gly Gly
180 185 190

Gly Ala Asn Gly Gly Gly Gly Gly Gly Gly Gly Gly Gly Ser Ala Pro
195 200 205

Gly Ser Phe Ala Leu Gly Ser Asn Thr Gln Leu His Met Asp Asn Ala
210 215 220

Ala Ser Tyr Ser Thr Val Ala Ala Gly Ala Gly Asn Lys Asp Phe Arg
225 230 235 240

Tyr Ser Ala Tyr Gly Val Arg Pro Leu Ala Asp Glu His Ser Pro Leu
245 250 255

Ile Thr Gly Ala Met Asp Thr Ser Ile Asp Asn Ser Trp Cys Leu Leu
260 265 270

Pro Ser Gln Thr Ser Thr Phe Ser Val Ser Ser Tyr Pro Met Leu Gly
275 280 285

Asn Leu Ser Glu Leu Asp Gln Asn Thr Ile Cys Ser Leu Pro Lys Val
290 295 300

Glu Arg Glu Pro Leu Ser Phe Phe Gly Ser Asp Tyr Val Thr Val Asp
305 310 315 320

Ser Gly Lys Gln Glu Asn Gln Thr Leu Arg Pro Phe Phe Asp Glu Trp
325 330 335

Pro Lys Ala Arg Asp Ser Trp Pro Asp Leu Ala Asp Asp Asn Ser Leu
340 345 350

Ala Thr Phe Ser Ala Thr Gln Leu Ser Ile Ser Ile Pro Met Ala Thr
355 360 365

Ser Asp Phe Ser Thr Thr Ser Ser Arg Ser His Asn Asp Glu
370 375 380

<210> 53
<211> 1416
<212> DNA
<213> oryza sativa

<400> 53

gagagctccg tatcaccggc ctctttccct tcccttcccc tccgatccaa tcccccttc 60

tcctcctcgc ggcgctcgct gagcatggcg gcggaggggg aggccaagaa ggacagcgcc 120
agcaaccctc ccgggggagg aggcggcgga ggtggagggg aggaggagga ggatagcagc 180
ctggctgtcg gggaggcggc ggtcggggtg ggcgaggctg gtggaggagg aggaggaggg 240
gagaaggcgg atcgagagga ggaggagggg aaggaggatg tggaggaggg cggcgtgtgt 300
aaggatctgg tgctcgtcga ggacgccgtc cccgtcgagg atccggagga agccgcagca 360
actgcagcac ttcaggaaga aatgaaagcg ctcgttgaat ccgtcccagt tggtgctggg 420
gcggcattca ccgcgatgca actacaggag cttgagcagc aatctcgtgt ctaccagtat 480
atggctgccc gtgtgcctgt gcctactcat ctcgtcttcc caatatggaa gagtgttact 540
ggtgcatctt ctgaaggcgc ccagaagtac ccgacattga tggggttggc aacactctgc 600
ttggactttg gaaagaaccc agaaccagaa cctgggaggt gccggcgaac tgatggaaag 660
aagtggcggt gctggagaaa tgcaattgca aatgagaaat attgcgaacg ccatatgcac 720
cgtggccgca agcgtcctgt acagcttgtt gtcgaggatg acgagcctga ttctacctca 780
gggtcgaaac cagcatctgg caaggccacc gaaggtggca agaagactga tgacaagagc 840
tcaagtagca agaagcttgc agtggcagca ccagctgctg tggagtctac atgattgatg 900
cagcatttag gagctgcata aagagcataa ctgtgctggc aattagagtt cgcttcttat 960
tgtaatcctg aaaagactgt agtctggtct agctataacc tcatcaagca agaaaagtgt 1020
ctgtggaaag aagccacaaa aactttcatt tagctgtcac tgaaattttc agtttaggtg 1080
tatagtttga tttagctttg ccgtgccctc tgccttcagg cagatgagcg gcattattgg 1140
ataaatcctc tctgactgac aatatcgcat tgtgactcaa gaagccgatg gaaggatctg 1200
cgagactaga tacgaagcta tttgttgtgt atcattttat atggcctgca caattgtgtg 1260
attttgtcag ttgcataaca tgtggaagat ccataatttt atgcactatg gagattcaat 1320
taccttcctg aatgtctgag cttcgacatg ttattggtta ttgtaactta aaagcaacct 1380
gagattcaat gtgaaagggt tttagattcc agcttc 1416

<210> 54
<211> 269
<212> PRT
<213> Oryza sativa

<400> 54

Met Ala Ala Glu Gly Glu Ala Lys Lys Asp Ser Ala Ser Asn Pro Pro
1               5                   10                  15

Gly Gly Gly Gly Gly Gly Gly Gly Gly Glu Glu Glu Glu Asp Ser Ser
            20                  25                  30

Leu Ala Val Gly Glu Ala Ala Val Gly Val Gly Glu Ala Gly Gly Gly
        35                  40                  45

Gly Gly Gly Gly Glu Lys Ala Asp Arg Glu Glu Glu Glu Gly Lys Glu
    50                  55                  60

Asp Val Glu Glu Gly Gly Val Cys Lys Asp Leu Val Leu Val Glu Asp
65 70 75 80

Ala Val Pro Val Glu Asp Pro Glu Glu Ala Ala Ala Thr Ala Ala Leu
85 90 95

Gln Glu Glu Met Lys Ala Leu Val Glu Ser Val Pro Val Gly Ala Gly
100 105 110

Ala Ala Phe Thr Ala Met Gln Leu Gln Glu Leu Glu Gln Gln Ser Arg
115 120 125

Val Tyr Gln Tyr Met Ala Ala Arg Val Pro Val Pro Thr His Leu Val
130 135 140

Phe Pro Ile Trp Lys Ser Val Thr Gly Ala Ser Ser Glu Gly Ala Gln
145 150 155 160

Lys Tyr Pro Thr Leu Met Gly Leu Ala Thr Leu Cys Leu Asp Phe Gly
165 170 175

Lys Asn Pro Glu Pro Glu Pro Gly Arg Cys Arg Arg Thr Asp Gly Lys
180 185 190

Lys Trp Arg Cys Trp Arg Asn Ala Ile Ala Asn Glu Lys Tyr Cys Glu
195 200 205

Arg His Met His Arg Gly Arg Lys Arg Pro Val Gln Leu Val Val Glu
210 215 220

Asp Asp Glu Pro Asp Ser Thr Ser Gly Ser Lys Pro Ala Ser Gly Lys
225 230 235 240

Ala Thr Glu Gly Gly Lys Lys Thr Asp Asp Lys Ser Ser Ser Ser Lys
245 250 255

Lys Leu Ala Val Ala Ala Pro Ala Ala Val Glu Ser Thr
260 265

<210> 55
<211> 1854
<212> DNA
<213> Populus tremuloides

<400> 55

tagtgaagct ccttctcatg tctcacctcc tgagaccaaa ccaaagattc ttggatctgt 60

gttaagtaag cgagaaagat cagcttcgtc tgctcaagat gattactgga ggacttcaaa 120

gatgccaaaa aatgatgatt tttctgtcac caaaacaatg tcgttgcacc aacccacttc 180

tttactgaga tctaattaca tgctttctga tgattctcgc caacaagagc acatgatgag 240

cttctcttct ccaagaccag aaacgactcc atttctaagc aaagatggtg agttagtgga 300

gagaagcaca caaaaccaca ctgccttaag ctttcgttac catcagaaca cagcttcttc 360
ttatattaga agtgcaggtt atgacaccgg aggcttgaat gcaggcatgc acgggcctct 420
tactggggtt agaggaccat ttactccatc tcagtggatg gagcttgaac atcaggcctt 480
gatctacaaa tacatcactg ctcgtgtgcc tgtgccttct aatttgatca ttcctctcaa 540
gaagtctgtc tacccttata gcttacctgg ctcctctact ggatccttcc ctcacaattc 600
attgggatgg agcgctttcc atcttggtta ccctggcaac aacactgatc cggagcctgg 660
aaggtgtcgt cggactgatg ggaagaaatg gcggtgctca agggatgctg tagctgacca 720
aaagtattgt gaaaggcaca taaacagagg ccgccatcgt tcaagaaagc ctgtggaagg 780
ccagactggc catgctgcca ctgggactgc cagttcaaag gtggtgccaa tgtcgaactc 840
gatgtcaaaa ttggcaataa ccagtggtgg tgcctccaac agcattgcga tgaccacgca 900
acaacagttc aaaattttgc agccggctgc tgccaacact tctgcagatg ttgatgtcaa 960
cagagcacaa gatgcacaga gcatttctat gatgtcttcc accatcaacc ggaaatctga 1020
tgagtcctct ttctttgttc ctaaacaaga tatcttaatg gagcagtgct ctcaaacaga 1080
gtttggattt gtctcctctg actctctcct caacccatcg cagaagagct cttacattaa 1140
ctctaagccc tacgagtctt ttctaaactt taatgacgaa gaaagccaag atcagcatcc 1200
ccttcgtcaa ttcattgatg agtggccgaa ggatcaatct aattgttctg tcattagctg 1260
gccagaagag ttgaaatctg actggaccca gctctccatg tcaatcccaa tggcctcatc 1320
agacttctca tcatcatcat cctcacccac acaagagaaa cttgccctct caccaatgag 1380
tttatcttgc gagtttgacc ctgtacaaat gggtttaagg gtgagcgttg accataatga 1440
atcaagccaa aagcaaacca actggatacc tatctcctgg gggacttcaa ttggtggccc 1500
tttaggagag gtcttgacca ccagcactag ccatgcggat tcctgcaaga gctcatcagc 1560
ccttagcctt ttgagagaag gttgtgatgg cagcccacag ttgggatctt ctccgacggg 1620
agtcttgcag aaatcaactt tctgttccct ttccaatagc agttctggga gcagcccaag 1680
agctgagagc aagaaaaaca atgacactgc tagtctgtat gaggatgtgg gtggttcgat 1740
aattgcaagt tcatcaccta ttccacccct gtaatcaagc gaactgtaag gatgaaacct 1800
gtcaaggaaa tgtgaagaag cttggagttt ctatttatct gataaattcc tgta 1854

<210> 56
<211> 607
<212> PRT
<213> Populus tremuloides

<400> 56

Met Asp Phe Gly Val Leu Gly Leu Glu Gly Leu Val Gly Pro Glu Thr
1 5 10 15

Ser Ser Glu Ala Pro Ser His Val Ser Pro Pro Glu Thr Lys Pro Lys
20 25 30

Ile Leu Gly Ser Val Leu Ser Lys Arg Glu Arg Ser Ala Ser Ser Ala
35 40 45

Gln Asp Asp Tyr Trp Arg Thr Ser Lys Met Pro Lys Asn Asp Asp Phe
50 55 60

Ser Val Thr Lys Thr Met Ser Leu His Gln Pro Thr Ser Leu Leu Arg
65 70 75 80

Ser Asn Tyr Met Leu Ser Asp Asp Ser Arg Gln Gln Glu His Met Met
85 90 95

Ser Phe Ser Ser Pro Arg Pro Glu Thr Thr Pro Phe Leu Ser Lys Asp
100 105 110

Gly Glu Leu Val Glu Arg Ser Thr Gln Asn His Thr Ala Leu Ser Phe
115 120 125

Arg Tyr His Gln Asn Thr Ala Ser Ser Tyr Ile Arg Ser Ala Gly Tyr
130 135 140

Asp Thr Gly Gly Leu Asn Ala Gly Met His Gly Pro Leu Thr Gly Val
145 150 155 160

Arg Gly Pro Phe Thr Pro Ser Gln Trp Met Glu Leu Glu His Gln Ala
165 170 175

Leu Ile Tyr Lys Tyr Ile Thr Ala Arg Val Pro Val Pro Ser Asn Leu
180 185 190

Ile Ile Pro Leu Lys Lys Ser Val Tyr Pro Tyr Ser Leu Pro Gly Ser
195 200 205

Ser Thr Gly Ser Phe Pro His Asn Ser Leu Gly Trp Ser Ala Phe His
210 215 220

Leu Gly Tyr Pro Gly Asn Asn Thr Asp Pro Glu Pro Gly Arg Cys Arg
225 230 235 240

Arg Thr Asp Gly Lys Lys Trp Arg Cys Ser Arg Asp Ala Val Ala Asp
245 250 255

Gln Lys Tyr Cys Glu Arg His Ile Asn Arg Gly Arg His Arg Ser Arg
260 265 270

Lys Pro Val Glu Gly Gln Thr Gly His Ala Ala Thr Gly Thr Ala Ser
275 280 285

Ser Lys Val Val Pro Met Ser Asn Ser Met Ser Lys Leu Ala Ile Thr
290 295 300

Ser Gly Gly Ala Ser Asn Ser Ile Ala Met Thr Thr Gln Gln Gln Phe

305 310 315 320

Lys Ile Leu Gln Pro Ala Ala Ala Asn Thr Ser Ala Asp Val Asp Val
325 330 335

Asn Arg Ala Gln Asp Ala Gln Ser Ile Ser Met Met Ser Ser Thr Ile
340 345 350

Asn Arg Lys Ser Asp Glu Ser Ser Phe Phe Val Pro Lys Gln Asp Ile
355 360 365

Leu Met Glu Gln Cys Ser Gln Thr Glu Phe Gly Phe Val Ser Ser Asp
370 375 380

Ser Leu Leu Asn Pro Ser Gln Lys Ser Ser Tyr Ile Asn Ser Lys Pro
385 390 395 400

Tyr Glu Ser Phe Leu Asn Phe Asn Asp Glu Glu Ser Gln Asp Gln His
405 410 415

Pro Leu Arg Gln Phe Ile Asp Glu Trp Pro Lys Asp Gln Ser Asn Cys
420 425 430

Ser Val Ile Ser Trp Pro Glu Glu Leu Lys Ser Asp Trp Thr Gln Leu
435 440 445

Ser Met Ser Ile Pro Met Ala Ser Ser Asp Phe Ser Ser Ser Ser Ser
450 455 460

Ser Pro Thr Gln Glu Lys Leu Ala Leu Ser Pro Met Ser Leu Ser Cys
465 470 475 480

Glu Phe Asp Pro Val Gln Met Gly Leu Arg Val Ser Val Asp His Asn
485 490 495

Glu Ser Ser Gln Lys Gln Thr Asn Trp Ile Pro Ile Ser Trp Gly Thr
500 505 510

Ser Ile Gly Gly Pro Leu Gly Glu Val Leu Thr Thr Ser Thr Ser His
515 520 525

Ala Asp Ser Cys Lys Ser Ser Ser Ala Leu Ser Leu Leu Arg Glu Gly
530 535 540

Cys Asp Gly Ser Pro Gln Leu Gly Ser Ser Pro Thr Gly Val Leu Gln
545 550 555 560

Lys Ser Thr Phe Cys Ser Leu Ser Asn Ser Ser Ser Gly Ser Ser Pro
565 570 575

Arg Ala Glu Ser Lys Lys Asn Asn Asp Thr Ala Ser Leu Tyr Glu Asp
580 585 590

Val Gly Gly Ser Ile Ile Ala Ser Ser Ser Pro Ile Pro Pro Leu
595 600 605

<210> 57
<211> 1848
<212> DNA
<213> Populus tremuloides

<400> 57

aagtaatagt ggtttcgctt ctcttgctag ttcagatcct gaagcaaagc agaagtacgg 60
atctgggttc ctgaagcaag agagatctgc cgcagccgat gacgattgga ggaactctaa 120
attggccaaa accgagtcaa tgctgcttga ccagagaaac acttttcttc tgaaatctag 180
caacaactct ctcttcactg atggacagca gcagcagcag atgctcagct tctcctgtcc 240
caaatcagct tcttcagggg agagaagctc cccaaatgcc atgttgccat actttcacct 300
cacatcttct gcttgtaata gaaatacagg ctacaactct ggaatcttca atgctgccag 360
catgcatggg gttttgactg agactagatg gccattcact caattacaat ggatggagct 420
tgaacatcag gccttgatct acaaatacat gactgcaaat gtgcctatac catctaatct 480
gctcatcccc attaggaaag ctcttgattc tgctgggttt tctagctttt ctggtggact 540
tttcaaaccc agtgcattgc aatggggtac tttccatatg ggtttctcca gcaacactga 600
tccggagcca ggacggtgtc gaagaacaga tgggaagaaa tggcggtgct caagagacgc 660
agttgctgat cagaagtatt gtgagcggca catgaacagg ggtcgccatc gttcaagaaa 720
gcctgtggaa ggacaatcag gccattccgc tgcggccacc accactttaa agccaatggc 780
caatggcact tcctcttttg catcagcatc agtggtgggg cttcgcagcg ctgtgtccga 840
cagccacact attgtgcata atcagcagca acctgccagt tcttctaatc tttctgccac 900
caatacgctc agcagggtgt tcctcgctac agagaatgta ggtgagagaa tgcaagatgc 960
atcgggctta tccatgctac catccagcat tgacctgaaa tccaaagaaa ctccattctt 1020
catatcaaaa caacagaact cttacggtga atccctgcaa aatgagtttg cacttgtcac 1080
ctccgactcc ctcctcaacc attcacagaa aagctcgtcc ttgatgagtt gcagaaattt 1140
tggttcgtct caggacctta ctgaccagga atctgtttca cagcactccc tccgccaatt 1200
tatggatgat tgtcctaaaa gtcattctga tcgctctgct gttgcttggc ctggacttga 1260
tctgcaatct gagagaaccc agctatcaat ttcaatcccc atggctcctg cagactttgt 1320
gtcatccact tcatcttcaa acaatgaaaa gatctctctc tccccgctga gattatcgcg 1380
tgaatttgat ccaataaaga tggggctggg agtgggagcc ggtagtgtcg ccaatgaacc 1440
aaaccaaagg caagcgaatt ggattcccat ttcttgggaa acttcaatgg gtggtccact 1500
tggggaggtt ttgcacaaca ccaataataa tgcaacagca gaatgcaaga atgaatcatc 1560
gctcaaccta atgacagaga gatgggacaa cagtcctcgg gtaggctcat ctcctaccgg 1620
ggtcttacaa aagtctgcct ttgcttctct ttcaaatagc agtgctggaa gcagcccaag 1680
agcagagaac aagaccattg aaggtggcaa tctctgcaat gaccttggat ctactatcgt 1740

gcattcttca tcattgcctg ccttgtaact ctctgacctg ccatttaaga agtcttcagc 1800

tgatgccaga ttatgaataa tttgtttttt aaagttctca atcagtct 1848

<210> 58
<211> 605
<212> PRT
<213> Populus tremuloides

<400> 58

```
Met Asp Phe Gly Val Gln Val Gly Leu Asp Gly Leu Val Gly Ser Asp
1               5                   10                  15

Thr Ser Asn Ser Gly Phe Ala Ser Leu Ala Ser Ser Asp Pro Glu Ala
            20                  25                  30

Lys Gln Lys Tyr Gly Ser Gly Phe Leu Lys Gln Glu Arg Ser Ala Ala
        35                  40                  45

Ala Asp Asp Asp Trp Arg Asn Ser Lys Leu Ala Lys Thr Glu Ser Met
    50                  55                  60

Leu Leu Asp Gln Arg Asn Thr Phe Leu Leu Lys Ser Ser Asn Asn Ser
65                  70                  75                  80

Leu Phe Thr Asp Gly Gln Gln Gln Gln Gln Met Leu Ser Phe Ser Cys
                85                  90                  95

Pro Lys Ser Ala Ser Ser Gly Glu Arg Ser Ser Pro Asn Ala Met Leu
            100                 105                 110

Pro Tyr Phe His Leu Thr Ser Ser Ala Cys Asn Arg Asn Thr Gly Tyr
        115                 120                 125

Asn Ser Gly Ile Phe Asn Ala Ala Ser Met His Gly Val Leu Thr Glu
    130                 135                 140

Thr Arg Trp Pro Phe Thr Gln Leu Gln Trp Met Glu Leu Glu His Gln
145                 150                 155                 160

Ala Leu Ile Tyr Lys Tyr Met Thr Ala Asn Val Pro Ile Pro Ser Asn
                165                 170                 175

Leu Leu Ile Pro Ile Arg Lys Ala Leu Asp Ser Ala Gly Phe Ser Ser
            180                 185                 190

Phe Ser Gly Gly Leu Phe Lys Pro Ser Ala Leu Gln Trp Gly Thr Phe
        195                 200                 205

His Met Gly Phe Ser Ser Asn Thr Asp Pro Glu Pro Gly Arg Cys Arg
    210                 215                 220
```

Arg Thr Asp Gly Lys Lys Trp Arg Cys Ser Arg Asp Ala Val Ala Asp
225 230 235 240

Gln Lys Tyr Cys Glu Arg His Met Asn Arg Gly Arg His Arg Ser Arg
245 250 255

Lys Pro Val Glu Gly Gln Ser Gly His Ser Ala Ala Ala Thr Thr Thr
260 265 270

Leu Lys Pro Met Ala Asn Gly Thr Ser Ser Phe Ala Ser Ala Ser Val
275 280 285

Val Gly Leu Arg Ser Ala Val Ser Asp Ser His Thr Ile Val His Asn
290 295 300

Gln Gln Gln Pro Ala Ser Ser Ser Asn Leu Ser Ala Thr Asn Thr Leu
305 310 315 320

Ser Arg Val Phe Leu Ala Thr Glu Asn Val Gly Glu Arg Met Gln Asp
325 330 335

Ala Ser Gly Leu Ser Met Leu Pro Ser Ser Ile Asp Leu Lys Ser Lys
340 345 350

Glu Thr Pro Phe Phe Ile Ser Lys Gln Gln Asn Ser Tyr Gly Glu Ser
355 360 365

Leu Gln Asn Glu Phe Ala Leu Val Thr Ser Asp Ser Leu Leu Asn His
370 375 380

Ser Gln Lys Ser Ser Ser Leu Met Ser Cys Arg Asn Phe Gly Ser Ser
385 390 395 400

Gln Asp Leu Thr Asp Gln Glu Ser Val Ser Gln His Ser Leu Arg Gln
405 410 415

Phe Met Asp Asp Cys Pro Lys Ser His Ser Asp Arg Ser Ala Val Ala
420 425 430

Trp Pro Gly Leu Asp Leu Gln Ser Glu Arg Thr Gln Leu Ser Ile Ser
435 440 445

Ile Pro Met Ala Pro Ala Asp Phe Val Ser Ser Thr Ser Ser Ser Asn
450 455 460

Asn Glu Lys Ile Ser Leu Ser Pro Leu Arg Leu Ser Arg Glu Phe Asp
465 470 475 480

Pro Ile Lys Met Gly Leu Gly Val Gly Ala Gly Ser Val Ala Asn Glu
485 490 495

Pro Asn Gln Arg Gln Ala Asn Trp Ile Pro Ile Ser Trp Glu Thr Ser

500 505 510

Met Gly Gly Pro Leu Gly Glu Val Leu His Asn Thr Asn Asn Asn Ala
515 520 525

Thr Ala Glu Cys Lys Asn Glu Ser Ser Leu Asn Leu Met Thr Glu Arg
530 535 540

Trp Asp Asn Ser Pro Arg Val Gly Ser Ser Pro Thr Gly Val Leu Gln
545 550 555 560

Lys Ser Ala Phe Ala Ser Leu Ser Asn Ser Ser Ala Gly Ser Ser Pro
565 570 575

Arg Ala Glu Asn Lys Thr Ile Glu Gly Gly Asn Leu Cys Asn Asp Leu
580 585 590

Gly Ser Thr Ile Val His Ser Ser Ser Leu Pro Ala Leu
595 600 605

<210> 59
<211> 1053
<212> DNA
<213> Populus tremuloides

<400> 59

atggcaagag cttgaacacc aagctctcat ttacaaatac atggtctctg gtgttcctgt 60
cccgccagaa ctcctctatt ctgtcaaaag aagcttggga tcttctttgg catcaagact 120
cttccctcac caacctattg ggtggggttg ttttcaggcg ggttttggca gaaaagcaga 180
cccagagcca ggaaggtgca gaagaacgga tggaaaaaaa tggaggtgct caaaggaagc 240
atacccagac tcaaaatatt gtgagaggca catgcacaga ggcagaagcc gttcaagaaa 300
gcctgtggaa cttacttcaa gtactactac aacagcaaca acaattcctt taacatcaat 360
caacagaaac ctctctaacc ccactatttc accctccagc tcctcttatt ctttctctca 420
cccttcatct gcggaatctg aagtttatgc ccatcaaaac ccttcgcatg gaaccttcct 480
taaccccttc ctttatcctc attcttcatc ttctggacct cctgattctg gtttttcacc 540
tctaaatagc acccctcaca acctgttttt ggagtctgga tcttctcctc aagttgacaa 600
agagcacagg tattatcatg gaatgaggga ggatgtggat gagagagctt tctttccaga 660
tggtttaggg agtgcaagag gtgttcaaga ttcatataac caattgacaa tgagttccta 720
caaaggttac tcactgtcac agtttcaaac ctttgctgat acttctaaag aagagcagca 780
acaaccaggg cagcactgct ttgttttggg cactgatatt atcaagtcat cagcaacaag 840
gtcaatcaag ttggagaaag aaactgaaac cctgaagcca ttgcaccatt tctttgatga 900
atgggaacca aaggacgcag actcttggct tgatcttgca tccagttcaa gacctcacac 960
ttctgatgat tgaagtctca ataatggatc tttgtaatat gacgaaaaca gtacttgttc 1020
gtgggtcatc aatgcctttc ttgcctcaaa tga 1053

<210> 60
<211> 340
<212> PRT
<213> Populus tremuloides

<400> 60

```
Met Leu Asn Thr Thr Ile Ser Arg Asn Arg Phe Pro Phe Thr Ala Thr
1               5                   10                  15

Gln Trp Gln Glu Leu Glu His Gln Ala Leu Ile Tyr Lys Tyr Met Val
            20                  25                  30

Ser Gly Val Pro Val Pro Pro Glu Leu Leu Tyr Ser Val Lys Arg Ser
        35                  40                  45

Leu Gly Ser Ser Leu Ala Ser Arg Leu Phe Pro His Gln Pro Ile Gly
    50                  55                  60

Trp Gly Cys Phe Gln Ala Gly Phe Gly Arg Lys Ala Asp Pro Glu Pro
65                  70                  75                  80

Gly Arg Cys Arg Arg Thr Asp Gly Lys Lys Trp Arg Cys Ser Lys Glu
                85                  90                  95

Ala Tyr Pro Asp Ser Lys Tyr Cys Glu Arg His Met His Arg Gly Arg
            100                 105                 110

Ser Arg Ser Arg Lys Pro Val Glu Leu Thr Ser Ser Thr Thr Thr Thr
        115                 120                 125

Ala Thr Thr Ile Pro Leu Thr Ser Ile Asn Arg Asn Leu Ser Asn Pro
    130                 135                 140

Thr Ile Ser Pro Ser Ser Ser Ser Tyr Ser Phe Ser His Pro Ser Ser
145                 150                 155                 160

Ala Glu Ser Glu Val Tyr Ala His Gln Asn Pro Ser His Gly Thr Phe
                165                 170                 175

Leu Asn Pro Phe Leu Tyr Pro His Ser Ser Ser Ser Gly Pro Pro Asp
            180                 185                 190

Ser Gly Phe Ser Pro Leu Asn Ser Thr Pro His Asn Leu Phe Leu Glu
        195                 200                 205

Ser Gly Ser Ser Pro Gln Val Asp Lys Glu His Arg Tyr Tyr His Gly
    210                 215                 220

Met Arg Glu Asp Val Asp Glu Arg Ala Phe Phe Pro Asp Gly Leu Gly
225                 230                 235                 240

Ser Ala Arg Gly Val Gln Asp Ser Tyr Asn Gln Leu Thr Met Ser Ser
```

245 250 255

Tyr Lys Gly Tyr Ser Leu Ser Gln Phe Gln Thr Phe Ala Asp Thr Ser
260 265 270

Lys Glu Glu Gln Gln Gln Pro Gly Gln His Cys Phe Val Leu Gly Thr
275 280 285

Asp Ile Ile Lys Ser Ser Ala Thr Arg Ser Ile Lys Leu Glu Lys Glu
290 295 300

Thr Glu Thr Leu Lys Pro Leu His His Phe Phe Asp Glu Trp Glu Pro
305 310 315 320

Lys Asp Ala Asp Ser Trp Leu Asp Leu Ala Ser Ser Ser Arg Pro His
325 330 335

Thr Ser Asp Asp
340

<210> 61
<211> 1265
<212> DNA
<213> Populus tremuloides

<400> 61

tcttctttc tcttccaggg taatatgata atgagtggag gaaacaggtt tcccttcact 60
gcatcccagt ggcaagagct tgagcatcaa gccctaatct acaagtacat ggtttcaggc 120
atccccatcc ctcccgatct tcttttcacc atcaaaagaa gtggctgctt ggactcttca 180
ctctcttcaa agctctttcc ttgccaacct ccacattttt cctggggctg ttttcagatg 240
ggtttgggaa ggaaaataga tccagaaccg gggaggtgca ggagaactga tggaaagaaa 300
tggagatgct caaaagaagc atacccagat tctaagtact gtgagaaaca tatgcataga 360
gggaagaacc gttcaagaaa gcctgtggaa gttgcaacac aatcaataac agcaccaact 420
gtctcatcaa tgaccagaaa ccactctaat aattcactac taacaacatc ccccacctct 480
ctttcgttat tgtcacctaa gacccaccac cagaatcacc ttcactatcc tgctcctgca 540
ggttatcatg cccatccaaa tcatcaattc ttgtcttctt ccagacccct tgggattggt 600
ctgtcccctc atgaaaatcc tactcacttg cttttggact ctggtggttc ttctctggcc 660
aatacagatt acagaagaaa caggaatgtt tatgggctga aagaggaggt tgatgagcat 720
gctttcttct cagaaccttc aggttctatg agaagcttgt ccggttcatc tttggatgat 780
gcttggcaac tcaccccact cacaatgaac tcttctcctt ctaccaccaa ctcttcaaag 840
caaaggagct tgtctagttt acacaacgaa tattcttact tgcagcttca aagcctgagt 900
gatcccgata ccccaaaaca acaaaagcag tgtcaacata actatcttct gggaagtagt 960
gatgtagaca gtctagggcc cataaaaatg gagaaggaaa aatcccaaaa gactgttcac 1020
cgtttctttg atgaatggcc accaaaggat aaagattcat ggcttgattt ggatgacaaa 1080

tcatcaaaaa gtgcatcagt ttcagcaacc ggactctcaa tatccattcc ctcctctcat 1140

gactttcttc caatcttcag ttcaagaact aataatggtg gttgatttta ctctggtggg 1200

tttctggccc aagatgtact tggtgggaag ggggggtca ccgccttctg tcaagaggcc 1260

tcaga 1265

<210> 62
<211> 386
<212> PRT
<213> Populus tremuloides

<400> 62

```
Met Ile Met Ser Gly Gly Asn Arg Phe Pro Phe Thr Ala Ser Gln Trp
1               5                   10                  15

Gln Glu Leu Glu His Gln Ala Leu Ile Tyr Lys Tyr Met Val Ser Gly
            20                  25                  30

Ile Pro Ile Pro Pro Asp Leu Leu Phe Thr Ile Lys Arg Ser Gly Cys
        35                  40                  45

Leu Asp Ser Ser Leu Ser Ser Lys Leu Phe Pro Cys Gln Pro Pro His
    50                  55                  60

Phe Ser Trp Gly Cys Phe Gln Met Gly Leu Gly Arg Lys Ile Asp Pro
65                  70                  75                  80

Glu Pro Gly Arg Cys Arg Arg Thr Asp Gly Lys Lys Trp Arg Cys Ser
                85                  90                  95

Lys Glu Ala Tyr Pro Asp Ser Lys Tyr Cys Glu Lys His Met His Arg
            100                 105                 110

Gly Lys Asn Arg Ser Arg Lys Pro Val Glu Val Ala Thr Gln Ser Ile
        115                 120                 125

Thr Ala Pro Thr Val Ser Ser Met Thr Arg Asn His Ser Asn Asn Ser
    130                 135                 140

Leu Leu Thr Thr Ser Pro Thr Ser Leu Ser Leu Leu Ser Pro Lys Thr
145                 150                 155                 160

His His Gln Asn His Leu His Tyr Pro Ala Pro Ala Gly Tyr His Ala
                165                 170                 175

His Pro Asn His Gln Phe Leu Ser Ser Ser Arg Pro Leu Gly Ile Gly
            180                 185                 190

Leu Ser Pro His Glu Asn Pro Thr His Leu Leu Leu Asp Ser Gly Gly
        195                 200                 205

Ser Ser Leu Ala Asn Thr Asp Tyr Arg Arg Asn Arg Asn Val Tyr Gly
```

210 215 220

Leu Lys Glu Glu Val Asp Glu His Ala Phe Phe Ser Glu Pro Ser Gly
225 230 235 240

Ser Met Arg Ser Leu Ser Gly Ser Ser Leu Asp Asp Ala Trp Gln Leu
245 250 255

Thr Pro Leu Thr Met Asn Ser Ser Pro Ser Thr Thr Asn Ser Ser Lys
260 265 270

Gln Arg Ser Leu Ser Ser Leu His Asn Glu Tyr Ser Tyr Leu Gln Leu
275 280 285

Gln Ser Leu Ser Asp Pro Asp Thr Pro Lys Gln Gln Lys Gln Cys Gln
290 295 300

His Asn Tyr Leu Leu Gly Ser Ser Asp Val Asp Ser Leu Gly Pro Ile
305 310 315 320

Lys Met Glu Lys Glu Lys Ser Gln Lys Thr Val His Arg Phe Phe Asp
325 330 335

Glu Trp Pro Pro Lys Asp Lys Asp Ser Trp Leu Asp Leu Asp Asp Lys
340 345 350

Ser Ser Lys Ser Ala Ser Val Ser Ala Thr Gly Leu Ser Ile Ser Ile
355 360 365

Pro Ser Ser His Asp Phe Leu Pro Ile Phe Ser Ser Arg Thr Asn Asn
370 375 380

Gly Gly
385

<210> 63
<211> 644
<212> DNA
<213> Populus tremuloides

<400> 63

ctagggactg gactgctaaa cggttggaac atggagggaa ataggcgcaa tggtcggtct 60
cggtcacctt caattgggct aggagttgag cttggacgtg gtggttctag tcaaagacca 120
ataactggct gcaaaaaacc ttatgggttc actattcttc aactgcatga gctagaactt 180
cagtctctta tctacaagta tatccaagct ggatttcctg taccttacca tcttgtttta 240
cctatatgga aaagtgttac tgcttccctt ggtggtctca gttcaagctt gtaccagctc 300
taccctagct ttatggggtg taagtgtaac ccattatatt tggaatataa gaaaggaatg 360
gaacatgagc cagggagatg taggagaacg gatggaaaga agtggaggtg tagcaaagag 420
gttcttccag atcaaaagta ctgtgacagg cacatacaca gaggacgcca gcgttcaaga 480

aagcttgtgg aagctgcttc tcatagtaat gccagcacca acctctccat ttctctccct 540
ggaatcggta gtgctagcgc ctagcagtac taatctctct ccaatatgtt gtctctctcc 600
aaagagtgtt ctccacaaga aatatgtaat aggagcagct gcta 644

<210> 64
<211> 177
<212> PRT
<213> Populus tremuloides

<400> 64

Met Glu Gly Asn Arg Arg Asn Gly Arg Ser Arg Ser Pro Ser Ile Gly
1 5 10 15

Leu Gly Val Glu Leu Gly Arg Gly Gly Ser Ser Gln Arg Pro Ile Thr
20 25 30

Gly Cys Lys Lys Pro Tyr Gly Phe Thr Ile Leu Gln Leu His Glu Leu
35 40 45

Glu Leu Gln Ser Leu Ile Tyr Lys Tyr Ile Gln Ala Gly Phe Pro Val
50 55 60

Pro Tyr His Leu Val Leu Pro Ile Trp Lys Ser Val Thr Ala Ser Leu
65 70 75 80

Gly Gly Leu Ser Ser Ser Leu Tyr Gln Leu Tyr Pro Ser Phe Met Gly
85 90 95

Cys Lys Cys Asn Pro Leu Tyr Leu Glu Tyr Lys Lys Gly Met Glu His
100 105 110

Glu Pro Gly Arg Cys Arg Arg Thr Asp Gly Lys Lys Trp Arg Cys Ser
115 120 125

Lys Glu Val Leu Pro Asp Gln Lys Tyr Cys Asp Arg His Ile His Arg
130 135 140

Gly Arg Gln Arg Ser Arg Lys Leu Val Glu Ala Ala Ser His Ser Asn
145 150 155 160

Ala Ser Thr Asn Leu Ser Ile Ser Leu Pro Gly Ile Gly Ser Ala Ser
165 170 175

Ala

<210> 65
<211> 1154
<212> DNA
<213> Populus tremuloides

<400> 65

caagtgaagt tgaagagaga agtgaaagaa atgagcaact catcagtcac agtggcgggg 60

gtgggatcaa gatcaccacc aggtttcacg atgtctcagt ggcatgagct ggagcatcaa 120
gttcttatct ttaagtgttt aaatgcaggg ttacctgtcc ctccttccct tctccttcct 180
attcgtaaga gttttcagct tctttcccct ggtttcttgc acccatcaaa tttgagctac 240
tgttcctatt ttgggaagaa gattgactca gagccaggga ggtgtcggag gacagatggc 300
aagaaatgga ggtgctccaa agatgctcac ccagactcca agtactgtga gcggcatatg 360
aatagaagcc gtaaccgttc aagaaagcct gtggaatcac aaactacctc tcagtccttg 420
tcaactgtgg catcagaaat tgcaactggg agcagcagca ttgggagcag agggtatcca 480
actaatcctg ggaccttagg tttgggaagt aatatgtcac gttggcagat ggagtctatg 540
ccttatggtg ttaatagtaa agactacagg tctctccatg gaccgaagcc tgaagcagat 600
gagaaaactt tcctaccaga agctttggga aatacaagaa gctttggaat gaactctact 660
gtggacagca cttggcatct cacatcccaa gtccctgcaa accctgtgcc agaatcaaga 720
aatggttctc ttttgcaaaa ctacccacaa gtacagacac tgcaggattt tgagccccta 780
actgttgatg ctgcatcgcc aaaacaacag cagcagcagc attatttatt tggaagggag 840
ttcagttcat caggatctat gaggcgggaa aatcagtctc ttcagcctct ctttgacgag 900
tggccaaaat gcagggatat ggattcccat ctcactgatc aaagatctaa caataactcg 960
tctgctgttc agctatcaat ggccattcca atggctccta accctgctgc gaggagttat 1020
cattccccaa atggtgagac aggtttatct ggaacatact tttccgcaac ctagacgccc 1080
ccaagacttg gtggaaaaca aatagatgag ttctaatcct ctcatgttat aatgcagatg 1140
cttgaaagga gcat 1154

<210> 66
<211> 347
<212> PRT
<213> Populus tremuloides

<400> 66

Met Ser Asn Ser Ser Val Thr Val Ala Gly Val Gly Ser Arg Ser Pro
1 5 10 15

Pro Gly Phe Thr Met Ser Gln Trp His Glu Leu Glu His Gln Val Leu
20 25 30

Ile Phe Lys Cys Leu Asn Ala Gly Leu Pro Val Pro Pro Ser Leu Leu
35 40 45

Leu Pro Ile Arg Lys Ser Phe Gln Leu Leu Ser Pro Gly Phe Leu His
50 55 60

Pro Ser Asn Leu Ser Tyr Cys Ser Tyr Phe Gly Lys Lys Ile Asp Ser
65 70 75 80

Glu Pro Gly Arg Cys Arg Arg Thr Asp Gly Lys Lys Trp Arg Cys Ser
85 90 95

Lys Asp Ala His Pro Asp Ser Lys Tyr Cys Glu Arg His Met Asn Arg
100 105 110

Ser Arg Asn Arg Ser Arg Lys Pro Val Glu Ser Gln Thr Thr Ser Gln
115 120 125

Ser Leu Ser Thr Val Ala Ser Glu Ile Ala Thr Gly Ser Ser Ser Ile
130 135 140

Gly Ser Arg Gly Tyr Pro Thr Asn Pro Gly Thr Leu Gly Leu Gly Ser
145 150 155 160

Asn Met Ser Arg Trp Gln Met Glu Ser Met Pro Tyr Gly Val Asn Ser
165 170 175

Lys Asp Tyr Arg Ser Leu His Gly Pro Lys Pro Glu Ala Asp Glu Lys
180 185 190

Thr Phe Leu Pro Glu Ala Leu Gly Asn Thr Arg Ser Phe Gly Met Asn
195 200 205

Ser Thr Val Asp Ser Thr Trp His Leu Thr Ser Gln Val Pro Ala Asn
210 215 220

Pro Val Pro Glu Ser Arg Asn Gly Ser Leu Leu Gln Asn Tyr Pro Gln
225 230 235 240

Val Gln Thr Leu Gln Asp Phe Glu Pro Leu Thr Val Asp Ala Ala Ser
245 250 255

Pro Lys Gln Gln Gln Gln Gln His Tyr Leu Phe Gly Arg Glu Phe Ser
260 265 270

Ser Ser Gly Ser Met Arg Arg Glu Asn Gln Ser Leu Gln Pro Leu Phe
275 280 285

Asp Glu Trp Pro Lys Cys Arg Asp Met Asp Ser His Leu Thr Asp Gln
290 295 300

Arg Ser Asn Asn Asn Ser Ser Ala Val Gln Leu Ser Met Ala Ile Pro
305 310 315 320

Met Ala Pro Asn Pro Ala Ala Arg Ser Tyr His Ser Pro Asn Gly Glu
325 330 335

Thr Gly Leu Ser Gly Thr Tyr Phe Ser Ala Thr
340 345

<210> 67
<211> 1875
<212> DNA
<213> Populus tremuloides

<400> 67

```
tagtcatgct ccttctcatg tctcacttcc tgagaccaaa ccaaagattc ttggatctgt     60
gttaactaag caagaaagat catcttcatc tgcatcagct caggatgatt actggagggc    120
ttcaaagatg ccaaaacttg atgatttctc ttccaccaaa acaatgccac tgcaccaacc    180
cgctcctttg ctgagaccta attctatgtt ttctaatgat tctcgccaac aagagcacat    240
gctaagcttc tcttctccaa aaccagaagc tactccattt ctcgttaaag atgctggctt    300
ggttgagaga aacacacaaa accacactgc cttgagtttt ccttactacc agcacgcacc    360
tctttccgct agcagaagtg caggttatgg cactggaaac ttgaatgcaa gcatgcaggg    420
gccttttact ggggttagag gaccatttac tccatctcag tggatggagc ttgaacacca    480
ggccttgatc tacaaataca tcactgcacg tgtgcctgtg ccttccaatt taatcattcc    540
tctcaagaaa tctctcaacc cttatggctt accttttccc tctgctggat cattccctcc    600
cagttcattg ggatggggca ctttccacct tggttaccct ggcaacaaca ctgatcagga    660
gcctggaagg tgtcgtcgga ctgatggcaa gaaatggcgg tgctcaaggg atgctgtagc    720
tgaccaaaaa tattgtgaaa ggcacataaa cagaggccgc catcgttcaa gaaagcctgt    780
ggaaggccag actggccatg ctgctactgg gactgccagt tcaaaggtgg tgccaatgtc    840
taactccatg ccaacctcga ttacaaccag tggcgctacc tcgaacagca ttgtgatcac    900
acagcaacag ttaaaaaatt ttcagccggc tgctgcttcc atctcttctg cagatgctcg    960
tgtcaacgga gcacaagatg cacggagggt ttctatgatg tcttccacta tcaaccggaa   1020
atctgacgag tctactttct gtattcctag acaagatatc ctatttgaac agtgctctca   1080
aacagagttt ggacttgtct cctatgattc tctcctcaac ccatcgcaga agagctctta   1140
ctttaacgct aaaccctacg agtcttttct aaactttagt gatgaagaaa gccatgatca   1200
gcatcccctt cgtcaattca ttgatgactg gccgaaggac caatcaaatc gttctgtcat   1260
tagctggcca gaagagttga aatctgactg tacccagctc tcaatgtcaa tctcaatggt   1320
ctcgtcagac ttctcgtcgt catcatcctc acttctgcga gagaaacttg ccttctcacc   1380
attgaggtta tctcgcgagt ttgaccctat acaaatgggt ttaagggtga gcggtgacca   1440
taatgaatca agccagaagc aagccaactg gatacctatc tcttggggaa cttcaattgg   1500
cggcccttta ggagaggtct tgaccaccag cgccagccat gcggattcct gcaaaagctc   1560
atcagctctt aacctttttaa gagagggttg ggatggcagc ccgcagctgg gatcttctcc   1620
aacaggagtc ttgcagaaat cgacttttgg ttcactttca aatagcagtt caggtagcag   1680
cccaagagca gagagcaaga aaaacaatga aagtgctagt ctgtatgagg atgttgttgg   1740
ttcgataatt gcaagtgatc ccctattcca tccctgtaat caagaaaatg gttaggatga   1800
aacttgtgaa gaagaagctt ggagttattt atcttattaa tttctgcaga ctgtttctcc   1860
ttgttgcttg ttccc                                                    1875
```

<210> 68
<211> 555
<212> PRT
<213> Populus tremuloides

<400> 68

```
Met Pro Lys Leu Asp Asp Phe Ser Ser Thr Lys Thr Met Pro Leu His
1               5                   10                  15

Gln Pro Ala Pro Leu Leu Arg Pro Asn Ser Met Phe Ser Asn Asp Ser
            20                  25                  30

Arg Gln Gln Glu His Met Leu Ser Phe Ser Ser Pro Lys Pro Glu Ala
        35                  40                  45

Thr Pro Phe Leu Val Lys Asp Ala Gly Leu Val Glu Arg Asn Thr Gln
    50                  55                  60

Asn His Thr Ala Leu Ser Phe Pro Tyr Tyr Gln His Ala Pro Leu Ser
65                  70                  75                  80

Ala Ser Arg Ser Ala Gly Tyr Gly Thr Gly Asn Leu Asn Ala Ser Met
                85                  90                  95

Gln Gly Pro Phe Thr Gly Val Arg Gly Pro Phe Thr Pro Ser Gln Trp
            100                 105                 110

Met Glu Leu Glu His Gln Ala Leu Ile Tyr Lys Tyr Ile Thr Ala Arg
        115                 120                 125

Val Pro Val Pro Ser Asn Leu Ile Ile Pro Leu Lys Lys Ser Leu Asn
    130                 135                 140

Pro Tyr Gly Leu Pro Phe Ser Ser Ala Gly Ser Phe Pro Pro Ser Ser
145                 150                 155                 160

Leu Gly Trp Gly Thr Phe His Leu Gly Tyr Pro Gly Asn Asn Thr Asp
                165                 170                 175

Gln Glu Pro Gly Arg Cys Arg Arg Thr Asp Gly Lys Lys Trp Arg Cys
            180                 185                 190

Ser Arg Asp Ala Val Ala Asp Gln Lys Tyr Cys Glu Arg His Ile Asn
        195                 200                 205

Arg Gly Arg His Arg Ser Arg Lys Pro Val Glu Gly Gln Thr Gly His
    210                 215                 220

Ala Ala Thr Gly Thr Ala Ser Ser Lys Val Val Pro Met Ser Asn Ser
225                 230                 235                 240

Met Pro Thr Ser Ile Thr Thr Ser Gly Ala Thr Ser Asn Ser Ile Val
                245                 250                 255
```

Ile Thr Gln Gln Gln Leu Lys Asn Phe Gln Pro Ala Ala Ala Ser Ile
260 265 270

Ser Ser Ala Asp Ala Arg Val Asn Gly Ala Gln Asp Ala Arg Arg Val
275 280 285

Ser Met Met Ser Ser Thr Ile Asn Arg Lys Ser Asp Glu Ser Thr Phe
290 295 300

Cys Ile Pro Arg Gln Asp Ile Leu Phe Glu Gln Cys Ser Gln Thr Glu
305 310 315 320

Phe Gly Leu Val Ser Tyr Asp Ser Leu Leu Asn Pro Ser Gln Lys Ser
325 330 335

Ser Tyr Phe Asn Ala Lys Pro Tyr Glu Ser Phe Leu Asn Phe Ser Asp
340 345 350

Glu Glu Ser His Asp Gln His Pro Leu Arg Gln Phe Ile Asp Asp Trp
355 360 365

Pro Lys Asp Gln Ser Asn Arg Ser Val Ile Ser Trp Pro Glu Glu Leu
370 375 380

Lys Ser Asp Cys Thr Gln Leu Ser Met Ser Ile Ser Met Val Ser Ser
385 390 395 400

Asp Phe Ser Ser Ser Ser Ser Ser Leu Leu Arg Glu Lys Leu Ala Phe
405 410 415

Ser Pro Leu Arg Leu Ser Arg Glu Phe Asp Pro Ile Gln Met Gly Leu
420 425 430

Arg Val Ser Gly Asp His Asn Glu Ser Ser Gln Lys Gln Ala Asn Trp
435 440 445

Ile Pro Ile Ser Trp Gly Thr Ser Ile Gly Gly Pro Leu Gly Glu Val
450 455 460

Leu Thr Thr Ser Ala Ser His Ala Asp Ser Cys Lys Ser Ser Ser Ala
465 470 475 480

Leu Asn Leu Leu Arg Glu Gly Trp Asp Gly Ser Pro Gln Leu Gly Ser
485 490 495

Ser Pro Thr Gly Val Leu Gln Lys Ser Thr Phe Gly Ser Leu Ser Asn
500 505 510

Ser Ser Ser Gly Ser Ser Pro Arg Ala Glu Ser Lys Lys Asn Asn Glu
515 520 525

Ser Ala Ser Leu Tyr Glu Asp Val Val Gly Ser Ile Ile Ala Ser Asp
530 535 540

Pro Leu Phe His Pro Cys Asn Gln Glu Asn Gly
545 550 555

<210> 69
<211> 1715
<212> DNA
<213> Populus tremuloides

<400> 69

aaaaattatt cttctttatt tttcatcatg atgacaacag atgatggctt aaacgtttca 60
aacaaggtag ctaaggaaat aaacactact agtagtatta gtaatgttga ttttggtgtg 120
aagctacatc aacctattga tcatcatcaa tcatttcctt ctagtactcc tatgatggtt 180
cctcatgtta atcaccaccg tccaatgttt gacaatggtc ccacatcatc atgtgataga 240
aacaagtctt tgatgaacta tataagtgat cgtatatacc gtgttgctgc tggtggtgct 300
accagtggcg gtgcagttgg ggttaggaat ttgcagcctt ttgacatttc tgaaacaagt 360
atctctacag cagcttctgc cttcagatcc ccaggaggca acatggcagc gtctttgggg 420
tttcctttta caaatacaca gtggaaagag cttgaaagac aagccatgat atacaactat 480
ataacggcct cagtccctgt gcctcctcaa tttctaattc caaccccaat ggggaatgga 540
ttgaatgtaa ggttctcaaa tggggcagat ctagaaccag ggaggtgtag gagaacagat 600
gggaagaaat ggaggtgctc aagagatgtg gcacctgatc agaaatactg tgagcgtcat 660
atgcatagag gcagaccccg ttcaagaaag catgtggaac tcaatgctag caacaataac 720
aacaagaaga accgccataa tcctgctatt tgtccagaag ctcctgttac cgtggccatt 780
tctaaaccca caatcaacaa cagcaacagt ggctctgcct ctcacgatca gtttttgggg 840
cctatgcctc agccatatat ccagacccca gtttttgtaa acaaaaccag cgagaagact 900
tcaacttatg atgttaatgg agcctatggt tccacattca aagaacccag gagcttggac 960
tggatgttga aaggggaagc tggtcctata gccaaaaatg atcaacaatg gccacatcta 1020
gtgcacaaag aaattgaact agctactgaa ggttccttta acagtgcttc tgttctcaaa 1080
cagcattacc aaggagagtc tttgaatttg aactcatttg gaaattttaa tgctagagaa 1140
gaccaacaaa gcaatcaata tagtctgttt cttgatgagg ctccaaggag ttttattgat 1200
gcatggtcta atgatgcaat ttctagaaac acaagttctg tttcctcaga tgggaagctc 1260
catctttccc ctctcagtct atcaatggga agcaataggt ctactgatga tgaaatgggt 1320
cagatccaaa tgggtttagg cctaatcaaa tcagatcgaa atgaagaatg tggtaacact 1380
agcagcgccc caggtggccc cttggcagag gtgttacaac tgaggacaag caacaccaca 1440
ggaaccaatc aatcttcttc tatgatggaa aatggtgatt ctattagtcc tccagctact 1500
acagtctctt ctccatctgg ggttttgcag aaaacacttg cctcattttc tgatagcagt 1560
ggtaatagca gtccaactct tgccagttca aggaccaaac ctgaaattgc catgctttgg 1620
ttaaatcaag gctaaatgtg ccactctcta gttagttaag ggcacacaag gccataaggg 1680

caatataaat ttttataagc ttgatatatt tttat 1715

<210> 70
<211> 535
<212> PRT
<213> Populus tremuloides

<400> 70

Met Met Thr Thr Asp Asp Gly Leu Asn Val Ser Asn Lys Val Ala Lys
1 5 10 15

Glu Ile Asn Thr Thr Ser Ser Ile Ser Asn Val Asp Phe Gly Val Lys
20 25 30

Leu His Gln Pro Ile Asp His His Gln Ser Phe Pro Ser Ser Thr Pro
35 40 45

Met Met Val Pro His Val Asn His His Arg Pro Met Phe Asp Asn Gly
50 55 60

Pro Thr Ser Ser Cys Asp Arg Asn Lys Ser Leu Met Asn Tyr Ile Ser
65 70 75 80

Asp Arg Ile Tyr Arg Val Ala Ala Gly Gly Ala Thr Ser Gly Gly Ala
85 90 95

Val Gly Val Arg Asn Leu Gln Pro Phe Asp Ile Ser Glu Thr Ser Ile
100 105 110

Ser Thr Ala Ala Ser Ala Phe Arg Ser Pro Gly Gly Asn Met Ala Ala
115 120 125

Ser Leu Gly Phe Pro Phe Thr Asn Thr Gln Trp Lys Glu Leu Glu Arg
130 135 140

Gln Ala Met Ile Tyr Asn Tyr Ile Thr Ala Ser Val Pro Val Pro Pro
145 150 155 160

Gln Phe Leu Ile Pro Thr Pro Met Gly Asn Gly Leu Asn Val Arg Phe
165 170 175

Ser Asn Gly Ala Asp Leu Glu Pro Gly Arg Cys Arg Arg Thr Asp Gly
180 185 190

Lys Lys Trp Arg Cys Ser Arg Asp Val Ala Pro Asp Gln Lys Tyr Cys
195 200 205

Glu Arg His Met His Arg Gly Arg Pro Arg Ser Arg Lys His Val Glu
210 215 220

Leu Asn Ala Ser Asn Asn Asn Asn Lys Lys Asn Arg His Asn Pro Ala
225 230 235 240

Ile Cys Pro Glu Ala Pro Val Thr Val Ala Ile Ser Lys Pro Thr Ile
245 250 255

Asn Asn Ser Asn Ser Gly Ser Ala Ser His Asp Gln Phe Phe Gly Pro
260 265 270

Met Pro Gln Pro Tyr Ile Gln Thr Pro Val Phe Val Asn Lys Thr Ser
275 280 285

Glu Lys Thr Ser Thr Tyr Asp Val Asn Gly Ala Tyr Gly Ser Thr Phe
290 295 300

Lys Glu Pro Arg Ser Leu Asp Trp Met Leu Lys Gly Glu Ala Gly Pro
305 310 315 320

Ile Ala Lys Asn Asp Gln Gln Trp Pro His Leu Val His Lys Glu Ile
325 330 335

Glu Leu Ala Thr Glu Gly Ser Phe Asn Ser Ala Ser Val Leu Lys Gln
340 345 350

His Tyr Gln Gly Glu Ser Leu Asn Leu Asn Ser Phe Gly Asn Phe Asn
355 360 365

Ala Arg Glu Asp Gln Gln Ser Asn Gln Tyr Ser Leu Phe Leu Asp Glu
370 375 380

Ala Pro Arg Ser Phe Ile Asp Ala Trp Ser Asn Asp Ala Ile Ser Arg
385 390 395 400

Asn Thr Ser Ser Val Ser Ser Asp Gly Lys Leu His Leu Ser Pro Leu
405 410 415

Ser Leu Ser Met Gly Ser Asn Arg Ser Thr Asp Asp Glu Met Gly Gln
420 425 430

Ile Gln Met Gly Leu Gly Leu Ile Lys Ser Asp Arg Asn Glu Glu Cys
435 440 445

Gly Asn Thr Ser Ser Ala Pro Gly Gly Pro Leu Ala Glu Val Leu Gln
450 455 460

Leu Arg Thr Ser Asn Thr Thr Gly Thr Asn Gln Ser Ser Ser Met Met
465 470 475 480

Glu Asn Gly Asp Ser Ile Ser Pro Pro Ala Thr Thr Val Ser Ser Pro
485 490 495

Ser Gly Val Leu Gln Lys Thr Leu Ala Ser Phe Ser Asp Ser Ser Gly
500 505 510

Asn Ser Ser Pro Thr Leu Ala Ser Ser Arg Thr Lys Pro Glu Ile Ala
515 520 525

Met Leu Trp Leu Asn Gln Gly
530 535

<210> 71
<211> 1053
<212> DNA
<213> Populus tremuloides

<400> 71

agcagcgggg atggcagctg ggggaatggg gacagcagcg atgacaatga ggtcaccatt 60
tacagtgtca cagtggcaag aactggaaca tcaagctttg atctataagt acatggtggc 120
aggtctgcct gttccacctg atcttgtgct ccctattcag aggagctttg aatccatttc 180
tcatagattc ttccaccatc ccaccatgag ctattgcact ttctatggca agaaggtgga 240
tccggaacca ggtcgatgca ggaggaccga cggcaagaag tggaggtgct ccaaagatgc 300
ctacccagac tccaagtact gtgagcgcca catgcaccgt ggccgcaacc gttcaagaaa 360
gcctgtggaa tcacaaacca tgacacagtc atcgtccacc gtgacatcac tgactgttac 420
aggaagcagc agtggaactg gaagcttcca gaaccttcca ttgcacacat atagcaatcc 480
ccagggcact gcttctggaa ctaaccaatc atattatcat atgaactcca ttccctacgg 540
aatcccaacc aaagattaca ggtatcttca agaacttacg cctgaaggtg gggagcatag 600
cttcttgtct gaagcctcag gaagcaacaa ggggcttcag atagactcac agctggacaa 660
tgcatggtct ttgatgcaat ccagagtctc atcattcccc acagagaaat caactgaaaa 720
ctcgatgttg caaagtaatc atccccagca ttcatttttc agtagtgatt tcaccaccag 780
ggaatctgtg aaacaggacg ggcagtctct tcgacccttc tttgatgagt ggcctaaaaa 840
ccgagatgcc tggtctggcc tcgagaatga tagttccaac cagacctcat tctctacaac 900
gcagctgtcg atatccattc caatggcctc atctgacttc tccacaagtt gtcgttctcc 960
acgagataac taagaggaca ctaagaattc acaaaacaaa gccaaaggtg gtcctggcca 1020
aagattaaca catacttgtg ttaaattttt gtg 1053

<210> 72
<211> 320
<212> PRT
<213> Populus tremuloides

<400> 72

Met Ala Ala Gly Gly Met Gly Thr Ala Ala Met Thr Met Arg Ser Pro
1               5                   10                  15

Phe Thr Val Ser Gln Trp Gln Glu Leu Glu His Gln Ala Leu Ile Tyr
            20                  25                  30

Lys Tyr Met Val Ala Gly Leu Pro Val Pro Pro Asp Leu Val Leu Pro
        35                  40                  45

Ile Gln Arg Ser Phe Glu Ser Ile Ser His Arg Phe Phe His His Pro
50 55 60

Thr Met Ser Tyr Cys Thr Phe Tyr Gly Lys Lys Val Asp Pro Glu Pro
65 70 75 80

Gly Arg Cys Arg Arg Thr Asp Gly Lys Lys Trp Arg Cys Ser Lys Asp
85 90 95

Ala Tyr Pro Asp Ser Lys Tyr Cys Glu Arg His Met His Arg Gly Arg
100 105 110

Asn Arg Ser Arg Lys Pro Val Glu Ser Gln Thr Met Thr Gln Ser Ser
115 120 125

Ser Thr Val Thr Ser Leu Thr Val Thr Gly Ser Ser Ser Gly Thr Gly
130 135 140

Ser Phe Gln Asn Leu Pro Leu His Thr Tyr Ser Asn Pro Gln Gly Thr
145 150 155 160

Ala Ser Gly Thr Asn Gln Ser Tyr Tyr His Met Asn Ser Ile Pro Tyr
165 170 175

Gly Ile Pro Thr Lys Asp Tyr Arg Tyr Leu Gln Glu Leu Thr Pro Glu
180 185 190

Gly Gly Glu His Ser Phe Leu Ser Glu Ala Ser Gly Ser Asn Lys Gly
195 200 205

Leu Gln Ile Asp Ser Gln Leu Asp Asn Ala Trp Ser Leu Met Gln Ser
210 215 220

Arg Val Ser Ser Phe Pro Thr Glu Lys Ser Thr Glu Asn Ser Met Leu
225 230 235 240

Gln Ser Asn His Pro Gln His Ser Phe Phe Ser Ser Asp Phe Thr Thr
245 250 255

Arg Glu Ser Val Lys Gln Asp Gly Gln Ser Leu Arg Pro Phe Phe Asp
260 265 270

Glu Trp Pro Lys Asn Arg Asp Ala Trp Ser Gly Leu Glu Asn Asp Ser
275 280 285

Ser Asn Gln Thr Ser Phe Ser Thr Thr Gln Leu Ser Ile Ser Ile Pro
290 295 300

Met Ala Ser Ser Asp Phe Ser Thr Ser Cys Arg Ser Pro Arg Asp Asn
305 310 315 320

<210> 73
<211> 1682

<212> DNA
<213> Populus tremuloides

<400> 73

```
gtctgttctt gtttttgtag atacacgaca atggagaaaa gagtatctga agaatcagcg      60
ccgtcgatga aactgtctct tgggattggt gctggtgatc atggtgatga tgatcaagat     120
gatagacacg tgttcccaca gttaacagag actcagttgc atgagcttaa acagcaagct     180
ttgatattca agtacatagt agctggtctt cgcgtgcctc ctgatcttgt agttcccatt     240
tggcatagtg ttgctagcag ctctcttggt tcatttagtg gtgctgatat ttataggcaa     300
ttcccaagct ttgtgggatt aagtcctcag ggatttgatt atagacaaat gatggatcca     360
gaacctggga gatgtagaag aactgatggg aagaaatgga ggtgtagcaa ggatgtagtt     420
gctggtcaga agtattgtga acgccatatg catagaggcc gtcaacgttc aagaaagctt     480
gtggaagctt ctcaaactgc tgctgcttct gaaaaaccat cacctcacaa ttcaagcaag     540
aattcagaca atccaaccac tcattcttct aatttagcca aagtaagttc acagataaaa     600
gccccacctc ttaataatac ccccaccatt ttaaccactt gcaccacaag ttgcaattct     660
gacattgaaa tcactggcat gagcttggcc actactgcta attctgactg caaaaacccc     720
tttacaacca tgactactag cattgttacc ggctacaaga acactgcaac aatgatcgct     780
agtgctgttc atgctgacat tacagccact ggtaacgact acaagagtag catcaactta     840
aagagacact acattgatga cagaaacagt aattgcagca actctgttac ttacaagggt     900
ataatcgaca gaaactgcag caacaaaaaa ataaaaaatg ctggtagcaa tgtatctcaa     960
ggattgaact tctccccgaa gagtgttctt caagttcaag gttgcggcgc ctcacacatt    1020
tacatgaatg atgtggaact tgaactggga aggtgtagaa gaacagatgg aaagaagtgg    1080
cgatgccgca gggatgttgt agctaatcag aagtattgcg agatgcacat gcaccgaggt    1140
tctaagcagc acttggaagc gtccaaacct gctgcaattc ccgctacaat cccatttgtc    1200
cctgggaatg ttcattcata tcctgcaacg aacttgccaa gtaaagcaga tcgcagaagc    1260
ttaaacaccg atctctgtat ttcgatccca acaagtcctc aactgatcat gaccaatgat    1320
gatacaagaa ctatcagcaa tagcagtgac actaccataa gcgacaccat gaggggcacc    1380
acaccaggac tggtatcaac acatgcaggt agtggggatc ccaaaagtcc tcctggtggt    1440
gacagagcca ggttattaaa gaaagctgaa gttatggagg cggcggtaga gatttacgag    1500
gaagtacaat ccggccgtgg ggtcagaagt tttatccaag gcagaagact tccaactttt    1560
ccctatgttt atatcacacc tctgctgtta atctgcaatt aattctagtc aaaccatgtg    1620
attaaagtta caagaggtgt aagagaagaa taacgtttaa ttccatccta tgcagacaac    1680
tg                                                                  1682
```

<210> 74
<211> 523
<212> PRT
<213> Populus tremuloides

<400> 74

```
Met Glu Lys Arg Val Ser Glu Glu Ser Ala Pro Ser Met Lys Leu Ser
1               5                   10                  15

Leu Gly Ile Gly Ala Gly Asp His Gly Asp Asp Asp Gln Asp Asp Arg
            20                  25                  30

His Val Phe Pro Gln Leu Thr Glu Thr Gln Leu His Glu Leu Lys Gln
        35                  40                  45

Gln Ala Leu Ile Phe Lys Tyr Ile Val Ala Gly Leu Arg Val Pro Pro
    50                  55                  60

Asp Leu Val Val Pro Ile Trp His Ser Val Ala Ser Ser Ser Leu Gly
65                  70                  75                  80

Ser Phe Ser Gly Ala Asp Ile Tyr Arg Gln Phe Pro Ser Phe Val Gly
                85                  90                  95

Leu Ser Pro Gln Gly Phe Asp Tyr Arg Gln Met Met Asp Pro Glu Pro
            100                 105                 110

Gly Arg Cys Arg Arg Thr Asp Gly Lys Lys Trp Arg Cys Ser Lys Asp
        115                 120                 125

Val Val Ala Gly Gln Lys Tyr Cys Glu Arg His Met His Arg Gly Arg
    130                 135                 140

Gln Arg Ser Arg Lys Leu Val Glu Ala Ser Gln Thr Ala Ala Ala Ser
145                 150                 155                 160

Glu Lys Pro Ser Pro His Asn Ser Ser Lys Asn Ser Asp Asn Pro Thr
                165                 170                 175

Thr His Ser Ser Asn Leu Ala Lys Val Ser Ser Gln Ile Lys Ala Pro
            180                 185                 190

Pro Leu Asn Asn Thr Pro Thr Ile Leu Thr Thr Cys Thr Thr Ser Cys
        195                 200                 205

Asn Ser Asp Ile Glu Ile Thr Gly Met Ser Leu Ala Thr Thr Ala Asn
    210                 215                 220

Ser Asp Cys Lys Asn Pro Phe Thr Thr Met Thr Thr Ser Ile Val Thr
225                 230                 235                 240

Gly Tyr Lys Asn Thr Ala Thr Met Ile Ala Ser Ala Val His Ala Asp
                245                 250                 255

Ile Thr Ala Thr Gly Asn Asp Tyr Lys Ser Ser Ile Asn Leu Lys Arg
```

260 265 270

His Tyr Ile Asp Asp Arg Asn Ser Asn Cys Ser Asn Ser Val Thr Tyr
275 280 285

Lys Gly Ile Ile Asp Arg Asn Cys Ser Asn Lys Lys Ile Lys Asn Ala
290 295 300

Gly Ser Asn Val Ser Gln Gly Leu Asn Phe Ser Pro Lys Ser Val Leu
305 310 315 320

Gln Val Gln Gly Cys Gly Ala Ser His Ile Tyr Met Asn Asp Val Glu
325 330 335

Leu Glu Leu Gly Arg Cys Arg Arg Thr Asp Gly Lys Lys Trp Arg Cys
340 345 350

Arg Arg Asp Val Val Ala Asn Gln Lys Tyr Cys Glu Met His Met His
355 360 365

Arg Gly Ser Lys Gln His Leu Glu Ala Ser Lys Pro Ala Ala Ile Pro
370 375 380

Ala Thr Ile Pro Phe Val Pro Gly Asn Val His Ser Tyr Pro Ala Thr
385 390 395 400

Asn Leu Pro Ser Lys Ala Asp Arg Arg Ser Leu Asn Thr Asp Leu Cys
405 410 415

Ile Ser Ile Pro Thr Ser Pro Gln Leu Ile Met Thr Asn Asp Asp Thr
420 425 430

Arg Thr Ile Ser Asn Ser Ser Asp Thr Thr Ile Ser Asp Thr Met Arg
435 440 445

Gly Thr Thr Pro Gly Leu Val Ser Thr His Ala Gly Ser Gly Asp Pro
450 455 460

Lys Ser Pro Pro Gly Gly Asp Arg Ala Arg Leu Leu Lys Lys Ala Glu
465 470 475 480

Val Met Glu Ala Ala Val Glu Ile Tyr Glu Glu Val Gln Ser Gly Arg
485 490 495

Gly Val Arg Ser Phe Ile Gln Gly Arg Arg Leu Pro Thr Phe Pro Tyr
500 505 510

Val Tyr Ile Thr Pro Leu Leu Leu Ile Cys Asn
515 520

<210> 75
<211> 1812
<212> DNA

<213> Populus tremuloides

<400> 75

```
aagtaatagt ggttttgctt ctcttgctgg ctcagatcct gaagcaaagc agaagttgta    60
cggatctggg ttcttgaagc aagagagacc tggcaacatc gatggtgatt ggaggagctc   120
taaattgtcg aaaactgagt caatgctgct tgagcagagt aacacttcac ttctgaaatc   180
tagctccaac tttctcttcg ctgatggaca gcagcagcag cagcagatgc tcagcttctc   240
ctatcccaga tcagctcctt cagcggagag aagctcccaa aatggcacat tgccctctgg   300
tatctacaat gctgccagca tgcatggggt tttgaccgag accagatggc cattcactca   360
atcacaatgg atggagcttg aacatcaggc cttgatctac aagtatatag cagcaaatgt   420
gcctatacca tctaatctgc tccttcccat tagaaaagct cttgattctg ctgggtttcc   480
tagcttttct gctggatttt tcaggcccaa tacattgcca tggggtgctt tccatatggg   540
tttctccagc aacactgatc cggagccagg acggtgtcga aggacagatg gaaagaaatg   600
gcggtgctca agagatgcag ttgccgatca gaagtattgt gagcggcata tgaacagggg   660
ccgccatcgt tcaagaaagc ctgtggaagg ccaatcaggc cattccgctg cagccgccac   720
cactgtaaag ccagccaatg gcacttcgtc ttctacatca tcatcagtgg tggggcttcg   780
cagcactgtg tccgacagcc tcactattgc tcataatcag cagcaagcag ctagtccatc   840
taatctttct gcctctaata cgctcagcag gatgttcctt actaaagaga atgtaggtga   900
gagaacgcag gatgcgacag ccttgtccat gcttcgatcc aacatggatc ttaaatctaa   960
agaaactcca ttcttcatat caaaacaaca aaactcatat ggggaatcct tacgaaatga  1020
gtttggactt gtcacctccg actccctcct caatcactca cagaaaagct catctttaat  1080
gagttgcaga aattttggtt cgtctcagga cctcactgac caggaatctg tttcacagca  1140
ctccctccgc caattcatgg atgattggcc taaaagtcag tctgatcgtt ctgctgtttc  1200
ttggcctgaa cttgatcagc aatctgagag aacccagcta tcgatttcaa tccccatggc  1260
tcctgcagac ttcatgtcat ctacttcctc cccaaacaat gaaaaagtca ctctctcccc  1320
attgagatta tcacgagaat ttgatccaat acagatggga ctgggagtgg gaggtggagg  1380
tggtggtatt gccaacgaac caaaccaaag gcaagccaac tggattccca tttcttgggg  1440
aacttcaatg ggtggtccgc tcggggaggt cttgcacaac accaataaca atgcagcagc  1500
agagtgcaag accacgtcat cgctgaacct gatgacctat agatgggaca acagtcctcg  1560
tataggttca tctccaactg gggtcttaca aaagtcagcg tttgcttccc tttcaaatag  1620
cagtgcggga agcagcccaa gagcagagaa caagaccaat gaaggtggca gtctctgcaa  1680
tgacctcctt ggatccacta ttgtgcattc ttcttcattg cctgccatgt aactctgttg  1740
atctgctgcc atccaagaag tctcctgtca tcgtagctga caaaacatgg agcactttgt  1800
ttttgaagtt gt                                                      1812
```

<210> 76
<211> 529

<212> PRT
<213> Populus tremuloides

<400> 76

```
Met Leu Leu Glu Gln Ser Asn Thr Ser Leu Leu Lys Ser Ser Ser Asn
1               5                   10                  15

Phe Leu Phe Ala Asp Gly Gln Gln Gln Gln Gln Gln Met Leu Ser Phe
            20                  25                  30

Ser Tyr Pro Arg Ser Ala Pro Ser Ala Glu Arg Ser Ser Gln Asn Gly
        35                  40                  45

Thr Leu Pro Ser Gly Ile Tyr Asn Ala Ala Ser Met His Gly Val Leu
    50                  55                  60

Thr Glu Thr Arg Trp Pro Phe Thr Gln Ser Gln Trp Met Glu Leu Glu
65                  70                  75                  80

His Gln Ala Leu Ile Tyr Lys Tyr Ile Ala Ala Asn Val Pro Ile Pro
                85                  90                  95

Ser Asn Leu Leu Leu Pro Ile Arg Lys Ala Leu Asp Ser Ala Gly Phe
            100                 105                 110

Pro Ser Phe Ser Ala Gly Phe Phe Arg Pro Asn Thr Leu Pro Trp Gly
        115                 120                 125

Ala Phe His Met Gly Phe Ser Ser Asn Thr Asp Pro Glu Pro Gly Arg
    130                 135                 140

Cys Arg Arg Thr Asp Gly Lys Lys Trp Arg Cys Ser Arg Asp Ala Val
145                 150                 155                 160

Ala Asp Gln Lys Tyr Cys Glu Arg His Met Asn Arg Gly Arg His Arg
                165                 170                 175

Ser Arg Lys Pro Val Glu Gly Gln Ser Gly His Ser Ala Ala Ala Ala
            180                 185                 190

Thr Thr Val Lys Pro Ala Asn Gly Thr Ser Ser Ser Thr Ser Ser Ser
        195                 200                 205

Val Val Gly Leu Arg Ser Thr Val Ser Asp Ser Leu Thr Ile Ala His
    210                 215                 220

Asn Gln Gln Gln Ala Ala Ser Pro Ser Asn Leu Ser Ala Ser Asn Thr
225                 230                 235                 240

Leu Ser Arg Met Phe Leu Thr Lys Glu Asn Val Gly Glu Arg Thr Gln
                245                 250                 255
```

Asp Ala Thr Ala Leu Ser Met Leu Arg Ser Asn Met Asp Leu Lys Ser
260 265 270

Lys Glu Thr Pro Phe Phe Ile Ser Lys Gln Gln Asn Ser Tyr Gly Glu
275 280 285

Ser Leu Arg Asn Glu Phe Gly Leu Val Thr Ser Asp Ser Leu Leu Asn
290 295 300

His Ser Gln Lys Ser Ser Ser Leu Met Ser Cys Arg Asn Phe Gly Ser
305 310 315 320

Ser Gln Asp Leu Thr Asp Gln Glu Ser Val Ser Gln His Ser Leu Arg
325 330 335

Gln Phe Met Asp Asp Trp Pro Lys Ser Gln Ser Asp Arg Ser Ala Val
340 345 350

Ser Trp Pro Glu Leu Asp Gln Gln Ser Glu Arg Thr Gln Leu Ser Ile
355 360 365

Ser Ile Pro Met Ala Pro Ala Asp Phe Met Ser Ser Thr Ser Ser Pro
370 375 380

Asn Asn Glu Lys Val Thr Leu Ser Pro Leu Arg Leu Ser Arg Glu Phe
385 390 395 400

Asp Pro Ile Gln Met Gly Leu Gly Val Gly Gly Gly Gly Gly Gly Ile
405 410 415

Ala Asn Glu Pro Asn Gln Arg Gln Ala Asn Trp Ile Pro Ile Ser Trp
420 425 430

Gly Thr Ser Met Gly Gly Pro Leu Gly Glu Val Leu His Asn Thr Asn
435 440 445

Asn Asn Ala Ala Ala Glu Cys Lys Thr Thr Ser Ser Leu Asn Leu Met
450 455 460

Thr Tyr Arg Trp Asp Asn Ser Pro Arg Ile Gly Ser Ser Pro Thr Gly
465 470 475 480

Val Leu Gln Lys Ser Ala Phe Ala Ser Leu Ser Asn Ser Ser Ala Gly
485 490 495

Ser Ser Pro Arg Ala Glu Asn Lys Thr Asn Glu Gly Gly Ser Leu Cys
500 505 510

Asn Asp Leu Leu Gly Ser Thr Ile Val His Ser Ser Ser Leu Pro Ala
515 520 525

Met

<210> 77
<211> 1017
<212> DNA
<213> Populus tremuloides

<400> 77

```
agtgatgacc atgaggtcgc catttacagt atcgcaatgg caagaactgg aacatcaagc     60
tttgatctat aagtacatgg tggcaggtct gcctgttcct cctgatcttg tgctccctat    120
tcagaggagt tttgagtcca tttctcatag attcttccac catcccgcca tgggctattg    180
cactttctat gggaagaagg tggatccgga gccaggtcaa tgcaggagga ccgacggcaa    240
gaagtggagg tgctccaaag atgcataccc gggctccaag tactgtgagc gccacatgca    300
ccgtggccgc aaccgttcaa gaaaggctgt ggaatcacaa accatgacac agtcatcgtc    360
cactgtgaca tcactgactg taacaggaag cagcagtgga acagggagct tccagaacct    420
tccactgcgc acatatggta atccccaggg cactggttct ggacctaacc aatcccatta    480
tcatatgaac tgcattcccc gtggaatccc aactaaagat tgcaggtatc ttcaaggact    540
gaagactgag ggtggcgagc atagcttctt gtctgaacct tcaggatgca aaaggggtct    600
ccagaaggac tcacagctag acaatgcctg gtctttgatg ctatccagag gctcatcatt    660
ccccacagag aaatcgactg acgactcgac gttgaagaat gattatcccc agcattcatt    720
tttcagtagt gatttcacca ccggagaacc cgtgaaacac gaagggcagt ctcttcgacc    780
cttctttgac gagtggcctg aggaccagga catttggtct ggcctcaaag ataatagatc    840
caactccacc tcattctcta caacgaagct gtcgatgtcc attccaattg cctcatctgg    900
cttctccaca agttctcgtt ctccacaaga aaactgagag gacagaatga gaatttacaa    960
agcacgatca aaggtgatcc tcaaccaaag attggtgtgt taacttgaaa actccta      1017
```

<210> 78
<211> 310
<212> PRT
<213> Populus tremuloides

<400> 78

Met Thr Met Arg Ser Pro Phe Thr Val Ser Gln Trp Gln Glu Leu Glu
1 5 10 15

His Gln Ala Leu Ile Tyr Lys Tyr Met Val Ala Gly Leu Pro Val Pro
20 25 30

Pro Asp Leu Val Leu Pro Ile Gln Arg Ser Phe Glu Ser Ile Ser His
35 40 45

Arg Phe Phe His His Pro Ala Met Gly Tyr Cys Thr Phe Tyr Gly Lys
50 55 60

Lys Val Asp Pro Glu Pro Gly Gln Cys Arg Arg Thr Asp Gly Lys Lys
65 70 75 80

Trp Arg Cys Ser Lys Asp Ala Tyr Pro Gly Ser Lys Tyr Cys Glu Arg
85 90 95

His Met His Arg Gly Arg Asn Arg Ser Arg Lys Ala Val Glu Ser Gln
100 105 110

Thr Met Thr Gln Ser Ser Ser Thr Val Thr Ser Leu Thr Val Thr Gly
115 120 125

Ser Ser Ser Gly Thr Gly Ser Phe Gln Asn Leu Pro Leu Arg Thr Tyr
130 135 140

Gly Asn Pro Gln Gly Thr Gly Ser Gly Pro Asn Gln Ser His Tyr His
145 150 155 160

Met Asn Cys Ile Pro Arg Gly Ile Pro Thr Lys Asp Cys Arg Tyr Leu
165 170 175

Gln Gly Leu Lys Thr Glu Gly Gly Glu His Ser Phe Leu Ser Glu Pro
180 185 190

Ser Gly Cys Lys Arg Gly Leu Gln Lys Asp Ser Gln Leu Asp Asn Ala
195 200 205

Trp Ser Leu Met Leu Ser Arg Gly Ser Ser Phe Pro Thr Glu Lys Ser
210 215 220

Thr Asp Asp Ser Thr Leu Lys Asn Asp Tyr Pro Gln His Ser Phe Phe
225 230 235 240

Ser Ser Asp Phe Thr Thr Gly Glu Pro Val Lys His Glu Gly Gln Ser
245 250 255

Leu Arg Pro Phe Phe Asp Glu Trp Pro Glu Asp Gln Asp Ile Trp Ser
260 265 270

Gly Leu Lys Asp Asn Arg Ser Asn Ser Thr Ser Phe Ser Thr Thr Lys
275 280 285

Leu Ser Met Ser Ile Pro Ile Ala Ser Ser Gly Phe Ser Thr Ser Ser
290 295 300

Arg Ser Pro Gln Glu Asn
305 310

<210> 79
<211> 1221
<212> DNA
<213> Populus tremuloides

<400> 79

acaaccctct gcaaagatgc caaaactcct catggatccc catcaaccac aacaacatcc 60

```
acactcatct gggtctgctg ccttcccttt gtttctaccc gagcccagct gcaaaaatag     120
taacctgtca gcatttcctg attcaaacac agctgcaaac accagacttc ctaagatcat     180
ggggaattac tttagcctgg aacagtggca agagctagag ctgcaggctt tgatctacag     240
attcatgtta gccggtgcag ctattcctcc ggagctcctc caaccaatca agaaaaccct     300
tcttcattct caccccctc catatttcct ccatcatcct cttcaattac attgctctta     360
ttatcagcca tcttggtatt ggggaagagc agccatggat ccggagccag gtcggtgccg     420
gagaacagat gggaagaaat ggcggtgctc cagagacgtg gtggcagggc acaagtattg     480
cgagcgccac ttgcaccgtg gccgcaaccg ttcaagaaag cctgtggaaa atcccacacc     540
tacaatatcc actaacatca cttgcattgg tattggaggt gcgggtggta ccgcatcagc     600
tgctgctttc aattgcagca ccacaccaac catatcagag gtggtcaatg agactcattt     660
ttcgcataca ctagaatccc cttccattca tctcaatcat agctccaaaa ctgaaagcaa     720
gggcttaatt ggaccaccac ctccaaatga ggttggtaac aggtctgatg gccacattct     780
gtggcatttt tttgatgact ggccacgatc cgttgatgaa tccgacaata tgaatgctgg     840
aagctcaatg aactctttaa cctgcctctc cgtttcaatg cctggaaact caccagcatc     900
agatgtgtca ttgaaattgt ccactgggaa taatattgca gaggaggagc cggagccagt     960
cccagccccg atccctagag gcaatacaag caattgggct gctgcaggat ggggcacaaa    1020
aattacaaac caggtggtga cttcaatggg gggacctctt gctgaggcgc tgaggtcctc    1080
cactaccaaa ctcatctccc acgaatgttc tgcaccagtt atgtcgcccc actgtttctg    1140
aaacttgatc tattttaggt tagtttgtgg tgtagtaaca catgcatgca tacacacaca    1200
cacacacaca cacacacatc a                                              1221
```

<210> 80
<211> 377
<212> PRT
<213> Populus tremuloides

<400> 80

```
Met Asp Phe His Leu Lys Gln Trp Arg Asn Gln His Glu Glu Ser Gly
1               5                   10                  15

Gln Gln Pro Ser Ala Lys Met Pro Lys Leu Leu Met Asp Pro His Gln
            20                  25                  30

Pro Gln Gln His Pro His Ser Ser Gly Ser Ala Ala Phe Pro Leu Phe
        35                  40                  45

Leu Pro Glu Pro Ser Cys Lys Asn Ser Asn Leu Ser Ala Phe Pro Asp
    50                  55                  60

Ser Asn Thr Ala Ala Asn Thr Arg Leu Pro Lys Ile Met Gly Asn Tyr
65                  70                  75                  80
```

```
Phe Ser Leu Glu Gln Trp Gln Glu Leu Glu Leu Gln Ala Leu Ile Tyr
                85                  90                  95

Arg Phe Met Leu Ala Gly Ala Ala Ile Pro Pro Glu Leu Leu Gln Pro
            100                 105                 110

Ile Lys Lys Thr Leu Leu His Ser His Pro Pro Pro Tyr Phe Leu His
        115                 120                 125

His Pro Leu Gln Leu His Cys Ser Tyr Tyr Gln Pro Ser Trp Tyr Trp
    130                 135                 140

Gly Arg Ala Ala Met Asp Pro Glu Pro Gly Arg Cys Arg Arg Thr Asp
145                 150                 155                 160

Gly Lys Lys Trp Arg Cys Ser Arg Asp Val Val Ala Gly His Lys Tyr
                165                 170                 175

Cys Glu Arg His Leu His Arg Gly Arg Asn Arg Ser Arg Lys Pro Val
            180                 185                 190

Glu Asn Pro Thr Pro Thr Ile Ser Thr Asn Ile Thr Cys Ile Gly Ile
        195                 200                 205

Gly Glu Leu Asp Gln Thr Thr Phe Ser Leu Phe Cys Phe Cys Phe Asn
    210                 215                 220

Leu Leu Ala His Pro Tyr Cys Ser Ser Lys Thr Glu Ser Lys Gly Leu
225                 230                 235                 240

Ile Gly Pro Pro Pro Pro Asn Glu Val Gly Asn Arg Ser Asp Gly His
                245                 250                 255

Ile Leu Trp His Phe Phe Asp Asp Trp Pro Arg Ser Val Asp Glu Ser
            260                 265                 270

Asp Asn Met Asn Ala Gly Ser Ser Met Asn Ser Leu Thr Cys Leu Ser
        275                 280                 285

Val Ser Met Pro Gly Asn Ser Pro Ala Ser Asp Val Ser Leu Lys Leu
    290                 295                 300

Ser Thr Gly Asn Asn Ile Ala Glu Glu Glu Pro Glu Pro Val Pro Ala
305                 310                 315                 320

Pro Ile Pro Arg Gly Asn Thr Ser Asn Trp Ala Ala Ala Gly Trp Gly
                325                 330                 335

Thr Lys Ile Thr Asn Gln Val Val Thr Ser Met Gly Gly Pro Leu Ala
            340                 345                 350

Glu Ala Leu Arg Ser Ser Thr Thr Lys Leu Ile Ser His Glu Cys Ser
```

355 360 365

Ala Pro Val Met Ser Pro His Cys Phe
370 375

<210> 81
<211> 1280
<212> DNA
<213> Populus tremuloides

<400> 81

```
gcgctcgctt gtgttgtggt gacaggagaa atgacaccat ctgtgaatga gagagtgctt     60
tttacagctg ctcagtggca agaacttgaa agacaaacca cgatttacaa gtacatgatg    120
gcttctgttc ctgtccctcc tgaactcctt atacccatca ccaaaaatca atcaaatgtc    180
cttcctccac ggtctaacag ttcactagaa ctgggaattc ctagcctgaa ctcatcagat    240
gcagaaccat ggagatgcaa aagaactgat gggaaaaaat ggaggtgttc aagagatgtg    300
gcacctgacc agaaatactg tgagaggcac tctcataaga gccgtccccg ttcaagaaag    360
cctgtggaat tacacactca tgactccccg aggacattga ccaacaataa cactaacacc    420
aacaatagca attactccac taatccacac ctgtttaatc aaaaacctta ctttccaagc    480
catttattta tgtttcctag tgccatggcc ccttctgcca gctcatatga tcaacccagg    540
agcttggaat ggctcttgaa aggcgagatt ttacccgttg ccagtaatta cagccaagaa    600
tggcagcatt tgaagagaga cagcatcaag ggtaatggca aagtgtacaa cgtttatgga    660
gaagagcagc cgctttgctc aaatacatat agaggtggcc attcattaca agctcagagg    720
ctaaatgatc attgcagcgt gttatcaagt cccaaatcaa ctactttgga aagggcttta    780
agtcctagcc tgacccaaga acaagagaca aggcacttca ttgatgcttg gtcaactaat    840
tcagggagag acgacattgg tgggattggt aaaaaaagtt acgtttcttc aagtgagaag    900
ttagtattgc cacattcagc tcttacattg tcaatgtcac ctggcactgg aagtgaaact    960
aataatgaag gaaatgggag tgctcaactg agtagttttg ggatcatggg attatcagat   1020
agagatcatc agagtgcgag tggcttgaga cctcagtgga tgatgagtca tggtggttca   1080
tggatagtat caccacctgg tggaccatta gctgaagcct tgtgtcttgg catttccagc   1140
aatgcaaaaa ctgcttccaa tttaccatcc ccttgcagca gtagctgtgg ccccaattaa   1200
tgtcaacaaa aaccaaccgc tatagtttgt tgtaaattct ggctgggtag aggccagagg   1260
gtagaagcta atgtggccca                                                1280
```

<210> 82
<211> 513
<212> PRT
<213> Populus tremuloides

<400> 82

Met Arg Ser Ser Trp Ser Arg Thr Arg Ser Gly Val Phe Val Asp Asp
1               5                   10                  15

Ile Gly Leu Gly Leu Arg Met Gln Asp Asn Leu Glu Ser Cys Ser Gly
20 25 30

Ser Ser Lys Arg Ser Val Thr Ala Met Ser Cys Asp His Glu Pro Ala
35 40 45

Ala His Glu Leu Ser Ser Ser Ser Cys Ser Gly Gly Gly Gly Gly Ser
50 55 60

Gly Pro Leu Phe Tyr Ser Thr Ser Asn His Val Thr Cys Leu Gly Asp
65 70 75 80

Ile Lys Asp Val Val Ala Ser Val Ser Ala Ser Gly Thr Gly Thr Pro
85 90 95

Asp Ala Ile Ala Glu Ser Lys Ser Leu Gln Tyr Pro Tyr Phe Ile Ser
100 105 110

Asp Ser Ser Pro Phe Thr Phe Asn Ser Ser Gly Glu Met Thr Pro Ser
115 120 125

Val Asn Glu Arg Val Leu Phe Thr Ala Ala Gln Trp Gln Glu Leu Glu
130 135 140

Arg Gln Thr Thr Ile Tyr Lys Tyr Met Met Ala Ser Val Pro Val Pro
145 150 155 160

Pro Glu Leu Leu Ile Pro Ile Thr Lys Asn Gln Ser Asn Val Leu Pro
165 170 175

Pro Arg Ser Asn Ser Ser Leu Glu Leu Gly Ile Pro Ser Leu Asn Ser
180 185 190

Ser Asp Ala Glu Pro Trp Arg Cys Lys Arg Thr Asp Gly Lys Lys Trp
195 200 205

Arg Cys Ser Arg Asp Val Ala Pro Asp Gln Lys Tyr Cys Glu Arg His
210 215 220

Ser His Lys Ser Arg Pro Arg Ser Arg Lys Pro Val Glu Leu His Thr
225 230 235 240

His Asp Ser Pro Arg Thr Leu Thr Asn Asn Asn Thr Asn Thr Asn Asn
245 250 255

Ser Asn Tyr Ser Thr Asn Pro His Leu Phe Asn Gln Lys Pro Tyr Phe
260 265 270

Pro Ser His Leu Phe Met Phe Pro Ser Ala Met Ala Pro Ser Ala Ser
275 280 285

Ser Tyr Asp Gln Pro Arg Ser Leu Glu Trp Leu Leu Lys Gly Glu Ile

290 295 300

Leu Pro Val Ala Ser Asn Tyr Ser Gln Glu Trp Gln His Leu Lys Arg
305 310 315 320

Asp Ser Ile Lys Gly Asn Gly Lys Val Tyr Asn Val Tyr Gly Glu Glu
325 330 335

Gln Pro Leu Cys Ser Asn Thr Tyr Arg Gly Gly His Ser Leu Gln Ala
340 345 350

Gln Arg Leu Asn Asp His Cys Ser Val Leu Ser Ser Pro Lys Ser Thr
355 360 365

Thr Leu Glu Arg Ala Leu Ser Pro Ser Leu Thr Gln Glu Gln Glu Thr
370 375 380

Arg His Phe Ile Asp Ala Trp Ser Thr Asn Ser Gly Arg Asp Asp Ile
385 390 395 400

Gly Gly Ile Gly Lys Lys Ser Tyr Val Ser Ser Ser Glu Lys Leu Val
405 410 415

Leu Pro His Ser Ala Leu Thr Leu Ser Met Ser Pro Gly Thr Gly Ser
420 425 430

Glu Thr Asn Asn Glu Gly Asn Gly Ser Ala Gln Leu Ser Ser Phe Gly
435 440 445

Ile Met Gly Leu Ser Asp Arg Asp His Gln Ser Ala Ser Gly Leu Arg
450 455 460

Pro Gln Trp Met Met Ser His Gly Gly Ser Trp Ile Val Ser Pro Pro
465 470 475 480

Gly Gly Pro Leu Ala Glu Ala Leu Cys Leu Gly Ile Ser Ser Asn Ala
485 490 495

Lys Thr Ala Ser Asn Leu Pro Ser Pro Cys Ser Ser Ser Cys Gly Pro
500 505 510

Asn

<210> 83
<211> 1623
<212> DNA
<213> Saccharum officinarum

<400> 83

ccagcatcca ctctctcatc agcagcctct tcttcttctc ccccaaatga gtgctgagtt 60

ttgtgctgct gcgggcatgg agctcggagt cggggatgtg atggggctgc agcaaggcat 120

```
cgccatcacc gcgccatcgc ccaggggcag cggcgacctg ggtcttctca agcgagcagc    180
cctcacccag gcagcagctg gcccctaccc ctcccccttc ctcgacgaac agaagatgct    240
caggttctcc aaggcggctc acacattgcc ctcagggcta ggcttggatt ttggaggccc    300
aagcgagcag gctttcctgc tgtccaggac caagaggcca tttactccct cgcagtggat    360
ggagctggag caccaggctc tgatatacaa gtatctcaat gcaaaggccc ccataccttc    420
cagcctgctc atttcaatca gcaagagctt cagatcatcc aatagagtga gctggaggcc    480
tctctatcaa ggctacacaa atgcagactc tgacccagag cctggaagat gccgacgaac    540
agatggaaag aagtggcgat gctccaagga ggcaatggct gatcacaagt actgtgagcg    600
gcacatcaac agaaaccgtc accgttcaag aaagcctgtg gaaaaccaac ccaaaaagac    660
caccaaggag gtgcctgctg ctgctagctc attgccatgt gctgggccac aaggttgctt    720
gaagaaggca aaagttaatg actccaagcc aggcactgtc agctgttgga cagatagttt    780
aaacaggaca atgttgagca gagagaaagc aaacaaaccg acggaggaca actctttgct    840
gcttaattct acgaatagcc agcccacctt gtccctgctc tctcaactga agcagcagaa    900
caaaccagat aagttaggtc ccacactgga aaatgagtca aactcagaca caatactgaa    960
agcctggggt ggcaaccagc ctagccacaa gagcatttcc tccacacagc accatgatgc   1020
tgaatccctc caatcagtcc ttcaaaattt cagcctagcc cagaatgaga agatggagtc   1080
agaaaagaac aaatattctg attccatgct agtttcatcg actttctatt ctgcagacgg   1140
tccacgatct acctgcctta cacctaacat gacacaagtg cagcaggatt gcatatcaag   1200
ctcttgggag atgcctcaag gtggacctct aggcgagatc ttaaccaact ccaagaatag   1260
tgaggactta agcaagtgtg aatcaaggtc atatggttgg ttattgaatc ttgaccatgc   1320
accatgattc ctcaatccat ggagagcttg acatagatgt ctcaccatgg aagcaaacaa   1380
tggtcagaaa aagaaggttc aaatgaccac attgtttgcc ccatgcatgc tcgctatcta   1440
catttgtatt tctgttttgt agcatttagc tagttgaatt atcagttctt ctgaatctgg   1500
ctgtatttta aacaaattct agtttgtgtc agatgatatc ttgcttgcta gatgtttcat   1560
gtctaacttt caacaggaac ttcagagatc cattttgatc aacagaaaac tgtttgaaga   1620
acc                                                                 1623
```

<210> 84
<211> 426
<212> PRT
<213> Saccharum officinarum

<400> 84

```
Met Ser Ala Glu Phe Cys Ala Ala Ala Gly Met Glu Leu Gly Val Gly
1               5                   10                  15

Asp Val Met Gly Leu Gln Gln Gly Ile Ala Ile Thr Ala Pro Ser Pro
            20                  25                  30

Arg Gly Ser Gly Asp Leu Gly Leu Leu Lys Arg Ala Ala Leu Thr Gln
```

35 40 45

Ala Ala Ala Gly Pro Tyr Pro Ser Pro Phe Leu Asp Glu Gln Lys Met
50 55 60

Leu Arg Phe Ser Lys Ala Ala His Thr Leu Pro Ser Gly Leu Gly Leu
65 70 75 80

Asp Phe Gly Gly Pro Ser Glu Gln Ala Phe Leu Leu Ser Arg Thr Lys
85 90 95

Arg Pro Phe Thr Pro Ser Gln Trp Met Glu Leu Glu His Gln Ala Leu
100 105 110

Ile Tyr Lys Tyr Leu Asn Ala Lys Ala Pro Ile Pro Ser Ser Leu Leu
115 120 125

Ile Ser Ile Ser Lys Ser Phe Arg Ser Ser Asn Arg Val Ser Trp Arg
130 135 140

Pro Leu Tyr Gln Gly Tyr Thr Asn Ala Asp Ser Asp Pro Glu Pro Gly
145 150 155 160

Arg Cys Arg Arg Thr Asp Gly Lys Lys Trp Arg Cys Ser Lys Glu Ala
165 170 175

Met Ala Asp His Lys Tyr Cys Glu Arg His Ile Asn Arg Asn Arg His
180 185 190

Arg Ser Arg Lys Pro Val Glu Asn Gln Pro Lys Lys Thr Thr Lys Glu
195 200 205

Val Pro Ala Ala Ala Ser Ser Leu Pro Cys Ala Gly Pro Gln Gly Cys
210 215 220

Leu Lys Lys Ala Lys Val Asn Asp Ser Lys Pro Gly Thr Val Ser Cys
225 230 235 240

Trp Thr Asp Ser Leu Asn Arg Thr Met Leu Ser Arg Glu Lys Ala Asn
245 250 255

Lys Pro Thr Glu Asp Asn Ser Leu Leu Leu Asn Ser Thr Asn Ser Gln
260 265 270

Pro Thr Leu Ser Leu Leu Ser Gln Leu Lys Gln Gln Asn Lys Pro Asp
275 280 285

Lys Leu Gly Pro Thr Leu Glu Asn Glu Ser Asn Ser Asp Thr Ile Leu
290 295 300

Lys Ala Trp Gly Gly Asn Gln Pro Ser His Lys Ser Ile Ser Ser Thr
305 310 315 320

Gln His His Asp Ala Glu Ser Leu Gln Ser Val Leu Gln Asn Phe Ser
325 330 335

Leu Ala Gln Asn Glu Lys Met Glu Ser Glu Lys Asn Lys Tyr Ser Asp
340 345 350

Ser Met Leu Val Ser Ser Thr Phe Tyr Ser Ala Asp Gly Pro Arg Ser
355 360 365

Thr Cys Leu Thr Pro Asn Met Thr Gln Val Gln Gln Asp Cys Ile Ser
370 375 380

Ser Ser Trp Glu Met Pro Gln Gly Gly Pro Leu Gly Glu Ile Leu Thr
385 390 395 400

Asn Ser Lys Asn Ser Glu Asp Leu Ser Lys Cys Glu Ser Arg Ser Tyr
405 410 415

Gly Trp Leu Leu Asn Leu Asp His Ala Pro
420 425

<210> 85
<211> 1515
<212> DNA
<213> Vitis vinifera

<400> 85

atgatgatga gtgcaagaaa caggtctcct ttcacagcat cacagtggca agagcttgaa 60
catcaagctc ttatcttcaa atatatagtg tcaggagtac caatcccagc tgatctcatc 120
tgcactgtca aaagaagctt ggactcttca ttgtcttcaa ggctatttcc tcaccaaccc 180
attgggtggg gttgttttca gatggggttt ggcaggaaag cagacccaga gccagggagg 240
tgcagaagaa ctgatggcaa gaaatggagg tgctccaaag aagcataccc agattcaaaa 300
tactgtgaga gacacatgca cagaggcaaa aaccgttcaa gaaagcctgt ggaagttatt 360
tcagctacaa acccttcacc aaccatctca tcaatcaact caaatccttc ctccaccacc 420
accaattctt actctctctc tcctctctct cctctctctt cttcaatgac ttctgaaacc 480
tcccatcccc atcaccattc ctaccacaac acctctcttt atcccttcct ctaccctcac 540
ccctcctctt ctagacctcc tggttcttgc ctatcacctc aagccaccag cagttacagc 600
acccatcatc tgtttttgga ctctgggtct tattcccagg ctgataggga ttacaggggt 660
gtggatgaga gagctttctt cccagaagct tcagggactg taaggggcct acatgattca 720
tatactccat taacaatgag ttcctccaag ggatactctc actttcagta tcaaagcccc 780
gctgataatc ccaaacagca gcaagaacag caagagcagc agcactgctt tgtcttgggc 840
actgatttca aatcgtcaag gccaattaaa gtagagagag atgatgaagc ccagaagcct 900
ctccaccatt tctttggaga gtggcctcta aagaacagag actcctggct tgaccttgag 960
gaggatccac caacccatgc atcattctcc accacccagc tctcaatttc aatcccaatg 1020

```
tcctcacaca agcttcttgc atcggattcc agaatccaaa ctggtactta gacttcactt    1080
cagatttggc cttcttgccc tttattttcc cttttctgct aagtctcatc ttctacttca    1140
tttttccccc ttgtgcagat ggatgagttc tcaatactgg ttcttctgat catggccaaa    1200
aaagtacttg tactggtggg ttcaatgctt ttcttgttga ttttgtgact gaaggaagtt    1260
tcttttgcca aatgtgcgga tgaataatgt agggcctata ggaggattct tgtctttgtg    1320
ctttctgagt tgctaatttt cattcttatc tttaaagaaa catgtttgaa tttgtagact    1380
tgtttgtttg acaggaagca tcttgatatg gtttttgagt ttgagtttga gttgttctct    1440
aatctctatg ttgttttgtg agttgtggcc accttttctt ttccctttgg cttctgctta    1500
ttgtacttca gaaag                                                     1515
```

<210> 86
<211> 356
<212> PRT
<213> vitis vinifera

<400> 86

```
Met Met Met Ser Ala Arg Asn Arg Ser Pro Phe Thr Ala Ser Gln Trp
1               5                   10                  15

Gln Glu Leu Glu His Gln Ala Leu Ile Phe Lys Tyr Ile Val Ser Gly
            20                  25                  30

Val Pro Ile Pro Ala Asp Leu Ile Cys Thr Val Lys Arg Ser Leu Asp
        35                  40                  45

Ser Ser Leu Ser Ser Arg Leu Phe Pro His Gln Pro Ile Gly Trp Gly
    50                  55                  60

Cys Phe Gln Met Gly Phe Gly Arg Lys Ala Asp Pro Glu Pro Gly Arg
65                  70                  75                  80

Cys Arg Arg Thr Asp Gly Lys Lys Trp Arg Cys Ser Lys Glu Ala Tyr
                85                  90                  95

Pro Asp Ser Lys Tyr Cys Glu Arg His Met His Arg Gly Lys Asn Arg
            100                 105                 110

Ser Arg Lys Pro Val Glu Val Ile Ser Ala Thr Asn Pro Ser Pro Thr
        115                 120                 125

Ile Ser Ser Ile Asn Ser Asn Pro Ser Ser Thr Thr Thr Asn Ser Tyr
    130                 135                 140

Ser Leu Ser Pro Leu Ser Pro Leu Ser Ser Ser Met Thr Ser Glu Thr
145                 150                 155                 160

Ser His Pro His His His Ser Tyr His Asn Thr Ser Leu Tyr Pro Phe
                165                 170                 175
```

Leu Tyr Pro His Pro Ser Ser Ser Arg Pro Pro Gly Ser Cys Leu Ser
180 185 190

Pro Gln Ala Thr Ser Ser Tyr Ser Thr His His Leu Phe Leu Asp Ser
195 200 205

Gly Ser Tyr Ser Gln Ala Asp Arg Asp Tyr Arg Gly Val Asp Glu Arg
210 215 220

Ala Phe Phe Pro Glu Ala Ser Gly Thr Val Arg Gly Leu His Asp Ser
225 230 235 240

Tyr Thr Pro Leu Thr Met Ser Ser Ser Lys Gly Tyr Ser His Phe Gln
245 250 255

Tyr Gln Ser Pro Ala Asp Asn Pro Lys Gln Gln Gln Glu Gln Gln Glu
260 265 270

Gln Gln His Cys Phe Val Leu Gly Thr Asp Phe Lys Ser Ser Arg Pro
275 280 285

Ile Lys Val Glu Arg Asp Asp Glu Ala Gln Lys Pro Leu His His Phe
290 295 300

Phe Gly Glu Trp Pro Leu Lys Asn Arg Asp Ser Trp Leu Asp Leu Glu
305 310 315 320

Glu Asp Pro Pro Thr His Ala Ser Phe Ser Thr Thr Gln Leu Ser Ile
325 330 335

Ser Ile Pro Met Ser Ser His Lys Leu Leu Ala Ser Asp Ser Arg Ile
340 345 350

Gln Thr Gly Thr
355

<210> 87
<211> 945
<212> DNA
<213> Zea mays

<400> 87

agagcgccgt atcacctgtc tctccgtcca ccgccgtctc gatccgcgcc aaagatacct 60
ttcccccacc ccttcctcgc gccgccgttt ggtgcgacca tgacggcgga gggggaggcc 120
aagaacccgt cggccggtgg cggaggggat aacccccagc accagcaggc tgcgccggcg 180
ccggcgccgg cacaggggga agtggcgcag gaggctgcag tgcaggggac gggacaagag 240
caggagcggg acaaggcgga tcgagaggtg cagggcggcg cgggggagaa ggacgacggc 300
gcgtgcagag atctggtcct ggtcgaggat ccggaggtcc tcgccgtcga ggatccggag 360
gaagctgcag caaccgcagc actccaggaa gaaatgaaag cgcttgtggc atcgatccct 420

gatggtgctg gagcagcatt cacagccatg cagcttcagg agctagagca gcagtcccgg 480
gtgtaccagt acatggctgc ccgagtacct gtgcctactc acctcgtctt cccggtatgg 540
aagagtgtga ccggtgcatc ctctgaaggc gcccagaagt accctacttt gatgggctta 600
gcaacgctct gcttggactt tgggaagaac ccggaaccag aaccagggag gtgtcggcga 660
acagatggta agaaatggcg atgttggaga aacactatcc caaacgagaa atactgcgaa 720
cgtcacatgc atcgtggccg caagcgtcct gtacaggttt tcctggagga cgacgagccc 780
gattctgctt cagggtcaaa acccgccgct cctggcaagg ctaccgaagg tgccaagaag 840
gccgatgaca agagcccaag cagcaagaag cttgcagtgg cagcgcctgc cgctgtgcag 900
tctacatagt caattgcagc tttagtagcc cgcagaaaga gcata 945

<210> 88
<211> 269
<212> PRT
<213> Zea mays

<400> 88

Met Thr Ala Glu Gly Glu Ala Lys Asn Pro Ser Ala Gly Gly Gly Gly
1 5 10 15

Asp Asn Pro Gln His Gln Gln Ala Ala Pro Ala Pro Ala Pro Ala Gln
20 25 30

Gly Glu Val Ala Gln Glu Ala Ala Val Gln Gly Thr Gly Gln Glu Gln
35 40 45

Glu Arg Asp Lys Ala Asp Arg Glu Val Gln Gly Gly Ala Gly Glu Lys
50 55 60

Asp Asp Gly Ala Cys Arg Asp Leu Val Leu Val Glu Asp Pro Glu Val
65 70 75 80

Leu Ala Val Glu Asp Pro Glu Glu Ala Ala Ala Thr Ala Ala Leu Gln
85 90 95

Glu Glu Met Lys Ala Leu Val Ala Ser Ile Pro Asp Gly Ala Gly Ala
100 105 110

Ala Phe Thr Ala Met Gln Leu Gln Glu Leu Glu Gln Gln Ser Arg Val
115 120 125

Tyr Gln Tyr Met Ala Ala Arg Val Pro Val Pro Thr His Leu Val Phe
130 135 140

Pro Val Trp Lys Ser Val Thr Gly Ala Ser Ser Glu Gly Ala Gln Lys
145 150 155 160

Tyr Pro Thr Leu Met Gly Leu Ala Thr Leu Cys Leu Asp Phe Gly Lys
165 170 175

Asn Pro Glu Pro Glu Pro Gly Arg Cys Arg Arg Thr Asp Gly Lys Lys
180 185 190

Trp Arg Cys Trp Arg Asn Thr Ile Pro Asn Glu Lys Tyr Cys Glu Arg
195 200 205

His Met His Arg Gly Arg Lys Arg Pro Val Gln Val Phe Leu Glu Asp
210 215 220

Asp Glu Pro Asp Ser Ala Ser Gly Ser Lys Pro Ala Ala Pro Gly Lys
225 230 235 240

Ala Thr Glu Gly Ala Lys Lys Ala Asp Asp Lys Ser Pro Ser Ser Lys
245 250 255

Lys Leu Ala Val Ala Ala Pro Ala Ala Val Gln Ser Thr
260 265

<210> 89
<211> 1372
<212> DNA
<213> Zea mays

<400> 89

gcctctgaca ccagcacaaa cctggagact actactagta ttggagtccc ctccacttcc 60
acctcccttg ccactgaagc gagagctctc ggagccgtcg tcctctgtct ctcatccttc 120
ttcgttgttg agcaaagcgg gctcgaggag gagatgatgc tgagcgggca cggcggcggg 180
aggcgcctgt tcacggcgtc gcagtggcag gagctggagc accaggcgct catcttcaaa 240
tacatggcct ccggcgcgcc cgtgccgcac gacctcgtcc tgccgctccg cctcgccacc 300
ggcgtcgaca ccgcgccctc cctcgccttc ccgccccagc cttcgccgtc gctggcgtac 360
tggggctgct atggcgcggg ggcgccgttc ggccgcaagg cggaggaccc ggagcccggg 420
cggtgccggc ggacggacgg caagaagtgg cgatgctcca gggaggccca cggagactcc 480
aagtactgcg agaagcacat ccaccgcggg aagagccgtt caagaaagcc tgtggaagtg 540
acctcccccg ccgcctaccg cccgtccgcg ttctccatct cgccgcctcg cgcggccgac 600
gcgccgccgc cgccgccggg cctcggccac ccgcagcagc agcatctccg ccacggcgct 660
ctctctccag caggccgcgc ccacgccgct ggcgctctcc agctccacct cgactcgagc 720
ctgcacgcgg cgtcgccgcc gccgtcctac cacaggtacg cccactccca cgctcactac 780
acgccgccgc cgccgccgtc gctctacgac tacgggcagt ccaaggagct tcgggaggcg 840
gcggagctca ggcggcggca cttccacgcg ctcggggccg acctgagcct cgacaagccg 900
ctggccgacg ccggggccgc ggagaagccc ctgcggcgtt tcttcgacga gtggccgcgg 960
gagagaggcg acacgaggcc gtcgtgggcg gggcggaggg acgcgacgca gctctccatc 1020
tccatccccg cggcttcgcc ctcctctgac cacgctgcct ctgccgccgc gcgatgccac 1080
aacgatggga gtgatcggtg catctcctag ctgcaactgc aatgcaagcc tgcaaccgcg 1140

```
tggattgttg ttgattggtg tagtttccta gctgcaattc aagcctgcaa cagcgagcag    1200
tgagcagcaa atgcgtgggg agggcacgca gctcaggctg atgcgcaaaa tccgaagcga    1260
gtcaagcagc aataggactc taggtctatg atttgatctt cctttgtagc agtacgttac    1320
caaaatgtta gctcgttgtt gttcggtgtg acattttcgt tcaggttgct cc            1372
```

<210> 90
<211> 318
<212> PRT
<213> Zea mays

<400> 90

```
Met Met Leu Ser Gly His Gly Gly Gly Arg Arg Leu Phe Thr Ala Ser
1               5                   10                  15

Gln Trp Gln Glu Leu Glu His Gln Ala Leu Ile Phe Lys Tyr Met Ala
            20                  25                  30

Ser Gly Ala Pro Val Pro His Asp Leu Val Leu Pro Leu Arg Leu Ala
        35                  40                  45

Thr Gly Val Asp Thr Ala Pro Ser Leu Ala Phe Pro Pro Gln Pro Ser
    50                  55                  60

Pro Ser Leu Ala Tyr Trp Gly Cys Tyr Gly Ala Gly Ala Pro Phe Gly
65                  70                  75                  80

Arg Lys Ala Glu Asp Pro Glu Pro Gly Arg Cys Arg Arg Thr Asp Gly
                85                  90                  95

Lys Lys Trp Arg Cys Ser Arg Glu Ala His Gly Asp Ser Lys Tyr Cys
            100                 105                 110

Glu Lys His Ile His Arg Gly Lys Ser Arg Ser Arg Lys Pro Val Glu
        115                 120                 125

Val Thr Ser Pro Ala Ala Tyr Arg Pro Ser Ala Phe Ser Ile Ser Pro
    130                 135                 140

Pro Arg Ala Ala Asp Ala Pro Pro Pro Pro Pro Gly Leu Gly His Pro
145                 150                 155                 160

Gln Gln Gln His Leu Arg His Gly Ala Leu Ser Pro Ala Gly Arg Ala
                165                 170                 175

His Ala Ala Gly Ala Leu Gln Leu His Leu Asp Ser Ser Leu His Ala
            180                 185                 190

Ala Ser Pro Pro Pro Ser Tyr His Arg Tyr Ala His Ser His Ala His
        195                 200                 205
```

Tyr Thr Pro Pro Pro Pro Pro Ser Leu Tyr Asp Tyr Gly Gln Ser Lys
210 215 220

Glu Leu Arg Glu Ala Ala Glu Leu Arg Arg Arg His Phe His Ala Leu
225 230 235 240

Gly Ala Asp Leu Ser Leu Asp Lys Pro Leu Ala Asp Ala Gly Ala Ala
245 250 255

Glu Lys Pro Leu Arg Arg Phe Phe Asp Glu Trp Pro Arg Glu Arg Gly
260 265 270

Asp Thr Arg Pro Ser Trp Ala Gly Ala Glu Asp Ala Thr Gln Leu Ser
275 280 285

Ile Ser Ile Pro Ala Ala Ser Pro Ser Ser Asp His Ala Ala Ser Ala
290 295 300

Ala Ala Arg Cys His Asn Asp Gly Ser Asp Arg Cys Ile Ser
305 310 315

<210> 91
<211> 1099
<212> DNA
<213> Zea mays

<400> 91

```
cgcatccgtt ctctatcgaa agggaggagg aggagcgcgc gggagtgggc tgggggccca     60
ccgatgctga gctcggcgtc ctcggccggg gcggccatgg ggatgggcgg cgggtaccaa    120
caccagccgc tgccactgcc gcagcgcggg gcggcggccg cggtcttcac cgccgcgcag    180
tgggcggagc tggagcagca ggcgctcatc tacaagtacc tcatggccgg cgtccccgtc    240
ccgcccgatc tcctccgccc cgccccccac gccgccgcct tctccttcgc cagccccgcc    300
gcgtcgccct tctaccatca ccaccaccac cacccgtccc tgagttacta cgcctactac    360
gggaagaagc tggacccgga gccgtggcgg tgccgccgca ccgacggcaa gaagtggcgg    420
tgctccaagg aggcgcaccc cgactccaag tactgcgagc gccacatgca ccgtggccgc    480
aaccgttcaa gaaagcctgt ggaatccaag accgcctcct cgccgcccca gctgtccacc    540
gtcgtcacca ccaccaccac ccgggaggcc gccgccgcga cgcccctcga gtccctcgcg    600
ggggcggggg gtaaggctca cggcctgtcc ctcggcggcg gggctggctc gtcgcacctc    660
agcgtcgacg cttcgaacac tcactttcgc tatggcagca agtaccctct tggagctaaa    720
tccgatgctg gcgagctgag cttcttctca ggagcaccag ggaactccag gggcttcacc    780
attgattctc cagcagataa ctcttggcac tccctgccat ccaacgtgcc cccgtttaca    840
ctgtccaagg gcagagattc tggcctcctg cctggagcgc caccagtcgt cgttcagcag    900
cagcggggcc ggcgctggtg ggttgctggg gagcgtgaag caggagaacc agccgctgag    960
gcccttcttc gacgagtggc ctgggacgcg ggactcgtgg tcggagatgg acgacgcgag   1020
gtccagtagg acctccttct cgacgaccca gctctccatc tccattccga tgcccagatg   1080

tgattgagaa cgaagctcg                                                1099
```

<210> 92
<211> 321
<212> PRT
<213> Zea mays

<400> 92

Met Leu Ser Ser Ala Ser Ser Ala Gly Ala Ala Met Gly Met Gly Gly
1               5                   10                  15

Gly Tyr Gln His Gln Pro Leu Pro Leu Pro Gln Arg Gly Ala Ala Ala
            20                  25                  30

Ala Val Phe Thr Ala Ala Gln Trp Ala Glu Leu Glu Gln Gln Ala Leu
        35                  40                  45

Ile Tyr Lys Tyr Leu Met Ala Gly Val Pro Val Pro Pro Asp Leu Leu
    50                  55                  60

Arg Pro Ala Pro His Ala Ala Ala Phe Ser Phe Ala Ser Pro Ala Ala
65                  70                  75                  80

Ser Pro Phe Tyr His His His His His His Pro Ser Leu Ser Tyr Tyr
                85                  90                  95

Ala Tyr Tyr Gly Lys Lys Leu Asp Pro Glu Pro Trp Arg Cys Arg Arg
            100                 105                 110

Thr Asp Gly Lys Lys Trp Arg Cys Ser Lys Glu Ala His Pro Asp Ser
        115                 120                 125

Lys Tyr Cys Glu Arg His Met His Arg Gly Arg Asn Arg Ser Arg Lys
    130                 135                 140

Pro Val Glu Ser Lys Thr Ala Ser Ser Pro Pro Gln Leu Ser Thr Val
145                 150                 155                 160

Val Thr Thr Thr Thr Thr Arg Glu Ala Ala Ala Ala Thr Pro Leu Glu
                165                 170                 175

Ser Leu Ala Gly Ala Gly Gly Lys Ala His Gly Leu Ser Leu Gly Gly
            180                 185                 190

Gly Ala Gly Ser Ser His Leu Ser Val Asp Ala Ser Asn Thr His Phe
        195                 200                 205

Arg Tyr Gly Ser Lys Tyr Pro Leu Gly Ala Lys Ser Asp Ala Gly Glu
    210                 215                 220

Leu Ser Phe Phe Ser Gly Ala Pro Gly Asn Ser Arg Gly Phe Thr Ile
225                 230                 235                 240

Asp Ser Pro Ala Asp Asn Ser Trp His Ser Leu Pro Ser Asn Val Pro
245 250 255

Pro Phe Thr Leu Ser Lys Gly Arg Asp Ser Gly Leu Leu Pro Gly Ala
260 265 270

Pro Pro Val Val Val Gln Gln Gln Arg Gly Arg Arg Trp Trp Val Ala
275 280 285

Gly Glu Arg Glu Ala Gly Glu Pro Ala Ala Glu Ala Leu Leu Arg Arg
290 295 300

Val Ala Trp Asp Ala Gly Leu Val Val Gly Asp Gly Arg Arg Glu Val
305 310 315 320

Gln

<210> 93
<211> 1328
<212> DNA
<213> zea mays

<400> 93

cctcccgtca gcctcttctt ctccccctga tgagcgctga gttctgtgct gccgccgctg 60
gtgctgtggc catggagctc ggagtcgggg atgtgatggg gctgcagcaa ggcatcgccg 120
ccgccaccgg gccatcgtcc ggagacagcg acctgggtct tctcaagcga gcaggcctcg 180
cccaggcagc cacctcctac ccctcccctt tcctcgacca acagaagatg ctcaggttct 240
ccaaggcggc ggcggctcac acgtcgccct caggcctaga tttcggagga ggcccaagcg 300
agcaggcttt cctgctgtcc aggaccaagc ggccgttcac cccgtcgcag tggatggagc 360
tggagcacca ggctctcata tacaagtatc tcaatgccaa ggcccccata ccttccagcc 420
tgctcgtttc catcagcaag agcttcaggt catccaacag agtgagctgg aggcctcttt 480
accaaggcta cgcaaacgca gactccgacc cagaacctgg gaggtgccgg cggacagacg 540
gaaagaagtg gcggtgctct aaggaggcga tgcctgatca caagtactgc gagcgccaca 600
tcaataggaa ccgccaccgt tcaagaaagc ctgtggaaaa ccaacctaga aagaccagca 660
aggaggtgcc taccgctgct gctggctcgt tgccgtgtgc cgggccacaa ggtagcttga 720
agaaggcaaa agttaatgac tccaagccag gcactggcag ctattggaca gatagcttaa 780
acaggacaat gctgagcagg gagaaggcaa acaaaccgac ggaagacgag tctttgctgc 840
ttagttctac gaagaacagc cagcccacct tgtccctgct cactcaactg aagcagcaga 900
acaaaccaga taagttaggt cccacaccgg aaaatgagcc gaactcggac acaatgttga 960
aagcctgggg tggcagccac cacaagaaca tttcctccac acagcgccat gacgctgaat 1020
ccctccaatc agtcctccaa aatttcagcc tagcccagaa tgacaggttg gagtcagaaa 1080
agaacagata ttctgattcc gtgctagtct catcggcttt ctattctgca gacggtccac 1140

aaactacctg ccttacacct aacatgacac aagtgcagca ggactgcata tcaagctcct 1200
gggagatgcc tcaaggtgga cctctaggcg agatcttaac gaactccaag attagtgagg 1260
actcaagcaa gtgtggatct aggtcatatg gttggctatt gaatcttgac catgcaccat 1320
gattcctc 1328

<210> 94
<211> 430
<212> PRT
<213> zea mays

<400> 94

Met Ser Ala Glu Phe Cys Ala Ala Ala Ala Gly Ala Val Ala Met Glu
1 5 10 15

Leu Gly Val Gly Asp Val Met Gly Leu Gln Gln Gly Ile Ala Ala Ala
20 25 30

Thr Gly Pro Ser Ser Gly Asp Ser Asp Leu Gly Leu Leu Lys Arg Ala
35 40 45

Gly Leu Ala Gln Ala Ala Thr Ser Tyr Pro Ser Pro Phe Leu Asp Gln
50 55 60

Gln Lys Met Leu Arg Phe Ser Lys Ala Ala Ala Ala His Thr Ser Pro
65 70 75 80

Ser Gly Leu Asp Phe Gly Gly Gly Pro Ser Glu Gln Ala Phe Leu Leu
85 90 95

Ser Arg Thr Lys Arg Pro Phe Thr Pro Ser Gln Trp Met Glu Leu Glu
100 105 110

His Gln Ala Leu Ile Tyr Lys Tyr Leu Asn Ala Lys Ala Pro Ile Pro
115 120 125

Ser Ser Leu Leu Val Ser Ile Ser Lys Ser Phe Arg Ser Ser Asn Arg
130 135 140

Val Ser Trp Arg Pro Leu Tyr Gln Gly Tyr Ala Asn Ala Asp Ser Asp
145 150 155 160

Pro Glu Pro Gly Arg Cys Arg Arg Thr Asp Gly Lys Lys Trp Arg Cys
165 170 175

Ser Lys Glu Ala Met Pro Asp His Lys Tyr Cys Glu Arg His Ile Asn
180 185 190

Arg Asn Arg His Arg Ser Arg Lys Pro Val Glu Asn Gln Pro Arg Lys
195 200 205

Thr Ser Lys Glu Val Pro Thr Ala Ala Ala Gly Ser Leu Pro Cys Ala
210 215 220

Gly Pro Gln Gly Ser Leu Lys Lys Ala Lys Val Asn Asp Ser Lys Pro
225 230 235 240

Gly Thr Gly Ser Tyr Trp Thr Asp Ser Leu Asn Arg Thr Met Leu Ser
245 250 255

Arg Glu Lys Ala Asn Lys Pro Thr Glu Asp Glu Ser Leu Leu Leu Ser
260 265 270

Ser Thr Lys Asn Ser Gln Pro Thr Leu Ser Leu Leu Thr Gln Leu Lys
275 280 285

Gln Gln Asn Lys Pro Asp Lys Leu Gly Pro Thr Pro Glu Asn Glu Pro
290 295 300

Asn Ser Asp Thr Met Leu Lys Ala Trp Gly Gly Ser His His Lys Asn
305 310 315 320

Ile Ser Ser Thr Gln Arg His Asp Ala Glu Ser Leu Gln Ser Val Leu
325 330 335

Gln Asn Phe Ser Leu Ala Gln Asn Asp Arg Leu Glu Ser Glu Lys Asn
340 345 350

Arg Tyr Ser Asp Ser Val Leu Val Ser Ser Ala Phe Tyr Ser Ala Asp
355 360 365

Gly Pro Gln Thr Thr Cys Leu Thr Pro Asn Met Thr Gln Val Gln Gln
370 375 380

Asp Cys Ile Ser Ser Ser Trp Glu Met Pro Gln Gly Gly Pro Leu Gly
385 390 395 400

Glu Ile Leu Thr Asn Ser Lys Ile Ser Glu Asp Ser Ser Lys Cys Gly
405 410 415

Ser Arg Ser Tyr Gly Trp Leu Leu Asn Leu Asp His Ala Pro
420 425 430

<210> 95
<211> 1440
<212> DNA
<213> Zea mays

<400> 95

gccaccaaga gccctccaac acacacctga cctccccttc ccccctctct ccgccgcccg 60
ttccccgcgc ctccgcccgt acgtcccgtt cccggtcggc cggccggtcc aaagggaggg 120
gaggaggagg ggcgcgggag tcggggcccg caccgatgct gagctcggca tcctcggccg 180
cgggggcggc catggggatg ggcggcggcg ggtacgcgca ccagccccg ccacagcgcg 240

cggtcttcac cgccgcgcag tgggcggagc tggagcagca ggcgctcatc tacaagtacc 300
tcatggccgg cgtccccgtc ccgcccgacc tcctcctccc cgtccgcccc ggccccgccg 360
ccgccttctc cttcgccggc cccgccgccg cgtcgccctt ctaccaccaa caccacccgt 420
ccctgagcta ctacgcctac tacggcaaga agctggaccc ggagccgtgg cggtgccgcc 480
gcaccgacgg caagaagtgg cggtgctcca aggaggcgca ccccgactcc aagtactgcg 540
agcgccacat gcaccgtggc cgcaaccgtt caagaaagcc tgtggaatcc aagaccgcct 600
cgtcgtcgtc gcccgcgcac ccgtcgccgc cccagctgtc caccgtcacc accaccgcgc 660
ctctcgagcc ccttgcagcg gcggggggca aggtccacgg cctgtccctc ggcggcggcg 720
ctgctggctc gtcgcacctc ggcgtcgatg cttcgaatgc tcactatcgt tatggtagca 780
acaggtaccc tctcggagct aaaccggacg gcggcgagtt gagcttcttc tcaggagcgt 840
catcggggaa caactcgagg ggtggcttca ccatcgactc tccatcagat aacaactcgt 900
ggcactccgc cctggcgtcc agcgtgcccc cgttcacgct gtcgacgaag agcggggact 960
ccggcctcct gcccggcgcc tacgcctcct actcccagtc ccactcccac atggagccgc 1020
cgcgggagct cgggcaggtc accatcgcct cgctggcgca ggagcaggag cgccagcagc 1080
cgttcagtgg tgggatgctc gggaacgtga agcaggagaa ccagaaccag ccgctgcggc 1140
ccttcttcga cgagtggccc gggacgcggg cggactcgtg gccgccggag atggacggcg 1200
cgccgcgggc cggcaggacc tccttctcct cctccaccac ccagctctcc atctccatcc 1260
cgatgcccag atgtgagctg catctcagaa accagaactc ttaattctgt tcgctgcccg 1320
aatcatgctt gaccgaaact tgttttctgc aggcgactga cgaggaaccg tcgatcgggc 1380
ggccactaga cggtggacgc tcacgctcac tagtgcgctg tcgcctggag tggagatcga 1440

<210> 96
<211> 382
<212> PRT
<213> Zea mays

<400> 96

Met Leu Ser Ser Ala Ser Ser Ala Ala Gly Ala Ala Met Gly Met Gly
1               5                   10                  15

Gly Gly Gly Tyr Ala His Gln Pro Pro Pro Gln Arg Ala Val Phe Thr
            20                  25                  30

Ala Ala Gln Trp Ala Glu Leu Glu Gln Gln Ala Leu Ile Tyr Lys Tyr
        35                  40                  45

Leu Met Ala Gly Val Pro Val Pro Pro Asp Leu Leu Leu Pro Val Arg
    50                  55                  60

Pro Gly Pro Ala Ala Ala Phe Ser Phe Ala Gly Pro Ala Ala Ala Ser
65                  70                  75                  80

Pro Phe Tyr His Gln His His Pro Ser Leu Ser Tyr Tyr Ala Tyr Tyr
85 90 95

Gly Lys Lys Leu Asp Pro Glu Pro Trp Arg Cys Arg Arg Thr Asp Gly
100 105 110

Lys Lys Trp Arg Cys Ser Lys Glu Ala His Pro Asp Ser Lys Tyr Cys
115 120 125

Glu Arg His Met His Arg Gly Arg Asn Arg Ser Arg Lys Pro Val Glu
130 135 140

Ser Lys Thr Ala Ser Ser Ser Ser Pro Ala His Pro Ser Pro Pro Gln
145 150 155 160

Leu Ser Thr Val Thr Thr Thr Ala Pro Leu Glu Pro Leu Ala Ala Ala
165 170 175

Gly Gly Lys Val His Gly Leu Ser Leu Gly Gly Gly Ala Ala Gly Ser
180 185 190

Ser His Leu Gly Val Asp Ala Ser Asn Ala His Tyr Arg Tyr Gly Ser
195 200 205

Asn Arg Tyr Pro Leu Gly Ala Lys Pro Asp Gly Gly Glu Leu Ser Phe
210 215 220

Phe Ser Gly Ala Ser Ser Gly Asn Asn Ser Arg Gly Gly Phe Thr Ile
225 230 235 240

Asp Ser Pro Ser Asp Asn Asn Ser Trp His Ser Ala Leu Ala Ser Ser
245 250 255

Val Pro Pro Phe Thr Leu Ser Thr Lys Ser Gly Asp Ser Gly Leu Leu
260 265 270

Pro Gly Ala Tyr Ala Ser Tyr Ser Gln Ser His Ser His Met Glu Pro
275 280 285

Pro Arg Glu Leu Gly Gln Val Thr Ile Ala Ser Leu Ala Gln Glu Gln
290 295 300

Glu Arg Gln Gln Pro Phe Ser Gly Gly Met Leu Gly Asn Val Lys Gln
305 310 315 320

Glu Asn Gln Asn Gln Pro Leu Arg Pro Phe Phe Asp Glu Trp Pro Gly
325 330 335

Thr Arg Ala Asp Ser Trp Pro Pro Glu Met Asp Gly Ala Pro Arg Ala
340 345 350

Gly Arg Thr Ser Phe Ser Ser Ser Thr Thr Gln Leu Ser Ile Ser Ile

355 360 365

Pro Met Pro Arg Cys Glu Leu His Leu Arg Asn Gln Asn Ser
370 375 380

<210> 97
<211> 1388
<212> DNA
<213> zea mays

<400> 97

```
gacaggttga gatggcgatg ccgtatgcct ctctttcccc ggcaggcgcc gccgaccacc     60
gctcctccac agccacggcg tccctcgtcc ccttctgccg ctccaccccg ctctccgcgg    120
gcggcgggct gggggaggag gacgcccagg cgagcgcgag gtggccggcc gcgaggccgg    180
tggtgccgtt cacgccggcg cagtaccagg agctggagca gcaggcgctc atatacaagt    240
acctggtggc cggcgtgccc gttccgccgg atctcgtggt tccaatccgc cgcggcctcg    300
actccctcgc tacccgcttc tacggccaac ccacactcgg gtacggaccg tacctgggga    360
ggaaactgga tccggagccc ggccggtgcc ggcgaacgga cggcaagaag tggcggtgct    420
ccaaggaagc cgccccggac tccaagtact gcgagcgcca catgcaccgc ggccgcaacc    480
gttcaagaaa gcctgtggaa acgcagctcg cgccccagtc ccaaccgccc gccgccgcgg    540
ccgtctccgc cgctccgccc ctggcagccg ccgccgccgc cgccaccaac ggcagcggct    600
tccagaacca ctctctctac ccggccatcg ccggcagcac tggtggtgga ggaggagttg    660
gcgggtccgg caatatctcc tccccgttct cctcgtcgat ggggggatcg tctcagctgc    720
acatggacag tgttgccagc tactcctacg cagctcttgg tggtggaact gcaaaggatc    780
tcaggtacaa cgcttacgga ataagatctc tggcggacga gcacaaccag ctgatcgcag    840
aagccatcga ctcgtcgata gagagccaga ggcgcctccc cagctcgtcg ttcccgctct    900
cgagctaccc acatctcggg gcgctgggcg acctgggcgg ccagaacagc acggtgagct    960
cgctgccgaa gatggagaag cagcagccgc cctcgtcctt cctagggaac gacaccgggg   1020
ccggcatggc catgggctcc gcctccgcga agcaggaggg ccagacgctg cggcacttct   1080
tcgacgagtg gcccaaggcg cgggactcct ggccgggcct ctccgacgag accgccagcc   1140
tcgcctcgtc ccccccggcg acccagctgt cgatgtccat acccatggcg tcctccgact   1200
tctccgtggc cagctcccag tcgcccaacg atgactaatg gtgcgtggat cgtcgcgttc   1260
tggccctttg tctatctccc ctccagtcct ccacccaccg cgcagtagta gctgcggaaa   1320
cagcccatgc tcctgtatat ttgtcggtca ttttccgtgt cagatctgtg taccaaacca   1380
agcggcgg                                                            1388
```

<210> 98
<211> 408
<212> PRT
<213> Zea mays

<400> 98

Met Ala Met Pro Tyr Ala Ser Leu Ser Pro Ala Gly Ala Ala Asp His
1               5                   10                  15

Arg Ser Ser Thr Ala Thr Ala Ser Leu Val Pro Phe Cys Arg Ser Thr
            20                  25                  30

Pro Leu Ser Ala Gly Gly Gly Leu Gly Glu Glu Asp Ala Gln Ala Ser
        35                  40                  45

Ala Arg Trp Pro Ala Ala Arg Pro Val Val Pro Phe Thr Pro Ala Gln
    50                  55                  60

Tyr Gln Glu Leu Glu Gln Gln Ala Leu Ile Tyr Lys Tyr Leu Val Ala
65                  70                  75                  80

Gly Val Pro Val Pro Pro Asp Leu Val Val Pro Ile Arg Arg Gly Leu
                85                  90                  95

Asp Ser Leu Ala Thr Arg Phe Tyr Gly Gln Pro Thr Leu Gly Tyr Gly
            100                 105                 110

Pro Tyr Leu Gly Arg Lys Leu Asp Pro Glu Pro Gly Arg Cys Arg Arg
        115                 120                 125

Thr Asp Gly Lys Lys Trp Arg Cys Ser Lys Glu Ala Ala Pro Asp Ser
    130                 135                 140

Lys Tyr Cys Glu Arg His Met His Arg Gly Arg Asn Arg Ser Arg Lys
145                 150                 155                 160

Pro Val Glu Thr Gln Leu Ala Pro Gln Ser Gln Pro Pro Ala Ala Ala
                165                 170                 175

Ala Val Ser Ala Ala Pro Pro Leu Ala Ala Ala Ala Ala Ala Ala Thr
            180                 185                 190

Asn Gly Ser Gly Phe Gln Asn His Ser Leu Tyr Pro Ala Ile Ala Gly
        195                 200                 205

Ser Thr Gly Gly Gly Gly Gly Val Gly Gly Ser Gly Asn Ile Ser Ser
    210                 215                 220

Pro Phe Ser Ser Ser Met Gly Gly Ser Ser Gln Leu His Met Asp Ser
225                 230                 235                 240

Val Ala Ser Tyr Ser Tyr Ala Ala Leu Gly Gly Gly Thr Ala Lys Asp
                245                 250                 255

Leu Arg Tyr Asn Ala Tyr Gly Ile Arg Ser Leu Ala Asp Glu His Asn
            260                 265                 270

Gln Leu Ile Ala Glu Ala Ile Asp Ser Ser Ile Glu Ser Gln Arg Arg

275 280 285

Leu Pro Ser Ser Ser Phe Pro Leu Ser Ser Tyr Pro His Leu Gly Ala
290 295 300

Leu Gly Asp Leu Gly Gly Gln Asn Ser Thr Val Ser Ser Leu Pro Lys
305 310 315 320

Met Glu Lys Gln Gln Pro Pro Ser Ser Phe Leu Gly Asn Asp Thr Gly
325 330 335

Ala Gly Met Ala Met Gly Ser Ala Ser Ala Lys Gln Glu Gly Gln Thr
340 345 350

Leu Arg His Phe Phe Asp Glu Trp Pro Lys Ala Arg Asp Ser Trp Pro
355 360 365

Gly Leu Ser Asp Glu Thr Ala Ser Leu Ala Ser Ser Pro Pro Ala Thr
370 375 380

Gln Leu Ser Met Ser Ile Pro Met Ala Ser Ser Asp Phe Ser Val Ala
385 390 395 400

Ser Ser Gln Ser Pro Asn Asp Asp
405

<210> 99
<211> 1506
<212> DNA
<213> zea mays

<400> 99

ccatctggcc atctcccctt cccctgctcc cccgaagcag caagccagcc tgcccacccg 60
cagccatcac ctccgccgct ctccaccatg aatcccatcc accagcacga catcgtaccc 120
aatccttcgt gactgttgcc tccgcgcatc tccgggagca atggaaggag gccgagatgt 180
gttcttaggt gcggcggcaa gggcgccgcc gccgccgccg tcttgcccgt ttcacggatc 240
cgctaccgcc acccgctccg gtggagcgca gatgctcagc ttctcctcca atggcgtagc 300
agggttgggt ctgtgctcag gtgccagcaa gatgcagggt gtgttgtcga gggtgaggag 360
gcccttcact ccgacgcagt ggatggagct ggagcaccag gccctgatct acaagcactt 420
cgctgtgaat gcccctgtgc cgtccagctt gctcctccct atcaaaagaa gcctcaatcc 480
atggagcagc cttggctcca gctcattggg atgggcacca tttcgttccg gctctgctga 540
tgcagaacca ggaagatgcc gccgcacaga tggcaagaag tggcggtgct ctagagatgc 600
tgtcggggac caaaaatact gtgagcgata cataaaacgt ggttgccacc gttcaagaaa 660
gcatgtggaa ggccgaaagg caacaccgac cactgcagat ccaaccatgg ctgtttctgg 720
tggttcattg ttgcacagcc atgctgttgc ttggcagcag cagggcaaaa gctcagctgc 780
taatgtgact gatccattct cactagggtc caacaggaat ttgctggata agcagaatct 840

aggtgaccag ttctctgtat ccacttccat ggactccttt gacttctcat catcacattc 900
ttccccaaac caagccaaag ttgcattttc accggtggcc atgcagcacg aacatgatca 960
gctgtatctt gtgcatggag ccggcagctc agcagaaaac gttaacaagt ctcaggatgg 1020
tcagctgcta gtctcgaggg aaacaattga cgacggacct ctgggcgagg tgttcaaggg 1080
caagagttgc cagtcagcat ccgcagacat cttaactgac cattggactt cgactcgtga 1140
cttgcgtcct ccaaccggag tcctacaaat gtctagcagc aacacagtgc cagcagagaa 1200
tcacacgagt aacagtagct atctcatggc gaggatggcg aattctcaga ccgtcccaac 1260
actccactga gtgttcatca ggctggtctt tgttgggacc acaaaataac tgaagccatg 1320
ttgatgtcct gagtttgctg atacagtgat actaggtttt cagtcgagtc ttgtaactcc 1380
tgttttagag ttgttatatg ttcacgtcat gttgcctttc attttcggtt tcattcagat 1440
gggtgtacta ataatttctt tccttcttac ctgtgaagga tttgagttcc aatctgagac 1500
gtgggt 1506

<210> 100
<211> 369
<212> PRT
<213> Zea mays

<400> 100

Met Glu Gly Gly Arg Asp Val Phe Leu Gly Ala Ala Ala Arg Ala Pro
1 5 10 15

Pro Pro Pro Pro Ser Cys Pro Phe His Gly Ser Ala Thr Ala Thr Arg
20 25 30

Ser Gly Gly Ala Gln Met Leu Ser Phe Ser Ser Asn Gly Val Ala Gly
35 40 45

Leu Gly Leu Cys Ser Gly Ala Ser Lys Met Gln Gly Val Leu Ser Arg
50 55 60

Val Arg Arg Pro Phe Thr Pro Thr Gln Trp Met Glu Leu Glu His Gln
65 70 75 80

Ala Leu Ile Tyr Lys His Phe Ala Val Asn Ala Pro Val Pro Ser Ser
85 90 95

Leu Leu Leu Pro Ile Lys Arg Ser Leu Asn Pro Trp Ser Ser Leu Gly
100 105 110

Ser Ser Ser Leu Gly Trp Ala Pro Phe Arg Ser Gly Ser Ala Asp Ala
115 120 125

Glu Pro Gly Arg Cys Arg Arg Thr Asp Gly Lys Lys Trp Arg Cys Ser
130 135 140

Arg Asp Ala Val Gly Asp Gln Lys Tyr Cys Glu Arg Tyr Ile Lys Arg

145 150 155 160

Gly Cys His Arg Ser Arg Lys His Val Glu Gly Arg Lys Ala Thr Pro
165 170 175

Thr Thr Ala Asp Pro Thr Met Ala Val Ser Gly Gly Ser Leu Leu His
180 185 190

Ser His Ala Val Ala Trp Gln Gln Gln Gly Lys Ser Ser Ala Ala Asn
195 200 205

Val Thr Asp Pro Phe Ser Leu Gly Ser Asn Arg Asn Leu Leu Asp Lys
210 215 220

Gln Asn Leu Gly Asp Gln Phe Ser Val Ser Thr Ser Met Asp Ser Phe
225 230 235 240

Asp Phe Ser Ser Ser His Ser Ser Pro Asn Gln Ala Lys Val Ala Phe
245 250 255

Ser Pro Val Ala Met Gln His Glu His Asp Gln Leu Tyr Leu Val His
260 265 270

Gly Ala Gly Ser Ser Ala Glu Asn Val Asn Lys Ser Gln Asp Gly Gln
275 280 285

Leu Leu Val Ser Arg Glu Thr Ile Asp Asp Gly Pro Leu Gly Glu Val
290 295 300

Phe Lys Gly Lys Ser Cys Gln Ser Ala Ser Ala Asp Ile Leu Thr Asp
305 310 315 320

His Trp Thr Ser Thr Arg Asp Leu Arg Pro Pro Thr Gly Val Leu Gln
325 330 335

Met Ser Ser Ser Asn Thr Val Pro Ala Glu Asn His Thr Ser Asn Ser
340 345 350

Ser Tyr Leu Met Ala Arg Met Ala Asn Ser Gln Thr Val Pro Thr Leu
355 360 365

His

<210> 101
<211> 1350
<212> DNA
<213> Zea mays

<400> 101

tagccgtgct ccgctcacct tctctcgcgc tacagtctca aggggtagct agccaagcta 60

ccaagctcgt caggaacgag agaaagaggc cggcggtgcg cggggatgat gatgatgagc 120

agcggccggg cgggcggcgg ggccaccgcg gggcggtacc cgttcacggc gtcgcagtgg 180
caggagctgg agcaccaggc gctcatctac aagtgcctgg cgtccggcaa gcccatccct 240
tcctacctca tgccgccgct ccgccgcatc ctcgactccg ccctcgccac gtcgccgtcc 300
ctcgcctacc cgccgcaacc ctcgctgggc tggggctgct tcgggatggg cttcacccgg 360
aaggccgacg aggacccgga gcccgggcgg tgccggcgca cggacggcaa gaagtggcgc 420
tgctccaagg aggcgtaccc ggactccaag tactgcgaga agcacatgca ccggggcaag 480
aaccgttcaa gaaagcctgt ggaaatgtcc ttggccacgc cggccccggc gccggccccc 540
gccgccgcca caaccgccac cgccacctca tccccggcgc cgtcctacca ccgcccggcc 600
cacgacgcca cgccgtctcc gtaccacgcg ctgtatggag gcggcggcgg cggcggcggt 660
agcccttact cggcgtcggc acgcccagga gcaaccggag gcggcggcgc gtaccaccac 720
gcgcagcatg tgagcccctt ccacctccac ctcgagacca cccacccgca cccgccgccg 780
ccctacaact actccgccga ccagagggac tacgcgtacg ggcacgcggc cgccaaggag 840
gtcggcgagc acgccttctt ctcggacggc gcgggcgagc gggtcgaccg ccaggccgcg 900
gcggggcagt ggcagttcag gcagctcggg gtggagacga agccgggccc cacgccgctg 960
ttccccgtcg ccgggtacgg gcacggcgcg gcgtcgccgt acggcgtcga gctgggcaag 1020
gacgacgacg agcaggagga gaggcgccgc cagcactgct tcgttcttgg agccgacctg 1080
cggctggagc ggccgtcgtc gggccatggc catggccatg gccatgacca tgacgacgcc 1140
gccgccgcgc agaagccgct ccggcccttc ttcgacgagt ggccgcacca gaagggggac 1200
aaggccgggt cgtggatggg gctcgacggc gagacgcagc tctccatgtc catccccatg 1260
gccgctaccg acctccccgt cacctcccgc ttccgtaacg acgagtgatg ccacatcaaa 1320
cctggcgctg gaaactcgga acgtatggtg 1350

<210> 102
<211> 400
<212> PRT
<213> Zea mays

<400> 102

Met Met Met Met Ser Ser Gly Arg Ala Gly Gly Gly Ala Thr Ala Gly
1 5 10 15

Arg Tyr Pro Phe Thr Ala Ser Gln Trp Gln Glu Leu Glu His Gln Ala
20 25 30

Leu Ile Tyr Lys Cys Leu Ala Ser Gly Lys Pro Ile Pro Ser Tyr Leu
35 40 45

Met Pro Pro Leu Arg Arg Ile Leu Asp Ser Ala Leu Ala Thr Ser Pro
50 55 60

Ser Leu Ala Tyr Pro Pro Gln Pro Ser Leu Gly Trp Gly Cys Phe Gly
65 70 75 80

Met Gly Phe Thr Arg Lys Ala Asp Glu Asp Pro Glu Pro Gly Arg Cys
85 90 95

Arg Arg Thr Asp Gly Lys Lys Trp Arg Cys Ser Lys Glu Ala Tyr Pro
100 105 110

Asp Ser Lys Tyr Cys Glu Lys His Met His Arg Gly Lys Asn Arg Ser
115 120 125

Arg Lys Pro Val Glu Met Ser Leu Ala Thr Pro Ala Pro Ala Pro Ala
130 135 140

Pro Ala Ala Ala Thr Thr Ala Thr Ala Thr Ser Ser Pro Ala Pro Ser
145 150 155 160

Tyr His Arg Pro Ala His Asp Ala Thr Pro Ser Pro Tyr His Ala Leu
165 170 175

Tyr Gly Gly Gly Gly Gly Gly Gly Gly Ser Pro Tyr Ser Ala Ser Ala
180 185 190

Arg Pro Gly Ala Thr Gly Gly Gly Gly Ala Tyr His His Ala Gln His
195 200 205

Val Ser Pro Phe His Leu His Leu Glu Thr Thr His Pro His Pro Pro
210 215 220

Pro Pro Tyr Asn Tyr Ser Ala Asp Gln Arg Asp Tyr Ala Tyr Gly His
225 230 235 240

Ala Ala Ala Lys Glu Val Gly Glu His Ala Phe Phe Ser Asp Gly Ala
245 250 255

Gly Glu Arg Val Asp Arg Gln Ala Ala Ala Gly Gln Trp Gln Phe Arg
260 265 270

Gln Leu Gly Val Glu Thr Lys Pro Gly Pro Thr Pro Leu Phe Pro Val
275 280 285

Ala Gly Tyr Gly His Gly Ala Ala Ser Pro Tyr Gly Val Glu Leu Gly
290 295 300

Lys Asp Asp Asp Glu Gln Glu Glu Arg Arg Arg Gln His Cys Phe Val
305 310 315 320

Leu Gly Ala Asp Leu Arg Leu Glu Arg Pro Ser Ser Gly His Gly His
325 330 335

Gly His Gly His Asp His Asp Asp Ala Ala Ala Ala Gln Lys Pro Leu
340 345 350

Arg Pro Phe Phe Asp Glu Trp Pro His Gln Lys Gly Asp Lys Ala Gly
355 360 365

Ser Trp Met Gly Leu Asp Gly Glu Thr Gln Leu Ser Met Ser Ile Pro
370 375 380

Met Ala Ala Thr Asp Leu Pro Val Thr Ser Arg Phe Arg Asn Asp Glu
385 390 395 400

<210> 103
<211> 1028
<212> DNA
<213> Zea mays

<400> 103

tcccttcacc gctgcctcga cccgcgccga aagatacctt tccccccctt cctctcgcgc 60
cgccgttttg gtgcgaccat ggcggcggag ggggaggcca agaacccgtc cggcggtggc 120
gaagggggta acccccagca ccagcaggca gtgcaggctg cgccggcgga gccgccaatg 180
gcacaggggg aagcggtgca ggaggctgga gcgcaggcga cgggacaaga gccggagggg 240
gagaaggcga atcgagatgg ggagggaagc gcgggggaga aggacgacgg cgcgtgcaga 300
gatctggttc tggttgagga tccggaggtg ctcgccgtcg aggacccgga ggaagctgca 360
gcaaccgcag cactccagga agaaatgaaa gcgctcgtgg catccgtccc tgacggtgct 420
ggggcagcat tcacagccat gcagcttcag gagctagagc agcagtcccg ggtttatcag 480
tacatggctg cccgagtacc tgtgcctact cacctcgtct tccccgtatg gaagagtgta 540
accggtgcat cctctgaagg cgcccagaag taccctactt tgttgggctt agcaacactc 600
tgcttggact tcgggaagaa ccctgaacca gaaccaggga ggtgccggcg aacggatggc 660
aaaaaatggc gatgttggag aaacactatt ccaaacgaga agtactgcga acgccgcatg 720
catcgcggtc gcaagcgtcc tgtacaggtc gtcgaggaag ccgagcctga ctctgcttca 780
ggctcaaaat ctgctcccgg caaggccacc gaaggcgcca agaaggttgg cgacaagagc 840
ccaggtagca agaagcttgc cgtggcggcg gcagctgcag ctgctgcgca gtctacgtaa 900
ttgatgcagc attttagtag tcgcaggaag agcatggcgg cgctggcaac tagcgccttc 960
ttttcattgc atgtgatctt tagctataac ctcatttagc acactcccag tggtgtccgt 1020
gggaggag 1028

<210> 104
<211> 273
<212> PRT
<213> Zea mays

<400> 104

```
Met Ala Ala Glu Gly Glu Ala Lys Asn Pro Ser Gly Gly Gly Glu Gly
1               5                   10                  15

Gly Asn Pro Gln His Gln Gln Ala Val Gln Ala Ala Pro Ala Glu Pro
            20                  25                  30

Pro Met Ala Gln Gly Glu Ala Val Gln Glu Ala Gly Ala Gln Ala Thr
        35                  40                  45

Gly Gln Glu Pro Glu Gly Glu Lys Ala Asn Arg Asp Gly Glu Gly Ser
    50                  55                  60

Ala Gly Glu Lys Asp Asp Gly Ala Cys Arg Asp Leu Val Leu Val Glu
65                  70                  75                  80

Asp Pro Glu Val Leu Ala Val Glu Asp Pro Glu Glu Ala Ala Ala Thr
                85                  90                  95

Ala Ala Leu Gln Glu Glu Met Lys Ala Leu Val Ala Ser Val Pro Asp
            100                 105                 110

Gly Ala Gly Ala Ala Phe Thr Ala Met Gln Leu Gln Glu Leu Glu Gln
        115                 120                 125

Gln Ser Arg Val Tyr Gln Tyr Met Ala Ala Arg Val Pro Val Pro Thr
    130                 135                 140

His Leu Val Phe Pro Val Trp Lys Ser Val Thr Gly Ala Ser Ser Glu
145                 150                 155                 160

Gly Ala Gln Lys Tyr Pro Thr Leu Leu Gly Leu Ala Thr Leu Cys Leu
                165                 170                 175

Asp Phe Gly Lys Asn Pro Glu Pro Glu Pro Gly Arg Cys Arg Arg Thr
            180                 185                 190

Asp Gly Lys Lys Trp Arg Cys Trp Arg Asn Thr Ile Pro Asn Glu Lys
        195                 200                 205

Tyr Cys Glu Arg Arg Met His Arg Gly Arg Lys Arg Pro Val Gln Val
    210                 215                 220

Val Glu Glu Ala Glu Pro Asp Ser Ala Ser Gly Ser Lys Ser Ala Pro
225                 230                 235                 240

Gly Lys Ala Thr Glu Gly Ala Lys Lys Val Gly Asp Lys Ser Pro Gly
                245                 250                 255

Ser Lys Lys Leu Ala Val Ala Ala Ala Ala Ala Ala Ala Ala Gln Ser
            260                 265                 270

Thr
```

<210> 105
<211> 1374
<212> DNA
<213> Zea mays

<400> 105

cagccaggta aggcaaaaga gagagggcgg aagcagcggc agagcggaga gggagagaga 60
agagcatata tgggcatggc gatgcccttt gcctccccgt ctccggcagc cgaccaccgc 120
ccctcctccc tcctcccctt ctgccgcgcc gcccctctct ccgcggcggg agaggacgcc 180
gcgcagcagc acgcgatgag cggcaggtgg gccgcgaggc cggcgctctt cacggcggcg 240
cagtacgagg agctggagca ccaggcgctc atatacaagt acctcgtcgc cggcgtgccc 300
gtcccgccgg acctcctcct ccccctgcgc cgaggcttcg tcttccacca gccacccgcc 360
cttgggtacg gcccctactt cggcaagaag gtggacccgg agcccgggcg gtgccggcgt 420
acggacggca agaagtggcg gtgctccaag gaggccgccc cggactccaa gtactgcgag 480
cgccacatgc accgcggccg caaccgttca agaaagcctg tggaagcgca gctcgcgccc 540
ccgccgcacg cccagccgcc gcagcagcag caggcccccg cgcccgctgc tggcttccag 600
aaccactcgc tgtacccgtc gatcctcaac ggcaacggcg gcggcgggtt aggtgctggt 660
gctggtggtg gcacgttcgg cctggggccc acctctcagc tgcacatgga cagtgccgct 720
gcctacgcga ctgctgccgg tggagggagc aaatatctca ggtactctgc atacggggtg 780
aaatctctgt cggacgagca cagcacgctc ttgtcgggcg gcatggatcc gtcgatgatg 840
gacaactcgt ggcgccttct gccatcccaa aacaacacat tccaagccac aagctaccct 900
gtgttcggca cgctgagtgg gctagacgag agcaccatcg cgtcgctgcc gaagacccag 960
agggagcccc tctctttctt cgggagcgac ttcgtgaccg ccgccaagca ggagaaccag 1020
acgctgcgcc ctttcttcga cgagtggccc aagtcgaggg actcgtggcc ggagctgggc 1080
gaggacggca gcctcggctt ctcggccacc cagctctcca tctccattcc catggcgacc 1140
tccgacttct ccaacaccag ctccagatcg ccgggtggaa taccgtcgag atgaacgagt 1200
accgtgcatg tggatcccag cgtcttaggg ttgacgactc ttcggtgctg gcctcatcgt 1260
atcatgctcc taaattttcg aacgatatat gccttatgta acgctatttc tctcattgtt 1320
acaacaccct ttacccgttt ggaattgtgt tgaagtggat ggtctgcgtt gctc 1374

<210> 106
<211> 374
<212> PRT
<213> Zea mays

<400> 106

Met Gly Met Ala Met Pro Phe Ala Ser Pro Ser Pro Ala Ala Asp His
1               5                   10                  15

Arg Pro Ser Ser Leu Leu Pro Phe Cys Arg Ala Ala Pro Leu Ser Ala
            20                  25                  30

Ala Gly Glu Asp Ala Ala Gln Gln His Ala Met Ser Gly Arg Trp Ala
        35                  40                  45

Ala Arg Pro Ala Leu Phe Thr Ala Ala Gln Tyr Glu Glu Leu Glu His

50 55 60

Gln Ala Leu Ile Tyr Lys Tyr Leu Val Ala Gly Val Pro Val Pro Pro
65 70 75 80

Asp Leu Leu Leu Pro Leu Arg Arg Gly Phe Val Phe His Gln Pro Pro
85 90 95

Ala Leu Gly Tyr Gly Pro Tyr Phe Gly Lys Lys Val Asp Pro Glu Pro
100 105 110

Gly Arg Cys Arg Arg Thr Asp Gly Lys Lys Trp Arg Cys Ser Lys Glu
115 120 125

Ala Ala Pro Asp Ser Lys Tyr Cys Glu Arg His Met His Arg Gly Arg
130 135 140

Asn Arg Ser Arg Lys Pro Val Glu Ala Gln Leu Ala Pro Pro Pro His
145 150 155 160

Ala Gln Pro Pro Gln Gln Gln Gln Ala Pro Ala Pro Ala Ala Gly Phe
165 170 175

Gln Asn His Ser Leu Tyr Pro Ser Ile Leu Asn Gly Asn Gly Gly Gly
180 185 190

Gly Leu Gly Ala Gly Ala Gly Gly Gly Thr Phe Gly Leu Gly Pro Thr
195 200 205

Ser Gln Leu His Met Asp Ser Ala Ala Ala Tyr Ala Thr Ala Ala Gly
210 215 220

Gly Gly Ser Lys Tyr Leu Arg Tyr Ser Ala Tyr Gly Val Lys Ser Leu
225 230 235 240

Ser Asp Glu His Ser Thr Leu Leu Ser Gly Gly Met Asp Pro Ser Met
245 250 255

Met Asp Asn Ser Trp Arg Leu Leu Pro Ser Gln Asn Asn Thr Phe Gln
260 265 270

Ala Thr Ser Tyr Pro Val Phe Gly Thr Leu Ser Gly Leu Asp Glu Ser
275 280 285

Thr Ile Ala Ser Leu Pro Lys Thr Gln Arg Glu Pro Leu Ser Phe Phe
290 295 300

Gly Ser Asp Phe Val Thr Ala Ala Lys Gln Glu Asn Gln Thr Leu Arg
305 310 315 320

Pro Phe Phe Asp Glu Trp Pro Lys Ser Arg Asp Ser Trp Pro Glu Leu
325 330 335

Gly Glu Asp Gly Ser Leu Gly Phe Ser Ala Thr Gln Leu Ser Ile Ser
340 345 350

Ile Pro Met Ala Thr Ser Asp Phe Ser Asn Thr Ser Ser Arg Ser Pro
355 360 365

Gly Gly Ile Pro Ser Arg
370

<210> 107
<211> 1172
<212> DNA
<213> Zea mays

<400> 107

gatatatggc gatgcccttt gcctccctgt ctccggcagc cgaccaccgc ccctcctccc 60
tcctccccta ctgccgcgcc gcccctctct ccgcggtggg agaggacgcc gccgcgcagg 120
cgcagcagca gcagcagcag cacgctatga gcggcaggtg ggcagcgagg ccgccggcgc 180
tcttcacagc ggcgcagtac gaggagctgg agcaccaggc gctcatatac aagtacctcg 240
tcgccggcgt gcccgtcccg ccggacctcc tcctccccct acgccgaggc ttcgtctacc 300
accaacccgc ccttgggtac gggccctact tcggcaagaa ggtggacccg gagcccgggc 360
ggtgccggcg tacggacggc aagaagtggc ggtgctccaa ggaggccgcc ccggactcca 420
agtactgcga gcgccacatg caccgcggcc gcaaccgttc aagaaagcct gtggaagcgc 480
agctcgtgcc cccgccgcac gcccagccgc agcagcaggc ccccgcgccc accgctggct 540
tccagagcca ccccatgtac ccatccatcc tcgccggcaa cggcggcggc ggcggcgggg 600
taggtggcgg tgctggcggt ggcacgttcg gcctgggccc cacctctcag ctgcgcatgg 660
acagtgccgc tgcttacgcg actgctgctg atggagggag caaagatctc aggtactctg 720
cctacggggt gaagtcactg tcggacgagc acagccagct cttgcccggc ggcggcggcg 780
gcatggacgc gtcaatggac aactcgtggc gcctgttgcc gtcccaaacc gccgccacgt 840
tccaagccac aagctaccct ctgttcggcg cgctgagcgg tctggacgag agcaccatcg 900
cctcgctgcc caagacgcag agggagcccc tctccttctt cgggagcgac ttcgtgaccc 960
cgaagcagga gaaccagacg ctgcgcccct tcttcgacga gtggcccaag tcgagggact 1020
cgtggccgga gctgaacgag gacaacagcc tcggctcctc ggccacccag ctctccacct 1080
ccatccccat ggcgccctcc gacttcaaca ccagctccag atcgccgaat ggaataccgt 1140
caagatgaac ctgagtaacc atgcggaccc ca 1172

<210> 108
<211> 380
<212> PRT
<213> Zea mays

<400> 108

Met Ala Met Pro Phe Ala Ser Leu Ser Pro Ala Ala Asp His Arg Pro

1 5 10 15

Ser Ser Leu Leu Pro Tyr Cys Arg Ala Ala Pro Leu Ser Ala Val Gly
20 25 30

Glu Asp Ala Ala Ala Gln Ala Gln Gln Gln Gln Gln Gln His Ala Met
35 40 45

Ser Gly Arg Trp Ala Ala Arg Pro Pro Ala Leu Phe Thr Ala Ala Gln
50 55 60

Tyr Glu Glu Leu Glu His Gln Ala Leu Ile Tyr Lys Tyr Leu Val Ala
65 70 75 80

Gly Val Pro Val Pro Pro Asp Leu Leu Leu Pro Leu Arg Arg Gly Phe
85 90 95

Val Tyr His Gln Pro Ala Leu Gly Tyr Gly Pro Tyr Phe Gly Lys Lys
100 105 110

Val Asp Pro Glu Pro Gly Arg Cys Arg Arg Thr Asp Gly Lys Lys Trp
115 120 125

Arg Cys Ser Lys Glu Ala Ala Pro Asp Ser Lys Tyr Cys Glu Arg His
130 135 140

Met His Arg Gly Arg Asn Arg Ser Arg Lys Pro Val Glu Ala Gln Leu
145 150 155 160

Val Pro Pro Pro His Ala Gln Pro Gln Gln Gln Ala Pro Ala Pro Thr
165 170 175

Ala Gly Phe Gln Ser His Pro Met Tyr Pro Ser Ile Leu Ala Gly Asn
180 185 190

Gly Gly Gly Gly Gly Gly Val Gly Gly Gly Ala Gly Gly Gly Thr Phe
195 200 205

Gly Leu Gly Pro Thr Ser Gln Leu Arg Met Asp Ser Ala Ala Ala Tyr
210 215 220

Ala Thr Ala Ala Asp Gly Gly Ser Lys Asp Leu Arg Tyr Ser Ala Tyr
225 230 235 240

Gly Val Lys Ser Leu Ser Asp Glu His Ser Gln Leu Leu Pro Gly Gly
245 250 255

Gly Gly Gly Met Asp Ala Ser Met Asp Asn Ser Trp Arg Leu Leu Pro
260 265 270

Ser Gln Thr Ala Ala Thr Phe Gln Ala Thr Ser Tyr Pro Leu Phe Gly
275 280 285

Ala Leu Ser Gly Leu Asp Glu Ser Thr Ile Ala Ser Leu Pro Lys Thr
290 295 300

Gln Arg Glu Pro Leu Ser Phe Phe Gly Ser Asp Phe Val Thr Pro Lys
305 310 315 320

Gln Glu Asn Gln Thr Leu Arg Pro Phe Phe Asp Glu Trp Pro Lys Ser
325 330 335

Arg Asp Ser Trp Pro Glu Leu Asn Glu Asp Asn Ser Leu Gly Ser Ser
340 345 350

Ala Thr Gln Leu Ser Thr Ser Ile Pro Met Ala Pro Ser Asp Phe Asn
355 360 365

Thr Ser Ser Arg Ser Pro Asn Gly Ile Pro Ser Arg
370 375 380

<210> 109
<211> 1154
<212> DNA
<213> Zea mays

<400> 109

agcgtgcatt gttgagcgag tgcggccaag caacgcgggc tcgaggagat gatgctgagc 60
gggcacggcg gcgggaggcg cctgttcacg gcgtcgcagt ggcaggagct cgagcaccag 120
gcgctcatct tcaagtacat ggcctcgggc gcgcccgtgc cgcacgacct cgtcctaccg 180
ctccgcctcg ccaccggcgt cgacaccgcg ccctccctcg ccttcccgcc ccagccttcg 240
ccgtcgctgg cgtactgggg ctgctacggc gcgggggcgc cgttcgtcgg ccgcaaggcg 300
gcggaggaca cggagccggg gcggtgccgg cggacggacg gcaagaagtg gcggtgctcc 360
agggaggccc acggcgactc caagtactgc gagaagcaca ttcaccgcgg gaagagccgt 420
tcaagaaagc ctgtggaagt gacctcctcc cccgccgccg gcgccgctgc ggcgtaccga 480
ccgtccgcga tctccaccat ctcgccgccc cgcgcggccg acgcgccgcc gccgagcctc 540
gcctacccgc agcagcatct cctccacggc gcctcctcct ccgcagcagc ccgcgccccc 600
gctggcgctc tccagctcca cctcgacgcg agcctgcacg cggcggcggc gtcgccatcg 660
ccgccgccgt cctaccacag gtacgcccac tacacaccgc cagcgtcgtc gctcttcccg 720
ggcggcggct acggctacga ctacgactac gggcagtcca aggagctcag gcgacggcac 780
ttccacgcgc tcggggccga cctgagcctc gacaagccgc tgcccgagcc cgacaccggc 840
tccgacgaga agcagcccct gcggcgtttc ttcgacgagt ggccgcggga gagcggcgac 900
atggcggcgg acgacgcgac gcagctttcc atctccatcc ccgcggcttc gccctccgac 960
ctcgctgcta cctccgcctc cgccgccgcc gcgcgattcc acaacgggga gtgatcggtc 1020
catctcctag ctgcagccct gcaacagcgt ggattgaccg ctgcatttcc tggctgcaat 1080
gcaagcctgc aacagcgagc agtaagccag tgacgtggat gcatctcgta gcggcaaacc 1140

ctgcttctgc ctct 1154

<210> 110
<211> 321
<212> PRT
<213> Zea mays

<400> 110

Met Met Leu Ser Gly His Gly Gly Gly Arg Arg Leu Phe Thr Ala Ser
1 5 10 15

Gln Trp Gln Glu Leu Glu His Gln Ala Leu Ile Phe Lys Tyr Met Ala
20 25 30

Ser Gly Ala Pro Val Pro His Asp Leu Val Leu Pro Leu Arg Leu Ala
35 40 45

Thr Gly Val Asp Thr Ala Pro Ser Leu Ala Phe Pro Pro Gln Pro Ser
50 55 60

Pro Ser Leu Ala Tyr Trp Gly Cys Tyr Gly Ala Gly Ala Pro Phe Val
65 70 75 80

Gly Arg Lys Ala Ala Glu Asp Thr Glu Pro Gly Arg Cys Arg Arg Thr
85 90 95

Asp Gly Lys Lys Trp Arg Cys Ser Arg Glu Ala His Gly Asp Ser Lys
100 105 110

Tyr Cys Glu Lys His Ile His Arg Gly Lys Ser Arg Ser Arg Lys Pro
115 120 125

Val Glu Val Thr Ser Ser Pro Ala Ala Gly Ala Ala Ala Ala Tyr Arg
130 135 140

Pro Ser Ala Ile Ser Thr Ile Ser Pro Pro Arg Ala Ala Asp Ala Pro
145 150 155 160

Pro Pro Ser Leu Ala Tyr Pro Gln Gln His Leu Leu His Gly Ala Ser
165 170 175

Ser Ser Ala Ala Ala Arg Ala Pro Ala Gly Ala Leu Gln Leu His Leu
180 185 190

Asp Ala Ser Leu His Ala Ala Ala Ala Ser Pro Ser Pro Pro Pro Ser
195 200 205

Tyr His Arg Tyr Ala His Tyr Thr Pro Pro Ala Ser Ser Leu Phe Pro
210 215 220

Gly Gly Gly Tyr Gly Tyr Asp Tyr Asp Tyr Gly Gln Ser Lys Glu Leu
225 230 235 240

Arg Arg Arg His Phe His Ala Leu Gly Ala Asp Leu Ser Leu Asp Lys
245 250 255

Pro Leu Pro Glu Pro Asp Thr Gly Ser Asp Glu Lys Gln Pro Leu Arg
260 265 270

Arg Phe Phe Asp Glu Trp Pro Arg Glu Ser Gly Asp Met Ala Ala Asp
275 280 285

Asp Ala Thr Gln Leu Ser Ile Ser Ile Pro Ala Ala Ser Pro Ser Asp
290 295 300

Leu Ala Ala Thr Ser Ala Ser Ala Ala Ala Ala Arg Phe His Asn Gly
305 310 315 320

Glu

<210> 111
<211> 1516
<212> DNA
<213> Zea mays

<400> 111

ttcggcacga cccaacaatg cacaccaaca tccactccct cgtcaggctc ctctccccca 60
aatgagcgct gagttctgcg ctgctgcggg tgtcgtggcc atggagctcg ggtcggaga 120
tgcgctgggg ctgcagcaag gcatcgcaat caccgcgcca tcgcccaggg acagcgacct 180
gggtcttctc aagcgagcag gcctcaccca ggctgcggct gctgccccct acccctcccc 240
cttccttgac ggggagaaga tgctcaggtt ctccaaggcg gctcacacat cgcactcagg 300
cttggatttt ggaggcccag gtgagcaggc tttcctgctg tccaggacca agatgccatt 360
tactccctcg cagtggatgg agctggggca ccaggctctg atatacaagt acctcaatgc 420
aaaggccccc ataccttcca gcctgctcat ttcaatcagc aagagcttca gatcatccaa 480
tagagtgagc tggaggcctc tgtatcaagg ctacacaaat gcagactctg acccagaacc 540
tgggagatgc cgacgaacgg atggaaagaa gtggcggtgc tccaaggaag caatggctga 600
tcacaagtac tgtgagcggc acatcaacag aaaccgtcac cgttcaagaa agcctgtgga 660
aaatcaacct aagaagacca ccaaggaggt gcctgctgct gctggctcat taccatgtgc 720
tgggccacaa ggtagcttga agaaggcaaa agttaatgac tccaagccag gcactgtcag 780
ctattgggca gatagtttaa acaggacaat gttgagcaga gagaaagcaa acaaaccgac 840
ggaagatagc tctttgctgc ttacttctac gaacagccaa cccacctggt ccctgctctc 900
tcagctgaag cagcaaaaca aaccagataa gttaggcccc acactggaaa atgagtcaaa 960
cccagacaca atattgaaag cctggggtgg caaccagcct agccacaaga gcatttcctc 1020
tacagagcgc catgatgctg aatccctcca atcagtcctt caaaatctca gcctagccca 1080
gaatgagaag atggagtcag aaaaggacaa atattctgat tccgtgctag tttcgtcgac 1140

```
tttctattct gcaggcggtc caagagctac ctgccttaca cctaacatga cacaggtgaa    1200
gcaggattgc atatcaagct cttgggagat gcctcaaggt ggacctctag gcgaaatctt    1260
aacgaactcc aagaatagca aggacttaag caagtgcaaa ccaaggtcat atggttggtt    1320
gttgaatctt gaccatgcac catgattcct caatccatga agagcttgac atagatgtcc    1380
catcatgtag gcaaacaatg gtcagaaaaa ggttatgacc acattgcttg ccccatgcat    1440
gcttgctatc tacatttgta tttctgttgc gtagcattta gctagttgaa ttatcagttc    1500
ttctggatac ggctgt                                                    1516
```

<210> 112
<211> 427
<212> PRT
<213> Zea mays

<400> 112

```
Met Ser Ala Glu Phe Cys Ala Ala Ala Gly Val Val Ala Met Glu Leu
1               5                   10                  15

Gly Val Gly Asp Ala Leu Gly Leu Gln Gln Gly Ile Ala Ile Thr Ala
            20                  25                  30

Pro Ser Pro Arg Asp Ser Asp Leu Gly Leu Leu Lys Arg Ala Gly Leu
        35                  40                  45

Thr Gln Ala Ala Ala Ala Ala Pro Tyr Pro Ser Pro Phe Leu Asp Gly
    50                  55                  60

Glu Lys Met Leu Arg Phe Ser Lys Ala Ala His Thr Ser His Ser Gly
65                  70                  75                  80

Leu Asp Phe Gly Gly Pro Gly Glu Gln Ala Phe Leu Leu Ser Arg Thr
                85                  90                  95

Lys Met Pro Phe Thr Pro Ser Gln Trp Met Glu Leu Gly His Gln Ala
            100                 105                 110

Leu Ile Tyr Lys Tyr Leu Asn Ala Lys Ala Pro Ile Pro Ser Ser Leu
        115                 120                 125

Leu Ile Ser Ile Ser Lys Ser Phe Arg Ser Ser Asn Arg Val Ser Trp
    130                 135                 140

Arg Pro Leu Tyr Gln Gly Tyr Thr Asn Ala Asp Ser Asp Pro Glu Pro
145                 150                 155                 160

Gly Arg Cys Arg Arg Thr Asp Gly Lys Lys Trp Arg Cys Ser Lys Glu
                165                 170                 175

Ala Met Ala Asp His Lys Tyr Cys Glu Arg His Ile Asn Arg Asn Arg
            180                 185                 190
```

His Arg Ser Arg Lys Pro Val Glu Asn Gln Pro Lys Lys Thr Thr Lys
195 200 205

Glu Val Pro Ala Ala Ala Gly Ser Leu Pro Cys Ala Gly Pro Gln Gly
210 215 220

Ser Leu Lys Lys Ala Lys Val Asn Asp Ser Lys Pro Gly Thr Val Ser
225 230 235 240

Tyr Trp Ala Asp Ser Leu Asn Arg Thr Met Leu Ser Arg Glu Lys Ala
245 250 255

Asn Lys Pro Thr Glu Asp Ser Ser Leu Leu Leu Thr Ser Thr Asn Ser
260 265 270

Gln Pro Thr Trp Ser Leu Leu Ser Gln Leu Lys Gln Gln Asn Lys Pro
275 280 285

Asp Lys Leu Gly Pro Thr Leu Glu Asn Glu Ser Asn Pro Asp Thr Ile
290 295 300

Leu Lys Ala Trp Gly Gly Asn Gln Pro Ser His Lys Ser Ile Ser Ser
305 310 315 320

Thr Glu Arg His Asp Ala Glu Ser Leu Gln Ser Val Leu Gln Asn Leu
325 330 335

Ser Leu Ala Gln Asn Glu Lys Met Glu Ser Glu Lys Asp Lys Tyr Ser
340 345 350

Asp Ser Val Leu Val Ser Ser Thr Phe Tyr Ser Ala Gly Gly Pro Arg
355 360 365

Ala Thr Cys Leu Thr Pro Asn Met Thr Gln Val Lys Gln Asp Cys Ile
370 375 380

Ser Ser Ser Trp Glu Met Pro Gln Gly Gly Pro Leu Gly Glu Ile Leu
385 390 395 400

Thr Asn Ser Lys Asn Ser Lys Asp Leu Ser Lys Cys Lys Pro Arg Ser
405 410 415

Tyr Gly Trp Leu Leu Asn Leu Asp His Ala Pro
420 425

<210> 113
<211> 1139
<212> DNA
<213> Zea mays

<400> 113

gtaggtcgtt cgcaggtagg taaccgtaac ctagctagct cgtcgggatg atgatgatga 60

gcggtcgagc ggccaccgcg gggcggtacc cgttcacggc gtcgcagtgg caggagctgg 120
agcaccaggc gctcatctac aagtgcctgg cgtccggcaa gcccatcccg tcctacctca 180
tgccaccgct ccgccgcatc ctcgactccg ccctcgccac gtcgccgtcg ctcgccgcct 240
tccagccgca accctcgctg gggtgggggg gctgcttcgg gatgggcttc agcaggaagc 300
ccgccgacga ggacccggag cccgggcggt gccggcgcac ggacggcaag aagtggcgct 360
gctccaagga ggcgtacccg gactccaagt actgcgagaa gcacatgcac cggggcaaga 420
accgttcaag aaagcctgtg gaaatgtcct tggccacgcc ggcgccgccg gcctcctccg 480
ctgccaccac ctcgacgtcc ccggcgccgt cctaccaccg cccggccccc gccgcgcacg 540
acgccgtgcc gtaccacgcg ccctacggcg ccgcgtacca tcacacgcag acgcaggtga 600
tgagcccctt ccacctccac ctcgagacca cccacccgca cccgccgccg ccgccgccct 660
actactacgc ggaccagagg gactacgcct acggcaagga ggtcggcgag cgcgccttct 720
tctccgacgg cgcgggggag agggaccgcc agcagcaggc cgcggggcag tggcagttca 780
agcagctcgg gacgatggag gcgacgaagc cgtgccccac ccccacgccg ctgctccccg 840
ccgccgggta cggcgtcggt caggccaagg aagacgagga ggaggaaacg cggcggcagc 900
agcagcagca ctgcttcgtt cttggcgccg acctgcggct ggcggagcgg ccgtcggggg 960
cacatgacga cgccgcgcag aagccgctcc ggcatttctt cgacgagtgg ccgcacgaga 1020
aagggagcaa ggcggggtgg tggattgggg gactcgacgg cgagacgacg cagctctcca 1080
tgtccatccc gatggcggcc gctgccgacc tccccgtcac ctcccgctac cgtacgtga 1139

<210> 114
<211> 363
<212> PRT
<213> Zea mays

<400> 114

Met Met Met Met Ser Gly Arg Ala Ala Thr Ala Gly Arg Tyr Pro Phe
1 5 10 15

Thr Ala Ser Gln Trp Gln Glu Leu Glu His Gln Ala Leu Ile Tyr Lys
20 25 30

Cys Leu Ala Ser Gly Lys Pro Ile Pro Ser Tyr Leu Met Pro Pro Leu
35 40 45

Arg Arg Ile Leu Asp Ser Ala Leu Ala Thr Ser Pro Ser Leu Ala Ala
50 55 60

Phe Gln Pro Gln Pro Ser Leu Gly Trp Gly Gly Cys Phe Gly Met Gly
65 70 75 80

Phe Ser Arg Lys Pro Ala Asp Glu Asp Pro Glu Pro Gly Arg Cys Arg
85 90 95

Arg Thr Asp Gly Lys Lys Trp Arg Cys Ser Lys Glu Ala Tyr Pro Asp
100 105 110

Ser Lys Tyr Cys Glu Lys His Met His Arg Gly Lys Asn Arg Ser Arg
115 120 125

Lys Pro Val Glu Met Ser Leu Ala Thr Pro Ala Pro Pro Ala Ser Ser
130 135 140

Ala Ala Thr Thr Ser Thr Ser Pro Ala Pro Ser Tyr His Arg Pro Ala
145 150 155 160

Pro Ala Ala His Asp Ala Val Pro Tyr His Ala Pro Tyr Gly Ala Ala
165 170 175

Tyr His His Thr Gln Thr Gln Val Met Ser Pro Phe His Leu His Leu
180 185 190

Glu Thr Thr His Pro His Pro Pro Pro Pro Pro Pro Tyr Tyr Tyr Ala
195 200 205

Asp Gln Arg Asp Tyr Ala Tyr Gly Lys Glu Val Gly Glu Arg Ala Phe
210 215 220

Phe Ser Asp Gly Ala Gly Glu Arg Asp Arg Gln Gln Gln Ala Ala Gly
225 230 235 240

Gln Trp Gln Phe Lys Gln Leu Gly Thr Met Glu Ala Thr Lys Pro Cys
245 250 255

Pro Thr Pro Thr Pro Leu Leu Pro Ala Ala Gly Tyr Gly Val Gly Gln
260 265 270

Ala Lys Glu Asp Glu Glu Glu Glu Thr Arg Arg Gln Gln Gln Gln His
275 280 285

Cys Phe Val Leu Gly Ala Asp Leu Arg Leu Ala Glu Arg Pro Ser Gly
290 295 300

Ala His Asp Asp Ala Ala Gln Lys Pro Leu Arg His Phe Phe Asp Glu
305 310 315 320

Trp Pro His Glu Lys Gly Ser Lys Ala Gly Trp Trp Ile Gly Gly Leu
325 330 335

Asp Gly Glu Thr Thr Gln Leu Ser Met Ser Ile Pro Met Ala Ala Ala
340 345 350

Ala Asp Leu Pro Val Thr Ser Arg Tyr Arg Thr
355 360

<210> 115
<211> 39

<212> PRT
<213> Artificial sequence

<220>
<223> QLQ domain

<400> 115

Arg Pro Pro Phe Thr Pro Thr Gln Trp Glu Glu Leu Glu His Gln Ala
1               5                   10                  15

Leu Ile Tyr Lys Tyr Met Val Ser Gly Val Pro Val Pro Pro Glu Leu
            20                  25                  30

Ile Phe Ser Ile Arg Arg Ser
        35

<210> 116
<211> 44
<212> PRT
<213> Artificial sequence

<220>
<223> WRC domain

<400> 116

Asp Pro Glu Pro Gly Arg Cys Arg Arg Thr Asp Gly Lys Lys Trp Arg
1               5                   10                  15

Cys Ser Arg Glu Ala Tyr Pro Asp Ser Lys Tyr Cys Glu Lys His Met
            20                  25                  30

His Arg Gly Arg Asn Arg Ala Arg Lys Ser Leu Asp
        35                  40

<210> 117
<211> 2194
<212> DNA
<213> Oryza sativa

<400> 117

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag attttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc tttttcgat tagatgcaag     540
gtacttacgc acacactttg tgctcatgtg catgtgtgag tgcacctcct caatacacgt     600
```

```
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                                2194
```

<210> 118
<211> 58
<212> DNA
<213> Artificial sequence

<220>
<223> primer: prm10010

<400> 118
ggggacaagt ttgtacaaaa aagcaggctt aaacaatgat gagtctaagt ggaagtag 58

<210> 119

<211> 52
<212> DNA
<213> Artificial sequence

<220>
<223> primer: prm10011

<400> 119
ggggaccact ttgtacaaga aagctgggta gctctactta attagctacc ag 52

<210> 120
<211> 633
<212> DNA
<213> Arabidopsis thaliana

<400> 120

atgcaacagc acctgatgca gatgcagccc atgatggctg gttactaccc cagcaatgtt 60
acctctgatc atatccaaca gtacttggac gaaaacaaat cgttgattct gaagattgtt 120
gagtctcaaa actctggaaa gcttagcgaa tgcgccgaga atcaagcaag gcttcaacgc 180
aacctaatgt acctagctgc aatagcagat tctcagcctc agccaccaag tgtgcatagc 240
cagtatggat ctgctggtgg tgggatgatt cagggagaag gagggtcaca ctatttgcag 300
cagcaacaag cgactcaaca gcaacagatg actcagcagt ctctaatggc ggctcgatct 360
tcaatgttgt atgctcagca acagcggcag cagcagcctt acgcgacgct tcagcatcag 420
caatcgcacc atagccagct tggaatgagc tcgagcagcg gaggaggagg aagcagtggt 480
ctccatatcc ttcagggaga ggctggtggg tttcatgatt ttggccgtgg gaagccggaa 540
atgggaagtg gtggtggcgg tgaaggcaga ggaggaagtt caggggatgg tggagaaacc 600
ctttacttga aatcatcaga tgatgggaat tga 633

<210> 121
<211> 210
<212> PRT
<213> Arabidopsis thaliana

<400> 121

Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Met Ile Gln Gly Glu Gly Gly Ser
                85                  90                  95

His Tyr Leu Gln Gln Gln Gln Ala Thr Gln Gln Gln Gln Met Thr Gln
            100                 105                 110

Gln Ser Leu Met Ala Ala Arg Ser Ser Met Leu Tyr Ala Gln Gln Gln
        115                 120                 125

Arg Gln Gln Gln Pro Tyr Ala Thr Leu Gln His Gln Gln Ser His His
    130                 135                 140

Ser Gln Leu Gly Met Ser Ser Ser Ser Gly Gly Gly Gly Ser Ser Gly
145                 150                 155                 160

Leu His Ile Leu Gln Gly Glu Ala Gly Gly Phe His Asp Phe Gly Arg
                165                 170                 175

Gly Lys Pro Glu Met Gly Ser Gly Gly Gly Gly Glu Gly Arg Gly Gly
            180                 185                 190

Ser Ser Gly Asp Gly Gly Glu Thr Leu Tyr Leu Lys Ser Ser Asp Asp
        195                 200                 205

Gly Asn
    210

<210> 122
<211> 588
<212> DNA
<213> Arabidopsis thaliana

<400> 122

```
atgcagcagc agcagtctcc gcaaatgttt ccgatggttc cgtcgattcc ccctgctaac      60
aacatcacta ccgaacagat ccaaaagtac cttgatgaga acaagaagct gattatggcc     120
atcatggaaa accagaatct cggtaaactt gctgagtgcg cccagtacca agctcttctc     180
cagaagaact tgatgtatct tgctgcaatt gctgatgctc aacccccacc acctacgcca     240
ggaccttcac catctacagc tgtcgctgcc cagatggcaa caccgcattc tgggatgcaa     300
ccacctagct acttcatgca acacccacaa gcatcccctg cagggatttt cgctccaagg     360
ggtcctttac agtttggtag cccactccag tttcaggatc cgcaacagca gcagcagata     420
catcagcaag ctatgcaagg acacatgggg attagaccaa tgggtatgac caacaacggg     480
atgcagcatg cgatgcaaca accagaaacc ggtcttggag gaaacgtggg gcttagagga     540
ggaaagcaag atggagcaga tggacaagga aaagatgatg gcaagtga                  588
```

<210> 123
<211> 195
<212> PRT
<213> Arabidopsis thaliana

<400> 123

```
Met Gln Gln Gln Gln Ser Pro Gln Met Phe Pro Met Val Pro Ser Ile
1               5                   10                  15
```

Pro Pro Ala Asn Asn Ile Thr Thr Glu Gln Ile Gln Lys Tyr Leu Asp
20 25 30

Glu Asn Lys Lys Leu Ile Met Ala Ile Met Glu Asn Gln Asn Leu Gly
35 40 45

Lys Leu Ala Glu Cys Ala Gln Tyr Gln Ala Leu Leu Gln Lys Asn Leu
50 55 60

Met Tyr Leu Ala Ala Ile Ala Asp Ala Gln Pro Pro Pro Pro Thr Pro
65 70 75 80

Gly Pro Ser Pro Ser Thr Ala Val Ala Ala Gln Met Ala Thr Pro His
85 90 95

Ser Gly Met Gln Pro Pro Ser Tyr Phe Met Gln His Pro Gln Ala Ser
100 105 110

Pro Ala Gly Ile Phe Ala Pro Arg Gly Pro Leu Gln Phe Gly Ser Pro
115 120 125

Leu Gln Phe Gln Asp Pro Gln Gln Gln Gln Gln Ile His Gln Gln Ala
130 135 140

Met Gln Gly His Met Gly Ile Arg Pro Met Gly Met Thr Asn Asn Gly
145 150 155 160

Met Gln His Ala Met Gln Gln Pro Glu Thr Gly Leu Gly Gly Asn Val
165 170 175

Gly Leu Arg Gly Gly Lys Gln Asp Gly Ala Asp Gly Gln Gly Lys Asp
180 185 190

Asp Gly Lys
195

<210> 124
<211> 672
<212> DNA
<213> Arabidopsis thaliana

<400> 124

atgcagcaat ctccacagat gattccgatg gttcttcctt catttccgcc caccaataat 60
atcaccaccg aacagatcca aaagtatctt gatgagaaca agaagctgat aatggcgatc 120
ttggaaaatc agaacctcgg taaacttgca gaatgtgctc agtatcaagc tcttctccag 180
aagaatttga tgtatctcgc tgcaattgcg gatgctcaac ctcagccacc agcagctaca 240
ctaacatcag gagccatgac tccccaagca atggctccta atccgtcatc aatgcagcca 300
ccaccaagct acttcatgca gcaacatcaa gctgtgggaa tggctcaaca aatacctcct 360
gggattttcc ctcctagagg tccattgcaa tttggtagcc cgcatcagtt tctggatccg 420

cagcaacagt tacatcaaca agctatgcaa gggcacatgg ggattagacc aatgggtttg 480
aataataaca acggactgca acatcaaatg caccaccatg aaactgctct tgccgcaaac 540
aatgcgggtc ctaacgatgc tagtggagga ggtaaaccgg atgggaccaa tatgagccag 600
agtggagctg atgggcaagg tggctcagcc gctagacatg gcggtggtga tgcaaaaact 660
gaaggaaaat ga 672

<210> 125
<211> 223
<212> PRT
<213> Arabidopsis thaliana

<400> 125

Met Gln Gln Ser Pro Gln Met Ile Pro Met Val Leu Pro Ser Phe Pro
1 5 10 15

Pro Thr Asn Asn Ile Thr Thr Glu Gln Ile Gln Lys Tyr Leu Asp Glu
20 25 30

Asn Lys Lys Leu Ile Met Ala Ile Leu Glu Asn Gln Asn Leu Gly Lys
35 40 45

Leu Ala Glu Cys Ala Gln Tyr Gln Ala Leu Leu Gln Lys Asn Leu Met
50 55 60

Tyr Leu Ala Ala Ile Ala Asp Ala Gln Pro Gln Pro Pro Ala Ala Thr
65 70 75 80

Leu Thr Ser Gly Ala Met Thr Pro Gln Ala Met Ala Pro Asn Pro Ser
85 90 95

Ser Met Gln Pro Pro Pro Ser Tyr Phe Met Gln Gln His Gln Ala Val
100 105 110

Gly Met Ala Gln Gln Ile Pro Pro Gly Ile Phe Pro Pro Arg Gly Pro
115 120 125

Leu Gln Phe Gly Ser Pro His Gln Phe Leu Asp Pro Gln Gln Gln Leu
130 135 140

His Gln Gln Ala Met Gln Gly His Met Gly Ile Arg Pro Met Gly Leu
145 150 155 160

Asn Asn Asn Asn Gly Leu Gln His Gln Met His His His Glu Thr Ala
165 170 175

Leu Ala Ala Asn Asn Ala Gly Pro Asn Asp Ala Ser Gly Gly Gly Lys
180 185 190

Pro Asp Gly Thr Asn Met Ser Gln Ser Gly Ala Asp Gly Gln Gly Gly
195 200 205

```
Ser Ala Ala Arg His Gly Gly Gly Asp Ala Lys Thr Glu Gly Lys
    210                 215                 220
```

<210> 126
<211> 627
<212> DNA
<213> Allium cepa

<400> 126

```
atgcagcagc cgcagccagc gatgggaacc atgggctcgg tgccacctac tagcatcacc     60
accgaacaga ttcaaaggta cttggatgag aacaaacagt taatattggc aattttggat    120
aatcaaaatt taggaagact gaatgagtgt gctcaatatc aagctcagct tcaaaagaat    180
ctgctttacc tggcagcaat agctgatgct cagcctcagt ctcctgcggt gcgtctgcag    240
atgatgcctc aaggtgcagc tgccacgcct caagctggaa accaatttat gcagcagcag    300
agccctaatt tccctcccaa aacaggaatg caatttactc ctcaacaagt acaagaattg    360
cagcagcaac agctacaaca tcagccacat atgatgcctc catttcaagg tcaaatgggt    420
atgagaccta tgaatggaat gcaggcagca atgcatgcag attcatctct tgcttataac    480
actaacaata agcaagatgc aggaaacgca gcttatgaaa atactgctgc caacacagat    540
ggttccattc aaaagaaaac agcaaatgat gatttagacc cttctgcagc aaaccctaga    600
aggtctgaag atgccaaatc atcatga                                         627
```

<210> 127
<211> 208
<212> PRT
<213> Allium cepa

<400> 127

Met Gln Gln Pro Gln Pro Ala Met Gly Thr Met Gly Ser Val Pro Pro
1 5 10 15

Thr Ser Ile Thr Thr Glu Gln Ile Gln Arg Tyr Leu Asp Glu Asn Lys
20 25 30

Gln Leu Ile Leu Ala Ile Leu Asp Asn Gln Asn Leu Gly Arg Leu Asn
35 40 45

Glu Cys Ala Gln Tyr Gln Ala Gln Leu Gln Lys Asn Leu Leu Tyr Leu
50 55 60

Ala Ala Ile Ala Asp Ala Gln Pro Gln Ser Pro Ala Val Arg Leu Gln
65 70 75 80

Met Met Pro Gln Gly Ala Ala Ala Thr Pro Gln Ala Gly Asn Gln Phe
85 90 95

Met Gln Gln Gln Ser Pro Asn Phe Pro Pro Lys Thr Gly Met Gln Phe
100 105 110

Thr Pro Gln Gln Val Gln Glu Leu Gln Gln Gln Gln Leu Gln His Gln
115 120 125

Pro His Met Met Pro Pro Phe Gln Gly Gln Met Gly Met Arg Pro Met
130 135 140

Asn Gly Met Gln Ala Ala Met His Ala Asp Ser Ser Leu Ala Tyr Asn
145 150 155 160

Thr Asn Asn Lys Gln Asp Ala Gly Asn Ala Ala Tyr Glu Asn Thr Ala
165 170 175

Ala Asn Thr Asp Gly Ser Ile Gln Lys Lys Thr Ala Asn Asp Asp Leu
180 185 190

Asp Pro Ser Ala Ala Asn Pro Arg Arg Ser Glu Asp Ala Lys Ser Ser
195 200 205

<210> 128
<211> 633
<212> DNA
<213> Aquilegia formosa × Aquilegia pubescens

<400> 128

```
atgcaacaca tgcagatgca gcccatgatg ccaccttata gtgccaacag cgtcactact     60
gatcatatcc aacagtactt ggatgaaaat aaggcgttga ttctgaagat acttgagaac    120
caaaattcgg gaaaagttag tgaatgtgca gagaaccaag caagacttca acgaaatctt    180
atgtatctgg ctgcaattgc tgattctcaa ccacagcctc ccaatatgca tgctcagtac    240
tctaatgcgg gtataccacc tggtgcacat tacctacaac accaacaggc ccaacagatg    300
acacaacagt cgctcatggc tgctcgatca aatatgctgt atgctcagcc aatcacagga    360
atgcagcaac agcaagcaat gcatagccag cttggcatga gctctggtgg taacagtgga    420
ctccacatga tgcacaatga gggcagcatg ggaggtagtg ggcacttgg aagctattct     480
gattatggcc gtggcagtgg tggtggagta actatcgcta gcaaacaaga tggtggaagt    540
ggttctggtg aaggacgagg tggaaactct ggaggccaaa gtgcagatgg aggtgaatct    600
ctttacctga aaaacagtga cgaagggaac taa                                 633
```

<210> 129
<211> 210
<212> PRT
<213> Aquilegia formosa x Aquilegia pubescens

<400> 129

```
Met Gln His Met Gln Met Gln Pro Met Met Pro Pro Tyr Ser Ala Asn
1               5                   10                  15

Ser Val Thr Thr Asp His Ile Gln Gln Tyr Leu Asp Glu Asn Lys Ala
            20                  25                  30
```

```
Leu Ile Leu Lys Ile Leu Glu Asn Gln Asn Ser Gly Lys Val Ser Glu
        35                  40                  45

Cys Ala Glu Asn Gln Ala Arg Leu Gln Arg Asn Leu Met Tyr Leu Ala
    50                  55                  60

Ala Ile Ala Asp Ser Gln Pro Gln Pro Pro Asn Met His Ala Gln Tyr
65                  70                  75                  80

Ser Asn Ala Gly Ile Pro Pro Gly Ala His Tyr Leu Gln His Gln Gln
                85                  90                  95

Ala Gln Gln Met Thr Gln Gln Ser Leu Met Ala Ala Arg Ser Asn Met
            100                 105                 110

Leu Tyr Ala Gln Pro Ile Thr Gly Met Gln Gln Gln Gln Ala Met His
        115                 120                 125

Ser Gln Leu Gly Met Ser Ser Gly Gly Asn Ser Gly Leu His Met Met
    130                 135                 140

His Asn Glu Gly Ser Met Gly Gly Ser Gly Ala Leu Gly Ser Tyr Ser
145                 150                 155                 160

Asp Tyr Gly Arg Gly Ser Gly Gly Gly Val Thr Ile Ala Ser Lys Gln
                165                 170                 175

Asp Gly Gly Ser Gly Ser Gly Glu Gly Arg Gly Gly Asn Ser Gly Gly
            180                 185                 190

Gln Ser Ala Asp Gly Gly Glu Ser Leu Tyr Leu Lys Asn Ser Asp Glu
        195                 200                 205

Gly Asn
    210
```

<210> 130
<211> 668
<212> DNA
<213> Aquilegia formosa x Aquilegia pubescens

<400> 130

atgcagcaac ctccgccaat gatgaacatg gtcccaccat ttcctcctac taacattaca 60
actgaacaga ttcaaaagta tctggatgag aacaaaacac tgattttggc aatattagac 120
aatcagaatc ttggaaaatt agccgaatgt gctcagtacc aagctcagct tcagaagaat 180
ctgatgtatc ttgctgcaat tgctgatgcc caaccgcaag ctcctgcagt tccctctcag 240
atgccaacac atccggcaat gcaacaggga ggacattata tgcaacatcc gcaagcagct 300
atgcctcaac agcccagtgg ttttccaccc aagtccccca tgcaatttaa ccctcagcaa 360
atgcaagagc agcaacggct gcagctacaa cagcaacacc aacaggcact tcaaggtcat 420
atgggcattc gacctggagt caacaatggt ttgcaaatgc atggggatgg taatgttgga 480

ggcagcagca gcggtggccc atcatcaacc ggtaacttac ctgatttctc acgcagtggt 540
gccggtcctg gtgctggttc tagttcattg gatgcacggg aaggtaagca agatggagtg 600
gaagcgggtg ctggtgatgg tcaaggcaat tcagcagcca gaaataatgg ttcaaatggg 660
gatacatg 668

<210> 131
<211> 222
<212> PRT
<213> Aquilegia formosa x Aquilegia pubescens

<400> 131

Met Gln Gln Pro Pro Pro Met Met Asn Met Val Pro Pro Phe Pro Pro
1 5 10 15

Thr Asn Ile Thr Thr Glu Gln Ile Gln Lys Tyr Leu Asp Glu Asn Lys
20 25 30

Thr Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Ala Pro Ala Val Pro Ser Gln
65 70 75 80

Met Pro Thr His Pro Ala Met Gln Gln Gly Gly His Tyr Met Gln His
85 90 95

Pro Gln Ala Ala Met Pro Gln Gln Pro Ser Gly Phe Pro Pro Lys Ser
100 105 110

Pro Met Gln Phe Asn Pro Gln Gln Met Gln Glu Gln Gln Arg Leu Gln
115 120 125

Leu Gln Gln Gln His Gln Gln Ala Leu Gln Gly His Met Gly Ile Arg
130 135 140

Pro Gly Val Asn Asn Gly Leu Gln Met His Gly Asp Gly Asn Val Gly
145 150 155 160

Gly Ser Ser Ser Gly Gly Pro Ser Ser Thr Gly Asn Leu Pro Asp Phe
165 170 175

Ser Arg Ser Gly Ala Gly Pro Gly Ala Gly Ser Ser Ser Leu Asp Ala
180 185 190

Arg Glu Gly Lys Gln Asp Gly Val Glu Ala Gly Ala Gly Asp Gly Gln
195 200 205

Gly Asn Ser Ala Ala Arg Asn Asn Gly Ser Asn Gly Asp Thr
210 215 220

<210> 132
<211> 633
<212> DNA
<213> Aspergillus officinalis

<400> 132

```
atgcagcagc acctgatgca gatgcagccc atgatggcaa cctacggttc accgaatcag    60
gtcaccaccg atatcattca gcagtatctg gacgagaaca agcagttgat tctggctatt   120
cttgaaaacc aaaattcagg aaaagctgat gaatgtgctg agaatcaggc taagcttcag   180
aggaatctga tgtatcttgc agccattgcg gatagccagc cccaagttcc taccattgct   240
cagtatcctc ccaacgctgt tgctgctatg caatcgagtg ctcgctacat gcaacaacac   300
caagcagctc aacagatgac ccctcaatct ctcatggctg ctcgctcctc aatgctctac   360
tcacagtccc caatgtctgc actccagcag caacagcagc aagcagcaat gcatagccag   420
ctcgccatga gctccggagg caacaacagc agcaccggag gattcaccat tcttcatggt   480
gaagctagca taggaggcaa tggctcaatg aattctggtg gagtctttgg agattttgga   540
cggagcagcg gtgggaagca agagactggg agcgaagggc acgggacaga gactcctatg   600
tacctgaaag gctctgaaga agaaggaaac tga                                633
```

<210> 133
<211> 210
<212> PRT
<213> Aspergillus officinalis

<400> 133

```
Met Gln Gln His Leu Met Gln Met Gln Pro Met Met Ala Thr Tyr Gly
1               5                   10                  15

Ser Pro Asn Gln Val Thr Thr Asp Ile Ile Gln Gln Tyr Leu Asp Glu
            20                  25                  30

Asn Lys Gln Leu Ile Leu Ala Ile Leu Glu Asn Gln Asn Ser Gly Lys
        35                  40                  45

Ala Asp Glu Cys Ala Glu Asn Gln Ala Lys Leu Gln Arg Asn Leu Met
    50                  55                  60

Tyr Leu Ala Ala Ile Ala Asp Ser Gln Pro Gln Val Pro Thr Ile Ala
65                  70                  75                  80

Gln Tyr Pro Pro Asn Ala Val Ala Ala Met Gln Ser Ser Ala Arg Tyr
                85                  90                  95

Met Gln Gln His Gln Ala Ala Gln Gln Met Thr Pro Gln Ser Leu Met
            100                 105                 110

Ala Ala Arg Ser Ser Met Leu Tyr Ser Gln Ser Pro Met Ser Ala Leu
```

115 120 125

Gln Gln Gln Gln Gln Gln Ala Ala Met His Ser Gln Leu Ala Met Ser
130 135 140

Ser Gly Gly Asn Asn Ser Ser Thr Gly Gly Phe Thr Ile Leu His Gly
145 150 155 160

Glu Ala Ser Ile Gly Gly Asn Gly Ser Met Asn Ser Gly Gly Val Phe
165 170 175

Gly Asp Phe Gly Arg Ser Ser Gly Gly Lys Gln Glu Thr Gly Ser Glu
180 185 190

Gly His Gly Thr Glu Thr Pro Met Tyr Leu Lys Gly Ser Glu Glu Glu
195 200 205

Gly Asn
210

<210> 134
<211> 558
<212> DNA
<213> Beta vulgaris

<400> 134

atgcagcaac aatcacctca aatgttcaac cacccacctt cacaacccac aattactacc 60
gaacaaattc aaaagtatct tgatgagaac aagcagttga ttttggcaat tatggaaagt 120
caaaactctg gaaaaatgaa tgaatgtgcc cagtatcaag ctcagctgca gaaaaacttg 180
atgtacttgg ctgcaattgc tgatgctcag ccaccagcac ctacagggcc ctctcagtct 240
cagccgcaga attctcagat gcctatgcaa tcgaccattc cacaaggccc ttttatgccg 300
cctcctaaac ctgcagttac cccacagcaa acaggtccca gattgccctt tgctctacag 360
tcgtttgatc agcagtcacc ccatatgcaa atgcaatacc aacagtccat ggcaggctcc 420
atgggtatga gaatgggtgg gaataatgtt ttacgccctt ccatccagac cggatatgga 480
gctccaacac attttatgga tgcacggaac agacaagatg gttctgacgc aagtctcggt 540
gatgatcatg gaaagtaa 558

<210> 135
<211> 185
<212> PRT
<213> Beta vulgaris

<400> 135

Met Gln Gln Gln Ser Pro Gln Met Phe Asn His Pro Pro Ser Gln Pro
1               5                   10                  15

Thr Ile Thr Thr Glu Gln Ile Gln Lys Tyr Leu Asp Glu Asn Lys Gln
            20                  25                  30

Leu Ile Leu Ala Ile Met Glu Ser Gln Asn Ser Gly Lys Met Asn Glu
        35                  40                  45

Cys Ala Gln Tyr Gln Ala Gln Leu Gln Lys Asn Leu Met Tyr Leu Ala
    50                  55                  60

Ala Ile Ala Asp Ala Gln Pro Pro Ala Pro Thr Gly Pro Ser Gln Ser
65                  70                  75                  80

Gln Pro Gln Asn Ser Gln Met Pro Met Gln Ser Thr Ile Pro Gln Gly
                85                  90                  95

Pro Phe Met Pro Pro Pro Lys Pro Ala Val Thr Pro Gln Gln Thr Gly
            100                 105                 110

Pro Arg Leu Pro Phe Ala Leu Gln Ser Phe Asp Gln Gln Ser Pro His
        115                 120                 125

Met Gln Met Gln Tyr Gln Gln Ser Met Ala Gly Ser Met Gly Met Arg
    130                 135                 140

Met Gly Gly Asn Asn Val Leu Arg Pro Ser Ile Gln Thr Gly Tyr Gly
145                 150                 155                 160

Ala Pro Thr His Phe Met Asp Ala Arg Asn Arg Gln Asp Gly Ser Asp
                165                 170                 175

Ala Ser Leu Gly Asp Asp His Gly Lys
            180                 185

<210> 136
<211> 615
<212> DNA
<213> Brachypodium distachyon

<400> 136

```
atgcagcagg cgatgtccat gtccccgggg tcggccggcg cggtgccgcc tccggccggc      60
atcaccacag agcagatcca aaagtatttg gatgaaaata agcaacttat tttggccatc     120
ctggaaaatc agaacctagg aaagttgact gaatgtgctc agtatcaagc tcaacttcag     180
aagaatctct tgtatctggc tgccattgcg gatgcccaac caccacagaa ccctggaagt     240
cgcccccaga tggtgcagcc tggtggtatg ccaggtgcag ggcattacat gtcgcaagta     300
ccaatgttcc ctccaagaac cccttttaacc ccacaacaga tgcaagagca acagcaccag    360
cagcttcagc agcagcaagc acaggctctt gctttcccca gccagatggt catgagacca     420
ggtactgtga acggcatgca gcctatgcaa gctgatctcc aagcagcagc agcagcacct     480
ggcctggcag acagccgagg aagtaagcag gacgcagcgg tagctggggc catctcggaa     540
ccttctggca ccgagagtca caagagtaca ggagcggatc atgaggcagg tggcgatgta     600
gctgagcaat cctaa                                                      615
```

<210> 137
<211> 204
<212> PRT
<213> Brachypodium distachyon

<400> 137

```
Met Gln Gln Ala Met Ser Met Ser Pro Gly Ser Ala Gly Ala Val Pro
1               5                   10                  15

Pro Pro Ala Gly Ile Thr Thr Glu Gln Ile Gln Lys Tyr Leu Asp Glu
            20                  25                  30

Asn Lys Gln Leu Ile Leu Ala Ile Leu Glu Asn Gln Asn Leu Gly Lys
        35                  40                  45

Leu Thr Glu Cys Ala Gln Tyr Gln Ala Gln Leu Gln Lys Asn Leu Leu
    50                  55                  60

Tyr Leu Ala Ala Ile Ala Asp Ala Gln Pro Pro Gln Asn Pro Gly Ser
65                  70                  75                  80

Arg Pro Gln Met Val Gln Pro Gly Gly Met Pro Gly Ala Gly His Tyr
                85                  90                  95

Met Ser Gln Val Pro Met Phe Pro Pro Arg Thr Pro Leu Thr Pro Gln
            100                 105                 110

Gln Met Gln Glu Gln Gln His Gln Gln Leu Gln Gln Gln Gln Ala Gln
        115                 120                 125

Ala Leu Ala Phe Pro Ser Gln Met Val Met Arg Pro Gly Thr Val Asn
    130                 135                 140

Gly Met Gln Pro Met Gln Ala Asp Leu Gln Ala Ala Ala Ala Ala Pro
145                 150                 155                 160

Gly Leu Ala Asp Ser Arg Gly Ser Lys Gln Asp Ala Ala Val Ala Gly
                165                 170                 175

Ala Ile Ser Glu Pro Ser Gly Thr Glu Ser His Lys Ser Thr Gly Ala
            180                 185                 190

Asp His Glu Ala Gly Gly Asp Val Ala Glu Gln Ser
        195                 200
```

<210> 138
<211> 591
<212> DNA
<213> Brassica napus

<400> 138

```
atgcagccca tgatggctgg ttactacccc agcaatgtca cctctgatca tatccagcag      60
tacttggatg agaacaagtc tttgattctg aagatagttg agtctcaaaa ctcaggaaag     120
```

```
ctcagcgagt gtgccgagaa tcaggcaagg cttcaacgca acctcatgta cttggctgca     180
atagcagatt ctcagcctca acctccaagc gtgcatagcc agtatggatc tgctggtggt     240
gggttgattc agggagaagg agcgtcacac tatttgcagc agcaacaggc gactcaacag     300
cagcagatga ctcagcagtc tcttatggca gctcgttctt caatgatgta tcagcagcag     360
caacagcctt atgcaacgct tcagcatcag cagttgcacc atagccagct tgggatgagc     420
tctagcagcg gaggaggaag cagtggtctc catatccttc agggagaggc tggtgggttt     480
catgaatttg gccgtgggaa gccggagatg ggaagtggtg aaggcagggg tggaagctca     540
ggggatggtg gagaaacact ctacttgaag tcatcagatg atgggaactg a              591
```

<210> 139
<211> 203
<212> PRT
<213> Brassica napus

<400> 139

Met Gln Gln His Leu Met Gln Met Gln Pro Met Met Ala Gly Tyr Tyr
1 5 10 15

Pro Ser Asn Val Thr Ser Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Ser Val His Ser
65 70 75 80

Gln Tyr Gly Ser Ala Gly Gly Gly Leu Ile Gln Gly Glu Gly Ala Ser
85 90 95

His Tyr Leu Gln Gln Gln Gln Ala Thr Gln Gln Gln Gln Met Thr Gln
100 105 110

Gln Ser Leu Met Ala Ala Arg Ser Ser Met Met Tyr Gln Gln Gln Gln
115 120 125

Gln Pro Tyr Ala Thr Leu Gln His Gln Gln Leu His His Ser Gln Leu
130 135 140

Gly Met Ser Ser Ser Ser Gly Gly Gly Ser Ser Gly Leu His Ile Leu
145 150 155 160

Gln Gly Glu Ala Gly Gly Phe His Glu Phe Gly Arg Gly Lys Pro Glu
165 170 175

Met Gly Ser Gly Glu Gly Arg Gly Gly Ser Ser Gly Asp Gly Gly Glu

180 185 190

Thr Leu Tyr Leu Lys Ser Ser Asp Asp Gly Asn
195 200

<210> 140
<211> 636
<212> DNA
<213> Brassica napus

<400> 140

```
atgcagcagc agcagcagca gcagcagcag cctccgcaaa tgtttccgat ggctccttcg    60
atgccgccaa ctaacatcac caccgaacag atccaaaagt accttgagga gaacaagaag   120
ctgataatgg caatcatgga aaatcagaat cttggcaagc ttgcagagtg tgcacagtac   180
caagctcttc tccagaagaa cttaatgtac ctcgctgcta ttgctgatgc tcaacctcct   240
ccatctaccg ctggagctac accaccacca gctatggctt cccagatggg ggcaccgcat   300
cctgggatgc aaccgccgag ctactttatg caacacccac aagcttcagg gatggctcaa   360
caagcaccac ccgctggtat cttccctccg agaggtcctt tgcagtttgg tagcccacac   420
cagcttcagg atccgcaaca gcagcatatg catcaacagg ctatgcaagg acacatgggg   480
atgcgaccaa tgggtatcaa caacaacaat gggatgcagc atcagatgca gcaacaacaa   540
ccagaaacct ctcttggagg aagcgctgca aacgtggggc ttagaggtgg aaagcaagat   600
ggagcagatg gacaaggaaa agatgatggc aaatga                             636
```

<210> 141
<211> 203
<212> PRT
<213> Brassica napus

<400> 141

```
Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Leu Ile Gln Gly Glu Gly Ala Ser
                85                  90                  95

His Tyr Leu Gln Gln Gln Gln Ala Thr Gln Gln Gln Gln Met Thr Gln
            100                 105                 110
```

Gln Ser Leu Met Ala Ala Arg Ser Ser Met Met Tyr Gln Gln Gln Gln
115 120 125

Gln Pro Tyr Ala Thr Leu Gln His Gln Gln Leu His His Ser Gln Leu
130 135 140

Gly Met Ser Ser Ser Ser Gly Gly Gly Ser Ser Gly Leu His Ile Leu
145 150 155 160

Gln Gly Glu Ala Gly Gly Phe His Glu Phe Gly Arg Gly Lys Pro Glu
165 170 175

Met Gly Ser Gly Glu Gly Arg Gly Gly Ser Ser Gly Asp Gly Gly Glu
180 185 190

Thr Leu Tyr Leu Lys Ser Ser Asp Asp Gly Asn
195 200

<210> 142
<211> 552
<212> DNA
<213> Chlamydomonas reinhardtii

<400> 142

atggcggcag cctcaaaacc tccaccgatg acgaccgaca aaatacaaga catgctggag 60
gagaatttca agttcattaa agccattgcc gagcagcaaa acttgggccg ggttcaagaa 120
gtgcaccagt accagcagaa gctgcaggag aacctgatgc tgctggccgc agtggcagac 180
acctactcca actcagcagc agcagcacag ccggggggag aagctggcgc agccgcaccc 240
gcggcggcca cggcacgacc acccaccgcg cctggagcgc cgctgggggc accgggagca 300
ccgccgcccg cggctccggc tctcacacca cagcagatcc acgcggccgt gcagcaggca 360
ctggccatga agcagcagca gcagcagcag cagcagcaac agccgcagca gccgcagcag 420
tcggcggtgg cgcagtacca gcaaccgcct caagcggggc tgcccatacc gggcgggatg 480
gcgcccgggc aaggcgcgcc gccaggcttc acgctaccgg cgccaccgcc cctgaaccta 540
gccgggcagt ag 552

<210> 143
<211> 183
<212> PRT
<213> Chlamydomonas reinhardtii

<400> 143

Met Ala Ala Ala Ser Lys Pro Pro Pro Met Thr Thr Asp Lys Ile Gln
1 5 10 15

Asp Met Leu Glu Glu Asn Phe Lys Phe Ile Lys Ala Ile Ala Glu Gln
20 25 30

Gln Asn Leu Gly Arg Val Gln Glu Val His Gln Tyr Gln Gln Lys Leu
35 40 45

Gln Glu Asn Leu Met Leu Leu Ala Ala Val Ala Asp Thr Tyr Ser Asn
50 55 60

Ser Ala Ala Ala Ala Gln Pro Gly Gly Glu Ala Gly Ala Ala Ala Pro
65 70 75 80

Ala Ala Ala Thr Ala Arg Pro Pro Thr Ala Pro Gly Ala Pro Leu Gly
85 90 95

Ala Pro Gly Ala Pro Pro Pro Ala Ala Pro Ala Leu Thr Pro Gln Gln
100 105 110

Ile His Ala Ala Val Gln Gln Ala Leu Ala Met Lys Gln Gln Gln Gln
115 120 125

Gln Gln Gln Gln Gln Gln Pro Gln Gln Pro Gln Gln Ser Ala Val Ala
130 135 140

Gln Tyr Gln Gln Pro Pro Gln Ala Gly Leu Pro Ile Pro Gly Gly Met
145 150 155 160

Ala Pro Gly Gln Gly Ala Pro Pro Gly Phe Thr Leu Pro Ala Pro Pro
165 170 175

Pro Leu Asn Leu Ala Gly Gln
180

<210> 144
<211> 663
<212> DNA
<213> Citrus sinensis

<400> 144

atgcaacagc acctgatgca gatgcagccc atgatggcag cttattatcc caacaacgtc 60
actactgacc acattcaaca gtatctagat gagaacaaat cattgatttt gaagattgtt 120
gagagccaga attcagggaa actgagcgag tgtgcagaga accaggcaag attgcagcgg 180
aatctcatgt acctggctgc tattgctgat gctcaacccc aaccacctag cgttcatgcc 240
cagttctctt ctggtggcat tatgcagcca ggagctcact atatgcaaca ccagcaatct 300
cagccaatga caccacagtc acttatggct gcacgctcat ccatggtgta ctctcaacag 360
caattttcag tgcttcagca acagcaagcc ttgcatggtc agcttggcat gagctctggt 420
ggtagctcag gacttcacat gctgcaaagt gagggtagta ctgcaggagg tagtggttca 480
cttgggggtg gggattcccc tgattttggc cgtggctcat ctggtgaagg cttgcactca 540
aggggaatgg ggagcaagca tgatataggc agttctggat ctgctgaagg acgaggaggg 600
agctcaggaa gccaagatgg aggcgaaact ctctacttga aaggggctga tgatggaaat 660
taa 663

<210> 145
<211> 219
<212> PRT
<213> Citrus sinensis

<400> 145

```
Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ala Gln Pro Gln Pro Pro Ser Val His Ala
65                  70                  75                  80

Gln Phe Ser Ser Gly Gly Ile Met Gln Pro Gly Ala His Tyr Met Gln
                85                  90                  95

His Gln Gln Ser Gln Pro Met Thr Pro Gln Ser Leu Met Ala Ala Arg
            100                 105                 110

Ser Ser Met Val Tyr Ser Gln Gln Gln Phe Ser Val Leu Gln Gln Gln
        115                 120                 125

Gln Ala Leu His Gly Gln Leu Gly Met Ser Ser Gly Gly Ser Ser Gly
    130                 135                 140

Leu His Met Leu Gln Ser Glu Gly Ser Thr Ala Gly Gly Ser Gly Ser
145                 150                 155                 160

Leu Gly Gly Gly Gly Phe Pro Asp Phe Gly Arg Gly Ser Ser Gly Glu
                165                 170                 175

Gly Leu His Ser Arg Gly Met Gly Ser Lys His Asp Ile Gly Ser Ser
            180                 185                 190

Gly Ser Ala Glu Gly Arg Gly Gly Ser Ser Gly Ser Gln Asp Gly Gly
        195                 200                 205

Glu Thr Leu Tyr Leu Lys Gly Ala Asp Asp Gly
    210                 215
```

<210> 146
<211> 636
<212> DNA
<213> Citrus sinensis

<400> 146

```
atgcagcagc caccgcaaat gatccctgtt atgccttcat ttccacccac caacatcacc    60
acagagcaga ttcaaaagta ccttgatgag aacaaaaagt tgattttggc aattttggac   120
aatcaaaatc ttggaaagct tacagaatgt gcccactatc aagctcagct tcaaaagaat   180
ttaatgtatt tagctgcaat tgctgatgca caaccacaag caccaacaat gcctcctcag   240
atggctccac atcctgcaat gcaagctagt gggtattaca tgcaacatcc tcaggcggca   300
gcaatggctc agcaacaagg aatctttccc caaaagatgc cattacaatt caataaccct   360
catcaactac aggatcctca acagcagcta caccaacatc aagccatgca agcacaaatg   420
ggaatgagac cgggtgccac taacaatggt atgcatccca tgcatgctga aagctctctt   480
ggaggtggca gcagtggagg accccccttca gcatcaggcc caggtgacat acgtggtgga   540
aataagcaag atgcctcgga ggctgggact actggtgctg atggccaggg cagttcggct   600
ggtgggcatg gtggggatgg agaggaggca aagtga                             636
```

<210> 147
<211> 211
<212> PRT
<213> Citrus sinensis

<400> 147

```
Met Gln Gln Pro Pro Gln Met Ile Pro Val Met Pro Ser Phe Pro Pro
1               5                   10                  15

Thr Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Thr
        35                  40                  45

Glu Cys Ala His Tyr Gln Ala Gln Leu Gln Lys Asn Leu Met Tyr Leu
    50                  55                  60

Ala Ala Ile Ala Asp Ala Gln Pro Gln Ala Pro Thr Met Pro Pro Gln
65                  70                  75                  80

Met Ala Pro His Pro Ala Met Gln Ala Ser Gly Tyr Tyr Met Gln His
                85                  90                  95

Pro Gln Ala Ala Ala Met Ala Gln Gln Gln Gly Ile Phe Pro Gln Lys
            100                 105                 110

Met Pro Leu Gln Phe Asn Asn Pro His Gln Leu Gln Asp Pro Gln Gln
        115                 120                 125

Gln Leu His Gln His Gln Ala Met Gln Ala Gln Met Gly Met Arg Pro
    130                 135                 140

Gly Ala Thr Asn Asn Gly Met His Pro Met His Ala Glu Ser Ser Leu
145                 150                 155                 160
```

Gly Gly Gly Ser Ser Gly Gly Pro Pro Ser Ala Ser Gly Pro Gly Asp
165 170 175

Ile Arg Gly Gly Asn Lys Gln Asp Ala Ser Glu Ala Gly Thr Thr Gly
180 185 190

Ala Asp Gly Gln Gly Ser Ser Ala Gly Gly His Gly Gly Asp Gly Glu
195 200 205

Glu Ala Lys
210

<210> 148
<211> 729
<212> DNA
<213> Cryptomeria japonica

<400> 148

atgcagcagc atctcatgca aatgcagccg atgatggcag cagcttacgc ttctaacaac 60
attaccactg atcacattca aaagtacttg gatgagaaca agcagttgat attagcaatt 120
atggacaatc aaaatctggg aaagcttaat gaatgtgcac agtaccaagc aaaacttcaa 180
cagaacttga tgtatctagc tgctattgct gattctcagc ctcaagttcc ggctgcacat 240
gctcagattc ctcctaatgc ggttgtgcag tctggtgggc ttttcatgca gcaccagcaa 300
gcacagcagc aagttactcc tcagtctctt atggcagcta gatcttccat gttgtatacc 360
cagcagccga tggctgcttt gcatcaagcc cagcagcagc agcaacaaca atctcttcac 420
agccatcttg gtataagttc tggaggaagc aatggcttgc acatgttgca tggtgaagca 480
aacatgggag gtaacgggcc tctctcatct ggaggcttcc ctgacttttc acgtggaact 540
ggggcctctg gtgaaggcat tcaggccaat aggggcatgt gtatagatcg tggtgcaaat 600
aagcatgatg gcgctggaac ggagaatgct catccaggcc caggggatgg gcgagggagt 660
tcgactggag gccagaatac agatgggtca gaacaatcat acctgaaagc ctcagaagag 720
gggaactag 729

<210> 149
<211> 242
<212> PRT
<213> cryptomeria japonica

<400> 149

Met Gln Gln His Leu Met Gln Met Gln Pro Met Met Ala Ala Ala Tyr
1 5 10 15

Ala Ser Asn Asn Ile Thr Thr Asp His Ile Gln Lys Tyr Leu Asp Glu
20 25 30

Asn Lys Gln Leu Ile Leu Ala Ile Met Asp Asn Gln Asn Leu Gly Lys
35 40 45

Leu Asn Glu Cys Ala Gln Tyr Gln Ala Lys Leu Gln Gln Asn Leu Met
50 55 60

Tyr Leu Ala Ala Ile Ala Asp Ser Gln Pro Gln Val Pro Ala Ala His
65 70 75 80

Ala Gln Ile Pro Pro Asn Ala Val Val Gln Ser Gly Gly Leu Phe Met
85 90 95

Gln His Gln Gln Ala Gln Gln Gln Val Thr Pro Gln Ser Leu Met Ala
100 105 110

Ala Arg Ser Ser Met Leu Tyr Thr Gln Gln Pro Met Ala Ala Leu His
115 120 125

Gln Ala Gln Gln Gln Gln Gln Gln Gln Ser Leu His Ser His Leu Gly
130 135 140

Ile Ser Ser Gly Gly Ser Asn Gly Leu His Met Leu His Gly Glu Ala
145 150 155 160

Asn Met Gly Gly Asn Gly Pro Leu Ser Ser Gly Gly Phe Pro Asp Phe
165 170 175

Ser Arg Gly Thr Gly Ala Ser Gly Glu Gly Ile Gln Ala Asn Arg Gly
180 185 190

Met Cys Ile Asp Arg Gly Ala Asn Lys His Asp Gly Ala Gly Thr Glu
195 200 205

Asn Ala His Pro Gly Pro Gly Asp Gly Arg Gly Ser Ser Thr Gly Gly
210 215 220

Gln Asn Thr Asp Gly Ser Glu Gln Ser Tyr Leu Lys Ala Ser Glu Glu
225 230 235 240

Gly Asn

<210> 150
<211> 651
<212> DNA
<213> Curcuma longa

<400> 150

atgcagcaat ctccacattc gctagccccc atgtcagcag cccctgttgc gaatattaca 60
acagaacaaa ttcaaaagta cttggatgag aataagcagc tcattttggc aatattggaa 120
aatcagaacc ttgggaaatt ggctgaatgt gctcagtacc aagcgcagct tcagaaaaat 180
ctactttatc ttgctgcaat tgctgatgct caacctaatg cacctgcagt tcgtccccag 240
cagatcatgc cacacggtac gataccacag ggaagccctt tcatgcaaca atcacccatc 300

ttccctcgag gtcctcttcc atataatcct caacaaatgc aagggcagct acatccccaa 360
cccccaggaa tggtgttccc aggccatatg ggcattaggc ccggcgctgt caacggctta 420
catggctcgc atactgaacc atctcatggt ggcactgcta atcccctcac aactccaagc 480
ttgtctggat tcccaccaac caactcagat ggacgtggga gtaagcaaga agccggcatc 540
gccatggtac ctgccgtagc tgagagccac aggaactcag gaagtgagcc tgtcagtggg 600
gatgctgatc aatcacatgc taaaagacca gaggatacaa agacaccatg a 651

<210> 151
<211> 216
<212> PRT
<213> Curcuma longa

<400> 151

Met Gln Gln Ser Pro His Ser Leu Ala Pro Met Ser Ala Ala Pro Val
1 5 10 15

Ala Asn Ile Thr Thr Glu Gln Ile Gln Lys Tyr Leu Asp Glu Asn Lys
20 25 30

Gln Leu Ile Leu Ala Ile Leu Glu Asn Gln Asn Leu Gly Lys Leu Ala
35 40 45

Glu Cys Ala Gln Tyr Gln Ala Gln Leu Gln Lys Asn Leu Leu Tyr Leu
50 55 60

Ala Ala Ile Ala Asp Ala Gln Pro Asn Ala Pro Ala Val Arg Pro Gln
65 70 75 80

Gln Ile Met Pro His Gly Thr Ile Pro Gln Gly Ser Pro Phe Met Gln
85 90 95

Gln Ser Pro Ile Phe Pro Arg Gly Pro Leu Pro Tyr Asn Pro Gln Gln
100 105 110

Met Gln Gly Gln Leu His Pro Gln Pro Pro Gly Met Val Phe Pro Gly
115 120 125

His Met Gly Ile Arg Pro Gly Ala Val Asn Gly Leu His Gly Ser His
130 135 140

Thr Glu Pro Ser His Gly Gly Thr Ala Asn Pro Leu Thr Thr Pro Ser
145 150 155 160

Leu Ser Gly Phe Pro Pro Thr Asn Ser Asp Gly Arg Gly Ser Lys Gln
165 170 175

Glu Ala Gly Ile Ala Met Val Pro Ala Val Ala Glu Ser His Arg Asn
180 185 190

Ser Gly Ser Glu Pro Val Ser Gly Asp Ala Asp Gln Ser His Ala Lys

195 200 205

Arg Pro Glu Asp Thr Lys Thr Pro
210 215

<210> 152
<211> 597
<212> DNA
<213> Euphorbia esula

<400> 152

atgcagcagc aaccgcagat gatgcctatg atgccttcat atccaccagc aaacattacc 60
acggagcaaa tccaaaagta tcttgatgaa aataaaaaat tgattttggc gatcttggat 120
aatcaaaatc ttggaaaact cgctgagtgt gcacagtatc aagccctgct gcaaaaaaat 180
ctgatgtatt tagccgcaat tgctgatgca caaccccaga ccccacccat gccacctcag 240
atgtccccac atccggctat gcaacaagga gcatattaca tgcaacatcc tcaggctgca 300
gcagcagcaa tggctcatca gtcgggtatt ttcccaccaa agatgtctcc gttacaattc 360
aataatcctc atcaaataca ggacccccag cagttacatc aagcagccct ccaagggcaa 420
atgggaatga ggcccatggg gcccaataac gggatgcatc cgatgcaccc cgaggcaaat 480
cttggaggat ctaatgatgg tcgtggagga aacaaacagg atgctccgga gacgggagca 540
tcgggaggtg atgggcaagg caattctggt ggtgatgggg ctgaagatgg gaaatga 597

<210> 153
<211> 198
<212> PRT
<213> Euphorbia esula

<400> 153

Met Gln Gln Gln Pro Gln Met Met Pro Met Met Pro Ser Tyr Pro Pro
1 5 10 15

Ala Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Leu Leu Gln Lys Asn Leu Met Tyr Leu
50 55 60

Ala Ala Ile Ala Asp Ala Gln Pro Gln Thr Pro Pro Met Pro Pro Gln
65 70 75 80

Met Ser Pro His Pro Ala Met Gln Gln Gly Ala Tyr Tyr Met Gln His
85 90 95

Pro Gln Ala Ala Ala Ala Ala Met Ala His Gln Ser Gly Ile Phe Pro
100 105 110

Pro Lys Met Ser Pro Leu Gln Phe Asn Asn Pro His Gln Ile Gln Asp
115 120 125

Pro Gln Gln Leu His Gln Ala Ala Leu Gln Gly Gln Met Gly Met Arg
130 135 140

Pro Met Gly Pro Asn Asn Gly Met His Pro Met His Pro Glu Ala Asn
145 150 155 160

Leu Gly Gly Ser Asn Asp Gly Arg Gly Gly Asn Lys Gln Asp Ala Pro
165 170 175

Glu Thr Gly Ala Ser Gly Gly Asp Gly Gln Gly Asn Ser Gly Gly Asp
180 185 190

Gly Ala Glu Asp Gly Lys
195

<210> 154
<211> 597
<212> DNA
<213> Fragaria vesca

<400> 154

atgcagcagc agccacagca gatgatgccc aacatgactt cgcttcctcc caataccatc 60
accaccgagc aaattcagaa gtgccttgat gagaacaaaa agttgattct agcaatattg 120
gacaatcaaa accttggaaa acttgctgag tgtgcccagt accaaactca gcttcaaaag 180
aatctcatgt atttagcagc aattgctgat gcacaaccac aaccacaacc acaagcacca 240
gcaatgccgg cccagcagct ggccccgcat cctgcgatgc aacaagctgg atattacatg 300
cagcatcctc aggctgcagc agcaatggct cagcaacagg gtcttttccc tcaaaagatg 360
caaatgcagt ttaatagccc acaacaaatg cacgagatgc agcagcagtt acaccaacag 420
gccatgcatg gccagatggg gatgagacct ggaggggcca atgggatgcc ttcaatgcat 480
catactgaga acacccatgg aggaagcaag caagacaact cagaggctgg ggcaggtggt 540
gatggccagg ggaactcagc cggtggccac agaagtggcg acggagagga caagtga 597

<210> 155
<211> 198
<212> PRT
<213> Fragaria vesca

<400> 155

Met Gln Gln Gln Pro Gln Gln Met Met Pro Asn Met Thr Ser Leu Pro
1 5 10 15

Pro Asn Thr Ile Thr Thr Glu Gln Ile Gln Lys Cys Leu Asp Glu Asn
20 25 30

Lys Lys Leu Ile Leu Ala Ile Leu Asp Asn Gln Asn Leu Gly Lys Leu
35 40 45

Ala Glu Cys Ala Gln Tyr Gln Thr Gln Leu Gln Lys Asn Leu Met Tyr
50 55 60

Leu Ala Ala Ile Ala Asp Ala Gln Pro Gln Pro Gln Pro Gln Ala Pro
65 70 75 80

Ala Met Pro Ala Gln Gln Leu Ala Pro His Pro Ala Met Gln Gln Ala
85 90 95

Gly Tyr Tyr Met Gln His Pro Gln Ala Ala Ala Ala Met Ala Gln Gln
100 105 110

Gln Gly Leu Phe Pro Gln Lys Met Gln Met Gln Phe Asn Ser Pro Gln
115 120 125

Gln Met His Glu Met Gln Gln Gln Leu His Gln Gln Ala Met His Gly
130 135 140

Gln Met Gly Met Arg Pro Gly Gly Ala Asn Gly Met Pro Ser Met His
145 150 155 160

His Thr Glu Asn Thr His Gly Gly Ser Lys Gln Asp Asn Ser Glu Ala
165 170 175

Gly Ala Gly Gly Asp Gly Gln Gly Asn Ser Ala Gly Gly His Arg Ser
180 185 190

Gly Asp Gly Glu Asp Lys
195

<210> 156
<211> 639
<212> DNA
<213> Glycine max

<400> 156

```
atgcagcagc acctgatgca gatgcagccc atgatggctg cctactaccc caacaacgtc      60
accactgatc acattcaaca gtacctggat gagaacaagt ccttgattct gaagattgtt     120
gaaagccaga attctggcaa gctgagcgag tgtgccgaga accaatcaag gctgcagaga     180
aatctcatgt acctagctgc aatagctgat tctcaaccac aaccatctcc attggctggt     240
cagtatcctt ctagtggact tgtgcagcag ggagcacact acatgcaggc tcaacaggct     300
cagcagatgt cacaacaaca gctaatggct tcgcgctcct cgctcctgta ctcccaacag     360
cctttctcag tgcttcaaca gcagcaaggc atgcacagcc aacttggcat gagctccagt     420
ggaagtcaag gcctccacat gctgcaaagt gaagccacta atgttggagg caatgcaacc     480
ataggaaccg gaggagggtt tccggacttt gtacgcattg gtagtggcaa gcaagatatt     540
ggaatctctg gtgaaggcag aggaggaaac tctagtggcc actctggtga tggtggtgag     600
acacttaatt acctgaaagc tgctggtgat ggaaactga                            639
```

<210> 157
<211> 212
<212> PRT
<213> Glycine max

<400> 157

```
Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ser Arg Leu Gln Arg Asn Leu Met Tyr
    50                  55                  60

Leu Ala Ala Ile Ala Asp Ser Gln Pro Gln Pro Ser Pro Leu Ala Gly
65                  70                  75                  80

Gln Tyr Pro Ser Ser Gly Leu Val Gln Gln Gly Ala His Tyr Met Gln
                85                  90                  95

Ala Gln Gln Ala Gln Gln Met Ser Gln Gln Gln Leu Met Ala Ser Arg
            100                 105                 110

Ser Ser Leu Leu Tyr Ser Gln Gln Pro Phe Ser Val Leu Gln Gln Gln
        115                 120                 125

Gln Gly Met His Ser Gln Leu Gly Met Ser Ser Ser Gly Ser Gln Gly
    130                 135                 140

Leu His Met Leu Gln Ser Glu Ala Thr Asn Val Gly Gly Asn Ala Thr
145                 150                 155                 160

Ile Gly Thr Gly Gly Gly Phe Pro Asp Phe Val Arg Ile Gly Ser Gly
                165                 170                 175

Lys Gln Asp Ile Gly Ile Ser Gly Glu Gly Arg Gly Gly Asn Ser Ser
            180                 185                 190

Gly His Ser Gly Asp Gly Gly Glu Thr Leu Asn Tyr Leu Lys Ala Ala
        195                 200                 205

Gly Asp Gly Asn
    210
```

<210> 158
<211> 633
<212> DNA
<213> Glycine max

<400> 158

atgcagcagc acctgatgca gatgcagccc atgatggcag gctactaccc caacaacgtc 60
accactgatc acattcagca gtatctggat gagaacaagt ccttaattct gaagattgtt 120
gaaagccaga attctggcaa gctgagcgag tgtgccgaga accaagcaag gcttcagaga 180
aatctcatgt acttagctgc aatagctgat tctcaacccc aaccacccac catgtctggt 240
cagtaccctc cgagtgggat gatgcagcag ggagcacagt acatgcaggc tcaacaacag 300
gcacagcaga tgacaccaca acaactaatg gcagcacgct catctctttt gtacgcacag 360
cagccgtact cagcacttca acagcagcaa gccatgcaca gtgcactggg gtcgagttcg 420
gggctccaca tgctgcaaag tgaaggcagc aatgtgaatg tgggaggagg gtttcctgac 480
tttgtgcgtg gcggcagctc cacaggggag ggtttgcaca gtggtggaag ggtatcatt 540
ggaagtagca agcaggaaat ggggggttca agtgaaggcc gcggtgaagg gggtgaaaac 600
ctctacctca aagttgctga tgatggaaac tag 633

<210> 159
<211> 210
<212> PRT
<213> Glycine max

<400> 159

Met Gln Gln His Leu Met Gln Met Gln Pro Met Met Ala Gly Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Met Ser Gly
65 70 75 80

Gln Tyr Pro Pro Ser Gly Met Met Gln Gln Gly Ala Gln Tyr Met Gln
85 90 95

Ala Gln Gln Gln Ala Gln Gln Met Thr Pro Gln Gln Leu Met Ala Ala
100 105 110

Arg Ser Ser Leu Leu Tyr Ala Gln Gln Pro Tyr Ser Ala Leu Gln Gln
115 120 125

Gln Gln Ala Met His Ser Ala Leu Gly Ser Ser Ser Gly Leu His Met
130 135 140

Leu Gln Ser Glu Gly Ser Asn Val Asn Val Gly Gly Gly Phe Pro Asp
145 150 155 160

Phe Val Arg Gly Gly Ser Ser Thr Gly Glu Gly Leu His Ser Gly Gly
165 170 175

Arg Gly Ile Ile Gly Ser Ser Lys Gln Glu Met Gly Gly Ser Ser Glu
180 185 190

Gly Arg Gly Glu Gly Gly Glu Asn Leu Tyr Leu Lys Val Ala Asp Asp
195 200 205

Gly Asn
210

<210> 160
<211> 642
<212> DNA
<213> Glycine max

<400> 160

atgcagcaga caccgccaat gattcctatg atgccttctt tcccacctac gaacataacc 60
accgagcaga ttcaaaaata ccttgatgag aacaagaagc tgattctggc aatattggac 120
aatcaaaatc ttggaaaact tgcagaatgt gcccagtacc aagctcagct tcaaaagaat 180
ttgatgtatt tagctgcaat tgctgatgcc cagcctcaaa ccccggccat gcctccgcag 240
atggcaccgc accctgccat gcaaccagga ttctatatgc aacatcctca ggctgctgca 300
gcagcaatgg ctcagcagca gcaaggaatg ttcccccaga aaatgccatt gcaatttggc 360
aatccacatc aaatgcagga acaacaacag cagctacacc agcaggccat ccaaggtcaa 420
atgggactta gacctggaga tataaataat ggcatgcatc caatgcacag tgaggctgct 480
cttggaggtg gaaacagcgg tggtccacct tcggctactg gtccaaacga tgcacgtggt 540
ggaagcaagc aagatgcctc tgaggctgga acagctggtg gagacggcca aggcagctcc 600
gcggctgctc ataacagtgg agatggtgaa gaggcaaagt ga 642

<210> 161
<211> 213
<212> PRT
<213> Glycine max

<400> 161

Met Gln Gln Thr Pro Pro Met Ile Pro Met Met Pro Ser Phe Pro Pro
1 5 10 15

Thr Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Thr Pro Ala Met Pro Pro Gln
65 70 75 80

Met Ala Pro His Pro Ala Met Gln Pro Gly Phe Tyr Met Gln His Pro
85 90 95

Gln Ala Ala Ala Ala Ala Met Ala Gln Gln Gln Gln Gly Met Phe Pro
100 105 110

Gln Lys Met Pro Leu Gln Phe Gly Asn Pro His Gln Met Gln Glu Gln
115 120 125

Gln Gln Gln Leu His Gln Gln Ala Ile Gln Gly Gln Met Gly Leu Arg
130 135 140

Pro Gly Asp Ile Asn Asn Gly Met His Pro Met His Ser Glu Ala Ala
145 150 155 160

Leu Gly Gly Gly Asn Ser Gly Gly Pro Pro Ser Ala Thr Gly Pro Asn
165 170 175

Asp Ala Arg Gly Gly Ser Lys Gln Asp Ala Ser Glu Ala Gly Thr Ala
180 185 190

Gly Gly Asp Gly Gln Gly Ser Ser Ala Ala Ala His Asn Ser Gly Asp
195 200 205

Gly Glu Glu Ala Lys
210

<210> 162
<211> 633
<212> DNA
<213> Glycine max

<400> 162

atgcagcaga caccgcctat gattcctatg atgccttcgt tcccacctac gaacataacc 60
accgagcaga ttcaaaaata ccttgatgag aacaagaagc tgattctggc aatattggac 120
aatcaaaatc ttggaaaact tgcagaatgt gcccagtacc aagctcagct tcaaaagaat 180
ttgatgtatt tagctgcaat tgctgatgcc cagcctcaaa caccagccat gcctccacag 240
atggcaccac accctgccat gcaaccagga ttctatatgc aacatcctca ggctgcagca 300
gcagcaatgg ctcagcagca gcagcaagga atgttccccc agaaaatgcc attgcaattt 360
ggcaatccac atcaaatgca ggaacaacag cagcagctac accagcaagc catccaaggt 420
caaatgggac tgagacctgg aggaataaat aatggcatgc atccaatgca caatgagggc 480
ggcaacagcg gtggtccacc ctcggctacc ggtccgaacg acgcacgtgg tggaagcaag 540
caagatgctt ctgaggctgg aacagctggt ggagatggcc aaggcagctc tgcagctgct 600
cataacagtg gagatggtga agaggcaaag tga 633

<210> 163
<211> 210
<212> PRT
<213> Glycine max

<400> 163

```
Met Gln Gln Thr Pro Pro Met Ile Pro Met Met Pro Ser Phe Pro Pro
1               5                   10                  15

Thr Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Gln Leu Gln Lys Asn Leu Met Tyr Leu
    50                  55                  60

Ala Ala Ile Ala Asp Ala Gln Pro Gln Thr Pro Ala Met Pro Pro Gln
65                  70                  75                  80

Met Ala Pro His Pro Ala Met Gln Pro Gly Phe Tyr Met Gln His Pro
                85                  90                  95

Gln Ala Ala Ala Ala Ala Met Ala Gln Gln Gln Gln Gln Gly Met Phe
            100                 105                 110

Pro Gln Lys Met Pro Leu Gln Phe Gly Asn Pro His Gln Met Gln Glu
        115                 120                 125

Gln Gln Gln Gln Leu His Gln Gln Ala Ile Gln Gly Gln Met Gly Leu
    130                 135                 140

Arg Pro Gly Gly Ile Asn Asn Gly Met His Pro Met His Asn Glu Gly
145                 150                 155                 160

Gly Asn Ser Gly Gly Pro Pro Ser Ala Thr Gly Pro Asn Asp Ala Arg
                165                 170                 175

Gly Gly Ser Lys Gln Asp Ala Ser Glu Ala Gly Thr Ala Gly Gly Asp
            180                 185                 190

Gly Gln Gly Ser Ser Ala Ala Ala His Asn Ser Gly Asp Gly Glu Glu
        195                 200                 205

Ala Lys
    210
```

<210> 164
<211> 633
<212> DNA
<213> Glycine soya

<400> 164

atgcagcaga caccgcctat gattcctatg atgccttcgt tcccacctac gaacataacc 60
accgagcaga ttcaaaaata ccttgatgag aacaagaagc tgattctggc aatattggac 120
aatcaaaatc ttggaaaact tgcagaatgt gcccagtacc aagctcagct tcaaaagaat 180
ttgatgtatt tagctgcaat tgctgatgcc cagcctcaaa caccagccat gcctccacag 240
atggcaccac accctgccat gcaaccagga ttctatatgc aacatcctca ggctgcagca 300
gcagcaatgg ctcagcagca gcagcaagga atgttccccc agaaaatgcc attgcaattt 360
ggcaatccac atcaaatgca ggaacaacag cagcagctac accagcaagc catccaaggt 420
caaatgggac tgagacctgg aggaataaat aatggcatgc atccaatgca caatgagggc 480
ggcaacagcg gtggtccacc ctcggctacc ggtccgaacg acgcacgtgg tggaagcaag 540
caagatgctt ctgaggctgg aacagctggt ggagatggcc aaggcagctc tgcagctgct 600
cataacagtg gagatggtga agaggcaaag tga 633

<210> 165
<211> 210
<212> PRT
<213> Glycine soya

<400> 165

Met Gln Gln Thr Pro Pro Met Ile Pro Met Met Pro Ser Phe Pro Pro
1 5 10 15

Thr Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Thr Pro Ala Met Pro Pro Gln
65 70 75 80

Met Ala Pro His Pro Ala Met Gln Pro Gly Phe Tyr Met Gln His Pro
85 90 95

Gln Ala Ala Ala Ala Ala Met Ala Gln Gln Gln Gln Gln Gly Met Phe
100 105 110

Pro Gln Lys Met Pro Leu Gln Phe Gly Asn Pro His Gln Met Gln Glu
115 120 125

Gln Gln Gln Gln Leu His Gln Gln Ala Ile Gln Gly Gln Met Gly Leu
130 135 140

Arg Pro Gly Gly Ile Asn Asn Gly Met His Pro Met His Asn Glu Gly
145 150 155 160

Gly Asn Ser Gly Gly Pro Pro Ser Ala Thr Gly Pro Asn Asp Ala Arg
165 170 175

Gly Gly Ser Lys Gln Asp Ala Ser Glu Ala Gly Thr Ala Gly Gly Asp
180 185 190

Gly Gln Gly Ser Ser Ala Ala Ala His Asn Ser Gly Asp Gly Glu Glu
195 200 205

Ala Lys
210

<210> 166
<211> 660
<212> DNA
<213> Gossypium arboreum

<220>
<221> misc_feature
<222> (309)..(309)
<223> n is a, c, g, or t

<400> 166

```
atgcagcagc acctgatgca gatgcagccc atgatggcag cttattatcc caacaacgtc      60
actactgatc atattcaaca gtatctcgat gagaacaagt cattgatctt aaagattgtt     120
gagagccaga attctgggaa attgagtgaa tgtgctgaga accaagcaag gctgcagcga     180
aacctcatgt acctggctgc cattgcggat tctcaacccc aaccacccac cgtgcatgca     240
cagtttccat ctggtggtat catgcagcaa ggagctgggc actacatgca gcaccaacaa     300
gctcaacana tgacacaaca gtcgcttatg gctgctcggt cctcaatgtt gtattctcag     360
caaccatttt ctgcactgca acaacaacaa caacaaggct ttgcacagtc agcttggcat     420
gagctctggc gggagcacag gcctttcata tgctgcaaac tgaatctagt actgcagggg     480
gcagtgagac accttgggcc cgagggttgt cctgatttgg acgggggtct tttggagagg     540
catccctggt ggcaggccaa tggccggggg aacaaccaaa aatccgggga ggccggctca     600
cctaagggcc gggaggagcc cttggggcag gggggggtga tgggggggaac ctcttcttaa    660
```

<210> 167
<211> 219
<212> PRT
<213> Gossypium arboreum

<220>
<221> UNSURE
<222> (103)..(103)
<223> Xaa can be any naturally ocurring amino acid

<400> 167.

```
Met Gln Gln His Leu Met Gln Met Gln Pro Met Met Ala Ala Tyr Tyr
1               5                   10                  15
```

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Val His Ala
65 70 75 80

Gln Phe Pro Ser Gly Gly Ile Met Gln Gln Gly Ala Gly His Tyr Met
85 90 95

Gln His Gln Gln Ala Gln Xaa Met Thr Gln Gln Ser Leu Met Ala Ala
100 105 110

Arg Ser Ser Met Leu Tyr Ser Gln Gln Pro Phe Ser Ala Leu Gln Gln
115 120 125

Gln Gln Gln Gln Gly Phe Ala Gln Ser Ala Trp His Glu Leu Trp Arg
130 135 140

Glu His Arg Pro Phe Ile Cys Cys Lys Leu Asn Leu Val Leu Gln Gly
145 150 155 160

Ala Val Arg His Leu Gly Pro Glu Gly Cys Pro Asp Leu Asp Gly Gly
165 170 175

Leu Leu Glu Arg His Pro Trp Trp Gln Ala Asn Gly Arg Gly Asn Asn
180 185 190

Gln Lys Ser Gly Glu Ala Gly Ser Pro Lys Gly Arg Glu Glu Pro Leu
195 200 205

Gly Gln Gly Gly Val Met Gly Gly Thr Ser Ser
210 215

<210> 168
<211> 690
<212> DNA
<213> Gossypium hirsutum

<400> 168

atgcagcagc acctgatgca gatgcagccc atgatggcag cttattatcc caacaacgtc 60
actactgatc atattcaaca gtatctcgat gagaacaagt cattgatctt aaagattgtt 120
gagagccaga attctgggaa attgagtgaa tgtgctgaga accaagcaag gctgcagcga 180
aacctcatgt acctggctgc cattgcggat tctcaacccc aaccacccac cgtgcatgca 240
cagtttccat ctggtggtat catgcagcca ggagctgggc actacatgca gcaccaacaa 300

gctcaacaaa tgacacaaca gtcgcttatg gctgctcggt cctcaatgtt gtattctcag 360
caaccatttt ctgcactgca acaacaacag cagcaagctt tgcacagtca gcttggcatg 420
agctctggcg gaagcacagg ccttcatatg ctgcaaactg aatctagtac tgcaggtggc 480
agtggagcac ttggggccgg agggtttcct gattttggac gtggttcttc tggagaaggc 540
atccatggtg gcaggccaat ggcaggtgga agcaagcaag atatcgggag tgccggctca 600
gctgaaggtc gtggaggaag ctctggtggt cagggtggtg gtgatggggg tgaaaccctt 660
tacttaaaag cagccgatga tgggaactga 690

<210> 169
<211> 229
<212> PRT
<213> Gossypium hirsutum

<400> 169

Met Gln Gln His Leu Met Gln Met Gln Pro Met Met Ala Ala Tyr Tyr
1 5 10 15

Pro Asn Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Pro Thr Val His Ala
65 70 75 80

Gln Phe Pro Ser Gly Gly Ile Met Gln Pro Gly Ala Gly His Tyr Met
85 90 95

Gln His Gln Gln Ala Gln Gln Met Thr Gln Gln Ser Leu Met Ala Ala
100 105 110

Arg Ser Ser Met Leu Tyr Ser Gln Gln Pro Phe Ser Ala Leu Gln Gln
115 120 125

Gln Gln Gln Gln Ala Leu His Ser Gln Leu Gly Met Ser Ser Gly Gly
130 135 140

Ser Thr Gly Leu His Met Leu Gln Thr Glu Ser Ser Thr Ala Gly Gly
145 150 155 160

Ser Gly Ala Leu Gly Ala Gly Gly Phe Pro Asp Phe Gly Arg Gly Ser
165 170 175

Ser Gly Glu Gly Ile His Gly Gly Arg Pro Met Ala Gly Gly Ser Lys
180 185 190

Gln Asp Ile Gly Ser Ala Gly Ser Ala Glu Gly Arg Gly Gly Ser Ser
195 200 205

Gly Gly Gln Gly Gly Gly Asp Gly Gly Glu Thr Leu Tyr Leu Lys Ala
210 215 220

Ala Asp Asp Gly Asn
225

<210> 170
<211> 642
<212> DNA
<213> Gossypium hirsutum

<400> 170

atgccgcagc caccgcaaat gattcctgtg atgccttcat atccacctac taatatcact 60
actgaacaga ttcagaagta ccttgatgag aataagaagt tgattttggc aattttggac 120
aatcagaatc ttggaaaact cgctgaatgc gcccagtatc aagctcagct gcaaaagaat 180
ttgatgtatt tagctgcaat tgcggatgct caacctcaat caacgccagc aatgtcgcct 240
cagatggcac cgcatccagc aatgcaaccc ggaggatatt ttatgcaaca tcctcaagct 300
gctgcaatgt cacagcaacc tggcatgtac cctcaaaagg tgccattgca attcaatagt 360
ccgcatcaaa tgcaggaccc tcagcacctc ctatatcagc agcatcaaca agcaatgcaa 420
ggtcaaatgg gaatcaggcc tgggggaccc aataatagca tgcatcccat gcattcagag 480
gctagccttg gaggcggcag cagtggtggt ccccctcaac cttcaggccc aagtgatgga 540
cgtgctggaa acaagcaaga gggctccgaa gctggtggta atgggcaggg cagcacaact 600
ggtgggcatg gtggcggtga tggagcggat gaggcaaagt ga 642

<210> 171
<211> 213
<212> PRT
<213> Gossypium hirsutum

<400> 171

Met Pro Gln Pro Pro Gln Met Ile Pro Val Met Pro Ser Tyr Pro Pro
1 5 10 15

Thr Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Ser Thr Pro Ala Met Ser Pro
65 70 75 80

Gln Met Ala Pro His Pro Ala Met Gln Pro Gly Gly Tyr Phe Met Gln
85 90 95

His Pro Gln Ala Ala Ala Met Ser Gln Gln Pro Gly Met Tyr Pro Gln
100 105 110

Lys Val Pro Leu Gln Phe Asn Ser Pro His Gln Met Gln Asp Pro Gln
115 120 125

His Leu Leu Tyr Gln Gln His Gln Gln Ala Met Gln Gly Gln Met Gly
130 135 140

Ile Arg Pro Gly Gly Pro Asn Asn Ser Met His Pro Met His Ser Glu
145 150 155 160

Ala Ser Leu Gly Gly Gly Ser Ser Gly Gly Pro Pro Gln Pro Ser Gly
165 170 175

Pro Ser Asp Gly Arg Ala Gly Asn Lys Gln Glu Gly Ser Glu Ala Gly
180 185 190

Gly Asn Gly Gln Gly Ser Thr Thr Gly Gly His Gly Gly Gly Asp Gly
195 200 205

Ala Asp Glu Ala Lys
210

<210> 172
<211> 630
<212> DNA
<213> Helianthus annuus

<400> 172

atgcagcagc acctgatgca gatgcagccc atgatggcag cctattatcc caccaacaac 60
gtcactactg atcatattca acagtacttg gatgaaaaca agtctctgat cttgaagatt 120
gttgagagcc aaaactctgg gaaaatggct gaatgtgcag aacatcaggc caagcttcag 180
agaaacctta tgtaccttgc tgcaattgct gattctcaac ctcaagcacc tagtcttcac 240
tctcagtatc ctcaaggtgg gatgatgcag cagcaggctg gaagtcacta catgcagcag 300
caccaacagg cacaacagat gtcaccacaa gcactcatgg ctgcacgctc atccatgatg 360
tacagtcagc agcagtactc ttcactacag cagcaagcaa tgcatagcca tctgggcatg 420
agttctggaa ctggaaccag tggacttcac atgctgcaga ccgacaataa tagcgcgggt 480
gtcagtggga cccacctaag tggtgggttc cccgactttg gtcgtaagca agatattggt 540
cccaccggtg aggggcgggg tggtggtagc tctggcggtg gagacggtgg cgagacgctc 600
tacttgaagt cgcctgataa aggtaactga 630

<210> 173
<211> 209

<212> PRT
<213> Helianthus annuus

<400> 173

```
Met Gln Gln His Leu Met Gln Met Gln Pro Met Met Ala Ala Tyr Tyr
1               5                   10                  15

Pro Thr Asn Asn Val Thr Thr Asp His Ile Gln Gln Tyr Leu Asp Glu
            20                  25                  30

Asn Lys Ser Leu Ile Leu Lys Ile Val Glu Ser Gln Asn Ser Gly Lys
        35                  40                  45

Met Ala Glu Cys Ala Glu His Gln Ala Lys Leu Gln Arg Asn Leu Met
    50                  55                  60

Tyr Leu Ala Ala Ile Ala Asp Ser Gln Pro Gln Ala Pro Ser Leu His
65                  70                  75                  80

Ser Gln Tyr Pro Gln Gly Gly Met Met Gln Gln Gln Ala Gly Ser His
                85                  90                  95

Tyr Met Gln Gln His Gln Gln Ala Gln Gln Met Ser Pro Gln Ala Leu
            100                 105                 110

Met Ala Ala Arg Ser Ser Met Met Tyr Ser Gln Gln Gln Tyr Ser Ser
        115                 120                 125

Leu Gln Gln Gln Ala Met His Ser His Leu Gly Met Ser Ser Gly Thr
    130                 135                 140

Gly Thr Ser Gly Leu His Met Leu Gln Thr Asp Asn Asn Ser Ala Gly
145                 150                 155                 160

Val Ser Gly Thr His Leu Ser Gly Gly Phe Pro Asp Phe Gly Arg Lys
                165                 170                 175

Gln Asp Ile Gly Pro Thr Gly Glu Gly Arg Gly Gly Gly Ser Ser Gly
            180                 185                 190

Gly Gly Asp Gly Gly Glu Thr Leu Tyr Leu Lys Ser Pro Asp Lys Gly
        195                 200                 205

Asn
```

<210> 174
<211> 561
<212> DNA
<213> Hordeum vulgare

<400> 174

atgcagcaag cgatgcccat gccgccggcg gcggcggcgc ctgggatgcc tccttctgcc 60
ggcctcagca ccgagcagat ccaaaagtac ctggatgaaa ataaacaact aattttggct 120

atcttggaaa atcagaacct gggaaagttg gcggaatgtg ctcagtatca agctcagctt 180
cagaagaatc ttttgtattt ggctgcgatt gctgatactc agccacagac ctctgtaagc 240
cgtcctcaga tggcaccacc tgctgcatcc ccaggggcag ggcattacat gtcacaggtg 300
ccaatgttcc ctccgaggac ccctctaacg cctcagcaga tgcaggagca gcaactacag 360
caacaacagg ctcagatgct tccgtttgct ggtcaaatgg ttgcgagacc cggggctgtc 420
aatggcattc cccaggcccc tcaagttgaa caaccagcct atgcagcagg tggggccagt 480
tccgagcctt ctggcaccga gagccacagg agcactggcg ccgataacga tggtgggagc 540
ggcttggctg accagtccta a 561

<210> 175
<211> 186
<212> PRT
<213> Hordeum vulgare

<400> 175

Met Gln Gln Ala Met Pro Met Pro Pro Ala Ala Ala Ala Pro Gly Met
1               5                   10                  15

Pro Pro Ser Ala Gly Leu Ser Thr Glu Gln Ile Gln Lys Tyr Leu Asp
            20                  25                  30

Glu Asn Lys Gln Leu Ile Leu Ala Ile Leu Glu Asn Gln Asn Leu Gly
        35                  40                  45

Lys Leu Ala Glu Cys Ala Gln Tyr Gln Ala Gln Leu Gln Lys Asn Leu
    50                  55                  60

Leu Tyr Leu Ala Ala Ile Ala Asp Thr Gln Pro Gln Thr Ser Val Ser
65                  70                  75                  80

Arg Pro Gln Met Ala Pro Pro Ala Ala Ser Pro Gly Ala Gly His Tyr
                85                  90                  95

Met Ser Gln Val Pro Met Phe Pro Pro Arg Thr Pro Leu Thr Pro Gln
            100                 105                 110

Gln Met Gln Glu Gln Gln Leu Gln Gln Gln Gln Ala Gln Met Leu Pro
        115                 120                 125

Phe Ala Gly Gln Met Val Ala Arg Pro Gly Ala Val Asn Gly Ile Pro
    130                 135                 140

Gln Ala Pro Gln Val Glu Gln Pro Ala Tyr Ala Ala Gly Gly Ala Ser
145                 150                 155                 160

Ser Glu Pro Ser Gly Thr Glu Ser His Arg Ser Thr Gly Ala Asp Asn
                165                 170                 175

Asp Gly Gly Ser Gly Leu Ala Asp Gln Ser

180 185

<210> 176
<211> 555
<212> DNA
<213> Lactuca serriola

<220>
<221> misc_feature
<222> (253)..(253)
<223> n is a, c, g, or t

<400> 176

atgaagcagc cgatgatgcc gaatccaatg atgtcttctt cgtttcctcc tacaaacatc 60

accaccgatc agatccaaaa gttccttgat gaaaacaagc aactaattat agcaataatg 120

agcaacctaa atcttggaaa gcttgctgaa tgtgcccagt accaagctct actccaaaaa 180

aatttgatgt atctagcagc cattgcagat gctcaaccac ctacacctac accaacacta 240

aatatctctt atnagatggg cccggttcca catccaggga tgccacagca aggtggattt 300

tacatggcgc agcagcaccc tcaggcggct gtaatgacgg ctcagccacc ttctggtttt 360

ccacaaccga tgcctggtat gcaatttaac agcccacagg ctattcaagg gcagatgggc 420

gggaggtccg gtgggccgcc aagctcagcc gctagtgatg tctggagagg aagcatgcaa 480

gatggtggtg gtggtgctgc tgctgatggt ggtaaggatg gtcatgctgg cggtggacct 540

gaggaagcaa agtaa 555

<210> 177
<211> 184
<212> PRT
<213> Lactuca serriola

<220>
<221> UNSURE
<222> (85)..(85)
<223> Xaa can be any naturally ocurring amino acid

<400> 177

Met Lys Gln Pro Met Met Pro Asn Pro Met Met Ser Ser Ser Phe Pro
1 5 10 15

Pro Thr Asn Ile Thr Thr Asp Gln Ile Gln Lys Phe Leu Asp Glu Asn
20 25 30

Lys Gln Leu Ile Ile Ala Ile Met Ser Asn Leu Asn Leu Gly Lys Leu
35 40 45

Ala Glu Cys Ala Gln Tyr Gln Ala Leu Leu Gln Lys Asn Leu Met Tyr
50 55 60

Leu Ala Ala Ile Ala Asp Ala Gln Pro Pro Thr Pro Thr Pro Thr Leu
65 70 75 80

Asn Ile Ser Tyr Xaa Met Gly Pro Val Pro His Pro Gly Met Pro Gln
85 90 95

Gln Gly Gly Phe Tyr Met Ala Gln Gln His Pro Gln Ala Ala Val Met
100 105 110

Thr Ala Gln Pro Pro Ser Gly Phe Pro Gln Pro Met Pro Gly Met Gln
115 120 125

Phe Asn Ser Pro Gln Ala Ile Gln Gly Gln Met Gly Gly Arg Ser Gly
130 135 140

Gly Pro Pro Ser Ser Ala Ala Ser Asp Val Trp Arg Gly Ser Met Gln
145 150 155 160

Asp Gly Gly Gly Gly Ala Ala Ala Asp Gly Gly Lys Asp Gly His Ala
165 170 175

Gly Gly Gly Pro Glu Glu Ala Lys
180

<210> 178
<211> 627
<212> DNA
<213> Lycopersicon esculentum

<400> 178

atgcagcagc acctgatgca gatgcagccc atgatggcag cttactatcc aacgaacgtc 60
actactgacc atattcaaca gtatttggat gaaaacaaat cactcattct gaagattgtt 120
gagagccaga actctgggaa actcagtgaa tgtgcggaga accaagctag gcttcagagg 180
aatctgatgt accttgctgc gattgctgat tcacaacctc aaccttctag catgcattct 240
cagttctctt ctggtgggat gatgcagcca gggacacaca gttacttgca gcagcagcag 300
cagcaacaac aagcgcaaca aatggcaaca caacaactca tggctgcaag atcctcgtcg 360
atgctctatg gacaacagca gcagcaatct cagttatcgc aatatcaaca aggcttgcat 420
agtagccaac tcggcatgag ttctggcagt ggcggaagca ctggacttca tcacatgctt 480
caaagtgaat catcacctca tggtggtggt ttctctcatg acttcggccg cgcaaataag 540
caagacattg ggagtagtat gtctgctgaa gggcgcggcg gaagttcagg tggtgagaat 600
ctttatctga aagcttctga ggattga 627

<210> 179
<211> 208
<212> PRT
<213> Lycopersicon esculentum

<400> 179

Met Gln Gln His Leu Met Gln Met Gln Pro Met Met Ala Ala Tyr Tyr
1 5 10 15

Pro Thr Asn Val Thr Thr Asp His Ile Gln Gln Tyr Leu Asp Glu Asn

20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Ser Ser Met His Ser
65 70 75 80

Gln Phe Ser Ser Gly Gly Met Met Gln Pro Gly Thr His Ser Tyr Leu
85 90 95

Gln Gln Gln Gln Gln Gln Gln Gln Ala Gln Gln Met Ala Thr Gln Gln
100 105 110

Leu Met Ala Ala Arg Ser Ser Ser Met Leu Tyr Gly Gln Gln Gln Gln
115 120 125

Gln Ser Gln Leu Ser Gln Tyr Gln Gln Gly Leu His Ser Ser Gln Leu
130 135 140

Gly Met Ser Ser Gly Ser Gly Gly Ser Thr Gly Leu His His Met Leu
145 150 155 160

Gln Ser Glu Ser Ser Pro His Gly Gly Gly Phe Ser His Asp Phe Gly
165 170 175

Arg Ala Asn Lys Gln Asp Ile Gly Ser Ser Met Ser Ala Glu Gly Arg
180 185 190

Gly Gly Ser Ser Gly Gly Glu Asn Leu Tyr Leu Lys Ala Ser Glu Asp
195 200 205

<210> 180
<211> 624
<212> DNA
<213> Malus domestica

<400> 180

atgcagcagc caccacaaat gatccccgtc atgccttcat ttcctcccac caacatcacc 60
accgaacaaa ttcagaagta ccttgatgac aacaaaaagt tgattctggc aatattggat 120
aatcaaaatc ttggaaaact tgctgagtgt gctcagtacc aggctctgct tcaaaagaat 180
ctgatgtatt tagcagcaat tgccgatgcg caaccacagg caccagctgc ccctccccag 240
atggccccac atcctgctat gcaacaggca ggatattaca tgcaacatcc tcaggcagca 300
gcaatggctc agcaacaggg tattttctcc ccaaagatgc cgatgcaatt caataacatg 360
catcaaatgc acgatccaca gcagcaccaa caagccatgc aagggcaaat gggaatgaga 420
cctggagggc ctaacggcat gccttccatg cttcatactg aggccacaca tggtggtggt 480

agtggcggcc caaattcagc tggagaccca aatgatgggc gtggaggaag caagcaagac 540
gcctctgagt ctggggcagg tggtgatggc caggggacct cagccggcgg gcgtggaact 600
ggtgatggag aggacggcaa gtga 624

<210> 181
<211> 207
<212> PRT
<213> Malus domestica

<400> 181

```
Met Gln Gln Pro Pro Gln Met Ile Pro Val Met Pro Ser Phe Pro Pro
1               5                   10                  15

Thr Asn Ile Thr Thr Glu Gln Ile Gln Lys Tyr Leu Asp Asp Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Leu Leu Gln Lys Asn Leu Met Tyr Leu
    50                  55                  60

Ala Ala Ile Ala Asp Ala Gln Pro Gln Ala Pro Ala Ala Pro Pro Gln
65                  70                  75                  80

Met Ala Pro His Pro Ala Met Gln Gln Ala Gly Tyr Tyr Met Gln His
                85                  90                  95

Pro Gln Ala Ala Ala Met Ala Gln Gln Gln Gly Ile Phe Ser Pro Lys
            100                 105                 110

Met Pro Met Gln Phe Asn Asn Met His Gln Met His Asp Pro Gln Gln
        115                 120                 125

His Gln Gln Ala Met Gln Gly Gln Met Gly Met Arg Pro Gly Gly Pro
    130                 135                 140

Asn Gly Met Pro Ser Met Leu His Thr Glu Ala Thr His Gly Gly Gly
145                 150                 155                 160

Ser Gly Gly Pro Asn Ser Ala Gly Asp Pro Asn Asp Gly Arg Gly Gly
                165                 170                 175

Ser Lys Gln Asp Ala Ser Glu Ser Gly Ala Gly Gly Asp Gly Gln Gly
            180                 185                 190

Thr Ser Ala Gly Gly Arg Gly Thr Gly Asp Gly Glu Asp Gly Lys
        195                 200                 205
```

<210> 182
<211> 636
<212> DNA
<213> Medicago trunculata

<400> 182

```
atgcagcagc acctgatgca gatgcagccc atgatggcag cttactatcc taacaacgtc     60
actactgatc atattcaaca gtatcttgat gagaacaagt ccttgattct caagattgtt    120
gaaagccaga acactggcaa gctcaccgag tgtgctgaga accaatcaag gcttcagaga    180
aatctcatgt acctagctgc aatagctgat tctcaacccc aaccacctac tatgcctggc    240
cagtaccctt caagtggaat gatgcagcag ggaggacact acatgcaggc tcaacaagct    300
cagcagatga cacaacaaca attaatggct gcacgttcct ctcttatgta tgctcaacag    360
cttcaacagc agcaagcctt gcaaagccaa cttggtatga attccagtgg aagtcaaggc    420
cttcacatgt tgcatagtga aggggctaat gttggaggca attcatctct aggggctggt    480
tttcctgatt ttggccgtag ctcagccggt gatggtttgc acggcagtgg taagcaagac    540
attggaagca ctgatggccg cggtggaagc tctagtggtc actctggtga tggcggcgaa    600
acactttacc tgaaatcttc tggtgatggg aattag                               636
```

<210> 183
<211> 211
<212> PRT
<213> Medicago trunculata

<400> 183

```
Met Gln Gln His Leu Met Gln Met Gln Pro Met Met Ala Ala Tyr Tyr
1               5                   10                  15

Pro Asn Asn Val Thr Thr Asp His Ile Gln Gln Tyr Leu Asp Glu Asn
            20                  25                  30

Lys Ser Leu Ile Leu Lys Ile Val Glu Ser Gln Asn Thr Gly Lys Leu
        35                  40                  45

Thr Glu Cys Ala Glu Asn Gln Ser Arg Leu Gln Arg Asn Leu Met Tyr
    50                  55                  60

Leu Ala Ala Ile Ala Asp Ser Gln Pro Gln Pro Pro Thr Met Pro Gly
65                  70                  75                  80

Gln Tyr Pro Ser Ser Gly Met Met Gln Gln Gly Gly His Tyr Met Gln
                85                  90                  95

Ala Gln Gln Ala Gln Gln Met Thr Gln Gln Gln Leu Met Ala Ala Arg
            100                 105                 110

Ser Ser Leu Met Tyr Ala Gln Gln Leu Gln Gln Gln Gln Ala Leu Gln
        115                 120                 125

Ser Gln Leu Gly Met Asn Ser Ser Gly Ser Gln Gly Leu His Met Leu
    130                 135                 140
```

His Ser Glu Gly Ala Asn Val Gly Gly Asn Ser Ser Leu Gly Ala Gly
145 150 155 160

Phe Pro Asp Phe Gly Arg Ser Ser Ala Gly Asp Gly Leu His Gly Ser
165 170 175

Gly Lys Gln Asp Ile Gly Ser Thr Asp Gly Arg Gly Gly Ser Ser Ser
180 185 190

Gly His Ser Gly Asp Gly Gly Glu Thr Leu Tyr Leu Lys Ser Ser Gly
195 200 205

Asp Gly Asn
210

<210> 184
<211> 639
<212> DNA
<213> Medicago trunculata

<400> 184

```
atgcagcaga cacctcaaat gattcctatg atgccttcat tcccacaaca aacaaacata      60
accactgagc agattcaaaa atatcttgat gagaacaaga agctgatcct ggcaatattg     120
gacaatcaaa atcttggaaa acttgcagaa tgtgcccagt accaagctca gcttcagaag     180
aatttgatgt atttagctgc aattgctgac gcgcagccac aaacaccggc cttgcctcca     240
cagatggccc cgcaccctgc gatgcaacaa ggattctata tgcaacatcc tcaggctgca     300
gcaatggctc agcaacaagg aatgttcccc caaaaaatgc caatgcagtt cggtaatccg     360
catcaaatgc aggatcagca gcatcagcag caacaacagc agctacatca gcaagctatg     420
caaggtcaaa tgggacttag acctggaggg ataaataacg gcatgcatcc aatgcacaac     480
gaggctgctc tcggaggtag cggcagtggt ggtcaaatga cgggcgtggt ggtggagcaa     540
gcaagatgct tcggagctgg gacagccggc ggtgatggtc aaggaacctc tgccgcagct     600
gcgcacaaca gtggagatgc ttcagaagaa ggaaagtaa                            639
```

<210> 185
<211> 213
<212> PRT
<213> Medicago trunculata

<400> 185

Met Gln Gln Thr Pro Gln Met Ile Pro Met Met Pro Ser Phe Pro Gln
1 5 10 15

Gln Thr Asn Ile Thr Thr Glu Gln Ile Gln Lys Tyr Leu Asp Glu Asn
20 25 30

Lys Lys Leu Ile Leu Ala Ile Leu Asp Asn Gln Asn Leu Gly Lys Leu
35 40 45

Ala Glu Cys Ala Gln Tyr Gln Ala Gln Leu Gln Lys Asn Leu Met Tyr

50 55 60

Leu Ala Ala Ile Ala Asp Ala Gln Pro Gln Thr Pro Ala Leu Pro Pro
65 70 75 80

Gln Met Ala Pro His Pro Ala Met Gln Gln Gly Phe Tyr Met Gln His
85 90 95

Pro Gln Ala Ala Ala Met Ala Gln Gln Gln Gly Met Phe Pro Gln Lys
100 105 110

Met Pro Met Gln Phe Gly Asn Pro His Gln Met Gln Asp Gln Gln His
115 120 125

Gln Gln Gln Gln Gln Gln Leu His Gln Gln Ala Met Gln Gly Gln Met
130 135 140

Gly Leu Arg Pro Gly Gly Ile Asn Asn Gly Met His Pro Met His Asn
145 150 155 160

Glu Ala Ala Leu Gly Gly Ser Gly Ser Gly Gly Pro Asn Asp Gly Arg
165 170 175

Gly Gly Gly Ser Lys Gln Asp Ala Ser Glu Ala Gly Thr Ala Gly Gly
180 185 190

Asp Gly Gln Gly Thr Ser Ala Ala Ala Ala His Asn Ser Gly Asp Ala
195 200 205

Ser Glu Glu Gly Lys
210

<210> 186
<211> 684
<212> DNA
<213> Oryza sativa

<400> 186

atgcagcagc aacacctgat gcagatgaac cagggcatga tggggggata tgcttcccct 60
accaccgtca ccactgatct cattcagcag tatctggatg agaacaagca gctgatcctg 120
gccatccttg acaaccagaa caatgggaag gtggaagagt gcgctcggaa ccaagctaag 180
ctccagcaca atctcatgta cctcgccgcc atcgccgaca gccagccgcc gcagacggcc 240
gccatgtccc agtatccgtc gaacctgatg atgcagtccg gggcgaggta catgccgcag 300
cagtcggcgc agatgatggc gccgcagtcg ctgatggcgg cgaggtcttc gatgatgtac 360
gcgcagccgg cgctgtcgcc gctccagcag cagcagcagc agcaggcggc ggcggcgcac 420
gggcagctgg gcatgggctc gggggcaccc accagcgggt tcagcatcct ccacggcgag 480
gccagcatgg gcggcggcgg cggcggcggt ggcgccggta acagcatgat gaacgccggc 540
gtgttctccg acttcggacg cggcggcggc ggcggcggca aggaggggtc cacctcgctg 600
tccgtcgacg tccggggcgc caactccggc gcccagagcg gcgacgggga gtacctcaag 660
ggcaccgagg aggaaggcag ctag 684

<210> 187
<211> 227
<212> PRT
<213> Oryza sativa

<400> 187

Met Gln Gln Gln His Leu Met Gln Met Asn Gln Gly Met Met Gly Gly
1 5 10 15

Tyr Ala Ser Pro Thr Thr Val Thr Thr Asp Leu Ile Gln Gln Tyr Leu
20 25 30

Asp Glu Asn Lys Gln Leu Ile Leu Ala Ile Leu Asp Asn Gln Asn Asn
35 40 45

Gly Lys Val Glu Glu Cys Ala Arg Asn Gln Ala Lys Leu Gln His Asn
50 55 60

Leu Met Tyr Leu Ala Ala Ile Ala Asp Ser Gln Pro Pro Gln Thr Ala
65 70 75 80

Ala Met Ser Gln Tyr Pro Ser Asn Leu Met Met Gln Ser Gly Ala Arg
85 90 95

Tyr Met Pro Gln Gln Ser Ala Gln Met Met Ala Pro Gln Ser Leu Met
100 105 110

Ala Ala Arg Ser Ser Met Met Tyr Ala Gln Pro Ala Leu Ser Pro Leu
115 120 125

Gln Gln Gln Gln Gln Gln Gln Ala Ala Ala Ala His Gly Gln Leu Gly
130 135 140

Met Gly Ser Gly Gly Thr Thr Ser Gly Phe Ser Ile Leu His Gly Glu
145 150 155 160

Ala Ser Met Gly Gly Gly Gly Gly Gly Gly Gly Ala Gly Asn Ser Met
165 170 175

Met Asn Ala Gly Val Phe Ser Asp Phe Gly Arg Gly Gly Gly Gly Gly
180 185 190

Gly Lys Glu Gly Ser Thr Ser Leu Ser Val Asp Val Arg Gly Ala Asn
195 200 205

Ser Gly Ala Gln Ser Gly Asp Gly Glu Tyr Leu Lys Gly Thr Glu Glu
210 215 220

Glu Gly Ser

225

<210> 188
<211> 558
<212> DNA
<213> Oryza sativa

<400> 188

atgcagcagc agccgatgcc gatgcccgcg caggcgccgc cgacggccgg aatcaccacc 60
gagcagatcc aaaagtatct ggatgaaaac aagcagctta ttttggctat tttggaaaat 120
cagaatctgg gaaagttggc agaatgtgct cagtatcaag cgcagcttca gaagaatctc 180
ttgtacttgg ctgcaattgc tgatactcaa ccgcagacca ctataagccg tccccagatg 240
gtgccgcatg gtgcatcgcc ggggttaggg gggcaataca tgtcgcaggt gccaatgttc 300
ccccccagga cccctctaac gccccagcag atgcaggagc agcagctgca gcaacagcaa 360
gcccagctgc tctcgttcgg cggtcagatg gttatgaggc ctggcgttgt gaatggcatt 420
cctcagcttc tgcaaggcga aatgcaccgc ggagcagatc accagaacgc tggcggggcc 480
acctcggagc cttccgagag ccacaggagc accggcaccg aaaatgacgg tggaagcgac 540
ttcggcgatc aatcctaa 558

<210> 189
<211> 185
<212> PRT
<213> Oryza sativa

<400> 189

Met Gln Gln Gln Pro Met Pro Met Pro Ala Gln Ala Pro Pro Thr Ala
1 5 10 15

Gly Ile Thr Thr Glu Gln Ile Gln Lys Tyr Leu Asp Glu Asn Lys Gln
20 25 30

Leu Ile Leu Ala Ile Leu Glu Asn Gln Asn Leu Gly Lys Leu Ala Glu
35 40 45

Cys Ala Gln Tyr Gln Ala Gln Leu Gln Lys Asn Leu Leu Tyr Leu Ala
50 55 60

Ala Ile Ala Asp Thr Gln Pro Gln Thr Thr Ile Ser Arg Pro Gln Met
65 70 75 80

Val Pro His Gly Ala Ser Pro Gly Leu Gly Gly Gln Tyr Met Ser Gln
85 90 95

Val Pro Met Phe Pro Pro Arg Thr Pro Leu Thr Pro Gln Gln Met Gln
100 105 110

Glu Gln Gln Leu Gln Gln Gln Gln Ala Gln Leu Leu Ser Phe Gly Gly
115 120 125

Gln Met Val Met Arg Pro Gly Val Val Asn Gly Ile Pro Gln Leu Leu
130 135 140

Gln Gly Glu Met His Arg Gly Ala Asp His Gln Asn Ala Gly Gly Ala
145 150 155 160

Thr Ser Glu Pro Ser Glu Ser His Arg Ser Thr Gly Thr Glu Asn Asp
165 170 175

Gly Gly Ser Asp Phe Gly Asp Gln Ser
180 185

<210> 190
<211> 618
<212> DNA
<213> oryza sativa

<400> 190

```
atgcagcagc agatggccat gccggcgggg gccgccgccg ccgcggtgcc gccggcggcc    60
ggcatcacca ccgagcagat ccaaaagtat ttggatgaaa ataaacagct aattttggcc   120
atcctggaaa atcaaaacct agggaagttg gctgaatgtg ctcagtacca agctcagctt   180
caaaagaatc tcttgtatct ggctgccatt gcagatgccc aaccacctca gaatccagga   240
agtcgccctc agatgatgca gcctggtgct accccaggtg ctgggcatta catgtcccaa   300
gtaccgatgt tccctccaag aactccctta accccacaac agatgcaaga gcagcagcag   360
cagcaactcc agcaacagca agctcaggct ctagccttcc ccggccagat gctaatgaga   420
ccaggtactg tcaatggcat gcaatctatc ccagttgctg accctgctcg cgcagccgat   480
cttcagacgg cagcaccggg ctcggtagat ggccgaggaa acaagcagga tgcaacctcg   540
gagccttccg ggaccgagag ccacaagagt gcgggagcag ataacgacgc aggcggtgac   600
atagcggaga agtcctga                                                 618
```

<210> 191
<211> 205
<212> PRT
<213> Oryza sativa

<400> 191

```
Met Gln Gln Gln Met Ala Met Pro Ala Gly Ala Ala Ala Ala Ala Val
1               5                   10                  15

Pro Pro Ala Ala Gly Ile Thr Thr Glu Gln Ile Gln Lys Tyr Leu Asp
            20                  25                  30

Glu Asn Lys Gln Leu Ile Leu Ala Ile Leu Glu Asn Gln Asn Leu Gly
        35                  40                  45

Lys Leu Ala Glu Cys Ala Gln Tyr Gln Ala Gln Leu Gln Lys Asn Leu
    50                  55                  60

Leu Tyr Leu Ala Ala Ile Ala Asp Ala Gln Pro Pro Gln Asn Pro Gly

65                  70                  75                  80

Ser Arg Pro Gln Met Met Gln Pro Gly Ala Thr Pro Gly Ala Gly His
                85                  90                  95

Tyr Met Ser Gln Val Pro Met Phe Pro Pro Arg Thr Pro Leu Thr Pro
            100                 105                 110

Gln Gln Met Gln Glu Gln Gln Gln Gln Gln Leu Gln Gln Gln Gln Ala
        115                 120                 125

Gln Ala Leu Ala Phe Pro Gly Gln Met Leu Met Arg Pro Gly Thr Val
    130                 135                 140

Asn Gly Met Gln Ser Ile Pro Val Ala Asp Pro Ala Arg Ala Ala Asp
145                 150                 155                 160

Leu Gln Thr Ala Ala Pro Gly Ser Val Asp Gly Arg Gly Asn Lys Gln
                165                 170                 175

Asp Ala Thr Ser Glu Pro Ser Gly Thr Glu Ser His Lys Ser Ala Gly
            180                 185                 190

Ala Asp Asn Asp Ala Gly Gly Asp Ile Ala Glu Lys Ser
        195                 200                 205
```

<210> 192
<211> 624
<212> DNA
<213> Panicum virgatum

<400> 192

```
atgcagcagc agatgcccat gcagtcggcg cccccggcga ccggcatcac caccgagcag      60
atccaaaagt atttggatga aaataagcag cttattttgg ccatcctgga aaatcagaac     120
ttaggaaagt tggctgaatg tgctcagtat caagctcagc ttcaaaagaa tctcttgtac     180
ctggctgcga ttgcagatgc ccaaccccaa ccaccacaga accctgcaag tcgcccacag     240
atgatgcaac ctggcatggt accaggtgca gggcattaca tgtcccaagt accaatgttc     300
ccgccaagaa caccattaac cccgcaacag atgcaagaac agcagcagca gcagcagcag     360
cttcaacagc agcaagcaca ggctcttgct ttcccgggac agatggtcat gagacctacc     420
attaatggca tgcagcctat gcaagccgac cctgctgccg ccgccgccag cctacagcag     480
tcagcacctg gccctactga tgggcgagga ggcaagcaag atgcaactgc tggggtgagc     540
acagagcctt ctggcaccga gagccacaag agcacaaccg cagcagatca cgatgtgggc     600
actgatgtcg cggagaaatc ctaa                                            624
```

<210> 193
<211> 207
<212> PRT
<213> Panicum virgatum

<400> 193

Met Gln Gln Gln Met Pro Met Gln Ser Ala Pro Pro Ala Thr Gly Ile
1 5 10 15

Thr Thr Glu Gln Ile Gln Lys Tyr Leu Asp Glu Asn Lys Gln Leu Ile
20 25 30

Leu Ala Ile Leu Glu Asn Gln Asn Leu Gly Lys Leu Ala Glu Cys Ala
35 40 45

Gln Tyr Gln Ala Gln Leu Gln Lys Asn Leu Leu Tyr Leu Ala Ala Ile
50 55 60

Ala Asp Ala Gln Pro Gln Pro Pro Gln Asn Pro Ala Ser Arg Pro Gln
65 70 75 80

Met Met Gln Pro Gly Met Val Pro Gly Ala Gly His Tyr Met Ser Gln
85 90 95

Val Pro Met Phe Pro Pro Arg Thr Pro Leu Thr Pro Gln Gln Met Gln
100 105 110

Glu Gln Gln Gln Gln Gln Gln Gln Leu Gln Gln Gln Gln Ala Gln Ala
115 120 125

Leu Ala Phe Pro Gly Gln Met Val Met Arg Pro Thr Ile Asn Gly Met
130 135 140

Gln Pro Met Gln Ala Asp Pro Ala Ala Ala Ala Ala Ser Leu Gln Gln
145 150 155 160

Ser Ala Pro Gly Pro Thr Asp Gly Arg Gly Gly Lys Gln Asp Ala Thr
165 170 175

Ala Gly Val Ser Thr Glu Pro Ser Gly Thr Glu Ser His Lys Ser Thr
180 185 190

Thr Ala Ala Asp His Asp Val Gly Thr Asp Val Ala Glu Lys Ser
195 200 205

<210> 194
<211> 645
<212> DNA
<213> Physcomitrella patens

<400> 194

atgcagcaaa tggcggcgta tacggggacg tctattacca cggagctaat ccagaagtat 60

ctggacgaga acaagcagct tatcctcgcg attcttgaca atcaaaacct tggaaagctg 120

aacgaatgcg ctatgtatca agcaaagttg cagcagaatc ttatgtacct cgcagccatt 180

gcggatgcac aacctcaagt gagccaaaac tcaactcagg tctcatcagg acaatctatg 240

cagccctctc agcaatatat tcaacagcag cagcagcagc agatgatgat gatgaatcag 300

cgaaactcaa tcccacaata catgcaacaa agccaacagg ggtcaccaaa cgcaccatca 360
ccgcagcagc agtcctacca cagccagcag ccgcaaggta tggttcccca gagaaactca 420
gacatgcact tggtgaaaaa ctctacagga ggcaacggta atcagacagg aggtagtgta 480
tccgagtatg gaaagcctga ggaatcccgg gaggggaccc caacaagctt aagcacaaga 540
aacgatggtc cacaggcggg ggcttctccg ttgggacaag cgagagaagg caatggagct 600
gctggagagg actctgaggc ttcttacttg aaaagctccg actaa 645

<210> 195
<211> 214
<212> PRT
<213> Physcomitrella patens

<400> 195

Met Gln Gln Met Ala Ala Tyr Thr Gly Thr Ser Ile Thr Thr Glu Leu
1 5 10 15

Ile Gln Lys Tyr Leu Asp Glu Asn Lys Gln Leu Ile Leu Ala Ile Leu
20 25 30

Asp Asn Gln Asn Leu Gly Lys Leu Asn Glu Cys Ala Met Tyr Gln Ala
35 40 45

Lys Leu Gln Gln Asn Leu Met Tyr Leu Ala Ala Ile Ala Asp Ala Gln
50 55 60

Pro Gln Val Ser Gln Asn Ser Thr Gln Val Ser Ser Gly Gln Ser Met
65 70 75 80

Gln Pro Ser Gln Gln Tyr Ile Gln Gln Gln Gln Gln Gln Gln Met Met
85 90 95

Met Met Asn Gln Arg Asn Ser Ile Pro Gln Tyr Met Gln Gln Ser Gln
100 105 110

Gln Gly Ser Pro Asn Ala Pro Ser Pro Gln Gln Gln Ser Tyr His Ser
115 120 125

Gln Gln Pro Gln Gly Met Val Pro Gln Arg Asn Ser Asp Met His Leu
130 135 140

Val Lys Asn Ser Thr Gly Gly Asn Gly Asn Gln Thr Gly Gly Ser Val
145 150 155 160

Ser Glu Tyr Gly Lys Pro Glu Glu Ser Arg Glu Gly Thr Pro Thr Ser
165 170 175

Leu Ser Thr Arg Asn Asp Gly Pro Gln Ala Gly Ala Ser Pro Leu Gly
180 185 190

Gln Ala Arg Glu Gly Asn Gly Ala Ala Gly Glu Asp Ser Glu Ala Ser
195 200 205

Tyr Leu Lys Ser Ser Asp
210

<210> 196
<211> 609
<212> DNA
<213> Physcomitrella patens

<400> 196

atgcagcaaa tggcgccgta tgcggggaca tctatcacta ctgagctcat ccagaagtac 60
ctggacgaga acaagcagct gattctcgca attctcgata atcaaaacct tggaaagctg 120
aacgaatgtg ctacgtatca agcaaagttg cagcagaatc ttatgtacct cgcagctatt 180
gcagacgcac aaccccaagt tccagcagca caaccaatgc aaccatcaca gcaatatatt 240
cagcagcagc agcagcagat gatgatgaat caacgaaatc agtacttgca gcaaaatcaa 300
caaggagtac aaaacgcacc atcaccgcag tcgcagcagt cgtatcacaa ccagcagcca 360
ggcatggtct cccagggaaa ctcggggatg cacatggtga atagttccat gggtggtaat 420
ggcaaccaga caggaggaaa tgtttccgag tatgggaaac cagaagattc ccgggagggg 480
actccaacaa gcttgaatac aaggaatgaa ggtccacaag cgggggcttc cccactggga 540
caagcaagag aagggaatgg tgcgccagga gaggattcag aggcttcata cttaaaaagc 600
tccgagtga 609

<210> 197
<211> 202
<212> PRT
<213> Physcomitrella patens

<400> 197

Met Gln Gln Met Ala Pro Tyr Ala Gly Thr Ser Ile Thr Thr Glu Leu
1               5                   10                  15

Ile Gln Lys Tyr Leu Asp Glu Asn Lys Gln Leu Ile Leu Ala Ile Leu
            20                  25                  30

Asp Asn Gln Asn Leu Gly Lys Leu Asn Glu Cys Ala Thr Tyr Gln Ala
        35                  40                  45

Lys Leu Gln Gln Asn Leu Met Tyr Leu Ala Ala Ile Ala Asp Ala Gln
    50                  55                  60

Pro Gln Val Pro Ala Ala Gln Pro Met Gln Pro Ser Gln Gln Tyr Ile
65                  70                  75                  80

Gln Gln Gln Gln Gln Gln Met Met Met Asn Gln Arg Asn Gln Tyr Leu
                85                  90                  95

Gln Gln Asn Gln Gln Gly Val Gln Asn Ala Pro Ser Pro Gln Ser Gln

            100                 105                 110

Gln Ser Tyr His Asn Gln Gln Pro Gly Met Val Ser Gln Gly Asn Ser
        115                 120                 125

Gly Met His Met Val Asn Ser Ser Met Gly Gly Asn Gly Asn Gln Thr
    130                 135                 140

Gly Gly Asn Val Ser Glu Tyr Gly Lys Pro Glu Asp Ser Arg Glu Gly
145                 150                 155                 160

Thr Pro Thr Ser Leu Asn Thr Arg Asn Glu Gly Pro Gln Ala Gly Ala
                165                 170                 175

Ser Pro Leu Gly Gln Ala Arg Glu Gly Asn Gly Ala Pro Gly Glu Asp
            180                 185                 190

Ser Glu Ala Ser Tyr Leu Lys Ser Ser Glu
        195                 200

<210> 198
<211> 654
<212> DNA
<213> Physcomitrella patens

<400> 198

```
atgatgcagc acatggcgac gtatgccagc tccaacatca caacggagct cattcagaag     60
tacttggacg agaataagca gttgattctc gctatcctcg acaaccaaaa cctcggcaag    120
ctcaatgagt gtgcaacgta tcaagcgaag ttgcagcaga atctcatgta tttggctgcc    180
atagctgatg ctcagccaca aggcccatct tcgcagatgc cagcacctgc gccggcgcca    240
accatgcaac cagctcagca gtacatgcaa cagcagcaac aactccggat gatgagccaa    300
cagaacagta tgatcccttc ctaccttcag cacagccaac aagcatcgca gcagaacttc    360
tacagtcaac aaagcctgct ctccggggga gggggctcaa tacacatgat gtccacggac    420
cgcggcatcg gaggcaatgg atcccaggct tcgccaggat attctgatgg agggcgggat    480
cagagccaaa tgggtttggc aatgcagggc gacatgcatg gtggaaacgg gacggacctg    540
gggtgctcct ccccgcttgg ccaccgagga gacggtggta acggccacgg ccaggggacc    600
gatgactctg aagcttccta cttgaaggga tctgatggga gcagtctgaa ttaa          654
```

<210> 199
<211> 217
<212> PRT
<213> Physcomitrella patens

<400> 199

```
Met Met Gln His Met Ala Thr Tyr Ala Ser Ser Asn Ile Thr Thr Glu
1               5                   10                  15

Leu Ile Gln Lys Tyr Leu Asp Glu Asn Lys Gln Leu Ile Leu Ala Ile
            20                  25                  30
```

Leu Asp Asn Gln Asn Leu Gly Lys Leu Asn Glu Cys Ala Thr Tyr Gln
35 40 45

Ala Lys Leu Gln Gln Asn Leu Met Tyr Leu Ala Ala Ile Ala Asp Ala
50 55 60

Gln Pro Gln Gly Pro Ser Ser Gln Met Pro Ala Pro Ala Pro Ala Pro
65 70 75 80

Thr Met Gln Pro Ala Gln Gln Tyr Met Gln Gln Gln Gln Gln Leu Arg
85 90 95

Met Met Ser Gln Gln Asn Ser Met Ile Pro Ser Tyr Leu Gln His Ser
100 105 110

Gln Gln Ala Ser Gln Gln Asn Phe Tyr Ser Gln Gln Ser Leu Leu Ser
115 120 125

Gly Gly Gly Gly Ser Ile His Met Met Ser Thr Asp Arg Gly Ile Gly
130 135 140

Gly Asn Gly Ser Gln Ala Ser Pro Gly Tyr Ser Asp Gly Gly Arg Asp
145 150 155 160

Gln Ser Gln Met Gly Leu Ala Met Gln Gly Asp Met His Gly Gly Asn
165 170 175

Gly Thr Asp Leu Gly Cys Ser Ser Pro Leu Gly His Arg Gly Asp Gly
180 185 190

Gly Asn Gly His Gly Gln Gly Thr Asp Asp Ser Glu Ala Ser Tyr Leu
195 200 205

Lys Gly Ser Asp Gly Ser Ser Leu Asn
210 215

<210> 200
<211> 648
<212> DNA
<213> Physcomitrella patens

<400> 200

atgatgcagc acatgacgac ctatgccagc tccaacatca ccacggagct cattcagaag 60
tacttggacg agaataaaca gctgattctc gccattctcg acaaccaaaa cctcggcaag 120
ctcaatgagt gtgcgacgta tcaagcgaag ctgcagcaga acctcatgta tctggccgct 180
atagctgatg cccagccaca aggcccatct acgcagatgc cagcgcccgc gccaactatg 240
caaccagctc aacaatacat gcaacagcag caacagctcc gcatgatgag ccaacaaaat 300
gccatgatcc cctcctacct gcagcaaagc caacaagttt cccagcagaa cttctacagc 360
caacagagcc tgcttaccgg cggtggcagc tccatccaca tgatgtccac tgatcgcggc 420

atgggaggca atgggtcaca agcctcacct ggatattctg acggagggcg agatcagaac 480

caattgggta tgacgatgca gggcgacatg catggtggaa acggcactga cttgggctgc 540

tcctcacctc tcggccaccg gggagatggc ggtggcggcc acggccaggg caacgacgac 600

tctgaagctt cttacttgaa gggttccgat ggcagcagtc tgaactag 648

<210> 201
<211> 215
<212> PRT
<213> Physcomitrella patens

<400> 201

Met Met Gln His Met Thr Thr Tyr Ala Ser Ser Asn Ile Thr Thr Glu
1 5 10 15

Leu Ile Gln Lys Tyr Leu Asp Glu Asn Lys Gln Leu Ile Leu Ala Ile
20 25 30

Leu Asp Asn Gln Asn Leu Gly Lys Leu Asn Glu Cys Ala Thr Tyr Gln
35 40 45

Ala Lys Leu Gln Gln Asn Leu Met Tyr Leu Ala Ala Ile Ala Asp Ala
50 55 60

Gln Pro Gln Gly Pro Ser Thr Gln Met Pro Ala Pro Ala Pro Thr Met
65 70 75 80

Gln Pro Ala Gln Gln Tyr Met Gln Gln Gln Gln Gln Leu Arg Met Met
85 90 95

Ser Gln Gln Asn Ala Met Ile Pro Ser Tyr Leu Gln Gln Ser Gln Gln
100 105 110

Val Ser Gln Gln Asn Phe Tyr Ser Gln Gln Ser Leu Leu Thr Gly Gly
115 120 125

Gly Ser Ser Ile His Met Met Ser Thr Asp Arg Gly Met Gly Gly Asn
130 135 140

Gly Ser Gln Ala Ser Pro Gly Tyr Ser Asp Gly Gly Arg Asp Gln Asn
145 150 155 160

Gln Leu Gly Met Thr Met Gln Gly Asp Met His Gly Gly Asn Gly Thr
165 170 175

Asp Leu Gly Cys Ser Ser Pro Leu Gly His Arg Gly Asp Gly Gly Gly
180 185 190

Gly His Gly Gln Gly Asn Asp Asp Ser Glu Ala Ser Tyr Leu Lys Gly
195 200 205

Ser Asp Gly Ser Ser Leu Asn
210 215

<210> 202
<211> 747
<212> DNA
<213> Picea sitchensis

<400> 202

atgcagcagc atctcatgca aatgcagccc atgatggcgg catacgcctc caacaacatc 60
accactgatc acatccagaa gtacctggat gagaacaagc agttgattct ggcaattctg 120
gacaaccaaa atcttggaaa gctcaatgag tgtgctcagt accaagcaaa acttcagcag 180
aatttgatgt atctggctgc gattgctgat tctcaaccac aagcacaaac tgcacatgct 240
cagattcctc ctaatgcagt gatgcagtct ggtgggcatt acatgcagca ccagcaggca 300
cagcaacaag tgactcctca gtctctgatg gcagctagat cttccatgct gtattctcag 360
cagccgatgg ctgctttgca tcaagctcag caacaacagc agcagcagca tcagcagcaa 420
caacaatctc ttcacagcca gcttggcata aattctggag gaagcagtgg attgcatatg 480
ttgcatggtg agacaaacat gggatgtaat gggcctctct catctggggg cttccctgaa 540
tttgggcgtg ggtctgctac ctctgctgaa ggtatgcagg ccaacagggg cttcactata 600
gatcgtggtt caaataagca ggatggagta ggatcagaga atgcccatcc aggtgctggt 660
gatggaagag ggagttcaac tggagggcag aatgcagatg agtcagaacc atcatacctg 720
aaagcctccg aagaagaagg aaactag 747

<210> 203
<211> 248
<212> PRT
<213> Picea sitchensis

<400> 203

```
Met Gln Gln His Leu Met Gln Met Gln Pro Met Met Ala Ala Tyr Ala
1               5                   10                  15

Ser Asn Asn Ile Thr Thr Asp His Ile Gln Lys Tyr Leu Asp Glu Asn
            20                  25                  30

Lys Gln Leu Ile Leu Ala Ile Leu Asp Asn Gln Asn Leu Gly Lys Leu
        35                  40                  45

Asn Glu Cys Ala Gln Tyr Gln Ala Lys Leu Gln Gln Asn Leu Met Tyr
    50                  55                  60

Leu Ala Ala Ile Ala Asp Ser Gln Pro Gln Ala Gln Thr Ala His Ala
65                  70                  75                  80

Gln Ile Pro Pro Asn Ala Val Met Gln Ser Gly Gly His Tyr Met Gln
                85                  90                  95

His Gln Gln Ala Gln Gln Gln Val Thr Pro Gln Ser Leu Met Ala Ala

            100                 105                 110

Arg Ser Ser Met Leu Tyr Ser Gln Gln Pro Met Ala Ala Leu His Gln
        115                 120                 125

Ala Gln Gln Gln Gln Gln Gln Gln His Gln Gln Gln Gln Gln Ser Leu
    130                 135                 140

His Ser Gln Leu Gly Ile Asn Ser Gly Gly Ser Ser Gly Leu His Met
145                 150                 155                 160

Leu His Gly Glu Thr Asn Met Gly Cys Asn Gly Pro Leu Ser Ser Gly
                165                 170                 175

Gly Phe Pro Glu Phe Gly Arg Gly Ser Ala Thr Ser Ala Glu Gly Met
            180                 185                 190

Gln Ala Asn Arg Gly Phe Thr Ile Asp Arg Gly Ser Asn Lys Gln Asp
        195                 200                 205

Gly Val Gly Ser Glu Asn Ala His Pro Gly Ala Gly Asp Gly Arg Gly
    210                 215                 220

Ser Ser Thr Gly Gly Gln Asn Ala Asp Glu Ser Glu Pro Ser Tyr Leu
225                 230                 235                 240

Lys Ala Ser Glu Glu Glu Gly Asn
                245
```

<210> 204
<211> 735
<212> DNA
<213> Pinus taeda

<400> 204

atgcagcagc acctcatgca aatgcagccc atgatggcgg cctacgcctc caacaatatc 60
accactgatc acatccagaa gtacctggat gagaacaagc agttgattct ggcaattttg 120
gacaaccaaa atctcggaaa gctcaatgag tgtgctcaat accaagcaaa acttcagcag 180
aatttgatgt atctggctgc tattgctgat tctcaacctc aagcacaaac tgcacatgct 240
cagattcctc caaatgcggt gatgcagtct ggtgggcatt acatgcagca tcaacaggca 300
cagcaacaag ttactcctca gtctctgatg gcagctagat cttccatact gtatgctcag 360
caacaacagc agcagcagca tcagcagcat cagcagcaac agcagcaaca acagtctctt 420
cacagccagc ttggcataaa ttctggagga agcagcggtt tgcatatgtt gcatggtgag 480
acaaacatgg gatgtaatgg gcctctgtca tctgggggat tccctgaatt tgggcgtggg 540
tctgctacct ctgctgatgg tatgcaggtg aacaggggct ttgctataga tcgtggttca 600
aacaagcagg atggagttgg atcagagaat gcccatgctg gtgctggtga tggaagaggg 660
agttcaactg gagggcagaa tgcagatgag tcagaaccat catacctgaa ggcctccgag 720

gaagaaggaa actag 735

<210> 205
<211> 244
<212> PRT
<213> Pinus taeda

<400> 205

Met Gln Gln His Leu Met Gln Met Gln Pro Met Met Ala Ala Tyr Ala
1 5 10 15

Ser Asn Asn Ile Thr Thr Asp His Ile Gln Lys Tyr Leu Asp Glu Asn
20 25 30

Lys Gln Leu Ile Leu Ala Ile Leu Asp Asn Gln Asn Leu Gly Lys Leu
35 40 45

Asn Glu Cys Ala Gln Tyr Gln Ala Lys Leu Gln Gln Asn Leu Met Tyr
50 55 60

Leu Ala Ala Ile Ala Asp Ser Gln Pro Gln Ala Gln Thr Ala His Ala
65 70 75 80

Gln Ile Pro Pro Asn Ala Val Met Gln Ser Gly Gly His Tyr Met Gln
85 90 95

His Gln Gln Ala Gln Gln Gln Val Thr Pro Gln Ser Leu Met Ala Ala
100 105 110

Arg Ser Ser Ile Leu Tyr Ala Gln Gln Gln Gln Gln Gln Gln His Gln
115 120 125

Gln His Gln Gln Gln Gln Gln Gln Gln Gln Ser Leu His Ser Gln Leu
130 135 140

Gly Ile Asn Ser Gly Gly Ser Ser Gly Leu His Met Leu His Gly Glu
145 150 155 160

Thr Asn Met Gly Cys Asn Gly Pro Leu Ser Ser Gly Gly Phe Pro Glu
165 170 175

Phe Gly Arg Gly Ser Ala Thr Ser Ala Asp Gly Met Gln Val Asn Arg
180 185 190

Gly Phe Ala Ile Asp Arg Gly Ser Asn Lys Gln Asp Gly Val Gly Ser
195 200 205

Glu Asn Ala His Ala Gly Ala Gly Asp Gly Arg Gly Ser Ser Thr Gly
210 215 220

Gly Gln Asn Ala Asp Glu Ser Glu Pro Ser Tyr Leu Lys Ala Ser Glu
225 230 235 240

Glu Glu Gly Asn

<210> 206
<211> 663
<212> DNA
<213> Populus trichocarpa

<400> 206

```
atgcaacagc acctgatgca gatgcagccc atgatggcag cctattaccc cagcaacgtc     60
actactgatc atattcaaca gtatctggac gaaaacaagt cattgatttt gaagattgtt    120
gagagccaga attcagggaa actcagtgag tgtgcagaga accaagcaag actgcaacaa    180
aatctcatgt acttggctgc aattgctgat tgtcagcccc aaccacctac catgcatgcc    240
cagttccctt ccagcggcat tatgcagcca ggagcacatt acatgcagca tcaacaagct    300
caacagatga caccacaagc ccttatggct gcacgctctt ctatgctgca gtatgctcaa    360
cagccattct cagcgcttca acaacagcaa gccttacaca gccagctcgg catgagctct    420
ggtggaagcg caggacttca tatgatgcaa agcgaggcta acactgcagg aggcagtgga    480
gctcttggtg ctggacgatt tcctgatttt ggcatggatg cctccagtag aggaatcgca    540
agtgggagca agcaagatat tcggagtgca gggtctagtg aagggcgagg aggaagctct    600
ggaggccagg gtggtgatgg aggtgaaacc ctttacttga aatctgctga tgatgggaac    660
tga                                                                  663
```

<210> 207
<211> 220
<212> PRT
<213> Populus trichocarpa

<400> 207

```
Met Gln Gln His Leu Met Gln Met Gln Pro Met Met Ala Ala Tyr Tyr
1               5                   10                  15

Pro Ser Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Gln Asn Leu Met Tyr
    50                  55                  60

Leu Ala Ala Ile Ala Asp Cys Gln Pro Gln Pro Pro Thr Met His Ala
65                  70                  75                  80

Gln Phe Pro Ser Ser Gly Ile Met Gln Pro Gly Ala His Tyr Met Gln
                85                  90                  95

His Gln Gln Ala Gln Gln Met Thr Pro Gln Ala Leu Met Ala Ala Arg
            100                 105                 110
```

Ser Ser Met Leu Gln Tyr Ala Gln Gln Pro Phe Ser Ala Leu Gln Gln
115 120 125

Gln Gln Ala Leu His Ser Gln Leu Gly Met Ser Ser Gly Gly Ser Ala
130 135 140

Gly Leu His Met Met Gln Ser Glu Ala Asn Thr Ala Gly Gly Ser Gly
145 150 155 160

Ala Leu Gly Ala Gly Arg Phe Pro Asp Phe Gly Met Asp Ala Ser Ser
165 170 175

Arg Gly Ile Ala Ser Gly Ser Lys Gln Asp Ile Arg Ser Ala Gly Ser
180 185 190

Ser Glu Gly Arg Gly Gly Ser Ser Gly Gly Gln Gly Gly Asp Gly Gly
195 200 205

Glu Thr Leu Tyr Leu Lys Ser Ala Asp Asp Gly Asn
210 215 220

<210> 208
<211> 627
<212> DNA
<213> Populus trichocarpa

<400> 208

atgcagcagc caccgcaaat gattcctgtc atttctccat ttccaccaac aaacatcacc 60
actgagcaga tccaaaagta ccttgacgaa aacaaaaagt tgattttggc tatattggac 120
aaccaaaacc ttggaaaact tgctgaatgt gcccagtatc aagcccagct gcagaagaat 180
ttgatgtatt tggctgcaat tgctgatgcc caaccacagg caccagcaat gcctccccag 240
atggccccgc atcctgcaat gcaacaaggg gcatattaca tgcaacatcc tcaggcagca 300
gcaatggctc agcagccagg tgttttcccc caaaagatgc tattacaatt caatgctgga 360
catcaaatgc aggatcctca gcagttacac caacaagcca tgcaagggca aataggaatt 420
agacctatag gggctaacaa tggcatgcat cccatgcacg ctgagattgc tcttggaagc 480
agtggccctt cagcaagtgc tggcacaaat gatgtacgtg ggggaagcaa acaggatgcc 540
tctgaggctg gcacaaccgg tgctgatggc ctagggggct ctgctgctgg gcataatggt 600
gctgacggtt ccgaggatgc aaaatga 627

<210> 209
<211> 208
<212> PRT
<213> Populus trichocarpa

<400> 209

Met Gln Gln Pro Pro Gln Met Ile Pro Val Ile Ser Pro Phe Pro Pro
1 5 10 15

Thr Asn Ile Thr Thr Glu Gln Ile Gln Lys Tyr Leu Asp Glu Asn Lys
20 25 30

Lys Leu Ile Leu Ala Ile Leu Asp Asn Gln Asn Leu Gly Lys Leu Ala
35 40 45

Glu Cys Ala Gln Tyr Gln Ala Gln Leu Gln Lys Asn Leu Met Tyr Leu
50 55 60

Ala Ala Ile Ala Asp Ala Gln Pro Gln Ala Pro Ala Met Pro Pro Gln
65 70 75 80

Met Ala Pro His Pro Ala Met Gln Gln Gly Ala Tyr Tyr Met Gln His
85 90 95

Pro Gln Ala Ala Ala Met Ala Gln Gln Pro Gly Val Phe Pro Gln Lys
100 105 110

Met Leu Leu Gln Phe Asn Ala Gly His Gln Met Gln Asp Pro Gln Gln
115 120 125

Leu His Gln Gln Ala Met Gln Gly Gln Ile Gly Ile Arg Pro Ile Gly
130 135 140

Ala Asn Asn Gly Met His Pro Met His Ala Glu Ile Ala Leu Gly Ser
145 150 155 160

Ser Gly Pro Ser Ala Ser Ala Gly Thr Asn Asp Val Arg Gly Gly Ser
165 170 175

Lys Gln Asp Ala Ser Glu Ala Gly Thr Thr Gly Ala Asp Gly Leu Gly
180 185 190

Gly Ser Ala Ala Gly His Asn Gly Ala Asp Gly Ser Glu Asp Ala Lys
195 200 205

<210> 210
<211> 438
<212> DNA
<213> Populus trichocarpa

<400> 210

atgcagcagt caccgcaaca aatgttgagc atcaccactg agcagattca aaagtactta 60
gaagagaaca agcagctgat tatggctata ctggagaatc agaacaaggg aaacgtttct 120
gaatgtgctt cgtatcaagc ccagttacag cagaacctga tgtacctagc aagaattgct 180
gatgcccaac cacaaggaac cacaatgcct tctcagatgc cccctcagca gcccgcagtg 240
aagcaagagc agtacatgca gccatctcaa gttgctatga ctcagcaacc aatttcttc 300
aatcagaagc tccctttcca aacgaacttt cagcatgagc agcagcaaca gctgccacca 360
cacctccaac agcaacactt cacccaagga cagatgagaa tgagacccgg tgtcactgat 420

caagattctg atgcctaa 438

<210> 211
<211> 145
<212> PRT
<213> Populus trichocarpa

<400> 211

Met Gln Gln Ser Pro Gln Gln Met Leu Ser Ile Thr Thr Glu Gln Ile
1               5                   10                  15

Gln Lys Tyr Leu Glu Glu Asn Lys Gln Leu Ile Met Ala Ile Leu Glu
            20                  25                  30

Asn Gln Asn Lys Gly Asn Val Ser Glu Cys Ala Ser Tyr Gln Ala Gln
        35                  40                  45

Leu Gln Gln Asn Leu Met Tyr Leu Ala Arg Ile Ala Asp Ala Gln Pro
    50                  55                  60

Gln Gly Thr Thr Met Pro Ser Gln Met Pro Pro Gln Gln Pro Ala Val
65                  70                  75                  80

Lys Gln Glu Gln Tyr Met Gln Pro Ser Gln Val Ala Met Thr Gln Gln
                85                  90                  95

Pro Ile Phe Phe Asn Gln Lys Leu Pro Phe Gln Thr Asn Phe Gln His
            100                 105                 110

Glu Gln Gln Gln Gln Leu Pro Pro His Leu Gln Gln Gln His Phe Thr
        115                 120                 125

Gln Gly Gln Met Arg Met Arg Pro Gly Val Thr Asp Gln Asp Ser Asp
    130                 135                 140

Ala
145

<210> 212
<211> 645
<212> DNA
<213> Prunus persica

<400> 212

atgcagcagc cacagcaaat gatccctgtg atgcctactt catttccacc cactaacatc 60
accaccgagc aaattcagaa gtaccttgac gagaacaaaa aattgattct ggcaatattg 120
gataatcaaa accttggaaa acttgctgag tgtgcccagt accaagctca gcttcaaaag 180
aatctgatgt atttagcagc tattgctgat gcacaaccac aggcaccaac agtgcctgct 240
cagatggccc cacatcctgc tatgcaacaa gcaggatatt acatgcaaca tcctcaggca 300
gcagcaatgg ctcagcaaca gggtattttc cccccaaaga tgccattgca gttcaataac 360
ccgcaccaaa tgcatgatgc agcacagcag ctacaccagc agcaccaaca agccatgcaa 420

gggcaaatgg gaatgagagc tggaggggcc aatggcatgc cttccatgca tcatactgaa 480
gccacacttg gtggtggtag tggtggcccc acttcaggtg gagggggtcc aaacgatggg 540
cgtggaggaa agcagcaaga ctactcagag gctgggacag gtggtgatgg ccaggggagc 600
tcagccggcg ggcatggcaa tggtgatgga gaagatggaa agtga 645

<210> 213
<211> 214
<212> PRT
<213> Prunus persica

<400> 213

Met Gln Gln Pro Gln Gln Met Ile Pro Val Met Pro Thr Ser Phe Pro
1 5 10 15

Pro Thr Asn Ile Thr Thr Glu Gln Ile Gln Lys Tyr Leu Asp Glu Asn
20 25 30

Lys Lys Leu Ile Leu Ala Ile Leu Asp Asn Gln Asn Leu Gly Lys Leu
35 40 45

Ala Glu Cys Ala Gln Tyr Gln Ala Gln Leu Gln Lys Asn Leu Met Tyr
50 55 60

Leu Ala Ala Ile Ala Asp Ala Gln Pro Gln Ala Pro Thr Val Pro Ala
65 70 75 80

Gln Met Ala Pro His Pro Ala Met Gln Gln Ala Gly Tyr Tyr Met Gln
85 90 95

His Pro Gln Ala Ala Ala Met Ala Gln Gln Gln Gly Ile Phe Pro Pro
100 105 110

Lys Met Pro Leu Gln Phe Asn Asn Pro His Gln Met His Asp Ala Ala
115 120 125

Gln Gln Leu His Gln Gln His Gln Gln Ala Met Gln Gly Gln Met Gly
130 135 140

Met Arg Ala Gly Gly Ala Asn Gly Met Pro Ser Met His His Thr Glu
145 150 155 160

Ala Thr Leu Gly Gly Gly Ser Gly Gly Pro Thr Ser Gly Gly Gly Gly
165 170 175

Pro Asn Asp Gly Arg Gly Gly Lys Gln Gln Asp Tyr Ser Glu Ala Gly
180 185 190

Thr Gly Gly Asp Gly Gln Gly Ser Ser Ala Gly Gly His Gly Asn Gly
195 200 205

Asp Gly Glu Asp Gly Lys
210

<210> 214
<211> 678
<212> DNA
<213> Saccharum officinarum

<400> 214

```
atgcagcagc aacacctgat gcagatgaac cagaacatga ttgggggcta cacctctcct      60
gccgctgtga caaccgatct catccagcag tacctggatg agaacaagca gctgatcctg     120
gccatcctcg acaaccagaa caatggcaag gtggaggagt gcgaacggca ccaagctaag     180
ctccagcaca acctcatgta cctggccgcc atcgccgaca gccagccacc acagactgca     240
ccactatcac aatacccgtc caacctgatg atgcagccgg gccctcggta catgccaccg     300
cagtccgggc agatgatgag cccgcagtcg ctaatggcgg cgcggtcctc catgatgtac     360
gcgcacccgt ccatgtcacc actccagcag cagcaggcag cgcacgggca gctgggcatg     420
gcttcagggg gcggcggtgg cacgaccagt gggttcaaca tcctccatgg cgaggccagt     480
atgggcggtg ctggtggcgc ttgtgccggc aacaacatga tgaacgccgg catgttctca     540
ggctttggcc gcagcggcag tggcgccaag gagggatcga cctcgctgtc ggttgacgtc     600
cgtggtggca ccagctccgg cgcgcaaagc ggggacggcg agtacctgaa agcaggcacc     660
gaggaagaag gcagttaa                                                   678
```

<210> 215
<211> 225
<212> PRT
<213> Saccharum officinarum

<400> 215

```
Met Gln Gln Gln His Leu Met Gln Met Asn Gln Asn Met Ile Gly Gly
1               5                   10                  15

Tyr Thr Ser Pro Ala Ala Val Thr Thr Asp Leu Ile Gln Gln Tyr Leu
            20                  25                  30

Asp Glu Asn Lys Gln Leu Ile Leu Ala Ile Leu Asp Asn Gln Asn Asn
        35                  40                  45

Gly Lys Val Glu Glu Cys Glu Arg His Gln Ala Lys Leu Gln His Asn
    50                  55                  60

Leu Met Tyr Leu Ala Ala Ile Ala Asp Ser Gln Pro Pro Gln Thr Ala
65                  70                  75                  80

Pro Leu Ser Gln Tyr Pro Ser Asn Leu Met Met Gln Pro Gly Pro Arg
                85                  90                  95

Tyr Met Pro Pro Gln Ser Gly Gln Met Met Ser Pro Gln Ser Leu Met
            100                 105                 110
```

Ala Ala Arg Ser Ser Met Met Tyr Ala His Pro Ser Met Ser Pro Leu
115 120 125

Gln Gln Gln Gln Ala Ala His Gly Gln Leu Gly Met Ala Ser Gly Gly
130 135 140

Gly Gly Gly Thr Thr Ser Gly Phe Asn Ile Leu His Gly Glu Ala Ser
145 150 155 160

Met Gly Gly Ala Gly Gly Ala Cys Ala Gly Asn Asn Met Met Asn Ala
165 170 175

Gly Met Phe Ser Gly Phe Gly Arg Ser Gly Ser Gly Ala Lys Glu Gly
180 185 190

Ser Thr Ser Leu Ser Val Asp Val Arg Gly Gly Thr Ser Ser Gly Ala
195 200 205

Gln Ser Gly Asp Gly Glu Tyr Leu Lys Ala Gly Thr Glu Glu Glu Gly
210 215 220

Ser
225

<210> 216
<211> 561
<212> DNA
<213> Saccharum officinarum

<400> 216

atgcagcagc cgatgcccat gcagccgcag gcgccggaga tgaccccggc cgccggaatc 60
accacggagc agatccaaaa gtatctggat gagaataagc agcttatttt ggctattttg 120
gaaaatcaga acctaggaaa attggcagaa tgtgctcagt atcaatcaca acttcagaag 180
aacctcttgt atctcgctgc aatcgcagat gcccaaccac agactgctgt aagccgccct 240
cagatggcgc cgcctggtgc attgcctgga gtagggcagt acatgtcaca ggtgcctatg 300
ttcccaccga ggacacctct aacaccccag cagatgcagg agcagcaact tcagcagcag 360
caggctcagc tgctaaattt cagtggccta atggttgcta gacctggcat ggtcaacggc 420
atgcctcagt ccattcaagt tcagcaagct cagccaccac cagcagggaa caaacaggat 480
gctggtgggg tcgcctcgga gccctcgggc attgagaacc acaggagcac tggtggtgat 540
aatgatggtg gaagcgacta g 561

<210> 217
<211> 186
<212> PRT
<213> Saccharum officinarum

<400> 217

Met Gln Gln Pro Met Pro Met Gln Pro Gln Ala Pro Glu Met Thr Pro
1 5 10 15

Ala Ala Gly Ile Thr Thr Glu Gln Ile Gln Lys Tyr Leu Asp Glu Asn
20 25 30

Lys Gln Leu Ile Leu Ala Ile Leu Glu Asn Gln Asn Leu Gly Lys Leu
35 40 45

Ala Glu Cys Ala Gln Tyr Gln Ser Gln Leu Gln Lys Asn Leu Leu Tyr
50 55 60

Leu Ala Ala Ile Ala Asp Ala Gln Pro Gln Thr Ala Val Ser Arg Pro
65 70 75 80

Gln Met Ala Pro Pro Gly Ala Leu Pro Gly Val Gly Gln Tyr Met Ser
85 90 95

Gln Val Pro Met Phe Pro Pro Arg Thr Pro Leu Thr Pro Gln Gln Met
100 105 110

Gln Glu Gln Gln Leu Gln Gln Gln Gln Ala Gln Leu Leu Asn Phe Ser
115 120 125

Gly Leu Met Val Ala Arg Pro Gly Met Val Asn Gly Met Pro Gln Ser
130 135 140

Ile Gln Val Gln Gln Ala Gln Pro Pro Pro Ala Gly Asn Lys Gln Asp
145 150 155 160

Ala Gly Gly Val Ala Ser Glu Pro Ser Gly Ile Glu Asn His Arg Ser
165 170 175

Thr Gly Gly Asp Asn Asp Gly Gly Ser Asp
180 185

<210> 218
<211> 642
<212> DNA
<213> Saccharum officinarum

<400> 218

atgcagcagc agatgcccat gccgccggcg cccgctgcgg cggcggcgcc cccggcggcc 60
ggcatcacca ccgagcagat ccaaaagtat ttggacgaaa ataagcaact tattttggcc 120
atcctggaaa atcagaactt aggaaagttg gctgaatgtg ctcagtatca agctcaactt 180
caaaagaacc tcttgtacct ggctgcgatt gctgatgccc aaccccagcc accacaaaac 240
cctgcaggtc gccctcagat gatgcaacct ggtatagtgc caggtgcggg gcattacatg 300
tcacaagtac caatgttccc tccaagaact ccattaaccc cacagcagat gcaagagcag 360
cagcagcaac agcttcagca gcagcaagcg caggctctta cattccctgg acagatggtc 420
atgagaccag ctaccatcaa cggcatacag cagcctatgc aagctgaccc tgcccgggca 480
gcggagctgc aacaaccacc acctatccca gctgacgggc gagtaagcaa gcagcaggac 540

acaacggctg gcgtgagctc agagccttct gccaatgaga gccacaagac cacaactgga 600
gcagatagtg aggcaggtgg tgacgtggcg gagaaatcct aa 642

<210> 219
<211> 213
<212> PRT
<213> Saccharum officinarum

<400> 219

Met Gln Gln Gln Met Pro Met Pro Pro Ala Pro Ala Ala Ala Ala Ala
1 5 10 15

Pro Pro Ala Ala Gly Ile Thr Thr Glu Gln Ile Gln Lys Tyr Leu Asp
20 25 30

Glu Asn Lys Gln Leu Ile Leu Ala Ile Leu Glu Asn Gln Asn Leu Gly
35 40 45

Lys Leu Ala Glu Cys Ala Gln Tyr Gln Ala Gln Leu Gln Lys Asn Leu
50 55 60

Leu Tyr Leu Ala Ala Ile Ala Asp Ala Gln Pro Gln Pro Pro Gln Asn
65 70 75 80

Pro Ala Gly Arg Pro Gln Met Met Gln Pro Gly Ile Val Pro Gly Ala
85 90 95

Gly His Tyr Met Ser Gln Val Pro Met Phe Pro Pro Arg Thr Pro Leu
100 105 110

Thr Pro Gln Gln Met Gln Glu Gln Gln Gln Gln Gln Leu Gln Gln Gln
115 120 125

Gln Ala Gln Ala Leu Thr Phe Pro Gly Gln Met Val Met Arg Pro Ala
130 135 140

Thr Ile Asn Gly Ile Gln Gln Pro Met Gln Ala Asp Pro Ala Arg Ala
145 150 155 160

Ala Glu Leu Gln Gln Pro Pro Pro Ile Pro Ala Asp Gly Arg Val Ser
165 170 175

Lys Gln Gln Asp Thr Thr Ala Gly Val Ser Ser Glu Pro Ser Ala Asn
180 185 190

Glu Ser His Lys Thr Thr Thr Gly Ala Asp Ser Glu Ala Gly Gly Asp
195 200 205

Val Ala Glu Lys Ser
210

<210> 220
<211> 645
<212> DNA
<213> solanum tuberosum

<400> 220

atgcagcagc acctgatgca gatgcagccc atgatggcag cttactatcc aacgaacgtc 60
actactgacc atattcaaca gtatttggat gagaacaaat cactcattct gaaaattgtt 120
gagagccaaa actcgggaaa actcagtgaa tgtgcagaga accaagctag gcttcagagg 180
aatctgatgt accttgctgc tattgctgat tcacaacctc agccttctag catgcattct 240
cagttctctt ctggtgggat gatgcagcca gggacacaca gttacctgca gcagcagcag 300
cagcaacaac aagcgcaaca aatggcaaca caacaactca tggctgcaag atcctcatca 360
atgctctatg gacaacaaca gcagcagcag cagcagtctc agttatcaca atttcaacaa 420
ggcttgcata gtagccaact tggcatgagt tctggcagtg gtggaagcac tggacttcat 480
cacatgcttc aaagtgaatc atcacctcat ggtggtggtt tctctcatga cttcggccgt 540
gcaaataagc aagacattgg agtagtatg tctgctgaag ggcgcggcgg aagctcaggt 600
ggtgatggtg gtgagaatct ttatctgaaa gcttctgagg attga 645

<210> 221
<211> 214
<212> PRT
<213> Solanum tuberosum

<400> 221

Met Gln Gln His Leu Met Gln Met Gln Pro Met Met Ala Ala Tyr Tyr
1 5 10 15

Pro Thr Asn Val Thr Thr Asp His Ile Gln Gln Tyr Leu Asp Glu Asn
20 25 30

Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
50 55 60

Leu Ala Ala Ile Ala Asp Ser Gln Pro Gln Pro Ser Ser Met His Ser
65 70 75 80

Gln Phe Ser Ser Gly Gly Met Met Gln Pro Gly Thr His Ser Tyr Leu
85 90 95

Gln Gln Gln Gln Gln Gln Gln Gln Ala Gln Gln Met Ala Thr Gln Gln
100 105 110

Leu Met Ala Ala Arg Ser Ser Ser Met Leu Tyr Gly Gln Gln Gln Gln
115 120 125

Gln Gln Gln Gln Ser Gln Leu Ser Gln Phe Gln Gln Gly Leu His Ser

130 135 140

Ser Gln Leu Gly Met Ser Ser Gly Ser Gly Gly Ser Thr Gly Leu His
145 150 155 160

His Met Leu Gln Ser Glu Ser Ser Pro His Gly Gly Gly Phe Ser His
165 170 175

Asp Phe Gly Arg Ala Asn Lys Gln Asp Ile Gly Ser Ser Met Ser Ala
180 185 190

Glu Gly Arg Gly Gly Ser Ser Gly Gly Asp Gly Gly Glu Asn Leu Tyr
195 200 205

Leu Lys Ala Ser Glu Asp
210

<210> 222
<211> 681
<212> DNA
<213> Solanum tuberosum

<400> 222

atgcagcagc agcacctgat gcagatgcag cccatgatgg cagcctatta tcccaacaat 60
gtcactactg atcatattca acagttcctg gatgagaaca aatcacttat tctgaagatt 120
gttgagagcc agaactctgg gaaaataagt gaatgtgcag agtcccaagc taaacttcag 180
agaaatctta tgtaccttgc agctattgct gattcacagc cccagcctcc tagtatgcat 240
tcacagttag cttctggtgg gatgatgcag ggaggggcac attatatgca gcaacaacaa 300
gctcaacaac tcacaacgca atcgcttatg gctgcagcaa gatcctcctc ctcaatgctc 360
tatggacaac aacaacaaca acaacaacaa caactatcat cattgcaaca acagcaagca 420
gcctttcata gccagcaact cggaatgagc agctctggtg gaggaagcag tagtggactt 480
cacatgctac aaagcgaaaa cactcatagt gctagcactg gtggtggtgg tttccctgac 540
tttggcagag gattaggcag tggaaacaag catgaaatgg gaagttctat gtctgatcaa 600
ggacggggcg gaagctcgag tggtcatggt ggtgatggag gtgagaatct ttacttgaaa 660
tcttctgaag atgggaatta g 681

<210> 223
<211> 226
<212> PRT
<213> solanum tuberosum

<400> 223

Met Gln Gln Gln His Leu Met Gln Met Gln Pro Met Met Ala Ala Tyr
1               5                   10                  15

Tyr Pro Asn Asn Val Thr Thr Asp His Ile Gln Gln Phe Leu Asp Glu
            20                  25                  30

Asn Lys Ser Leu Ile Leu Lys Ile Val Glu Ser Gln Asn Ser Gly Lys
        35                  40                  45

Ile Ser Glu Cys Ala Glu Ser Gln Ala Lys Leu Gln Arg Asn Leu Met
    50                  55                  60

Tyr Leu Ala Ala Ile Ala Asp Ser Gln Pro Gln Pro Pro Ser Met His
65                  70                  75                  80

Ser Gln Leu Ala Ser Gly Gly Met Met Gln Gly Gly Ala His Tyr Met
                85                  90                  95

Gln Gln Gln Gln Ala Gln Gln Leu Thr Thr Gln Ser Leu Met Ala Ala
            100                 105                 110

Ala Arg Ser Ser Ser Ser Met Leu Tyr Gly Gln Gln Gln Gln Gln Gln
        115                 120                 125

Gln Gln Gln Leu Ser Ser Leu Gln Gln Gln Gln Ala Ala Phe His Ser
    130                 135                 140

Gln Gln Leu Gly Met Ser Ser Ser Gly Gly Gly Ser Ser Ser Gly Leu
145                 150                 155                 160

His Met Leu Gln Ser Glu Asn Thr His Ser Ala Ser Thr Gly Gly Gly
                165                 170                 175

Gly Phe Pro Asp Phe Gly Arg Gly Leu Gly Ser Gly Asn Lys His Glu
            180                 185                 190

Met Gly Ser Ser Met Ser Asp Gln Gly Arg Gly Gly Ser Ser Ser Gly
        195                 200                 205

His Gly Gly Asp Gly Gly Glu Asn Leu Tyr Leu Lys Ser Ser Glu Asp
    210                 215                 220

Gly Asn
225

<210> 224
<211> 615
<212> DNA
<213> solanum tuberosum

<400> 224

atgcagcaac caccacccat gattccaatg atgccctctt ttccttctcc taatatcact 60
actgagcaga ttcaaaagta cctggacgag aacaagacat tgattttggc catattggac 120
catcaaaatc ttgggaaact agctgaatgt gcacagtacc aggctaaact tcagaagaac 180
ttgatgtact tggccgctat tgctgatgct caaccacaat caccagctat tccaacgcaa 240
atggctcctc atcctgcaat gcaacaagga ggattttaca tgcagcaccc tcaggctgca 300
gccatgactc aacaacaagg tatgtttact tcaaagatgc cactgcagtt caacaaccca 360

cagcaactac acgatcagca gcagcttcaa catcaacatc aacatcaaca actacagcga 420
cagcagcaag gtatgcaact tggaggtgcc aacagtggaa tgcactccac tcttggtagt 480
acaagtaatg ttagccagct tacaacttca ggtgctggtg atgcacgcgg aggaaacaaa 540
caagacaact ctgaagcggg tgctgatggt caggctagct cagtgactgc ccaagtctcg 600
gaagaacgca agtga 615

<210> 225
<211> 204
<212> PRT
<213> solanum tuberosum

<400> 225

Met Gln Gln Pro Pro Pro Met Ile Pro Met Met Pro Ser Phe Pro Ser
1               5                   10                  15

Pro Asn Ile Thr Thr Glu Gln Ile Gln Lys Tyr Leu Asp Glu Asn Lys
            20                  25                  30

Thr Leu Ile Leu Ala Ile Leu Asp His Gln Asn Leu Gly Lys Leu Ala
        35                  40                  45

Glu Cys Ala Gln Tyr Gln Ala Lys Leu Gln Lys Asn Leu Met Tyr Leu
    50                  55                  60

Ala Ala Ile Ala Asp Ala Gln Pro Gln Ser Pro Ala Ile Pro Thr Gln
65                  70                  75                  80

Met Ala Pro His Pro Ala Met Gln Gln Gly Gly Phe Tyr Met Gln His
                85                  90                  95

Pro Gln Ala Ala Ala Met Thr Gln Gln Gln Gly Met Phe Thr Ser Lys
            100                 105                 110

Met Pro Leu Gln Phe Asn Asn Pro Gln Gln Leu His Asp Gln Gln Gln
        115                 120                 125

Leu Gln His Gln His Gln His Gln Gln Leu Gln Arg Gln Gln Gln Gly
    130                 135                 140

Met Gln Leu Gly Gly Ala Asn Ser Gly Met His Ser Thr Leu Gly Ser
145                 150                 155                 160

Thr Ser Asn Val Ser Gln Leu Thr Thr Ser Gly Ala Gly Asp Ala Arg
                165                 170                 175

Gly Gly Asn Lys Gln Asp Asn Ser Glu Ala Gly Ala Asp Gly Gln Ala
            180                 185                 190

Ser Ser Val Thr Ala Gln Val Ser Glu Glu Arg Lys
        195                 200

<210> 226
<211> 678
<212> DNA
<213> Sorghum bicolor

<400> 226

atgcagcagc aacacctgat gcagatgaac cagaacatga ttgggggcta cacctctcct 60
gccgctgtga ccaccgatct catccagcag tacctggatg agaacaagca gctgatcctg 120
gccatcctcg acaaccagaa caatggaaag gtggaggagt gcgaacggca ccaagctaag 180
ctccagcaca acctcatgta cctggcggcc atcgctgaca gccagccacc acagactgca 240
ccactatcac agtacccgtc caacctgatg atgcagccag gccctcggta catgccaccg 300
cagtccgggc agatgatgag cccgcagtcg ctaatggcgg cgcggtcctc catgatgtac 360
gcgcacccgt ccatgtcgcc actccagcag cagcaggcag cgcacggcca gctgggcatg 420
gcttcagggg gcggcggtgg cacgaccagt gggttcagca tcctccacgg cgaggccagc 480
atgggcggtg ctgctggcgc aggcaccggc aacagcatga tgaacgccgg catgttctca 540
ggctttggcc gcagcggcag tggcgccaag gagggatcga cctcgctgtc tgttgacgtc 600
cgtggtggca ccagctccgg cgcgcagagc ggggacggcg agtacctgaa agcaggcacc 660
gaggaagaag gcagttaa 678

<210> 227
<211> 225
<212> PRT
<213> Sorghum bicolor

<400> 227

Met Gln Gln Gln His Leu Met Gln Met Asn Gln Asn Met Ile Gly Gly
1 5 10 15

Tyr Thr Ser Pro Ala Ala Val Thr Thr Asp Leu Ile Gln Gln Tyr Leu
20 25 30

Asp Glu Asn Lys Gln Leu Ile Leu Ala Ile Leu Asp Asn Gln Asn Asn
35 40 45

Gly Lys Val Glu Glu Cys Glu Arg His Gln Ala Lys Leu Gln His Asn
50 55 60

Leu Met Tyr Leu Ala Ala Ile Ala Asp Ser Gln Pro Pro Gln Thr Ala
65 70 75 80

Pro Leu Ser Gln Tyr Pro Ser Asn Leu Met Met Gln Pro Gly Pro Arg
85 90 95

Tyr Met Pro Pro Gln Ser Gly Gln Met Met Ser Pro Gln Ser Leu Met
100 105 110

Ala Ala Arg Ser Ser Met Met Tyr Ala His Pro Ser Met Ser Pro Leu

115 120 125

Gln Gln Gln Gln Ala Ala His Gly Gln Leu Gly Met Ala Ser Gly Gly
130 135 140

Gly Gly Gly Thr Thr Ser Gly Phe Ser Ile Leu His Gly Glu Ala Ser
145 150 155 160

Met Gly Gly Ala Ala Gly Ala Gly Thr Gly Asn Ser Met Met Asn Ala
165 170 175

Gly Met Phe Ser Gly Phe Gly Arg Ser Gly Ser Gly Ala Lys Glu Gly
180 185 190

Ser Thr Ser Leu Ser Val Asp Val Arg Gly Gly Thr Ser Ser Gly Ala
195 200 205

Gln Ser Gly Asp Gly Glu Tyr Leu Lys Ala Gly Thr Glu Glu Glu Gly
210 215 220

Ser
225

<210> 228
<211> 561
<212> DNA
<213> Sorghum bicolor

<400> 228

```
atgcagcagc cgatgcccat gcagccgcag gcgccggcga tgaccccggc cgccggaatc     60
accacggagc agatccaaaa gtatctggat gagaataagc agcttatttt ggctattttg    120
gaaaatcaga acctaggaaa attggcagaa tgtgctcagt atcaatcaca acttcagaag    180
aacctcttgt atctcgctgc aatcgcagat gcccaaccac agactgctgt aagccgccct    240
cagatggcgc cgcctggtgc attgcctgga gtagggcagt acatgtcaca ggtgcctatg    300
ttcccaccaa ggacacctct cacaccccaa cagatgcagg agcagcaact tcagcagcag    360
caggctcagt tgctaaattt cagtggccag atggttgcta gacctggcat ggtcaacggc    420
atgcctcagt ccattcaagc tcaacaagct cagccatcac cagcattgaa caaacaggat    480
gctggtggag tcgcctcaga gccctcgggc actgagagcc acaggagcac tggtggtgat    540
aatgatggtg gaagcgacta g                                               561
```

<210> 229
<211> 186
<212> PRT
<213> Sorghum bicolor

<400> 229

Met Gln Gln Pro Met Pro Met Gln Pro Gln Ala Pro Ala Met Thr Pro
1 5 10 15

Ala Ala Gly Ile Thr Thr Glu Gln Ile Gln Lys Tyr Leu Asp Glu Asn
20 25 30

Lys Gln Leu Ile Leu Ala Ile Leu Glu Asn Gln Asn Leu Gly Lys Leu
35 40 45

Ala Glu Cys Ala Gln Tyr Gln Ser Gln Leu Gln Lys Asn Leu Leu Tyr
50 55 60

Leu Ala Ala Ile Ala Asp Ala Gln Pro Gln Thr Ala Val Ser Arg Pro
65 70 75 80

Gln Met Ala Pro Pro Gly Ala Leu Pro Gly Val Gly Gln Tyr Met Ser
85 90 95

Gln Val Pro Met Phe Pro Pro Arg Thr Pro Leu Thr Pro Gln Gln Met
100 105 110

Gln Glu Gln Gln Leu Gln Gln Gln Gln Ala Gln Leu Leu Asn Phe Ser
115 120 125

Gly Gln Met Val Ala Arg Pro Gly Met Val Asn Gly Met Pro Gln Ser
130 135 140

Ile Gln Ala Gln Gln Ala Gln Pro Ser Pro Ala Leu Asn Lys Gln Asp
145 150 155 160

Ala Gly Gly Val Ala Ser Glu Pro Ser Gly Thr Glu Ser His Arg Ser
165 170 175

Thr Gly Gly Asp Asn Asp Gly Gly Ser Asp
180 185

<210> 230
<211> 645
<212> DNA
<213> Sorghum bicolor

<400> 230

atgcagcagc agatgcccat gccgccggcg cccgctgcgg cggcggcgac ggcgcccccg 60
gcggccggca tcaccaccga gcagatccag aagtatttgg acgaaaataa gcaacttatt 120
ttggccatcc tagaaaatca gaacttagga aagttggctg aatgtgctca gtatcaagct 180
caacttcaaa agaacctctt gtacctggct gcgattgctg atgcccaacc ccgaccaccg 240
caaaaccctg caggtcgccc tcagatgatg caacctggta tagtgccagg tgcagggcat 300
tacatgtcac aagtaccaat gttccctcca agaactccat taaccccaca gcaaatgcaa 360
gagcagcagc agcaacagct tcagcagcag caagcgcagg ctcttgcatt ccctgggcag 420
atggtcatga gaccagctac catcaacggc atgcagcagc ctatgcaggc tgaccctgcc 480
cgggcagcgg agctgcaaca gccagcatct gtcccagccg acgggcgagt aagcaagcag 540
gacacagcgg ctggggtgag ctcagagcct tctgccaatg agagccacaa gaccacaacc 600

ggagcagata gtgaggcagg tggagacgtg gcggagaaat cctaa 645

<210> 231
<211> 214
<212> PRT
<213> Sorghum bicolor

<400> 231

```
Met Gln Gln Gln Met Pro Met Pro Pro Ala Pro Ala Ala Ala Ala Ala
1               5                   10                  15

Thr Ala Pro Pro Ala Ala Gly Ile Thr Thr Glu Gln Ile Gln Lys Tyr
            20                  25                  30

Leu Asp Glu Asn Lys Gln Leu Ile Leu Ala Ile Leu Glu Asn Gln Asn
        35                  40                  45

Leu Gly Lys Leu Ala Glu Cys Ala Gln Tyr Gln Ala Gln Leu Gln Lys
    50                  55                  60

Asn Leu Leu Tyr Leu Ala Ala Ile Ala Asp Ala Gln Pro Arg Pro Pro
65                  70                  75                  80

Gln Asn Pro Ala Gly Arg Pro Gln Met Met Gln Pro Gly Ile Val Pro
                85                  90                  95

Gly Ala Gly His Tyr Met Ser Gln Val Pro Met Phe Pro Pro Arg Thr
            100                 105                 110

Pro Leu Thr Pro Gln Gln Met Gln Glu Gln Gln Gln Gln Gln Leu Gln
        115                 120                 125

Gln Gln Gln Ala Gln Ala Leu Ala Phe Pro Gly Gln Met Val Met Arg
    130                 135                 140

Pro Ala Thr Ile Asn Gly Met Gln Gln Pro Met Gln Ala Asp Pro Ala
145                 150                 155                 160

Arg Ala Ala Glu Leu Gln Gln Pro Ala Ser Val Pro Ala Asp Gly Arg
                165                 170                 175

Val Ser Lys Gln Asp Thr Ala Ala Gly Val Ser Ser Glu Pro Ser Ala
            180                 185                 190

Asn Glu Ser His Lys Thr Thr Thr Gly Ala Asp Ser Glu Ala Gly Gly
        195                 200                 205

Asp Val Ala Glu Lys Ser
    210
```

<210> 232
<211> 552
<212> DNA
<213> Taraxacum officinale

<400> 232

atgaagcagc cgatgatgcc taatccaatg atgtcttctc cgtttcctcc ttccaacatc 60
accaccgatc agatccaaaa gttcctagac gaaaacaagc aactgatatt agcaataatg 120
aacaaccaaa acctaggaaa gcttgctgaa tgtgcccagt atcaagctct actccaaaag 180
aatttaatgt atctagcagc cattgcagat gctcaaccac caacaccaac accaacacca 240
aatatctcat ctcagatggg cccggttcca catccaggga tgccacaaca aggtggattc 300
tacatggggc agcaccctca agcggcagta atggcggctc agccaccttc tggtttccca 360
caaccaatgc caggcatgca gtttaatacc ccacagggta tccaaggtca gatgggcggg 420
aggtccggtg ggccaccaaa ctcagctggc ggcgatgttt ggagaggaag catgcaagat 480
ggtggtggtg gcggtgttga tggtggtaag gatggtcatg ctggcggtgg tccaccggag 540
gaaggaaagt ga 552

<210> 233
<211> 183
<212> PRT
<213> Taraxacum officinale

<400> 233

Met Lys Gln Pro Met Met Pro Asn Pro Met Met Ser Ser Pro Phe Pro
1               5                   10                  15

Pro Ser Asn Ile Thr Thr Asp Gln Ile Gln Lys Phe Leu Asp Glu Asn
            20                  25                  30

Lys Gln Leu Ile Leu Ala Ile Met Asn Asn Gln Asn Leu Gly Lys Leu
        35                  40                  45

Ala Glu Cys Ala Gln Tyr Gln Ala Leu Leu Gln Lys Asn Leu Met Tyr
    50                  55                  60

Leu Ala Ala Ile Ala Asp Ala Gln Pro Pro Thr Pro Thr Pro Thr Pro
65                  70                  75                  80

Asn Ile Ser Ser Gln Met Gly Pro Val Pro His Pro Gly Met Pro Gln
                85                  90                  95

Gln Gly Gly Phe Tyr Met Gly Gln His Pro Gln Ala Ala Val Met Ala
            100                 105                 110

Ala Gln Pro Pro Ser Gly Phe Pro Gln Pro Met Pro Gly Met Gln Phe
        115                 120                 125

Asn Thr Pro Gln Gly Ile Gln Gly Gln Met Gly Gly Arg Ser Gly Gly
    130                 135                 140

Pro Pro Asn Ser Ala Gly Gly Asp Val Trp Arg Gly Ser Met Gln Asp

145 150 155 160

Gly Gly Gly Gly Gly Val Asp Gly Gly Lys Asp Gly His Ala Gly Gly
165 170 175

Gly Pro Pro Glu Glu Gly Lys
180

<210> 234
<211> 636
<212> DNA
<213> Taraxacum officinale

<400> 234

```
atgcagcagc aacagcatca acaaccaccg caatcgcaac tgcaaccgcc caccttaaac    60
tccggtgctc cattttctcc caatgcgatc acctccgacc agattcaaaa gtgtctggat   120
gacaacgaga acctgattat agcaatattg gaaaatcaaa atcttgggaa atttcaggag   180
tgtgctcagt atcaagccat tctccaaaag aacttaatgt atttagctgc aattgctgat   240
gctcaaccac caacacaaca accatcaact cctcaaatgc caccaaattc cattcctcaa   300
caaccaaaca attacatgca acaacaacac caaatcaccg cccctcaaca aggcggcata   360
ggtggtggtg gcggtgttcc aaaactaccc tttcaactta atgcccttcg tacacaagat   420
caacaacaac aattactcca atttcaacaa caacaacaac ttcaagcaca aatggggatg   480
agacctagtt cccaagatgg gatgcttgga atgcatcaag ctatgcagtc tgcactcgcg   540
ggcaatccgg gcagtttgat ggatggtaga gggaacaagc aagatggatc agaggcggcg   600
gcttctggtg gtggtggtgg taatagagaa tcatga                             636
```

<210> 235
<211> 211
<212> PRT
<213> Taraxacum officinale

<400> 235

Met Gln Gln Gln Gln His Gln Gln Pro Pro Gln Ser Gln Leu Gln Pro
1 5 10 15

Pro Thr Leu Asn Ser Gly Ala Pro Phe Ser Pro Asn Ala Ile Thr Ser
20 25 30

Asp Gln Ile Gln Lys Cys Leu Asp Asp Asn Glu Asn Leu Ile Ile Ala
35 40 45

Ile Leu Glu Asn Gln Asn Leu Gly Lys Phe Gln Glu Cys Ala Gln Tyr
50 55 60

Gln Ala Ile Leu Gln Lys Asn Leu Met Tyr Leu Ala Ala Ile Ala Asp
65 70 75 80

Ala Gln Pro Pro Thr Gln Gln Pro Ser Thr Pro Gln Met Pro Pro Asn
85 90 95

Ser Ile Pro Gln Gln Pro Asn Asn Tyr Met Gln Gln Gln His Gln Ile
100 105 110

Thr Ala Pro Gln Gln Gly Gly Ile Gly Gly Gly Gly Gly Val Pro Lys
115 120 125

Leu Pro Phe Gln Leu Asn Ala Leu Arg Thr Gln Asp Gln Gln Gln Gln
130 135 140

Leu Leu Gln Phe Gln Gln Gln Gln Gln Leu Gln Ala Gln Met Gly Met
145 150 155 160

Arg Pro Ser Ser Gln Asp Gly Met Leu Gly Met His Gln Ala Met Gln
165 170 175

Ser Ala Leu Ala Gly Asn Pro Gly Ser Leu Met Asp Gly Arg Gly Asn
180 185 190

Lys Gln Asp Gly Ser Glu Ala Ala Ala Ser Gly Gly Gly Gly Gly Asn
195 200 205

Arg Glu Ser
210

<210> 236
<211> 678
<212> DNA
<213> Triticum aestivum

<400> 236

```
atgcagcagc aacacctgat gcagatgaac cagagcatga tggggggcta cgcttcctct      60
accactgtca ccactgatct cattcagcag tacctggatg agaacaagca gctgatcctg     120
gccatcctcg acaaccagaa caacggcaag gtggaggagt gcgcacggaa ccaagctaag     180
ctccagcaga acctcatgta cctcgccgcc atcgccgaca gccagcctcc gcagacggca     240
tcgctgtctc agtacccgtc caacctgatg atgcagtccg ggccgcggta catgcagcag     300
cagtcggcgc agatgatgtc gccgcagtcg ctgatggcgg cgcggtcgtc gatgatgtac     360
gcgcagcagg ccatgtcgcc gctccagcag cagcagcagc agcagcacca ggcggccgcg     420
cacggccagc tggggatgtc ctccggcgcg accaccgggt tcaacctcct ccacggcgag     480
gccagcatgg gcggcggcgg cggcggcgcc agtggcaaca gcatgatgaa cgccggcgtc     540
ttctcggact accgccgcgg cggcagcggc gccaaggagg ggtcgacctc gctgtcggcc     600
gacgctcgcg gcgccaactc tggcgcgcac agcggcgacg gggagtacct caagggcacc     660
gaggaggaag gaagctag                                                   678
```

<210> 237
<211> 225
<212> PRT
<213> Triticum aestivum

<400> 237

```
Met Gln Gln Gln His Leu Met Gln Met Asn Gln Ser Met Met Gly Gly
1               5                   10                  15

Tyr Ala Ser Ser Thr Thr Val Thr Thr Asp Leu Ile Gln Gln Tyr Leu
            20                  25                  30

Asp Glu Asn Lys Gln Leu Ile Leu Ala Ile Leu Asp Asn Gln Asn Asn
        35                  40                  45

Gly Lys Val Glu Glu Cys Ala Arg Asn Gln Ala Lys Leu Gln Gln Asn
    50                  55                  60

Leu Met Tyr Leu Ala Ala Ile Ala Asp Ser Gln Pro Pro Gln Thr Ala
65                  70                  75                  80

Ser Leu Ser Gln Tyr Pro Ser Asn Leu Met Met Gln Ser Gly Pro Arg
                85                  90                  95

Tyr Met Gln Gln Gln Ser Ala Gln Met Met Ser Pro Gln Ser Leu Met
            100                 105                 110

Ala Ala Arg Ser Ser Met Met Tyr Ala Gln Gln Ala Met Ser Pro Leu
        115                 120                 125

Gln Gln Gln Gln Gln Gln Gln His Gln Ala Ala Ala His Gly Gln Leu
    130                 135                 140

Gly Met Ser Ser Gly Ala Thr Thr Gly Phe Asn Leu Leu His Gly Glu
145                 150                 155                 160

Ala Ser Met Gly Gly Gly Gly Gly Gly Ala Ser Gly Asn Ser Met Met
                165                 170                 175

Asn Ala Gly Val Phe Ser Asp Tyr Arg Arg Gly Gly Ser Gly Ala Lys
            180                 185                 190

Glu Gly Ser Thr Ser Leu Ser Ala Asp Ala Arg Gly Ala Asn Ser Gly
        195                 200                 205

Ala His Ser Gly Asp Gly Glu Tyr Leu Lys Gly Thr Glu Glu Glu Gly
    210                 215                 220

Ser
225
```

<210> 238
<211> 558
<212> DNA
<213> Triticum aestivum

<400> 238

atgcagcaag cgatgcccat gccgccggcg gcggcggcgc cggggatgcc tccgtctgct 60
ggcctcagca ccgagcagat ccaaaagtac ctggatgaaa ataagcaact aattttggct 120
atcttggaaa atcagaacct gggaaagttg gcggaatgtg ctcagtatca agctcagctt 180
cagaagaatc ttttgtattt ggctgcaatc gctgatactc agccacagac cactgtaagc 240
cgtcctcaga tggcaccacc tagtgcatcc ccaggggcag ggcattacat gtcacaggtg 300
ccaatgttcc ctccgaggac ccctctaacg cctcagcaga tgcaggagca gcaactacag 360
cagcaacagg ctcagatgct tccgtttgct ggtcaaatgg ttgcgagacc tggggctgtc 420
aatggcatgc ctcaggcccc tcaagttgaa ccagcctatg cagcaggtgg ggccagttct 480
gagccttctg gcactgagag ccacaggagc actggtgccg ataatgacgg ggggagcggc 540
tgggctgatc agtcctaa 558

<210> 239
<211> 185
<212> PRT
<213> Triticum aestivum

<400> 239

Met Gln Gln Ala Met Pro Met Pro Pro Ala Ala Ala Ala Pro Gly Met
1               5                   10                  15

Pro Pro Ser Ala Gly Leu Ser Thr Glu Gln Ile Gln Lys Tyr Leu Asp
            20                  25                  30

Glu Asn Lys Gln Leu Ile Leu Ala Ile Leu Glu Asn Gln Asn Leu Gly
        35                  40                  45

Lys Leu Ala Glu Cys Ala Gln Tyr Gln Ala Gln Leu Gln Lys Asn Leu
    50                  55                  60

Leu Tyr Leu Ala Ala Ile Ala Asp Thr Gln Pro Gln Thr Thr Val Ser
65                  70                  75                  80

Arg Pro Gln Met Ala Pro Pro Ser Ala Ser Pro Gly Ala Gly His Tyr
                85                  90                  95

Met Ser Gln Val Pro Met Phe Pro Pro Arg Thr Pro Leu Thr Pro Gln
            100                 105                 110

Gln Met Gln Glu Gln Gln Leu Gln Gln Gln Gln Ala Gln Met Leu Pro
        115                 120                 125

Phe Ala Gly Gln Met Val Ala Arg Pro Gly Ala Val Asn Gly Met Pro
    130                 135                 140

Gln Ala Pro Gln Val Glu Pro Ala Tyr Ala Ala Gly Gly Ala Ser Ser
145                 150                 155                 160

Glu Pro Ser Gly Thr Glu Ser His Arg Ser Thr Gly Ala Asp Asn Asp

165 170 175

Gly Gly Ser Gly Trp Ala Asp Gln Ser
180 185

<210> 240
<211> 603
<212> DNA
<213> Triticum aestivum

<400> 240

```
atgcagcagg cgatgtcctt gcccccggga gcggtcggcg cggtgtcctc gccggccggc      60
atcaccaccg agcagatcca aaagtatttg gatgaaaata agcaacttat tttggccatc     120
cttgaaaatc agaacctagg aaagttggct gaatgtgctc agtatcaagc tcaactccaa     180
aagaatctct tgtatctagc tgctatcgcg gatgcccaac caccacagaa ccctacaagt     240
caccctcaga tggtgcagcc tggtagtatg caaggtgcag ggcattacat gtcacaagta     300
ccaatgttcc ctccaagaac gcctttaacc ccacagcaga tgcaagagca gcagcaccag     360
cagcttcagc agcagcaagc ccaggccctt tctttccccg cccaggtggt catgagacca     420
ggcaccgtca acggcatgca gcagcctatg caagcagccg gcgacctcca gccagcagca     480
gcacctggag ggagcaagca ggacgccgca gtggctgggg ccagctcgga accatctggc     540
accaagagcc acaagaacgc gggagcagag gaggtgggcg ctgatgtagc agaacaatcc     600
taa                                                                   603
```

<210> 241
<211> 200
<212> PRT
<213> Triticum aestivum

<400> 241

```
Met Gln Gln Ala Met Ser Leu Pro Pro Gly Ala Val Gly Ala Val Ser
1               5                   10                  15

Ser Pro Ala Gly Ile Thr Thr Glu Gln Ile Gln Lys Tyr Leu Asp Glu
            20                  25                  30

Asn Lys Gln Leu Ile Leu Ala Ile Leu Glu Asn Gln Asn Leu Gly Lys
        35                  40                  45

Leu Ala Glu Cys Ala Gln Tyr Gln Ala Gln Leu Gln Lys Asn Leu Leu
    50                  55                  60

Tyr Leu Ala Ala Ile Ala Asp Ala Gln Pro Pro Gln Asn Pro Thr Ser
65                  70                  75                  80

His Pro Gln Met Val Gln Pro Gly Ser Met Gln Gly Ala Gly His Tyr
                85                  90                  95

Met Ser Gln Val Pro Met Phe Pro Pro Arg Thr Pro Leu Thr Pro Gln
            100                 105                 110
```

Gln Met Gln Glu Gln Gln His Gln Gln Leu Gln Gln Gln Gln Ala Gln
115 120 125

Ala Leu Ser Phe Pro Ala Gln Val Val Met Arg Pro Gly Thr Val Asn
130 135 140

Gly Met Gln Gln Pro Met Gln Ala Ala Gly Asp Leu Gln Pro Ala Ala
145 150 155 160

Ala Pro Gly Gly Ser Lys Gln Asp Ala Ala Val Ala Gly Ala Ser Ser
165 170 175

Glu Pro Ser Gly Thr Lys Ser His Lys Asn Ala Gly Ala Glu Glu Val
180 185 190

Gly Ala Asp Val Ala Glu Gln Ser
195 200

<210> 242
<211> 672
<212> DNA
<213> vitis vinifera

<400> 242

atgcagcagc acctgatgca gatgcagccc atgatggcag cctattaccc cagcaacgtc 60
accactgatc acattcagca gtatcttgat gaaaacaagt cattgattct gaagattgtt 120
gagagccaga attcaggaaa attgactgaa tgtgcagaga accaggcaag actacagaga 180
aacctcatgt acctggctgc aattgctgat tctcaacccc aaccacccac catgcatgct 240
cagttccctc ctagtggcat tgttcagcca ggagctcact acatgcaaca ccaacaagct 300
caacaaatga caccacagtc gctcctggct gcacgctcct ccatgctgta cacccaacaa 360
ccattttcgg ccctgcaaca acaacaagcc atccatagcc agcttggcat gggctctggt 420
ggaagtgcag gacttcacat gctgcaaagc gaggggagta atccaggagg caatggaaca 480
ctggggactg gtgggtttcc tgatttcagc cgtggaactt ctggagaagg cctgcaggct 540
gcaggcaggg gaatggctgg tgggagcaag caagatatgg gaaatgcaga agggcgagga 600
gggaactcag gaggtcaggg tggggatgga ggtgagactc tttacttgaa agctgctgaa 660
gatgggaatt ga 672

<210> 243
<211> 223
<212> PRT
<213> vitis vinifera

<400> 243

Met Gln Gln His Leu Met Gln Met Gln Pro Met Met Ala Ala Tyr Tyr
1               5                   10                  15

Pro Ser Asn Val Thr Thr Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Thr Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Thr Met His Ala
65                  70                  75                  80

Gln Phe Pro Pro Ser Gly Ile Val Gln Pro Gly Ala His Tyr Met Gln
                85                  90                  95

His Gln Gln Ala Gln Gln Met Thr Pro Gln Ser Leu Leu Ala Ala Arg
            100                 105                 110

Ser Ser Met Leu Tyr Thr Gln Gln Pro Phe Ser Ala Leu Gln Gln Gln
        115                 120                 125

Gln Ala Ile His Ser Gln Leu Gly Met Gly Ser Gly Gly Ser Ala Gly
    130                 135                 140

Leu His Met Leu Gln Ser Glu Gly Ser Asn Pro Gly Gly Asn Gly Thr
145                 150                 155                 160

Leu Gly Thr Gly Gly Phe Pro Asp Phe Ser Arg Gly Thr Ser Gly Glu
                165                 170                 175

Gly Leu Gln Ala Ala Gly Arg Gly Met Ala Gly Gly Ser Lys Gln Asp
            180                 185                 190

Met Gly Asn Ala Glu Gly Arg Gly Gly Asn Ser Gly Gly Gln Gly Gly
        195                 200                 205

Asp Gly Gly Glu Thr Leu Tyr Leu Lys Ala Ala Glu Asp Gly Asn
    210                 215                 220

<210> 244
<211> 675
<212> DNA
<213> vitis vinifera

<400> 244

```
atgcagcaac accttatgca gatgcagcct atgatggcag gaagccataa cctcagcagc    60
atcactactg atcacatcca acagtaccta gatgagaaca agtctttgat tttgaaaatt   120
cttgagagcc aaaattcagg gaaactcagt gaatgtgcgg agaaccaagc aagacttcag   180
cgaaacctta tgtaccttgc tgcaattgct gattgccaac cacaaccacc atccctgcag   240
gctcagtttt cccccaatat ggtcatgcaa ccaggagtca actacatgca gcaccaacaa   300
tcccaacaga tgatgccaca gtcactaatg gcagcccgag cacccatgtt gtatgctcag   360

cagcatccat atttggcatt gcagcaacaa caagctctac aaagccagct tggcatgagc   420
tccactggaa tgggtggaat ccacatgcta caaagtgaac ctaatgttgg agggaatggg   480
actggagcct tttccgatct tggtcgcagc atgactgggg agggcttgtc ggctgtgagc   540
aggggactgg gtagtgcaag caagcaagat gtggggagtg taggctctgc ggaaggtcga   600
cgtggctact tgggagggca aggtgcagat aaaggagaaa ctctttactt taaaagtgct   660
gaagaaaagg actga                                                    675
```

<210> 245
<211> 224
<212> PRT
<213> vitis vinifera

<400> 245

Met Gln Gln His Leu Met Gln Met Gln Pro Met Met Ala Gly Ser His
1 5 10 15

Asn Leu Ser Ser Ile Thr Thr Asp His Ile Gln Gln Tyr Leu Asp Glu
20 25 30

Asn Lys Ser Leu Ile Leu Lys Ile Leu Glu Ser Gln Asn Ser Gly Lys
35 40 45

Leu Ser Glu Cys Ala Glu Asn Gln Ala Arg Leu Gln Arg Asn Leu Met
50 55 60

Tyr Leu Ala Ala Ile Ala Asp Cys Gln Pro Gln Pro Pro Ser Leu Gln
65 70 75 80

Ala Gln Phe Ser Pro Asn Met Val Met Gln Pro Gly Val Asn Tyr Met
85 90 95

Gln His Gln Gln Ser Gln Gln Met Met Pro Gln Ser Leu Met Ala Ala
100 105 110

Arg Ala Pro Met Leu Tyr Ala Gln Gln His Pro Tyr Leu Ala Leu Gln
115 120 125

Gln Gln Gln Ala Leu Gln Ser Gln Leu Gly Met Ser Ser Thr Gly Met
130 135 140

Gly Gly Ile His Met Leu Gln Ser Glu Pro Asn Val Gly Gly Asn Gly
145 150 155 160

Thr Gly Ala Phe Ser Asp Leu Gly Arg Ser Met Thr Gly Glu Gly Leu
165 170 175

Ser Ala Val Ser Arg Gly Leu Gly Ser Ala Ser Lys Gln Asp Val Gly
180 185 190

Ser Val Gly Ser Ala Glu Gly Arg Arg Gly Tyr Leu Gly Gly Gln Gly

195 200 205

Ala Asp Lys Gly Glu Thr Leu Tyr Phe Lys Ser Ala Glu Glu Lys Asp
210 215 220

<210> 246
<211> 669
<212> DNA
<213> vitis vinifera

<400> 246

```
atgcagcaga acccccagat gatacctgtt atgccttctt ttccacccaa caacatcact      60
accgagcaga ttcagaagta tctcgatgag aataaaaaat tgattctggc aatattggac     120
aatcaaaacc ttggaaagct tgctgagtgt gcacagtacc aagctcagct tcaaaagaat     180
ttgatgtatc tagctgcaat tgctgatgct cagccacagg caccaccgac aatgcctccc     240
cagatggccc cacaccctgc aatgcagcag ggagggtact acatgcagca tccccaggcg     300
gcagcaatgg ctcagcaacc tggtcttttc cctcccaaga tgcccttaca atttggtaac     360
ccacatcaac ttcaggagca agcacagcag ctgcagcagc tacagcaaca agccatgcaa     420
gggcagatgg gcatgagacc tggaggggcc aacaacggca tgcatcccat gcatcctgag     480
gccactcttg gtggtggcag cagtggtggc cctccaccat ctgccggcct cagtgatgca     540
cgcggaggtg gcaagcaaga cacttccgaa gcaggggctt ctggtggtga tggtcagggg     600
agctcagctg ctgggcatgg cggcgatggc gaatcaccct acttgaaggg gtcagaggat     660
ggaaagtga                                                             669
```

<210> 247
<211> 222
<212> PRT
<213> vitis vinifera

<400> 247

```
Met Gln Gln Asn Pro Gln Met Ile Pro Val Met Pro Ser Phe Pro Pro
1               5                   10                  15

Asn Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45

Glu Cys Ala Gln Tyr Gln Ala Gln Leu Gln Lys Asn Leu Met Tyr Leu
    50                  55                  60

Ala Ala Ile Ala Asp Ala Gln Pro Gln Ala Pro Pro Thr Met Pro Pro
65                  70                  75                  80

Gln Met Ala Pro His Pro Ala Met Gln Gln Gly Gly Tyr Tyr Met Gln
                85                  90                  95
```

His Pro Gln Ala Ala Ala Met Ala Gln Gln Pro Gly Leu Phe Pro Pro
100 105 110

Lys Met Pro Leu Gln Phe Gly Asn Pro His Gln Leu Gln Glu Gln Ala
115 120 125

Gln Gln Leu Gln Gln Leu Gln Gln Gln Ala Met Gln Gly Gln Met Gly
130 135 140

Met Arg Pro Gly Gly Ala Asn Asn Gly Met His Pro Met His Pro Glu
145 150 155 160

Ala Thr Leu Gly Gly Gly Ser Ser Gly Gly Pro Pro Pro Ser Ala Gly
165 170 175

Leu Ser Asp Ala Arg Gly Gly Gly Lys Gln Asp Thr Ser Glu Ala Gly
180 185 190

Ala Ser Gly Gly Asp Gly Gln Gly Ser Ser Ala Ala Gly His Gly Gly
195 200 205

Asp Gly Glu Ser Pro Tyr Leu Lys Gly Ser Glu Asp Gly Lys
210 215 220

<210> 248
<211> 597
<212> DNA
<213> Vitis vinifera

<400> 248

atgcagcagc aaccaccaca gatgatgaac attgcgcctt catttcctcc caccgccatc 60
accactgagc agattcagaa gtatctggat gagaacaaac aattgattct ggcaattctg 120
gaaaaccaga cccttggaaa actcgccgag tgtgcccaat atcaagccca gcttcagaag 180
aacttgatat atctagctgc aattgctgat gcccaaccac cagcaccaac agtgcctcct 240
cagatgccta tacatcatgc catgcaacaa gggcattaca tgcaacaccc tcaggctgct 300
gcagctcagc aacaaccagg catgtttggt gcaaagttgc ctttccagct tagcgatcag 360
caacagcagc agcagcatca tttttacac ctccaacaac agcaacccat ccaagggctc 420
atgggcatga ggcctatcat caacaatggc atgcatcagg ccatgcaaac tgggcttggt 480
gctttgagcg gtttcatgga tgtacgtgga agcaagccag atggctcaga ggttggtttt 540
ggtgatggcc aagggaagtt tgcttctgga catggcagtg gaaatagaga ttcctaa 597

<210> 249
<211> 198
<212> PRT
<213> vitis vinifera

<400> 249

Met Gln Gln Gln Pro Pro Gln Met Met Asn Ile Ala Pro Ser Phe Pro
1               5                   10                  15

Pro Thr Ala Ile Thr Thr Glu Gln Ile Gln Lys Tyr Leu Asp Glu Asn
            20                  25                  30

Lys Gln Leu Ile Leu Ala Ile Leu Glu Asn Gln Thr Leu Gly Lys Leu
        35                  40                  45

Ala Glu Cys Ala Gln Tyr Gln Ala Gln Leu Gln Lys Asn Leu Ile Tyr
    50                  55                  60

Leu Ala Ala Ile Ala Asp Ala Gln Pro Pro Ala Pro Thr Val Pro Pro
65                  70                  75                  80

Gln Met Pro Ile His His Ala Met Gln Gln Gly His Tyr Met Gln His
                85                  90                  95

Pro Gln Ala Ala Ala Ala Gln Gln Gln Pro Gly Met Phe Gly Ala Lys
            100                 105                 110

Leu Pro Phe Gln Leu Ser Asp Gln Gln Gln Gln Gln Gln His His Phe
        115                 120                 125

Leu His Leu Gln Gln Gln Gln Pro Ile Gln Gly Leu Met Gly Met Arg
    130                 135                 140

Pro Ile Ile Asn Asn Gly Met His Gln Ala Met Gln Thr Gly Leu Gly
145                 150                 155                 160

Ala Leu Ser Gly Phe Met Asp Val Arg Gly Ser Lys Pro Asp Gly Ser
                165                 170                 175

Glu Val Gly Phe Gly Asp Gly Gln Gly Lys Phe Ala Ser Gly His Gly
            180                 185                 190

Ser Gly Asn Arg Asp Ser
        195

<210> 250
<211> 735
<212> DNA
<213> volvox carteri

<400> 250

atggcggcag tgtcaggtca ggccaagcca gccccaatga caacggagcg aatccaagaa 60
atgcttgaag aaaacttcaa gtttatcaag gcactcgccg aacagcaaaa ccttggtcga 120
atgcaggacg tcatccagtt ccagcagaag cttcaagaaa accttatgct actagcagct 180
gtggcggaca cctactcatc agcgtctgca acgggagctg ctgcccaggc gactgcagct 240
cccggcatgg cggcacaggc ggcccggccg cccgctgctg ctctccctgg aacagctgtt 300
gctacggcgg cgctcccgct ccctggcttg ccgcaacaac agcaaccgca gcagcagcag 360
cagccgcagc agcagccagg gcagcccagc ccgctaatga tggtcatgcc gggctcttcc 420

gggacgcagg cgcctacgca actgggggcc atgcaactca cgcagcagca gatacaagct 480
gcggtacagc aagcgctagt ccgacagcag cagcagcaac aacaacagca gcagcaaaat 540
ccaaatccgt ttcaggggca gcaattccag ctgccacagt cacttcagca gccgcagtcc 600
ctgggacagc agccagtttt aagtgcgatg gggggccctgg gagggccctt ggctggtcag 660
gggacggcgg cagggcaggc gggagcaggt ggtttccagc tgcccgcggc gcctcctttg 720
aaccttgggc tttga 735

<210> 251
<211> 244
<212> PRT
<213> volvox carteri

<400> 251

Met Ala Ala Val Ser Gly Gln Ala Lys Pro Ala Pro Met Thr Thr Glu
1 5 10 15

Arg Ile Gln Glu Met Leu Glu Glu Asn Phe Lys Phe Ile Lys Ala Leu
20 25 30

Ala Glu Gln Gln Asn Leu Gly Arg Met Gln Asp Val Ile Gln Phe Gln
35 40 45

Gln Lys Leu Gln Glu Asn Leu Met Leu Leu Ala Ala Val Ala Asp Thr
50 55 60

Tyr Ser Ser Ala Ser Ala Thr Gly Ala Ala Ala Gln Ala Thr Ala Ala
65 70 75 80

Pro Gly Met Ala Ala Gln Ala Ala Arg Pro Pro Ala Ala Ala Leu Pro
85 90 95

Gly Thr Ala Val Ala Thr Ala Ala Leu Pro Leu Pro Gly Leu Pro Gln
100 105 110

Gln Gln Gln Pro Gln Gln Gln Gln Gln Pro Gln Gln Gln Pro Gly Gln
115 120 125

Pro Ser Pro Leu Met Met Val Met Pro Gly Ser Ser Gly Thr Gln Ala
130 135 140

Pro Thr Gln Leu Gly Ala Met Gln Leu Thr Gln Gln Gln Ile Gln Ala
145 150 155 160

Ala Val Gln Gln Ala Leu Val Arg Gln Gln Gln Gln Gln Gln Gln Gln
165 170 175

Gln Gln Gln Asn Pro Asn Pro Phe Gln Gly Gln Gln Phe Gln Leu Pro
180 185 190

Gln Ser Leu Gln Gln Pro Gln Ser Leu Gly Gln Gln Pro Val Leu Ser

195 200 205

Ala Met Gly Ala Leu Gly Gly Pro Leu Ala Gly Gln Gly Thr Ala Ala
210 215 220

Gly Gln Ala Gly Ala Gly Gly Phe Gln Leu Pro Ala Ala Pro Pro Leu
225 230 235 240

Asn Leu Gly Leu

<210> 252
<211> 699
<212> DNA
<213> welwitschia mirabilis

<400> 252

```
atgcaaatgc agcctatgat aggggcagga tactcctcca atagcatcac cactgatcac      60
attcagaagt atttggatga gaataggcaa ttgattctgg caattcttga caaccaaaat     120
cttggaaagt tgaatgaatg tgcacaatac caagccaggc ttcaacaaaa tttaatgtat     180
ttggctgcta ttgctgattc ccaaccgcaa acacctgctg cacatgccca gatcgcatcc     240
aatgcaatgc ttcaagcagg tggtcattat atgcagcacc agcagacagt aactccccag     300
tcacttcttg ctgcaaggtc atctatgctt tacagtcagc aacctatgac tgcattccat     360
caagcacagc agcagcagca acaacaatct ctccatggcc agttggggat aaattcagga     420
ggaaacaatg gattacatat tcttcatggt gaaacaagca tgggtagtaa tggacctctc     480
acgacaggaa gctttcctga ttttgggcga ggttcagtaa attgtgatct attgcagggc     540
aataggagct tgactattga ccgtggctct agcaagattg atgggcttgg aacagagaat     600
acgcaccctg tagaagggcg aggaggagca agtgtgggcc aaaacacaga agaaggggca     660
ccatcttatt tgaaagcttc tgaggatgag ggaagctag                            699
```

<210> 253
<211> 232
<212> PRT
<213> welwitschia mirabilis

<400> 253

```
Met Gln Met Gln Pro Met Ile Gly Ala Gly Tyr Ser Ser Asn Ser Ile
1               5                   10                  15

Thr Thr Asp His Ile Gln Lys Tyr Leu Asp Glu Asn Arg Gln Leu Ile
            20                  25                  30

Leu Ala Ile Leu Asp Asn Gln Asn Leu Gly Lys Leu Asn Glu Cys Ala
        35                  40                  45

Gln Tyr Gln Ala Arg Leu Gln Gln Asn Leu Met Tyr Leu Ala Ala Ile
    50                  55                  60
```

```
Ala Asp Ser Gln Pro Gln Thr Pro Ala Ala His Ala Gln Ile Ala Ser
65                  70                  75                  80

Asn Ala Met Leu Gln Ala Gly Gly His Tyr Met Gln His Gln Gln Thr
                85                  90                  95

Val Thr Pro Gln Ser Leu Leu Ala Ala Arg Ser Ser Met Leu Tyr Ser
            100                 105                 110

Gln Gln Pro Met Thr Ala Phe His Gln Ala Gln Gln Gln Gln Gln Gln
        115                 120                 125

Gln Ser Leu His Gly Gln Leu Gly Ile Asn Ser Gly Gly Asn Asn Gly
    130                 135                 140

Leu His Ile Leu His Gly Glu Thr Ser Met Gly Ser Asn Gly Pro Leu
145                 150                 155                 160

Thr Thr Gly Ser Phe Pro Asp Phe Gly Arg Gly Ser Val Asn Cys Asp
                165                 170                 175

Leu Leu Gln Gly Asn Arg Ser Leu Thr Ile Asp Arg Gly Ser Ser Lys
            180                 185                 190

Ile Asp Gly Leu Gly Thr Glu Asn Thr His Pro Val Glu Gly Arg Gly
        195                 200                 205

Gly Ala Ser Val Gly Gln Asn Thr Glu Glu Gly Ala Pro Ser Tyr Leu
    210                 215                 220

Lys Ala Ser Glu Asp Glu Gly Ser
225                 230
```

<210> 254
<211> 684
<212> DNA
<213> Zea mays

<400> 254

atgcagcagc aacacctgat gcagatgaac cagaacatga tggggggcta cacctctcct 60
gccgccgtga ccaccgatct catccagcag cacctggacg agaacaagca gctgatcctg 120
gccatcctcg acaaccagaa caatggcaag gcggaggagt gcgaacggca ccaagctaag 180
ctccagcaca acctcatgta cctggccgcc atcgctgaca gccagccgcc acagaccgcg 240
ccactatcac agtacccgtc caacctgatg atgcagccgg gccctcggta catgccaccg 300
cagtccgggc agatgatgaa cccgcagtcg ctgatggcgg cgcggtcctc catgatgtac 360
gcgcacccgt ccctgtcgcc actccagcag cagcaggcgg cgcacggaca gctgggtatg 420
gctccagggg gcggcggtgg cggcacgacc agcgggttca gcatcctcca cggcgaggcc 480
agcatgggcg gtggtggtgc tggcgcaggc gccggcaaca acatgatgaa cgccggcatg 540
ttctcgggct ttggccgcag cggcagtggc gccaaggaag ggtcgacctc tctgtcggtt 600

gacgtccggg gtggaaccag ctccggcgcg cagagcgggg acggcgagta cctcaaagtc 660
ggcaccgagg aagaaggcag ttag 684

<210> 255
<211> 227
<212> PRT
<213> Zea mays

<400> 255

Met Gln Gln Gln His Leu Met Gln Met Asn Gln Asn Met Met Gly Gly
1               5                   10                  15

Tyr Thr Ser Pro Ala Ala Val Thr Thr Asp Leu Ile Gln Gln His Leu
            20                  25                  30

Asp Glu Asn Lys Gln Leu Ile Leu Ala Ile Leu Asp Asn Gln Asn Asn
        35                  40                  45

Gly Lys Ala Glu Glu Cys Glu Arg His Gln Ala Lys Leu Gln His Asn
    50                  55                  60

Leu Met Tyr Leu Ala Ala Ile Ala Asp Ser Gln Pro Pro Gln Thr Ala
65                  70                  75                  80

Pro Leu Ser Gln Tyr Pro Ser Asn Leu Met Met Gln Pro Gly Pro Arg
                85                  90                  95

Tyr Met Pro Pro Gln Ser Gly Gln Met Met Asn Pro Gln Ser Leu Met
            100                 105                 110

Ala Ala Arg Ser Ser Met Met Tyr Ala His Pro Ser Leu Ser Pro Leu
        115                 120                 125

Gln Gln Gln Gln Ala Ala His Gly Gln Leu Gly Met Ala Pro Gly Gly
    130                 135                 140

Gly Gly Gly Gly Thr Thr Ser Gly Phe Ser Ile Leu His Gly Glu Ala
145                 150                 155                 160

Ser Met Gly Gly Gly Gly Ala Gly Ala Gly Ala Gly Asn Asn Met Met
                165                 170                 175

Asn Ala Gly Met Phe Ser Gly Phe Gly Arg Ser Gly Ser Gly Ala Lys
            180                 185                 190

Glu Gly Ser Thr Ser Leu Ser Val Asp Val Arg Gly Gly Thr Ser Ser
        195                 200                 205

Gly Ala Gln Ser Gly Asp Gly Glu Tyr Leu Lys Val Gly Thr Glu Glu
    210                 215                 220

Glu Gly Ser
225

<210> 256
<211> 549
<212> DNA
<213> Zea mays

<400> 256

atgcagcagc cgatgcacat gcagccacag gcgccggcga taaccccagc tgccggaatc 60
agcacggagc agatccaaaa gtatctggat gagaataagc agcttatttt ggctattttg 120
gaaaatcaga acctaggaaa attggcagaa tgtgctcagt atcaatcaca acttcagaag 180
aacctcttgt atctcgctgc aatcgcagat gctcaaccgc agactgctgt aagccgccct 240
cagatggcgc cgcctggtgg atcgcctgga gtagggcagt acatgtcaca ggtgcctatg 300
ttcccaccga ggacacctct tacaccccag cagatgcagg agcagcagct tcagcagcag 360
caggctcagt tgctaaactt cagtggccaa atggttgcta gaccaggcat ggtcaacggc 420
atggctcagt ccatgcaagc tcagctacca ccgggtgtga acaagcagga tgctggtggg 480
gtcgcctctg agccctcggg caccgagagc cacaggagca ctggtggtga cgatggtgga 540
agcgactag 549

<210> 257
<211> 182
<212> PRT
<213> Zea mays

<400> 257

Met Gln Gln Pro Met His Met Gln Pro Gln Ala Pro Ala Ile Thr Pro
1 5 10 15

Ala Ala Gly Ile Ser Thr Glu Gln Ile Gln Lys Tyr Leu Asp Glu Asn
20 25 30

Lys Gln Leu Ile Leu Ala Ile Leu Glu Asn Gln Asn Leu Gly Lys Leu
35 40 45

Ala Glu Cys Ala Gln Tyr Gln Ser Gln Leu Gln Lys Asn Leu Leu Tyr
50 55 60

Leu Ala Ala Ile Ala Asp Ala Gln Pro Gln Thr Ala Val Ser Arg Pro
65 70 75 80

Gln Met Ala Pro Pro Gly Gly Ser Pro Gly Val Gly Gln Tyr Met Ser
85 90 95

Gln Val Pro Met Phe Pro Pro Arg Thr Pro Leu Thr Pro Gln Gln Met
100 105 110

Gln Glu Gln Gln Leu Gln Gln Gln Gln Ala Gln Leu Leu Asn Phe Ser
115 120 125

Gly Gln Met Val Ala Arg Pro Gly Met Val Asn Gly Met Ala Gln Ser
130 135 140

Met Gln Ala Gln Leu Pro Pro Gly Val Asn Lys Gln Asp Ala Gly Gly
145 150 155 160

Val Ala Ser Glu Pro Ser Gly Thr Glu Ser His Arg Ser Thr Gly Gly
165 170 175

Asp Asp Gly Gly Ser Asp
180

<210> 258
<211> 663
<212> DNA
<213> Zea mays

<400> 258

atgcagcagc agatgcccat gccgccggcg cccgctgccg ccgcggcggc ggcgcccccg 60
gcggcaggca tcactaccga gcagatccag aagtatttgg acgaaaataa gcaacttatt 120
ttggccatcc tggaaaatca gaacttaggg aagttggctg aatgtgctca gtatcaagct 180
caacttcaaa agaacctctt gtacctggct gcgattgctg atgcccaacc ccagcctccg 240
caaaaccctg caggtcgccc tcagatgatg cagcctggta tagtgccagg tgcggggcat 300
tacatgtcac aagtaccaat gttccctcca agaaccccat taaccccaca gcagatgcag 360
gagcagcagc aacaacaaca gtttcagcag cagcagcagc aagtgcaggc tcttacattt 420
cctggacaga tggtcatgag accaggcacc atcaacggca tgcagcagca gcagcctatg 480
caggctgacc ctgcccgggc agcagcggag ctgcagcagg cagcacctat cccagctgac 540
gggcgaggaa gcaagcagga caccgcgggt ggggcgagct cagagccttc tgccaatgag 600
agccacaaga gcgccaccgg agcagatacc gaggcaggtg gcgacgtggc cgagaaatcc 660
taa 663

<210> 259
<211> 220
<212> PRT
<213> Zea mays

<400> 259

Met Gln Gln Gln Met Pro Met Pro Pro Ala Pro Ala Ala Ala Ala Ala
1               5                   10                  15

Ala Ala Pro Pro Ala Ala Gly Ile Thr Thr Glu Gln Ile Gln Lys Tyr
            20                  25                  30

Leu Asp Glu Asn Lys Gln Leu Ile Leu Ala Ile Leu Glu Asn Gln Asn
        35                  40                  45

Leu Gly Lys Leu Ala Glu Cys Ala Gln Tyr Gln Ala Gln Leu Gln Lys
    50                  55                  60

Asn Leu Leu Tyr Leu Ala Ala Ile Ala Asp Ala Gln Pro Gln Pro Pro
65                  70                  75                  80

Gln Asn Pro Ala Gly Arg Pro Gln Met Met Gln Pro Gly Ile Val Pro
                85                  90                  95

Gly Ala Gly His Tyr Met Ser Gln Val Pro Met Phe Pro Pro Arg Thr
            100                 105                 110

Pro Leu Thr Pro Gln Gln Met Gln Glu Gln Gln Gln Gln Gln Gln Phe
        115                 120                 125

Gln Gln Gln Gln Gln Gln Val Gln Ala Leu Thr Phe Pro Gly Gln Met
    130                 135                 140

Val Met Arg Pro Gly Thr Ile Asn Gly Met Gln Gln Gln Gln Pro Met
145                 150                 155                 160

Gln Ala Asp Pro Ala Arg Ala Ala Ala Glu Leu Gln Gln Ala Ala Pro
                165                 170                 175

Ile Pro Ala Asp Gly Arg Gly Ser Lys Gln Asp Thr Ala Gly Gly Ala
            180                 185                 190

Ser Ser Glu Pro Ser Ala Asn Glu Ser His Lys Ser Ala Thr Gly Ala
        195                 200                 205

Asp Thr Glu Ala Gly Gly Asp Val Ala Glu Lys Ser
    210                 215                 220

<210> 260
<211> 1173
<212> DNA
<213> Homo sapiens

<400> 260

atgggcggca acatgtctgt ggctttcgcg gccccgaggc agcgaggcaa gggggagatc 60
actcccgctg cgattcagaa gatgttggat gacaataacc atcttattca gtgtataatg 120
gactctcaga ataaaggaaa gacctcagag tgttctcagt atcagcagat gttgcacaca 180
aacttggtat accttgctac aatagcagat tctaatcaaa atatgcagtc tcttttacca 240
gcaccaccca cacagaatat gcctatgggt cctggaggga tgaatcagag cggccctccc 300
ccacctccac gctctcacaa catgccttca gatggaatgg taggtggggg tcctcctgca 360
ccgcacatgc agaaccagat gaacggccag atgcctgggc ctaaccatat gcctatgcag 420
ggacctggac ccaatcaact caatatgaca aacagttcca tgaatatgcc ttcaagtagc 480
catggatcca tgggaggtta caaccattct gtgccatcat cacagagcat gccagtacag 540
aatcagatga caatgagtca gggacaacca atgggaaact atggtcccag accaaatatg 600
agtatgcagc caaaccaagg tccaatgatg catcagcagc ctccttctca gcaatacaat 660

atgccacagg gaggcggaca gcattaccaa ggacagcagc cacctatggg aatgatgggt 720
caagttaacc aaggcaatca tatgatgggt cagagacaga ttcctcccta tagacctcct 780
caacagggcc caccacagca gtactcaggc caggaagact attacgggga ccaatacagt 840
catggtggac aaggtcctcc agaaggcatg aaccagcaat attaccctga tggaaattca 900
cagtatggcc aacagcaaga tgcataccag ggaccacctc cacaacaggg atatccaccc 960
cagcagcagc agtacccagg gcagcaaggt tacccaggac agcagcaggg ctacggtcct 1020
tcacagggtg gtccaggtcc tcagtatcct aactacccac agggacaagg tcagcagtat 1080
ggaggatata gaccaacaca gcctggacca ccacagccac cccagcagag gccttatgga 1140
tatgaccagg gacagtatgg aaattaccag cag 1173

<210> 261
<211> 391
<212> PRT
<213> Homo sapiens

<400> 261

Met Gly Gly Asn Met Ser Val Ala Phe Ala Ala Pro Arg Gln Arg Gly
1               5                   10                  15

Lys Gly Glu Ile Thr Pro Ala Ala Ile Gln Lys Met Leu Asp Asp Asn
            20                  25                  30

Asn His Leu Ile Gln Cys Ile Met Asp Ser Gln Asn Lys Gly Lys Thr
        35                  40                  45

Ser Glu Cys Ser Gln Tyr Gln Gln Met Leu His Thr Asn Leu Val Tyr
    50                  55                  60

Leu Ala Thr Ile Ala Asp Ser Asn Gln Asn Met Gln Ser Leu Leu Pro
65                  70                  75                  80

Ala Pro Pro Thr Gln Asn Met Pro Met Gly Pro Gly Gly Met Asn Gln
                85                  90                  95

Ser Gly Pro Pro Pro Pro Pro Arg Ser His Asn Met Pro Ser Asp Gly
            100                 105                 110

Met Val Gly Gly Gly Pro Pro Ala Pro His Met Gln Asn Gln Met Asn
        115                 120                 125

Gly Gln Met Pro Gly Pro Asn His Met Pro Met Gln Gly Pro Gly Pro
    130                 135                 140

Asn Gln Leu Asn Met Thr Asn Ser Ser Met Asn Met Pro Ser Ser Ser
145                 150                 155                 160

His Gly Ser Met Gly Gly Tyr Asn His Ser Val Pro Ser Ser Gln Ser
                165                 170                 175

Met Pro Val Gln Asn Gln Met Thr Met Ser Gln Gly Gln Pro Met Gly
180 185 190

Asn Tyr Gly Pro Arg Pro Asn Met Ser Met Gln Pro Asn Gln Gly Pro
195 200 205

Met Met His Gln Gln Pro Pro Ser Gln Gln Tyr Asn Met Pro Gln Gly
210 215 220

Gly Gly Gln His Tyr Gln Gly Gln Gln Pro Pro Met Gly Met Met Gly
225 230 235 240

Gln Val Asn Gln Gly Asn His Met Met Gly Gln Arg Gln Ile Pro Pro
245 250 255

Tyr Arg Pro Pro Gln Gln Gly Pro Pro Gln Gln Tyr Ser Gly Gln Glu
260 265 270

Asp Tyr Tyr Gly Asp Gln Tyr Ser His Gly Gly Gln Gly Pro Pro Glu
275 280 285

Gly Met Asn Gln Gln Tyr Tyr Pro Asp Gly Asn Ser Gln Tyr Gly Gln
290 295 300

Gln Gln Asp Ala Tyr Gln Gly Pro Pro Pro Gln Gln Gly Tyr Pro Pro
305 310 315 320

Gln Gln Gln Gln Tyr Pro Gly Gln Gln Gly Tyr Pro Gly Gln Gln Gln
325 330 335

Gly Tyr Gly Pro Ser Gln Gly Gly Pro Gly Pro Gln Tyr Pro Asn Tyr
340 345 350

Pro Gln Gly Gln Gly Gln Gln Tyr Gly Gly Tyr Arg Pro Thr Gln Pro
355 360 365

Gly Pro Pro Gln Pro Pro Gln Gln Arg Pro Tyr Gly Tyr Asp Gln Gly
370 375 380

Gln Tyr Gly Asn Tyr Gln Gln
385 390

<210> 262
<211> 54
<212> PRT
<213> Artificial sequence

<220>
<223> SNH domain from Arath_SYT1 (comprised in SEQ ID NO 121)

<400> 262

Val Thr Ser Asp His Ile Gln Gln Tyr Leu Asp Glu Asn Lys Ser Leu

1 5 10 15

Ile Leu Lys Ile Val Glu Ser Gln Asn Ser Gly Lys Leu Ser Glu Cys
20 25 30

Ala Glu Asn Gln Ala Arg Leu Gln Arg Asn Leu Met Tyr Leu Ala Ala
35 40 45

Ile Ala Asp Ala Gln Thr
50

<210> 263
<211> 45
<212> PRT
<213> Artificial sequence

<220>
<223> most conserved residues comprised in the SNH domain

<220>
<221> UNSURE
<222> (2)..(3)
<223> Xaa can be any naturally ocurring amino acid

<220>
<221> UNSURE
<222> (5)..(6)
<223> Xaa can be any naturally ocurring amino acid

<220>
<221> UNSURE
<222> (8)..(10)
<223> Xaa can be any naturally ocurring amino acid

<220>
<221> UNSURE
<222> (12)..(20)
<223> Xaa can be any naturally ocurring amino acid

<220>
<221> UNSURE
<222> (22)..(29)
<223> Xaa can be any naturally ocurring amino acid

<220>
<221> UNSURE
<222> (31)..(32)
<223> Xaa can be any naturally ocurring amino acid

<220>
<221> UNSURE
<222> (34)..(35)
<223> Xaa can be any naturally ocurring amino acid

<220>
<221> UNSURE

<222> (38)..(39)
<223> Xaa can be any naturally ocurring amino acid

<220>
<221> UNSURE
<222> (42)..(43)
<223> Xaa can be any naturally ocurring amino acid

<400> 263

Gln Xaa Xaa Leu Xaa Xaa Asn Xaa Xaa Xaa Ile Xaa Xaa Xaa Xaa Xaa
1 5 10 15

Xaa Xaa Xaa Xaa Gly Xaa Xaa Xaa Xaa Xaa Xaa Xaa Xaa Gln Xaa Xaa
20 25 30

Leu Xaa Xaa Asn Leu Xaa Xaa Leu Ala Xaa Xaa Ala Asp
35 40 45

<210> 264
<211> 74
<212> PRT
<213> Artificial sequence

<220>
<223> SSXT domain InterPro007726 (PFam05030) comprised in SEQ ID NO 121

<400> 264

Gln Met Gln Pro Met Met Ala Gly Tyr Tyr Pro Ser Asn Val Thr Ser
1 5 10 15

Asp His Ile Gln Gln Tyr Leu Asp Glu Asn Lys Ser Leu Ile Leu Lys
20 25 30

Ile Val Glu Ser Gln Asn Ser Gly Lys Leu Ser Glu Cys Ala Glu Asn
35 40 45

Gln Ala Arg Leu Gln Arg Asn Leu Met Tyr Leu Ala Ala Ile Ala Asp
50 55 60

Ser Gln Pro Gln Pro Pro Ser Val His Ser
65 70

<210> 265
<211> 53
<212> DNA
<213> Artificial sequence

<220>
<223> primer: prm06681

<400> 265
ggggacaagt ttgtacaaaa aagcaggctt aaacaatgca acagcacctg atg 53

<210> 266
<211> 50
<212> DNA
<213> Artificial sequence

<220>
<223> primer: prm06682

<400> 266
ggggaccact ttgtacaaga aagctgggtc atcattaaga ttccttgtgc 50

<210> 267
<211> 1828
<212> DNA
<213> Artificial sequence

<220>
<223> SEQ ID NO: 1 + SEQ ID NO: 120

<220>
<221> Unsure
<222> (1195)..(1195)
<223> n can be no nucleotide to any number of nucleotides

<220>
<221> misc_feature
<222> (1195)..(1195)
<223> n is a, c, g, or t

<400> 267

atgatgagtc taagtggaag tagcgggaga acaataggaa ggcctccatt tacaccaaca 60
caatgggaag aactggaaca tcaagcccta atctacaagt acatggtctc tggtgttcct 120
gtcccacctg agctcatctt ctccattaga agaagcttgg acacttcctt ggtctctaga 180
ctccttcctc accaatccct tggatggggg tgttaccaga tgggatttgg gagaaaacca 240
gatccagagc caggaagatg cagaagaaca gatggtaaga aatggagatg ctcaagagaa 300
gcttacccag attcgaagta ctgtgaaaaa cacatgcaca gaggaagaaa ccgtgccaga 360
aaatctcttg atcagaatca gacaacaaca actcctttaa catcaccatc tctctcattc 420
accaacaaca acaacccaag tcccaccttg tcttcttctt cttcctctaa ttcctcttct 480
actacttatt ctgcttcttc ttcttcaatg gatgcctaca gtaacagtaa taggtttggg 540
cttggtggaa gtagtagtaa cactagaggt tatttcaaca gccattctct tgattatcct 600
tatccttcta cttcacccaa acaacaacaa caaactcttc atcatgcttc cgctttgtca 660
cttcatcaaa atactaattc tacttctcag ttcaatgtct tagcctctgc tactgaccac 720
aaagacttca ggtactttca agggattggg gagagagttg gaggagttgg ggagagaacg 780
ttctttccag aagcatctag aagctttcaa gattctccat accatcatca ccaacaaccg 840
ttagcaacag tgatgaatga tccgtaccac cactgtagta ctgatcataa taagattgat 900
catcatcaca catactcatc ctcatcatca tctcaacatc ttcatcatga tcatgatcat 960
agacagcaac agtgttttgt tttgggcgcc gacatgttca acaaacctac aagaagtgtc 1020
cttgcaaact catcaagaca agatcaaaat caagaagaag atgagaaaga ttcatcagag 1080
tcgtccaaga agtctctaca tcacttcttt ggtgaggact gggcacagaa caagaacagt 1140
tcagattctt ggcttgacct ttcttcccac tcaagactcg acactggtag ctaanatgca 1200
acagcacctg atgcagatgc agcccatgat ggctggttac taccccagca atgttacctc 1260
tgatcatatc caacagtact tggacgaaaa caaatcgttg attctgaaga ttgttgagtc 1320
tcaaaactct ggaaagctta gcgaatgcgc cgagaatcaa gcaaggcttc aacgcaacct 1380
aatgtaccta gctgcaatag cagattctca gcctcagcca ccaagtgtgc atagccagta 1440
tggatctgct ggtggtggga tgattcaggg agaaggaggg tcacactatt tgcagcagca 1500

acaagcgact caacagcaac agatgactca gcagtctcta atggcggctc gatcttcaat 1560
gttgtatgct cagcaacagc ggcagcagca gccttacgcg acgcttcagc atcagcaatc 1620
gcaccatagc cagcttggaa tgagctcgag cagcggagga ggaggaagca gtggtctcca 1680
tatccttcag ggagaggctg gtgggtttca tgattttggc cgtgggaagc cggaaatggg 1740
aagtggtggt ggcggtgaag gcagaggagg aagttcaggg gatggtggag aaaccctttta 1800
cttgaaatca tcagatgatg ggaattga 1828

<210> 268
<211> 1828
<212> DNA
<213> Artificial sequence

<220>
<223> SEQ ID NO: 120 + SEQ ID NO: 1

<220>
<221> Unsure
<222> (634)..(634)
<223> n can be no nucleotide to any number of nucleotides

<220>
<221> misc_feature
<222> (634)..(634)
<223> n is a, c, g, or t

<400> 268

```
atgcaacagc acctgatgca gatgcagccc atgatggctg gttactaccc cagcaatgtt      60
acctctgatc atatccaaca gtacttggac gaaaacaaat cgttgattct gaagattgtt     120
gagtctcaaa actctggaaa gcttagcgaa tgcgccgaga atcaagcaag gcttcaacgc     180
aacctaatgt acctagctgc aatagcagat tctcagcctc agccaccaag tgtgcatagc     240
cagtatggat ctgctggtgg tgggatgatt cagggagaag gagggtcaca ctatttgcag     300
cagcaacaag cgactcaaca gcaacagatg actcagcagt ctctaatggc ggctcgatct     360
tcaatgttgt atgctcagca acagcggcag cagcagcctt acgcgacgct tcagcatcag     420
caatcgcacc atagccagct tggaatgagc tcgagcagcg gaggaggagg aagcagtggt     480
ctccatatcc ttcagggaga ggctggtggg tttcatgatt ttggccgtgg gaagccggaa     540
atgggaagtg gtggtggcgg tgaaggcaga ggaggaagtt caggggatgg tggagaaacc     600
ctttacttga aatcatcaga tgatgggaat tganatgatg agtctaagtg gaagtagcgg     660
gagaacaata ggaaggcctc catttacacc aacacaatgg gaagaactgg aacatcaagc     720
cctaatctac aagtacatgg tctctggtgt tcctgtccca cctgagctca tcttctccat     780
tagaagaagc ttggacactt ccttggtctc tagactcctt cctcaccaat cccttggatg     840
ggggtgttac cagatgggat ttgggagaaa accagatcca gagccaggaa gatgcagaag     900
aacagatggt aagaaatgga gatgctcaag agaagcttac ccagattcga agtactgtga     960
aaaacacatg cacagaggaa gaaaccgtgc cagaaaatct cttgatcaga atcagacaac    1020
aacaactcct ttaacatcac catctctctc attcaccaac aacaacaacc caagtcccac    1080
```

cttgtcttct tcttcttcct ctaattcctc ttctactact tattctgctt cttcttcttc 1140
aatggatgcc tacagtaaca gtaataggtt tgggcttggt ggaagtagta gtaacactag 1200
aggttatttc aacagccatt ctcttgatta tccttatcct tctacttcac ccaaacaaca 1260
acaacaaact cttcatcatg cttccgcttt gtcacttcat caaaatacta attctacttc 1320
tcagttcaat gtcttagcct ctgctactga ccacaaagac ttcaggtact ttcaagggat 1380
tggggagaga gttggaggag ttggggagag aacgttcttt ccagaagcat ctagaagctt 1440
tcaagattct ccataccatc atcaccaaca accgttagca acagtgatga atgatccgta 1500
ccaccactgt agtactgatc ataataagat tgatcatcat cacacatact catcctcatc 1560
atcatctcaa catcttcatc atgatcatga tcatagacag caacagtgtt ttgttttggg 1620
cgccgacatg ttcaacaaac ctacaagaag tgtccttgca aactcatcaa gacaagatca 1680
aaatcaagaa gaagatgaga aagattcatc agagtcgtcc aagaagtctc tacatcactt 1740
ctttggtgag gactgggcac agaacaagaa cagttcagat tcttggcttg acctttcttc 1800
ccactcaaga ctcgacactg gtagctaa 1828

<210> 269
<211> 608
<212> PRT
<213> Artificial sequence

<220>
<223> SEQ ID NO: 2 + SEQ ID NO: 121

<220>
<221> UNSURE
<222> (398)..(398)
<223> Xaa can be no amino acid to any number of aminod acids

<220>
<221> UNSURE
<222> (398)..(398)
<223> Xaa can be no amino acid to any number of amino acids

<400> 269

Met Met Ser Leu Ser Gly Ser Ser Gly Arg Thr Ile Gly Arg Pro Pro
1 5 10 15

Phe Thr Pro Thr Gln Trp Glu Glu Leu Glu His Gln Ala Leu Ile Tyr
20 25 30

Lys Tyr Met Val Ser Gly Val Pro Val Pro Pro Glu Leu Ile Phe Ser
35 40 45

Ile Arg Arg Ser Leu Asp Thr Ser Leu Val Ser Arg Leu Leu Pro His
50 55 60

Gln Ser Leu Gly Trp Gly Cys Tyr Gln Met Gly Phe Gly Arg Lys Pro
65 70 75 80

Asp Pro Glu Pro Gly Arg Cys Arg Arg Thr Asp Gly Lys Lys Trp Arg

85 90 95

Cys Ser Arg Glu Ala Tyr Pro Asp Ser Lys Tyr Cys Glu Lys His Met
100 105 110

His Arg Gly Arg Asn Arg Ala Arg Lys Ser Leu Asp Gln Asn Gln Thr
115 120 125

Thr Thr Thr Pro Leu Thr Ser Pro Ser Leu Ser Phe Thr Asn Asn Asn
130 135 140

Asn Pro Ser Pro Thr Leu Ser Ser Ser Ser Ser Ser Asn Ser Ser Ser
145 150 155 160

Thr Thr Tyr Ser Ala Ser Ser Ser Ser Met Asp Ala Tyr Ser Asn Ser
165 170 175

Asn Arg Phe Gly Leu Gly Gly Ser Ser Ser Asn Thr Arg Gly Tyr Phe
180 185 190

Asn Ser His Ser Leu Asp Tyr Pro Tyr Pro Ser Thr Ser Pro Lys Gln
195 200 205

Gln Gln Gln Thr Leu His His Ala Ser Ala Leu Ser Leu His Gln Asn
210 215 220

Thr Asn Ser Thr Ser Gln Phe Asn Val Leu Ala Ser Ala Thr Asp His
225 230 235 240

Lys Asp Phe Arg Tyr Phe Gln Gly Ile Gly Glu Arg Val Gly Gly Val
245 250 255

Gly Glu Arg Thr Phe Phe Pro Glu Ala Ser Arg Ser Phe Gln Asp Ser
260 265 270

Pro Tyr His His His Gln Gln Pro Leu Ala Thr Val Met Asn Asp Pro
275 280 285

Tyr His His Cys Ser Thr Asp His Asn Lys Ile Asp His His His Thr
290 295 300

Tyr Ser Ser Ser Ser Ser Ser Gln His Leu His His Asp His Asp His
305 310 315 320

Arg Gln Gln Gln Cys Phe Val Leu Gly Ala Asp Met Phe Asn Lys Pro
325 330 335

Thr Arg Ser Val Leu Ala Asn Ser Ser Arg Gln Asp Gln Asn Gln Glu
340 345 350

Glu Asp Glu Lys Asp Ser Ser Glu Ser Ser Lys Lys Ser Leu His His
355 360 365

```
Phe Phe Gly Glu Asp Trp Ala Gln Asn Lys Asn Ser Ser Asp Ser Trp
    370                 375                 380

Leu Asp Leu Ser Ser His Ser Arg Leu Asp Thr Gly Ser Xaa Met Gln
385                 390                 395                 400

Gln His Leu Met Gln Met Gln Pro Met Met Ala Gly Tyr Tyr Pro Ser
                405                 410                 415

Asn Val Thr Ser Asp His Ile Gln Gln Tyr Leu Asp Glu Asn Lys Ser
            420                 425                 430

Leu Ile Leu Lys Ile Val Glu Ser Gln Asn Ser Gly Lys Leu Ser Glu
        435                 440                 445

Cys Ala Glu Asn Gln Ala Arg Leu Gln Arg Asn Leu Met Tyr Leu Ala
    450                 455                 460

Ala Ile Ala Asp Ser Gln Pro Gln Pro Pro Ser Val His Ser Gln Tyr
465                 470                 475                 480

Gly Ser Ala Gly Gly Gly Met Ile Gln Gly Glu Gly Gly Ser His Tyr
                485                 490                 495

Leu Gln Gln Gln Gln Ala Thr Gln Gln Gln Gln Met Thr Gln Gln Ser
            500                 505                 510

Leu Met Ala Ala Arg Ser Ser Met Leu Tyr Ala Gln Gln Gln Arg Gln
        515                 520                 525

Gln Gln Pro Tyr Ala Thr Leu Gln His Gln Gln Ser His His Ser Gln
    530                 535                 540

Leu Gly Met Ser Ser Ser Ser Gly Gly Gly Gly Ser Ser Gly Leu His
545                 550                 555                 560

Ile Leu Gln Gly Glu Ala Gly Gly Phe His Asp Phe Gly Arg Gly Lys
                565                 570                 575

Pro Glu Met Gly Ser Gly Gly Gly Gly Glu Gly Arg Gly Gly Ser Ser
            580                 585                 590

Gly Asp Gly Gly Glu Thr Leu Tyr Leu Lys Ser Ser Asp Asp Gly Asn
        595                 600                 605
```

<210> 270
<211> 608
<212> PRT
<213> Artificial sequence

<220>
<223> SEQ ID NO: 121 + SEQ ID NO: 2

<220>

<221> UNSURE
<222> (211)..(211)
<223> Xaa can be no amino acid to any number of aminod acids

<220>
<221> UNSURE
<222> (211)..(211)
<223> Xaa can be no amino acid to any number of amino acids

<400> 270

```
Met Gln Gln His Leu Met Gln Met Gln Pro Met Met Ala Gly Tyr Tyr
1               5                   10                  15

Pro Ser Asn Val Thr Ser Asp His Ile Gln Gln Tyr Leu Asp Glu Asn
            20                  25                  30

Lys Ser Leu Ile Leu Lys Ile Val Glu Ser Gln Asn Ser Gly Lys Leu
        35                  40                  45

Ser Glu Cys Ala Glu Asn Gln Ala Arg Leu Gln Arg Asn Leu Met Tyr
    50                  55                  60

Leu Ala Ala Ile Ala Asp Ser Gln Pro Gln Pro Pro Ser Val His Ser
65                  70                  75                  80

Gln Tyr Gly Ser Ala Gly Gly Gly Met Ile Gln Gly Glu Gly Gly Ser
                85                  90                  95

His Tyr Leu Gln Gln Gln Gln Ala Thr Gln Gln Gln Gln Met Thr Gln
            100                 105                 110

Gln Ser Leu Met Ala Ala Arg Ser Ser Met Leu Tyr Ala Gln Gln Gln
        115                 120                 125

Arg Gln Gln Gln Pro Tyr Ala Thr Leu Gln His Gln Gln Ser His His
    130                 135                 140

Ser Gln Leu Gly Met Ser Ser Ser Ser Gly Gly Gly Gly Ser Ser Gly
145                 150                 155                 160

Leu His Ile Leu Gln Gly Glu Ala Gly Gly Phe His Asp Phe Gly Arg
                165                 170                 175

Gly Lys Pro Glu Met Gly Ser Gly Gly Gly Gly Glu Gly Arg Gly Gly
            180                 185                 190

Ser Ser Gly Asp Gly Gly Glu Thr Leu Tyr Leu Lys Ser Ser Asp Asp
        195                 200                 205

Gly Asn Xaa Met Met Ser Leu Ser Gly Ser Ser Gly Arg Thr Ile Gly
    210                 215                 220
```

Arg Pro Pro Phe Thr Pro Thr Gln Trp Glu Glu Leu Glu His Gln Ala
225 230 235 240

Leu Ile Tyr Lys Tyr Met Val Ser Gly Val Pro Val Pro Pro Glu Leu
245 250 255

Ile Phe Ser Ile Arg Arg Ser Leu Asp Thr Ser Leu Val Ser Arg Leu
260 265 270

Leu Pro His Gln Ser Leu Gly Trp Gly Cys Tyr Gln Met Gly Phe Gly
275 280 285

Arg Lys Pro Asp Pro Glu Pro Gly Arg Cys Arg Arg Thr Asp Gly Lys
290 295 300

Lys Trp Arg Cys Ser Arg Glu Ala Tyr Pro Asp Ser Lys Tyr Cys Glu
305 310 315 320

Lys His Met His Arg Gly Arg Asn Arg Ala Arg Lys Ser Leu Asp Gln
325 330 335

Asn Gln Thr Thr Thr Thr Pro Leu Thr Ser Pro Ser Leu Ser Phe Thr
340 345 350

Asn Asn Asn Asn Pro Ser Pro Thr Leu Ser Ser Ser Ser Ser Ser Asn
355 360 365

Ser Ser Ser Thr Thr Tyr Ser Ala Ser Ser Ser Ser Met Asp Ala Tyr
370 375 380

Ser Asn Ser Asn Arg Phe Gly Leu Gly Gly Ser Ser Ser Asn Thr Arg
385 390 395 400

Gly Tyr Phe Asn Ser His Ser Leu Asp Tyr Pro Tyr Pro Ser Thr Ser
405 410 415

Pro Lys Gln Gln Gln Gln Thr Leu His His Ala Ser Ala Leu Ser Leu
420 425 430

His Gln Asn Thr Asn Ser Thr Ser Gln Phe Asn Val Leu Ala Ser Ala
435 440 445

Thr Asp His Lys Asp Phe Arg Tyr Phe Gln Gly Ile Gly Glu Arg Val
450 455 460

Gly Gly Val Gly Glu Arg Thr Phe Phe Pro Glu Ala Ser Arg Ser Phe
465 470 475 480

Gln Asp Ser Pro Tyr His His His Gln Gln Pro Leu Ala Thr Val Met
485 490 495

Asn Asp Pro Tyr His His Cys Ser Thr Asp His Asn Lys Ile Asp His
500 505 510

His His Thr Tyr Ser Ser Ser Ser Ser Ser Gln His Leu His His Asp
515 520 525

His Asp His Arg Gln Gln Gln Cys Phe Val Leu Gly Ala Asp Met Phe
530 535 540

Asn Lys Pro Thr Arg Ser Val Leu Ala Asn Ser Ser Arg Gln Asp Gln
545 550 555 560

Asn Gln Glu Glu Asp Glu Lys Asp Ser Ser Glu Ser Ser Lys Lys Ser
565 570 575

Leu His His Phe Phe Gly Glu Asp Trp Ala Gln Asn Lys Asn Ser Ser
580 585 590

Asp Ser Trp Leu Asp Leu Ser Ser His Ser Arg Leu Asp Thr Gly Ser
595 600 605

## Description

The present invention relates generally to the field of molecular biology and concerns a method for increasing various plant yield-related traits by increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, wherein said yield-related traits are increased relative to plants having increased expression of one of: (i) a nucleic acid sequence encoding a GRF polypeptide, or (ii) a nucleic acid sequence encoding a SYT polypeptide. The present invention also concerns plants having increased expression of (i) a nucleic acid sequence encoding a GRF polypeptide; and of (ii) a nucleic acid sequence encoding a SYT polypeptide, wherein said plants have increased yield-related traits relative to plants having increased expression of one of: (i) a nucleic acid sequence encoding a GRF polypeptide; or (ii) a nucleic acid sequence encoding a SYT polypeptide. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as com, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

Another trait of importance is that of improved abiotic stress tolerance. Abiotic stress is a primary cause of crop loss worldwide, reducing average yields for most major crop plants by more than 50% (Wang et al. (2003) Planta 218: 1-14). Abiotic stresses may be caused by drought, salinity, extremes of temperature, chemical toxicity, excess or deficiency of nutrients (macroelements and/or microelements), radiation and oxidative stress. The ability to increase plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increase yield-related traits (seed yield and/or biomass) in plants may be through modification of the inherent growth mechanisms of a plant, such as the cell cycle or various signalling pathways involved in plant growth or in defense mechanisms.

It has now been found that various yield-related traits may be increased in plants by increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, wherein said yield-related traits are increased relative to plants having increased expression of one of: (i) a nucleic acid sequence encoding a GRF polypeptide; or (ii) a nucleic acid sequence encoding a SYT polypeptide. The increased yield-related traits comprise one or more of: increased early vigour, increased aboveground biomass, increased total seed yield per plant, increased seed filling rate, increased number of (filled) seeds, increased harvest index and increased thousand kernel weight (TKW).

**Background relating to Growth-Regulating Factor (GRF) polypeptides**

DNA-binding proteins are proteins that comprise any of many DNA-binding domains and thus have a specific or general affinity to DNA. DNA-binding proteins include for example transcription factors that modulate the process of transcription, nucleases that cleave DNA molecules, and histones that are involved in DNA packaging in the cell nucleus.

Transcription factors are usually defined as proteins that show sequence-specific DNA binding affinity and that are capable of activating and/or repressing transcription. The *Arabidopsis thaliana* genome codes for at least 1533 transcriptional regulators, accounting for -5.9% of its estimated total number of genes (Riechmann et al. (2000) Science 290: 2105-2109). The Database of Rice Transcription Factors (DRTF) is a collection of known and predicted transcription factors of *Oryza sativa L. ssp. indica* and *Oryza sativa L. ssp. japonica,* and currently contains 2,025 putative transcription factors (TF) gene models in *indica* and 2,384 in *japonica,* distributed in 63 families (Gao et al. (2006) Bioinformatics 2006, 22(10):1286-7).

One of these families is the Growth-Regulating Factor (GRF) family of transcription factors, which is specific to plants. At least nine GRF polypeptides have been identified in *Arabidopsis thaliana* (Kim et al. (2003) Plant J 36: 94-104), and at least twelve in Oryza sativa (Choi et al. (2004) Plant Cell Physiol 45(7): 897-904). The GRF polypeptides are characterized by the presence in their N-terminal half of at least two highly conserved domains, named after the most conserved amino acids within each domain: (i) a QLQ domain (InterPro accession IPR014978, PFAM accession PF08880), where the most conserved amino acids of the domain are Gln-Leu-Gln; and (ii) a WRC domain (InterPro accession IPR014977, PFAM accession PF08879), where the most conserved amino acids of the domain are Trp-Arg-Cys. The WRC domain further contains two distinctive structural features, namely, the WRC domain is enriched in basic amino acids Lys and Arg, and further comprises three Cys and one His residues in a conserved spacing ($CX_9CX_{10}CX_2H$),

designated as the Effector of Transcription (ET) domain (Ellerstrom et al. (2005) Plant Molec Biol 59: 663-681). The conserved spacing of cysteine and histidine residues in the ET domain is reminiscent of zinc finger (zinc-binding) proteins. In addition, a nuclear localisation signal (NLS) is usually comprised in the GRF polypeptide sequences.

Interaction of some GRF polypeptides with a small family of transcriptional coactivators, GRF-interacting factors (GIF1 to GIF3, also called synovial sarcoma translocation SYT1 to SYT3), has been demonstrated using a yeast two-hyrid interaction assay (Kim & Kende (2004) Proc Natl Acad Sci 101: 13374-13379).

The name GRF has also been given to another type of polypeptides, belonging to the 14-3-3 family of polypeptides (de Vetten & Ferl (1994) Plant Physiol 106: 1593-1604), that are totally unrelated the GRF polypeptides useful in performing the methods of the invention.

Transgenic *Arabidopsis thaliana* plants transformed with a rice GRF (OsGRF1) polypeptide under the control of a viral constitutive 35S CaMV promoter displayed curly leaves, severely reduced elongation of the primary inflorescence, and delayed bolting (van der Knapp et al. (2000) Plant Physiol 122: 695-704). Transgenic *Arabidopsis thaliana* plants transformed with either one of two *Arabidopsis* GRF polypeptides (AtGRF1 and AtGRF2) developed larger leaves and cotyledons, were delayed in bolting, and were partially sterile (due to lack of viable pollen), compared to wild type plants (Kim et al. (2003) Plant J 36: 94-104).

In US patent application US2006/0048240, an *Arabidopsis thaliana* GRF polypeptide is identified as SEQ ID NO: 33421. In US patent application US 2007/0022495, an *Arabidopsis thaliana* GRF polypeptide is identified as SEQ ID NO: 1803 (also therein referred to as G1438). Transgenic *Arabidopsis* plants overexpressing G1438 using the 35S CaMV promoter present dark green leaves.

**Background relating to synovial sarcoma translocation (SYT) polypeptides**

SYT is a transcriptional co-activator that, in plants, forms a functional complex with transcription activators of the GRF (growth-regulating factor) family of proteins (Kim HJ, Kende H (2004) Proc Nat Acad Sc 101: 13374-9). SYT is called GIF for GRF-interacting factor in this paper, and AN3 for angustifolia3 in Horiguchi et al. (2005) Plant J 43: 68-78. The GRF transcription activators share structural domains (in the N-terminal region) with the SWI/SNF proteins of the chromatin-remodelling complexes in yeast (van der Knaap E et al., (2000) Plant Phys 122: 695-704). Transcriptional co-activators of these complexes are proposed to be involved in recruiting SWI/SNF complexes to enhancer and promoter regions to effect local chromatin remodelling (review Näär AM et al., (2001) Annu Rev Biochem 70: 475-501). The alteration in local chromatin structure modulates transcriptional activation. More precisely, SYT is proposed to interact with plant SWI/SNF complex to affect transcriptional activation of GRF target gene(s) (Kim HJ, Kende H (2004) Proc Nat Acad Sc 101: 13374-9).

SYT belongs to a gene family of three members in *Arabidopsis.* The SYT polypeptide shares homology with the human SYT. The human SYT polypeptide was shown to be a transcriptional co-activator (Thaete et al. (1999) Hum Molec Genet 8: 585-591). Three domains characterize the mammalian SYT polypeptide:

(i) the N-terminal SNH (SYT N-terminal homology) domain, conserved in mammals, plants, nematodes and fish;
(ii) the C-terminal QPGY-rich domain, composed predominantly of glycine, proline, glutamine and tyrosine, occurring at variable intervals;
(iii) a methionine-rich (Met-rich) domain located between the two previous domains.

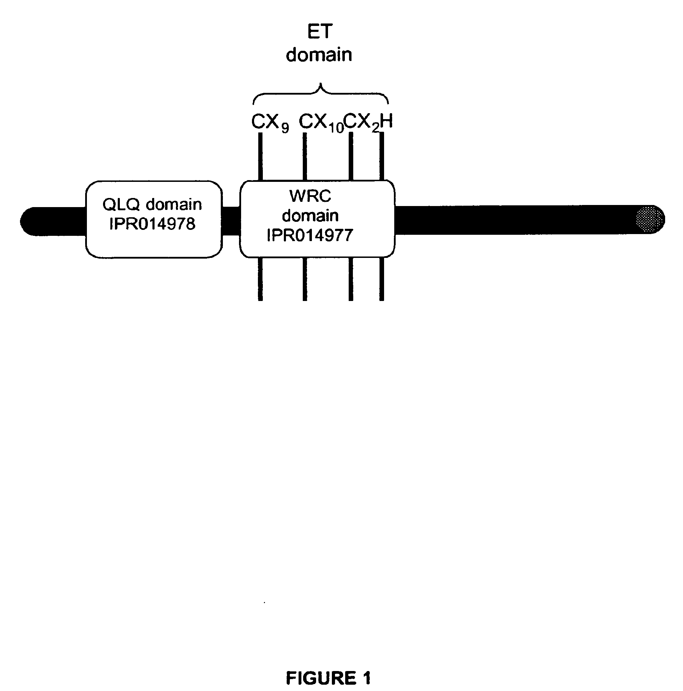

In plant SYT polypeptides, the SNH domain is well conserved. The C-terminal domain is rich in glycine and glutamine, but not in proline or tyrosine. It has therefore been named the QG-rich domain in contrast to the QPGY domain of mammals. As with mammalian SYT, a Met-rich domain may be identified N-terminally of the QG domain. The QG-rich domain may be taken to be substantially the C-terminal remainder of the polypeptide (minus the SHN domain); the Met-rich domain is typically comprised within the first half of the QG-rich (from the N-terminus to the C-terminus). A second Met-rich domain may precede the SNH domain in plant SYT polypeptides (see Fig 1).

A SYT loss-of function mutant and transgenic plants with reduced expression of SYT was reported to develop small and narrow leaves and petals, which have fewer cells (Kim HJ, Kende H (2004) Proc Nat Acad Sc 101: 13374-9).

Overexpression of AN3 in Arabidopsis thaliana resulted in plants with leaves that were 20-30% larger than those of the wild type (Horiguchi et al. (2005) Plant J 43: 68-78).

In Japanese patent application 2004-350553, a method for controlling the size of leaves in the horizontal direction is described, by controlling the expression of the AN3 gene.

Surprisingly, it has now been found that increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide gives plants having increased yield-related traits relative to plants having increased expression of one of: (i) a nucleic acid sequence encoding a GRF polypeptide; or (ii) a nucleic acid sequence encoding a SYT polypeptide. According to one embodiment, there is provided a method for increasing various plant yield-related traits by increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, wherein said yield-related traits are increased relative to plants having increased expression of one of: (i) a nucleic acid sequence encoding a GRF polypeptide, or (ii) a nucleic acid sequence encoding a SYT polypeptide. The increased yield-related traits comprise one or more of: increased early vigour, increased aboveground biomass, increased total seed yield per plant, increased seed filling rate, increased number of (filled) seeds, increased harvest index or increased thousand kernel weight (TKW).

**Definitions**

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence (s)/nucleotide sequence(s)

The terms "polynucleotide(s)", "nucleic acid sequence(s)", "nucleotide sequence(s)", "nucleic acid(s)" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tang·100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |

(continued)

| Residue | Conservative Substitutions | Residue | Conservative Substitutions |
|---|---|---|---|
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acid molecules are in solution. The hybridisation process can also occur with one of the complementary nucleic acid molecules immobilised to a matrix such as magnetic beads, Sepharose beads or any other resin. The hybridisation process can furthermore occur with one of the complementary nucleic acid molecules immobilised to a solid support such as a nitro-cellulose or nylon membrane or immobilised by e.g. photolithography to, for example, a siliceous glass support (the latter known as nucleic acid sequence arrays or microarrays or as nucleic acid sequence chips). In order to allow hybridisation to occur, the nucleic acid molecules are generally thermally or chemically denatured to melt a double strand into two single strands and/or to remove hairpins or other secondary structures from single stranded nucleic add molecules.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acid sequences may deviate in sequence and still encode a substantially identical polypeptide, due to the degeneracy of the genetic code. Therefore medium stringency hybridisation conditions may sometimes be needed to identify such nucleic acid sequence molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid sequence strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$
[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid sequence hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acid molecules of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

<u>Splice variant</u>

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

<u>Allelic variant</u>

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

<u>Gene shuffling/Directed evolution</u>

Gene shuffling or directed evolution consists of iterations of DNA shuffling followed by appropriate screening and/or selection to generate variants of nucleic acid sequences or portions thereof encoding proteins having a modified biological activity (Castle et al., (2004) Science 304(5674): 1151-4; US patents 5,811,238 and 6,395,547).

<u>Regulatory element/Control seauence/Promoter</u>

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid sequence control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, increasers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or increases expression of a nucleic acid sequence molecule in a cell, tissue or organ.

A "plant promoter" comprises regulatory elements, which mediate the expression of a coding sequence segment in plant cells. The "plant promoter" preferably originates from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid sequence molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid sequence used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a level that is in all instances below that obtained under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of plant constitutive promoters

| Gene Source | Reference |
|---|---|
| Actin | McElroy et al, Plant Cell, 2: 163-171, 1990 |
| HMGB | WO 2004/070039 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| Rubisco small subunit | US 4,962,028 |
| OCS | Leisner (1988) Proc Natl Acad Sci USA 85(5): 2553 |

(continued)

| Gene Source | Reference |
|---|---|
| SAD1 | Jain et al., Crop Science, 39 (6), 1999: 1696 |
| SAD2 | Jain et al., Crop Science, 39 (6), 1999: 1696 |
| V-ATPase | WO 01/14572 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| Rice RCc3 | Xu et al (1995) Plant Mol Biol 27(2): 237-48 |
| Arabidopsis phosphate transporter PHT1 | Kovama *et al.,* 2005 |
| Medicago phosphate transporter | Xiao *et al.,* 2006 |
| Arabidopsis Pyk10 | Nitz et al. (2001) Plant Sci 161 (2): 337-346 |
| Tobacco root-specific genes RB7, RD2, RD5, RH12 | Conkling et al. (1990) Plant Phys 93(3): 1203-1211 |
| Barley root-specific lectin | Lerner & Raikhel (1989) Plant Phys 91: 124-129 |
| Root-specific hydroxy-proline rich protein | Keller & Lamb (1989) Genes & Dev 3:1639-1646 |
| Arabidopsis CDC27B/hobbit | Blilou et al. (2002) Genes & Dev 16:2566-2575 |

A seed-specific promoter is transcriptionally active predominantly in seed tissue, but not necessarily exclusively in seed tissue (in cases of leaky expression). The seed-specific promoter may be active during seed development and/or during germination. Examples of seed-specific promoters are shown in Table 2c below. Further examples of seed-specific promoters are given in Qing Qu and Takaiwa (Plant Biotechnol. J. 2, 113-125, 2004).

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| Legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| Zein | Matzke et al Plant Mol Biol, 14(3):323-32 1990 |
| NapA | Stalberg et al, Planta 199: 515-519, 1996. |
| Wheat LMW and HMW glutenin-1 | Mol Gen Genet 216:81-90, 1989; NAR 17:461-2, 1989 |
| Wheat SPA | Albani et al, Plant Cell, 9: 171-184, 1997 |
| Wheat α, β, γ-gliadins | EMBO J. 3:1409-15, 1984 |
| Barley ltr1 promoter | Diaz et al. (1995) Mol Gen Genet 248(5):592-8 |
| Barley B1, C, D, hordein | Theor Appl Gen 98:1253-62, 1999; Plant J 4:343-55, 1993; Mol Gen Genet 250:750-60, 1996 |
| Barley DOF | Mena et al, The Plant Journal, 116(1): 53-62, 1998 |
| blz2 | EP99106056.7 |
| Synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| Maize ESR gene family | Plant J 12:235-46, 1997 |
| Sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PR00117, putative rice 40S ribosomal protein | WO 2004/070039 |
| PRO0136, rice alanine aminotransferase | Unpublished |
| PRO0147, trypsin inhibitor ITR1 (barley) | Unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| Cathepsin β-like gene | Cejudo et al, Plant Mol Biol 20:849-856, 1992 |

(continued)

| Gene source | Reference |
|---|---|
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38, 1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2d below.

**Table 2d:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of meristem-specific promoters which may be used to perform the methods of the invention are shown in Table 2e below.

**Table 2e:** Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, preferably the expression level is increased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription increasers or translation increasers. Isolated nucleic acid sequences which serve as promoter or increaser elements may be introduced in an appropriate position (typically upstream) of a non-heterologous form of a polynucleotide so as to upregulate expression of a nucleic acid sequence encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron increasement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid sequence encoding the protein of interest (target gene), or from any nucleic acid sequence capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A method for the reduction or substantial elimination of endogenous gene expression is by RNA-mediated silencing using an inverted repeat of a nucleic acid sequence or a part thereof (in this case a stretch of substantially contiguous nucleotides derived from the gene of interest, or from any nucleic acid sequence capable of encoding an orthologue, paralogue or homologue of the protein of interest), preferably capable of forming a hairpin structure. Another example of an RNA silencing method involves the introduction of nucleic acid sequences or parts thereof (in this case a stretch of substantially contiguous nucleotides derived from the gene of interest, or from any nucleic acid sequence capable of encoding an orthologue, paralogue or homologue of the protein of interest) in a sense orientation into a plant. Another example of an RNA silencing method involves the use of antisense nucleic acid sequences. Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682). Other methods, such as the use of antibodies directed to an endogenous polypeptide for inhibiting its function in planta, or interference in the signalling pathway in which a polypeptide is involved, will be well known to the skilled man. Artificial and/or natural microRNAs (miRNAs) may be used to knock out gene expression and/or mRNA translation. Endogenous miRNAs are single stranded small RNAs of typically 19-24 nucleotides long. Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs (Schwab et al., (2005) Dev Cell 8(4):517-27). Convenient tools for design and generation of amiRNAs and their precursors are also available to the public (Schwab et al., (2006) Plant Cell 18(5):1121-33).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid sequence to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid sequence construct of the invention. These marker genes enable the identification of a successful transfer of the nucleic acid sequence molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acid sequences have been introduced successfully, the process according to the invention for introducing the nucleic acid sequences advantageously employs techniques which enable the removal or excision of these marker genes. One such a method is what is known as co-transformation. The co-transformation method employs two vectors simultaneously for the transformation, one vector bearing the nucleic acid sequence according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid sequence (known as the Ac/Ds technology). The transformants can be crossed with a transposase source or the transformants are transformed with a nucleic acid sequence construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acid sequences used in the method of the invention are not at their natural locus in the genome of said plant, it being possible for the nucleic acid sequences to be expressed homologously or heterologously. However, as mentioned, transgenic also means that, while the nucleic acid sequence according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acid sequences according to the invention at an unnatural locus in the genome, i.e. homologous or, preferably, heterologous expression of the nucleic acid sequences takes place. Preferred transgenic plants are mentioned herein.

Transformation

The term "introduction" or "transformation" as referred to herein encompass the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et al. (Nat. Biotechnol 14(6): 745-50, 1996) or Frame et al. (Plant Physiol 129(1): 13-22, 2002). Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acid sequences or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation increaser or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acid sequences encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid sequence at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per acre for a crop and year, which is determined by dividing total production (includes both harvested and appraised production) by planted acres. The term "yield" of a plant may relate to vegetative biomass, to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Increase

The terms "increase", "improve" or "increase" are interchangeable and shall mean in the sense of the application at least a 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per hectare or acre; b) increased number of flowers per panicle and/or per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; f) increased number of primary panicles; (g) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in yield may also manifest itself as an increase in seed area and/or seed length and/or seed width and/or seed perimeter. Increased seed yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid sequence of interest. The term "plant" also encompasses plant cells, suspension cultures, callus tissue, embryos, meristematic regions, gametophytes, sporophytes, pollen and microspores, again wherein each of the aforementioned comprises the gene/nucleic acid sequence of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp.,

*Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Omithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., Poa spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Triticale sp., Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

**Detailed description of the invention**

Surprisingly, it has now been found that increasing expression in a plant of a nucleic acid sequence encoding a GRF polypeptide gives plants having increased yield-related traits relative to control plants. According to a first embodiment, the present invention provides a method for increasing yield-related traits in plants relative to control plants, comprising increasing expression in a plant of a nucleic acid sequence encoding a GRF polypeptide.

Surprisingly, it has now been found that increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide gives plants having increased yield-related traits relative to plants having increased expression of one of: (i) a nucleic acid sequence encoding a GRF polypeptide; or (ii) a nucleic acid sequence encoding a SYT polypeptide. According to a first embodiment, there is provided a method for increasing various plant yield-related traits by increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, wherein said yield-related traits are increased relative to plants having increased expression of one of: (i) a nucleic acid sequence encoding a GRF polypeptide, or (ii) a nucleic acid sequence encoding a SYT polypeptide. The increased yield-related traits comprise one or more of: increased early vigour, increased aboveground biomass, increased total seed yield per plant, increased seed filling rate, increased number of (filled) seeds, increased harvest index or increased thousand kernel weight (TKW).

*Detailed description relating to Growth-Regulating Factor (GRF) polypeptides*

A preferred method for increasing expression of a nucleic acid sequence encoding a GRF polypeptide is by introducing and expressing in a plant a nucleic acid sequence encoding a GRF polypeptide.

Any reference hereinafter to a "GRF protein useful in the methods of the invention" is taken to mean a GRF polypeptide as defined herein. Any reference hereinafter to a "GRF nucleic acid sequence useful in the methods of the invention" is taken to mean a nucleic acid sequence capable of encoding such a GRF polypeptide. The nucleic acid sequence to be introduced into a plant (and therefore useful in performing the methods of the invention) is any nucleic acid sequence encoding the type of polypeptide, which will now be described, hereafter also named "*GRF* nucleic acid sequence" or "*GRF* gene".

A "GRF polypeptide" as defined herein refers to any polypeptide comprising: (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115 (comprised in SEQ ID NO: 2); and (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116 (comprised in SEQ ID NO: 2).

Alternatively or additionally, a "GRF polypeptide" as defined herein refers to any polypeptide comprising: (i) a QLQ domain with an InterPro accession IPR014978 (PFAM accession PF08880); (ii) a WRC domain with an InterPro accession IPR014977 (PFAM accession PF08879); and (iii) an Effector of Transcription (ET) domain comprising three Cys and one His residues in a conserved spacing ($CX_9CX_{10}CX_2H$).

Alternatively or additionally, a "GRF polypeptide" as defined herein refers to any polypeptide having in increasing order of preference at least 50%, 55%, 60%, 65%, 70%, 75%, 80%, 85%, 90%, 95%, 98%, 99% or more amino acid sequence identity to the GRF polypeptide as represented by SEQ ID NO: 2 or to any of the full length polypeptide sequences given in Table A.1 herein.

Alternatively or additionally, a "GRF polypeptide" interacts with GRF-interacting factor (GIF; (GIF1 to GIF3; also called synovial sarcoma translocation SYT1 to SYT3) polypeptides, such as the ones presented in Table A.2 herein, in a yeast two-hybrid interaction assay.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32: D134-D137, (2004)), or Pfam (Bateman et al., Nucleic Acids Research 30(1): 276-280 (2002). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)).

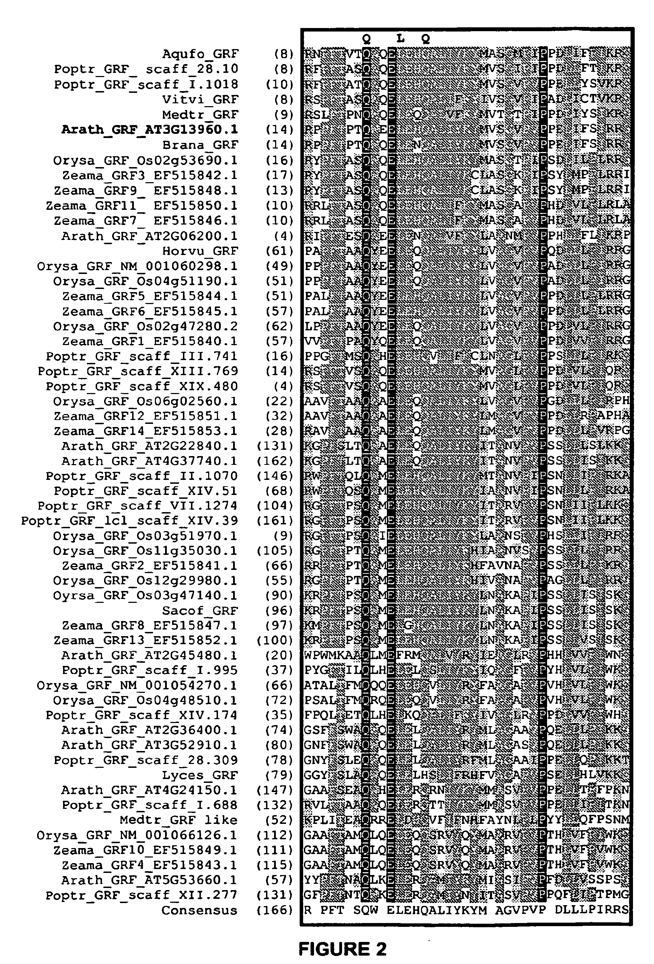

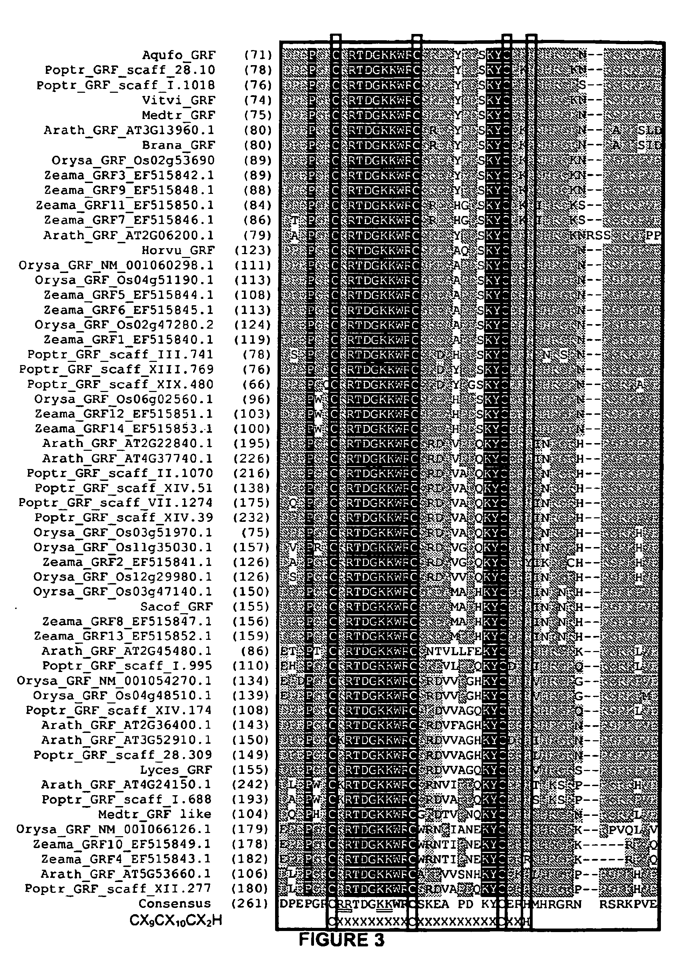

Analysis of the polypeptide sequence of SEQ ID NO: 2 is presented below in Examples 2 and 4 herein. For example, a GRF polypeptide as represented by SEQ ID NO: 2 comprises a QLQ domain with an InterPro accession IPR014978 (PFAM accession PF08880) and a WRC domain with an InterPro accession IPR014977 (PFAM accession PF08879) in the InterPro domain database. Domains may also be identified using routine techniques, such as by sequence alignment. An alignment of the QLQ domain of the polypeptides of Table A.1 herein, is shown in Figure 2, and and alignment of the WRC domain of the polypeptides of Table A.1 herein, is shown in Figure 3. Such alignments are useful for identifying the most conserved amino acids between the GRF polypeptides, such as the QLQ and WRC amino acid residues.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., (2003) BMC Bioinformatics, 10: 29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic add sequence or polypeptide sequence or over selected domains or conserved motif(s), using the programs mentioned above using the default parameters. Outside of the QLQ domain and of the WRC domain, GRF polypeptides reputedly have low amino add sequence identity. Example 3 herein describes in Table B.1 the percentage identity between the GRF polypeptide as represented by SEQ ID NO: 2 and the GRF polypeptides listed in Table A, which can be as low as 15% amino acid sequence identity. The percentage identity can be substantially increased if the identity calculation is performed between the QLQ domain SEQ ID NO: 2 (as represented by SEQ ID NO: 115 comprised in SEQ ID NO: 2; QLQ domain of the GRF polypeptides of Table A.1 represented in Figure 2) and the QLQ domains of the polypeptides useful in performing the invention. Similarly, the percentage identity can be substantially increased if the identity calculation is performed between the WRC domain SEQ ID NO: 2 (as represented by SEQ ID NO: 116 comprised in SEQ ID NO: 2; WRC domain of the GRF polypeptides of Table A.1 represented in Figure 3) and the WRC domains of the polypeptides useful in performing the invention. Percentage identity over the QLQ domain amongst the polypeptide sequences useful in performing the methods of the invention ranges between 25 % and 99% amino acid identity, and percentage identity over the WRC domain amongst the polypeptide sequences useful in performing the methods of the invention ranges between 60 % and 99% amino acid identity. As can also be observed in Figure 3, the WRC domain is better conserved amongst the different GRF polypeptides than the QLQ domain, as shown in Figure 2.

The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP and others.

Furthermore, GRF polypeptides useful in the methods of the present invention (at least in their native form) typically, but not necessarily, have transcriptional regulatory activity and capacity to interact with other proteins. Therefore, GRF polypeptides with reduced transcriptional regulatory activity, without transcriptional regulatory activity, with reduced protein-protein interaction capacity, or with no protein-protein interaction capacity, may equally be useful in the methods of the present invention. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). GRF polypeptides are capable of transcriptional activation of reporter genes in yeast cells (Kim & Kende (2004) Proc Natl Acad Sci 101 (36): 13374-13379). GRF polypeptides are also capable of interacting with GRF-interacting factor polypeptides (GIF1 to GIF3; also called synovial sarcoma translocation (SYT)) *in vivo* in yeast cells, using a yeast two-hybrid protein-protein interaction assay (Kim & Kende, *supra*). *In vitro* binding assays are also used to show that GRF polypeptides and SYT (or GIF) polypeptides are interacting partners (Kim & Kende, *supra*).

The present invention is illustrated by transforming plants with the nucleic acid sequence represented by SEQ ID NO: 1, encoding the GRF polypeptide sequence of SEQ ID NO: 2. However, performance of the invention is not restricted to these sequences; the methods of the invention may advantageously be performed using any nucleic acid sequence encoding a GRF polypeptide as defined herein.

Examples of nucleic acid sequences encoding GRF polypeptides are given in Table A.1 of Example 1 herein. Such nucleic acid sequences are useful in performing the methods of the invention. The polypeptide sequences given in Table A.1 of Example 1 are example sequences of orthologues and paralogues of the GRF polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A.1 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acid sequences encoding homologues and derivatives of any one of the polypeptide sequences given in Table A.1 of Example 1, the terms "homologue" and "derivative" being as defined herein. Also useful in the methods of the invention are nucleic acid sequences encoding homologues and derivatives of orthologues or paralogues of any one of the polypeptide sequences given in Table A.1 of Example 1. Homologues and derivatives useful in the methods of the present invention have substantially the same biological and functional activity as the unmodified protein from which they are derived.

Further nucleic acid variants useful in practising the methods of the invention include portions of nucleic acid sequences encoding GRF polypeptides, nucleic acid sequences hybridising to nucleic acid sequences encoding GRF polypeptides, splice variants of nucleic acid sequences encoding GRF polypeptides, allelic variants of nucleic acid sequences encoding GRF polypeptides and variants of nucleic acid sequences encoding GRF polypeptides obtained by gene shuffling. The terms hybridising sequence, splice variant, allelic variant and gene shuffling are as described herein.

Nucleic acid sequences encoding GRF polypeptides need not be full-length nucleic acid sequences, since performance of the methods of the invention does not rely on the use of full-length nucleic acid sequences. According to the present invention, there is provided a method for increasing yield-related traits, in plants, comprising introducing and expressing in a plant a portion of any one of the nucleic acid sequences given in Table A.1 of Example 1, or a portion of a nucleic acid sequence encoding an orthologue, paralogue or homologue of any of the polypeptide sequences given in Table A.1 of Example 1.

A portion of a nucleic acid sequence may be prepared, for example, by making one or more deletions to the nucleic acid sequence. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a GRF polypeptide as defined herein, and have substantially the same biological activity as the polypeptide sequences given in Table A.1 of Example 1. Preferably, the portion is a portion of any one of the nucleic acid sequences given in Table A.1 of Example 1, or is a portion of a nucleic acid sequence encoding an orthologue or paralogue of any one of the polypeptide sequences given in Table A.1 of Example 1. Preferably the portion is, in increasing order of preference at least 400, 450, 500, 550, 600, 650, 700, 750, 800, 850, 900, 950, 1000, 1050, 1100, 1150, 1190 consecutive nucleotides in length, the consecutive nucleotides being of any one of the nucleic acid sequences given in Table A.1 of Example 1, or of a nucleic acid sequence encoding an orthologue or paralogue of any one of the polypeptide sequences given in Table A.1 of Example 1. Preferably, the portion is a portion of a nucleic sequence encoding a polypeptide sequence polypeptide comprising: (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115 (comprised in SEQ ID NO: 2); and (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116 (comprised in SEQ ID NO: 2). Most preferably the portion is a portion of the nucleic acid sequence of SEQ ID NO: 1.

Another nucleic acid sequence variant useful in the methods of the invention is a nucleic acid sequence capable of hybridising, under reduced stringency conditions, preferably under stringent conditions, with a nucleic acid sequence encoding a GRF polypeptide as defined herein, or with a portion as defined herein.

According to the present invention, there is provided a method for increasing yield-related traits in plants, comprising introducing and expressing in a plant a nucleic acid sequence capable of hybridizing to any one of the nucleic acid sequences given in Table A.1 of Example 1, or comprising introducing and expressing in a plant a nucleic acid sequence capable of hybridising to a nucleic acid sequence encoding an orthologue, paralogue or homologue of any of the nucleic acid sequences given in Table A.1 of Example 1.

Hybridising sequences useful in the methods of the invention encode a GRF polypeptide as defined herein, and have substantially the same biological activity as the polypeptide sequences given in Table A.1 of Example 1. Preferably, the hybridising sequence is capable of hybridising to any one of the nucleic acid sequences given in Table A.1 of Example 1, or to a portion of any of these sequences, a portion being as defined above, or wherein the hybridising sequence is capable of hybridising to a nucleic acid sequence encoding an orthologue or paralogue of any one of the polypeptide sequences given in Table A.1 of Example 1. Preferably, the hybridising sequence is capable of hybridising to a nucleic acid sequence encoding a polypeptide sequence comprising: (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115 (comprised in SEQ ID NO: 2); and (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116 (comprised in SEQ ID NO: 2). Most preferably, the hybridising sequence is capable of hybridising to a nucleic acid sequence as represented by SEQ ID NO: 1 or to a portion thereof.

Another nucleic acid sequence variant useful in the methods of the invention is a splice variant encoding a GRF polypeptide as defined hereinabove, a splice variant being as defined herein.

According to the present invention, there is provided a method for increasing yield-related traits, comprising introducing and expressing in a plant a splice variant of any one of the nucleic add sequences given in Table A.1 of Example 1, or a splice variant of a nucleic acid sequence encoding an orthologue, paralogue or homologue of any of the polypeptide sequences given in Table A.1 of Example 1.

Preferred splice variants are splice variants of a nucleic acid sequence represented by SEQ ID NO: 1, or a splice variant of a nucleic acid sequence encoding an orthologue or paralogue of SEQ ID NO: 2. Preferably, the splice variant is a splice variant of a nucleic acid sequence encoding a polypeptide sequence comprising: (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115 (comprised in SEQ ID NO: 2); and (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116 (comprised in SEQ ID NO: 2).

Another nucleic acid sequence variant useful in performing the methods of the invention is an allelic variant of a nucleic acid sequence encoding a GRF polypeptide as defined hereinabove, an allelic variant being as defined herein.

According to the present invention, there is provided a method for increasing yield-related traits, comprising introducing and expressing in a plant an allelic variant of any one of the nucleic acid sequences given in Table A.1 of Example 1, or comprising introducing and expressing in a plant an allelic variant of a nucleic acid sequence encoding an orthologue, paralogue or homologue of any of the polypeptide sequences given in Table A.1 of Example 1.

The allelic variants useful in the methods of the present invention have substantially the same biological activity as the GRF polypeptide of SEQ ID NO: 2 and any of the polypeptide sequences depicted in Table A.1 of Example 1. Allelic variants exist in nature, and encompassed within the methods of the present invention is the use of these natural alleles. Preferably, the allelic variant is an allelic variant of SEQ ID NO: 1 or an allelic variant of a nucleic acid sequence encoding an orthologue or paralogue of SEQ ID NO: 2. Preferably, the allelic variant is an allelic variant of a polypeptide sequence comprising: (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115 (comprised in SEQ ID NO: 2); and (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116 (comprised in SEQ ID NO: 2).

Gene shuffling or directed evolution may also be used to generate variants of nucleic acid sequences encoding GRF polypeptides as defined above, the term "gene shuffling" being as defined herein.

According to the present invention, there is provided a method for increasing yield-related traits, comprising introducing and expressing in a plant a variant of any one of the nucleic acid sequences given in Table A.1 of Example 1, or comprising introducing and expressing in a plant a variant of a nucleic acid sequence encoding an orthologue, paralogue or homologue of any of the polypeptide sequences given in Table A.1 of Example 1, which variant nucleic acid sequence is obtained by gene shuffling.

Preferably, the variant nucleic acid sequence obtained by gene shuffling encodes a polypeptide sequence comprising: (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115 (comprised in SEQ ID NO: 2); and (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116 (comprised in SEQ ID NO: 2).

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acid sequences encoding GRF polypeptides may be derived from any natural or artificial source. The nucleic acid sequence may be modified from its native form in composition and/or genomic environment through deliberate human manipulation. Preferably the nucleic acid sequence encoding a GRF polypeptide is from a plant, further preferably from a dicotyledonous plant, more preferably from the family Brassicaceae, most preferably the nucleic acid sequence is from *Arabidopsis thaliana.*

*Detailed description relating to synovial sarcoma translocation (SYT) polypeptides*

A preferred method for increasing expression of a nucleic acid sequence encoding a SYT polypeptide is by introducing and expressing in a plant a nucleic acid sequence encoding a SYT polypeptide.

Any reference hereinafter to a "SYT protein useful in the methods of the invention" is taken to mean a SYT polypeptide as defined herein. Any reference hereinafter to a "SYT nucleic acid sequence useful in the methods of the invention" is taken to mean a nucleic acid sequence capable of encoding such a SYT polypeptide. The nucleic acid sequence to be introduced into a plant (and therefore useful in performing the methods of the invention) is any nucleic acid sequence encoding the type of polypeptide, which will now be described, hereafter also named "*SYT* nucleic acid sequence" or "*SYT* gene".

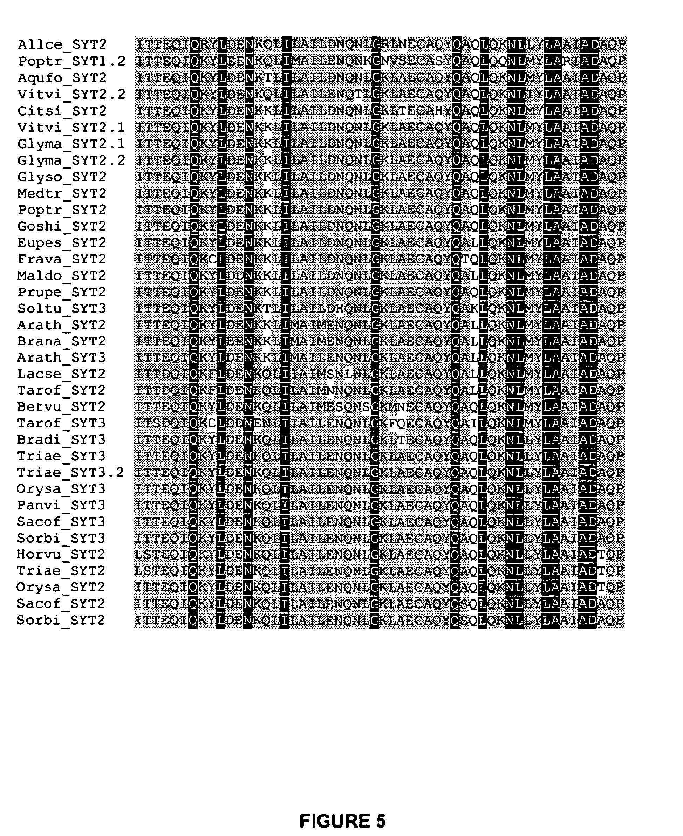

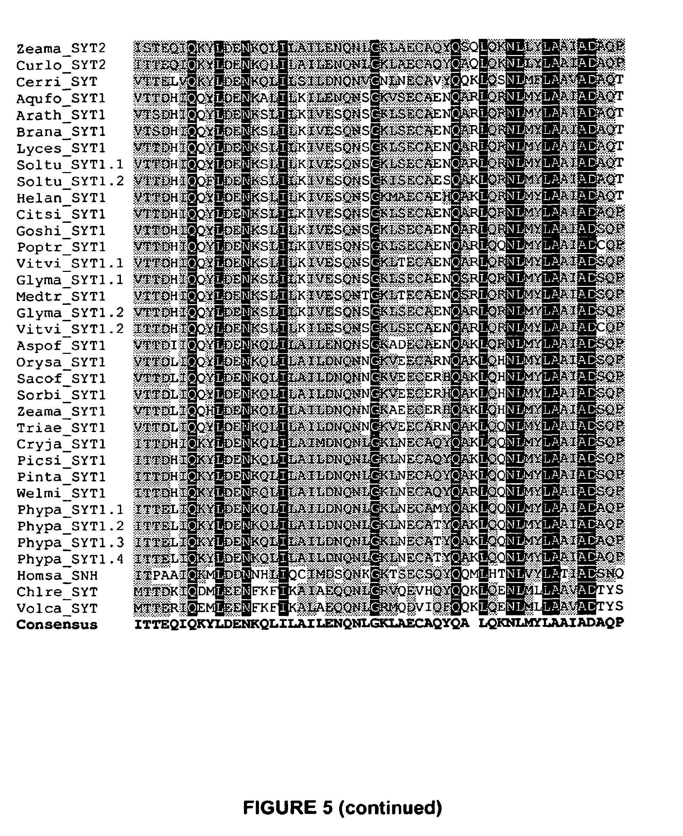

The term "SYT polypeptide" as defined herein refers to a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and (ii) a Met-rich domain; and (iii) a QG-rich domain. Preferably, the SNH domain comprises the most conserved residues as represented by SEQ ID NO: 263, and shown in black in Figure 5.

Alternatively or additionally, a "SYT polypeptide" as defined herein refers to any polypeptide comprising a domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SSXT domain with an InterPro accession IPR007726 (PFAM accession PF05030, SSXT protein (N-terminal region)) of SEQ ID NO: 264. Alternatively or additionally, a "SYT polypeptide" as defined herein refers to any polypeptide having in increasing order of preference at least 20%, 25%, 30%, 35%, 40%, 45%, 50%, 55%, 60%, 65%, 70%, 75%, 80%, 85%, 90%, 95%, 98%, 99% or more amino acid sequence identity to the SYT polypeptide as represented by SEQ ID NO: 121 or to any of the full length polypeptide sequences given in Table A.2 herein.

Alternatively or additionally, a "SYT polypeptide" interacts with Growth-Regulating Factor (GRF) polypeptides in a yeast two-hybrid interaction assay, for examples with the GRF polypeptide sequences given in Table A.1 herein.

Analysis of the SYT polypeptide sequence of SEQ ID NO: 121 is presented below in Examples 2 and 4 herein.

For example, a SYT polypeptide as represented by SEQ ID NO: 121 comprises an SSXT domain with an InterPro accession IPR007726 (PFAM accession PF05030) in the InterPro domain database. Domains may also be identified using routine techniques, such as by sequence alignment. An alignment of the SNH domain of the polypeptides of Table A.2 herein, is shown in Figure 5. Such alignments are useful for identifying the most conserved amino acids between the SYT polypeptides, such as the most conserved residues represented in SEQ ID NO: 263.

Methods for the alignment of sequences for comparison are well known in the art, as briefly described hereinabove. The sequence identity values may be determined over the entire nucleic acid sequence or polypeptide sequence or over selected domains or conserved motif(s), using the programs mentioned above using the default parameters. Outside of the SNH domain and of the SSXT domain, SYT polypeptides reputedly have low amino acid sequence identity. Example 3 herein describes in Table B.2 the percentage identity between the SYT polypeptide as represented by SEQ ID NO: 121 and the SYT polypeptides listed in Table A.2, which can be as low as 25% amino acid sequence identity. The percentage amino acid identity can be substantially increased if the identity calculation is performed between the SNH domain as represented by SEQ ID NO: 262 (comprised in SEQ ID NO: 121) and the SNH domain of the SYT polypeptides of Table A.2 (represented in Figure 5). Similarly, the percentage identity can be substantially increased if the identity calculation is performed between the SSXT domain as represented by SEQ ID NO: 264 (comprised in SEQ ID NO: 121) and the SSXT domains of the SYT polypeptides useful in performing the invention. The SNH domain, which is comprised in the SSXT domain, is better conserved amongst the different SYT polypeptides than the SSXT domain.

Furthermore, SYT polypeptides useful in the methods of the present invention (at least in their native form) typically, but not necessarily, have transcriptional regulatory activity and capacity to interact with other proteins. Therefore, SYT polypeptides with reduced transcriptional regulatory activity, without transcriptional regulatory activity, with reduced protein-protein interaction capacity, or with no protein-protein interaction capacity, may equally be useful in the methods of the present invention. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). SYT polypeptides are capable of interacting with GRF polypeptides *in vivo* in yeast cells, using a yeast two-hybrid protein-protein interaction assay (Kim & Kende, *supra*). *In vitro* binding assays are also used to show that GRF polypeptides and SYT polypeptides are interacting partners (Kim & Kende, *supra*).

The present invention is illustrated by transforming plants with the nucleic acid sequence represented by SEQ ID NO: 120, encoding the SYT polypeptide sequence of SEQ ID NO: 121. However, performance of the invention is not restricted to these sequences; the methods of the invention may advantageously be performed using any nucleic acid sequence encoding a SYT polypeptide as defined herein.

Examples of nucleic acid sequences encoding SYT polypeptides are given in Table A.2 of Example 1 herein. Such nucleic acid sequences are useful in performing the methods of the invention. The polypeptide sequences given in Table A.2 of Example 1 are example sequences of orthologues and paralogues of the SYT polypeptide represented by SEQ ID NO: 121, the terms "orthologues" and "paralogues" being as defined hereinabove. Further orthologues and paralogues may readily be identified by performing reciprocal blast searches (as described herein above).

Nucleic acid variants may also be useful in practising the methods of the invention. Examples of such variants include nucleic acid sequences encoding homologues and derivatives of any one of the polypeptide sequences given in Table A.2 of Example 1, the terms "homologue" and "derivative" being as defined herein. Also useful in the methods of the invention are nucleic acid sequences encoding homologues and derivatives of orthologues or paralogues of any one of the polypeptide sequences given in Table A.2 of Example 1. Homologues and derivatives useful in the methods of the present invention have substantially the same biological and functional activity as the unmodified protein from which they are derived.

Further nucleic acid variants useful in practising the methods of the invention include portions of nucleic acid sequences encoding SYT polypeptides, nucleic acid sequences hybridising to nucleic acid sequences encoding SYT polypeptides, splice variants of nucleic acid sequences encoding SYT polypeptides, allelic variants of nucleic acid sequences encoding SYT polypeptides and variants of nucleic acid sequences encoding SYT polypeptides obtained by gene shuffling. The terms hybridising sequence, splice variant, allelic variant and gene shuffling are as described herein.

Nucleic acid sequences encoding SYT polypeptides need not be full-length nucleic acid sequences, since performance of the methods of the invention does not rely on the use of full-length nucleic acid sequences. According to the present invention, there is provided a method for increasing yield-related traits, in plants, comprising introducing and expressing in a plant a portion of any one of the nucleic acid sequences given in Table A.2 of Example 1, or a portion of a nucleic acid sequence encoding an orthologue, paralogue or homologue of any of the polypeptide sequences given in Table A.2 of Example 1.

A portion of a nucleic acid sequence may be prepared, for example, by making one or more deletions to the nucleic acid sequence. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a SYT polypeptide as defined herein, and have substantially the same biological activity as the polypeptide sequences given in Table A.2 of Example 1. Preferably, the portion is a portion of any one of the nucleic acid sequences given in Table A.2 of Example 1, or is a portion of a nucleic acid sequence encoding an orthologue or paralogue of any one of the polypeptide sequences given in Table A.2 of Example 1. Preferably the portion is, in increasing order of preference at least 100, 125, 150, 175, 200 or 225 consecutive nucleotides in length, preferably at least 250, 275, 300, 325, 350, 375, 400, 425, 450 or 475 consecutive nucleotides in length, further preferably least 500, 525, 550, 575, 600, 625, 650, 675, 700 or 725 consecutive nucleotides in length, or as long as a full length SYT nucleic acid sequence, the consecutive nucleotides being of any one of the nucleic acid sequences given in Table A.2 of Example 1, or of a nucleic acid sequence encoding an orthologue or paralogue of any one of the polypeptide sequences given in Table A.2 of Example 1. Preferably, the portion is a portion of a nucleic sequence encoding a polypeptide sequence polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and (ii) a Met-rich domain; and (iii) a QG-rich domain. Preferably, the SNH domain comprises the most conserved residues as represented by SEQ ID NO: 263, and shown in black in Figure 5. Most preferably the portion is a portion of the nucleic acid sequence of SEQ ID NO: 120.

Another nucleic acid sequence variant useful in the methods of the invention is a nucleic acid sequence capable of hybridising, under reduced stringency conditions, preferably under stringent conditions, with a nucleic acid sequence encoding a SYT polypeptide as defined herein, or with a portion as defined herein.

According to the present invention, there is provided a method for increasing yield-related traits in plants, comprising introducing and expressing in a plant a nucleic acid sequence capable of hybridizing to any one of the nucleic acid sequences given in Table A.2 of Example 1, or comprising introducing and expressing in a plant a nucleic acid sequence capable of hybridising to a nucleic acid sequence encoding an orthologue, paralogue or homologue of any of the nucleic acid sequences given in Table A.2 of Example 1.

Hybridising sequences useful in the methods of the invention encode a SYT polypeptide as defined herein, and have substantially the same biological activity as the polypeptide sequences given in Table A.2 of Example 1. Preferably, the hybridising sequence is capable of hybridising to any one of the nucleic acid sequences given in Table A.2 of Example 1, or to a portion of any of these sequences, a portion being as defined above, or wherein the hybridising sequence is capable of hybridising to a nucleic acid sequence encoding an orthologue or paralogue of any one of the polypeptide sequences given in Table A.2 of Example 1. Preferably, the hybridising sequence is capable of hybridising to a nucleic acid sequence encoding a polypeptide sequence comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and (ii) a Met-rich domain; and (iii) a QG-rich domain. Preferably, the SNH domain comprises the most conserved residues as represented by SEQ ID NO: 263, and shown in black in Figure 5. Most preferably, the hybridising sequence is capable of hybridising to a nucleic acid sequence as represented by SEQ ID NO: 120 or to a portion thereof.

Another nucleic acid sequence variant useful in the methods of the invention is a splice variant encoding a SYT polypeptide as defined hereinabove, a splice variant being as defined herein.

According to the present invention, there is provided a method for increasing yield-related traits, comprising introducing and expressing in a plant a splice variant of any one of the nucleic acid sequences given in Table A.2 of Example 1, or a splice variant of a nucleic acid sequence encoding an orthologue, paralogue or homologue of any of the polypeptide sequences given in Table A.2 of Example 1.

Preferred splice variants are splice variants of a nucleic acid sequence represented by SEQ ID NO: 120, or a splice variant of a nucleic acid sequence encoding an orthologue or paralogue of SEQ ID NO: 121. Preferably, the splice variant is a splice variant of a nucleic acid sequence encoding a polypeptide sequence comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and (ii) a Met-rich domain; and (iii) a QG-rich domain. Preferably, the SNH domain comprises the most conserved residues as represented by SEQ ID NO: 263, and shown in black in Figure 5.

Another nucleic acid sequence variant useful in performing the methods of the invention is an allelic variant of a nucleic acid sequence encoding a SYT polypeptide as defined hereinabove, an allelic variant being as defined herein.

According to the present invention, there is provided a method for increasing yield-related traits, comprising introducing and expressing in a plant an allelic variant of any one of the nucleic acid sequences given in Table A.2 of Example 1, or comprising introducing and expressing in a plant an allelic variant of a nucleic acid sequence encoding an orthologue, paralogue or homologue of any of the polypeptide sequences given in Table A.2 of Example 1.

The allelic variants useful in the methods of the present invention have substantially the same biological activity as the SYT polypeptide of SEQ ID NO: 121 and any of the polypeptide sequences depicted in Table A.2 of Example 1. Allelic variants exist in nature, and encompassed within the methods of the present invention is the use of these natural alleles. Preferably, the allelic variant is an allelic variant of SEQ ID NO: 120 or an allelic variant of a nucleic acid sequence encoding an orthologue or paralogue of SEQ ID NO: 121. Preferably, the allelic variant is an allelic variant of a polypeptide sequence comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and (ii) a Met-rich domain; and (iii) a QG-rich domain. Preferably, the SNH domain comprises the most conserved residues as represented by SEQ ID NO: 263, and shown in black in Figure 5.

Gene shuffling or directed evolution may also be used to generate variants of nucleic acid sequences encoding SYT polypeptides as defined above, the term "gene shuffling" being as defined herein.

According to the present invention, there is provided a method for increasing yield-related traits, comprising introducing and expressing in a plant a variant of any one of the nucleic acid sequences given in Table A.2 of Example 1, or comprising introducing and expressing in a plant a variant of a nucleic acid sequence encoding an orthologue, paralogue or homologue of any of the polypeptide sequences given in Table A.2 of Example 1, which variant nucleic acid sequence is obtained by gene shuffling.

Preferably, the variant nucleic acid sequence obtained by gene shuffling encodes a polypeptide sequence comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and (ii) a Met-rich domain; and (iii) a QG-rich domain. Preferably, the SNH domain comprises the most conserved residues as represented by SEQ ID NO: 263, and shown in black in Figure 5.

Furthermore, nucleic acid sequence variants may also be obtained by site-directed mutagenesis. Several methods are available to achieve site-directed mutagenesis, the most common being PCR based methods (Current Protocols in Molecular Biology. Wiley Eds.).

Nucleic acid sequences encoding SYT polypeptides may be derived from any natural or artificial source. The nucleic acid sequence may be modified from its native form in composition and/or genomic environment through deliberate human manipulation. Preferably the nucleic acid sequence encoding a SYT polypeptide is from a plant, further preferably from a dicotyledonous plant, more preferably from the family Brassicaceae, most preferably the nucleic acid sequence is from *Arabidopsis thaliana.*

Performance of the methods of the invention, i.e., increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, gives plants having increased yield-related traits relative to plants having increased expression of one of: (i) a nucleic acid sequence encoding a GRF polypeptide; or (ii) a nucleic acid sequence encoding a SYT polypeptide. The terms "yield" and "seed yield" are described in more detail in the "definitions" section herein.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Performance of the methods of the invention, i.e., increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, gives plants having increased yield-related traits relative to plants having increased expression of one of: (i) a nucleic acid sequence encoding a GRF polypeptide; or (ii) a nucleic acid sequence encoding a SYT polypeptide. Preferably said increased yield-related trait is one or more of: (i) increased early vigour; (ii) increased aboveground biomass; (iii) increased total seed yield per plant; (iv) increased seed filling rate; (v) increased number of (filled) seeds; (vi) increased harvest index; or (vii) increased thousand kernel weight (TKW).

Since the transgenic plants according to the present invention have increased yield-related traits, it is likely that these plants exhibit an increased growth rate (during at least part of their life cycle), relative to the growth rate of control plants at a corresponding stage in their life cycle.

"Control plant" may include, as specified in the "definition" section, corresponding wild type plants, or corresponding plants without the gene of interest, or corresponding plants having increased expression of one of: (i) a nucleic acid sequence encoding a GRF polypeptide; or (ii) a nucleic acid sequence encoding a SYT polypeptide. A "control plant" as used herein refers not only to whole plants, but also to plant parts, including seeds and seed parts.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect increased (early) vigour. The increase in growth rate may alter the harvest cycle of a plant allowing plants to be sown later and/or harvested sooner than would otherwise be possible (a similar effect may be obtained with earlier flowering time; delayed flowering is usually not a desirede trait in crops). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises increasing expression in a plant of: (i) a nucleic acid sequence encoding a Growth-Regulating Factor (GRF) polypeptide; and of (ii) a nucleic add sequence encoding a synovial sarcoma translocation (SYT) polypeptide, which plants have increased growth rate relative to plants having increased expression of one of: (i) a nucleic acid sequence encoding a GRF polypeptide; or (ii) a nucleic acid sequence encoding a SYT polypeptide.

Increased yield-related traits occur whether the plant is under non-stress conditions or whether the plant is exposed to various stresses compared to control plants grown under comparable conditions. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes, and insects. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild stress conditions having increased yield-related traits, relative to control plants grown under comparable conditions. Therefore, according to the present invention, there is provided a method for increasing yield-related traits in plants grown under non-stress conditions or under mild stress conditions, which method comprises increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, which plants have increased yield-related traits relative to plants having increased expression of one of: (i) a nucleic acid sequence encoding a GRF polypeptide; or (ii) a nucleic acid sequence encoding a SYT polypeptide, grown under comparable conditions.

Performance of the methods according to the present invention results in plants grown under abiotic stress conditions having increased yield-related traits relative to control plants grown under comparable stress conditions. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. Since diverse environmental stresses activate similar pathways, the exemplification of the present invention with drought stress should not be seen as a limitation to drought stress, but more as a screen to indicate the involvement of GRF polypeptides as defined above, in increasing yield-related traits relative to control plants grown in comparable stress conditions, in abiotic stresses in general.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, $MgCl_2$, $CaCl_2$, amongst others.

Performance of the methods of the invention gives plants having increased yield-related traits, under abiotic stress conditions relative to control plants grown in comparable stress conditions. Therefore, according to the present invention, there is provided a method for increasing yield-related traits in plants grown under abiotic stress conditions, which method comprises increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, which plants have increased yield-related traits relative to plants having increased expression of one of: (i) a nucleic acid sequence encoding a GRF polypeptide; or (ii) a nucleic acid sequence encoding a SYT polypeptide, grown under comparable stress conditions.

Another example of abiotic environmental stress is the reduced availability of one or more nutrients that need to be assimilated by the plants for growth and development. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor for crop plant growth and production (Frink et al. (1999) Proc Natl Acad Sci USA 96(4): 1175-1180), and has as well a major impact on protein accumulation and amino acid composition. Therefore, of great interest are crop plants with increased yield-related traits, when grown under nitrogen-limiting conditions.

Performance of the methods of the invention gives plants grown under conditions of reduced nutrient availability, particularly under conditions of reduced nitrogen availablity, having increased yield-related traits relative to control plants grown under comparable stress conditions. Therefore, according to the present invention, there is provided a method for increasing yield-related traits in plants grown under conditions of reduced nutrient availablity, preferably reduced nitrogen availability, which method comprises increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, which plants have increased yield-related traits relative to plants having increased expression of one of: (i) a nucleic acid sequence encoding a GRF polypeptide; or (ii) a nucleic acid sequence encoding a SYT polypeptide, grown under comparable stress conditions. Reduced nutrient availability may result from a deficiency or excess of nutrients such as nitrogen, phosphates and other phosphorous-containing compounds, potassium, calcium, cadmium, magnesium, manganese, iron and boron, amongst others. Preferably, reduced nutrient availablity is reduced nitrogen availability.

The present invention encompasses plants or parts thereof (including seeds) or cells thereof obtainable by the methods according to the present invention. The plants or parts thereof or cells thereof comprise (i) an isolated nucleic acid transgene encoding a Growth-Regulating Factor (GRF) polypeptide; and (ii) an isolated nucleic acid transgene encoding a synovial sarcoma translocation (SYT) polypeptide.

As mentioned above, a preferred method for increasing expression of: (i) a nucleic acid sequence encoding a GRF polypeptide; and (ii) a nucleic acid sequence encoding a SYT polypeptide, is by introducing and expressing in a plant: (i) a nucleic acid sequence encoding a GRF polypeptide; and (ii) a nucleic acid sequence encoding a SYT polypeptide. Therefore, according to the present invention, there is provided a method for increasing yield-related traits in plants, which method comprises introducing and expressing in a plant: (i) a nucleic acid sequence encoding a GRF polypeptide; and (ii) a nucleic acid sequence encoding a SYT polypeptide, which plants have increased yield-related traits relative to plants having increased expression of one of: (i) a nucleic acid sequence encoding a GRF polypeptide; or (ii) a nucleic acid sequence encoding a SYT polypeptide.

Methods for introducing and expressing two or more transgenes (also called gene stacking) in transgenic plants are well known in the art (see for example, a review by Halpin (2005) Plant Biotech J (3): 141-155. Gene stacking can proceed by interative steps, where two or more transgenes can be sequentially introduced into a plant by crossing a plant containing one transgene with individuals harbouring other transgenes or, alternatively, by re-transforming (or super-transforming) a plant containing one transgene with new genes.

According to the present invention, there is provided a method for increasing yield-related traits in plants, which method comprises sequentially introducing and expressing in a plant: (i) a nucleic acid sequence encoding a GRF polypeptide; and (ii) a nucleic acid sequence encoding a SYT polypeptide, which plants have increased yield-related traits relative to plants having increased expression of one of: (i) a nucleic acid sequence encoding a GRF polypeptide; or (ii) a nucleic acid sequence encoding a SYT polypeptide.

Preferably, the nucleic acid sequences of (i) and (ii) are sequentially introduced and expressed by crossing. A crossing is performed between a female parent plant comprising an introduced and expressed isolated nucleic acid sequence encoding a GRF polypeptide, and a male parent plant comprising an introduced and expressed isolated nucleic acid sequence encoding a SYT polypeptide, or reciprocally, and by selecting in the progeny for the presence and expression of both transgenes. Therefore, according to the present invention, there is provided a method for increasing yield-related traits in plants, by crossing a female parent plant comprising an introduced and expressed isolated nucleic acid sequence encoding a GRF polypeptide, and a male parent plant comprising an introduced and expressed isolated nucleic acid sequence encoding a SYT polypeptide, or reciprocally, and by selecting in the progeny for the presence and expression of both transgenes, wherein said plants have increased yield-related traits relative to the parent plants, or to any other control plants as defined herein.

Alternatively the nucleic acid sequences of (i) and (ii) are sequentially introduced and expressed by re-transformation. Re-transformation is performed by introducing and expressing a nucleic acid sequence encoding GRF polypeptide into a plant, plant part, or plant cell comprising an introduced and expressed nucleic acid sequence encoding a SYT polypeptide, or reciprocally, and by selecting in the progeny for the presence and expression of both transgenes. Therefore, according to the present invention, there is provided a method for increasing yield-related traits in plants, by re-transformation performed by introducing and expressing a nucleic acid sequence encoding GRF polypeptide into a plant, plant part, or plant cell comprising an introduced and expressed nucleic acid sequence encoding a SYT polypeptide, or reciprocally, and by selecting in the progeny for the presence and expression of both transgenes, wherein said plants have increased yield-related traits relative to the plants having increased expression of one of: (i) a nucleic acid sequence encoding a GRF polypeptide; or (ii) a nucleic acid sequence encoding a SYT polypeptide, or to any other control plants as defined herein.

Alternatively, gene stacking can occur via simultaneous transformation, or co-transformation, which is faster and can be used in a whole range of transformation techniques, as described in the "definition" section herein.

When using *Agrobacterium* transformation for example, the transgenes (at least two) can be present in a number of conformations that are well known in the art, some of which are recited below:

(i) the nucleic acid encoding sequences are fused to form a single polypeptide when translated, and placed under the control of a single promoter;

(ii) the nucleic acid encoding sequences are sequentially placed downstream of a single promoter, separated by nucleic acid signals that influence mRNA synthesis (internal ribosome entry sites IRES, 2A stuttering signals, etc..), or polypeptide synthesis (polyproteins separated by protease substrate sites, etc..);

(iii) the nucleic acid encoding sequences are independently driven by separate promoters, and the promoter-nucleic acid encoding sequences combinations are located within one T-DNA;

(iv) the nucleic acid encoding sequences are independently driven by separate promoters, and the promoter- nucleic acid encoding sequences combinations are located in different T-DNAs on one plasmid;

(v) the nucleic acid encoding sequences are independently driven by separate promoters, and the promoter-coding sequence combinations are located in different T-DNAs on different plasmids hosted in one or in separate *Agrobacterium* strains.

When direct genetic transformation is considered, using physical or chemical delivery systems (e.g., microprojectile bombardment, PEG, electroporation, liposome, glass needles, etc.), the transgenes (at least two) can also be present in a number of conformations, but essentially do not need to be comprised in a vector capable of being replicated in Agrobacteria or viruses, intermediates of the genetic transformation. The two transgenes can be comprised in one or more nucleic acid molecules, but simultaneously used for the genetic transformation process.

According to the present invention, there is provided a method for increasing yield-related traits in plants, which method comprises simultaneously introducing and expressing in a plant: (i) a nucleic acid sequence encoding a GRF polypeptide; and (ii) a nucleic acid sequence encoding a SYT polypeptide, which plants have increased yield-related traits relative to plants having increased expression of one of: (i) a nucleic acid sequence encoding a GRF polypeptide; or (ii) a nucleic acid sequence encoding a SYT polypeptide.

The nucleic acid sequences of (i) and (ii) that are simultaneously introduced and expressed, are comprised in one or more nucleic acid molecules. Therefore, according to the present invention is provided increasing yield-related traits in plants, which method comprises simultaneously introducing and expressing in a plant: (i) a nucleic acid sequence encoding a GRF polypeptide; and (ii) a nucleic acid sequence encoding a SYT polypeptide, comprised in one or more nucleic acid molecules, which plants have increased yield-related traits relative to plants having increased expression of one of: (i) a nucleic acid sequence encoding a GRF polypeptide; or (ii) a nucleic acid sequence encoding a SYT polypeptide.

The invention also provides genetic constructs and vectors to facilitate introduction and/or increased expression in plants of nucleic acid sequences encoding GRF polypeptides. The gene constructs may be inserted into vectors, which may be commercially available, suitable for transforming into plants and for expression of the gene of interest in the transformed cells. The invention also provides use of a gene construct as defined herein in the methods of the invention.

More specifically, the present invention provides a construct comprising:

(a) a nucleic acid sequence encoding a GRF polypeptide as defined above;
(b) a nucleic acid sequence encoding a SYT polypeptide as defined above;
(c) one or more control sequences capable of increasing expression of the nucleic acid sequence of (a) and of (b); and optionally
(d) a transcription termination sequence.

The nucleic acid sequences of (a) and (b) can be comprised in one nucleic acid molecule as represented by SEQ ID NO: 267 or by SEQ ID NO: 268, which nucleic acid molecule encodes a polypeptide sequence as represented by SEQ ID NO: 269 or by SEQ ID NO: 270.

The term "control sequence" and "termination sequence" are as defined herein. Preferably, one of the control sequences of a construct is a constitutive promoter. An example of a constitutive promoter is a GOS2 promoter, preferably a rice GOS2 promoter, more preferably a GOS2 promoter as represented by SEQ ID NO: 117.

In one construct, a single control sequence is used to drive the expression of both nucleic acid sequences of (a) and (b) comprised in one nucleic acid molecule as represented by SEQ ID NO: 267 or by SEQ ID NO: 268, which nucleic acid molecule encodes a polypeptide sequence as represented by SEQ ID NO: 269 or by SEQ ID NO: 270.

The present invention also provides for a mixture of constructs useful for example, for simultaneous introduction and expression in plants of (a) a nucleic acid sequence encoding a GRF polypeptide as defined above; and of (b) a nucleic acid sequence encoding a SYT polypeptide as defined above, wherein at least one construct comprises:

(a) a nucleic acid sequence encoding a GRF polypeptide as defined above;
(b) one or more control sequences capable of driving expression of the nucleic acid sequence of (a); and optionally
(c) a transcription termination sequence,

and wherein at least one other construct comprises:

(d) a nucleic acid sequence encoding a SYT polypeptide as defined above;
(e) one or more control sequences capable of driving expression of the nucleic acid sequence of (d); and optionally
(f) a transcription termination sequence.

Preferably, one of the control sequences of a construct is a constitutive promoter. An example of a constitutive promoter is a GOS2 promoter, preferably a rice GOS2 promoter, more preferably a GOS2 promoter as represented by SEQ ID NO: 117.

The invention also provides for the use of a construct comprising: (a) a nucleic acid sequence encoding a GRF polypeptide as defined above; and (b) a nucleic acid sequence encoding a SYT polypeptide as defined above, or of a mixture of constructs comprising (a) and (b) as defined above, in a method for making plants having increased yield-related traits relative to plants having increased expression of one of: (a) a nucleic acid sequence encoding a GRF polypeptide, or (b) a nucleic acid sequence encoding a SYT polypeptide, which increased yield-related traits are one or more of: (i) increased early vigour; (ii) increased aboveground biomass; (iii) increased total seed yield per plant; (iv) increased seed filling rate; (v) increased number of (filled) seeds; (vi) increased harvest index; or (vii) increased thousand kernel weight (TKW).

The invention also provides for plants, plant parts or plant cells transformed with a construct comprising: (a) a nucleic acid sequence encoding a GRF polypeptide as defined above; and (b) a nucleic acid sequence encoding a SYT polypeptide as defined above, or with a mixture of constructs comprising (a) and (b) as defined above.

Plants are transformed with one or more vectors comprising any of the nucleic acid sequences described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to increase expression of the nucleic acid sequence. A constitutive promoter is particularly useful in the methods.

Other organ-specific promoters, for example for preferred expression in leaves, stems, tubers, meristems, seeds (embryo and/or endosperm), are useful in performing the methods of the invention. See the "Definitions" section herein for definitions of the various promoter types.

It should be clear that the applicability of the present invention is not restricted to: (i) a nucleic acid sequence encoding the GRF polypeptide, as represented by SEQ ID NO: 1, with expression driven by a constitutive promoter; or (ii) a nucleic acid sequence encoding the SYT polypeptide, as represented by SEQ ID NO: 120, with expression driven by a constitutive promoter.

Optionally, one or more terminator sequences may be used in the construct introduced into a plant. Additional regulatory elements may include transcriptional as well as translational increasers. Those skilled in the art will be aware of terminator and increaser sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, increaser, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acid sequences, it is advantageous to use marker genes (or reporter genes). Therefore, the genetic construct may optionally comprise a selectable marker gene. Selectable markers are described in more detail in the "definitions" section herein.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having increased yield-related traits, comprising introduction and expression in a plant of: (i) any nucleic acid sequence encoding a GRF polypeptide as defined hereinabove; and (ii) any nucleic acid sequence encoding a SYT polypeptide as defined hereinabove, which plants have increased yield-related traits relative to plants having increased expression of: (i) any nucleic acid sequence encoding a GRF polypeptide as defined hereinabove; or (ii) any nucleic acid sequence encoding a SYT polypeptide as defined hereinabove.

More specifically, the present invention provides a method for the production of transgenic plants having increased yield-related traits relative to plants having increased expression of one of: (i) a nucleic acid sequence encoding a GRF polypeptide, or (ii) a nucleic acid sequence encoding a SYT polypeptide, comprising:

a. introducing and expressing in a plant, plant part, or plant cell, a nucleic acid sequence encoding a GRF polypeptide as defined above, under the control of a constitutive promoter; and
b. introducing and expressing in a plant, plant part, or plant cell, a nucleic acid sequence encoding a SYT polypeptide as defined above, under the control of a constitutive promoter; and
c. cultivating the plant cell, plant part, or plant under conditions promoting plant growth and development.

The nucleic acid sequence of (i) may be any of the nucleic acid sequences capable of encoding a GRF polypeptide as defined herein, and the nucleic acid sequence of (ii) may be any of the nucleic acid sequences capable of encoding a SYT polypeptide as defined herein.

The nucleic acid sequence may be introduced directly into a plant cell or into the plant itself (including introduction into a tissue, organ or any other part of a plant). According to a preferred feature of the present invention, the nucleic acid sequence is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing (i) an isolated nucleic acid sequence encoding a GRF polypeptide as defined hereinabove, operably linked to a constitutive promoter; and (ii) an isolated nucleic acid sequence encoding a SYT polypeptide as defined hereinabove, operably linked to a constitutive promoter. Preferred host cells according to the invention are plant cells. Host plants for the nucleic acid sequences or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants, which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant comprising: (i) an isolated nucleic acid sequence encoding a GRF (as defined hereinabove); and (ii) an isolated nucleic acid sequence encoding a SYT (as defined hereinabove), such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

Methods for increasing expression of nucleic acid sequences or genes, or gene products, are well documented in the art and examples are provided in the definitions section.

As mentioned above, a preferred method for increasing expression of (i) a nucleic acid sequence encoding a GRF polypeptide; and (ii) a nucleic acid sequence encoding a SYT polypeptide, is by introducing and expressing in a plant (i) a nucleic acid sequence encoding a GRF polypeptide; and (ii) a nucleic acid sequence encoding a SYT polypeptide; however the effects of performing the method, i.e. increasing yield-related traits, may also be achieved using other well known techniques, including but not limited to T-DNA activation tagging, TILLING, homologous recombination. A description of these techniques is provided in the definitions section.

The present invention also encompasses use of (i) nucleic acid sequences encoding GRF polypeptides as described herein; and nucleic acid sequences encoding SYT polypeptides as described herein, and use of these GRF polypeptides and SYT polypeptides in increasing any of the aforementioned yield-related traits in plants, under normal growth conditions, under abiotic stress growth (preferably osmotic stress growth conditions) conditions, and under growth conditions of reduced nutrient availability, preferably under conditions of reduced nitrogen availability.

Nucleic acid sequences encoding GRF polypeptides and SYT polypeptides described herein, or the polypeptides themselves, may find use in breeding programmes in which a DNA marker is identified that may be genetically linked to a polypeptide-encoding gene. The genes/ nucleic acid sequences, or the GRF polypeptides and SYT polypeptides themselves may be used to define a molecular marker. This DNA or protein marker may then be used in breeding programmes to select plants having increased yield-related traits, as defined hereinabove in the methods of the invention.

Allelic variants of a gene/nucleic acid sequence encoding a GRF polypeptide and SYT polypeptide may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield-related traits. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acid sequences encoding GRF polypeptides and SYT polypeptides may also be used as probes for genetically and physically mapping the genes that they are a part of, and as markers for traits linked to those genes. Such information may be useful in plant breeding in order to develop lines with desired phenotypes. Such use of nucleic acid sequences encoding a GRF polypeptide and/or a SYT polypeptide requires only a nucleic acid sequence of at least 15 nucleotides in length. The nucleic acid sequences encoding a GRF polypeptide and/or a SYT polypeptide may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acid sequences encoding a GRF polypeptide. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid sequence encoding a specific polypeptide in the genetic map previously obtained using this population (Botstein et al. (1980) Am. J. Hum. Genet. 32: 314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid sequence probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid sequence probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid sequence amplification-based methods for genetic and physical mapping may be carried out using the nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic acid sequence Res. 18:3671), Radiation Hybrid Mapping (Walter et al. (1997) Nat. Genet. 7:22-28) and Happy Mapping (Dear and Cook (1989) Nucleic acid sequence Res. 17:6795-6807). For these methods, the sequence of a nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield-related traits, as described hereinbefore. These traits may also be combined with other economically advantageous traits, such as further yield-increasing traits, tolerance to abiotic and biotic stresses, tolerance to herbicides, insectides, traits modifying various architectural features and/or biochemical and/or physiological features.

**Description of figures**

The present invention will now be described with reference to the following figures in which:

**Figure 1** represents a cartoon of a GRF polypeptide as represented by SEQ ID NO: 2, which comprises the following features: (i) a QLQ domain with an InterPro accession IPR014978 (PFAM accession PF08880); (ii) a WRC domain with an InterPro accession IPR014977 (PFAM accession PF08879); and (iii) an Effector of Transcription (ET) domain comprising three Cys and one His residues in a conserved spacing ($CX_9CX_{10}CX_2H$), and located within the WRC domain.

**Figure 2** shows an AlignX (from Vector NTI 10.3, Invitrogen Corporation) multiple sequence alignment of the QLQ domain of GRF polypeptides from Table A.1 (as represented by SEQ ID NO: 115 for SEQ ID NO: 2). The conserved QLQ amino acid residues are located on the top of the multiple alignement. Two other very conserved residues (boxed in black) are E (Glu) and P (Pro).

**Figure 3** shows an AlignX (from Vector NTI 10.3, Invitrogen Corporation) multiple sequence alignment of the WRC domain of GRF polypeptides from Table A.1 (as represented by SEQ ID NO: 116 for SEQ ID NO: 2). The conserved WRC amino acid residues are in bold in the consensus sequence. The three Cys and one His residues in a conserved spacing ($CX_9CX_{10}CX_2H$), designated as the Effector of Transcription (ET) domain, are boxed vertically across the alignement, and also identified at the bottom of the alignment. The putative nuclear localisation signal (NLS) comprised in the WRC domain, is double-underlined.

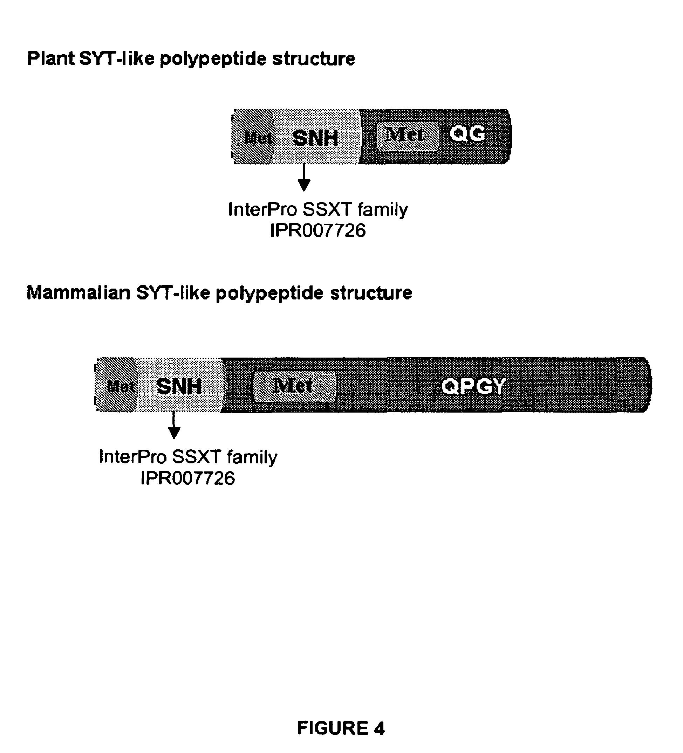

**Figure 4** shows the typical domain structure of SYT polypeptides from plants and mammals. The conserved SNH domain is located at the N-terminal end of the polypeptide. The C-terminal remainder of the polypeptide consists of a QG-rich domain in plant SYT polypeptides, and of a QPGY-rich domain in mammalian SYT polypeptides. A Met-rich domain is typically comprised within the first half of the QG-rich (from the N-term to the C-term) in plants or QPGY-rich in mammals. A second Met-rich domain may precede the SNH domain in plant SYT polypeptides

**Figure 5** shows a multiple alignment of the N-terminal end of several SYT polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The SNH domain is boxed across the plant and human SYT polypeptides. The last line in the alignment consists of a consensus sequence derived from the aligned sequences.

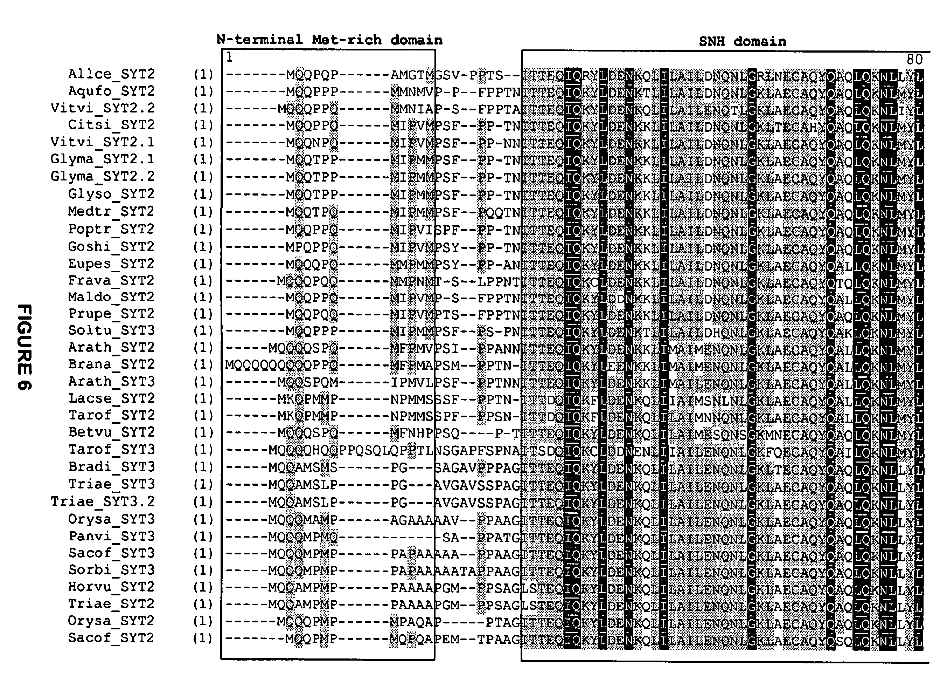

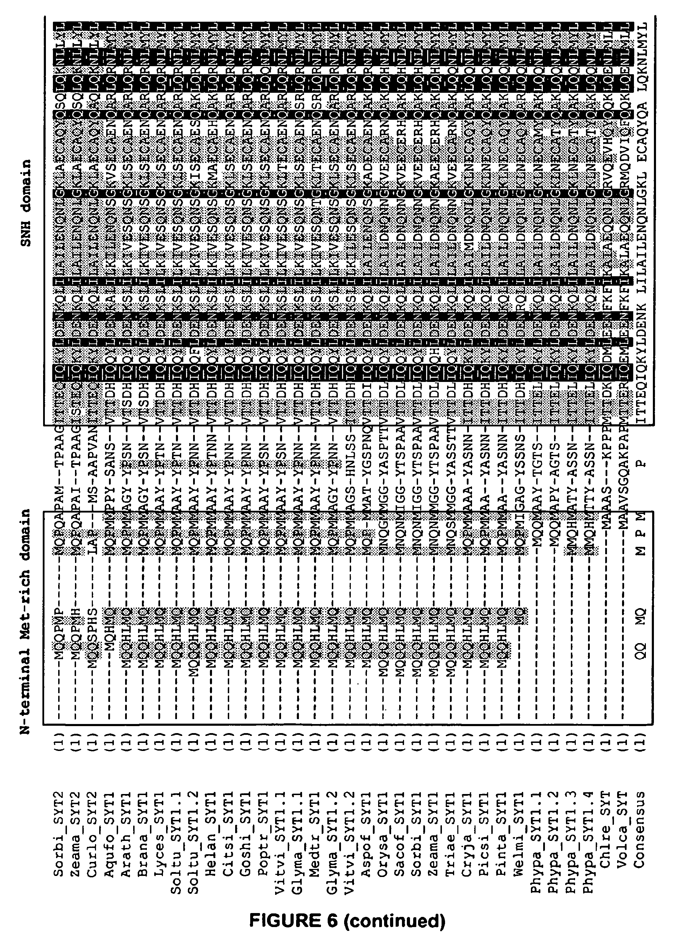

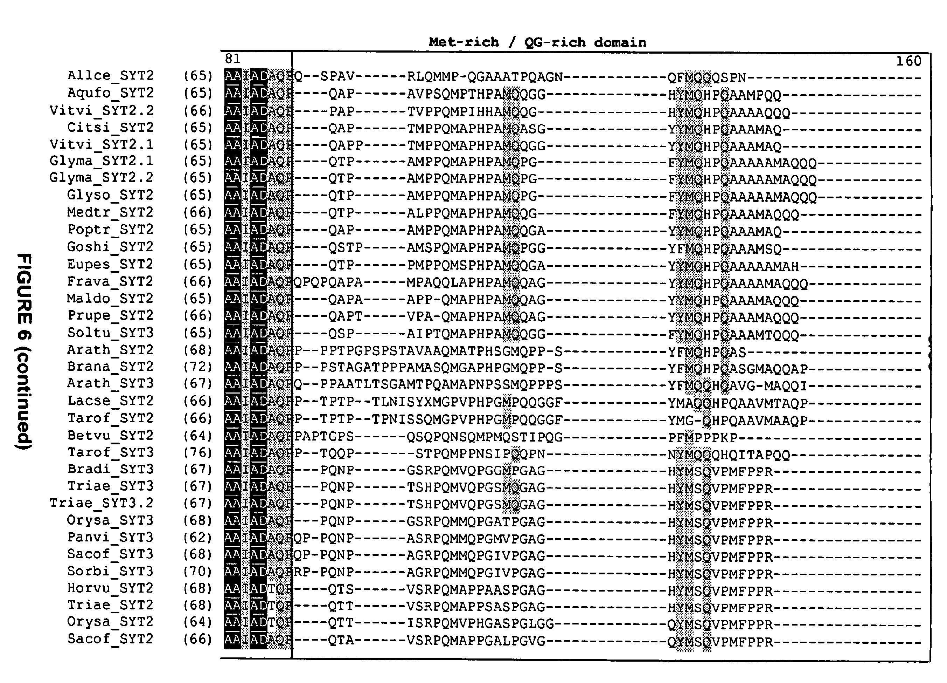

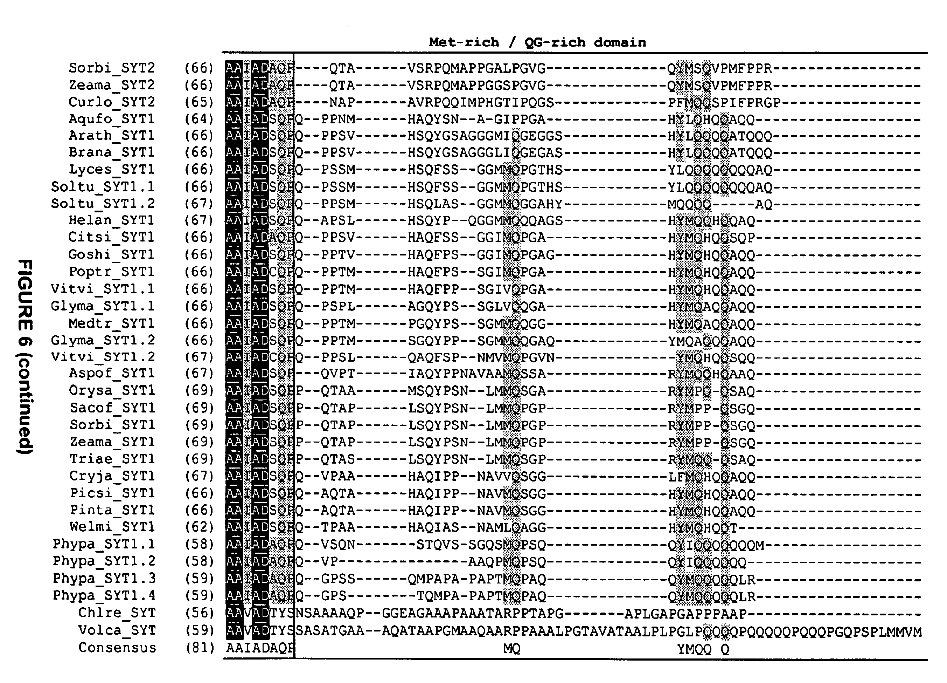

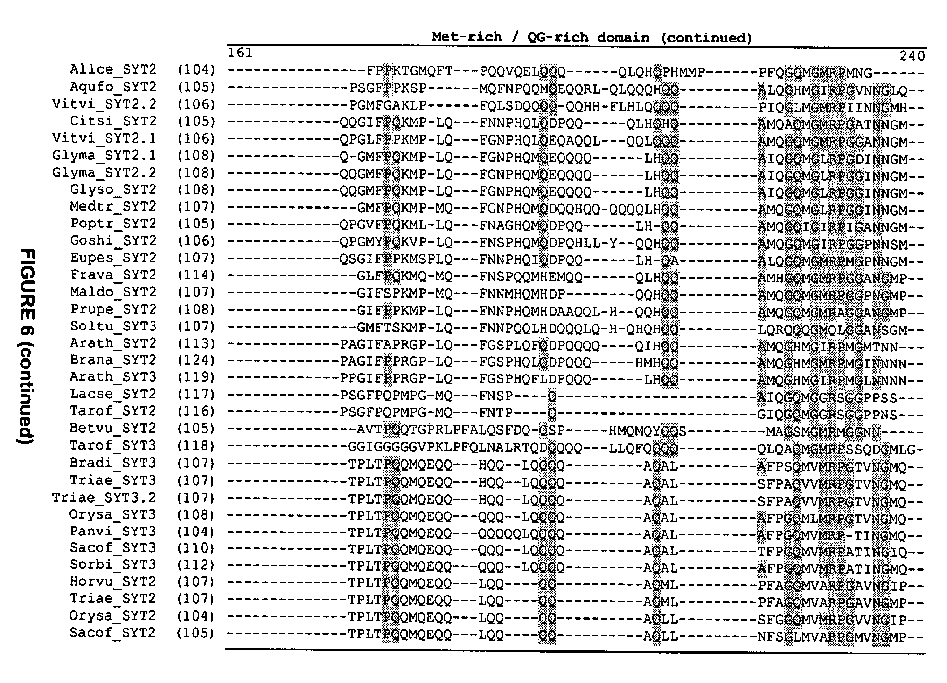

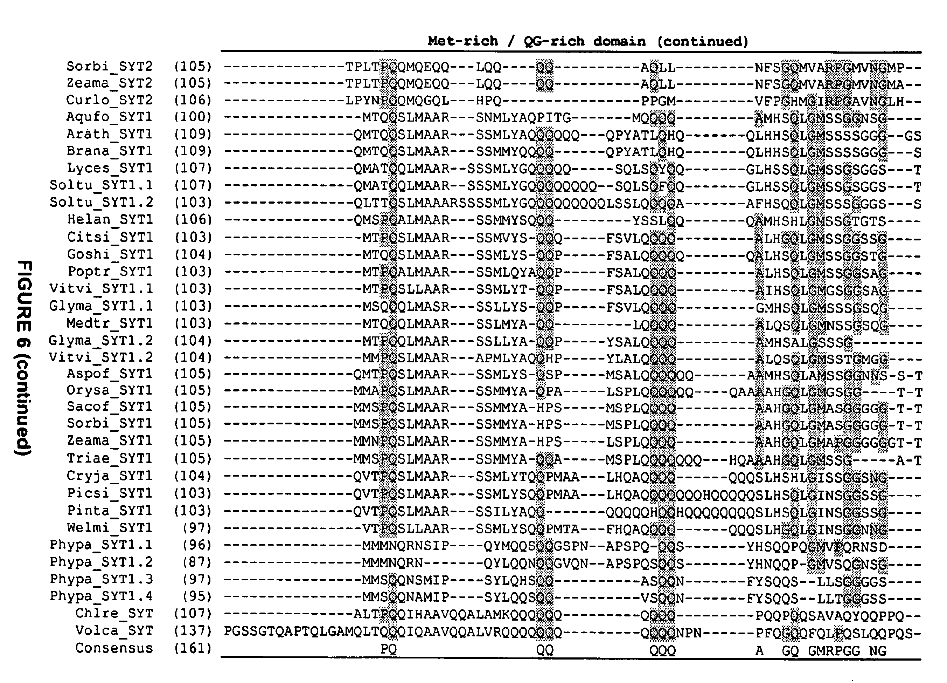

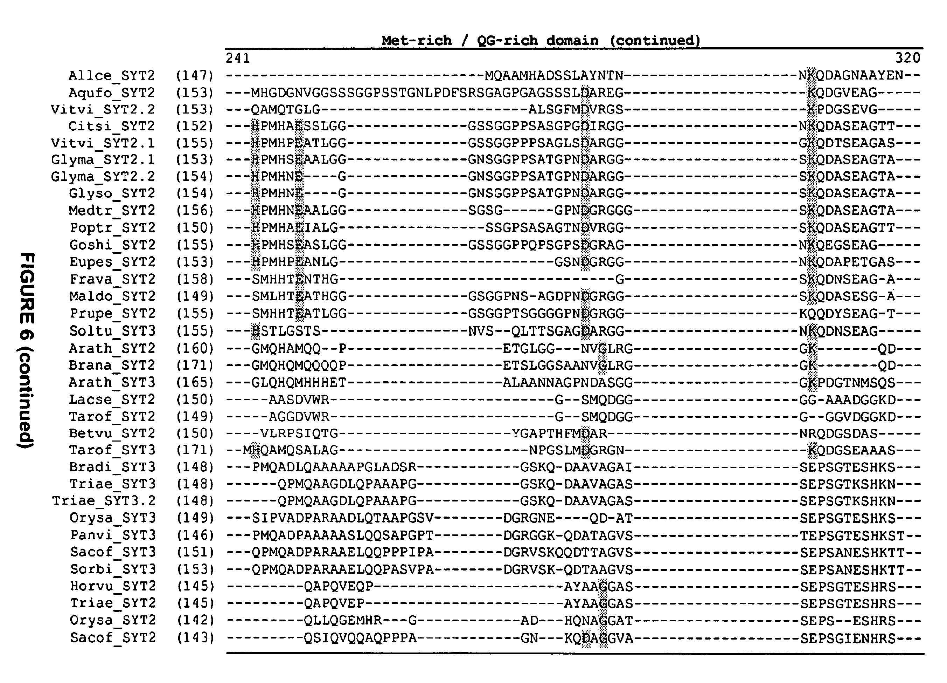

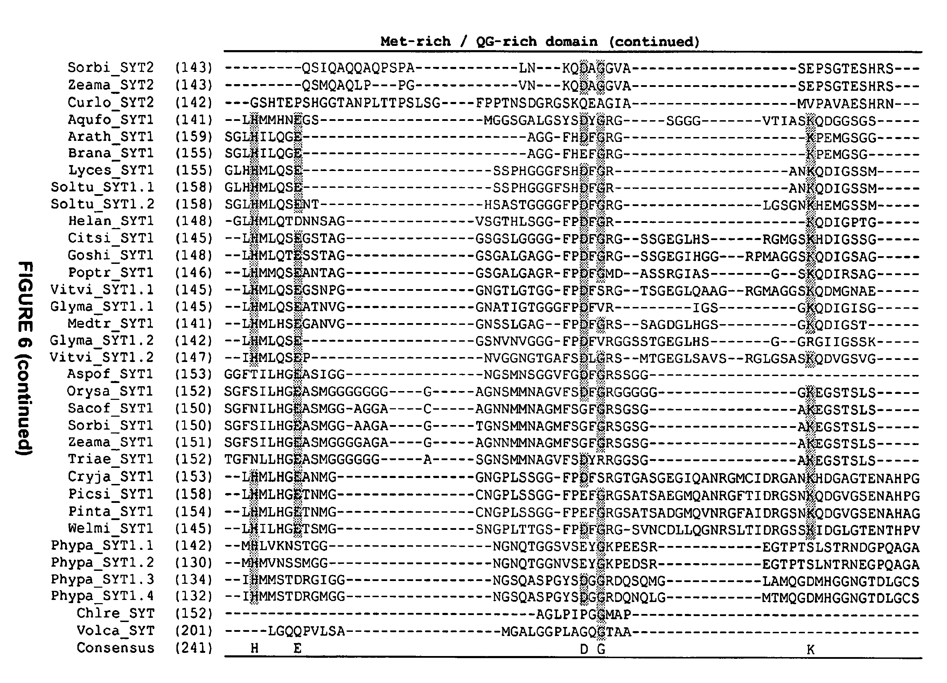

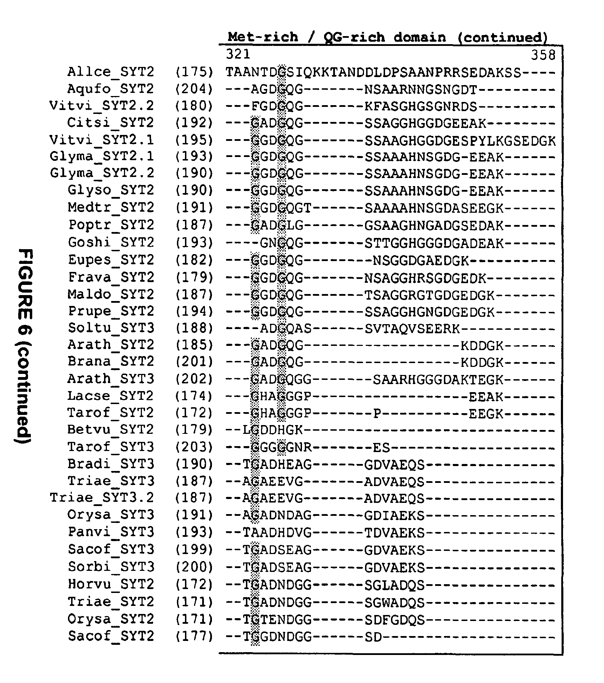

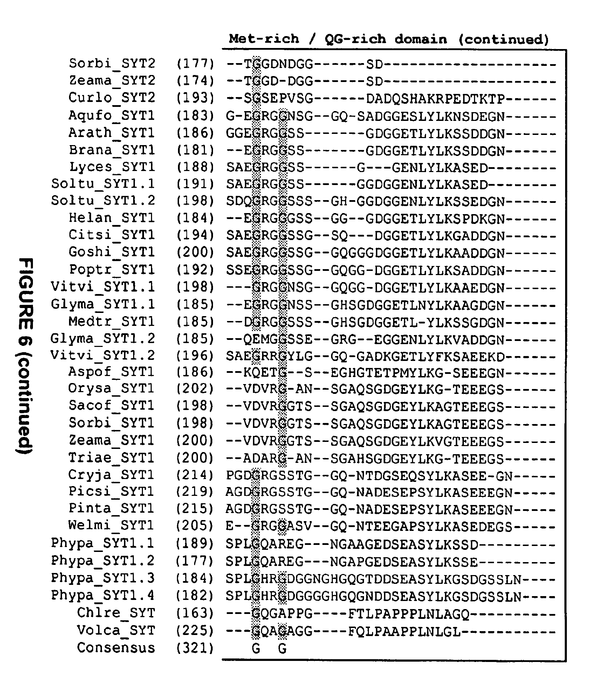

**Figure 6** shows a multiple alignment of several plant SYT polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The two main domains, from N-terminal to C-terminal, are boxed and identified as SNH domain and the Met-rich/QG-rich domain. Additionally, the N-terminal Met-rich domain is also boxed, and the positions of SEQ ID NO: 90 and SEQ ID NO 91 are underlined in bold.

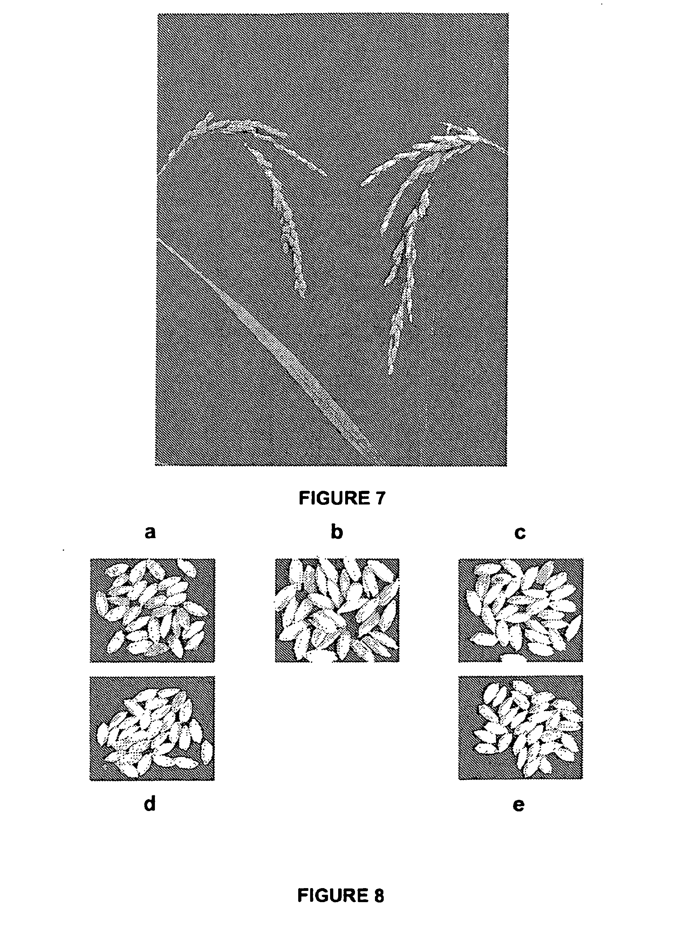

**Figure 7** shows on the left a panicle from a rice plant (*Oryza sativa* ssp. Japonica cv.

Nipponbare) transformed with a control vector, and on the right a panicle from a rice plant (*Oryza sativa* ssp. Japonica cv. Nipponbare) transformed with two constructs: (1) a nucleic acid sequence encoding a GRF polypeptide under the control of a GOS2 promoter (pGOS2) from rice; and (2) a nucleic acid sequence encoding a SYT polypeptide under the control of a GOS2 promoter (pGOS2) from rice;

**Figure 8** shows on the top row, from left to right, 30 mature rice seeds (*Oryza sativa* ssp. Japonica cv. Nipponbare) from:

a. plants transformed with one construct comprising a nucleic acid sequence encoding a SYT polypeptide under the control of a GOS2 promoter (pGOS2) from rice;
b. plants transformed with two constructs: (1) a nucleic acid sequence encoding a GRF polypeptide under the control of a GOS2 promoter (pGOS2) from rice; and (2) a nucleic acid sequence encoding a SYT polypeptide under the control of a GOS2 promoter (pGOS2) from rice;
c. plants transformed with one construct comprising a nucleic acid sequence encoding a GRF polypeptide under the control of a GOS2 promoter (pGOS2) from rice;
d. nullizygote plants (control plants) from a;
e. nullizygote plants (control plants) from c;

**Figure 9** shows the binary vector for increased expression in *Oryza sativa* of a nucleic acid sequence encoding a GRF polypeptide under the control of a GOS2 promoter (pGOS2) from rice, or alternatively for increased expression in *Oryza sativa* of a nucleic acid sequence encoding a SYT polypeptide under the control of a GOS2 promoter (pGOS2) from rice.

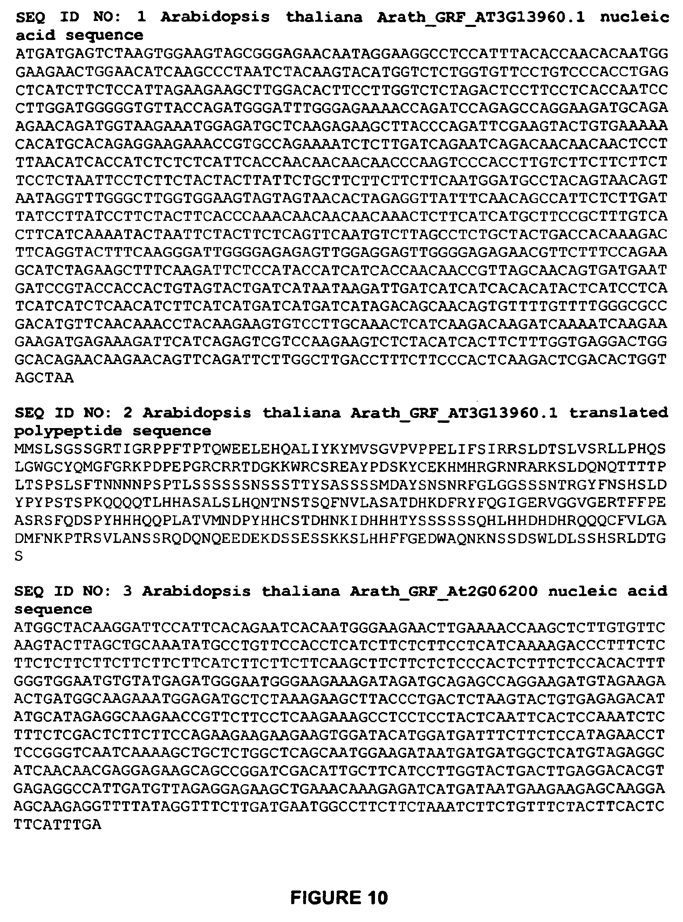

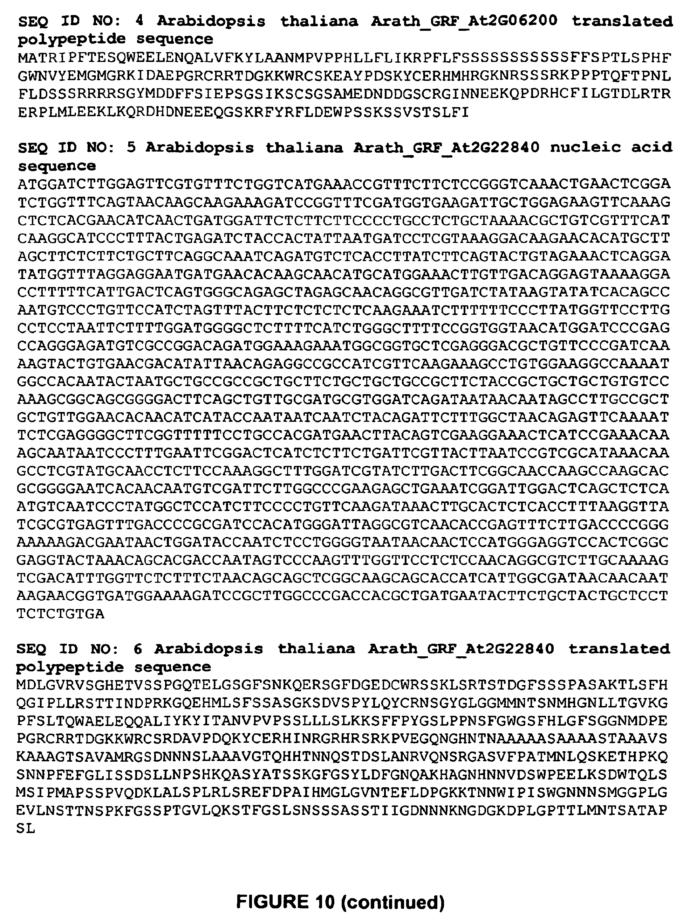

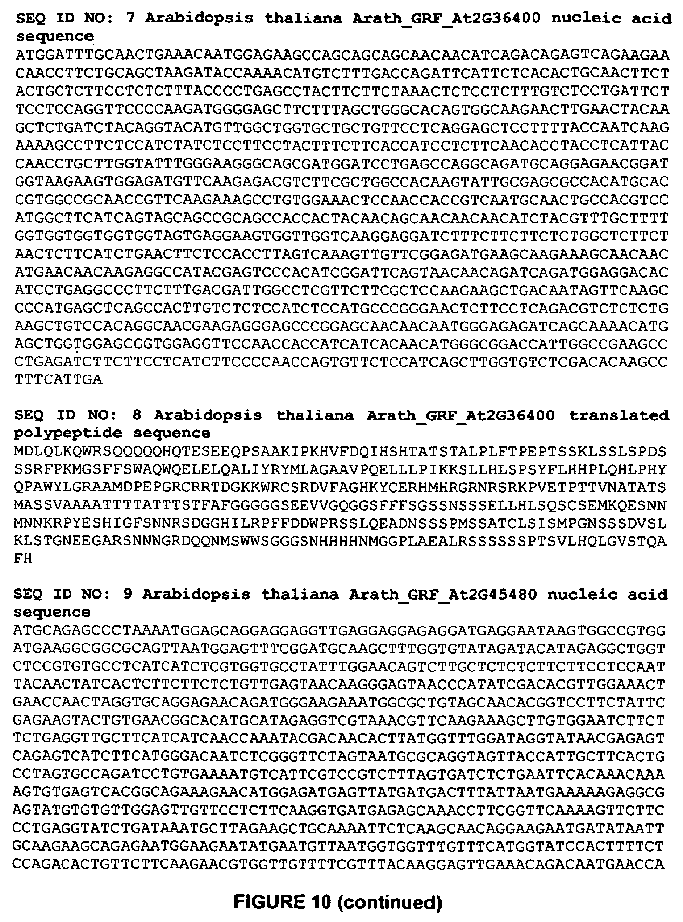

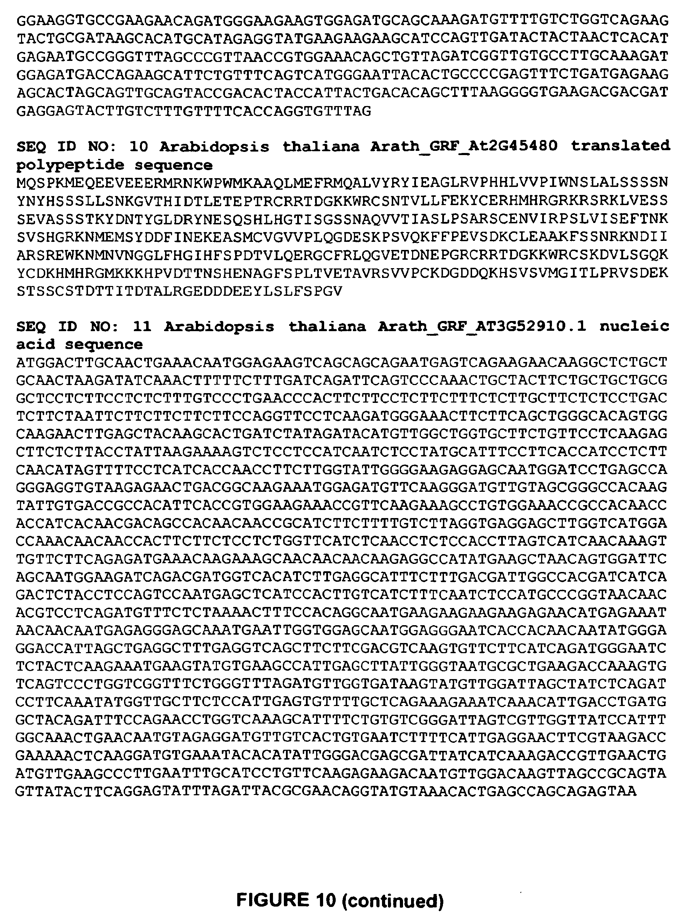

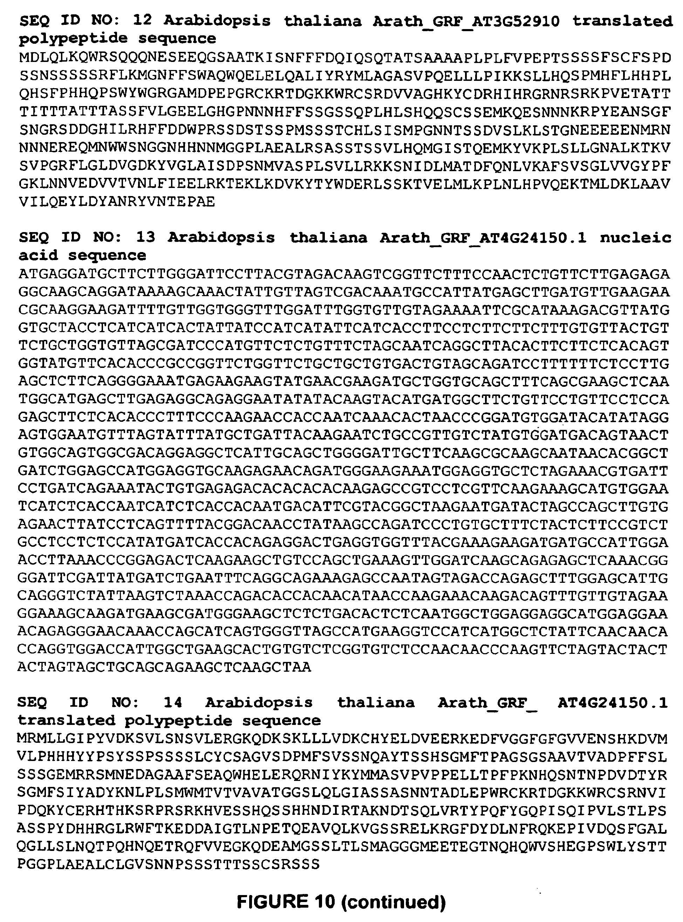

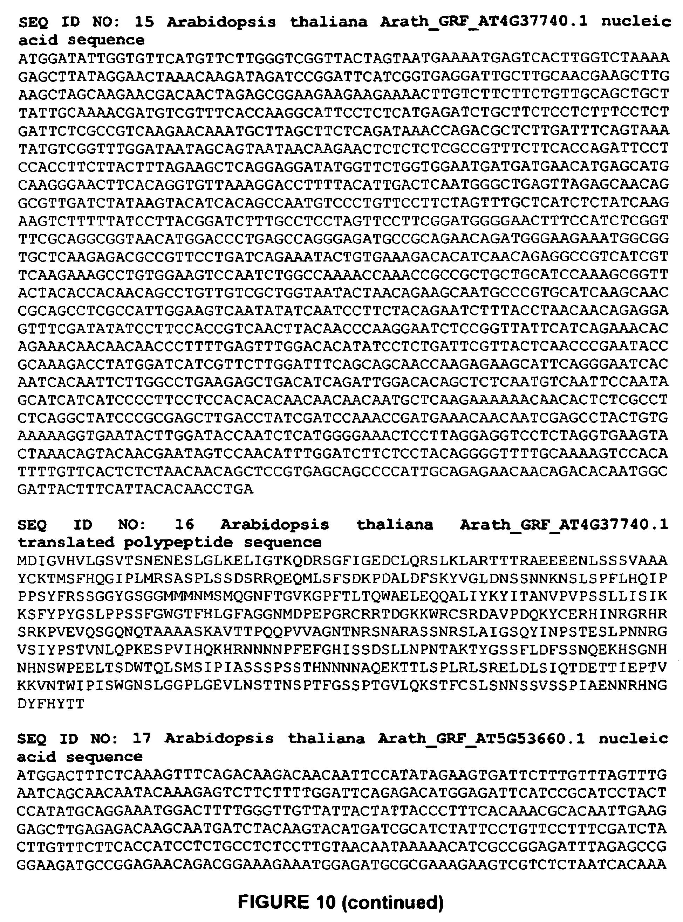

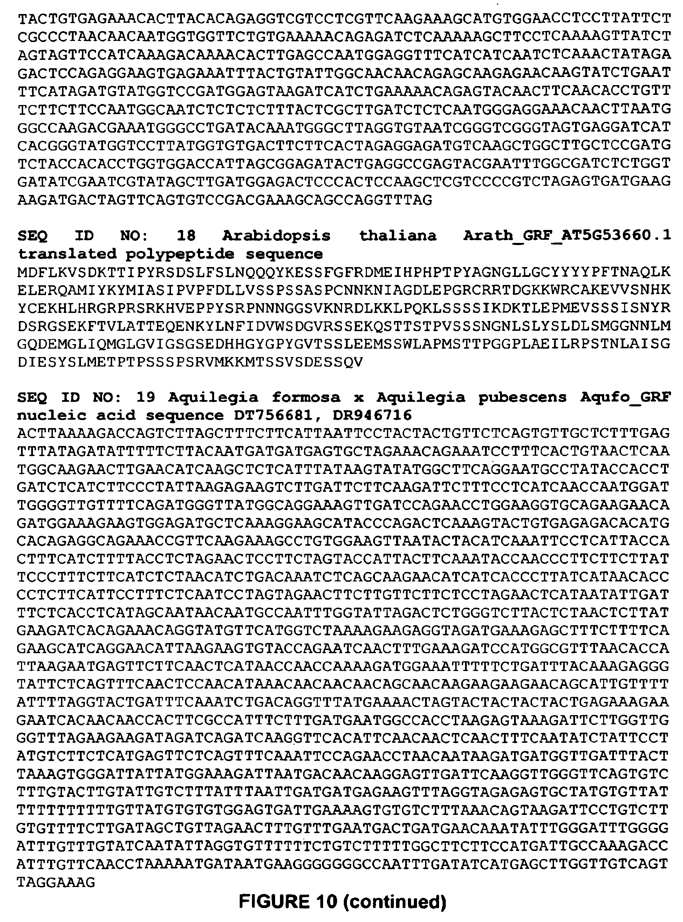

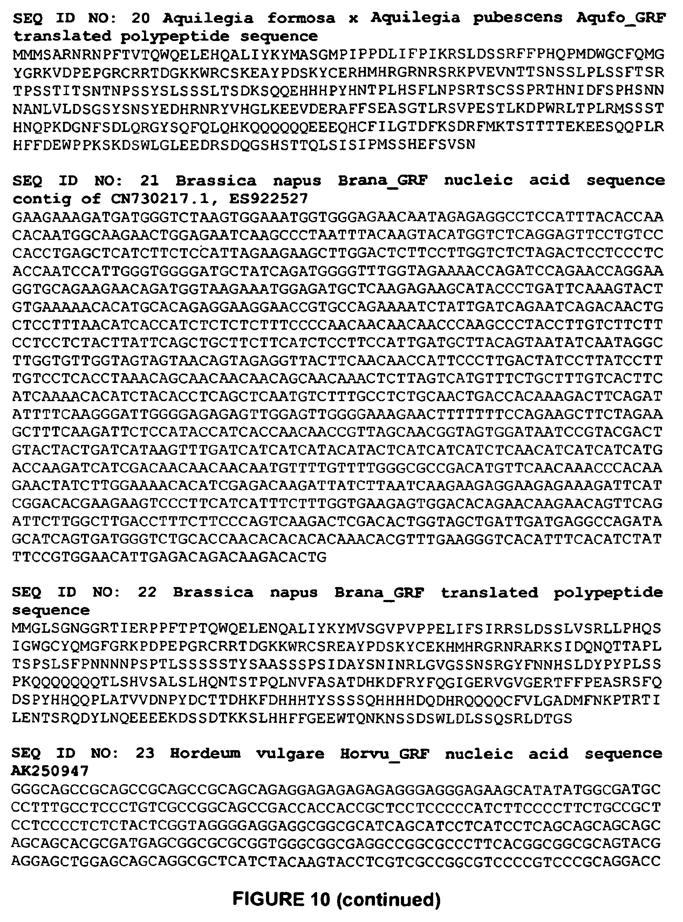

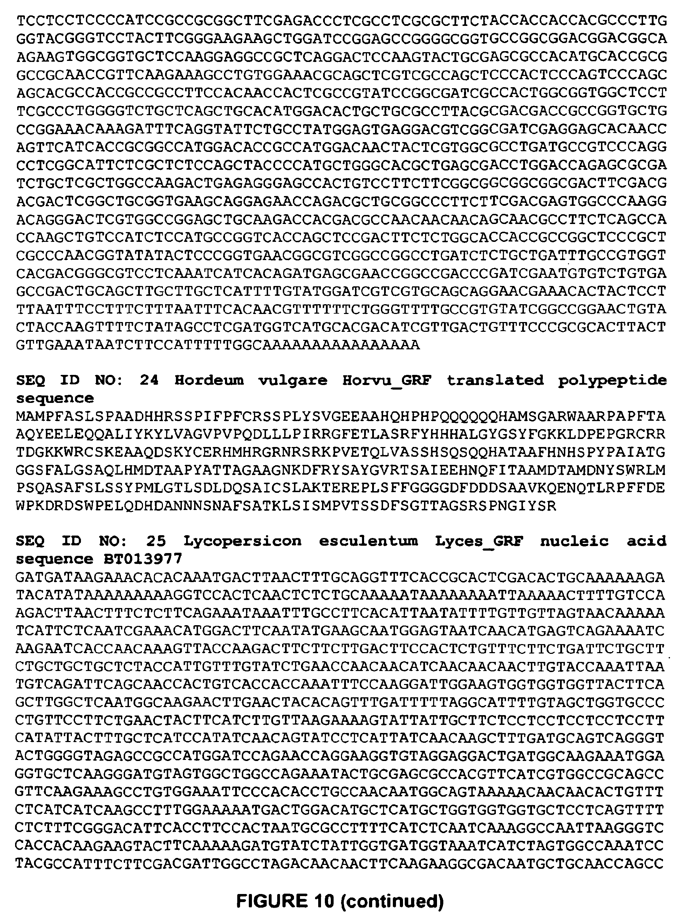

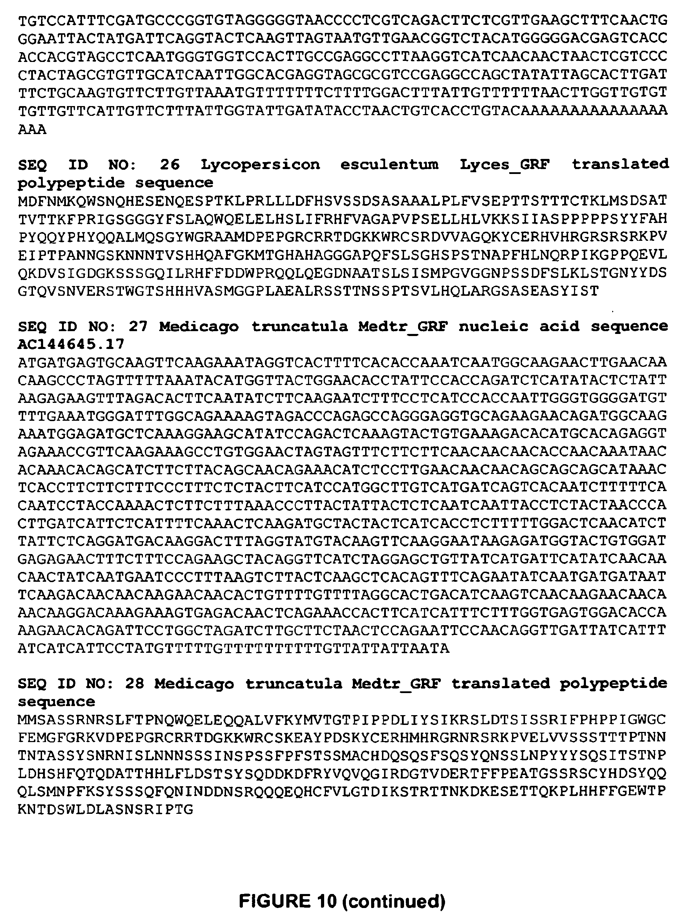

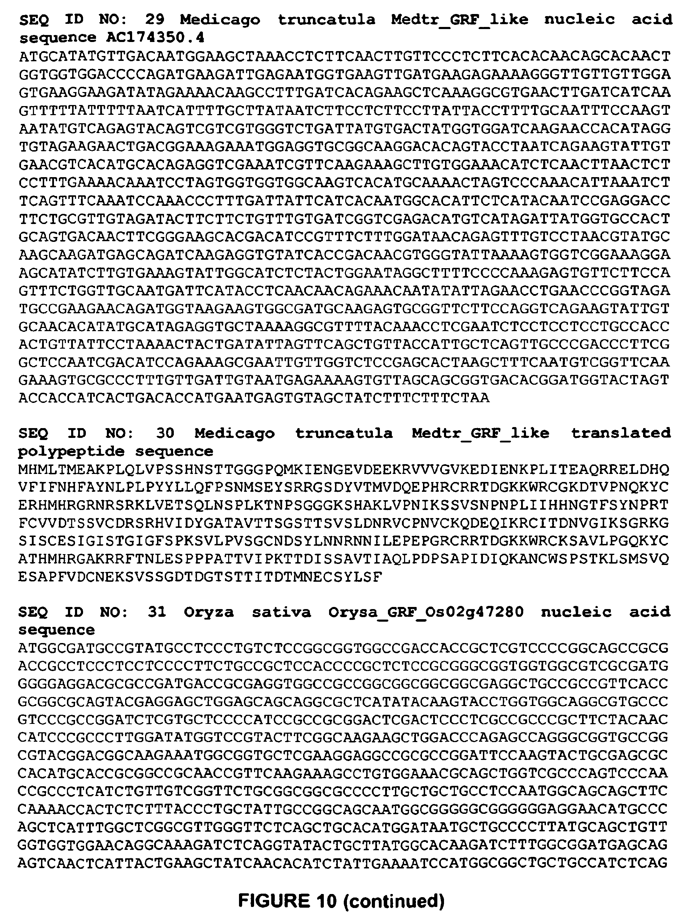

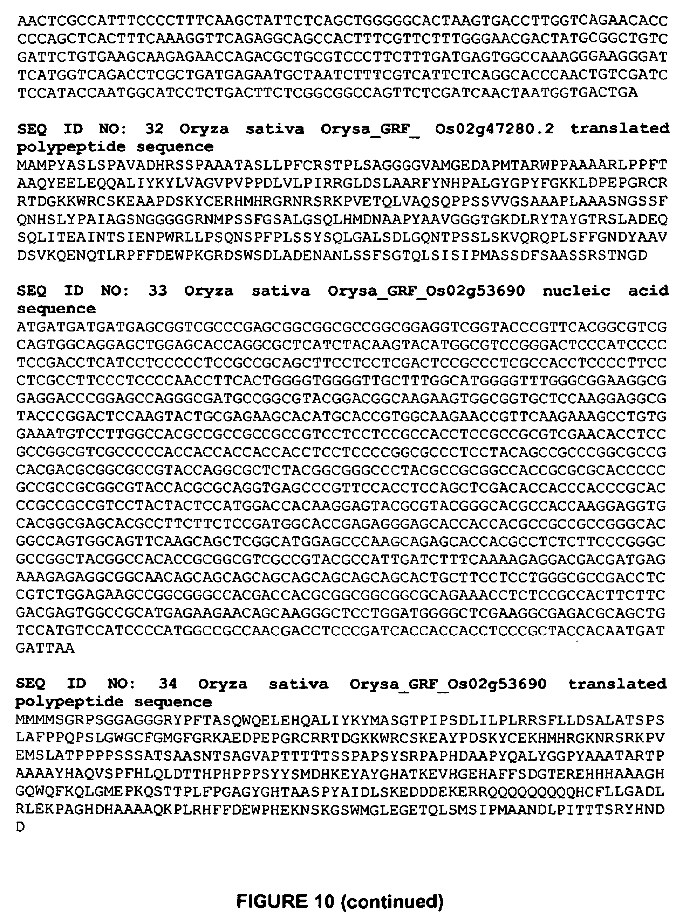

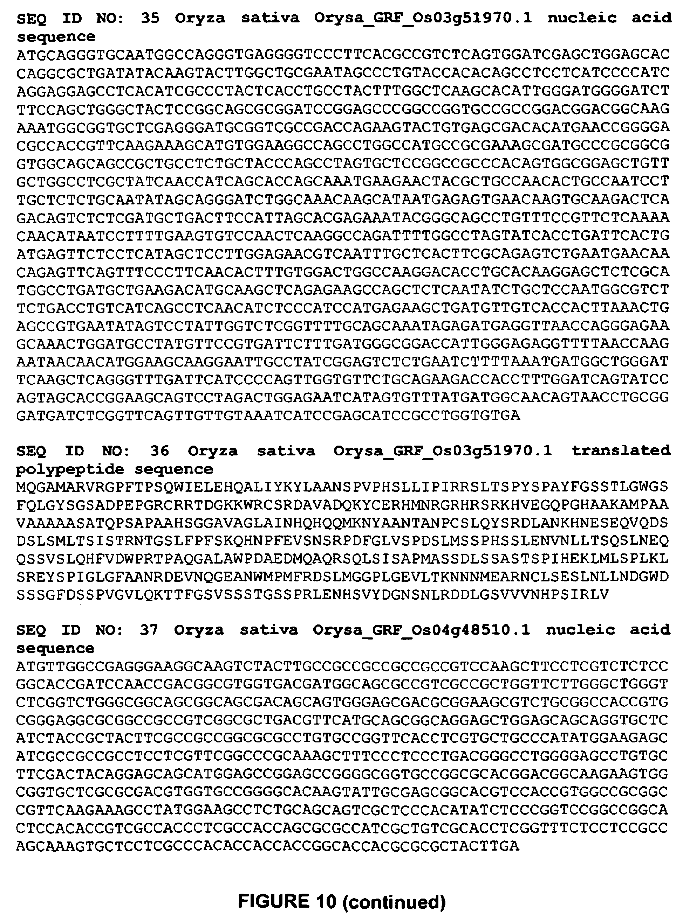

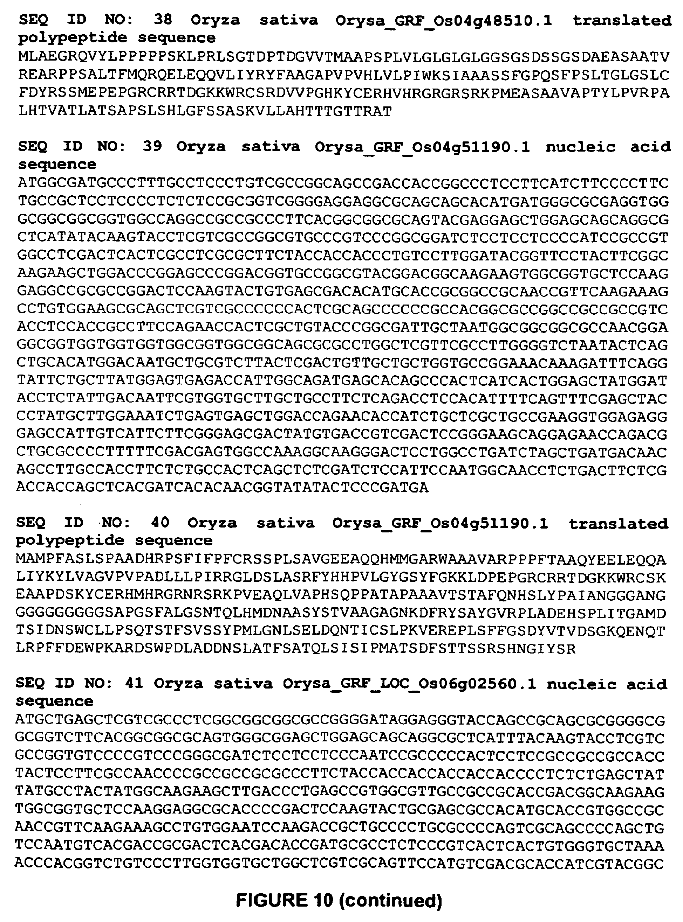

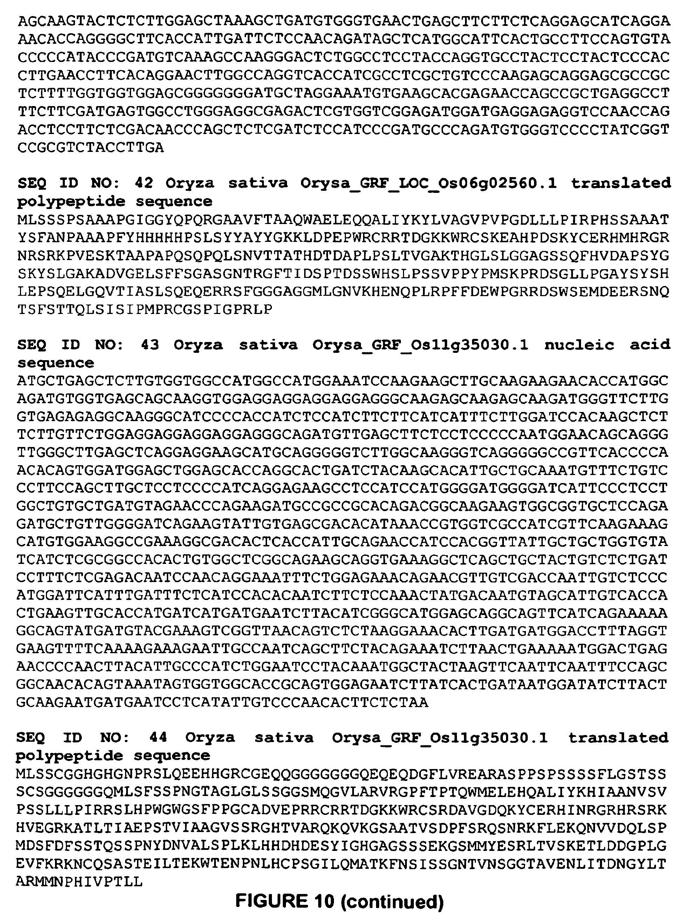

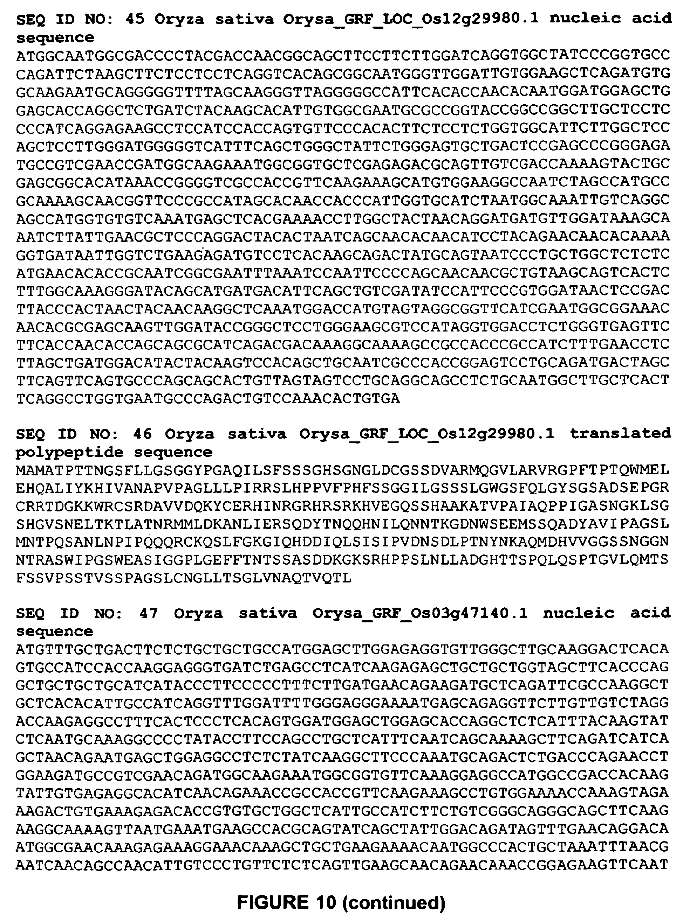

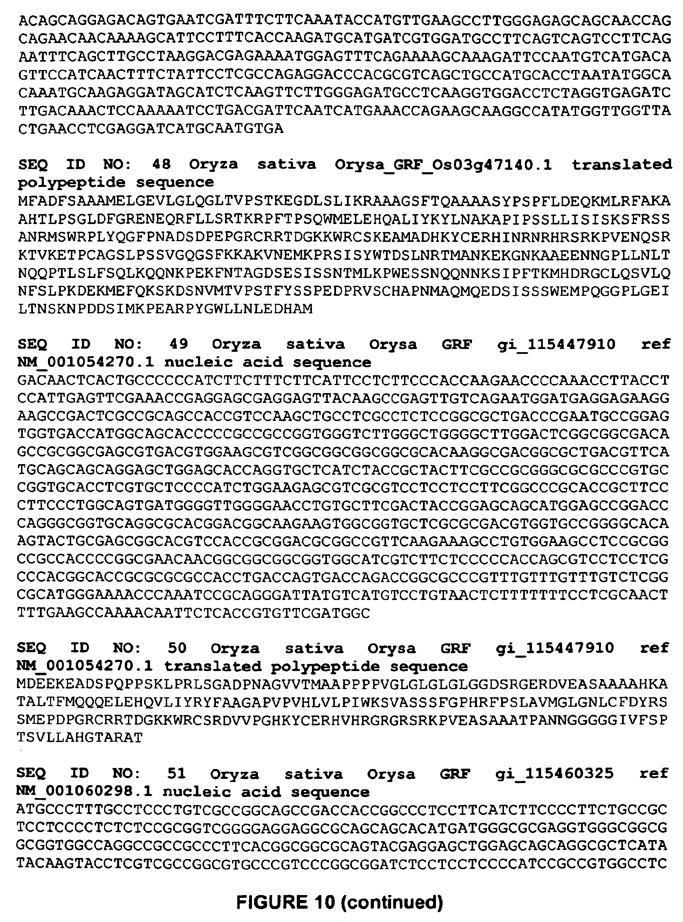

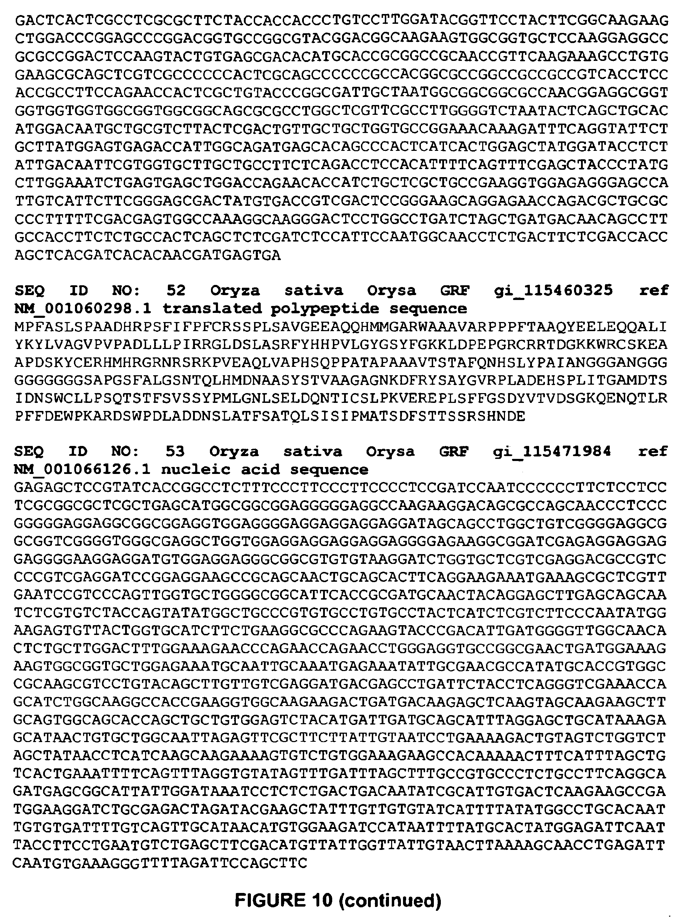

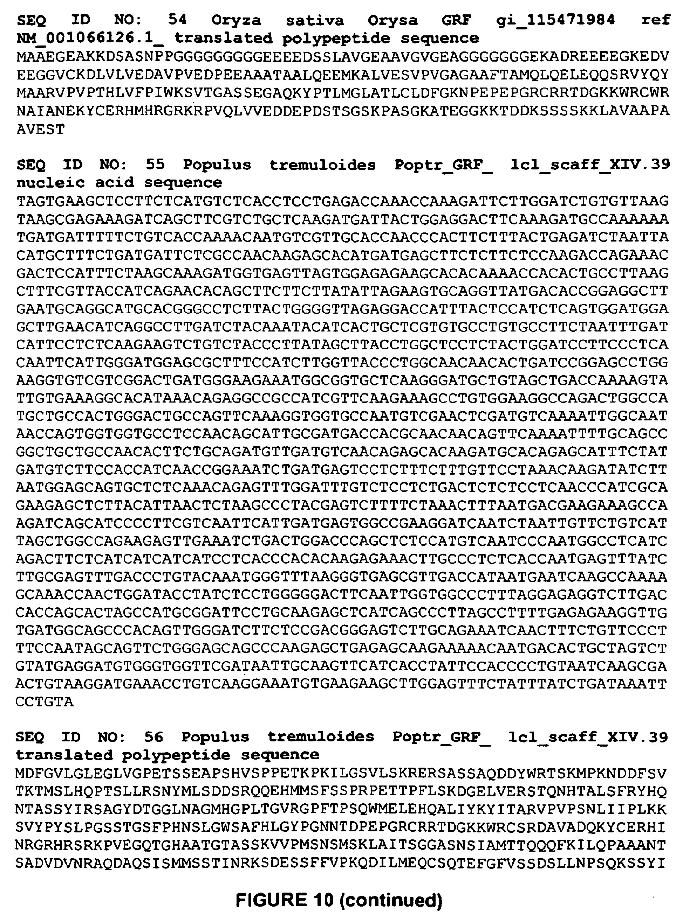

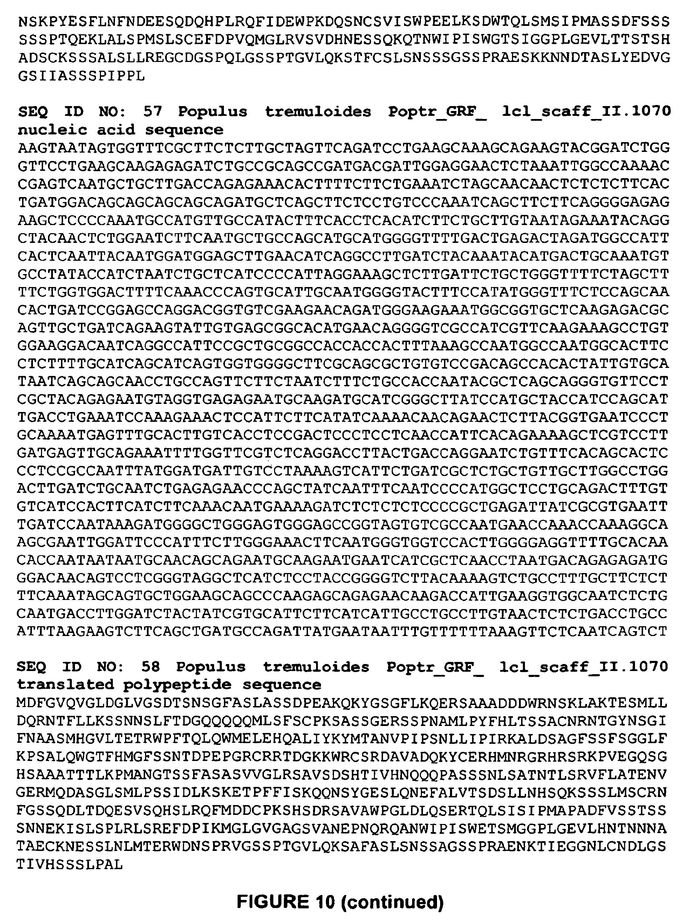

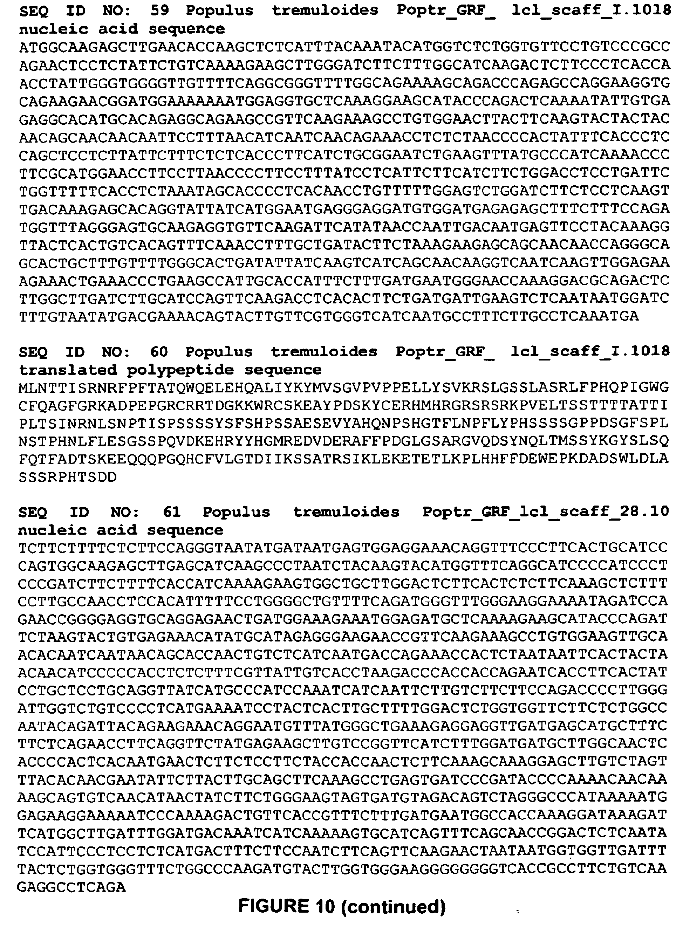

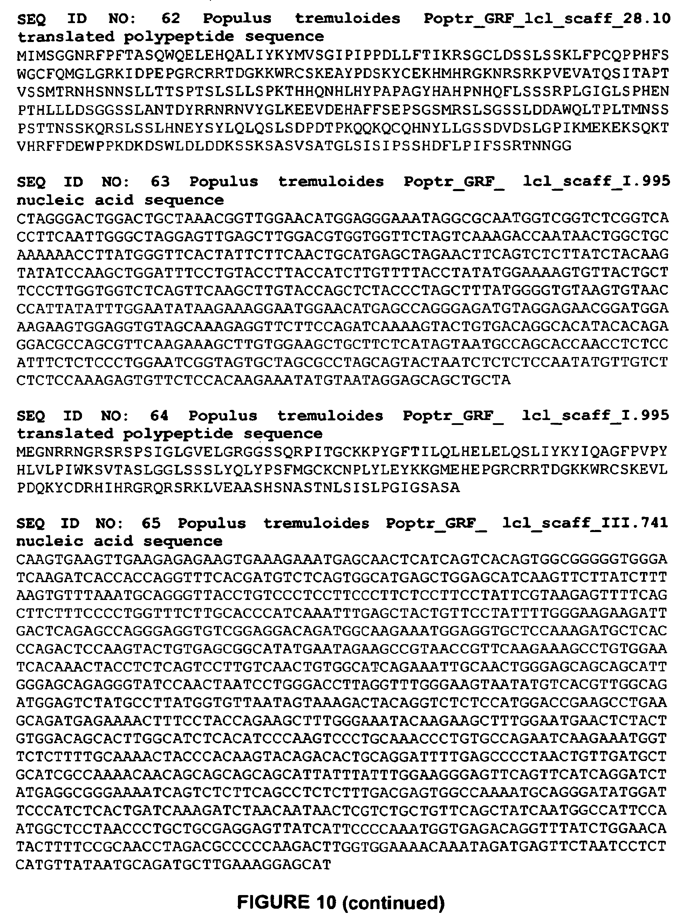

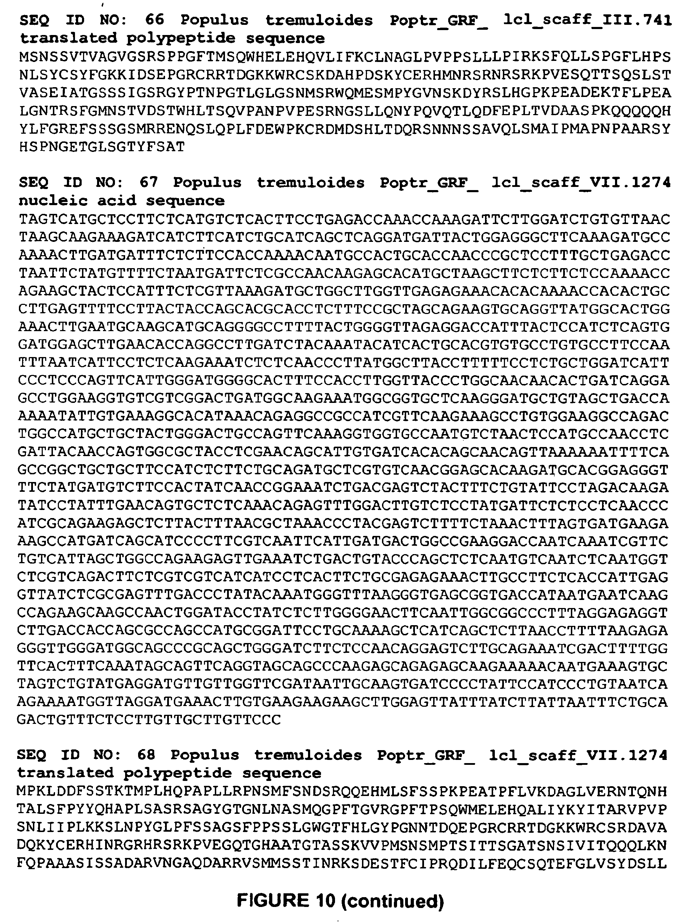

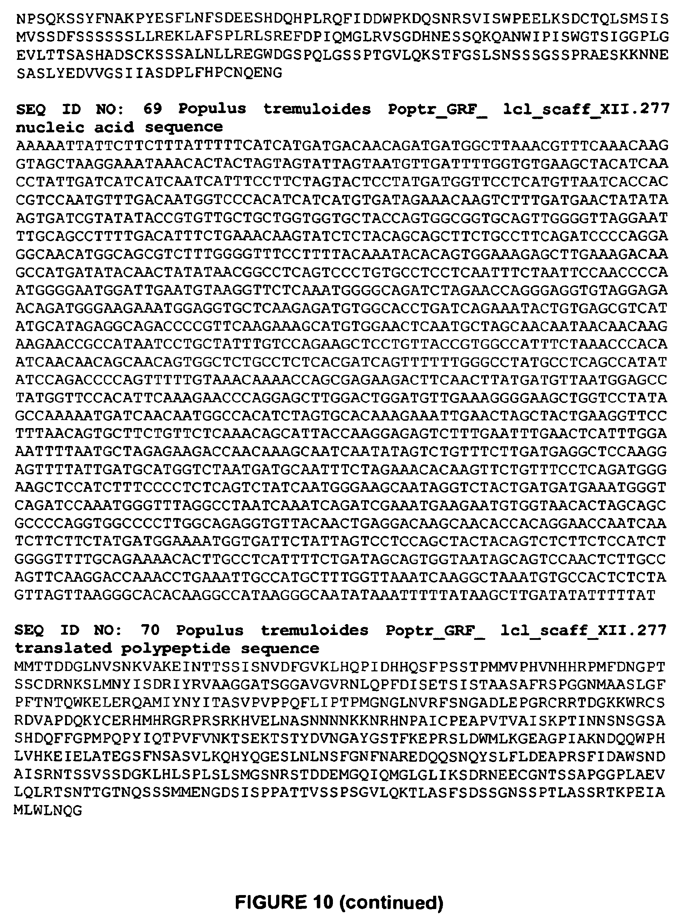

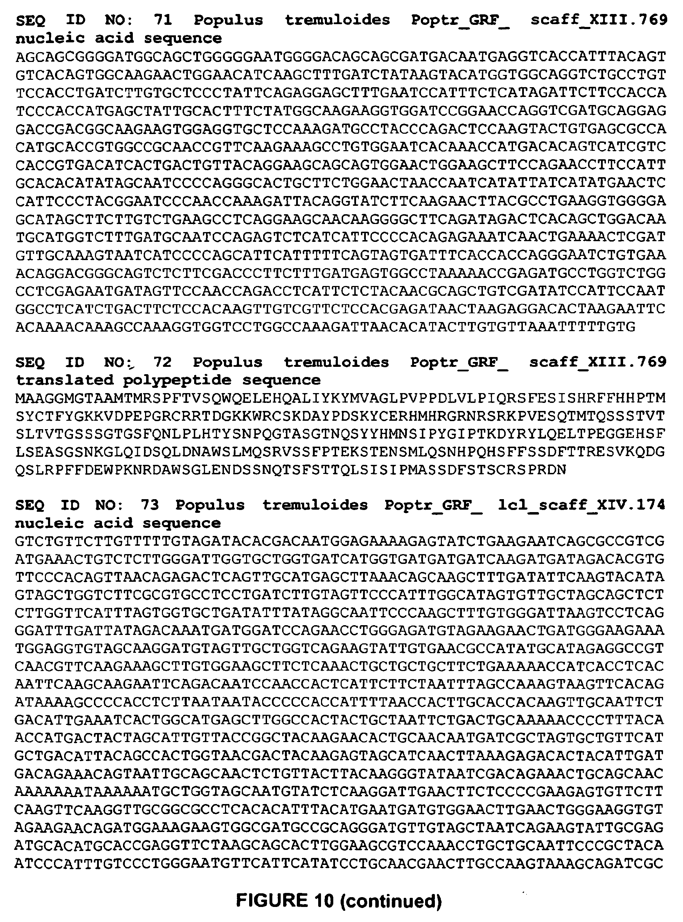

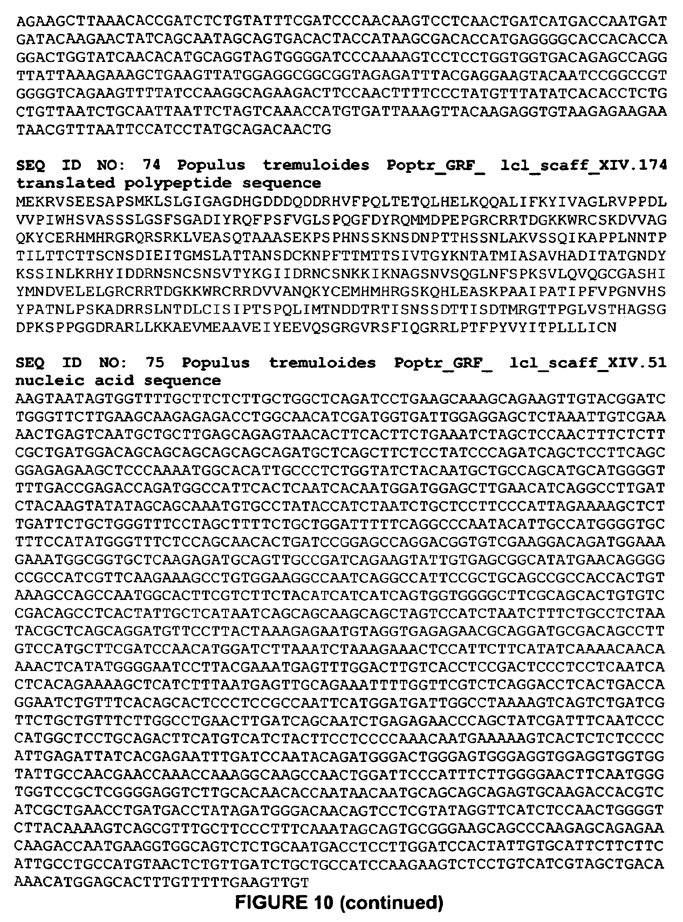

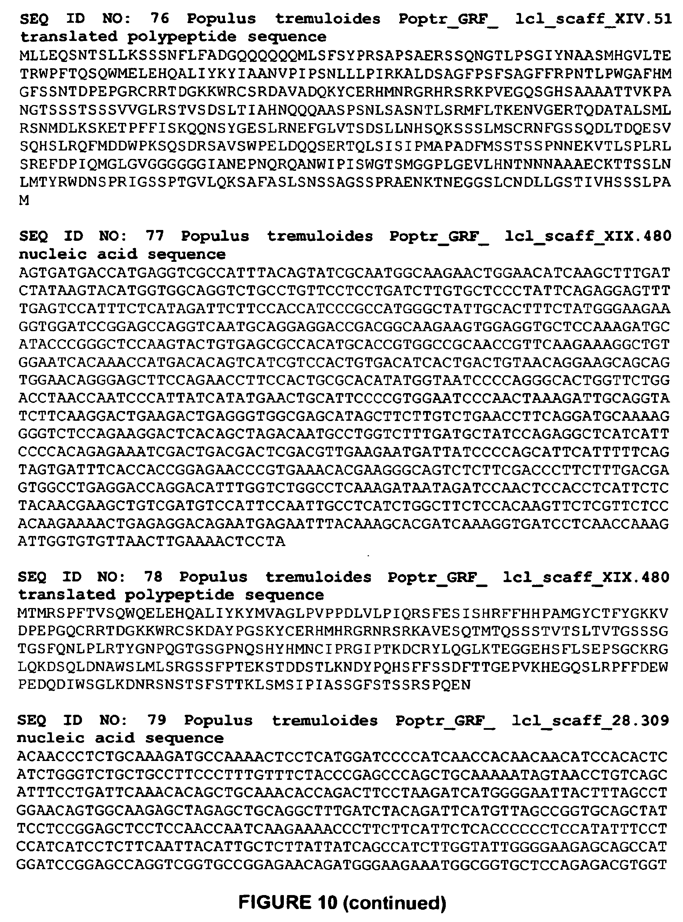

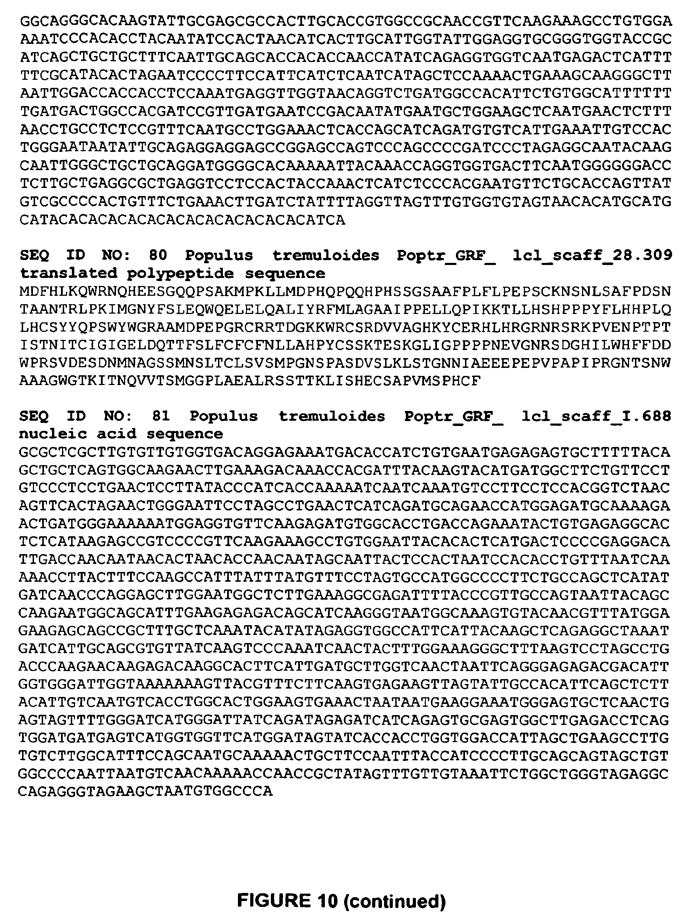

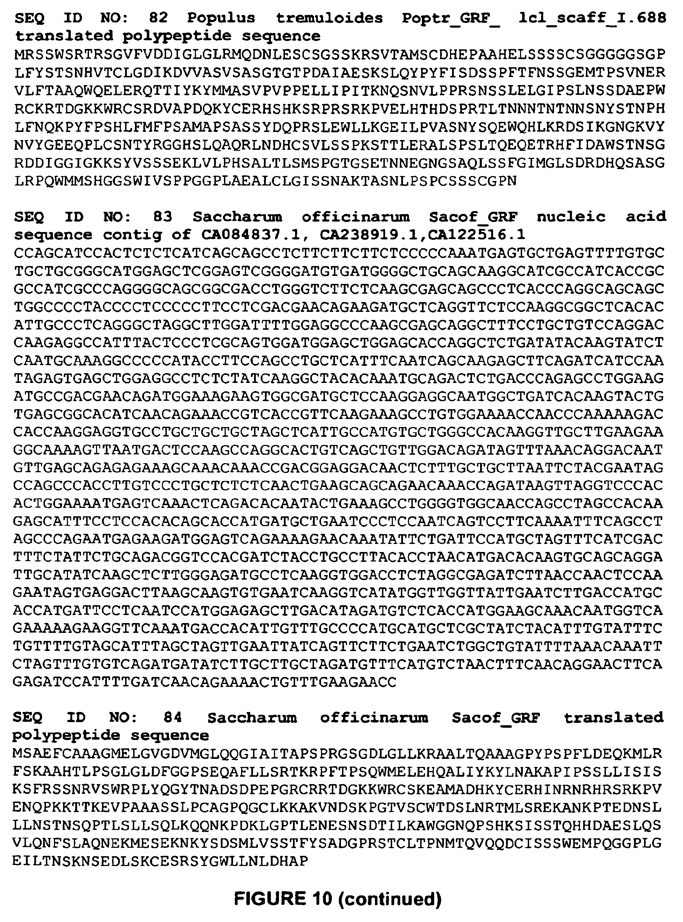

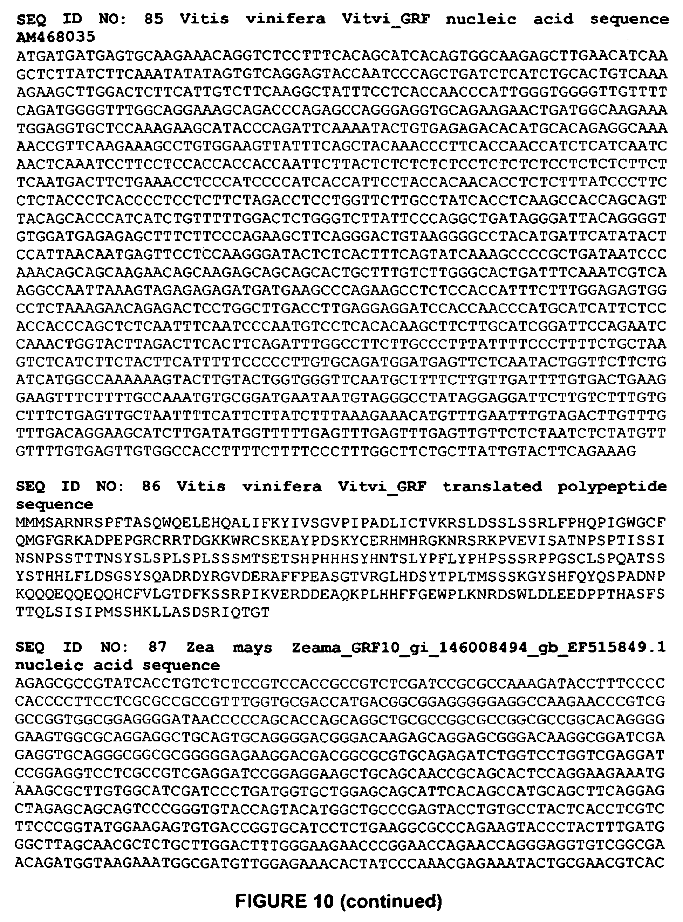

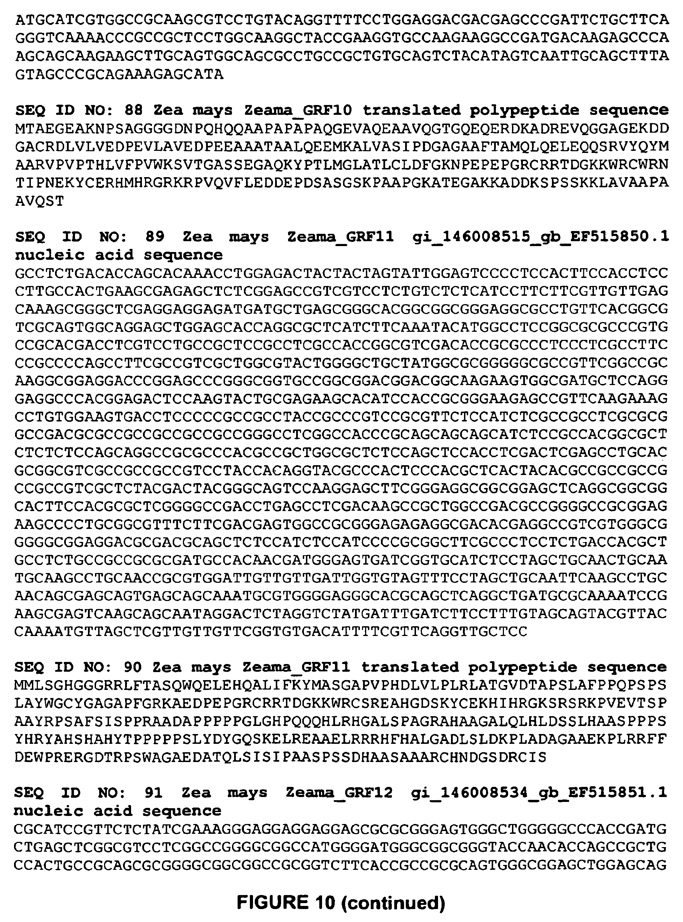

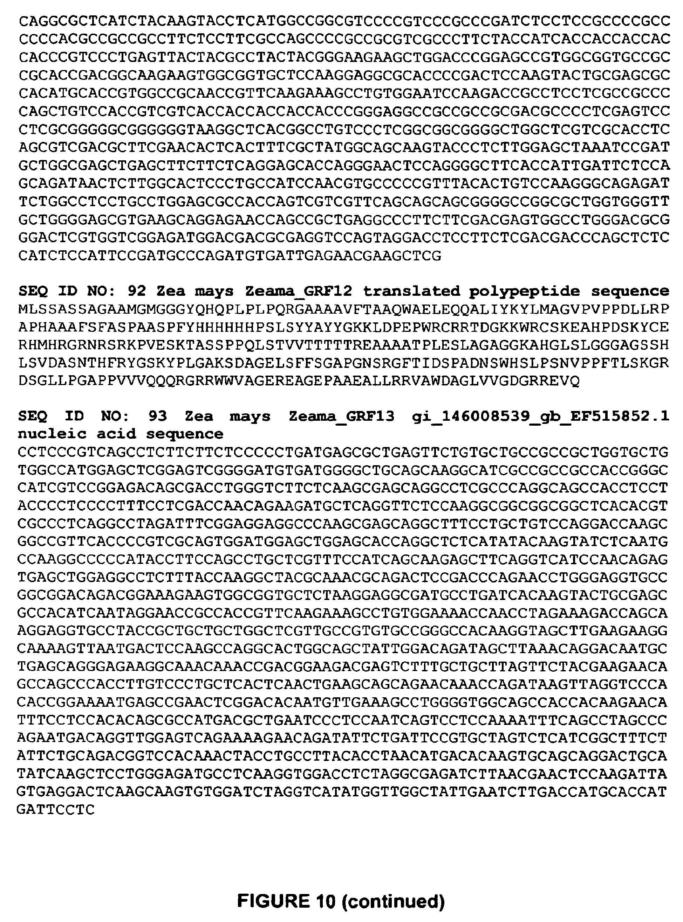

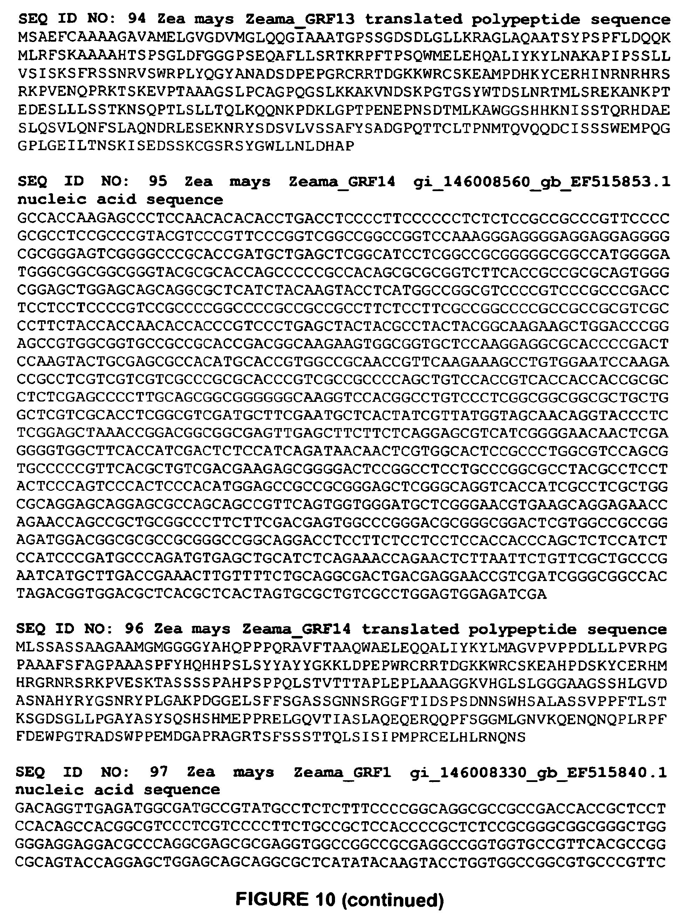

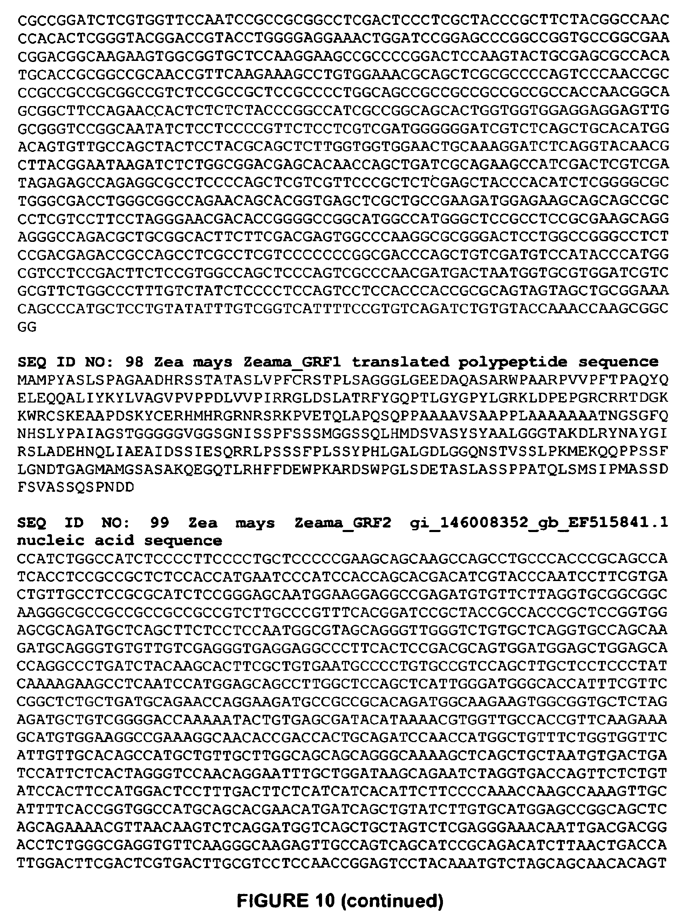

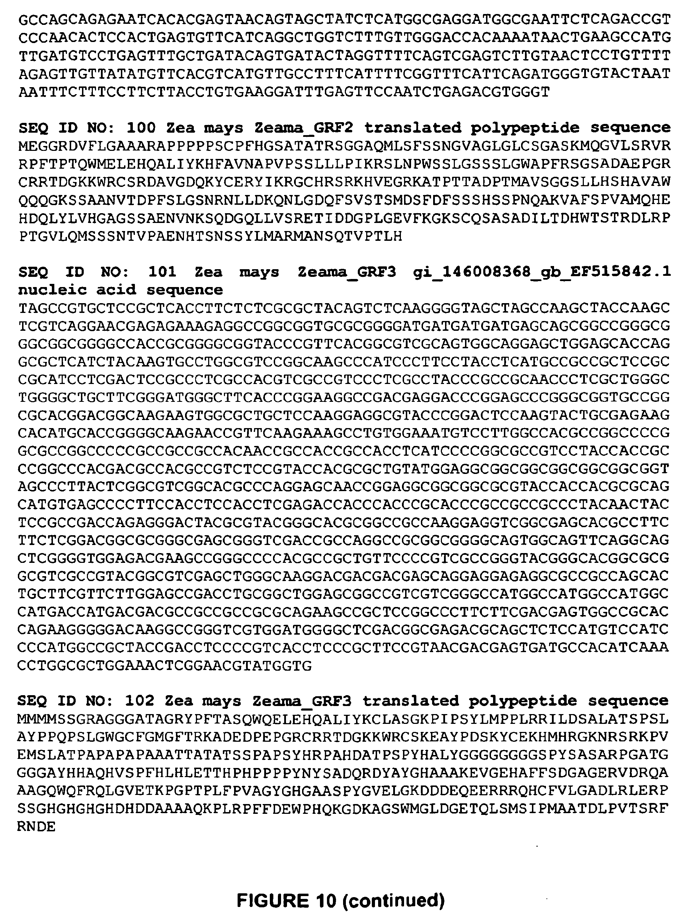

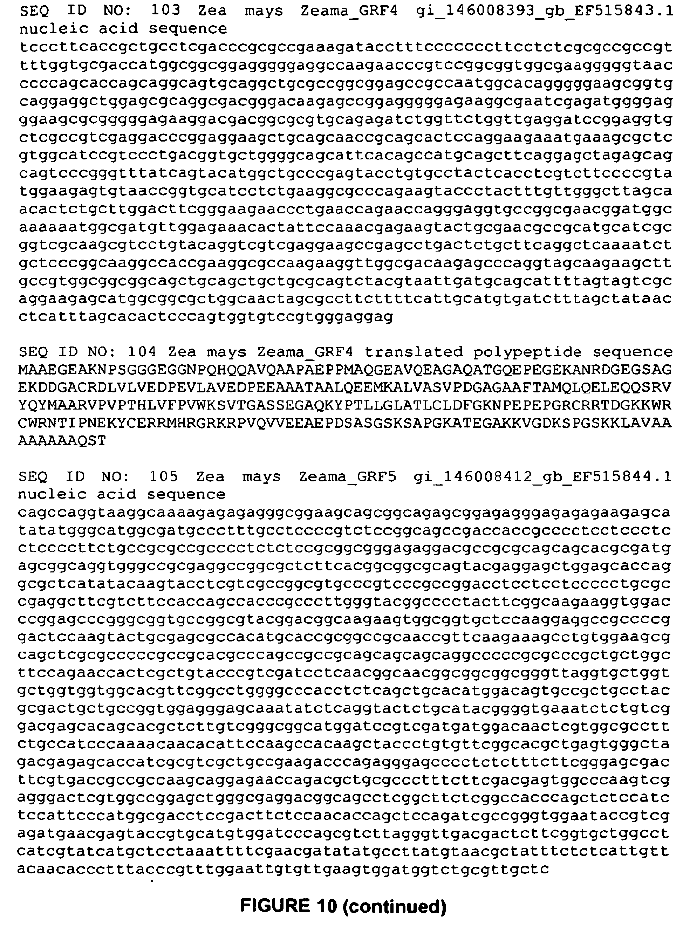

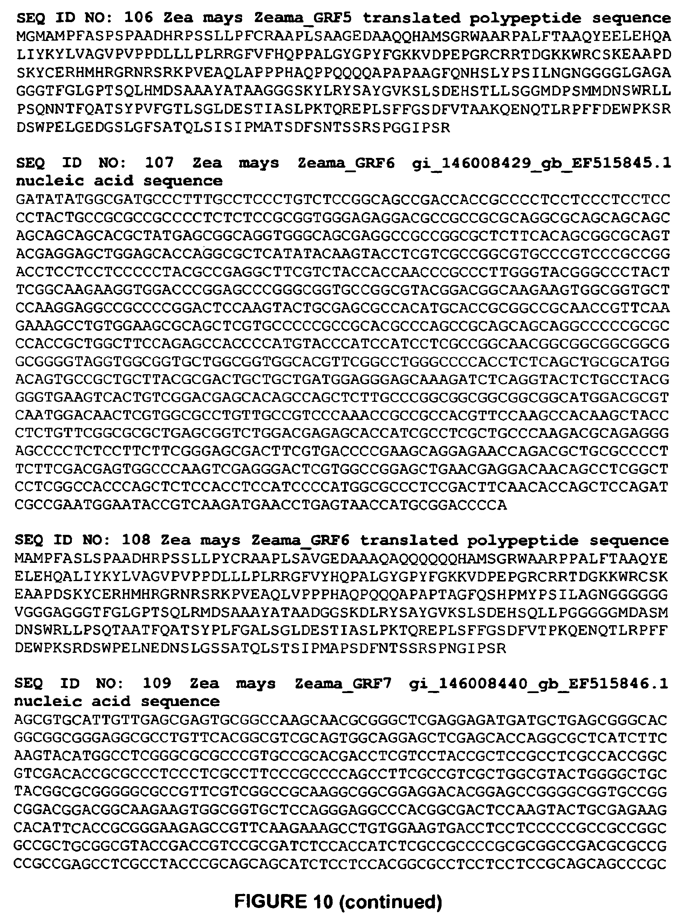

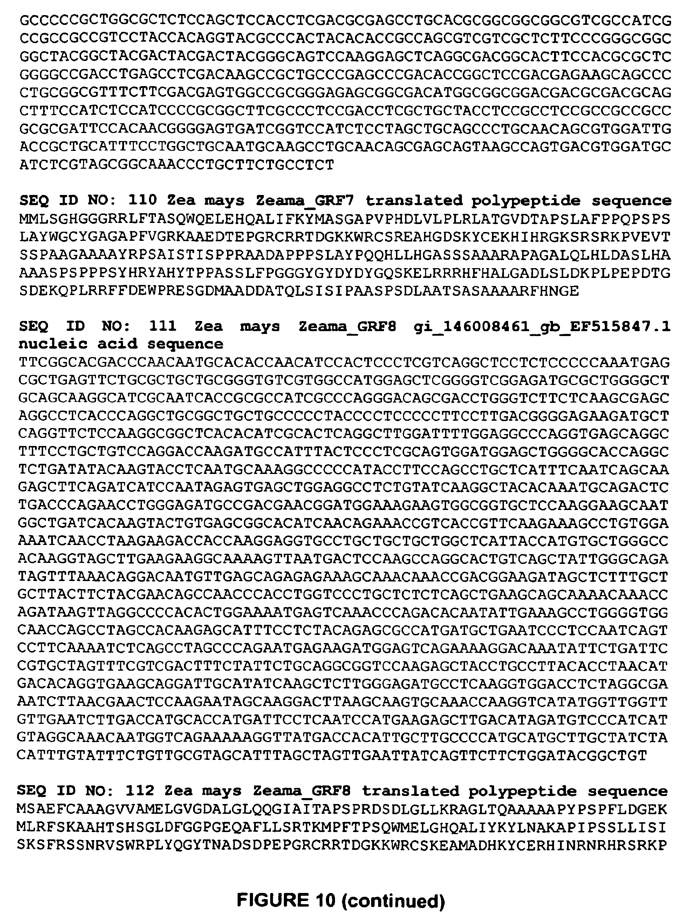

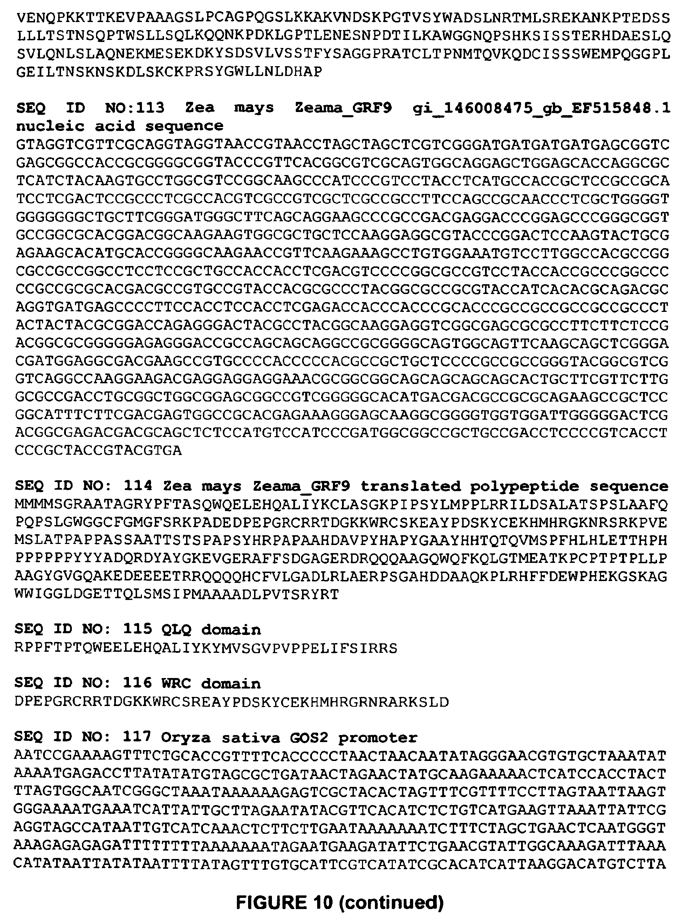

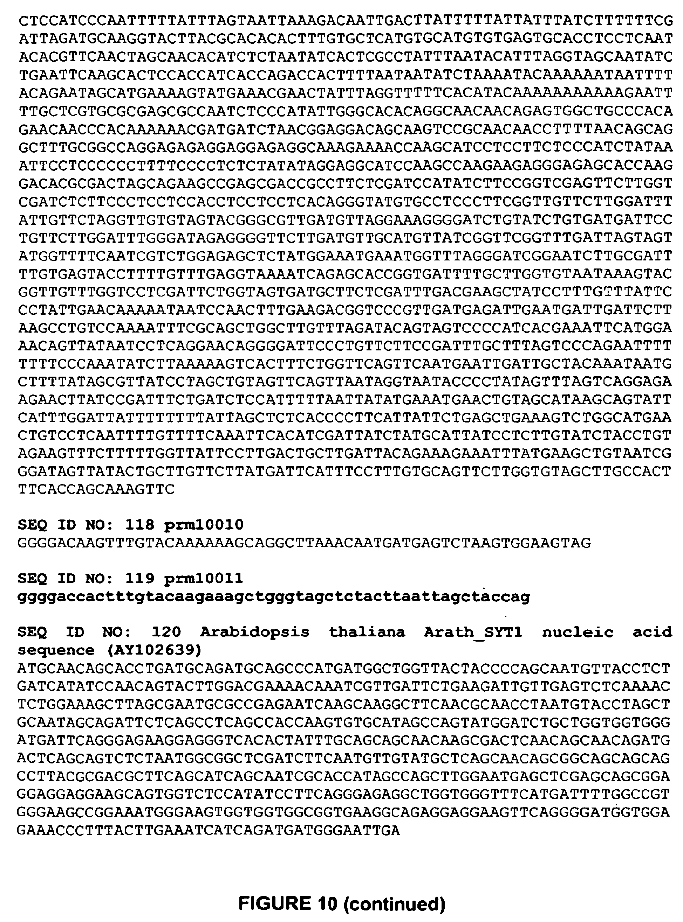

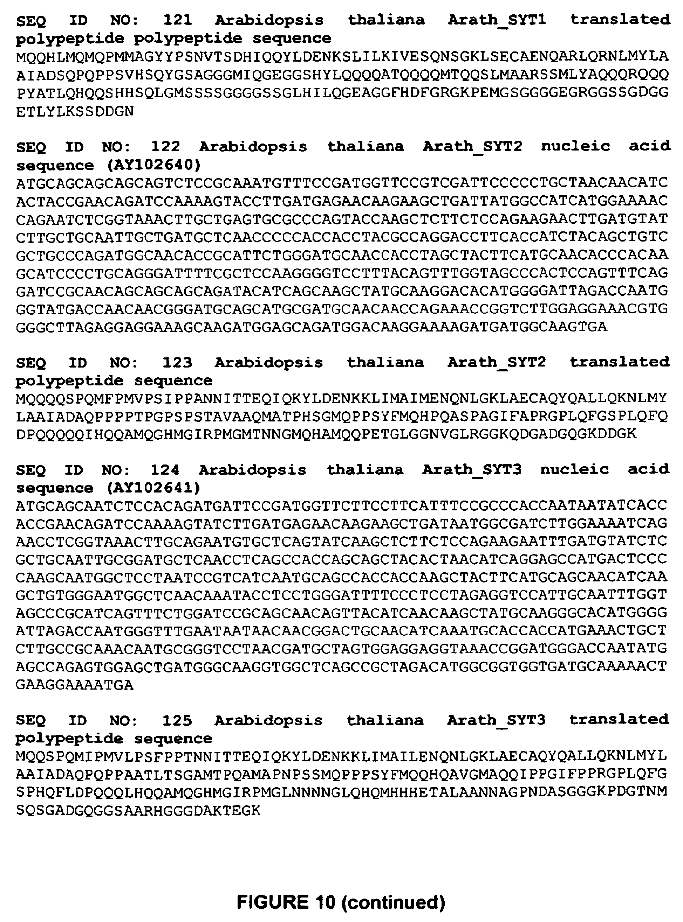

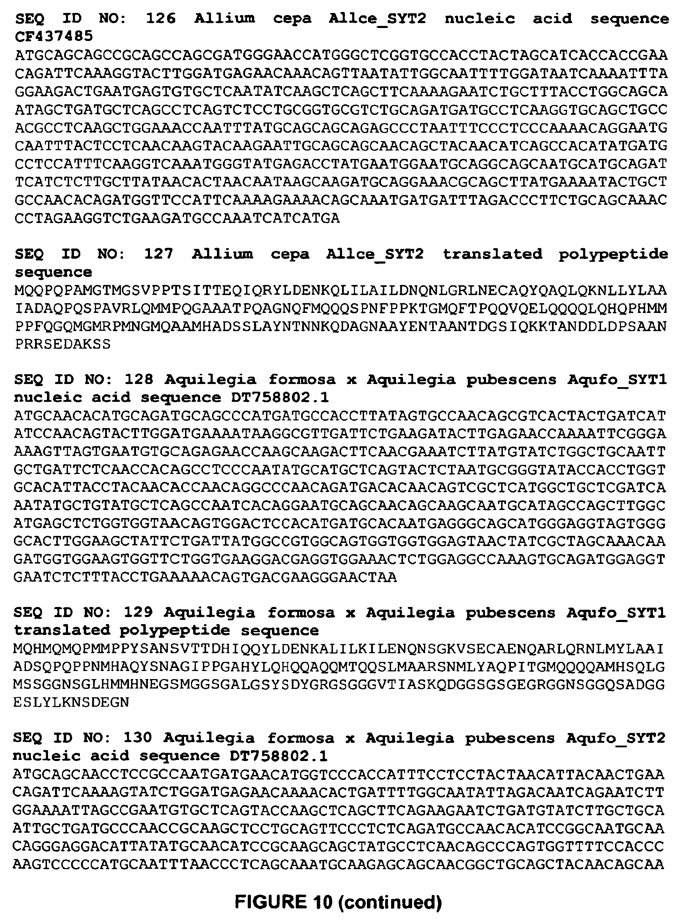

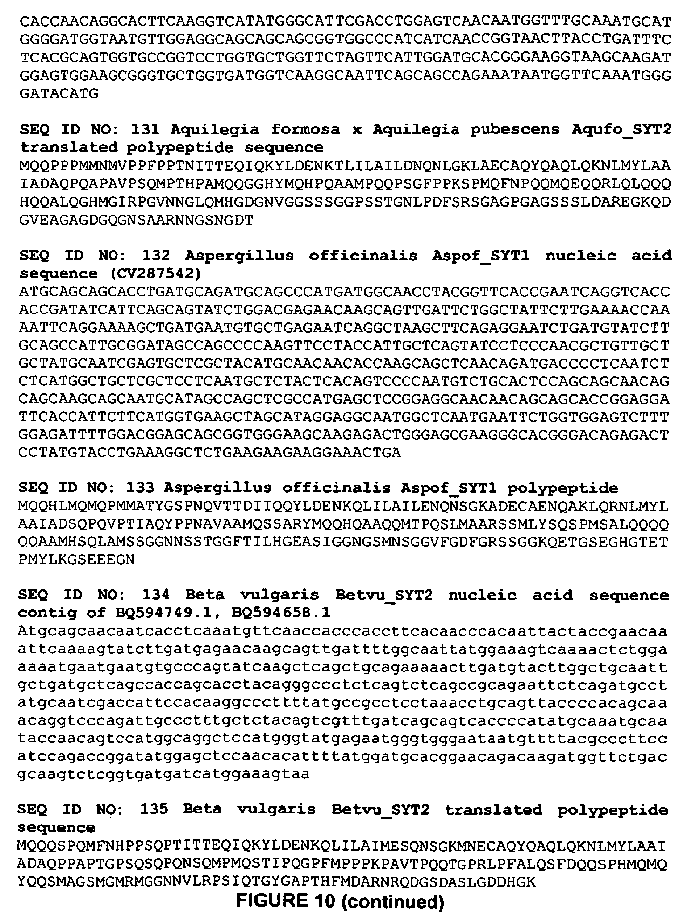

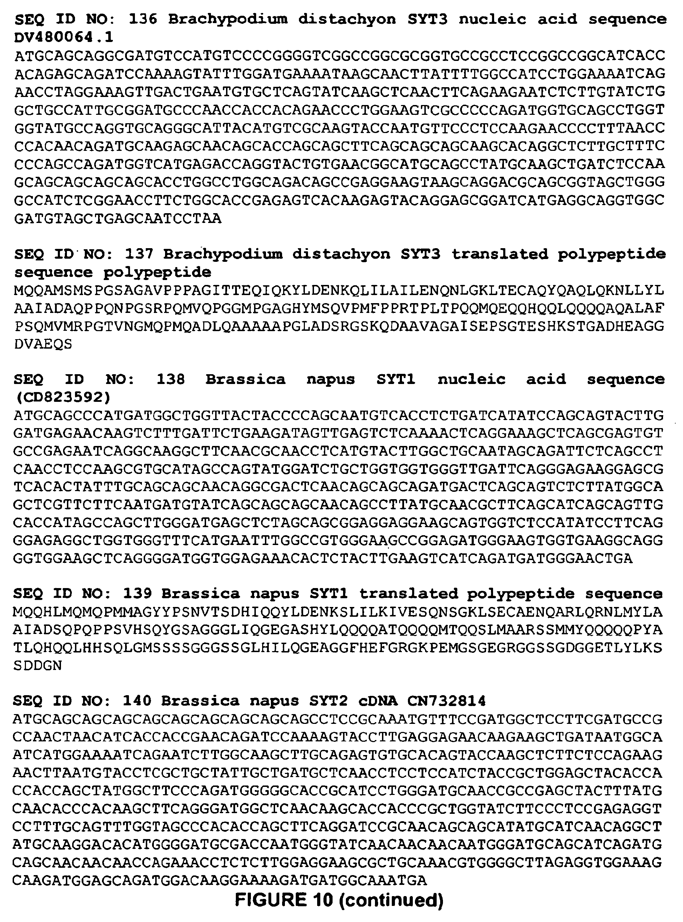

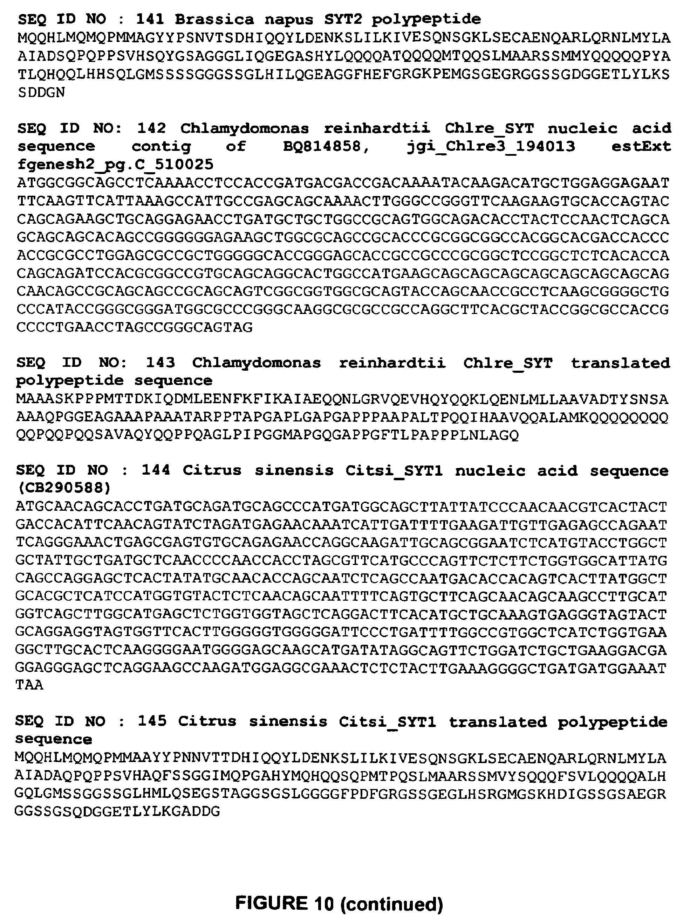

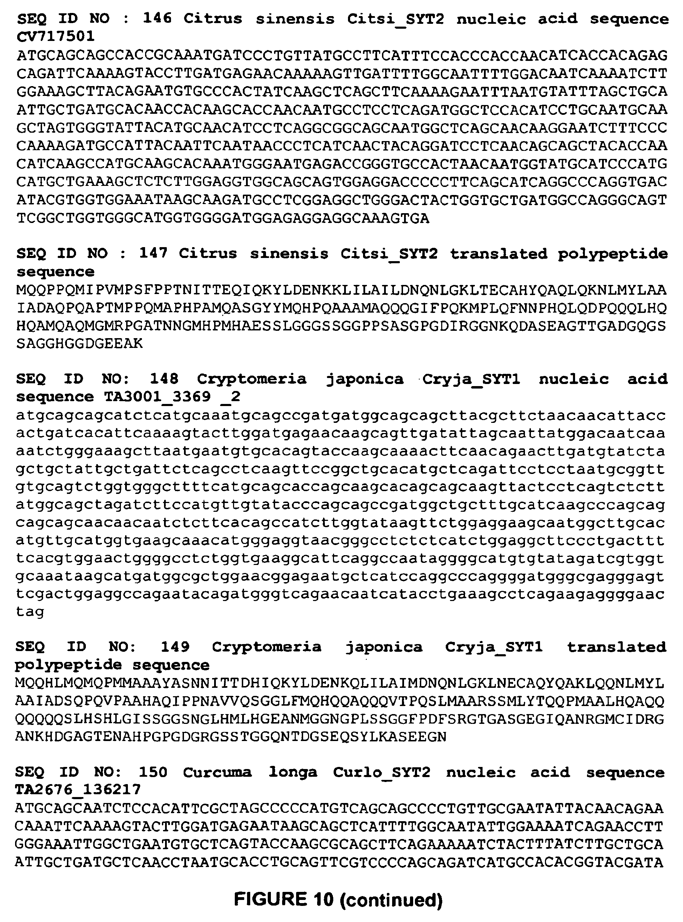

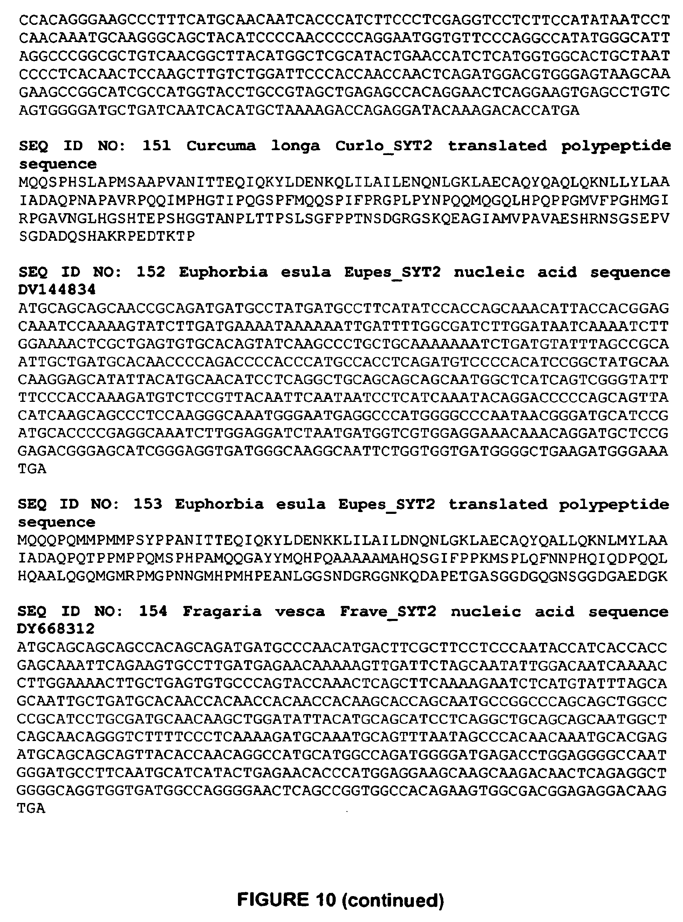

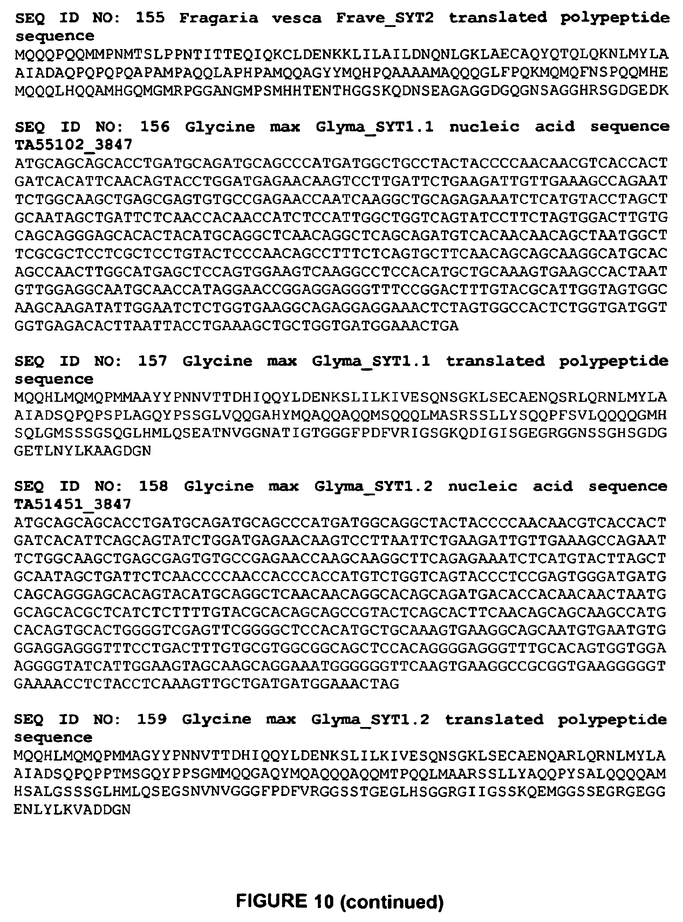

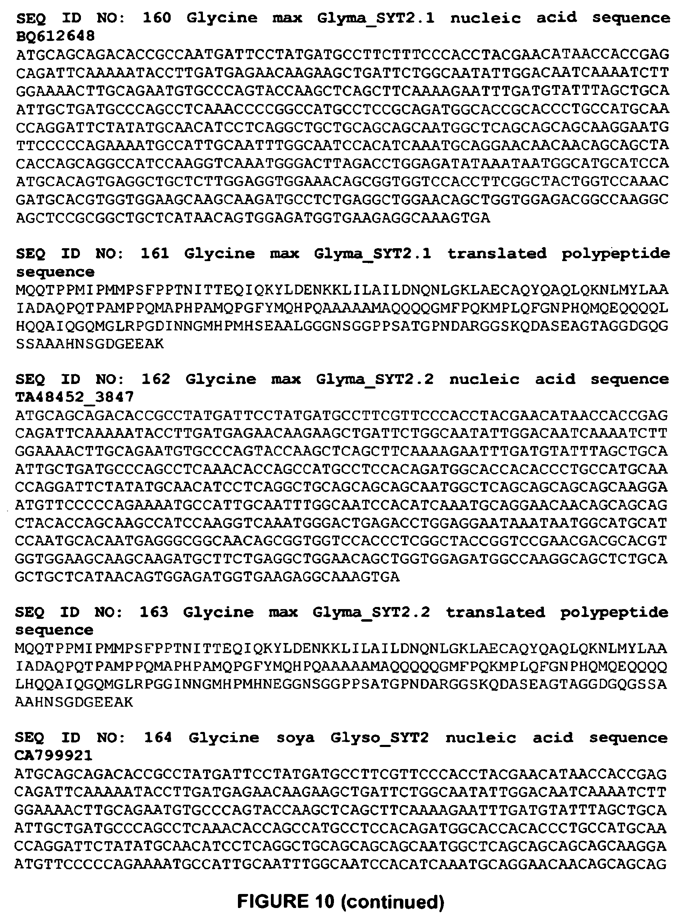

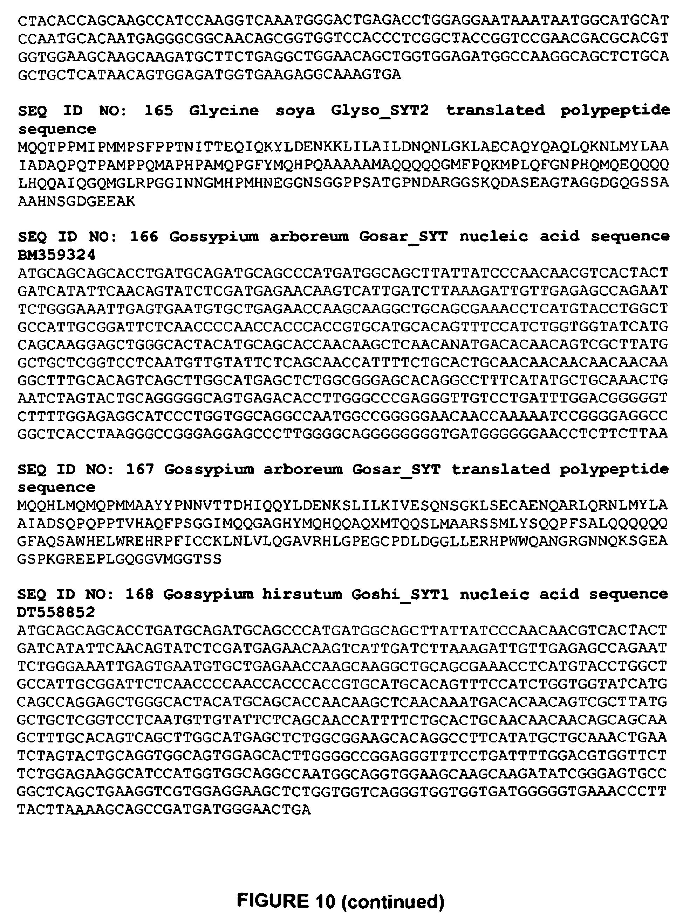

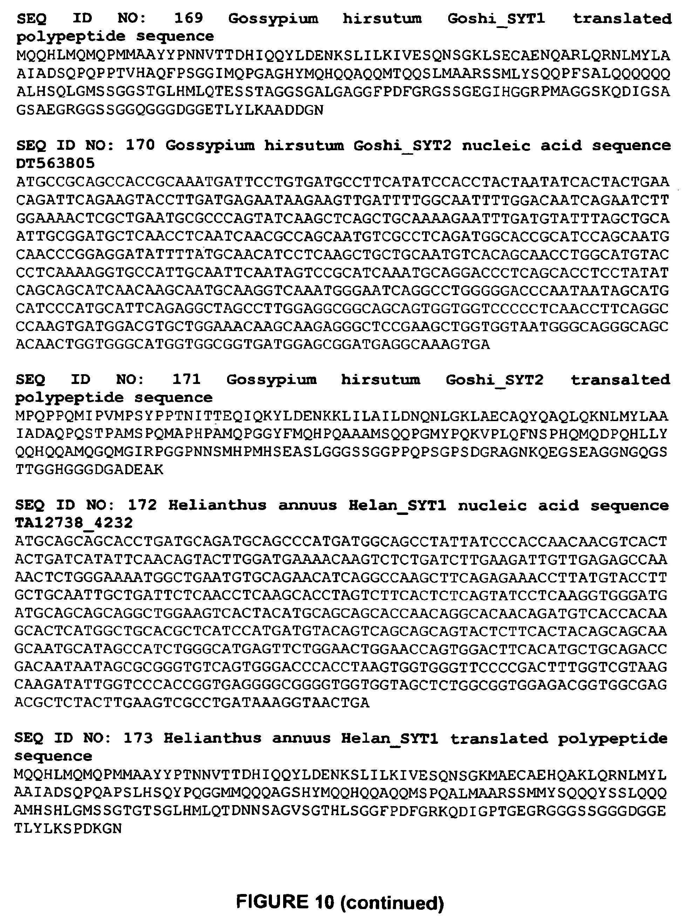

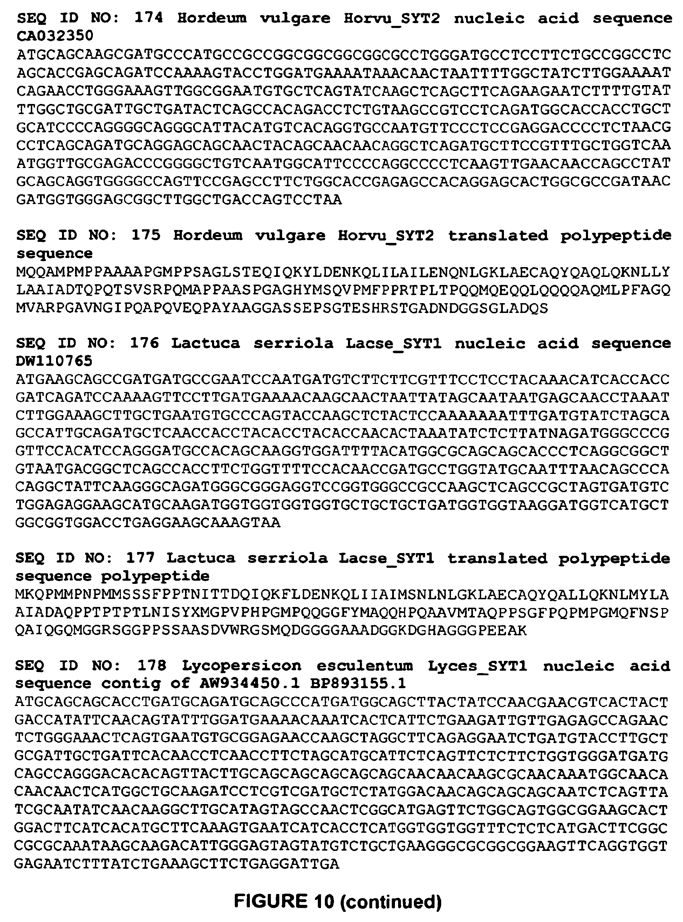

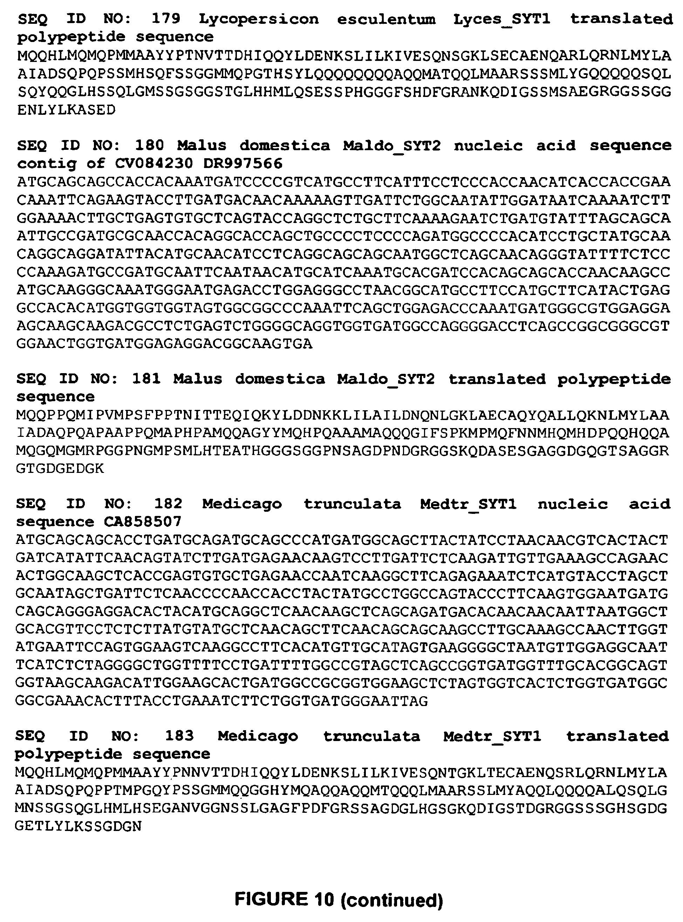

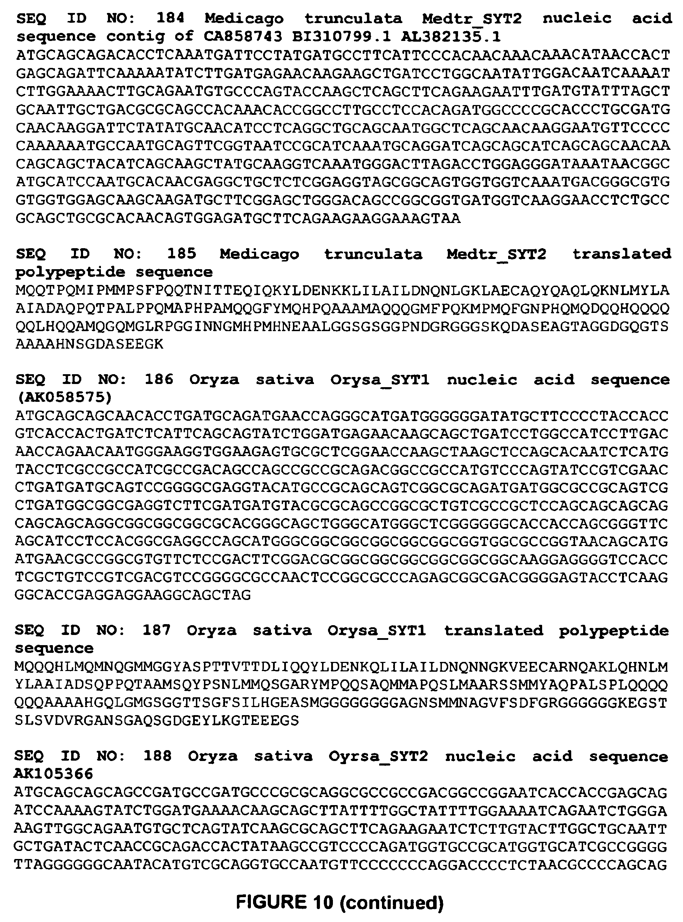

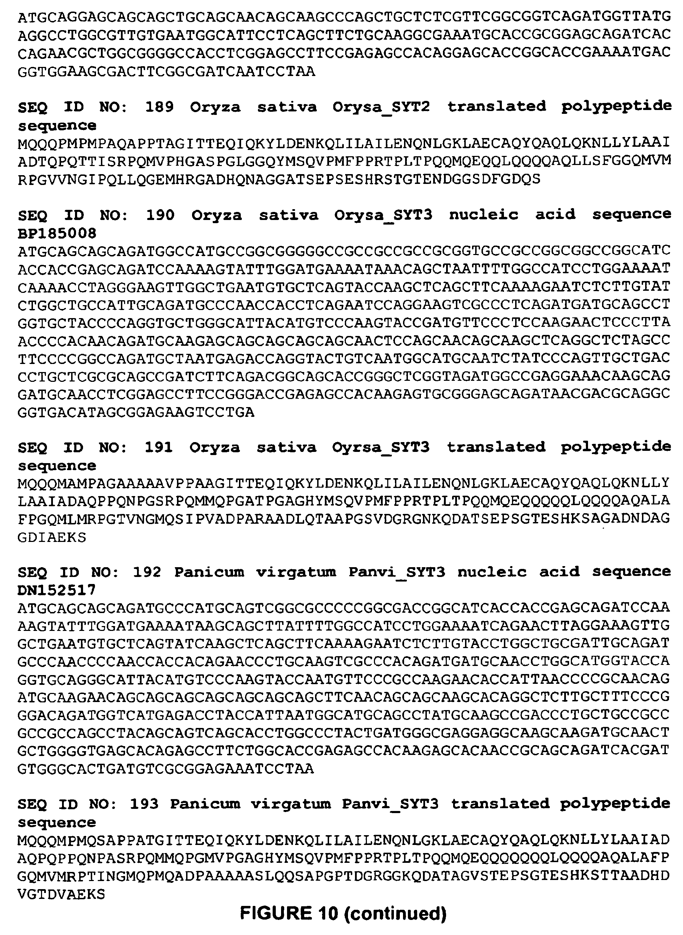

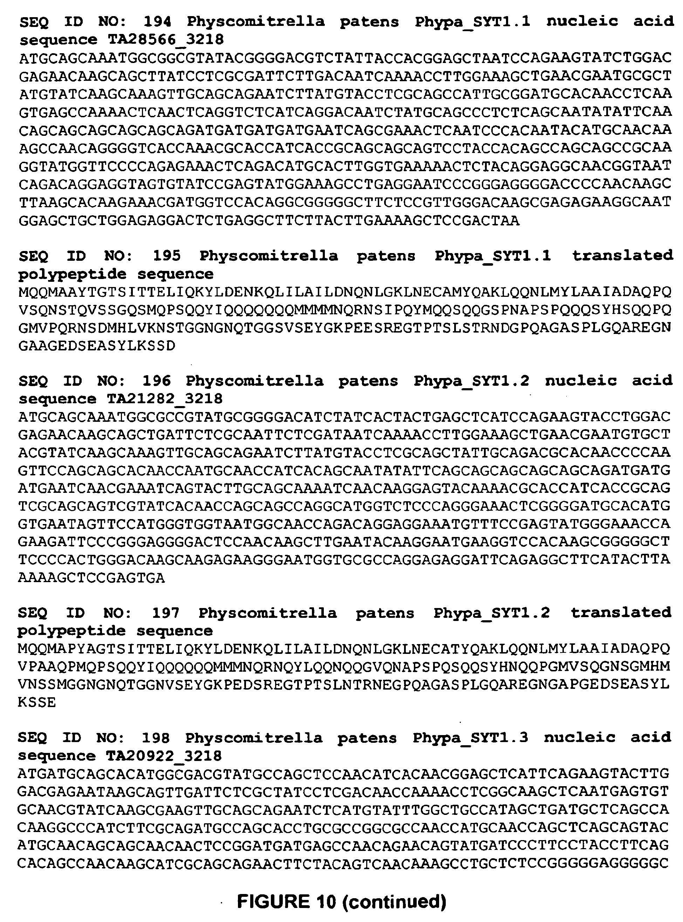

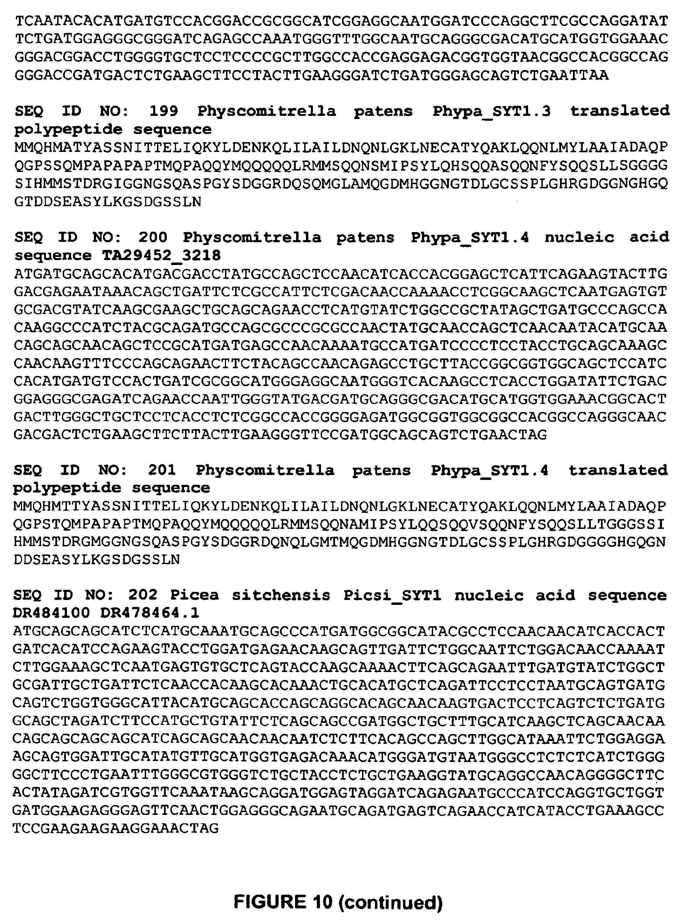

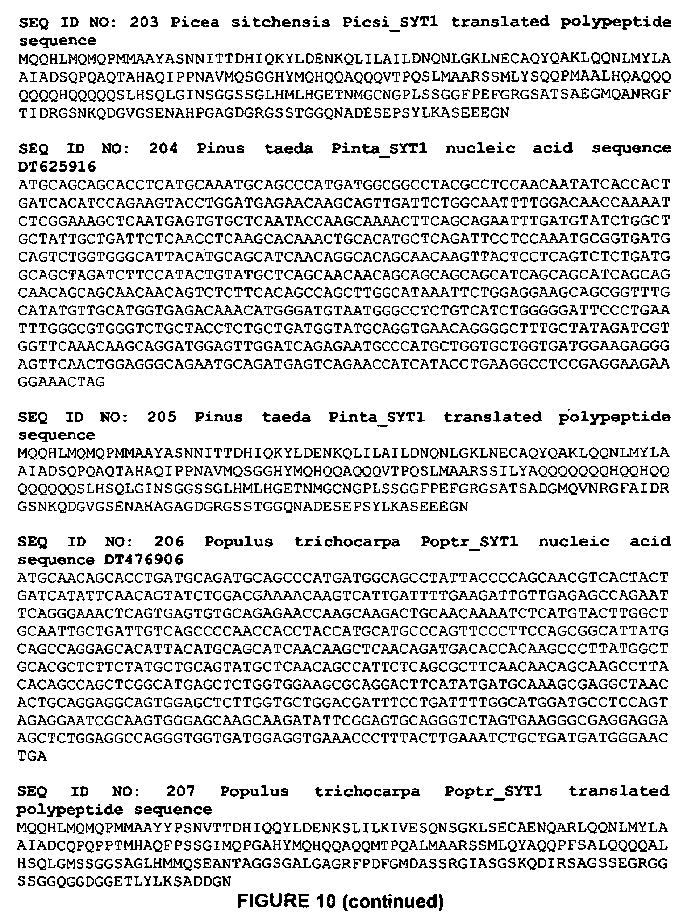

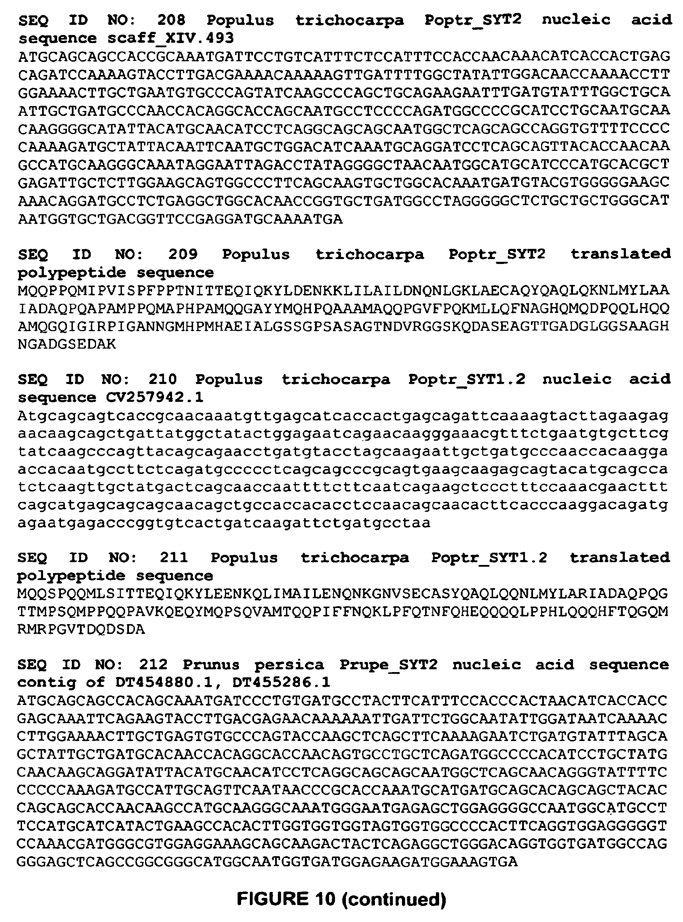

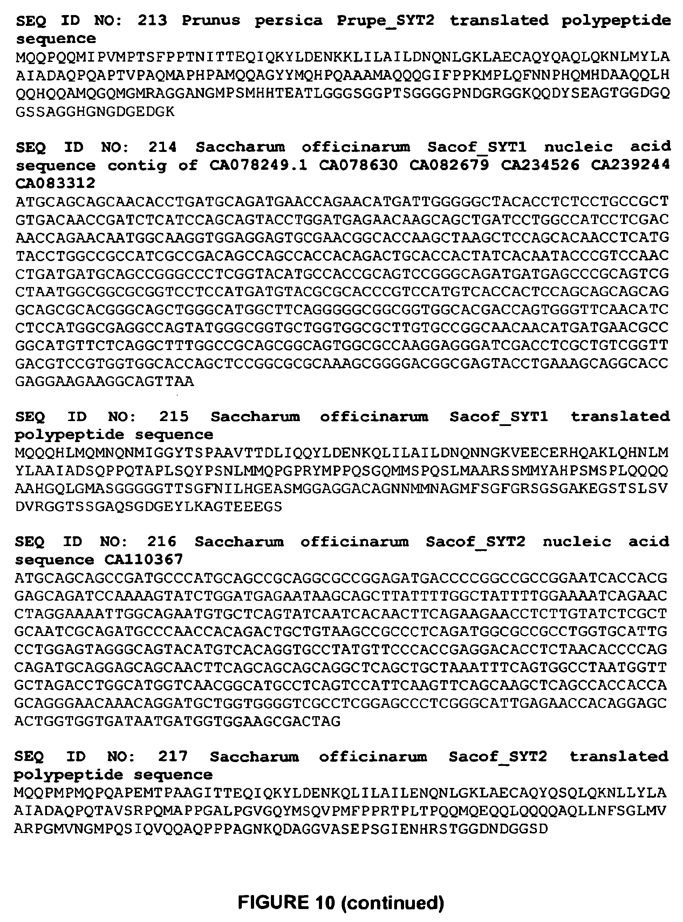

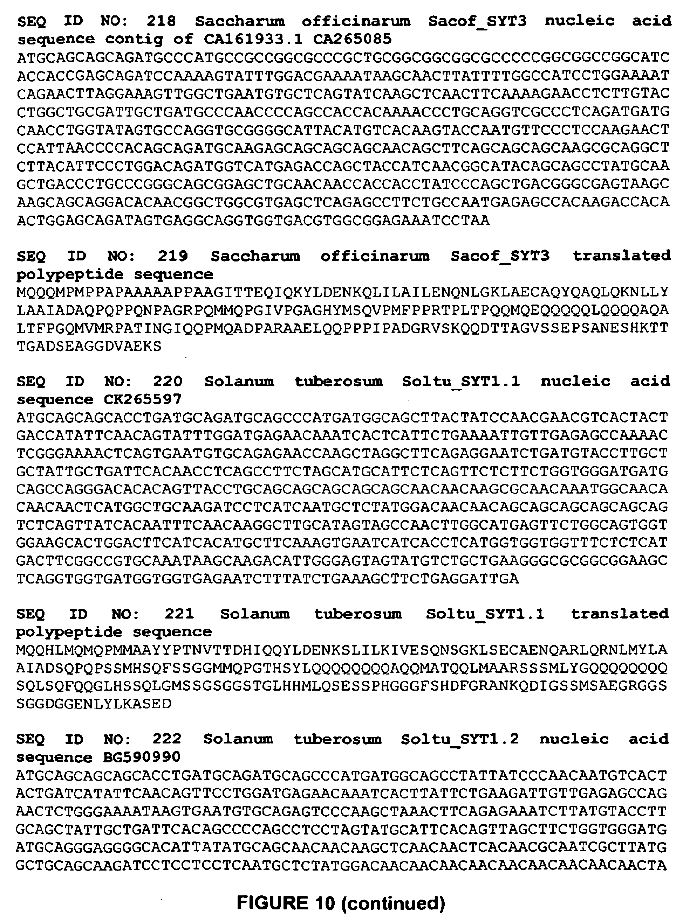

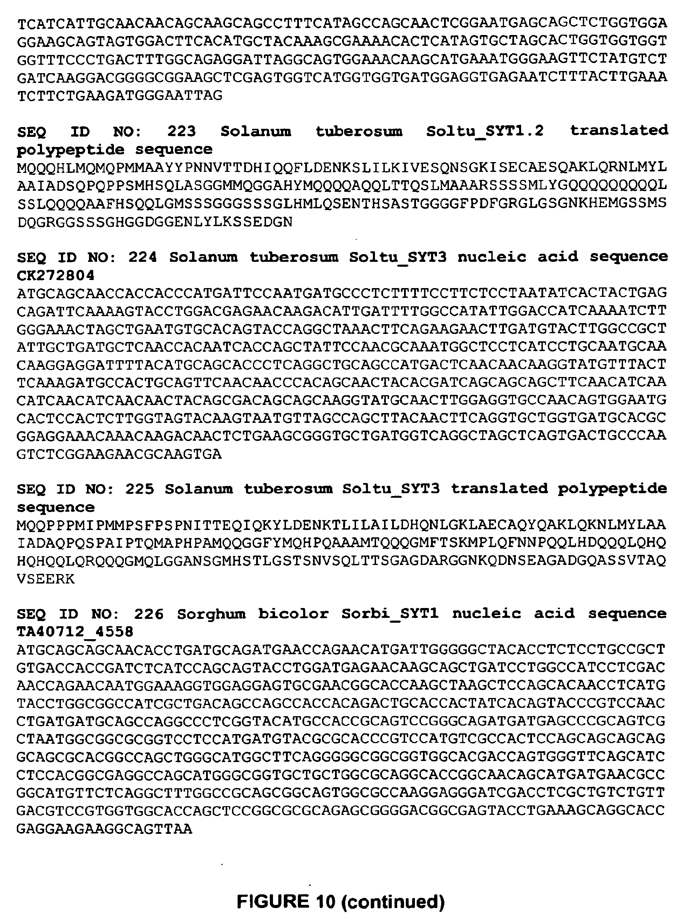

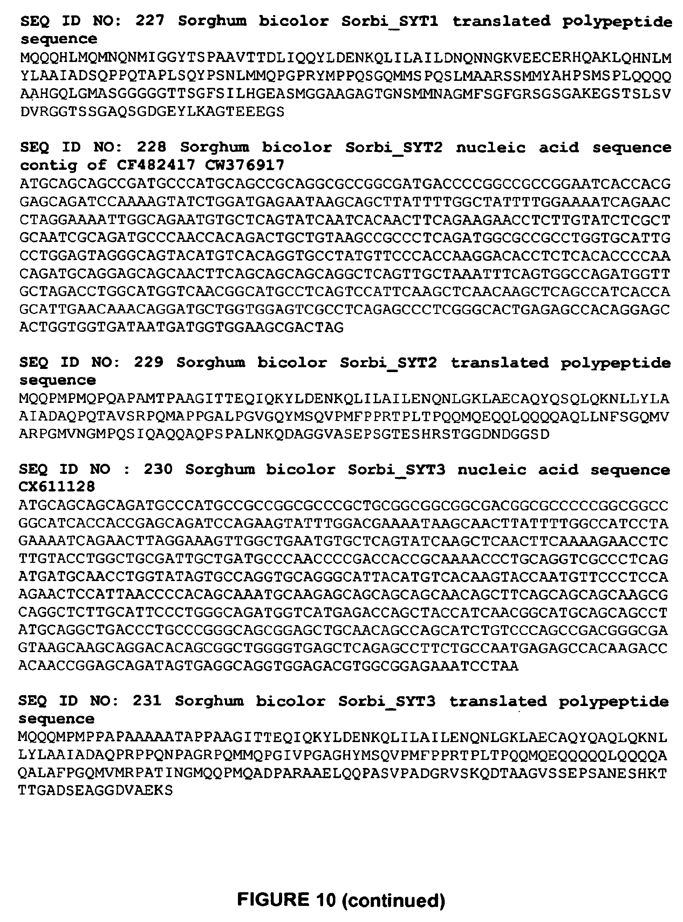

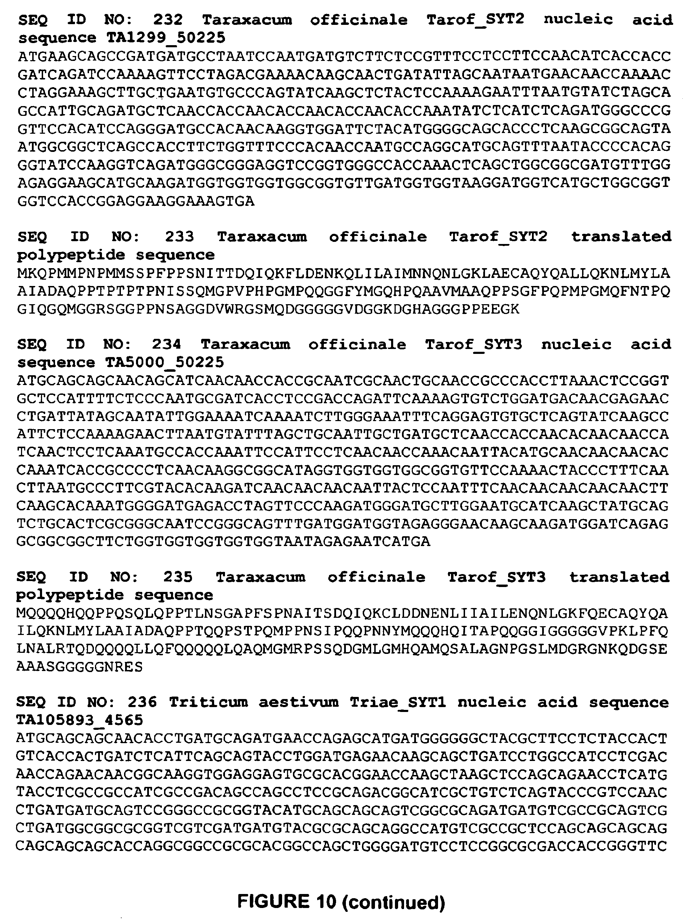

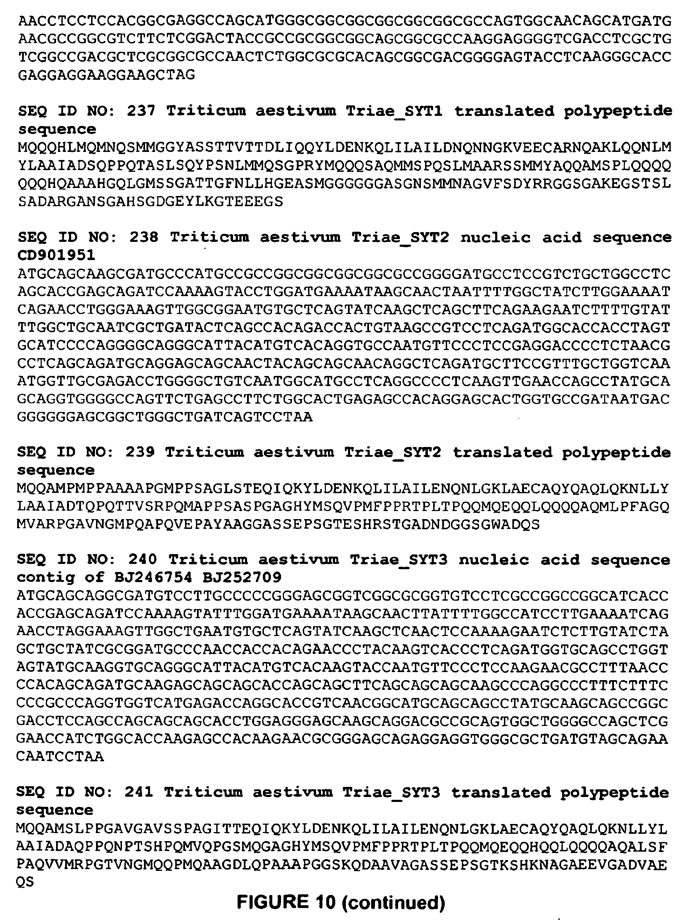

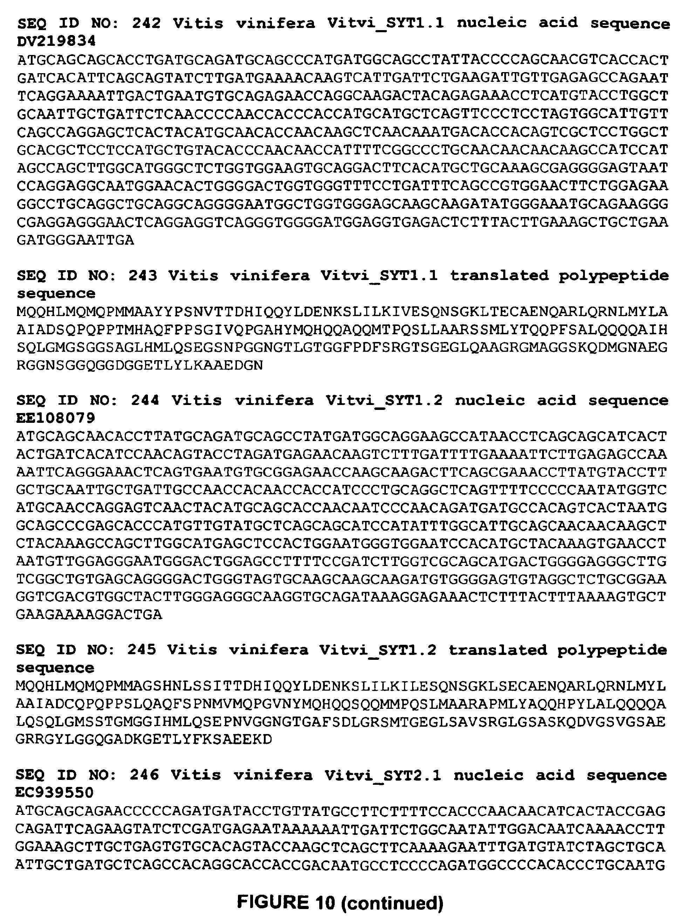

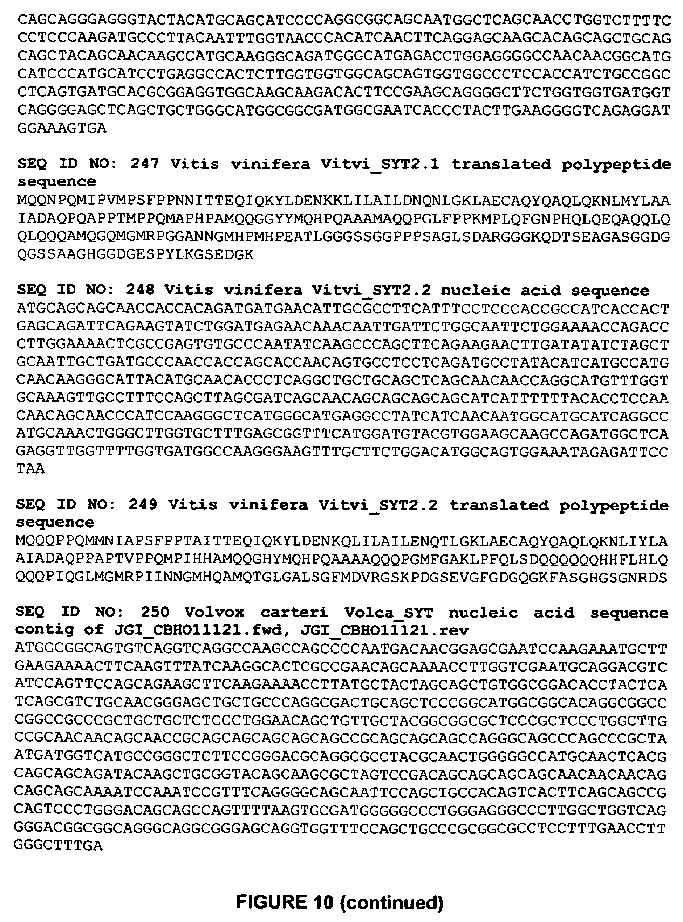

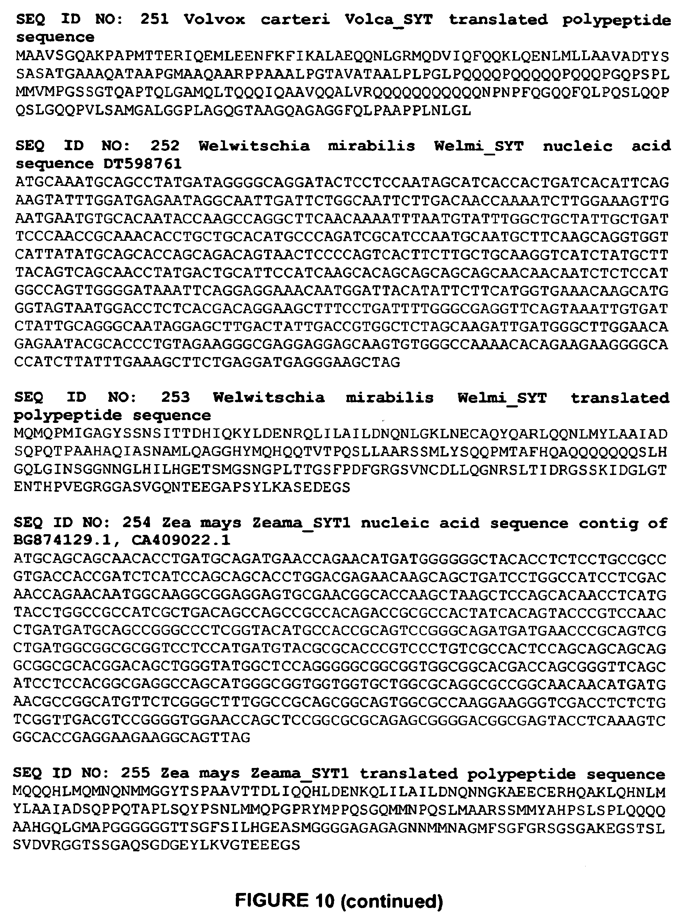

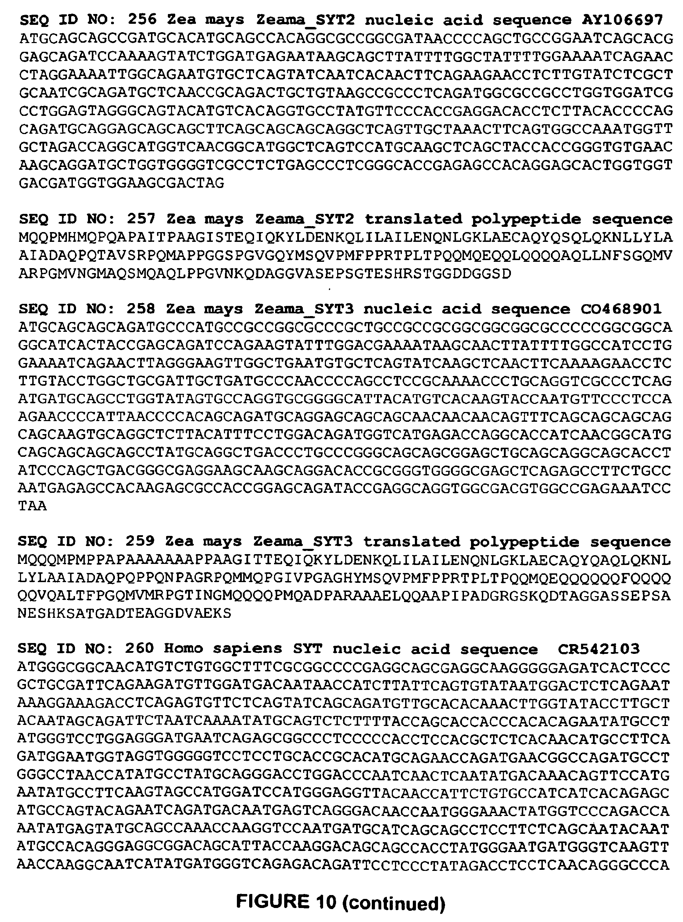

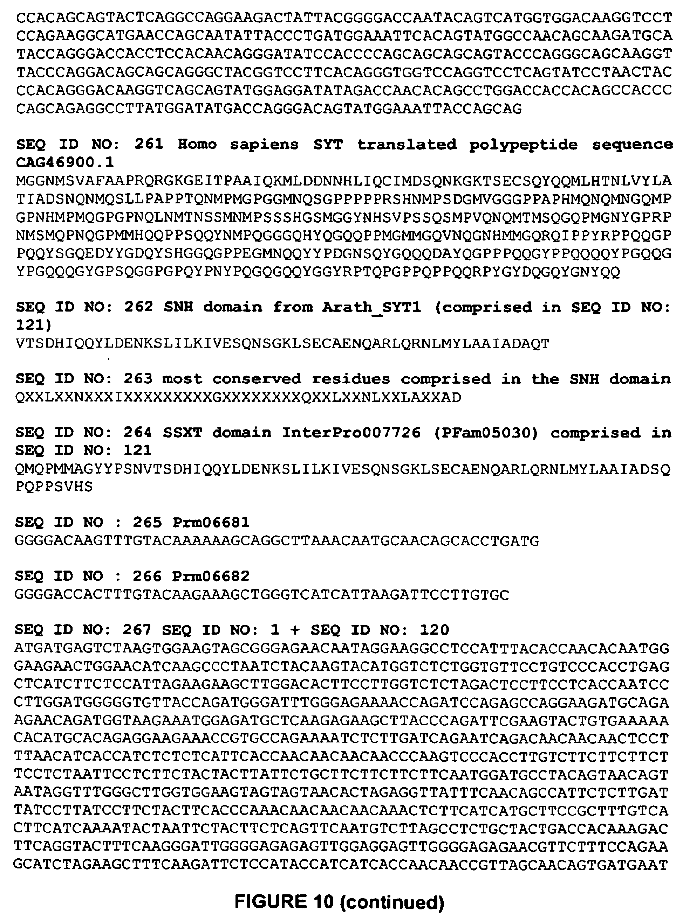

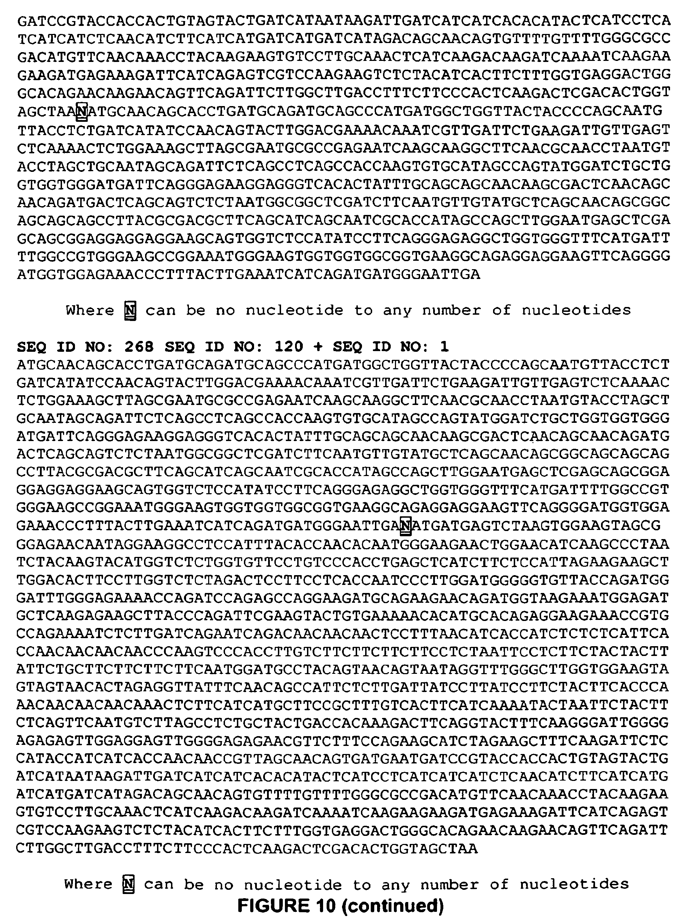

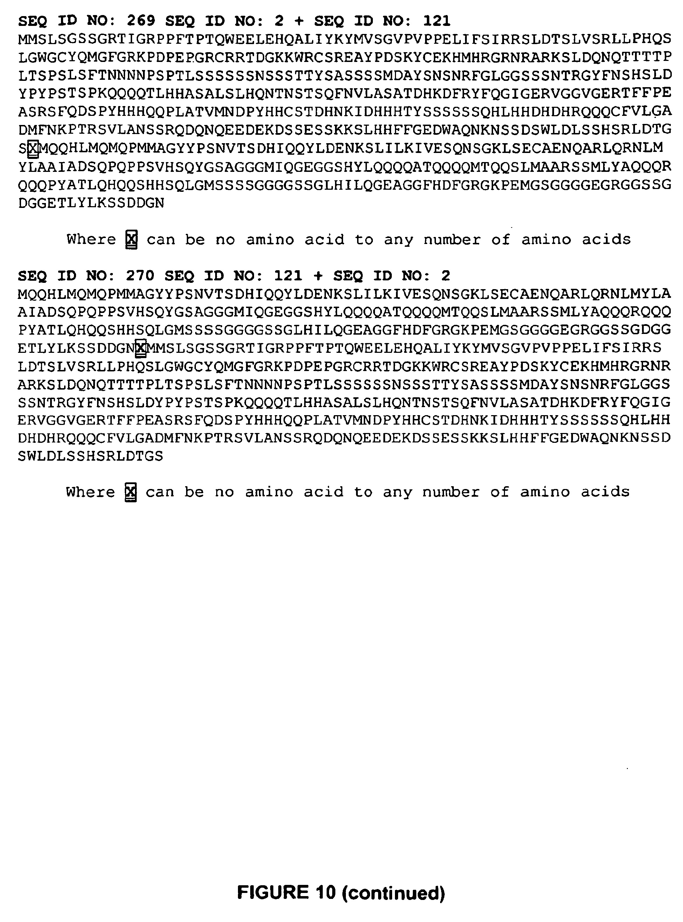

**Figure 10** details examples of sequences useful in performing the methods according to the present invention.

**Examples**

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

***Example 1: Identification of sequences related to the nucleic acid sequence used in the methods of the invention***

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid sequence or polypeptide sequences to sequence databases and by calculating the statistical significance of matches. For example, the polypeptide encoded by the nucleic acid sequence of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid sequence (or polypeptide) sequences over a particular length. In some instances, the default parameters may be adjusted to modify the stringency of the search. For example the E-value may be increased to show less stringent matches. This way, short nearly exact matches may be identified.

Table A.1 provides a list of nucleic acid sequences related encoding GRF polypeptides useful in performing the methods of the present invention. Table A.2 provides a list of nucleic acid sequences related encoding SYT polypeptides useful in performing the methods of the present invention.

**Table A.1:** Examples of GRF polypeptide sequences, and encoding nucleic acid sequences:

| Name | Source organism | Nucleic acid SEQ ID NO: | Polypeptide SEQ ID NO: | Database accession number |
|---|---|---|---|---|
| Arath_GRF_ At3G13960.1 | *Arabidopsis thaliana* | 1 | 2 | AT3G13960.1 |
| Arath_GRF_ At2G06200.1 | *Arabidopsis thaliana* | 3 | 4 | At2G06200.1 |
| Arath_GRF_ At2G22840.1 | *Arabidopsis thaliana* | 5 | 6 | At2G22840.1 |
| Arath_GRF_ At2G36400.1 | *Arabidopsis thaliana* | 7 | 8 | At2G36400.1 |

(continued)

| Name | Source organism | Nucleic acid SEQ ID NO: | Polypeptide SEQ ID NO: | Database accession number |
|---|---|---|---|---|
| Arath_GRF_ At2G45480.1 | *Arabidopsis thaliana* | 9 | 10 | At2G45480.1 |
| Arath_GRF_ At3G52910.1 | *Arabidopsis thaliana* | 11 | 12 | At3G52910.1 |
| Arath_GRF_ At4G24150.1 | *Arabidopsis thaliana* | 13 | 14 | At4G24150.1 |
| Arath_GRF_ At4G37740.1 | *Arabidopsis thaliana* | 15 | 16 | At4G37740.1 |
| Arath_GRF_ At5G53660.1 | *Arabidopsis thaliana* | 17 | 18 | At5G53660.1 |
| Aqufo_GRF | *Aquilegia formosa x Aquilegia pubescens* | 19 | 20 | DT756681.1 DR946716.1 |
| Brana_GRF | *Brassica napus* | 21 | 22 | CN730217.1 ES922527 |
| Horvu_GRF | *Hordeum vulgare* | 23 | 24 | AK250947.1 |
| Lyces_GRF | *Lycopersicon esculentum* | 25 | 26 | BT013977.1 |
| Medtr_GRF | *Medicago truncatula* | 27 | 28 | AC 144645.17 |
| Medtr_GRF like | *Medicago truncatula* | 29 | 30 | AC174350.4 |
| Orysa_GRF_ Os02g47280 .2 | *Oryza sativa* | 31 | 32 | Os02g47280.2 |
| Orysa_GRF_ Os02g53690 .1 | *Oryza sativa* | 33 | 34 | Os02g53690.1 |
| Orysa_GRF_ Os03g51970 .1 | *Oryza sativa* | 35 | 36 | Os03g51970.1 |
| Orysa_GRF_ Os04g48510 .1 | *Oryza sativa* | 37 | 38 | Os04g48510.1 |
| Orysa_GRF_ Os04g51190 .1 | *Oryza sativa* | 39 | 40 | Os04g51190.1 |
| Orysa_GRF_ Os06g02560 .1 | *Oryza sativa* | 41 | 42 | Os06g02560.1 |
| Orysa_GRF_ Os11g35030 .1 | *Oryza sativa* | 43 | 44 | Os11g35030.1 |
| Orysa_GRF_ Os12g29980 .1 | *Oryza sativa* | 45 | 46 | Os12g29980.1 |
| Oyrsa_GRF_ Os03g47140 .1 | *Oryza sativa* | 47 | 48 | Os03g47140.1 |
| Orysa_GRF_gi_ 11544791 0_ref_ NM_001054270.1 | *Oryza sativa* | 49 | 50 | NM_00105427 0.1 |

(continued)

| Name | Source organism | Nucleic acid SEQ ID NO: | Polypeptide SEQ ID NO: | Database accession number |
|---|---|---|---|---|
| Orysa_GRF_gi_ 11546032 5_ref_ NM_001060298.1 | *Oryza sativa* | 51 | 52 | NM_00106029 8.1 |
| Orysa_GRF_gi_ 11547198 4_ref_ NM_001066126.1 | *Oryza sativa* | 53 | 54 | NM_00106612 6.1 |
| Poptr_GRF_lcl_ scaff_XIV. 39 | *Populus tremuloides* | 55 | 56 | lcl_scaff_XIV.3 9 |
| Poptr_GRF_lcl_ scaff_II.10 70 | *Populus tremuloides* | 57 | 58 | lcl_scaff_II.107 0 |
| Poptr_GRF_lcl_ scaff_I.10 18 | *Populus tremuloides* | 59 | 60 | lcl_scaff_I.101 8 |
| Poptr_GRF_lcl_ scaff_28.1 0 | *Populus tremuloides* | 61 | 62 | lcl_scaff_28.10 |
| Poptr_GRF_lcl_ scaff_I.99 5 | *Populus tremuloides* | 63 | 64 | lcl_scaff_I.995 |
| Poptr_GRF_lcl_ scaff_III.7 41 | *Populus tremuloides* | 65 | 66 | lcl_scaff_III.74 1 |
| Poptr_GRF_lcl_ scaff_VII. 1274 | *Populus tremuloides* | 67 | 68 | lcl_scaff_VII.1 274 |
| Poptr_GRF_lcl_ scaff_XII. 277 | *Populus tremuloides* | 69 | 70 | lcl_scaff_XII.2 77 |
| Poptr_GRF_lcl_ scaff_XIII. 769 | *Populus tremuloides* | 71 | 72 | lcl_scaff_XIII.7 69 |
| Poptr_GRF_lcl_ scaff_XIV. 174 | *Populus tremuloides* | 73 | 74 | lcl_scaff_XIV.1 74 |
| Poptr_GRF_lcl_ scaff_XIV. 51 | *Populus tremuloides* | 75 | 76 | lcl_scaff_XIV.5 1 |
| Poptr_GRF_lcl_ scaff_XIX. 480 | *Populus tremuloides* | 77 | 78 | lcl_scaff_XIX.4 80 |
| Poptr_GRF_lcl_ scaff_28.3 09 | *Populus tremuloides* | 79 | 80 | lcl_scaff_28.30 9 |
| Poptr_GRF_lcl_ scaff_I.68 8 | *Populus tremuloides* | 81 | 82 | lcl_scaff_I.688 |
| Sacof_GRF | *Saccharum officinarum* | 83 | 84 | CA084837.1 CA238919.1 CA122516.1 |
| Vitvi_GRF | *Vitis vinifera* | 85 | 86 | AM468035 |
| Zeama_ GRF10_gi_14600 8494_gb_ EF515849.1 | *Zea mays* | 87 | 88 | EF515849.1 |

(continued)

| Name | Source organism | Nucleic acid SEQ ID NO: | Polypeptide SEQ ID NO: | Database accession number |
|---|---|---|---|---|
| Zeama_ GRF11_gi_14600 8515_gb_ EF515850.1 | *Zea mays* | 89 | 90 | EF515850.1 |
| Zeama_ GRF12_gi_14600 8534_gb_ EF515851.1 | *Zea mays* | 91 | 92 | EF515851.1 |
| Zeama_ GRF13_gi_14600 8539_gb_ EF515852.1 | *Zea mays* | 93 | 94 | EF515852.1 |
| Zeama_ GRF14_gi_14600 8560_gb_ EF515853.1 | *Zea mays* | 95 | 96 | EF515853.1 |
| Zeama_GRF1_gi_ 146008 330_gb_ EF515840.1 | *Zea mays* | 97 | 98 | EF515840.1 |
| Zeama_GRF2_gi_ 146008 352_gb_ EF515841.1 | *Zea mays* | 99 | 100 | EF515841.1 |
| Zeama_GRF3_gi_ 146008 368_gb_ EF515842.1 | *Zea mays* | 101 | 102 | EF515842.1 |
| Zeama_GRF4_gi_ 146008 393_gb_ EF515843.1 | *Zea mays* | 103 | 104 | EF515843.1 |
| Zeama_GRF5_gi_ 146008 412_gb_ EF515844.1 | *Zea mays* | 105 | 106 | EF515844.1 |
| Zeama_GRF6_gi_ 146008 429_gb_ EF515845.1 | *Zea mays* | 107 | 108 | EF515845.1 |
| Zeama_GRF7_gi_ 146008 440_gb_ EF515846.1 | *Zea mays* | 109 | 110 | EF515846.1 |
| Zeama_GRF8_gi_ 146008 461_gb_ EF515847.1 | *Zea mays* | 111 | 112 | EF515847.1 |
| Zeama_GRF9_gi_ 146008 475_gb_ EF515848.1 | *Zea mays* | 113 | 114 | EF515848.1 |

**Table A2:** Examples of SYT polypeptide sequences, and encoding nucleic acid sequences:

| Name | Source organism | Nucleic acid sequence SEQ ID NO | Translated polypeptide sequence SEQ ID NO | Database accession number |
|---|---|---|---|---|
| Arath_SYT1 | *Arabidopsis thaliana* | 120 | 121 | AY102639.1 |
| Arath_SYT2 | *Arabidopsis thaliana* | 122 | 123 | AY102640.1 |
| Arath_SYT3 | *Arabidopsis thaliana* | 124 | 125 | AY102641.1 |
| Allce_SYT2 | *Allium cepa* | 126 | 127 | CF437485 |
| Aqufo_SYT1 | *Aquilegia formosa x Aquilegia pubescens* | 128 | 129 | DT758802.1 |
| Aqufo_SYT2 | *Aquilegia formosa x Aquilegia pubescens* | 130 | 131 | TA15831_33861 8 T25K16.15 |
| Aspof_SYT1 | *Aspergillus officinalis* | 132 | 133 | CV287542 |
| Betvu_SYT2 | *Beta vulgaris* | 134 | 135 | BQ594749.1 BQ594658.1 |
| Bradi_SYT3 | *Brachypodium distachyon* | 136 | 137 | DV480064.1 |
| Brana_SYT1 | *Brassica napus* | 138 | 139 | CD823592 |
| Brana_SYT2 | *Brassica napa* | 140 | 141 | CN732814 |
| Chlre_SYT | *Chlamydomonas reinhardtii* | 142 | 143 | BQ814858, jgi_ Chlre3_1940 13_estExt_fgene sh2_pg.C_5100 25 |
| Citsi_SYT1 | *Citrus sinensis* | 144 | 145 | CB290588 |
| Citsi_SYT2 | *Citrus sinensis* | 146 | 147 | CV717501 |
| Cryja_SYT1 | *Cryptomeria japonica* | 148 | 149 | TA3001_3369 _2 |
| Curlo_SYT2 | *Curcuma longa* | 150 | 151 | TA2676_136217 |
| Eupes_SYT2 | *Euphoria esula* | 152 | 153 | DV144834 |
| Frave_SYT2 | *Fragaria vesca* | 154 | 155 | DY668312 |
| Glyma-SYT1.1 | *Glycine max* | 156 | 157 | TA55102_3847 |
| Glyma_SYT1.2 | *Glycine max* | 158 | 159 | TA51451_3847 |
| Glyma_SYT2.1 | *Glycine max* | 160 | 161 | BQ612648 |
| Glyma_SYT2.2 | *Glycine max* | 162 | 163 | TA48452_3847 |
| Glyso_SYT2 | *Glycine soya* | 164 | 165 | CA799921 |
| Gosar_SYT1 | *Gossypium arboreum* | 166 | 167 | BM359324 |
| Goshi_SYT1 | *Gossypium hirsutum* | 168 | 169 | DT558852 |
| Goshi_SYT2 | *Gossypium hirsutum* | 170 | 171 | DT563805 |
| Helan_SYT1 | *Helianthus annuus* | 172 | 173 | TA12738_4232 |
| Horvu_SYT2 | *Hordeum vulgare* | 174 | 175 | CA032350 |
| Lacse_SYT2 | *Lactuca serriola* | 176 | 177 | DW110765 |

(continued)

| Name | Source organism | Nucleic acid sequence SEQ ID NO | Translated polypeptide sequence SEQ ID NO | Database accession number |
|---|---|---|---|---|
| Lyces_SYT1 | *Lycopersicon esculentum* | 178 | 179 | AW934450.1 BP893155.1 |
| Maldo_SYT2 | *Malus domestica* | 180 | 181 | CV084230 DR997566 |
| Medtr_SYT1 | *Medicago truncula*ta | 182 | 183 | CA858507.1 |
| Medtr_SYT2 | *Medicago truncul*ata | 184 | 185 | CA858743 B1310799.1 AL382135.1 |
| Orysa_SYT1 | *Oryza sativa* | 186 | 187 | AK058575 |
| Orysa_SYT2 | *Oryza sativa* | 188 | 189 | AK105366 |
| Orysa_SYT3 | *Oryza sativa* | 190 | 191 | BP185008 |
| Panvi_SYT3 | *Panicum virgatum* | 192 | 193 | DN152517 |
| Phypa_SYT1.1 | *Physcomitrella patens* | 194 | 195 | TA28566_3218 |
| Phypa_SYT1.2 | *Physcomitrella patens* | 196 | 197 | TA21282_3218 |
| Phypa_SYT1.3 | *Physcomitrella patens* | 198 | 199 | TA20922_3218 |
| Phypa_SYT1.4 | *Physcomitrella patens* | 200 | 201 | TA29452_3218 |
| Picsi_SYT1 | *Picea sitcherisis* | 202 | 203 | DR484100 DR478464.1 |
| Pinta_SYT1 | *Pinus taeda* | 204 | 205 | DT625916 |
| Poptr_SYT1 | *Populus trichocarpa* | 206 | 207 | DT476906 |
| Poptr_SYT2 | *Populus trichocarpa* | 208 | 209 | scaff_XIV.493 |
| Poptr_SYT1.2 | *Populus trichocarpa* | 210 | 211 | CV257942.1 |
| Prupe_SYT2 | *Prunus persica* | 212 | 213 | DT454880.1 DT455286.1 |
| Sacof_SYT1 | *Saccharum officinarum* | 214 | 215 | CA078249.1 CA078630 CA082679 CA234526 CA239244 CA083312 |
| Sacof_SYT2 | *Saccharum officinarum* | 216 | 217 | CA110367 |
| Sacof_SYT3 | *Saccharum officinarum* | 218 | 219 | CA161933.1 CA265085 |
| Soltu_SYT1.1 | *Solanum tuberosum* | 220 | 221 | CK265597 |
| Soltu_SYT1.2 | *Solanum tuberosum* | 222 | 223 | BG590990 |

(continued)

| Name | Source organism | Nucleic acid sequence SEQ ID NO | Translated polypeptide sequence SEQ ID NO | Database accession number |
|---|---|---|---|---|
| Soltu_SYT3 | *Solanum tuberosum* | 224 | 225 | CK272804 |
| Sorbi_SYT1 | *Sorghum bicolor* | 226 | 227 | TA40712_4558 |
| Sorbi_SYT2 | *Sorghum bicolor* | 228 | 229 | CF482417 CW376917 |
| Sorbi_SYT3 | *Sorghum bicolor* | 230 | 231 | CX611128 |
| Taxof_SYT2 | *Taraxacum officinale* | 232 | 233 | TA1299_50225 |
| Taxof_SYT3 | *Taraxacum officinale* | 234 | 235 | TA5000_50225 |
| Triae_SYT1 | *Triticum aestivum* | 236 | 237 | TA105893_4565 |
| Triae_SYT2 | *Triticum aestivum* | 238 | 239 | CD901951 |
| Triae_SYT3 | *Triticum aestivum* | 240 | 241 | BJ246754 BJ252709 |
| Vitvi_SYT1.1 | *Vitis vinifera* | 242 | 243 | DV219834 |
| Vitvi_SYT1.2 | *Vitis vinifera* | 244 | 245 | EE108079 |
| Vitvi_SYT2.1 | *Vitis vinifera* | 246 | 247 | EC939550 |
| Vitvi_SYT2.2 | *Vitis vinifera* | 248 | 249 | EE094148.1 EC964169.1 |
| Volca_SYT | *Volvox carteri* | 250 | 251 | JGI_CBHO1112 1.fwdJGI_CBHO 11121.rev |
| Welmi_SYT | *Welwitschia mirabilis* | 252 | 253 | DT598761 |
| Zeama_SYT1 | *Zea mays* | 254 | 255 | BG874129.1 CA409022.1 |
| Zeama_SYT2 | *Zea mays* | 256 | 257 | AY106697 |
| Zeama_SYT3 | *Zea mays* | 258 | 259 | CO468901 |
| Homsa_SYT | *Homo sapiens* | 260 | 261 | CR542103 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute, for example for poplar and *Ostreococcus tauri.*

***Example 2: Alignment of polypeptide sequences useful in performing the methods of the invention***

*Alignment of GRF polypeptide sequences*

Mutliple sequence alignment of all the GRF polypeptide sequences in Table A.1 was performed using the AlignX algorithm (from Vector NTI 10.3, Invitrogen Corporation). Results of the alignment for the QLQ domain of GRF polypeptides from Table A.1 (as represented by SEQ ID NO: 115 for SEQ ID NO: 2) are shown in Figure 2 of the present application. The conserved QLQ amino acid residues are located on the top of the multiple alignement. Two other very conserved residues (boxed in black) are E (Glu) and P (Pro). Results of the alignment for the WRC domain of the GRF polypeptides from Table A.1 (as represented by SEQ ID NO: 116 for SEQ ID NO: 2) are shown in Figure 3 of the present application. The conserved WRC amino acid residues are in bold in the consensus sequence. The Effector of Transcription (ET) domain, comprising three Cys and one His residues in a conserved spacing ($CX_9CX_{10}CX_2H$), is identified at the bottom of the alignment.

*Alignment of SYT polypeptide sequences*

Mutliple sequence alignment of all the SYT polypeptide sequences in Table A.2 was performed using the AlignX algorithm (based on a modified ClustalW algorithm; from Vector NTI 10.3, Invitrogen Corporation) with default settings for gap opening penalty of 10 and a gap extension of 0.05), and is shown in Figure 6, The two main domains, from N-terminal to C-terminal, are boxed and identified as SNH domain and the Met-rich/QG-rich domain. Additionally, the N-terminal Met-rich domain is also boxed.

Results of the alignment for the SNH domain of SYT polypeptides from Table A.2 (as represented by SEQ ID NO: 115 for SEQ ID NO: 2) are shown in Figure 5 of the present application. The most conserved amino acid residues within the SNH domain, as represented by SEQ ID NO: 263, are boxed in black.

***Example 3: Calculation of global percentage identity between polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62
First Gap: 12
Extending gap: 2

Results of the software analysis are shown in Table B.1 for the global similarity and identity over the full length of the GRF polypeptide sequences (excluding the partial polypeptide sequences), and in Table B.2 for the global similarity and identity over the full length of the SYT polypeptide sequences.

The percentage identity between the full length GRF polypeptide sequences useful in performing the methods of the invention can be as low as 15 % amino acid identity compared to SEQ ID NO: 2.

The percentage identity can be substantially increased if the identity calculation is performed between the QLQ domain SEQ ID NO: 2 (as represented by SEQ ID NO: 115 comprised in SEQ ID NO: 2; QLQ domain of the GRF polypeptides of Table A.1 represented in Figure 2) and the QLQ domains of the polypeptides useful in performing the invention. Similarly, the percentage identity can be substantially increased if the identity calculation is performed between the WRC domain SEQ ID NO: 2 (as represented by SEQ ID NO: 116 comprised in SEQ ID NO: 2; WRC domain of the GRF polypeptides of Table A.1 represented in Figure 3) and the WRC domains of the polypeptides useful in performing the invention. Percentage identity over the QLQ domain amongst the polypeptide sequences useful in performing the methods of the invention ranges between 25 % and 99% amino acid identity, and percentage identity over the WRC domain amongst the polypeptide sequences useful in performing the methods of the invention ranges between 60 % and 99% amino acid identity. As can also be observed in Figure 3, the WRC domain is better conserved amongst the different GRF polypeptides than the QLQ domain, as shown in Figure 2

The percentages in amino acid acid identity between the QLQ domains, and the percentage identity between the WRC domains are significantly higher than the percentage amino acid identity calculated between the full length GRF polypeptide sequences.

**Table B.1** : MatGAT results for global similarity and identity over the full length of the GRF polypeptide sequences.

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| 1. Aqufo_GRF | | 31 | 22 | 25 | 23 | **38** | 22 | 19 | 22 | 23 | 39 | 31 | 21 | 46 | 23 | 18 | 34 | 15 | 33 | 41 | 29 | 18 | 34 | 35 | 23 | 23 | 23 |
| 2. Arath_GRF_AT2G06200.1 | 43 | | 18 | 23 | 20 | **28** | 18 | 17 | 18 | 21 | 27 | 26 | 21 | 32 | 19 | 21 | 25 | 21 | 26 | 28 | 22 | 21 | 25 | 27 | 20 | 22 | 20 |
| 3. Arath_GRF_AT2G22840.1 | 36 | 25 | | 26 | 19 | **22** | 22 | 23 | 57 | 21 | 21 | 24 | 27 | 22 | 20 | 16 | 24 | 15 | 26 | 23 | 32 | 21 | 24 | 22 | 30 | 31 | 28 |
| 4. Arath_GRF_AT2G36400.1 | 43 | 31 | 38 | | 23 | **27** | 48 | 23 | 26 | 26 | 24 | 26 | 47 | 25 | 20 | 24 | 28 | 19 | 28 | 25 | 25 | 23 | 28 | 27 | 24 | 25 | 25 |
| 5. Arath_GRF_AT2G45480.1 | 38 | 30 | 33 | 39 | | **21** | 21 | 16 | 17 | 23 | 22 | 23 | 21 | 24 | 29 | 16 | 23 | 16 | 21 | 22 | 23 | 17 | 22 | 24 | 18 | 22 | 21 |
| 6. Arath_GRF_AT3G13960.1 | 53 | 38 | 34 | 44 | 34 | | **23** | **18** | **21** | **22** | **83** | **29** | **22** | **45** | **21** | **16** | **29** | **16** | **29** | **35** | **26** | **17** | **28** | **27** | **23** | **25** | **22** |
| 7. Arath_GRF_AT3G52910.1 | 34 | 26 | 40 | 56 | 36 | 36 | | 20 | 24 | 22 | 23 | 22 | 31 | 22 | 19 | 17 | 22 | 15 | 23 | 21 | 23 | 18 | 22 | 20 | 23 | 22 | 22 |
| 8. Arath_GRF_AT4G24150.1 | 31 | 25 | 38 | 36 | 32 | 30 | 35 | | 23 | 25 | 19 | 21 | 25 | 18 | 16 | 17 | 21 | 18 | 23 | 19 | 17 | 18 | 22 | 20 | 22 | 23 | 20 |
| 9. Arath_GRF_AT4G37740.1 | 35 | 24 | 72 | 38 | 33 | 31 | 40 | 39 | | 21 | 23 | 23 | 26 | 23 | 20 | 18 | 23 | 17 | 24 | 21 | 27 | 19 | 24 | 24 | 29 | 29 | 26 |
| 10. Arath_GRF_AT5G53660.1 | 37 | 30 | 35 | 40 | 35 | 37 | 34 | 36 | 33 | | 24 | 27 | 25 | 23 | 23 | 19 | 24 | 14 | 25 | 22 | 22 | 18 | 24 | 25 | 23 | 24 | 25 |
| 11. Brana_GRF | 54 | 39 | 33 | 41 | 35 | 90 | 33 | 33 | 34 | 39 | | 28 | 21 | 47 | 21 | 16 | 29 | 15 | 31 | 35 | 26 | 18 | 29 | 28 | 21 | 24 | 24 |
| 12. Horvu_GRF | 49 | 34 | 35 | 42 | 39 | 44 | 35 | 32 | 35 | 41 | 47 | | 25 | 25 | 23 | 21 | 68 | 20 | 62 | 29 | 24 | 22 | 70 | 42 | 25 | 28 | 25 |
| 13. Lyces_GRF | 42 | 30 | 38 | 64 | 37 | 41 | 43 | 38 | 36 | 41 | 40 | 42 | | 24 | 21 | 25 | 25 | 18 | 27 | 22 | 24 | 25 | 25 | 25 | 23 | 26 | 24 |
| 14. Medtr_GRF | 61 | 44 | 34 | 38 | 36 | 65 | 34 | 31 | 36 | 38 | 63 | 44 | 40 | | 22 | 17 | 31 | 14 | 31 | 38 | 26 | 17 | 28 | 30 | 22 | 24 | 22 |
| 15. Medtr_GRF\like | 37 | 27 | 32 | 33 | 46 | 37 | 33 | 31 | 34 | 37 | 37 | 37 | 34 | 36 | | 16 | 22 | 20 | 24 | 20 | 20 | 18 | 22 | 21 | 24 | 23 | 22 |
| 16. Orysa_GRF_NM_001054270.1 | 27 | 37 | 23 | 31 | 25 | 24 | 23 | 24 | 24 | 28 | 25 | 29 | 32 | 27 | 24 | | 22 | 35 | 22 | 18 | 16 | 66 | 21 | 20 | 21 | 21 | 19 |
| 17. Orysa_GRF_NM_001060298.1 | 53 | 35 | 36 | 44 | 35 | 46 | 35 | 32 | 35 | 42 | 46 | 78 | 41 | 48 | 36 | 30 | | 20 | 70 | 33 | 24 | 23 | 98 | 42 | 25 | 27 | 26 |
| 18. Orysa_GRF_NM_001066126.1 | 27 | 38 | 24 | 29 | 28 | 29 | 23 | 26 | 25 | 26 | 28 | 30 | 31 | 29 | 29 | 46 | 32 | | 20 | 14 | 16 | 34 | 20 | 14 | 20 | 18 | 18 |
| 19. Orysa_GRF_Os02g47280.2 | 51 | 38 | 36 | 47 | 36 | 46 | 36 | 35 | 35 | 39 | 49 | 73 | 44 | 47 | 37 | 30 | 78 | 29 | | 34 | 25 | 24 | 70 | 41 | 25 | 29 | 24 |
| 20. Orysa_GRF_Os02g53690.1 | 57 | 38 | 36 | 43 | 36 | 52 | 33 | 32 | 34 | 36 | 52 | 49 | 40 | 52 | 33 | 26 | 49 | 26 | 50 | | 27 | 20 | 33 | 34 | 23 | 25 | 22 |
| 21. Orysa_GRF_Os03g51970.1 | 40 | 31 | 49 | 40 | 39 | 40 | 37 | 29 | 45 | 40 | 40 | 38 | 38 | 40 | 38 | 23 | 38 | 24 | 40 | 40 | | 18 | 24 | 25 | 28 | 38 | 25 |
| 22. Orysa_GRF_Os04g48510.1 | 29 | 41 | 26 | 30 | 28 | 26 | 24 | 27 | 26 | 31 | 28 | 31 | 33 | 30 | 28 | 71 | 32 | 47 | 32 | 29 | 24 | | 23 | 22 | 23 | 22 | 19 |
| 23. Orysa_GRF_Os04g51190.1 | 52 | 35 | 35 | 44 | 34 | 45 | 36 | 32 | 34 | 41 | 46 | 79 | 41 | 44 | 35 | 29 | 98 | 31 | 78 | 50 | 37 | 32 | | 42 | 24 | 27 | 26 |
| 24. Orysa_GRF_Os06g02560.1 | 52 | 38 | 32 | 38 | 37 | 41 | 33 | 29 | 33 | 40 | 45 | 56 | 40 | 46 | 34 | 28 | 54 | 24 | 55 | 47 | 35 | 32 | 54 | | 24 | 25 | 23 |
| 25. Orysa_GRF_Os11g35030.1 | 38 | 32 | 43 | 37 | 36 | 38 | 35 | 34 | 40 | 38 | 38 | 38 | 39 | 35 | 37 | 27 | 37 | 29 | 40 | 39 | 45 | 29 | 39 | 36 | | 37 | 28 |
| 26. Orysa_GRF_Os12g29980.1 | 39 | 33 | 46 | 40 | 37 | 40 | 35 | 37 | 43 | 39 | 40 | 41 | 40 | 38 | 39 | 27 | 41 | 28 | 42 | 37 | 55 | 29 | 41 | 37 | 54 | | 28 |
| 27. Oyrsa_GRF_Os03g47140.1 | 37 | 30 | 39 | 40 | 40 | 37 | 35 | 34 | 36 | 40 | 42 | 39 | 38 | 32 | 38 | 26 | 36 | 28 | 37 | 35 | 44 | 27 | 40 | 34 | 43 | 48 | |
| 28. Poptr_GRF_lcl_scaff_28.10 | 67 | 43 | 34 | 40 | 39 | 52 | 37 | 31 | 34 | 37 | 55 | 53 | 42 | 60 | 37 | 24 | 55 | 27 | 53 | 55 | 40 | 27 | 55 | 48 | 38 | 38 | 38 |
| 29. Poptr_GRF_lcl_scaff_28.309 | 40 | 32 | 36 | 65 | 33 | 38 | 46 | 33 | 35 | 42 | 38 | 42 | 59 | 41 | 33 | 30 | 40 | 32 | 41 | 39 | 34 | 33 | 41 | 42 | 39 | 40 | 39 |

| | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 |
|---|---|---|---|---|---|---|---|---|---|---|---|---|---|---|---|---|---|---|---|---|---|---|---|---|---|---|---|---|---|---|
| 1. Aqufo_GRF | 54 | 23 | 47 | 21 | 18 | 20 | 34 | 25 | 21 | 44 | 23 | 21 | 24 | 43 | 22 | 57 | 15 | 34 | 28 | 23 | 33 | 34 | 25 | 37 | 13 | 32 | 32 | 33 | 22 | 38 |
| 2. Arath_GRF_AT2G06200.1 | 34 | 22 | 32 | 16 | 24 | 16 | 27 | 17 | 15 | 30 | 19 | 15 | 18 | 30 | 20 | 32 | 20 | 29 | 27 | 20 | 26 | 24 | 23 | 27 | 20 | 27 | 25 | 28 | 20 | 30 |
| 3. Arath_GRF_AT2G22840.1 | 22 | 26 | 22 | 24 | 17 | 42 | 22 | 40 | 27 | 21 | 20 | 44 | 39 | 21 | 29 | 23 | 16 | 21 | 22 | 29 | 23 | 25 | 29 | 21 | 15 | 25 | 24 | 21 | 30 | 22 |
| 4. Arath_GRF_AT2G36400.1 | 23 | 51 | 27 | 26 | 20 | 25 | 25 | 27 | 23 | 27 | 22 | 25 | 27 | 27 | 27 | 24 | 18 | 26 | 30 | 25 | 29 | 31 | 27 | 26 | 19 | 26 | 25 | 24 | 25 | 24 |
| 5. Arath_GRF_AT2G45480.1 | 22 | 19 | 23 | 18 | 17 | 17 | 23 | 21 | 19 | 24 | 28 | 20 | 21 | 23 | 19 | 23 | 17 | 21 | 21 | 18 | 25 | 22 | 21 | 21 | 17 | 21 | 20 | 22 | 19 | 19 |
| **6. Arath_GRF_AT3G13960.1** | **36** | **21** | **41** | **20** | **17** | **22** | **27** | **23** | **21** | **31** | **22** | **22** | **25** | **30** | **24** | **40** | **15** | **29** | **26** | **23** | **28** | **29** | **25** | **33** | **15** | **28** | **29** | **31** | **23** | **33** |
| 7. Arath_GRF_AT3G52910.1 | 24 | 37 | 22 | 22 | 14 | 23 | 22 | 22 | 21 | 23 | 23 | 22 | 25 | 21 | 22 | 21 | 14 | 19 | 21 | 22 | 22 | 24 | 22 | 21 | 14 | 21 | 20 | 20 | 23 | 20 |
| 8. Arath_GRF_AT4G24150.1 | 19 | 22 | 20 | 32 | 15 | 21 | 19 | 20 | 25 | 20 | 17 | 20 | 22 | 18 | 21 | 19 | 19 | 17 | 22 | 22 | 21 | 22 | 20 | 17 | 17 | 22 | 22 | 18 | 22 | 18 |
| 9. Arath_GRF_AT4G37740.1 | 21 | 24 | 23 | 24 | 17 | 38 | 21 | 42 | 27 | 23 | 19 | 42 | 35 | 23 | 27 | 22 | 15 | 20 | 21 | 29 | 23 | 24 | 28 | 20 | 16 | 23 | 22 | 21 | 28 | 20 |
| 10. Arath_GRF_AT5G53660.1 | 22 | 28 | 26 | 24 | 18 | 22 | 22 | 25 | 27 | 23 | 21 | 23 | 24 | 23 | 25 | 22 | 14 | 22 | 24 | 24 | 25 | 24 | 24 | 21 | 13 | 26 | 23 | 22 | 24 | 21 |
| 11. Brana_GRF | 37 | 21 | 44 | 19 | 17 | 20 | 30 | 22 | 21 | 31 | 23 | 21 | 24 | 31 | 24 | 41 | 13 | 29 | 27 | 25 | 26 | 30 | 24 | 34 | 13 | 28 | 28 | 30 | 23 | 34 |
| 12. Horvu_GRF | 32 | 25 | 30 | 21 | 21 | 23 | 35 | 23 | 23 | 39 | 21 | 22 | 26 | 38 | 24 | 31 | 20 | 31 | 35 | 25 | 38 | 54 | 26 | 30 | 19 | 63 | 64 | 30 | 23 | 29 |
| 13. Lyces_GRF | 22 | 45 | 26 | 25 | 21 | 23 | 24 | 25 | 23 | 25 | 22 | 25 | 24 | 25 | 24 | 20 | 20 | 23 | 27 | 25 | 25 | 23 | 25 | 23 | 19 | 25 | 24 | 23 | 24 | 24 |
| 14. Medtr_GRF | 43 | 24 | 56 | 22 | 18 | 22 | 29 | 22 | 21 | 36 | 23 | 21 | 23 | 35 | 23 | 53 | 12 | 30 | 26 | 22 | 27 | 29 | 25 | 33 | 13 | 28 | 27 | 32 | 22 | 35 |
| 15. Medtr_GRF\like | 22 | 19 | 20 | 21 | 16 | 19 | 25 | 20 | 21 | 23 | 29 | 19 | 22 | 22 | 21 | 21 | 19 | 20 | 21 | 21 | 22 | 22 | 24 | 22 | 20 | 25 | 25 | 20 | 21 | 23 |
| 16. Orysa_GRF_NM_001054270.1 | 16 | 23 | 18 | 18 | 38 | 16 | 19 | 16 | 16 | 21 | 18 | 16 | 17 | 21 | 20 | 17 | 36 | 21 | 23 | 21 | 20 | 23 | 22 | 18 | 34 | 23 | 21 | 22 | 20 | 20 |
| 17. Orysa_GRF_NM_001060298.1 | 36 | 25 | 32 | 22 | 20 | 22 | 36 | 25 | 24 | 43 | 19 | 22 | 25 | 39 | 25 | 34 | 19 | 34 | 37 | 26 | 41 | 63 | 28 | 35 | 18 | 69 | 68 | 35 | 26 | 32 |
| 18. Orysa_GRF_NM_001066126.1 | 12 | 19 | 15 | 18 | 29 | 16 | 16 | 15 | 16 | 14 | 16 | 16 | 14 | 15 | 19 | 15 | 73 | 17 | 20 | 19 | 15 | 19 | 19 | 15 | 73 | 19 | 20 | 20 | 18 | 19 |
| 19. Orysa_GRF_Os02g47280.2 | 33 | 27 | 32 | 23 | 20 | 23 | 35 | 25 | 25 | 44 | 23 | 23 | 23 | 42 | 25 | 34 | 20 | 32 | 35 | 28 | 41 | 72 | 26 | 32 | 20 | 61 | 59 | 31 | 25 | 31 |
| 20. Orysa_GRF_Os02g53690.1 | 41 | 23 | 38 | 19 | 19 | 22 | 30 | 23 | 20 | 35 | 23 | 21 | 25 | 35 | 21 | 42 | 15 | 43 | 30 | 22 | 32 | 36 | 26 | 69 | 16 | 33 | 33 | 43 | 21 | 64 |
| 21. Orysa_GRF_Os03g51970.1 | 27 | 21 | 28 | 22 | 15 | 37 | 25 | 38 | 24 | 25 | 22 | 34 | 43 | 26 | 26 | 27 | 14 | 25 | 25 | 26 | 27 | 27 | 30 | 26 | 14 | 25 | 25 | 27 | 26 | 28 |
| 22. Orysa_GRF_Os04g48510.1 | 18 | 24 | 19 | 18 | 38 | 18 | 22 | 17 | 17 | 23 | 20 | 16 | 18 | 21 | 22 | 19 | 34 | 21 | 23 | 21 | 23 | 24 | 26 | 19 | 32 | 23 | 21 | 22 | 20 | 20 |
| 23. Orysa_GRF_Os04g51190.1 | 36 | 24 | 31 | 22 | 21 | 22 | 36 | 25 | 24 | 42 | 22 | 22 | 24 | 39 | 26 | 34 | 19 | 34 | 36 | 26 | 41 | 62 | 28 | 34 | 19 | 71 | 69 | 35 | 26 | 32 |
| 24. Orysa_GRF_Os06g02560.1 | 36 | 27 | 30 | 21 | 21 | 22 | 39 | 24 | 21 | 42 | 24 | 22 | 26 | 41 | 22 | 33 | 15 | 35 | 57 | 24 | 68 | 39 | 27 | 34 | 14 | 42 | 41 | 36 | 23 | 33 |
| 25. Orysa_GRF_Os11g35030.1 | 21 | 24 | 25 | 25 | 19 | 29 | 23 | 29 | 23 | 23 | 20 | 27 | 29 | 22 | 29 | 23 | 18 | 23 | 23 | 29 | 25 | 24 | 55 | 22 | 18 | 24 | 24 | 22 | 29 | 21 |
| 26. Orysa_GRF_Os12g29980.1 | 24 | 26 | 26 | 22 | 17 | 32 | 26 | 34 | 24 | 27 | 24 | 31 | 37 | 27 | 26 | 27 | 16 | 25 | 24 | 28 | 24 | 27 | 42 | 25 | 16 | 28 | 26 | 25 | 26 | 25 |
| 27. Oyrsa_GRF_Os03g47140.1 | 22 | 26 | 25 | 23 | 18 | 27 | 23 | 27 | 23 | 26 | 21 | 27 | 25 | 24 | 71 | 22 | 15 | 24 | 24 | 65 | 25 | 26 | 28 | 22 | 17 | 25 | 25 | 23 | 67 | 22 |
| 28. Poptr_GRF_lcl_scaff_28.10 | | 25 | 44 | 21 | 19 | 23 | 35 | 24 | 22 | 41 | 22 | 21 | 25 | 39 | 22 | 52 | 14 | 35 | 28 | 25 | 35 | 32 | 25 | 37 | 14 | 33 | 31 | 33 | 23 | 38 |
| 29. Poptr_GRF_lcl_scaff_28.309 | 42 | | 26 | 24 | 20 | 24 | 24 | 24 | 22 | 26 | 21 | 23 | 24 | 25 | 25 | 25 | 19 | 24 | 27 | 25 | 25 | 25 | 23 | 23 | 19 | 25 | 24 | 24 | 25 | 24 |

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 |
|---|---|---|---|---|---|---|---|---|---|---|---|---|---|---|---|---|---|---|---|---|---|---|---|---|---|---|---|
| 30. Poptr_GRF_lcl_scaff_I.1018 | 62 | 43 | 32 | 39 | 35 | 58 | 35 | 29 | 32 | 42 | 59 | 45 | 39 | 71 | 33 | 27 | 50 | 28 | 48 | 52 | 40 | 31 | 48 | 48 | 38 | 37 | 35 |
| 31. Poptr_GRF_lcl_scaff_I.688 | 32 | 25 | 39 | 36 | 32 | 31 | 37 | 46 | 38 | 35 | 32 | 35 | 38 | 33 | 33 | 22 | 34 | 26 | 36 | 33 | 36 | 24 | 34 | 33 | 34 | 37 | 37 |
| 32. Poptr_GRF_lcl_scaff_I.995 | 26 | 34 | 22 | 27 | 24 | 24 | 20 | 22 | 22 | 28 | 25 | 26 | 28 | 28 | 22 | 51 | 26 | 42 | 27 | 27 | 21 | 50 | 26 | 28 | 25 | 24 | 24 |
| 33. Poptr_GRF_lcl_scaff_II.1070 | 31 | 24 | 59 | 36 | 32 | 33 | 39 | 35 | 54 | 34 | 31 | 34 | 33 | 32 | 33 | 21 | 33 | 24 | 34 | 33 | 50 | 24 | 33 | 31 | 40 | 46 | 39 |
| 34. Poptr_GRF_lcl_scaff_III.741 | 52 | 38 | 34 | 38 | 36 | 45 | 32 | 31 | 33 | 36 | 45 | 53 | 38 | 48 | 38 | 28 | 53 | 27 | 50 | 43 | 38 | 32 | 53 | 58 | 38 | 40 | 36 |
| 35. Poptr_GRF_lcl_scaff_VII.1274 | 38 | 25 | 57 | 37 | 34 | 35 | 41 | 37 | 58 | 37 | 34 | 35 | 36 | 34 | 33 | 22 | 36 | 25 | 36 | 34 | 52 | 25 | 37 | 32 | 41 | 46 | 41 |
| 36. Poptr_GRF_lcl_scaff_XII.277 | 34 | 25 | 40 | 37 | 33 | 33 | 37 | 42 | 44 | 38 | 33 | 33 | 34 | 32 | 33 | 22 | 32 | 24 | 35 | 32 | 37 | 22 | 33 | 31 | 36 | 37 | 35 |
| 37. Poptr_GRF_lcl_scaff_XIII.769 | 57 | 42 | 32 | 42 | 38 | 46 | 32 | 30 | 34 | 34 | 46 | 53 | 39 | 53 | 35 | 31 | 57 | 26 | 57 | 48 | 37 | 35 | 56 | 57 | 38 | 39 | 38 |
| 38. Poptr_GRF_lcl_scaff_XIV.174 | 33 | 25 | 36 | 35 | 43 | 35 | 39 | 34 | 36 | 35 | 35 | 35 | 35 | 35 | 42 | 23 | 31 | 25 | 36 | 34 | 37 | 25 | 32 | 34 | 33 | 35 | 35 |
| 39. Poptr_GRF_lcl_scaff_XIV.39 | 34 | 22 | 59 | 36 | 33 | 32 | 38 | 34 | 55 | 35 | 30 | 34 | 36 | 32 | 32 | 21 | 32 | 24 | 33 | 33 | 47 | 22 | 32 | 30 | 38 | 42 | 38 |
| 40. Poptr_GRF_lcl_scaff_XIV.51 | 37 | 27 | 60 | 41 | 35 | 35 | 42 | 40 | 54 | 37 | 36 | 37 | 36 | 37 | 37 | 22 | 36 | 23 | 35 | 40 | 57 | 24 | 38 | 36 | 43 | 52 | 40 |
| 41. Poptr_GRF_lcl_scaff_XIX.480 | 54 | 42 | 32 | 40 | 35 | 44 | 31 | 28 | 32 | 36 | 47 | 51 | 38 | 49 | 32 | 33 | 54 | 28 | 52 | 47 | 35 | 34 | 53 | 53 | 35 | 37 | 35 |
| 42. Sacof_GRF | 37 | 28 | 41 | 39 | 37 | 39 | 37 | 35 | 39 | 36 | 41 | 37 | 40 | 35 | 37 | 27 | 37 | 28 | 38 | 35 | 43 | 30 | 40 | 35 | 45 | 46 | 82 |
| 43. Vitvi_GRF | 70 | 43 | 35 | 41 | 35 | 56 | 33 | 32 | 33 | 37 | 58 | 48 | 39 | 69 | 34 | 26 | 51 | 24 | 50 | 58 | 40 | 29 | 51 | 50 | 38 | 40 | 36 |
| 44. Zeama_GRF10_EF515849.1 | 26 | 36 | 23 | 29 | 27 | 27 | 23 | 26 | 25 | 26 | 26 | 31 | 32 | 26 | 30 | 44 | 32 | 81 | 32 | 22 | 24 | 44 | 32 | 28 | 30 | 28 | 29 |
| 45. Zeama_GRF11_EF515850.1 | 50 | 41 | 29 | 41 | 33 | 42 | 28 | 25 | 30 | 35 | 42 | 44 | 35 | 45 | 33 | 31 | 46 | 30 | 45 | 53 | 35 | 35 | 46 | 47 | 35 | 36 | 33 |
| 46. Zeama_GRF12_EF515851.1 | 44 | 38 | 31 | 40 | 32 | 41 | 30 | 30 | 30 | 39 | 44 | 46 | 38 | 42 | 31 | 32 | 46 | 33 | 45 | 40 | 34 | 32 | 45 | 67 | 36 | 35 | 34 |
| 47. Zeama_GRF13_EF515852.1 | 37 | 29 | 39 | 40 | 37 | 40 | 36 | 37 | 39 | 36 | 38 | 40 | 37 | 35 | 38 | 26 | 39 | 29 | 41 | 35 | 44 | 28 | 40 | 36 | 43 | 47 | 78 |
| 48. Zeama_GRF14_EF515853.1 | 49 | 36 | 33 | 45 | 36 | 43 | 35 | 30 | 33 | 42 | 42 | 53 | 42 | 43 | 34 | 28 | 55 | 27 | 54 | 47 | 40 | 30 | 54 | 77 | 39 | 36 | 39 |
| 49. Zeama_GRF1_EF515840.1 | 50 | 35 | 38 | 47 | 37 | 47 | 36 | 34 | 34 | 39 | 45 | 67 | 41 | 43 | 38 | 29 | 74 | 29 | 79 | 51 | 42 | 30 | 74 | 50 | 40 | 41 | 43 |
| 50. Zeama_GRF2_EF515841.1 | 42 | 35 | 38 | 41 | 36 | 41 | 30 | 31 | 37 | 45 | 41 | 40 | 38 | 41 | 39 | 29 | 43 | 31 | 40 | 40 | 46 | 33 | 43 | 42 | 66 | 54 | 42 |
| 51. Zeama_GRF3_EF515842.1 | 51 | 36 | 33 | 41 | 38 | 49 | 34 | 27 | 31 | 36 | 49 | 45 | 40 | 46 | 36 | 27 | 48 | 25 | 50 | 80 | 38 | 29 | 48 | 46 | 37 | 39 | 33 |
| 52. Zeama_GRF4_EF515843.1 | 24 | 36 | 24 | 30 | 27 | 28 | 21 | 25 | 26 | 25 | 28 | 31 | 31 | 26 | 28 | 45 | 31 | 80 | 32 | 27 | 24 | 44 | 32 | 26 | 30 | 27 | 29 |
| 53. Zeama_GRF5_EF515844.1 | 50 | 35 | 35 | 42 | 35 | 42 | 34 | 32 | 34 | 40 | 43 | 75 | 40 | 41 | 36 | 31 | 80 | 31 | 72 | 48 | 38 | 32 | 80 | 54 | 38 | 41 | 39 |
| 54. Zeama_GRF6_EF515845.1 | 50 | 36 | 35 | 40 | 35 | 44 | 33 | 30 | 36 | 39 | 45 | 76 | 40 | 42 | 37 | 30 | 80 | 32 | 71 | 46 | 38 | 31 | 81 | 51 | 38 | 39 | 36 |
| 55. Zeama_GRF7_EF515846.1 | 48 | 41 | 31 | 39 | 34 | 44 | 29 | 27 | 31 | 37 | 45 | 42 | 35 | 47 | 32 | 32 | 46 | 31 | 45 | 54 | 35 | 34 | 45 | 48 | 34 | 36 | 35 |
| 56. Zeama_GRF8_EF515847.1 | 38 | 29 | 39 | 38 | 36 | 38 | 34 | 37 | 40 | 37 | 38 | 37 | 39 | 35 | 38 | 27 | 37 | 30 | 38 | 35 | 43 | 28 | 39 | 34 | 46 | 44 | 79 |
| 57. Zeama_GRF9_EF515848.1 | 57 | 42 | 31 | 37 | 31 | 49 | 32 | 29 | 31 | 32 | 50 | 45 | 40 | 52 | 35 | 31 | 45 | 27 | 45 | 73 | 40 | 31 | 45 | 46 | 35 | 39 | 33 |

| | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 |
|---|---|---|---|---|---|---|---|---|---|---|---|---|---|---|---|---|---|---|---|---|---|---|---|---|---|---|---|---|---|---|
| 30. Poptr_GRFscaff_I.1018 | 59 | 39 | | 22 | 20 | 19 | 32 | 23 | 22 | 36 | 21 | 21 | 23 | 36 | 23 | 56 | 14 | 34 | 31 | 24 | 31 | 33 | 29 | 36 | 14 | 30 | 30 | 34 | 24 | 37 |
| 31. Poptr_GRFscaff_I.688 | 36 | 33 | 31 | | 16 | 25 | 20 | 25 | 29 | 20 | 21 | 24 | 24 | 21 | 25 | 21 | 16 | 20 | 21 | 25 | 23 | 25 | 22 | 20 | 16 | 25 | 21 | 21 | 24 | 19 |
| 32. Poptr_GRFscaff_I.995 | 25 | 29 | 28 | 21 | | 14 | 19 | 16 | 13 | 22 | 17 | 14 | 15 | 21 | 18 | 19 | 30 | 21 | 23 | 17 | 20 | 19 | 21 | 18 | 29 | 20 | 20 | 22 | 17 | 18 |
| 33. Poptr_GRFscaff_II.1070 | 34 | 32 | 31 | 39 | 19 | | 22 | 47 | 28 | 22 | 21 | 50 | 75 | 21 | 27 | 21 | 15 | 20 | 20 | 27 | 22 | 24 | 28 | 20 | 16 | 23 | 22 | 19 | 25 | 20 |
| 34. Poptr_GRFscaff_III.741 | 51 | 41 | 50 | 32 | 28 | 31 | | 23 | 20 | 47 | 22 | 20 | 25 | 44 | 24 | 33 | 15 | 30 | 34 | 25 | 36 | 32 | 25 | 29 | 16 | 35 | 36 | 30 | 24 | 32 |
| 35. Poptr_GRFscaff_VII.1274 | 35 | 35 | 33 | 39 | 20 | 62 | 34 | | 28 | 23 | 23 | 74 | 50 | 21 | 26 | 25 | 15 | 22 | 22 | 25 | 23 | 26 | 31 | 21 | 16 | 25 | 25 | 22 | 25 | 21 |
| 36. Poptr_GRFscaff_XII.277 | 35 | 34 | 33 | 46 | 18 | 43 | 31 | 41 | | 22 | 22 | 28 | 27 | 22 | 25 | 22 | 17 | 19 | 20 | 24 | 22 | 23 | 22 | 20 | 16 | 24 | 24 | 19 | 25 | 18 |
| 37. Poptr_GRFscaff_XIII.769 | 54 | 41 | 54 | 29 | 31 | 30 | 63 | 32 | 31 | | 23 | 22 | 25 | 81 | 26 | 41 | 13 | 33 | 35 | 25 | 40 | 39 | 26 | 33 | 13 | 41 | 40 | 32 | 25 | 34 |
| 38. Poptr_GRFscaff_XIV.174 | 34 | 35 | 35 | 37 | 23 | 37 | 33 | 39 | 39 | 33 | | 23 | 24 | 25 | 20 | 22 | 18 | 20 | 23 | 21 | 23 | 23 | 21 | 21 | 18 | 22 | 23 | 19 | 19 | 21 |
| 39. Poptr_GRFscaff_XIV.39 | 33 | 31 | 30 | 36 | 19 | 68 | 30 | 78 | 40 | 30 | 37 | | 45 | 21 | 25 | 21 | 14 | 19 | 21 | 25 | 22 | 23 | 28 | 19 | 14 | 24 | 23 | 19 | 26 | 19 |
| 40. Poptr_GRFscaff_XIV.51 | 38 | 34 | 34 | 41 | 20 | 79 | 35 | 66 | 45 | 34 | 41 | 61 | | 22 | 26 | 24 | 16 | 23 | 23 | 25 | 24 | 26 | 30 | 24 | 14 | 25 | 25 | 22 | 25 | 23 |
| 41. Poptr_GRFscaff_XIX.480 | 52 | 39 | 53 | 30 | 31 | 30 | 59 | 31 | 29 | 88 | 31 | 30 | 32 | | 22 | 39 | 15 | 30 | 33 | 24 | 37 | 37 | 26 | 32 | 17 | 40 | 40 | 32 | 22 | 32 |
| 42. Sacof_GRF | 38 | 39 | 35 | 38 | 24 | 40 | 40 | 40 | 38 | 37 | 33 | 37 | 42 | 35 | | 22 | 18 | 22 | 25 | 86 | 24 | 25 | 30 | 22 | 17 | 27 | 26 | 23 | 91 | 21 |
| 43. Vitvi_GRF | 65 | 40 | 70 | 31 | 27 | 30 | 51 | 34 | 32 | 54 | 34 | 31 | 37 | 53 | 36 | | 14 | 34 | 29 | 22 | 33 | 33 | 26 | 40 | 13 | 31 | 32 | 36 | 23 | 42 |
| 44. Zeama_GRF10_EF515849 | 25 | 34 | 25 | 24 | 41 | 23 | 25 | 23 | 24 | 28 | 24 | 22 | 24 | 26 | 28 | 27 | | 17 | 21 | 17 | 14 | 19 | 18 | 15 | 86 | 19 | 19 | 19 | 18 | 16 |
| 45. Zeama_GRF11_EF515850 | 49 | 38 | 46 | 29 | 29 | 28 | 46 | 31 | 29 | 50 | 30 | 27 | 31 | 48 | 34 | 46 | 28 | | 32 | 23 | 34 | 33 | 26 | 41 | 18 | 33 | 31 | 75 | 22 | 41 |
| 46. Zeama_GRF12_EF515851 | 45 | 39 | 45 | 30 | 32 | 27 | 50 | 31 | 30 | 52 | 32 | 28 | 33 | 48 | 36 | 45 | 33 | 46 | | 27 | 61 | 33 | 24 | 29 | 20 | 34 | 34 | 32 | 24 | 31 |
| 47. Zeama_GRF13_EF515852 | 37 | 37 | 34 | 37 | 23 | 41 | 38 | 39 | 38 | 37 | 34 | 38 | 42 | 35 | 90 | 35 | 26 | 35 | 35 | | 24 | 26 | 29 | 22 | 18 | 27 | 26 | 23 | 86 | 22 |
| 48. Zeama_GRF14_EF515853 | 50 | 41 | 45 | 35 | 28 | 32 | 52 | 33 | 32 | 52 | 35 | 31 | 36 | 48 | 38 | 48 | 25 | 45 | 67 | 38 | | 38 | 26 | 33 | 15 | 40 | 39 | 34 | 24 | 34 |
| 49. Zeama_GRF1_EF515840 | 49 | 42 | 45 | 37 | 25 | 37 | 46 | 38 | 37 | 52 | 36 | 34 | 41 | 50 | 42 | 48 | 30 | 46 | 43 | 42 | 52 | | 29 | 35 | 18 | 57 | 57 | 32 | 25 | 34 |
| 50. Zeama_GRF2_EF515841 | 40 | 36 | 43 | 33 | 29 | 39 | 41 | 40 | 35 | 42 | 33 | 38 | 41 | 38 | 40 | 42 | 30 | 39 | 36 | 41 | 42 | 42 | | 23 | 19 | 28 | 26 | 27 | 30 | 24 |
| 51. Zeama_GRF3_EF515842 | 53 | 39 | 49 | 33 | 25 | 30 | 41 | 33 | 31 | 45 | 34 | 29 | 33 | 43 | 35 | 54 | 24 | 52 | 42 | 33 | 50 | 52 | 38 | | 15 | 33 | 34 | 41 | 22 | 72 |
| 52. Zeama_GRF4_EF515843 | 25 | 32 | 28 | 26 | 41 | 24 | 27 | 23 | 24 | 28 | 25 | 23 | 24 | 30 | 27 | 28 | 90 | 31 | 33 | 28 | 26 | 31 | 33 | 25 | | 19 | 20 | 20 | 18 | 16 |
| 53. Zeama_GRF5_EF515844.1 | 52 | 41 | 45 | 34 | 26 | 33 | 49 | 35 | 33 | 52 | 34 | 33 | 35 | 52 | 38 | 47 | 32 | 46 | 44 | 38 | 53 | 68 | 43 | 47 | 31 | | 87 | 33 | 27 | 31 |
| 54. Zeama_GRF6_EF515845.1 | 51 | 41 | 43 | 33 | 26 | 33 | 51 | 35 | 33 | 53 | 33 | 32 | 36 | 51 | 37 | 46 | 31 | 44 | 43 | 38 | 53 | 71 | 38 | 47 | 31 | 90 | | 33 | 25 | 32 |
| 55. Zeama_GRF7_EF515846.1 | 47 | 36 | 49 | 31 | 30 | 28 | 48 | 31 | 29 | 49 | 29 | 27 | 32 | 47 | 35 | 51 | 32 | 83 | 46 | 35 | 47 | 44 | 38 | 52 | 31 | 47 | 43 | | 23 | 40 |
| 56. Zeama_GRF8_EF515847.1 | 38 | 38 | 36 | 37 | 23 | 39 | 37 | 40 | 37 | 35 | 33 | 38 | 41 | 35 | 94 | 37 | 27 | 33 | 34 | 91 | 38 | 40 | 43 | 36 | 29 | 39 | 35 | 34 | | 21 |
| 57. Zeama_GRF9_EF515848.1 | 52 | 39 | 52 | 30 | 27 | 31 | 45 | 32 | 29 | 47 | 32 | 28 | 36 | 45 | 35 | 59 | 25 | 54 | 43 | 35 | 48 | 44 | 39 | 79 | 27 | 45 | 44 | 54 | 33 | |

**Table B.2** : MatGAT results for global similarity and identity over the full length of the SYT polypeptide sequences.

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 |
|---|---|---|---|---|---|---|---|---|---|---|---|---|---|---|---|---|---|---|---|---|---|---|---|---|---|---|---|---|---|---|---|---|---|---|---|---|
| 1. Allce_SYT2 | | 34 | 49 | 31 | 46 | 46 | 34 | 39 | 50 | 32 | 40 | 38 | 29 | 32 | 47 | 36 | 46 | 49 | 47 | 29 | 34 | 48 | 48 | 48 | 32 | 46 | 35 | 48 | 39 | 31 | 43 | 34 | 48 | 35 | 50 | 51 |
| 2. Aqufo_SYT1 | 53 | | 39 | 60 | 37 | 38 | 58 | 35 | 36 | 60 | 35 | 34 | 26 | 69 | 41 | 55 | 36 | 44 | 40 | 64 | 62 | 39 | 41 | 41 | 70 | 43 | 60 | 36 | 38 | 60 | 42 | 64 | 43 | 54 | 36 | 34 |
| 3. Aqufo_SYT2 | 61 | 56 | | 38 | 50 | 52 | 38 | 45 | 47 | 38 | 50 | 34 | 29 | 42 | 61 | 46 | 47 | 59 | 56 | 39 | 40 | 65 | 64 | 64 | 42 | 60 | 44 | 45 | 45 | 39 | 60 | 44 | 64 | 42 | 45 | 48 |
| 4. Arath_SYT1 | 49 | 78 | 52 | | 36 | 36 | 56 | 35 | 35 | 95 | 36 | 31 | 27 | 67 | 37 | 51 | 32 | 44 | 37 | 65 | 65 | 38 | 38 | 38 | 66 | 38 | 67 | 35 | 35 | 71 | 40 | 65 | 40 | 50 | 36 | 34 |
| 5. Arath_SYT2 | 59 | 54 | 62 | 50 | | 64 | 38 | 52 | 45 | 36 | 78 | 34 | 30 | 39 | 56 | 39 | 43 | 61 | 53 | 36 | 38 | 55 | 54 | 54 | 36 | 55 | 38 | 43 | 49 | 37 | 54 | 39 | 56 | 33 | 43 | 46 |
| 6. Arath_SYT3 | 59 | 53 | 67 | 52 | 68 | | 38 | 41 | 43 | 35 | 65 | 33 | 31 | 37 | 60 | 40 | 44 | 60 | 54 | 37 | 36 | 61 | 62 | 62 | 39 | 60 | 37 | 43 | 45 | 37 | 61 | 40 | 60 | 35 | 41 | 40 |
| 7. Aspof_SYT1 | 55 | 74 | 51 | 67 | 54 | 54 | | 36 | 35 | 56 | 38 | 36 | 27 | 58 | 42 | 56 | 32 | 44 | 42 | 59 | 59 | 41 | 39 | 39 | 59 | 45 | 58 | 36 | 36 | 53 | 45 | 57 | 42 | 61 | 39 | 34 |
| 8. Betvu_SYT2 | 47 | 45 | 55 | 47 | 61 | 52 | 47 | | 41 | 35 | 48 | 38 | 32 | 35 | 46 | 35 | 40 | 44 | 46 | 33 | 36 | 42 | 44 | 44 | 36 | 46 | 38 | 39 | 43 | 38 | 44 | 34 | 44 | 33 | 40 | 40 |
| 9. Bradi_SYT3 | 63 | 51 | 56 | 50 | 56 | 53 | 46 | 51 | | 34 | 40 | 33 | 32 | 36 | 50 | 37 | 48 | 46 | 45 | 35 | 35 | 50 | 51 | 51 | 38 | 47 | 35 | 68 | 41 | 36 | 47 | 37 | 50 | 36 | 66 | 80 |
| 10. Brana_SYT1 | 49 | 77 | 52 | 96 | 52 | 50 | 68 | 50 | 50 | | 34 | 30 | 25 | 66 | 37 | 50 | 34 | 42 | 37 | 67 | 64 | 38 | 37 | 37 | 66 | 36 | 67 | 35 | 34 | 70 | 38 | 65 | 39 | 50 | 37 | 34 |
| 11. Brana_SYT2 | 60 | 53 | 65 | 54 | 83 | 73 | 55 | 56 | 51 | 53 | | 37 | 32 | 36 | 57 | 36 | 40 | 62 | 52 | 35 | 36 | 54 | 54 | 54 | 34 | 55 | 36 | 43 | 48 | 35 | 56 | 36 | 56 | 35 | 41 | 41 |
| 12. Cerri_SYT\partial | 54 | 49 | 46 | 44 | 48 | 47 | 51 | 50 | 46 | 46 | 48 | | 26 | 33 | 36 | 33 | 34 | 39 | 38 | 32 | 32 | 38 | 38 | 38 | 32 | 36 | 31 | 35 | 31 | 32 | 36 | 30 | 37 | 34 | 33 | 36 |
| 13. Chlre_SYT | 39 | 35 | 39 | 36 | 42 | 38 | 37 | 45 | 46 | 33 | 40 | 37 | | 24 | 29 | 27 | 25 | 28 | 30 | 24 | 24 | 28 | 31 | 31 | 23 | 29 | 27 | 33 | 28 | 25 | 27 | 26 | 29 | 25 | 29 | 27 |
| 14. Citsi_SYT1 | 47 | 83 | 59 | 80 | 55 | 55 | 71 | 46 | 49 | 77 | 53 | 47 | 32 | | 42 | 58 | 34 | 43 | 39 | 71 | 72 | 41 | 42 | 42 | 87 | 43 | 70 | 35 | 35 | 68 | 43 | 73 | 43 | 54 | 37 | 35 |
| 15. Citsi_SYT2 | 61 | 58 | 72 | 54 | 66 | 69 | 58 | 57 | 61 | 55 | 68 | 51 | 40 | 61 | | 44 | 45 | 73 | 67 | 40 | 40 | 82 | 81 | 81 | 45 | 78 | 40 | 44 | 50 | 40 | 78 | 46 | 76 | 40 | 44 | 47 |
| 16. Cryja_SYT | 48 | 70 | 57 | 64 | 51 | 53 | 68 | 42 | 48 | 64 | 48 | 46 | 36 | 73 | 57 | | 41 | 43 | 39 | 53 | 54 | 43 | 44 | 44 | 63 | 44 | 53 | 34 | 36 | 50 | 43 | 56 | 45 | 52 | 37 | 33 |
| 17. Curlo_SYT | 62 | 49 | 59 | 46 | 54 | 57 | 44 | 47 | 61 | 46 | 55 | 46 | 35 | 50 | 58 | 53 | | 46 | 41 | 37 | 34 | 47 | 47 | 47 | 37 | 44 | 37 | 46 | 39 | 34 | 47 | 36 | 46 | 35 | 48 | 48 |
| 18. Eupes_SYT2 | 62 | 59 | 68 | 59 | 73 | 67 | 58 | 54 | 56 | 59 | 74 | 55 | 39 | 57 | 80 | 54 | 59 | | 67 | 42 | 43 | 73 | 74 | 74 | 42 | 69 | 40 | 48 | 50 | 40 | 73 | 42 | 73 | 43 | 50 | 48 |
| 19. Frava_SYT2 | 61 | 57 | 64 | 53 | 64 | 62 | 55 | 56 | 54 | 52 | 59 | 50 | 41 | 56 | 75 | 51 | 56 | 76 | | 39 | 37 | 68 | 69 | 69 | 39 | 62 | 39 | 44 | 51 | 38 | 72 | 41 | 68 | 40 | 46 | 46 |
| 20. Glyma_SYT1.1 | 49 | 79 | 55 | 79 | 51 | 56 | 71 | 47 | 50 | 81 | 51 | 45 | 33 | 79 | 60 | 67 | 53 | 58 | 57 | | 73 | 38 | 39 | 39 | 73 | 39 | 71 | 34 | 34 | 66 | 43 | 79 | 41 | 51 | 36 | 32 |
| 21. Glyma_SYT1.2 | 50 | 74 | 53 | 77 | 53 | 50 | 71 | 51 | 49 | 75 | 53 | 50 | 33 | 79 | 56 | 67 | 48 | 55 | 50 | 83 | | 39 | 41 | 41 | 71 | 44 | 65 | 35 | 34 | 62 | 39 | 74 | 39 | 50 | 38 | 37 |
| 22. Glyma_SYT2.1 | 61 | 59 | 75 | 54 | 67 | 72 | 54 | 52 | 61 | 55 | 67 | 50 | 35 | 61 | 89 | 55 | 63 | 80 | 75 | 56 | 53 | | 97 | 97 | 42 | 75 | 38 | 46 | 50 | 40 | 75 | 42 | 84 | 41 | 47 | 48 |
| 23. Glyma_SYT2.2 | 59 | 61 | 74 | 52 | 68 | 72 | 53 | 55 | 61 | 53 | 67 | 51 | 42 | 61 | 87 | 56 | 63 | 81 | 77 | 58 | 54 | 98 | | ## | 41 | 73 | 37 | 48 | 51 | 41 | 75 | 41 | 84 | 40 | 47 | 49 |
| 24. Glyso_SYT2 | 59 | 61 | 74 | 52 | 68 | 72 | 53 | 55 | 61 | 53 | 67 | 51 | 42 | 61 | 87 | 56 | 63 | 81 | 77 | 58 | 54 | 98 | ## | | 41 | 73 | 37 | 48 | 51 | 41 | 75 | 41 | 84 | 40 | 47 | 49 |
| 25. Goshi_SYT1 | 45 | 81 | 57 | 78 | 49 | 55 | 70 | 46 | 50 | 76 | 49 | 45 | 31 | 90 | 61 | 75 | 48 | 55 | 53 | 81 | 79 | 59 | 56 | 56 | | 44 | 71 | 35 | 34 | 68 | 45 | 73 | 42 | 53 | 36 | 35 |
| 26. Goshi_SYT2 | 59 | 61 | 73 | 55 | 65 | 73 | 57 | 57 | 54 | 52 | 69 | 47 | 39 | 61 | 87 | 58 | 57 | 78 | 72 | 57 | 56 | 86 | 85 | 85 | 60 | | 40 | 46 | 50 | 39 | 73 | 42 | 73 | 41 | 45 | 46 |
| 27. Helan_SYT1 | 54 | 77 | 58 | 81 | 52 | 53 | 71 | 52 | 47 | 81 | 49 | 45 | 35 | 82 | 58 | 68 | 53 | 56 | 54 | 82 | 79 | 53 | 53 | 53 | 82 | 57 | | 33 | 37 | 66 | 45 | 68 | 40 | 50 | 35 | 37 |
| 28. Horvu_SYT2 | 61 | 51 | 51 | 47 | 53 | 53 | 48 | 50 | 74 | 48 | 56 | 48 | 47 | 46 | 54 | 44 | 55 | 58 | 56 | 45 | 45 | 52 | 57 | 57 | 45 | 54 | 44 | | 39 | 35 | 45 | 37 | 45 | 33 | 78 | 69 |
| 29. Lacse_SYT2 | 50 | 51 | 54 | 47 | 59 | 52 | 46 | 52 | 51 | 50 | 61 | 45 | 39 | 49 | 60 | 45 | 49 | 63 | 58 | 47 | 47 | 59 | 60 | 60 | 47 | 62 | 52 | 51 | | 35 | 48 | 38 | 48 | 36 | 41 | 43 |
| 30. Lyces_SYT1 | 48 | 75 | 53 | 81 | 51 | 56 | 65 | 49 | 51 | 80 | 51 | 46 | 36 | 77 | 57 | 64 | 46 | 53 | 56 | 78 | 74 | 56 | 57 | 57 | 75 | 54 | 79 | 49 | 49 | | 40 | 64 | 41 | 47 | 39 | 36 |
| 31. Maldo_SYT2 | 55 | 60 | 70 | 56 | 66 | 70 | 59 | 56 | 58 | 53 | 68 | 49 | 36 | 61 | 84 | 56 | 59 | 80 | 79 | 59 | 53 | 82 | 82 | 82 | 60 | 83 | 61 | 57 | 59 | 58 | | 45 | 75 | 41 | 46 | 45 |
| 32. Medtr_SYT1 | 53 | 83 | 62 | 78 | 54 | 56 | 72 | 48 | 51 | 78 | 53 | 44 | 39 | 83 | 65 | 68 | 51 | 52 | 54 | 90 | 83 | 57 | 58 | 58 | 82 | 60 | 80 | 51 | 51 | 77 | 58 | | 43 | 53 | 39 | 35 |
| 33. Medtr_SYT2 | 59 | 60 | 73 | 55 | 69 | 70 | 56 | 56 | 58 | 54 | 70 | 50 | 39 | 61 | 84 | 58 | 62 | 81 | 75 | 58 | 56 | 90 | 89 | 89 | 58 | 85 | 56 | 53 | 57 | 60 | 82 | 60 | | 40 | 49 | 49 |
| 34. Orysa_SYT1 | 49 | 65 | 55 | 61 | 48 | 51 | 71 | 47 | 46 | 60 | 47 | 49 | 34 | 67 | 52 | 65 | 48 | 52 | 51 | 62 | 60 | 54 | 52 | 52 | 66 | 54 | 62 | 43 | 43 | 60 | 53 | 61 | 52 | | 35 | 36 |
| 35. Orysa_SYT2 | 62 | 48 | 53 | 47 | 55 | 50 | 51 | 50 | 72 | 49 | 53 | 49 | 43 | 47 | 52 | 48 | 56 | 58 | 55 | 48 | 48 | 55 | 56 | 56 | 45 | 55 | 46 | 84 | 53 | 50 | 55 | 50 | 58 | 46 | | 68 |
| 36. Orysa_SYT3 | 63 | 51 | 58 | 48 | 58 | 50 | 45 | 49 | 87 | 49 | 54 | 51 | 37 | 46 | 58 | 44 | 60 | 58 | 56 | 48 | 48 | 57 | 60 | 60 | 47 | 55 | 48 | 76 | 51 | 49 | 54 | 51 | 59 | 47 | 74 | |

| | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 |
|---|---|---|---|---|---|---|---|---|---|---|---|---|---|---|---|---|---|---|---|---|---|---|---|---|---|---|---|---|---|---|---|---|---|---|---|
| 1. Alice_SYT2 | 51 | 37 | 39 | 38 | 38 | 32 | 34 | 34 | 48 | 36 | 47 | 32 | 49 | 49 | 34 | 31 | 46 | 31 | 50 | 50 | 37 | 36 | 35 | 47 | 48 | 48 | 35 | 32 | 47 | 45 | 28 | 36 | 35 | 49 | 48 |
| 2. Aqufo_SYT1 | 36 | 40 | 43 | 42 | 41 | 56 | 55 | 69 | 40 | 29 | 40 | 50 | 36 | 34 | 60 | 59 | 39 | 50 | 36 | 34 | 39 | 33 | 53 | 35 | 33 | 33 | 68 | 62 | 44 | 36 | 25 | 56 | 50 | 36 | 35 |
| 3. Aqufo_SYT2 | 47 | 37 | 40 | 41 | 40 | 45 | 47 | 42 | 61 | 39 | 62 | 39 | 46 | 46 | 40 | 40 | 62 | 39 | 46 | 44 | 46 | 45 | 43 | 44 | 47 | 47 | 41 | 39 | 63 | 58 | 29 | 44 | 39 | 43 | 44 |
| **4. Arath_SYT1** | **35** | **37** | **36** | **40** | **40** | **50** | **52** | **66** | **37** | **31** | **38** | **45** | **35** | **32** | **72** | **70** | **39** | **46** | **33** | **32** | **37** | **35** | **50** | **34** | **34** | **34** | **62** | **56** | **40** | **39** | **25** | **47** | **46** | **34** | **32** |
| 5. Arath_SYT2 | 45 | 33 | 32 | 36 | 37 | 37 | 37 | 39 | 53 | 38 | 54 | 36 | 45 | 43 | 36 | 36 | 49 | 36 | 46 | 45 | 50 | 44 | 35 | 45 | 46 | 46 | 39 | 36 | 56 | 53 | 29 | 36 | 36 | 46 | 42 |
| 6. Arath_SYT3 | 45 | 37 | 38 | 37 | 39 | 35 | 36 | 38 | 62 | 35 | 57 | 34 | 41 | 43 | 36 | 37 | 50 | 35 | 44 | 43 | 43 | 43 | 35 | 43 | 42 | 42 | 37 | 36 | 60 | 50 | 28 | 35 | 35 | 40 | 44 |
| 7. Aspof_SYT1 | 39 | 36 | 41 | 44 | 43 | 56 | 56 | 57 | 39 | 33 | 46 | 56 | 36 | 34 | 53 | 56 | 39 | 56 | 36 | 34 | 40 | 37 | 60 | 36 | 33 | 33 | 58 | 54 | 44 | 37 | 27 | 52 | 55 | 37 | 35 |
| 8. Betvu_SYT2 | 43 | 35 | 37 | 36 | 37 | 34 | 34 | 35 | 42 | 41 | 44 | 33 | 41 | 37 | 37 | 32 | 42 | 33 | 41 | 37 | 43 | 45 | 32 | 39 | 40 | 40 | 36 | 35 | 43 | 52 | 27 | 32 | 32 | 40 | 38 |
| 9. Bradi_SYT3 | 78 | 37 | 37 | 38 | 38 | 36 | 38 | 38 | 47 | 35 | 48 | 36 | 67 | 77 | 36 | 35 | 43 | 36 | 69 | 78 | 42 | 43 | 37 | 68 | 85 | 85 | 36 | 36 | 49 | 49 | 28 | 36 | 35 | 70 | 77 |
| 10. Brana_SYT1 | 34 | 37 | 35 | 40 | 38 | 49 | 50 | 67 | 36 | 32 | 36 | 46 | 35 | 32 | 70 | 67 | 39 | 47 | 33 | 32 | 37 | 34 | 48 | 34 | 32 | 32 | 63 | 54 | 39 | 38 | 24 | 44 | 47 | 35 | 33 |
| 11. Brana_SYT2 | 43 | 35 | 38 | 36 | 36 | 37 | 36 | 36 | 56 | 37 | 55 | 34 | 43 | 43 | 34 | 35 | 51 | 34 | 43 | 43 | 50 | 43 | 35 | 44 | 43 | 43 | 36 | 35 | 55 | 51 | 31 | 31 | 34 | 42 | 42 |
| 12. Cerri_SYT\partial | 34 | 38 | 39 | 35 | 37 | 35 | 33 | 31 | 34 | 36 | 37 | 37 | 36 | 35 | 32 | 31 | 39 | 35 | 35 | 33 | 32 | 32 | 34 | 34 | 34 | 34 | 32 | 32 | 35 | 36 | 25 | 35 | 34 | 35 | 33 |
| 13. Chlre_SYT | 32 | 26 | 26 | 26 | 24 | 29 | 29 | 24 | 27 | 30 | 28 | 22 | 32 | 32 | 26 | 25 | 29 | 22 | 31 | 28 | 30 | 28 | 26 | 33 | 32 | 32 | 24 | 23 | 28 | 30 | 48 | 28 | 21 | 31 | 31 |
| 14. Citsi_SYT1 | 39 | 39 | 40 | 44 | 42 | 57 | 57 | 82 | 41 | 27 | 41 | 50 | 33 | 37 | 68 | 70 | 37 | 50 | 37 | 36 | 37 | 36 | 52 | 35 | 36 | 36 | 80 | 68 | 46 | 40 | 24 | 53 | 50 | 38 | 36 |
| 15. Citsi_SYT2 | 49 | 41 | 42 | 42 | 41 | 45 | 46 | 44 | 79 | 40 | 79 | 37 | 44 | 46 | 42 | 41 | 68 | 38 | 44 | 43 | 51 | 46 | 41 | 44 | 48 | 48 | 43 | 40 | 79 | 59 | 27 | 39 | 37 | 45 | 44 |
| 16. Cryja_SYT | 34 | 41 | 44 | 46 | 45 | 85 | 82 | 59 | 42 | 29 | 44 | 47 | 37 | 35 | 51 | 52 | 44 | 48 | 37 | 36 | 38 | 33 | 52 | 35 | 35 | 35 | 65 | 54 | 47 | 37 | 23 | 70 | 44 | 36 | 37 |
| 17. Curlo_SYT | 47 | 37 | 38 | 33 | 34 | 38 | 40 | 32 | 49 | 31 | 47 | 36 | 48 | 49 | 34 | 34 | 40 | 37 | 49 | 48 | 41 | 34 | 37 | 45 | 48 | 48 | 36 | 33 | 47 | 44 | 24 | 39 | 35 | 46 | 47 |
| 18. Eupes_SYT2 | 48 | 39 | 40 | 39 | 40 | 44 | 44 | 44 | 73 | 40 | 70 | 40 | 47 | 45 | 41 | 43 | 63 | 40 | 49 | 45 | 54 | 47 | 41 | 48 | 46 | 46 | 43 | 42 | 73 | 58 | 25 | 41 | 40 | 50 | 44 |
| 19. Frava_SYT2 | 46 | 38 | 38 | 40 | 42 | 39 | 40 | 40 | 66 | 43 | 73 | 39 | 45 | 44 | 39 | 40 | 61 | 39 | 46 | 44 | 51 | 42 | 39 | 43 | 46 | 46 | 39 | 37 | 65 | 58 | 28 | 37 | 39 | 46 | 44 |
| 20. Glyma_SYT1.1 | 33 | 40 | 36 | 41 | 40 | 53 | 52 | 73 | 41 | 29 | 40 | 50 | 35 | 32 | 66 | 68 | 38 | 50 | 36 | 32 | 36 | 35 | 51 | 35 | 33 | 33 | 74 | 61 | 46 | 36 | 25 | 48 | 48 | 38 | 36 |
| 21. Glyma_SYT1.2 | 33 | 39 | 39 | 41 | 41 | 54 | 54 | 72 | 40 | 31 | 39 | 47 | 39 | 32 | 62 | 65 | 39 | 48 | 39 | 33 | 37 | 33 | 51 | 37 | 35 | 35 | 74 | 63 | 43 | 38 | 26 | 49 | 48 | 37 | 32 |
| 22. Glyma_SYT2.1 | 51 | 42 | 42 | 42 | 42 | 43 | 45 | 42 | 77 | 38 | 73 | 37 | 47 | 49 | 41 | 37 | 66 | 37 | 48 | 47 | 51 | 44 | 42 | 45 | 49 | 49 | 42 | 41 | 77 | 61 | 27 | 41 | 38 | 46 | 47 |
| 23. Glyma_SYT2.2 | 51 | 42 | 40 | 40 | 41 | 44 | 45 | 41 | 77 | 39 | 75 | 35 | 48 | 49 | 41 | 38 | 67 | 35 | 46 | 48 | 52 | 44 | 42 | 47 | 50 | 50 | 42 | 41 | 76 | 61 | 27 | 41 | 37 | 47 | 47 |
| 24. Glyso_SYT2 | 51 | 42 | 40 | 40 | 41 | 44 | 45 | 41 | 77 | 39 | 75 | 35 | 48 | 49 | 41 | 38 | 67 | 35 | 46 | 48 | 52 | 44 | 42 | 47 | 50 | 50 | 42 | 41 | 76 | 61 | 27 | 41 | 37 | 47 | 47 |
| 25. Goshi_SYT1 | 38 | 38 | 37 | 42 | 42 | 62 | 62 | 85 | 42 | 28 | 45 | 48 | 34 | 37 | 69 | 67 | 39 | 47 | 35 | 36 | 36 | 34 | 54 | 33 | 38 | 38 | 84 | 66 | 46 | 38 | 26 | 57 | 48 | 36 | 38 |
| 26. Goshi_SYT2 | 46 | 40 | 41 | 43 | 43 | 44 | 45 | 44 | 72 | 38 | 73 | 36 | 46 | 47 | 40 | 40 | 63 | 36 | 47 | 46 | 50 | 43 | 40 | 45 | 47 | 47 | 42 | 39 | 73 | 58 | 27 | 41 | 37 | 47 | 45 |
| 27. Helan_SYT1 | 37 | 38 | 39 | 43 | 42 | 53 | 53 | 70 | 41 | 30 | 42 | 50 | 35 | 35 | 65 | 67 | 37 | 50 | 35 | 35 | 40 | 36 | 51 | 32 | 35 | 35 | 67 | 58 | 43 | 39 | 25 | 47 | 49 | 36 | 36 |
| 28. Horvu_SYT2 | 63 | 34 | 36 | 38 | 36 | 35 | 36 | 35 | 45 | 35 | 46 | 31 | 76 | 65 | 35 | 34 | 44 | 31 | 77 | 65 | 40 | 40 | 34 | 97 | 67 | 67 | 37 | 34 | 46 | 48 | 28 | 34 | 32 | 76 | 65 |
| 29. Lacse_SYT2 | 40 | 32 | 37 | 35 | 36 | 33 | 36 | 33 | 51 | 36 | 49 | 32 | 42 | 38 | 35 | 35 | 47 | 32 | 42 | 39 | 89 | 38 | 34 | 39 | 40 | 40 | 35 | 35 | 49 | 42 | 25 | 33 | 34 | 42 | 40 |
| 30. Lyces_SYT1 | 35 | 41 | 37 | 38 | 36 | 51 | 51 | 65 | 41 | 31 | 37 | 45 | 38 | 34 | 97 | 70 | 41 | 45 | 38 | 35 | 35 | 36 | 49 | 36 | 36 | 36 | 64 | 58 | 43 | 36 | 26 | 47 | 45 | 37 | 34 |
| 31. Maldo_SYT2 | 46 | 39 | 41 | 42 | 40 | 44 | 45 | 44 | 74 | 38 | 85 | 40 | 45 | 44 | 40 | 40 | 65 | 40 | 46 | 45 | 52 | 46 | 41 | 43 | 47 | 47 | 43 | 42 | 74 | 58 | 30 | 41 | 39 | 43 | 46 |
| 32. Medtr_SYT1 | 37 | 40 | 40 | 42 | 41 | 56 | 58 | 74 | 43 | 31 | 44 | 51 | 35 | 34 | 64 | 67 | 44 | 52 | 36 | 35 | 39 | 36 | 53 | 36 | 36 | 36 | 73 | 61 | 51 | 41 | 24 | 51 | 51 | 39 | 36 |
| 33. Medtr_SYT2 | 51 | 40 | 41 | 41 | 40 | 44 | 46 | 44 | 77 | 41 | 75 | 36 | 47 | 47 | 41 | 41 | 67 | 37 | 48 | 48 | 50 | 45 | 41 | 47 | 50 | 50 | 41 | 41 | 76 | 61 | 28 | 42 | 37 | 46 | 48 |
| 34. Orysa_SYT1 | 37 | 36 | 37 | 38 | 37 | 52 | 52 | 53 | 37 | 28 | 41 | 82 | 32 | 34 | 48 | 53 | 38 | 82 | 32 | 35 | 37 | 36 | 88 | 33 | 36 | 36 | 53 | 49 | 43 | 36 | 24 | 48 | 83 | 31 | 37 |
| 35. Orysa_SYT2 | 62 | 38 | 35 | 38 | 37 | 37 | 37 | 36 | 45 | 38 | 48 | 35 | 75 | 63 | 38 | 35 | 43 | 35 | 76 | 63 | 43 | 39 | 36 | 77 | 65 | 65 | 36 | 36 | 47 | 47 | 26 | 37 | 34 | 75 | 62 |
| 36. Orysa_SYT3 | 78 | 39 | 39 | 38 | 39 | 36 | 38 | 35 | 46 | 34 | 47 | 40 | 68 | 79 | 36 | 37 | 44 | 39 | 67 | 79 | 43 | 43 | 36 | 70 | 75 | 75 | 34 | 37 | 48 | 47 | 26 | 36 | 38 | 67 | 80 |

| | | | | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|
| 37. Panvi_SYT3 | 63 | 51 | 56 | 49 | 53 | 56 | 51 | 53 | 84 | 52 | 55 | 45 | 44 | 54 | 59 | 47 | 60 | 58 | 56 | 46 | 46 | 62 | 62 | 62 | 51 | 59 | 51 | 72 | 51 | 50 | 57 | 52 | 62 | 52 | 70 | 85 |
| 38. Phypa_SYT1.1 | 53 | 57 | 49 | 58 | 45 | 51 | 51 | 44 | 48 | 56 | 49 | 50 | 38 | 57 | 54 | 54 | 52 | 50 | 48 | 60 | 55 | 57 | 57 | 57 | 53 | 53 | 54 | 49 | 44 | 58 | 52 | 56 | 55 | 48 | 46 | 53 |
| 39. Phypa_SYT1.2 | 51 | 60 | 51 | 52 | 46 | 48 | 55 | 50 | 51 | 56 | 51 | 49 | 38 | 56 | 54 | 55 | 53 | 50 | 49 | 53 | 52 | 54 | 54 | 54 | 53 | 52 | 56 | 47 | 49 | 54 | 54 | 54 | 52 | 48 | 45 | 54 |
| 40. Phypa_SYT1.3 | 51 | 59 | 52 | 56 | 48 | 49 | 57 | 47 | 49 | 54 | 50 | 48 | 35 | 58 | 55 | 59 | 47 | 50 | 50 | 57 | 57 | 54 | 50 | 50 | 57 | 57 | 57 | 47 | 43 | 50 | 53 | 56 | 52 | 49 | 45 | 51 |
| 41. Phypa_SYT1.4 | 51 | 59 | 49 | 56 | 50 | 50 | 56 | 46 | 53 | 52 | 50 | 47 | 34 | 58 | 54 | 57 | 47 | 51 | 52 | 56 | 56 | 56 | 53 | 53 | 57 | 55 | 58 | 47 | 46 | 48 | 49 | 55 | 54 | 50 | 47 | 51 |
| 42. Picsi_SYT1 | 46 | 71 | 57 | 64 | 49 | 48 | 67 | 42 | 47 | 63 | 48 | 45 | 38 | 70 | 59 | 90 | 51 | 54 | 52 | 67 | 67 | 55 | 55 | 55 | 75 | 59 | 67 | 45 | 42 | 64 | 57 | 67 | 57 | 65 | 48 | 47 |
| 43. Pinta_SYT1 | 48 | 71 | 59 | 65 | 49 | 50 | 67 | 43 | 49 | 62 | 48 | 44 | 39 | 71 | 58 | 87 | 53 | 55 | 53 | 67 | 66 | 56 | 54 | 54 | 76 | 59 | 67 | 45 | 43 | 65 | 58 | 68 | 59 | 63 | 46 | 48 |
| 44. Poptr_SYT1 | 50 | 83 | 58 | 80 | 54 | 53 | 70 | 46 | 48 | 79 | 51 | 46 | 35 | 90 | 61 | 72 | 47 | 56 | 54 | 82 | 80 | 57 | 56 | 56 | 91 | 61 | 83 | 45 | 47 | 76 | 59 | 83 | 61 | 66 | 46 | 46 |
| 45. Poptr_SYT2 | 61 | 60 | 72 | 54 | 64 | 71 | 56 | 54 | 57 | 56 | 71 | 43 | 39 | 60 | 86 | 56 | 63 | 81 | 75 | 61 | 55 | 83 | 83 | 83 | 57 | 83 | 61 | 55 | 62 | 58 | 80 | 60 | 83 | 51 | 56 | 56 |
| 46. Poptr_SYT3 | 45 | 40 | 46 | 43 | 49 | 42 | 41 | 50 | 43 | 45 | 44 | 49 | 39 | 37 | 46 | 38 | 41 | 47 | 52 | 41 | 41 | 47 | 48 | 48 | 39 | 46 | 41 | 46 | 45 | 42 | 46 | 41 | 49 | 37 | 44 | 42 |
| 47. Prupe_SYT2 | 56 | 59 | 72 | 56 | 63 | 67 | 62 | 55 | 58 | 54 | 67 | 48 | 36 | 58 | 82 | 57 | 60 | 76 | 80 | 58 | 53 | 80 | 82 | 82 | 60 | 82 | 58 | 53 | 59 | 51 | 87 | 60 | 83 | 54 | 56 | 56 |
| 48. Sacof_SYT1 | 49 | 64 | 53 | 58 | 47 | 50 | 68 | 48 | 50 | 58 | 48 | 52 | 33 | 64 | 50 | 61 | 50 | 52 | 51 | 63 | 58 | 52 | 50 | 50 | 62 | 52 | 59 | 41 | 43 | 60 | 51 | 61 | 52 | 88 | 44 | 51 |
| 49. Sacof_SYT2 | 59 | 49 | 51 | 50 | 55 | 49 | 48 | 53 | 72 | 50 | 54 | 50 | 44 | 46 | 54 | 46 | 57 | 58 | 55 | 48 | 51 | 54 | 55 | 55 | 46 | 55 | 46 | 86 | 56 | 53 | 53 | 46 | 53 | 45 | 84 | 72 |
| 50. Sacof_SYT3 | 60 | 49 | 56 | 48 | 53 | 56 | 47 | 47 | 80 | 50 | 57 | 44 | 46 | 51 | 55 | 48 | 61 | 53 | 53 | 47 | 49 | 58 | 58 | 58 | 51 | 58 | 48 | 71 | 46 | 52 | 54 | 48 | 56 | 45 | 70 | 84 |
| 51. Soltu_SYT1 | 51 | 75 | 55 | 80 | 49 | 56 | 62 | 48 | 50 | 79 | 48 | 46 | 36 | 78 | 58 | 64 | 50 | 54 | 52 | 79 | 70 | 56 | 55 | 55 | 76 | 56 | 77 | 49 | 47 | 97 | 54 | 77 | 58 | 60 | 49 | 49 |
| 52. Soltu_SYT2 | 47 | 74 | 56 | 80 | 54 | 56 | 65 | 47 | 50 | 78 | 53 | 45 | 34 | 81 | 58 | 65 | 51 | 57 | 54 | 80 | 75 | 51 | 56 | 56 | 81 | 56 | 78 | 46 | 49 | 77 | 55 | 74 | 56 | 64 | 47 | 49 |
| 53. Soltu_SYT3 | 58 | 57 | 70 | 54 | 61 | 59 | 52 | 53 | 55 | 56 | 64 | 49 | 38 | 52 | 75 | 53 | 53 | 74 | 72 | 53 | 53 | 75 | 74 | 74 | 50 | 75 | 55 | 54 | 58 | 58 | 76 | 56 | 75 | 47 | 53 | 57 |
| 54. Sorbi_SYT1 | 49 | 63 | 54 | 60 | 46 | 50 | 68 | 44 | 49 | 58 | 48 | 51 | 33 | 64 | 50 | 62 | 52 | 52 | 51 | 64 | 56 | 52 | 50 | 50 | 60 | 52 | 59 | 42 | 42 | 59 | 51 | 61 | 52 | 88 | 44 | 51 |
| 55. Sorbi_SYT2 | 61 | 50 | 52 | 47 | 57 | 54 | 48 | 53 | 73 | 50 | 53 | 49 | 43 | 47 | 55 | 46 | 58 | 60 | 57 | 48 | 51 | 56 | 54 | 54 | 45 | 57 | 46 | 87 | 52 | 52 | 56 | 48 | 56 | 45 | 86 | 71 |
| 56. Sorbi_SYT3 | 62 | 50 | 55 | 48 | 53 | 55 | 48 | 46 | 82 | 50 | 56 | 48 | 38 | 51 | 53 | 48 | 60 | 54 | 52 | 46 | 45 | 58 | 58 | 58 | 50 | 57 | 48 | 72 | 48 | 52 | 54 | 50 | 59 | 45 | 71 | 84 |
| 57. Tarof_SYT2 | 47 | 52 | 55 | 51 | 61 | 51 | 52 | 51 | 48 | 52 | 60 | 47 | 40 | 50 | 62 | 49 | 51 | 65 | 60 | 51 | 49 | 60 | 61 | 61 | 50 | 63 | 54 | 50 | 92 | 46 | 62 | 52 | 58 | 47 | 52 | 49 |
| 58. Tarof_SYT3 | 50 | 51 | 57 | 50 | 54 | 54 | 50 | 56 | 57 | 49 | 56 | 45 | 37 | 51 | 60 | 47 | 48 | 55 | 52 | 52 | 49 | 57 | 56 | 56 | 50 | 57 | 53 | 51 | 46 | 52 | 58 | 54 | 57 | 46 | 47 | 57 |
| 59. Triae_SYT1 | 51 | 65 | 57 | 64 | 48 | 50 | 72 | 48 | 47 | 61 | 48 | 48 | 39 | 68 | 54 | 67 | 49 | 50 | 51 | 65 | 63 | 56 | 54 | 54 | 67 | 56 | 63 | 44 | 43 | 65 | 54 | 64 | 54 | 92 | 47 | 47 |
| 60. Triae_SYT2 | 61 | 50 | 51 | 46 | 55 | 53 | 49 | 51 | 74 | 48 | 55 | 48 | 47 | 46 | 53 | 45 | 54 | 58 | 54 | 46 | 47 | 52 | 56 | 56 | 44 | 54 | 46 | 99 | 50 | 50 | 53 | 48 | 55 | 43 | 84 | 75 |
| 61. Triae_SYT3 | 60 | 49 | 57 | 49 | 58 | 52 | 46 | 50 | 90 | 48 | 56 | 47 | 44 | 48 | 60 | 44 | 60 | 59 | 56 | 49 | 49 | 59 | 60 | 60 | 51 | 61 | 49 | 75 | 50 | 51 | 59 | 51 | 60 | 46 | 74 | 82 |
| 62. Triae_SYT3.2 | 60 | 49 | 57 | 49 | 58 | 52 | 46 | 50 | 90 | 48 | 56 | 47 | 44 | 48 | 60 | 44 | 60 | 59 | 56 | 49 | 49 | 59 | 60 | 60 | 51 | 61 | 49 | 75 | 50 | 51 | 59 | 51 | 60 | 46 | 74 | 82 |
| 63. Vitvi_SYT1.1 | 50 | 81 | 57 | 76 | 53 | 54 | 72 | 46 | 48 | 77 | 51 | 45 | 35 | 90 | 59 | 76 | 50 | 55 | 52 | 82 | 83 | 56 | 59 | 59 | 91 | 58 | 82 | 47 | 48 | 74 | 60 | 83 | 52 | 65 | 46 | 45 |
| 64. Vitvi_SYT1.2 | 44 | 76 | 55 | 70 | 51 | 54 | 65 | 50 | 47 | 67 | 53 | 49 | 32 | 80 | 56 | 67 | 51 | 55 | 50 | 73 | 76 | 56 | 56 | 56 | 78 | 56 | 73 | 44 | 49 | 69 | 56 | 75 | 59 | 62 | 47 | 47 |
| 65. Vitvi_SYT2.1 | 59 | 61 | 75 | 57 | 67 | 73 | 57 | 54 | 60 | 54 | 68 | 47 | 37 | 65 | 83 | 60 | 60 | 79 | 70 | 64 | 55 | 83 | 82 | 82 | 63 | 82 | 58 | 55 | 59 | 56 | 78 | 65 | 84 | 57 | 55 | 58 |
| 66. Vitvi_SYT2.2 | 56 | 49 | 64 | 53 | 67 | 61 | 49 | 64 | 55 | 55 | 63 | 50 | 41 | 54 | 68 | 49 | 54 | 67 | 65 | 51 | 49 | 70 | 70 | 70 | 49 | 68 | 53 | 56 | 51 | 50 | 68 | 56 | 69 | 47 | 53 | 53 |
| 67. Volca_SYT | 39 | 34 | 41 | 38 | 39 | 39 | 38 | 37 | 42 | 36 | 42 | 37 | 54 | 38 | 37 | 39 | 38 | 34 | 39 | 38 | 39 | 37 | 38 | 38 | 37 | 37 | 36 | 42 | 35 | 39 | 40 | 36 | 39 | 35 | 38 | 40 |
| 68. Welmi_SYT1 | 54 | 71 | 60 | 64 | 53 | 53 | 66 | 47 | 47 | 61 | 48 | 49 | 36 | 71 | 54 | 83 | 50 | 56 | 51 | 65 | 64 | 58 | 57 | 57 | 74 | 57 | 66 | 45 | 45 | 63 | 59 | 66 | 60 | 64 | 48 | 47 |
| 69. Zeama_SYT1 | 49 | 62 | 53 | 59 | 45 | 50 | 68 | 43 | 45 | 58 | 48 | 48 | 32 | 63 | 50 | 57 | 49 | 51 | 51 | 60 | 60 | 52 | 50 | 50 | 62 | 52 | 59 | 43 | 42 | 58 | 52 | 61 | 52 | 88 | 44 | 50 |
| 70. Zeama_SYT2 | 59 | 50 | 48 | 46 | 54 | 48 | 49 | 52 | 74 | 50 | 51 | 51 | 45 | 50 | 52 | 45 | 55 | 60 | 57 | 50 | 47 | 55 | 55 | 55 | 47 | 55 | 48 | 83 | 53 | 51 | 53 | 48 | 53 | 45 | 84 | 72 |
| 71. Zeama_SYT3 | 58 | 49 | 55 | 47 | 50 | 54 | 46 | 46 | 80 | 49 | 52 | 42 | 41 | 51 | 53 | 50 | 59 | 52 | 54 | 49 | 46 | 56 | 56 | 56 | 52 | 57 | 49 | 69 | 49 | 48 | 54 | 51 | 57 | 48 | 68 | 84 |

| | | | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|
| 37. Panvi_SYT3 | | 39 | 36 | 39 | 39 | 37 | 37 | 36 | 49 | 38 | 48 | 36 | 66 | 83 | 37 | 36 | 45 | 36 | 68 | 83 | 42 | 40 | 36 | 64 | 71 | 71 | 35 | 39 | 50 | 47 | 28 | 36 | 37 | 66 | 82 |
| 38. Phypa_SYT1.1 | 50 | | 82 | 50 | 51 | 42 | 44 | 42 | 39 | 35 | 39 | 34 | 35 | 38 | 42 | 40 | 40 | 34 | 36 | 36 | 34 | 33 | 37 | 33 | 36 | 36 | 39 | 38 | 41 | 38 | 24 | 42 | 35 | 36 | 39 |
| 39. Phypa_SYT1.2 | 47 | 87 | | 51 | 53 | 43 | 44 | 40 | 41 | 36 | 42 | 37 | 36 | 37 | 39 | 40 | 38 | 36 | 36 | 38 | 38 | 32 | 41 | 33 | 36 | 36 | 40 | 42 | 42 | 39 | 26 | 44 | 35 | 36 | 36 |
| 40. Phypa_SYT1.3 | 51 | 65 | 65 | | 93 | 45 | 46 | 43 | 41 | 35 | 40 | 38 | 39 | 41 | 39 | 38 | 39 | 39 | 38 | 39 | 39 | 33 | 42 | 38 | 34 | 34 | 43 | 42 | 42 | 36 | 27 | 43 | 37 | 39 | 39 |
| 41. Phypa_SYT1.4 | 51 | 67 | 68 | 96 | | 46 | 46 | 42 | 40 | 36 | 42 | 36 | 35 | 40 | 39 | 36 | 41 | 35 | 38 | 40 | 38 | 34 | 38 | 36 | 36 | 36 | 42 | 42 | 43 | 36 | 27 | 41 | 35 | 36 | 39 |
| 42. Picsi_SYT1 | 49 | 52 | 53 | 56 | 56 | | 94 | 59 | 42 | 29 | 43 | 47 | 37 | 36 | 52 | 53 | 44 | 48 | 37 | 35 | 37 | 35 | 53 | 35 | 37 | 37 | 61 | 54 | 47 | 38 | 25 | 71 | 47 | 35 | 36 |
| 43. Pinta_SYT1 | 49 | 55 | 55 | 57 | 57 | 95 | | 58 | 42 | 30 | 44 | 47 | 38 | 37 | 51 | 54 | 44 | 48 | 37 | 35 | 36 | 36 | 52 | 36 | 40 | 40 | 60 | 54 | 48 | 40 | 26 | 69 | 46 | 36 | 39 |
| 44. Poptr_SYT1 | 49 | 59 | 56 | 58 | 56 | 72 | 71 | | 43 | 29 | 44 | 49 | 34 | 34 | 66 | 69 | 39 | 49 | 37 | 34 | 37 | 35 | 53 | 33 | 37 | 37 | 82 | 69 | 49 | 39 | 24 | 52 | 50 | 35 | 38 |
| 45. Poptr_SYT2 | 61 | 51 | 56 | 50 | 55 | 55 | 54 | 60 | | 40 | 74 | 38 | 45 | 43 | 41 | 40 | 63 | 37 | 45 | 43 | 51 | 43 | 39 | 46 | 47 | 47 | 40 | 40 | 76 | 60 | 28 | 42 | 37 | 45 | 44 |
| 46. Poptr_SYT3 | 45 | 46 | 44 | 42 | 43 | 38 | 39 | 36 | 48 | | 39 | 26 | 37 | 35 | 29 | 28 | 40 | 26 | 39 | 34 | 40 | 37 | 28 | 35 | 34 | 34 | 30 | 30 | 40 | 44 | 25 | 28 | 27 | 39 | 34 |
| 47. Prupe_SYT2 | 57 | 51 | 53 | 50 | 50 | 56 | 56 | 59 | 78 | 47 | | 39 | 46 | 46 | 37 | 40 | 67 | 38 | 47 | 46 | 51 | 45 | 42 | 46 | 46 | 46 | 42 | 40 | 77 | 60 | 27 | 40 | 38 | 46 | 46 |
| 48. Sacof_SYT1 | 47 | 48 | 50 | 50 | 48 | 60 | 62 | 63 | 52 | 37 | 52 | | 35 | 35 | 45 | 50 | 38 | 98 | 34 | 37 | 36 | 33 | 79 | 33 | 34 | 34 | 48 | 48 | 39 | 35 | 23 | 47 | 95 | 34 | 35 |
| 49. Sacof_SYT2 | 73 | 45 | 49 | 50 | 47 | 45 | 46 | 47 | 53 | 44 | 54 | 44 | | 64 | 38 | 36 | 43 | 32 | 96 | 65 | 44 | 41 | 32 | 76 | 64 | 64 | 34 | 37 | 48 | 44 | 27 | 34 | 32 | 90 | 64 |
| 50. Sacof_SYT3 | 87 | 51 | 47 | 52 | 52 | 49 | 50 | 48 | 52 | 44 | 55 | 48 | 70 | | 33 | 35 | 44 | 35 | 66 | 95 | 38 | 40 | 34 | 65 | 73 | 73 | 33 | 37 | 48 | 45 | 29 | 36 | 35 | 64 | 90 |
| 51. Soltu_SYT1 | 53 | 59 | 53 | 51 | 53 | 65 | 63 | 77 | 58 | 40 | 53 | 59 | 51 | 48 | | 73 | 39 | 45 | 39 | 36 | 35 | 36 | 49 | 36 | 36 | 36 | 64 | 57 | 44 | 36 | 27 | 47 | 43 | 37 | 35 |
| 52. Soltu_SYT2 | 53 | 59 | 56 | 50 | 48 | 65 | 67 | 82 | 58 | 39 | 56 | 63 | 48 | 50 | 80 | | 39 | 50 | 33 | 34 | 36 | 36 | 52 | 34 | 34 | 34 | 65 | 56 | 47 | 37 | 24 | 49 | 48 | 33 | 36 |
| 53. Soltu_SYT3 | 57 | 51 | 50 | 50 | 52 | 53 | 53 | 53 | 74 | 49 | 74 | 50 | 54 | 55 | 54 | 54 | | 38 | 43 | 43 | 49 | 44 | 37 | 44 | 44 | 44 | 39 | 37 | 65 | 55 | 26 | 41 | 35 | 44 | 43 |
| 54. Sorbi_SYT1 | 47 | 48 | 48 | 51 | 47 | 62 | 62 | 63 | 51 | 37 | 52 | 99 | 45 | 48 | 58 | 62 | 50 | | 34 | 36 | 35 | 33 | 79 | 33 | 35 | 35 | 50 | 48 | 39 | 35 | 23 | 47 | 95 | 33 | 33 |
| 55. Sorbi_SYT2 | 74 | 46 | 50 | 50 | 48 | 45 | 46 | 48 | 53 | 46 | 54 | 46 | 97 | 71 | 51 | 46 | 53 | 46 | | 67 | 44 | 41 | 32 | 78 | 66 | 66 | 34 | 36 | 48 | 44 | 25 | 35 | 34 | 92 | 65 |
| 56. Sorbi_SYT3 | 86 | 48 | 48 | 53 | 52 | 49 | 48 | 48 | 54 | 44 | 54 | 48 | 70 | 97 | 52 | 50 | 55 | 48 | 73 | | 40 | 42 | 34 | 66 | 74 | 74 | 34 | 39 | 47 | 46 | 28 | 37 | 35 | 65 | 91 |
| 57. Tarof_SYT2 | 49 | 44 | 50 | 47 | 47 | 46 | 45 | 50 | 62 | 50 | 61 | 46 | 54 | 44 | 45 | 48 | 60 | 45 | 52 | 46 | | 40 | 34 | 40 | 41 | 41 | 36 | 36 | 50 | 46 | 25 | 37 | 35 | 43 | 39 |
| 58. Tarof_SYT3 | 54 | 47 | 47 | 46 | 47 | 46 | 47 | 50 | 58 | 44 | 54 | 46 | 53 | 54 | 51 | 50 | 55 | 46 | 52 | 57 | 47 | | 37 | 40 | 41 | 41 | 35 | 37 | 45 | 51 | 29 | 34 | 35 | 41 | 40 |
| 59. Triae_SYT1 | 51 | 51 | 53 | 54 | 52 | 67 | 67 | 67 | 52 | 38 | 56 | 87 | 47 | 46 | 64 | 66 | 49 | 86 | 43 | 47 | 44 | 52 | | 34 | 36 | 36 | 52 | 50 | 43 | 36 | 22 | 49 | 77 | 31 | 37 |
| 60. Triae_SYT2 | 72 | 47 | 44 | 48 | 47 | 46 | 46 | 45 | 55 | 45 | 53 | 44 | 84 | 71 | 50 | 45 | 54 | 44 | 86 | 71 | 49 | 49 | 44 | | 67 | 67 | 36 | 33 | 47 | 49 | 27 | 33 | 34 | 76 | 65 |
| 61. Triae_SYT3 | 80 | 47 | 50 | 46 | 51 | 47 | 50 | 49 | 58 | 44 | 57 | 50 | 72 | 79 | 50 | 49 | 59 | 49 | 74 | 81 | 50 | 53 | 49 | 75 | | 99 | 37 | 35 | 49 | 48 | 27 | 38 | 35 | 67 | 73 |
| 62. Triae_SYT3.2 | 80 | 47 | 50 | 46 | 51 | 47 | 50 | 49 | 58 | 44 | 57 | 50 | 72 | 79 | 50 | 49 | 59 | 49 | 74 | 81 | 50 | 53 | 49 | 75 | 99 | | 37 | 35 | 49 | 48 | 27 | 38 | 35 | 67 | 73 |
| 63. Vitvi_SYT1.1 | 49 | 56 | 53 | 59 | 58 | 74 | 73 | 91 | 54 | 39 | 59 | 61 | 45 | 45 | 74 | 81 | 52 | 62 | 45 | 47 | 44 | 52 | 65 | 46 | 49 | 49 | | 69 | 45 | 38 | 25 | 56 | 49 | 34 | 36 |
| 64. Vitvi_SYT1.2 | 50 | 52 | 51 | 58 | 58 | 69 | 68 | 80 | 56 | 41 | 55 | 65 | 50 | 48 | 68 | 72 | 51 | 64 | 50 | 50 | 47 | 51 | 64 | 45 | 48 | 48 | 81 | | 43 | 39 | 24 | 48 | 48 | 37 | 39 |
| 65. Vitvi_SYT2.1 | 60 | 53 | 52 | 51 | 54 | 60 | 61 | 65 | 79 | 47 | 81 | 53 | 55 | 56 | 59 | 64 | 73 | 53 | 56 | 56 | 59 | 56 | 60 | 55 | 58 | 58 | 63 | 59 | | 60 | 32 | 45 | 39 | 46 | 47 |
| 66. Vitvi_SYT2.2 | 53 | 50 | 53 | 47 | 46 | 48 | 51 | 52 | 70 | 52 | 67 | 47 | 51 | 49 | 51 | 50 | 64 | 47 | 51 | 51 | 56 | 61 | 47 | 56 | 57 | 57 | 52 | 52 | 67 | | 26 | 37 | 34 | 48 | 47 |
| 67. Volca_SYT | 38 | 37 | 35 | 37 | 36 | 41 | 41 | 38 | 39 | 31 | 37 | 37 | 38 | 41 | 39 | 39 | 38 | 37 | 37 | 42 | 34 | 41 | 37 | 42 | 39 | 39 | 38 | 39 | 42 | 37 | | 23 | 23 | 25 | 27 |
| 68. Welmi_SYT1 | 50 | 53 | 56 | 60 | 57 | 83 | 82 | 69 | 59 | 36 | 57 | 63 | 45 | 48 | 62 | 67 | 52 | 63 | 47 | 50 | 49 | 48 | 66 | 44 | 50 | 50 | 69 | 66 | 59 | 49 | 39 | | 45 | 35 | 37 |
| 69. Zeama_SYT1 | 49 | 46 | 45 | 48 | 49 | 60 | 59 | 63 | 50 | 36 | 52 | 97 | 42 | 45 | 56 | 59 | 48 | 97 | 46 | 46 | 44 | 47 | 86 | 44 | 48 | 48 | 60 | 63 | 54 | 45 | 37 | 61 | | 33 | 35 |
| 70. Zeama_SYT2 | 71 | 44 | 47 | 51 | 49 | 45 | 45 | 46 | 52 | 46 | 52 | 46 | 93 | 68 | 50 | 45 | 54 | 44 | 94 | 69 | 53 | 51 | 44 | 83 | 74 | 74 | 44 | 48 | 52 | 56 | 36 | 44 | 45 | | 61 |
| 71. Zeama_SYT3 | 84 | 47 | 46 | 51 | 51 | 49 | 55 | 52 | 55 | 42 | 54 | 45 | 69 | 92 | 49 | 50 | 53 | 46 | 69 | 92 | 46 | 53 | 49 | 69 | 80 | 80 | 49 | 51 | 57 | 51 | 39 | 50 | 49 | 64 | |

The percentage identity between the full length SYT polypeptide sequences useful in performing the methods of the invention can be as low as 25 % amino acid identity compared to the polypeptide sequence of SEQ ID NO: 121 (see Table B.2 and Figure 6).

The percentage identity can be substantially increased if the identity calculation is performed between the SNH domain as represented by SEQ ID NO: 262 (comprised in SEQ ID NO: 121) and the SNH domains of the polypeptides useful in performing the invention. Percentage identity over the SNH domain amongst the polypeptide sequences useful in performing the methods of the invention ranges between 30 % and 99% amino acid identity.

The percentages in amino acid acid identity between the SNH domain of the polypeptides of Table A.2 are significantly higher than the percentage amino acid identity calculated between the full length SYT polypeptide sequences.

***Example 4: Identification of domains comprised in polypeptide sequences useful in performing the methods of the invention***

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 2 are presented in Table C.1.

**Table C.1:** InterPro scan results of the polypeptide sequence as represented by SEQ ID NO: 2

| InterPro accession number and name | Integrated database name | Integrated database accession number | Integrated database accession name |
|---|---|---|---|
| IPR014977 WRC domain | PFAM | PF08879 | WRC |
| IPR014978 QLQ domain | PFAM | PF08880 | QLQ |

The results of the InterPro scan of the polypeptide sequence as represented by SEQ ID NO: 121 are presented in Table C.2.

**Table C.2:** InterPro scan results of the polypeptide sequence as represented by SEQ ID NO: 2

| InterPro accession number and name | Integrated database name | Integrated database accession number | Integrated database accession name |
|---|---|---|---|
| IPR007726 SSXT domain/family | PFAM | PF05030 | SSXT protein (N-terminal region) |
| IPR007726 SSXT domain/family | Panther | PTHR23107 | SYNOVIAL SARCOMA ASSOCIATED SS18 PROTEIN |

Furthermore, the presence of a Met-rich domain or a QG-rich domain in the SYT polypeptide sequences may also readily be identified. As shown in Figure 6, the Met-rich domain and QG-rich domain follows the SNH domain. The QG-rich domain may be taken to be substantially the C-terminal remainder of the polypeptide (minus the SHN domain); the Met-rich domain is typically comprised within the first half of the QG-rich (from the N-term to the C-term) domain. Primary amino acid composition (in %) to determine if a polypeptide domain is rich in specific amino acids may be calculated using software programs from the ExPASy server (Gasteiger E et al. (2003) ExPASy: the proteomics server for in-depth protein knowledge and analysis. Nucleic Acids Res 31:3784-3788), in particular the ProtParam tool. The composition of the polypeptide of interest may then be compared to the average amino acid composition (in %) in the Swiss-Prot Protein Sequence data bank (Table C.3). Within this databank, the average Met (M) content is of 2.37%, the average Gln (Q) content is of 3.93% and the average Gly (G) content is of 6.93% (Table C.3). As defined herein, a Met-rich domain or a QG-rich domain has Met content (in %) or a Gin and Gly content (in %) above the average amino acid composition (in %) in the Swiss-Prot Protein Sequence data bank. For example in SEQ ID NO: 121, the Met-rich domain at the N-terminal preceding the SNH domain (from amino acid positions 1 to 24) has Met content of 20.8 % and a QG-rich domain (from amino acid positions 71 to 200) has a Gin (Q) content of 18.6 % and a Gly (G) content of 21.4 %.

Preferably, the Met domain as defined herein has a Met content (in %) that is at least 1.25, 1.5, 1.75, 2.0, 2.25, 2.5, 2.75, 3.0, 3.25, 3.5, 3.75, 4.0, 4.25, 4.5, 4.75, 5.0, 5.25, 5.0, 5.75, 6.0, 6.25, 6.5, 6.75, 7.0, 7.25, 7.5, 7.75, 8.0, 8.25, 8.5, 8.75, 9.0, 9.25, 9.5, 9.75, 10 or more as much as the average amino acid composition (in %) of said kind of protein sequences, which are included in the Swiss-Prot Protein Sequence data bank. Preferably, the QG-rich domain as defined herein has a Gln (Q) content and/or a Gly (G) content that is at least 1.25, 1.5, 1.75, 2.0, 2.25, 2.5, 2.75, 3.0, 3.25, 3.5, 3.75, 4.0, 4.25, 4.5, 4.75, 5.0, 5.25, 5.0, 5.75, 6.0, 6.25, 6.5, 6.75, 7.0, 7.25, 7.5, 7.75, 8.0, 8.25, 8.5, 8.75, 9.0, 9.25, 9.5, 9.75, 10 or more as much as the average amino acid composition (in %) of said kind of protein sequences, which are included in the Swiss-Prot Protein Sequence data bank.

**Table C.3:** Mean amino acid composition (%) of proteins in SWISS PROT Protein Sequence data bank (July 2004):

| **Residue** | **%** | **Residue** | **%** |
|---|---|---|---|
| A = Ala | 7.80 | M = Met | 2.37 |
| C = Cys | 1.57 | N = Asn | 4.22 |
| D=Asp | 5.30 | P = Pro | 4.85 |
| E = Glu | 6.59 | Q = Gln | 3.93 |
| F = Phe | 4.02 | R = Arg | 5.29 |
| G = Gly | 6.93 | S = Ser | 6.89 |
| H = His | 2.27 | T = Thr | 5.46 |
| I = Ile | 5.91 | V = Val | 6.69 |
| K = Lys | 5.93 | W = Trp | 1.16 |
| L = Leu | 9.62 | Y=Tyr | 3.09 |

***Example 5: Subcellular localisation prediction of the GRF polypeptide sequences useful in performing the methods of the invention***

Experimental methods for protein localization range from immunolocalization to tagging of proteins using green fluorescent protein (GFP) or beta-glucuronidase (GUS). For example, a GRF polypeptide fused to a GUS reporter gene was used to transform transiently onion epidermal cells (van der Knapp et al. (2000) Plant Phys 122: 695-704). The nucleus was identified as the subcellular compartiment of the GRF polypeptide. Such methods to identify subcellular compartmentalisation of GRF polypeptides are well known in the art.

A predicted nuclear localisation signal (NLS) was found by multiple sequence alignment, followed by eye inspection, in the WRC domain (CRRTDGKKWRC) of the GRF polypeptide of Table A. An NLS is one or more short sequences of positively charged lysines or arginines.

Computational prediction of protein localisation from sequence data was performed. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP and others.

LOCtree is an algorithm that can predict the subcellular localization and DNA-binding propensity of non-membrane proteins in non-plant and plant eukaryotes as well as prokaryotes. LOCtree classifies eukaryotic animal proteins into one of five subcellular classes, while plant proteins are classified into one of six classes and prokaryotic proteins are classified into one of three classes. Table D below shows the output of LOCtree using the polypeptide sequence information of SEQ ID NO: 2. High confidence predictions have reliability index values greater than 5.

**Table D** Output of LOCtree using the polypeptide sequence information of SEQ ID NO: 2.

| **Predicted Localization** | **Reliability index** | **Intermediate localization prediction (output of different SVMs in hierarchical tree)** | **Reliability index** |
|---|---|---|---|
| DNA binding | 6 | Not secreted, Nuclear, DNA-binding | 8,6,9 |

The predicted subcellular compartment of the GRF polypeptide as represented by SEQ ID NO: 2 using the LOCTree algorithm is the the nucleus.

***Example 6: Assay related to the polypeptide sequences useful in performing the methods of the invention***

GRF polypeptides and SYT polypeptids useful in the methods of the present invention (at least in their native form) typically, but not necessarily, have transcriptional regulatory activity and capacity to interact with other proteins. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). GRF polypeptides are capable of transcriptional activation of reporter genes in yeast cells (Kim & Kende (2004) Proc Natl Acad Sci 101(36): 13374-13379). GRF polypeptides are also capable of interacting with SYT polypeptides (also called GRF interacting factor or GIF) *in vivo* in yeast cells, using a yeast two-hybrid protein-protein interaction assay (Kim & Kende, *supra*). *In vitro* binding assays are also used to show that GRF polypeptides and SYT polypeptides are interacting partners (Kim & Kende, *supra*). The experiments described in this publication are useful in characterizing GRF polypeptides and SYT polypeptides, and are well known in the art.

***Example 7: Cloning of nucleic acid sequences useful in performing the methods of the invention***

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

*Cloning of a nucleic acid sequence as represented by SEQ ID NO: 1*

The *Arabidopsis thaliana* cDNA encoding the GRF polypeptide sequence as represented by SEQ ID NO: 2 was amplified by PCR using as template an *Arabidopsis* cDNA bank synthesized from mRNA extracted from mixed plant tissues. primer prm08136 SEQ ID NO: 42;,: 5'- ggggaccactttgtacaagaaagctgggttaaaaaccattttaacgcacg), The following primers, which include the AttB sites for Gateway recombination, were used for PCR amplification:

1) Prm 10010 (SEQ ID NO: 118, sense):
5'-GGGGACAAGTTTGTACAAAAAAGCAGGCTTAAACAATGATGAGTCTAAGTGGAAGTAG-3'
2) Prm 10011 (SEQ ID NO: 119, reverse, complementary):
5'-GGGGACCACTTTGTACAAGAAAGCTGGGTAGCTCTACTTAATTAGCTACCAG-3'

*Cloning of a nucleic acid sequence as represented by SEQ ID NO: 120*

The *Arabidopsis thaliana* cDNA encoding the SYT polypeptide sequence as represented by SEQ ID NO: 121 was amplified by PCR using as template an *Arabidopsis* cDNA bank synthesized from mRNA extracted from mixed plant tissues. The following primers, which include the AttB sites for Gateway recombination, were used for PCR amplification:

1) Prm06681 (SEQ ID NO: 265, sense):
5'-GGGGACAAGTTTGTACAAAAAAGCAGGCTTAAACA**ATG**CAACAGCACCTGATG-3'
2) Prm 06682 (SEQ ID NO: 266, reverse, complementary):
5'- GGGGACCACTTTGTACAAGAAAGCTGGGTCATCATTAAGATTCCTTGTGC-3'

PCR reactions were independently performed for SEQ ID NO: 1 and SEQ ID NO: 120, using Hifi Taq DNA polymerase in standard conditions. A PCR fragment of the expected length (including attB sites) was amplified and purified also using standard methods. The first step of the Gateway procedure, the BP reaction, was then performed, during which the PCR fragment recombined in vivo with the pDONR201 plasmid to produce, according to the Gateway terminology, an "entry done". Plasmid pDONR201 was purchased from Invitrogen, as part of the Gateway® technology.

***Example 8: Expression vector construction using the nucleic acid sequences as represented by SEQ ID NO: 1 and by SEQ ID NO: 120***

The entry clones independently comprising SEQ ID NO: 1 and SEQ ID NO: 120 were subsequently used independently in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 117) for constitutive expression was located upstream of this Gateway cassette.

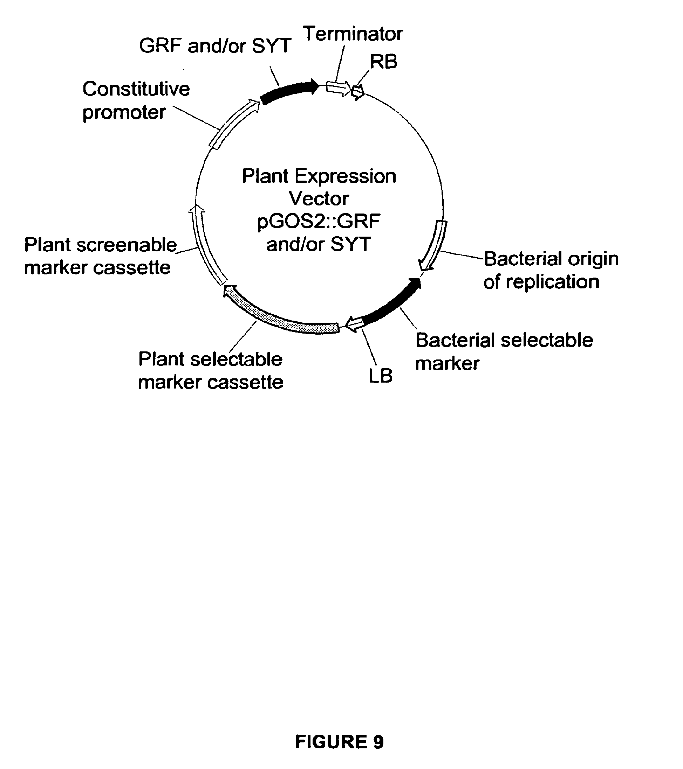

After the LR recombination step, the resulting expression vectors pGOS2::GRFand pGOS2:: SYT (Figure 9) were independently transformed into *Agrobacterium* strain LBA4044 according to methods well known in the art.

### *Example 9: Plant transformation*

*Rice transformation*

The *Agrobacterium* containing the expression vector pGOS2:: SYT was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing each individual expression vector was used independently for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for each construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

*Rice re-transformation*

By rice re-transformation is meant herein the transformation of rice plants already transgenic for another construct.

In particular, seeds harvested from transgenic homozygous plants expressing the nucleic acid sequence coding for a SYT polypeptide were re-transformed with the expression vector of Example 7. Except for this difference in initial plant source material, and the use of a different selectable marker for the re-transformation compared to the selectable marker for the initial transformation, the rest of the procedure was as described above.

### *Example 10: Phenotypic evaluation procedure*

<u>10.1 Evaluation setup</u>

Approximately 35 independent T0 rice re-transformants were generated. These plants were further transferred from a tissue culture chamber to a greenhouse for growing and harvest of T1 seed. Greenhouse conditions were of shorts days (12 hours light), 28°C in the light and 22°C in the dark, and a relative humidity of 70%.

PCR checks were performed to check for the presence of (i) the isolated nucleic acid transfene encoding a GRF polypeptide as represented by SEQ ID NO: 2; and of (ii) the isolated nucleic acid transfene encoding a SYT polypeptide as represented by SEQ ID NO: 121. PCR checks were also done for the presence and copy number of promoters, terminators and plant selectable markers. Selected transgenic plants were further grown until homozygous for both transgene loci.

10.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

10.3 Parameters measured

*Seed-related parameter measurements*

Individual seed parameters (including width, length, area) were measured using a custommade device consisting of two main components, a weighing and imaging device, coupled to software for image analysis.

***Example 11: Results of seed size measurements from seeds harvested from re-transformed rice plants***

Homozygous transgenic rice plants expressing the nucleic acid sequence coding for a SYT polypeptide as represented by SEQ ID NO: 121 under the control of a constitutive promoter were re-transformed with the expression vector of Example 7 hereinabove, comprising the nucleic acid sequence coding for the GRF polypeptide of SEQ ID NO: 2, also under the control of a constitutive promoter. The re-transformed rice plants were further grown until homozygous for both loci.

Figure 7 shows on the left a panicle from a rice plant (Oryza sativa ssp. Japonica cv. Nipponbare) transformed with a control vector, and on the right a panicle from a rice plant (Oryza sativa ssp. Japonica cv. Nipponbare) transformed with two constructs: (1) a nucleic acid sequence encoding a GRF polypeptide under the control of a GOS2 promoter (pGOS2) from rice; and (2) a nucleic acid sequence encoding a SYT polypeptide under the control of a GOS2 promoter (pGOS2) from rice. The rice plants transformed with both constructs are homozygous for both loci. Plant biomass, number of panicles, panicle size, seed number and and seed size are clearly increased in the re-transformed rice compared to the same parameters in rice transformed with a control vector.

Seeds harvested from the re-transformed rice plants, and homozygous for both loci, were harvested, and samples of 30 seeds were photographed. Figure 8 shows on the top row, from left to right, 30 mature rice seeds (Oryza sativa ssp. Japonica cv. Nipponbare) from:

(a) plants transformed with one construct comprising a nucleic acid sequence encoding a SYT polypeptide as represented by SEQ ID NO: 120, under the control of a GOS2 promoter (pGOS2) from rice;
(b) plants transformed with two constructs: (1) a nucleic acid sequence encoding a GRF polypeptide as represented by SEQ ID NO: 2, under the control of a GOS2 promoter (pGOS2) from rice; and (2) a nucleic acid sequence encoding a SYT polypeptide as represented by SEQ ID NO: 120, under the control of a GOS2 promoter (pGOS2) from rice;
(c) plants transformed with one construct comprising a nucleic acid sequence encoding a GRF polypeptide as represented by SEQ ID NO: 2, under the control of a GOS2 promoter (pGOS2) from rice;
(d) nullizygote plants (control plants) from a;
(e) nullizygote plants (control plants) from c;

An increase in seed size was visible by simple eye inspection.

The homozygous seeds from 6 transgenic events were then imaged to estimate average seed area, average seed length, and average seed width, and then compared to the ame parameters measured in (i) homozygous seeds from plants transformed with one construct comprising a nucleic acid sequence encoding a SYT polypeptide; and in (ii) seeds from control plants (nullizygotes) from (i). Results are shown in the Table E below.

**Table E:** Results of seed area, seed length and seed width measurements of seeds harvested from homozygous re-transformed rice plants relative to suitable control seeds.

| | **Compared to homozygous seeds from plants transformed with one construct comprising a nucleic acid sequence encoding a SYT polypeptide** | **Compared to seeds from control plants (nullizygotes)** |
|---|---|---|
| Seed area | At least 11 % increase | At least 26% increase |
| Seed length | At least 8% increase | At least 21 % increase |
| Seed width | At least 3% increase | At least 6% increase |

***Example 12: Examples of transformation of other crops***

*Com transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa)* is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2S04, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton *(Gossypium hirsutum* L.) transformation is performed using *Agrobacterium tumefaciens,* on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then co-cultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

## Claims

1. A method for increasing plant yield-related traits, comprising increasing expression in a plant of: (i) a nucleic acid sequence encoding a Growth-Regulating Factor (GRF) polypeptide; and of (ii) a nucleic acid sequence encoding a synovial sarcoma translocation (SYT) polypeptide, wherein said yield-related traits are increased relative to plants having increased expression of one of: (i) a nucleic acid sequence encoding a GRF polypeptide, or (ii) a nucleic acid sequence encoding a SYT polypeptide,
wherein said GRF polypeptide comprises:

   (i) a domain having at least 50%, 55%, 60%, 65%, 70%, 75%, 80%, 85%, 90%, 95%, 98%, 99% or more amino acid sequence identity to a QLQ domain as represented by SEQ ID NO: 115; and
   (ii) a domain having at least 50%, 55%, 60%, 65%, 70%, 75%, 80%, 85%, 90%, 95%, 98%, 99% or more amino acid sequence identity to a WRC domain as represented by SEQ ID NO: 116, and

   wherein said SYT polypeptide comprises from N-terminal to C-terminal:

   (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 262; and
   (ii) a Met-rich domain; and
   (iii) a QG-rich domain.

2. Method according to claim 1, wherein said GRF polypeptide comprises: (i) a QLQ domain with an InterPro accession IPR014978 (PFAM accession PF08880); (ii) a WRC domain with an InterPro accession IPR014977 (PFAM accession PF08879); and (iii) an Effector of Transcription (ET) domain comprising three Cys and one His residues in a conserved spacing ($CX_9CX_{10}CX_2H$).

3. Method according to any preceding claim, wherein said GRF polypeptide has in increasing order of preference at least 50%, 55%, 60%, 65%, 70%, 75%, 80%, 85%, 90%, 95%, 98%, 99% or more amino acid sequence identity to the GRF polypeptide as represented by SEQ ID NO: 2 or to any of the polypeptide sequences given in Table A.1 herein.

4. Method according to any preceding claim, wherein said nucleic acid sequence encoding a GRF polypeptide is represented by any one of the nucleic acid sequence SEQ ID NOs given in Table A.1 or a portion thereof, or a sequence capable of hybridising with any one of the nucleic acid sequences SEQ ID NOs given in Table A.1.

5. Method according to any preceding claim, wherein said nucleic acid sequence encodes an orthologue or paralogue of any of the GRF polypeptide sequence SEQ ID NOs given in Table A.1.

6. Method according to any preceding claim, wherein said nucleic acid sequence encoding a GRF polypeptide is operably linked to a constitutive promoter, more preferably to a GOS2 promoter, most preferably to a GOS2 promoter from rice as represented by SEQ ID NO: 117.

7. Method according to any preceding claim, wherein said nucleic acid sequence encoding a GRF polypeptide is of plant origin, preferably from a dicotyledonous plant, further preferably from the family Brassicaceae, most preferably from *Arabidopsis thaliana.*

8. Method according to claim 1, wherein said SYT polypeptide further comprises the most conserved residues of the SNH domain as represented by SEQ ID NO: 263, and shown in black in Figure 5.

9. Method according to any one of claims 1 to 8, wherein said SYT polypeptide comprises a domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SSXT domain with an InterPro accession IPR007726 of SEQ ID NO: 264.

10. Method according to any one of claims 1 to 8, wherein said SYT polypeptide has in increasing order of preference at least 20%, 25%, 30%, 35%, 40%, 45%, 50%, 55%, 60%, 65%, 70%, 75%, 80%, 85%, 90%, 95%, 98%, 99% or more amino acid sequence identity to the SYT polypeptide as represented by SEQ ID NO: 121 or to any of the full length polypeptide sequences given in Table A.2 herein.

11. Method according to any one of claims 1 to 10, wherein said nucleic acid sequence encoding a SYT polypeptide is represented by any one of the nucleic acid sequence SEQ ID NOs given in Table A.2 or a portion thereof, or a sequence capable of hybridising with any one of the nucleic acid sequences SEQ ID NOs given in Table A.2.

12. Method according to any one of claims 1 to 11, wherein said nucleic acid sequence encodes an orthologue or paralogue of any of the SYT polypeptide sequence SEQ ID NOs given in Table A.2.

13. Method according to any one of claims 1 to 12, wherein said nucleic acid sequence encoding a SYT polypeptide is operably linked to a constitutive promoter, more preferably to a GOS2 promoter, most preferably to a GOS2 promoter from rice as represented by SEQ ID NO: 117.

14. Method according to any one of claims 1 to 13, wherein said nucleic acid sequence encoding a SYT polypeptide is of plant origin, preferably from a dicotyledonous plant, further preferably from the family Brassicaceae, most preferably from *Arabidopsis thaliana.*

15. Method according to any of claims 1 to 14, wherein said increased expression is effected by introducing and expressing in a plant: (i) said nucleic acid sequence encoding said GRF polypeptide; and (ii) said nucleic acid sequence encoding said SYT polypeptide.

16. Method according to claim 15, wherein said nucleic acid sequences of (i) and (ii) are sequentially introduced and expressed in a plant, preferably by re-transformation.

17. Method according to claim 16, wherein said re-transformation is performed by introducing and expressing a nucleic acid sequence encoding GRF polypeptide into a plant, plant part, or plant cell comprising an introduced and expressing nucleic acid sequence encoding a SYT polypeptide, or reciprocally.

18. Method according to claim 15, wherein said nucleic acid sequences of (i) and (ii) are simultaneously introduced and expressed in a plant.

19. Method according to claim 18, wherein said nucleic acid sequences of (i) and (ii) are 19. Method according to claim 18, wherein said nucleic acid sequences of (i) and (ii) are comprised in one or more nucleic acid molecules.

**20.** Method according to any preceding claim, wherein said increased yield-related trait is one or more of: (i) increased early vigour; (ii) increased aboveground biomass; (iii) increased total seed yield per plant; (iv) increased seed filling rate; (v) increased number of (filled) seeds; (vi) increased harvest index; or (vii) increased thousand kernel weight (TKW).

**21.** Method according to any preceding claim, wherein said nucleic acid sequence encoding a GRF polypeptide and said nucleic acid sequence encoding a SYT polypeptide are operably and sequentially linked to a constitutive promoter, preferably to a plant constitutive promoter, more preferably to a GOS2 promoter, most preferably to a GOS2 promoter from rice as represented by SEQ ID NO: 117.

**22.** Plants, parts thereof including seeds, or plant cells obtainable by a method according to any preceding claim, wherein said plant, part or cell thereof comprises (i) an isolated nucleic acid transgene encoding a GRF polypeptide and (ii) an isolated nucleic acid transgene encoding a SYT polypeptide.

**23.** Construct comprising:

(a) a nucleic acid sequence encoding a GRF polypeptide as defined in any one of claims 1 to 5 and 7
(b) a nucleic acid sequence encoding a SYT polypeptide as defined in any one of claims 1, 8 to 12 and 14;
(c) one or more control sequences capable of driving expression of the nucleic acid sequence of (a) and of (b); and optionally
(d) a transcription termination sequence

wherein said SYT polypeptide and said GRF polypeptide are capable of interacting with one another.

**24.** Construct according to claim 23, said control sequence is at least one constitutive promoter, preferably a GOS2 promoter, more preferably a GOS2 promoter as represented by SEQ ID NO: 117.

**25.** Mixture of constructs, wherein at least one construct comprises:

(a) a nucleic acid sequence encoding a GRF polypeptide as defined in any one of claims 1 to 5 and 7;
(b) one or more control sequences capable of driving expression of the nucleic acid sequence of (a); and optionally
(c) a transcription termination sequence,

and wherein at least one other construct comprises:

(d) a nucleic acid sequence encoding a SYT polypeptide as defined in any one of claims 1, 8 to 12 and 14;
(e) one or more control sequences capable of driving expression of the nucleic acid sequence of (d); and optionally
(f) a transcription termination sequence.

wherein said SYT polypeptide and said GRF polypeptide are capable of interacting with one another.

**26.** Construct according to claim 25, wherein said control sequence of (b) and/or (e) is at least one constitutive promoter, preferably a GOS2 promoter, more preferably a GOS2 promoter as represented by SEQ ID NO: 117.

**27.** Use of a construct according to 23 or 24, or of a mixture of constructs according 27. Use of a construct according to claims 23 or 24, or of a mixture of constructs according to claim 25, in a method for making plants having increased yield-related traits relative to plants having increased expression of one of: (a) a nucleic acid sequence encoding a GRF polypeptide, or (b) a nucleic acid sequence encoding a SYT polypeptide, which increased yield-related traits are one or more of: (i) increased early vigour; (ii) increased aboveground biomass; (iii) increased total seed yield per plant; (iv) increased seed filling rate; (v) increased number of (filled) seeds; (vi) increased harvest index; or (vii) increased thousand kernel weight (TKW).

**28.** Plant, plant part or plant cell transformed with a construct according to claims 23 or 24, or of a mixture of constructs according to claim 25.

**29.** Method for the production of transgenic plants having increased yield-related traits relative to plants having increased expression of one of: (i) a nucleic acid sequence encoding a GRF polypeptide, or (ii) a nucleic acid sequence encoding a SYT polypeptide, comprising:

a. introducing and expressing in a plant, plant part, or plant cell, a nucleic acid sequence encoding a GRF polypeptide as defined in any one of claims 1 to 5 and 7, under the control of a constitutive promoter; and
b. introducing and expressing in a plant, plant part, or plant cell, a nucleic acid sequence encoding a SYT polypeptide as defined in any one of claims 1, 8 to 12 and 14, under the control of a constitutive promoter; and
c. cultivating the plant cell, plant part, or plant under conditions promoting plant growth and development.

**30.** Transgenic plant having increased yield-related traits relative to plants having increased expression of one of: (i) a nucleic acid sequence encoding a GRF polypeptide; or (ii) a nucleic acid sequence encoding a SYT polypeptide, resulting from increased expression of: (i) a nucleic acid sequence encoding a GRF polypeptide as defined in any one of claims 1 to 5 and 7; and (ii) a nucleic acid sequence encoding a SYT polypeptide as defined in any one of claims 1, 8 to 12 and 14, or a transgenic plant cell or transgenic plant part derived from said transgenic plant.

**31.** Transgenic plant according to claim 22, 28 or 30, wherein said plant is a crop plant or a monocot or a cereal, such as rice, maize, wheat, barley, millet, rye, triticale, sorghum and oats, or a transgenic plant cell derived from said transgenic plant.

**32.** Harvestable parts of a plant according to claim 31, comprising (i) an isolated nucleic acid sequence encoding a GRF polypeptide; and (ii) an isolated nucleic acid sequence encoding a SYT polypeptide, wherein said harvestable parts are preferably seeds.

**33.** Use of (i) a nucleic acid sequence encoding a GRF polypeptide as defined in any one of claims 1 to 5 and 7; and of (ii) a nucleic acid sequence encoding a SYT polypeptide as defined in any one of claims 1, 8 to 12 and 14, in increasing yield-related traits in plants relative to plants having increased expression of one of: (i) a nucleic acid sequence encoding a GRF polypeptide, or (ii) a nucleic acid sequence encoding a SYT polypeptide, which increased yield-related traits are one or more of: (i) increased early vigour; (ii) increased aboveground biomass; (iii) increased total seed yield per plant; (iv) increased seed filling rate; (v) increased number of (filled) seeds; (vi) increased harvest index; or (vii) increased thousand kernel weight (TKW).

## Patentansprüche

**1.** Verfahren zur Erhöhung von pflanzlichen Ertragsmerkmalen, bei dem man die Expression von Folgendem: (i) einer Nukleinsäuresequenz, die für ein "Growth-Regulating Factor" (GRF)-Polypeptid kodiert; und (ii) einer Nukleinsäuresequenz, die für ein "Synovial Sarcoma Translocation" (SYT)-Polypeptid kodiert, in einer Pflanze erhöht, wobei die Ertragsmerkmale im Vergleich zu Pflanzen mit einer erhöhten Expression von einem der Folgenden:

(i) einer Nukleinsäuresequenz, die für ein GRF-Polypeptid kodiert, oder (ii) einer Nukleinsäuresequenz, die für ein SYT-Polypeptid kodiert, erhöht sind, wobei das GRF-Polypeptid Folgendes umfasst:

(i) eine Domäne mit mindestens 50%, 55%, 60%, 65%, 70%, 75%, 80%, 85%, 90%, 95%, 98%, 99% oder mehr Aminosäuresequenzidentität zu einer QLQ-Domäne gemäß SEQ ID NO: 115; und
(ii) eine Domäne mit mindestens 50%, 55%, 60%, 65%, 70%, 75%, 80%, 85%, 90%, 95%, 98%, 99% oder mehr Aminosäuresequenzidentität zu einer WRC-Domäne gemäß SEQ ID NO: 116 und

wobei das SYT-Polypeptid vom N-terminalen zum C-terminalen Ende Folgendes umfasst:

(i) eine SNH-Domäne mit in steigender Bevorzugung mindestens 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% Sequenzidentität zu der SNH-Domäne von SEQ ID NO: 262; und
(ii) eine Met-reiche Domäne; und
(iii) eine QG-reiche Domäne.

**2.** Verfahren nach Anspruch 1, wobei das GRF-Polypeptid Folgendes umfasst: (i) eine QLQ-Domäne mit einem InterPro-Eintrag IPR014978 (PFAM-Eintrag PF08880); (ii) eine WRC-Domäne mit einem InterPro-Eintrag IPR014977 (PFAM-Eintrag PF08879); und (iii) eine "Effector of Transcription" (ET)-Domäne umfassend drei Cys-Reste und einen His-Rest in einer konservierten Beabstandung ($CX_9CX_{10}CX_2H$).

**3.** Verfahren nach einem vorhergehenden Anspruch, wobei das GRF-Polypeptid in steigender Bevorzugung mindestens 50%, 55%, 60%, 65%, 70%, 75%, 80%, 85%, 90%, 95%, 98%, 99% oder mehr Aminosäuresequenzidentität zu dem GRF-Polypeptid gemäß SEQ ID NO: 2 oder zu einer der Polypeptidsequenzen in Tabelle A.1 im vorliegenden Text aufweist.

**4.** Verfahren nach einem vorhergehenden Anspruch, wobei die Nukleinsäuresequenz, die für ein GRF-Polypeptid kodiert, von einer der Nukleinsäuresequenz-SEQ ID NOs in Tabelle A.1 oder einem Teil davon, oder einer Sequenz, die fähig ist, mit einer der Nukleinsäuresequenzen SEQ ID NOs in Tabelle A.1 zu hybridisieren, dargestellt wird.

**5.** Verfahren nach einem vorhergehenden Anspruch, wobei die Nukleinsäuresequenz für ein Ortholog oder Paralog von einer der GRF-Polypeptidsequenz-SEQ ID NOs in Tabelle A.1 kodiert.

**6.** Verfahren nach einem vorhergehenden Anspruch, wobei die Nukleinsäuresequenz, die für ein GRF-Polypeptid kodiert, operativ mit einem konstitutiven Promoter, stärker bevorzugt mit einem GOS2-Promoter, am stärksten bevorzugt mit einem GOS2-Promoter aus Reis gemäß SEQ ID NO: 117, verknüpft ist.

**7.** Verfahren nach einem vorhergehenden Anspruch, wobei die Nukleinsäuresequenz, die für ein GRF-Polypeptid kodiert, von einer Pflanze, vorzugsweise von einer dikotylen Pflanze, stärker bevorzugt von der Familie *Brassicaceae,* am stärksten bevorzugt von *Arabidopsis thaliana,* stammt.

**8.** Verfahren nach Anspruch 1, wobei das SYT-Polypeptid weiterhin die am stärksten konservierten Reste der SNH-Domäne gemäß SEQ ID NO: 263 umfasst und in Abbildung 5 schwarz dargestellt ist.

**9.** Verfahren nach einem der Ansprüche 1 bis 8, wobei das SYT-Polypeptid eine Domäne mit in steigender Bevorzugung mindestens 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% Sequenzidentität zu der SSXT-Domäne mit einem InterPro-Eintrag IPR007726 gemäß SEQ ID NO: 264 aufweist.

**10.** Verfahren nach einem der Ansprüche 1 bis 8, wobei das SYT-Polypeptid eine Domäne mit in steigender Bevorzugung mindestens 20%, 25%, 30%, 35%, 40%, 45%, 50%, 55%, 60%, 65%, 70%, 75%, 80%, 85%, 90%, 95%, 98%, 99% oder mehr Aminosäuresequenzidentität zu dem SYT-Polypeptid gemäß SEQ ID NO: 121 oder zu einem der Volllängen-Polypeptidsequenzen in Tabelle A.2 im vorliegenden Text aufweist.

**11.** Verfahren nach einem der Ansprüche 1 bis 10, wobei die Nukleinsäuresequenz, die für ein SYT-Polypeptid kodiert, von einer der Nukleinsäuresequenz-SEQ ID NOs in Tabelle A.2 oder einem Teil davon, oder einer Sequenz, die fähig ist, mit einer der Nukleinsäuresequenzen SEQ ID NOs in Tabelle A.2 zu hybridisieren, dargestellt wird.

**12.** Verfahren nach einem der Ansprüche 1 bis 11, wobei die Nukleinsäuresequenz für ein Ortholog oder Paralog von einer der SYT-Polypeptidsequenz-SEQ ID NOs in Tabelle A.2 kodiert.

**13.** Verfahren nach einem der Ansprüche 1 bis 12, wobei die Nukleinsäuresequenz, die für ein SYT-Polypeptid kodiert, operativ mit einem konstitutiven Promoter, stärker bevorzugt mit einem GOS2-Promoter, am stärksten bevorzugt mit einem GOS2-Promoter aus Reis gemäß SEQ ID NO: 117, verknüpft ist.

**14.** Verfahren nach einem der Ansprüche 1 bis 13, wobei die Nukleinsäuresequenz, die für ein SYT-Polypeptid kodiert, von einer Pflanze, vorzugsweise von einer dikotylen Pflanze, stärker bevorzugt von der Familie *Brassicaceae,* am stärksten bevorzugt von *Arabidopsis thaliana,* stammt.

**15.** Verfahren nach einem der Ansprüche 1 bis 14, wobei die erhöhte Expression dadurch bewirkt wird, dass man in eine(r) Pflanze Folgendes einführt und exprimiert: (i) die Nukleinsäuresequenz, die für das GRF-Polypeptid kodiert, oder (ii) die Nukleinsäuresequenz, die für das SYT-Polypeptid kodiert.

**16.** Verfahren nach Anspruch 15, wobei die Nukleinsäuresequenzen von (i) und (ii) der Reihe nach in eine(r) Pflanze eingeführt und exprimiert werden, vorzugsweise durch Retransformation.

**17.** Verfahren nach Anspruch 16, wobei die Retransformation dadurch erfolgt, dass man eine Nukleinsäuresequenz, die für ein GRF-Polypeptid kodiert, in eine(r) Pflanze, einen/einem Pflanzenteil oder eine(r) Pflanzenzelle umfassend eine eingeführte und exprimierte Nukleinsäuresequenz, die für ein SYT-Polypeptid kodiert, oder umgekehrt, einführt und exprimiert.

**18.** Verfahren nach Anspruch 15, wobei die Nukleinsäuresequenzen von (i) und (ii) gleichzeitig in eine(r) Pflanze eingeführt und exprimiert werden.

**19.** Verfahren nach Anspruch 18, wobei die Nukleinsäuresequenzen von (i) und (ii) in einem oder mehreren Nukleinsäuremolekülen umfasst sind.

**20.** Verfahren nach einem vorhergehenden Anspruch, wobei es sich bei dem erhöhten Ertragsmerkmal um eines oder mehrere der folgenden handelt: (i) erhöhte Jungpflanzenwüchsigkeit; (ii) erhöhte oberirdische Biomasse; (iii) erhöhter Gesamtsamenertrag pro Pflanze; (iv) erhöhte Samenfüllungsrate; (v) erhöhte Anzahl (gefüllter) Samen; (vi) erhöhter Harvest Index; oder (vii) erhöhtes Tausendkorngewicht (TKW).

**21.** Verfahren nach einem vorhergehenden Anspruch, wobei die Nukleinsäuresequenz, die für ein GRF-Polypeptid kodiert und die Nukleinsäuresequenz, die für ein SYT-Polypeptid kodiert, operativ und sequentiell mit einem konstitutiven Promoter, bevorzugt mit einem pflanzlichen konstitutiven Promoter, stärker bevorzugt mit einem GOS2-Promoter, am stärksten bevorzugt mit einem GOS2-Promoter aus Reis gemäß SEQ ID NO: 117, verknüpft sind.

**22.** Pflanzen, Teile davon einschließlich Samen oder Pflanzenzellen, die nach einem Verfahren nach einem vorhergehenden Anspruch erhältlich sind, wobei die Pflanze, der Teil oder die Zelle davon Folgendes umfasst: (i) ein isoliertes Nukleinsäuretransgen, das für ein GRF-Polypeptid kodiert, und (ii) ein isoliertes Nukleinsäuretransgen, das für ein SYT-Polypeptid kodiert.

**23.** Konstrukt, das Folgendes umfasst:

(a) eine Nukleinsäuresequenz, die für ein GRF-Polypeptid wie in einem der Ansprüche 1 bis 5 und 7 definiert kodiert;
(b) eine Nukleinsäuresequenz, die für ein SYT-Polypeptid wie in einem der Ansprüche 1, 8 bis 12 und 14 definiert kodiert;
(c) eine oder mehrere Kontrollsequenzen, die die Erhöhung der Expression der Nukleinsäuresequenz gemäß (a) und gemäß (b) voranzutreiben vermag/vermögen; sowie gegebenenfalls
(d) eine Transkriptionsterminationssequenz,

wobei das SYT-Polypeptid und das GRF-Polypeptid in der Lage sind, miteinander zu interagieren.

**24.** Konstrukt nach Anspruch 23, wobei es sich bei der Kontrollsequenz um mindestens einen konstitutiven Promoter, vorzugweise um einen GOS2-Promoter, stärker bevorzugt einen GOS2-Promoter gemäß SEQ ID NO: 117, handelt.

**25.** Mischung von Konstrukten, wobei mindestens ein Konstrukt Folgendes umfasst:

(a) eine Nukleinsäuresequenz, die für ein GRF-Polypeptid wie in einem der Ansprüche 1 bis 5 und 7 definiert kodiert;
(b) eine oder mehrere Kontrollsequenzen, die fähig sind, die Expression der Nukleinsäuresequenz von (a) voranzutreiben; sowie gegebenenfalls
(c) eine Transkriptionsterminationssequenz,

und wobei mindestens ein anderes Konstrukt Folgendes umfasst:

(d) eine Nukleinsäuresequenz, die für ein SYT-Polypeptid wie in einem der Ansprüche 1, 8 bis 12 und 14 definiert kodiert;
(e) eine oder mehrere Kontrollsequenzen, die fähig sind, die Expression der Nukleinsäuresequenz von (d) voranzutreiben; sowie gegebenenfalls
(f) eine Transkriptionsterminationssequenz,

wobei das SYT-Polypeptid und das GRF-Polypeptid in der Lage sind, miteinander zu interagieren.

**26.** Konstrukt nach Anspruch 25, wobei es sich bei der Kontrollsequenz von (b) und/oder (e) um mindestens einen konstitutiven Promoter, vorzugweise um einen GOS2-Promoter, stärker bevorzugt einen GOS2-Promoter gemäß SEQ ID NO: 117, handelt.

**27.** Verwendung eines Konstrukts nach den Ansprüchen 23 oder 24 oder von einer Mischung von Konstrukten nach Anspruch 25, bei einem Verfahren zur Herstellung von Pflanzen mit erhöhten Ertragsmerkmalen im Vergleich zu Pflanzen mit einer erhöhten Expression von einem der Folgenden: (a) einer Nukleinsäuresequenz, die für ein GRF-Polypeptid kodiert oder (b) einer Nukleinsäuresequenz, die für ein SYT-Polypeptid kodiert, wobei es sich bei den erhöhten Ertragsmerkmalen um eines oder mehrere der Folgenden handelt: (i) e r h ö h t e Jungpflanzenwüchsigkeit; (ii) erhöhte oberirdische Biomasse; (iii) erhöhter Gesamtsamenertrag pro Pflanze; (iv) erhöhte Samenfüllungsrate; (v) erhöhte Anzahl (gefüllter) Samen; (vi) erhöhter Harvest Index; oder (vii) erhöhtes Tausendkorngewicht (TKW).

**28.** Pflanze, Pflanzenteil oder Pflanzenzelle, die/der mit einem Konstrukt nach den Ansprüchen 23 oder 24 oder einer Mischung von Konstrukten nach Anspruch 25 transformiert ist.

**29.** Verfahren zur Herstellung transgener Pflanzen mit erhöhten Ertragsmerkmalen im Vergleich zu Pflanzen mit erhöhter Expression entweder (i) einer für ein GRF-Polypeptid kodierenden Nukleinsäuresequenz oder (ii) einer für ein SYT-Polypeptid kodierenden Nukleinsäuresequenz, wobei man

a. eine für ein GRF-Polypeptid mit der in einem der Ansprüche 1 bis 5 und 7 angegebenen Bedeutung kodierende Nukleinsäuresequenz unter Kontrolle eines konstitutiven Promoters in eine Pflanze, einen Pflanzenteil oder eine Pflanzenzelle einführt und darin exprimiert und
b. eine für ein SYT-Polypeptid mit der in einem der Ansprüche 1, 8 bis 12 und 14 angegebenen Bedeutung kodierende Nukleinsäuresequenz unter Kontrolle eines konstitutiven Promoters in eine Pflanze, einen Pflanzenteil oder eine Pflanzenzelle einführt und darin exprimiert und
c. die Pflanzenzelle, den Pflanzenteil oder die Pflanze unter Pflanzenwachstum und - entwicklung fördernden Bedingungen kultiviert.

**30.** Transgene Pflanze mit erhöhten Ertragsmerkmalen im Vergleich zu Pflanzen mit einer erhöhten Expression von einem der Folgenden: (i) eines Nukleinsäuresequenz, die für ein GRF-Polypeptid kodiert oder (ii) einer Nukleinsäuresequenz, die für ein SYT-Polypeptid kodiert, die das Ergebnis einer erhöhten Expression von Folgendem sind: (i) einer Nukleinsäuresequenz, die für ein GRF-Polypeptid nach einem der Ansprüche 1 bis 5 und 7 definiert kodiert; u n d (ii) einer Nukleinsäuresequenz, die für ein SYT-Polypeptid nach einem der Ansprüche 1, 8 bis 12 und 14 definiert kodiert, oder eine transgene Pflanzenzelle oder ein transgener Pflanzenteil, die/der von der transgenen Pflanze abstammt.

**31.** Transgene Pflanze nach Anspruch 22, 28 oder 30, wobei es sich bei der Pflanze um eine Kulturpflanze oder eine monokotyle Pflanze oder ein Getreide, wie Reis, Mais, Weizen, Gerste, Hirse, Roggen, Triticale, Sorghum und Hafer, oder um eine transgene Pflanzenzelle, die von der transgenen Pflanze stammt, handelt.

**32.** Erntbare Teile einer Pflanzen nach Anspruch 31, umfassend: (i) Nukleinsäuresequenz, die für ein GRF-Polypeptid kodiert eine isolierte, oder (ii) eine isolierte Nukleinsäuresequenz, die für ein SYT-Polypeptid kodiert, wobei es sich bei den erntbaren Teilen vorzugsweise um Samen handelt.

**33.** Verwendung von (i) einer Nukleinsäuresequenz, die für ein GRF-Polypeptid wie in einem der Ansprüche 1 bis 5 und 7 definiert kodiert; und von (ii) einer Nukleinsäuresequenz, die für ein SYT-Polypeptid wie in einem der Ansprüche 1, 8 bis 12 und 14 definiert kodiert, bei der Erhöhung von Ertragsmerkmalen in Pflanzen im Vergleich zu Pflanzen mit einer erhöhten Expression von einem der Folgenden: (i) einer Nukleinsäuresequenz, die für ein GRF-Polypeptid kodiert, oder (ii) einer Nukleinsäuresequenz, die für ein SYT-Polypeptid kodiert, wobei es sich bei den erhöhten Ertragsmerkmalen um eines oder mehrere der Folgenden handelt: (i) erhöhte Jungpflanzenwüchsigkeit; (ii) erhöhte oberirdische Biomasse; (iii) erhöhter Gesamtsamenertrag pro Pflanze; (iv) erhöhte Samenfüllungsrate; (v) erhöhte Anzahl (gefüllter) Samen; (vi) erhöhter Harvest Index; oder (vii) erhöhtes Tausendkorngewicht (TKW).

## Revendications

**1.** Procédé pour augmenter les caractères liés au rendement végétal, comprenant l'augmentation de l'expression dans une plante (i) d'une séquence d'acide nucléique codant pour un polypeptide facteur régulateur de croissance (GRF), et (ii) d'une séquence d'acide nucléique codant pour un polypeptide de la translocation du sarcome synovial (SYT), lesdits caractères liés au rendement étant augmentés par comparaison avec des plantes ayant une expression accrue de l'une : (i) d'une séquence d'acide nucléique codant pour un polypeptide GRF, ou (ii) d'une séquence d'acide nucléique codant pour un polypeptide SYT,

dans lequel ledit polypeptide GRF comprend :

(i) un domaine ayant une identité de séquence d'acides aminés d'au moins 50 %, 55 %, 60 %, 65 %, 70 %, 75 %, 80 %, 85 %, 90 %, 95 %, 98 %, 99 % ou plus avec une séquence QLQ telle que représentée par SEQ ID N° 115 ; et
(ii) un domaine ayant une identité de séquence d'acides aminés d'au moins 50 %, 55 %, 60 %, 65 %, 70 %, 75 %, 80 %, 85 %, 90 %, 95 %, 98 %, 99 % ou plus avec un domaine WRC tel que représenté par SEQ ID N° 116 ; et ledit polypeptide SYT comprenant, du site N-terminal au site C-terminal :

(i) un domaine SNH ayant, dans l'ordre croissant de préférence, une identité de séquence d'au moins 20 %, 25 %, 30 %, 35 %, 40 %, 45 %, 50 %, 55 %, 60 %, 65 %, 70 %, 75 %, 80 %, 85 %, 90 %, 91 %, 92 %, 93 %, 94 %, 95 %, 96 %, 97 %, 98 %, 99 %, avec le domaine SNH de SEQ ID N° 262 ; et
(ii) un domaine riche en Met ; et
(iii) un domaine riche en QG.

**2.** Procédé selon la revendication 1, dans lequel ledit polypeptide GRF comprend : (i) un domaine QLQ ayant un numéro d'accession InterPro IPR014978 (numéro d'accession PFAM PF08880) ; (ii) un domaine WRC ayant un numéro d'accession InterPro IPR014977 (numéro d'accession PFAM PF08879) ; et (iii) un domaine effecteur de transcription (ET), comprenant trois résidus Cys et un résidu His selon un espacement conservé ($CX_9CX_{10}CX_2H$).

**3.** Procédé selon l'une quelconque des revendications précédentes, dans lequel ledit polypeptide GRF a, dans l'ordre croissant de préférence, une identité de séquence d'acides aminés d'au moins 50 %, 55 %, 60 %, 65 %, 70 %, 75 %, 80 %, 85 %, 90 %, 95 %, 98 %, 99 % ou plus avec le polypeptide GRF tel que représenté par SEQ ID N° 2, ou avec l'une quelconque des séquences polypeptidiques présentées dans le Tableau A.1 de l'invention.

**4.** Procédé selon l'une quelconque des revendications précédentes, dans lequel ladite séquence d'acide nucléique codant pour un polypeptide GRF est représentée par l'une quelconque des séquences d'acides nucléiques SEQ ID N° présentées dans le Tableau A.1, ou d'une portion de ces dernières, ou par une séquence capable de s'hybrider à l'une quelconque des séquences d'acides nucléiques SEQ ID N° présentées dans le Tableau A.1.

**5.** Procédé selon l'une quelconque des revendications précédentes, dans lequel ladite séquence d'acide nucléique code pour un orthologue ou un paralogue de l'une quelconque des séquences des polypeptides GRF SEQ ID N°, présentées dans le Tableau A.1.

**6.** Procédé selon l'une quelconque des revendications précédentes, dans lequel ladite séquence d'acide nucléique codant pour un polypeptide GRF est liée d'une manière opérationnelle à un promoteur constitutif, plus particulièrement à un promoteur GOS2, tout spécialement à un promoteur GOS2 du riz, tel que représenté par SEQ ID N° 117.

**7.** Procédé selon l'une quelconque des revendications précédentes, dans lequel ladite séquence d'acide nucléique codant pour un polypeptide GRF est provient d'une plante, provient de préférence d'une plante dicotylédone, plus particulièrement de la famille des Brassicaceae, et tout spécialement d'*Arabidopsis thaliana*.

**8.** Procédé selon la revendication 1, dans lequel ledit polypeptide SYT comprend en outre les résidus les plus conservés du domaine SNH tel que représenté par SEQ ID N° 263, et présenté en noir sur la Figure 5.

**9.** Procédé selon l'une quelconque des revendications 1 à 8, dans lequel ledit polypeptide SYT comprend un domaine ayant, dans l'ordre croissant de préférence, une identité de séquence d'au moins 20 %, 25 %, 30 %, 35 %, 40 %, 45 %, 50 %, 55 %, 60 %, 65 %, 70 %, 75 %, 80 %, 85 %, 90 %, 91 %, 92 %, 93 %, 94 %, 95 %, 96 %, 97 %, 98 %, 99 % avec le domaine SSXT ayant un numéro d'accession InterPro IPR007726, et ayant la séquence SEQ ID N° 264.

**10.** Procédé selon l'une quelconque des revendications 1 à 8, dans lequel ledit polypeptide SYT a, dans l'ordre croissant de préférence, une identité de séquence d'acides aminés d'au moins 20 %, 25 %, 30 %, 35 %, 40 %, 45 %, 50 %, 55 %, 60 %, 65 %, 70 %, 75 %, 80 %, 85 %, 90 %, 95 %, 98 %, 99 % ou plus avec le polypeptide SYT tel que représenté par SEQ ID N° 121, ou avec l'une quelconque des séquences polypeptidiques complètes présentées dans le Tableau A.2 de l'invention.

**11.** Procédé selon l'une quelconque des revendications 1 à 10, dans lequel ladite séquence d'acide nucléique codant pour un polypeptide SYT est représentée par l'une quelconque des séquences d'acides nucléiques SEQ ID N° présentées dans le Tableau A.2 ou par une portion de ces dernières, ou par une séquence capable de s'hybrider à l'une quelconque des séquences d'acides nucléiques SEQ ID N° présentées dans le Tableau A.2.

**12.** Procédé selon l'une quelconque des revendications 1 à 11, dans lequel ladite séquence d'acide nucléique code pour un orthologue ou un paralogue de l'une quelconque des séquences des polypeptides SYT SEQ ID N° présentées dans le Tableau A.2.

**13.** Procédé selon l'une quelconque des revendications 1 à 12, dans lequel ladite séquence d'acide nucléique codant pour un polypeptide SYT est liée d'une manière opérationnelle à un promoteur constitutif, plus particulièrement à un promoteur GOS2, tout spécialement à un promoteur GOS2 du riz, tel que représenté par SEQ ID N° 117.

**14.** Procédé selon l'une quelconque des revendications 1 à 13, dans lequel ladite séquence d'acide nucléique codant pour un polypeptide SYT provient d'une plante, de préférence d'une plante dicotylédone, plus particulièrement de la famille des Brassicaceae, tout spécialement d'*Arabidopsis thaliana.*

**15.** Procédé selon l'une quelconque des revendications 1 à 14, dans lequel ladite augmentation de l'expression est réalisée par introduction et expression, dans une plante : (i) de ladite séquence d'acide nucléique codant pour ledit polypeptide GRF ; et (ii) de ladite séquence d'acide nucléique codant pour ledit polypeptide SYT.

**16.** Procédé selon la revendication 15, dans lequel lesdites séquences d'acides nucléiques (i) et (ii) sont introduites et exprimées successivement dans une plante, de préférence par retransformation.

**17.** Procédé selon la revendication 16, dans lequel ladite retransformation est réalisée par introduction et expression d'une séquence d'acide nucléique codant pour un polypeptide GRF dans une plante, une partie de plante ou une cellule végétale comprenant une séquence d'acide nucléique introduite et exprimée, codant pour un polypeptide SYT, ou inversement.

**18.** Procédé selon la revendication 15, dans lequel lesdites séquences d'acides nucléiques (i) et (ii) sont introduites et exprimées simultanément dans une plante.

**19.** Procédé selon la revendication 18, dans lequel lesdites séquences d'acides nucléiques (i) et (ii) sont comprises dans une ou plusieurs molécules d'acides nucléiques.

**20.** Procédé selon l'une quelconque des revendications précédentes, dans lequel ledit caractère lié au rendement est un ou plusieurs des caractères suivants : (i) une vigueur précoce accrue ; (ii) une biomasse aérienne accrue ; (iii) un rendement total des graines accru par plante ; (iv) un taux de remplissage des graines accru ; (v) un nombre de graines (remplies) accru ; (vi) un indice de récolte accru ; ou (vii) un poids de mille grains (TKW) accru.

**21.** Procédé selon l'une quelconque des revendications précédentes, dans lequel ladite séquence d'acide nucléique codant pour un polypeptide GRF et ladite séquence d'acide nucléique codant pour un polypeptide SYT sont liées d'une manière opérationnelle et séquentielle à un promoteur constitutif, de préférence à un promoteur constitutif végétal, plus particulièrement à un promoteur GOS2, tout spécialement à un promoteur GOS2 du riz tel que représenté par SEQ ID N° 117.

**22.** Plantes, parties de ces dernières, y compris les graines, ou cellules végétales, pouvant être obtenues par un procédé selon l'une quelconque des revendications précédentes, où ladite plante, ladite partie ou cellule de cette dernière, comprend (i) un transgène d'acide nucléique isolé codant pour un polypeptide GRF et (ii) un transgène d'acide nucléique isolé codant pour un polypeptide SYT.

**23.** Construction comprenant :

(a) une séquence d'acide nucléique codant pour un polypeptide GRF tel que défini dans l'une quelconque des revendications 1 à 5 et 7 ;
(b) une séquence d'acide nucléique codant pour un polypeptide SYT tel que défini dans l'une quelconque des revendications 1, 8 à 12 et 14 ;
(c) une ou plusieurs séquences régulatrices, capables de commander l'expression des séquences d'acides nucléiques (a) et (b) ; et, en option
(d) une séquence terminatrice de transcription,

dans laquelle ledit polypeptide SYT et ledit polypeptide GRF sont capables d'interagir l'un avec l'autre.

**24.** Construction selon la revendication 23, dans laquelle ladite séquence régulatrice est au moins un promoteur constitutif, de préférence un promoteur GOS2, plus particulièrement un promoteur GOS2 tel que représenté par SEQ ID N° 117.

**25.** Mélange de constructions, dans lequel au moins une construction comprend :

(a) une séquence d'acide nucléique codant pour un polypeptide GRF tel que défini dans l'une quelconque des revendications 1 à 5 et 7 ;
(b) une ou plusieurs séquences régulatrices capables de commander l'expression de la séquence d'acide nucléique (a) ; et en option
(c) une séquence terminatrice de transcription,

et dans lequel au moins une autre construction comprend :

(d) une séquence d'acide nucléique codant pour un polypeptide SYT tel que défini dans l'une quelconque des revendications 1, 8 à 12 et 14 ;
(e) une ou plusieurs séquences régulatrices capables de commander l'expression de la séquence d'acide nucléique (d) ; et en option
(f) une séquence terminatrice de transcription,

ledit polypeptide SYT et ledit polypeptide GRF étant capables d'interagir l'un avec l'autre.

**26.** Construction selon la revendication 25, dans lequel lesdites séquences régulatrices (b) et/ou (e) sont au moins un promoteur constitutif, de préférence un promoteur GOS2, plus particulièrement un promoteur GOS2 tel que représenté par SEQ ID N° 117.

**27.** Utilisation d'une construction selon les revendications 23 ou 24, ou d'un mélange de constructions selon la revendication 25, dans un procédé de fabrication de plantes ayant des caractères liés à un rendement accru par rapport à des plantes ayant une expression accrue : (a) d'une séquence d'acide nucléique codant pour un polypeptide GRF, ou (b) d'une séquence d'acide nucléique codant pour un polypeptide SYT, ces caractères liés à un rendement accru étant un ou plusieurs des caractères suivants : (i) une vigueur précoce accrue ; (ii) une biomasse aérienne accrue ; (iii) un rendement total des graines accru par plante ; (iv) un taux de remplissage des graines accru ; (v) un nombre de graines (remplies) accru ; (vi) un indice de récolte accru ; ou (vii) un poids de mille grains (TKW) accru.

**28.** Plante, partie de plante ou cellule végétale transformée avec une construction selon les revendications 23 ou 24, ou avec un mélange de constructions selon la revendication 25.

**29.** Procédé de production de plantes transgéniques ayant des caractères liés à un rendement accru par comparaison avec des plantes dont l'expression a été augmentée : (i) d'une séquence d'acide nucléique codant pour un polypeptide GRF, ou (ii) d'une séquence d'acide nucléique codant pour un polypeptide SYT, comprenant :

a. l'introduction et l'expression dans une plante, une partie de plante ou une cellule végétale, d'une séquence d'acide nucléique codant pour un polypeptide GRF tel que défini dans l'une quelconque des revendications 1 à 5 et 7, sous le contrôle d'un promoteur constitutif ; et
b. l'introduction et l'expression dans une plante, dans une partie de plante ou dans une cellule végétale, d'une séquence d'acide nucléique codant pour un polypeptide SYT tel que défini dans l'une quelconque des revendications 1, 8 à 12 et 14, sous le contrôle d'un promoteur constitutif ; et
c. la culture de la cellule végétale, de la partie de plante ou de la plante, dans des conditions qui favorisent la croissance et le développement de la plante.

**30.** Plante transgénique ayant des caractères liés à un rendement accru par comparaison avec des plantes dont l'expression a été augmentée: (i) d'une séquence d'acide nucléique codant pour un polypeptide GRF ; ou (ii) d'une séquence d'acide nucléique codant pour un polypeptide SYT, résultant d'une augmentation de l'expression (i) d'une séquence d'acide nucléique codant pour un polypeptide GRF tel que défini dans l'une quelconque des revendications 1 à 5 et 7 ; et (ii) d'une séquence d'acide nucléique codant pour un polypeptide SYT tel que défini dans l'une quelconque des revendications 1, 8 à 12 et 14, ou cellule de plante transgénique ou partie de plante transgénique qui dérive de ladite plante transgénique.

**31.** Plante transgénique selon la revendication 22, 28 ou 30, ladite plante étant une plante de culture ou une monocotylédone ou une céréale telle que le riz, le maïs, le blé, l'orge, le millet, le seigle, le triticale, le sorgho et l'avoine, ou cellule de plante transgénique qui dérive de ladite plante transgénique.

**32.** Parties récoltables d'une plante selon la revendication 31, comprenant (i) une séquence d'acide nucléique isolée codant pour un polypeptide GRF ; et (ii) une séquence d'acide nucléique isolée codant pour un polypeptide SYT, lesdites parties récoltables étant de préférence des graines.

**33.** Utilisation (i) d'une séquence d'acide nucléique codant pour un polypeptide GRF tel que défini dans l'une quelconque des revendications 1 à 5 et 7 ; et (ii) d'une séquence d'acide nucléique codant pour un polypeptide SYT tel que défini dans l'une quelconque des revendications 1, 8 à 12 et 14, pour accroître les caractères liés au rendement dans des plantes par comparaison avec des plantes dont l'expression a été augmentée : (i) d'une séquence d'acide nucléique codant pour un polypeptide GRF ou (ii) d'une séquence d'acide nucléique codant pour un polypeptide SYT, lesdits caractères liés à un rendement accrus étant un ou plusieurs des caractères suivants : (i) une vigueur précoce accrue ; (ii) une biomasse aérienne accrue ; (iii) un rendement total des graines accru par plante ; (iv) un taux de remplissage des graines accru ; (v) un nombre de graines (remplies) accru ; (vi) un indice de récolte accru ; ou (vii) un poids de mille grains (TKW) accru.

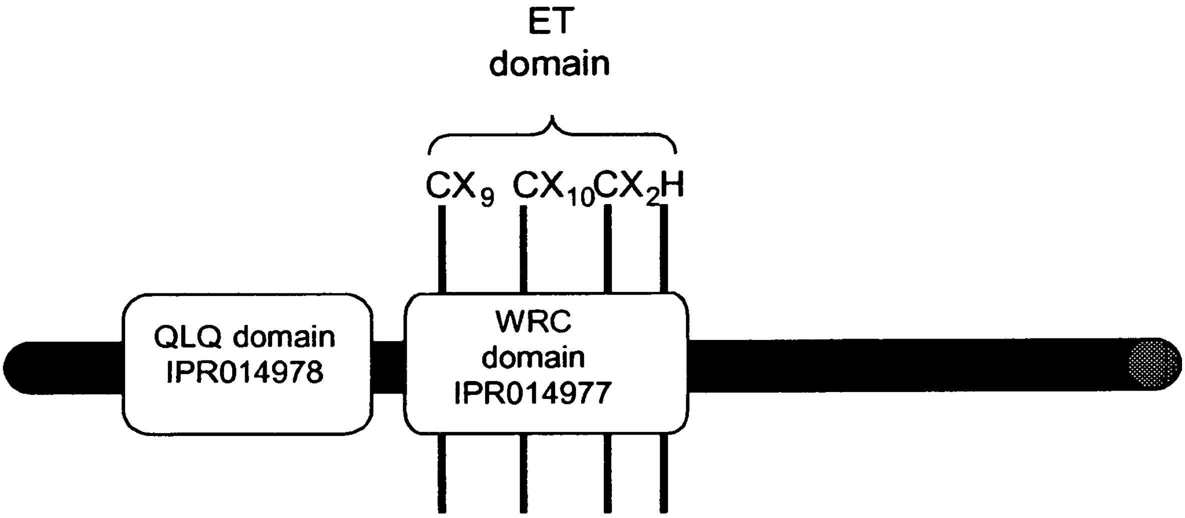
ET
domain
CX9 CX10CX2H
QLQ domain
IPR014978
WRC
domain
IPR014977

FIGURE 1

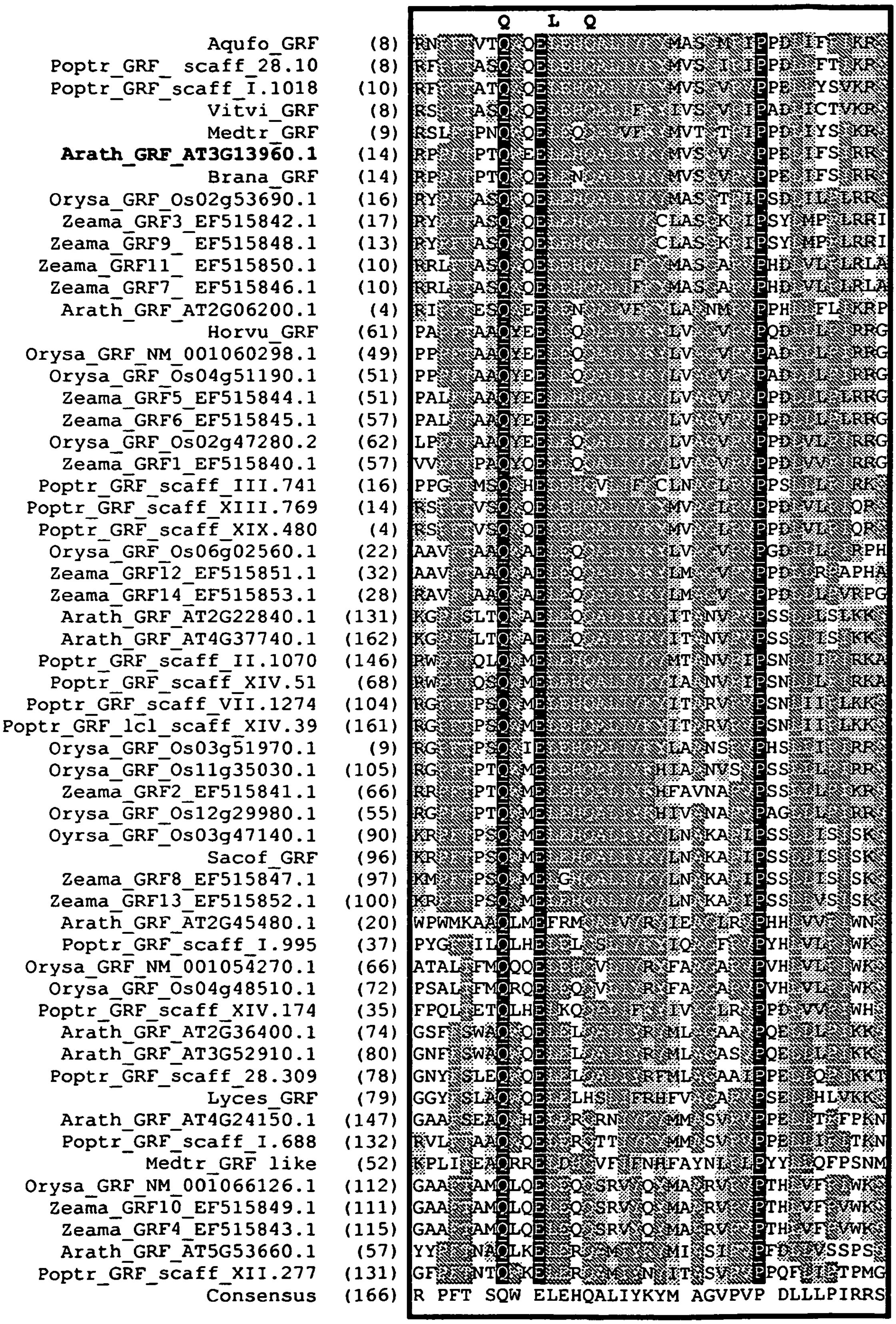
Q L Q
Aqufo_GRF (8)
Poptr_GRF_ scaff_28.10 (8)
Poptr_GRF_scaff_I.1018 (10)
Vitvi_GRF (8)
Medtr_GRF (9)
Arath_GRF_AT3G13960.1 (14)
Brana_GRF (14)
Orysa_GRF_Os02g53690.1 (16)
Zeama_GRF3_EF515842.1 (17)
Zeama_GRF9_ EF515848.1 (13)
Zeama_GRF11_ EF515850.1 (10)
Zeama_GRF7_ EF515846.1 (10)
Arath_GRF_AT2G06200.1 (4)
Horvu_GRF (61)
Orysa_GRF_NM_001060298.1 (49)
Orysa_GRF_Os04g51190.1 (51)
Zeama_GRF5_EF515844.1 (51)
Zeama_GRF6_EF515845.1 (57)
Orysa_GRF_Os02g47280.2 (62)
Zeama_GRF1_EF515840.1 (57)
Poptr_GRF_scaff_III.741 (16)
Poptr_GRF_scaff_XIII.769 (14)
Poptr_GRF_scaff_XIX.480 (4)
Orysa_GRF_Os06g02560.1 (22)
Zeama_GRF12_EF515851.1 (32)
Zeama_GRF14_EF515853.1 (28)
Arath_GRF_AT2G22840.1 (131)
Arath_GRF_AT4G37740.1 (162)
Poptr_GRF_scaff_II.1070 (146)
Poptr_GRF_scaff_XIV.51 (68)
Poptr_GRF_scaff_VII.1274 (104)
Poptr_GRF_lcl_scaff_XIV.39 (161)
Orysa_GRF_Os03g51970.1 (9)
Orysa_GRF_Os11g35030.1 (105)
Zeama_GRF2_EF515841.1 (66)
Orysa_GRF_Os12g29980.1 (55)
Oyrsa_GRF_Os03g47140.1 (90)
Sacof_GRF (96)
Zeama_GRF8_EF515847.1 (97)
Zeama_GRF13_EF515852.1 (100)
Arath_GRF_AT2G45480.1 (20)
Poptr_GRF_scaff_I.995 (37)
Orysa_GRF_NM_001054270.1 (66)
Orysa_GRF_Os04g48510.1 (72)
Poptr_GRF_scaff_XIV.174 (35)
Arath_GRF_AT2G36400.1 (74)
Arath_GRF_AT3G52910.1 (80)
Poptr_GRF_scaff_28.309 (78)
Lyces_GRF (79)
Arath_GRF_AT4G24150.1 (147)
Poptr_GRF_scaff_I.688 (132)
Medtr_GRF like (52)
Orysa_GRF_NM_001066126.1 (112)
Zeama_GRF10_EF515849.1 (111)
Zeama_GRF4_EF515843.1 (115)
Arath_GRF_AT5G53660.1 (57)
Poptr_GRF_scaff_XII.277 (131)
Consensus (166) R PFT SQW ELEHQALIYKYM AGVPVP DLLLPIRRS

**FIGURE 2**

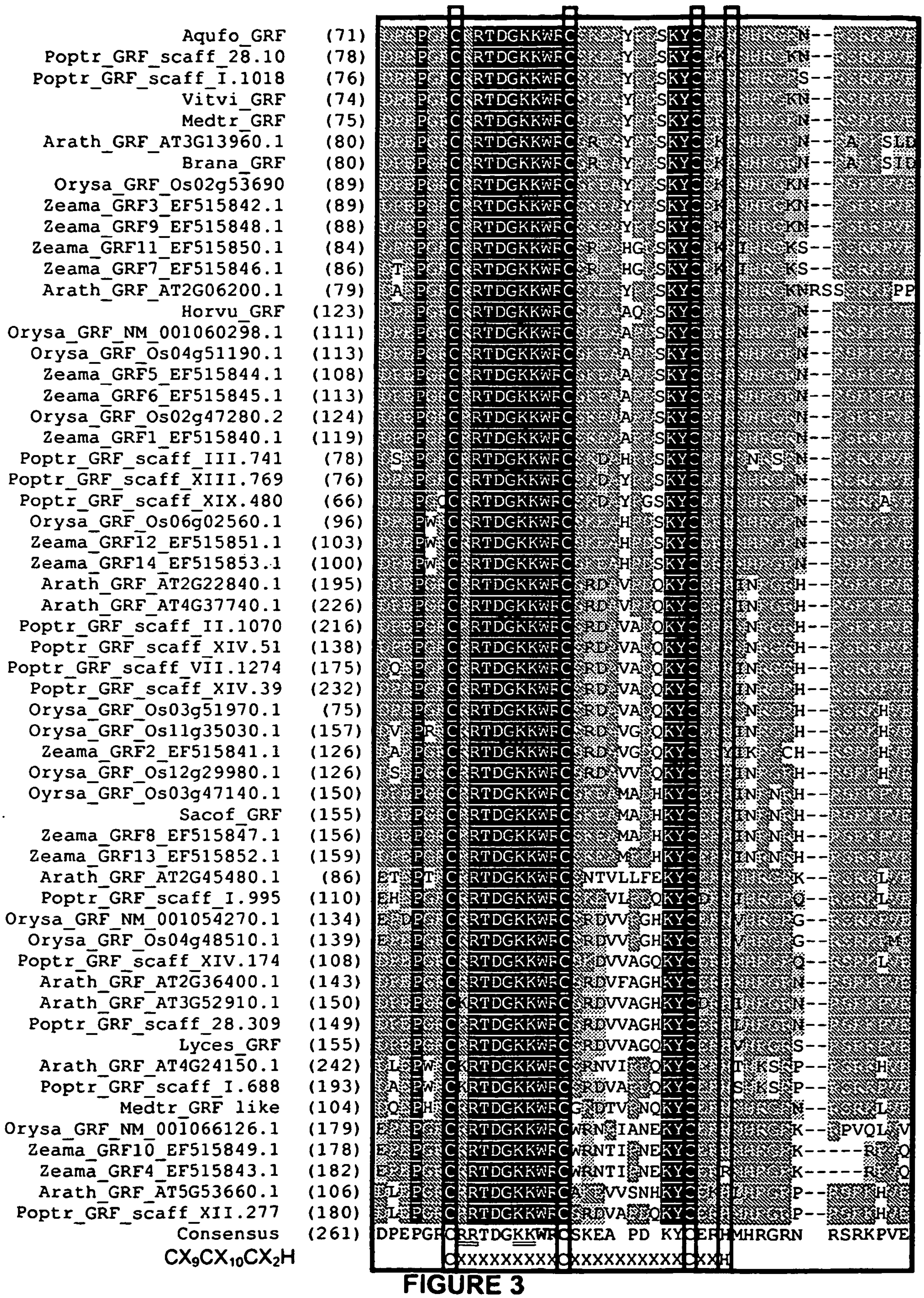
Aqufo_GRF (71)
Poptr_GRF_scaff_28.10 (78)
Poptr_GRF_scaff_I.1018 (76)
Vitvi_GRF (74)
Medtr_GRF (75)
Arath_GRF_AT3G13960.1 (80)
Brana_GRF (80)
Orysa_GRF_Os02g53690 (89)
Zeama_GRF3_EF515842.1 (89)
Zeama_GRF9_EF515848.1 (88)
Zeama_GRF11_EF515850.1 (84)
Zeama_GRF7_EF515846.1 (86)
Arath_GRF_AT2G06200.1 (79)
Horvu_GRF (123)
Orysa_GRF_NM_001060298.1 (111)
Orysa_GRF_Os04g51190.1 (113)
Zeama_GRF5_EF515844.1 (108)
Zeama_GRF6_EF515845.1 (113)
Orysa_GRF_Os02g47280.2 (124)
Zeama_GRF1_EF515840.1 (119)
Poptr_GRF_scaff_III.741 (78)
Poptr_GRF_scaff_XIII.769 (76)
Poptr_GRF_scaff_XIX.480 (66)
Orysa_GRF_Os06g02560.1 (96)
Zeama_GRF12_EF515851.1 (103)
Zeama_GRF14_EF515853.1 (100)
Arath_GRF_AT2G22840.1 (195)
Arath_GRF_AT4G37740.1 (226)
Poptr_GRF_scaff_II.1070 (216)
Poptr_GRF_scaff_XIV.51 (138)
Poptr_GRF_scaff_VII.1274 (175)
Poptr_GRF_scaff_XIV.39 (232)
Orysa_GRF_Os03g51970.1 (75)
Orysa_GRF_Os11g35030.1 (157)
Zeama_GRF2_EF515841.1 (126)
Orysa_GRF_Os12g29980.1 (126)
Oyrsa_GRF_Os03g47140.1 (150)
Sacof_GRF (155)
Zeama_GRF8_EF515847.1 (156)
Zeama_GRF13_EF515852.1 (159)
Arath_GRF_AT2G45480.1 (86)
Poptr_GRF_scaff_I.995 (110)
Orysa_GRF_NM_001054270.1 (134)
Orysa_GRF_Os04g48510.1 (139)
Poptr_GRF_scaff_XIV.174 (108)
Arath_GRF_AT2G36400.1 (143)
Arath_GRF_AT3G52910.1 (150)
Poptr_GRF_scaff_28.309 (149)
Lyces_GRF (155)
Arath_GRF_AT4G24150.1 (242)
Poptr_GRF_scaff_I.688 (193)
Medtr_GRF like (104)
Orysa_GRF_NM_001066126.1 (179)
Zeama_GRF10_EF515849.1 (178)
Zeama_GRF4_EF515843.1 (182)
Arath_GRF_AT5G53660.1 (106)
Poptr_GRF_scaff_XII.277 (180)
Consensus (261) DPEPGRCRRTDGKKWRCSKEA PD KYCERHMHRGRN RSRKPVE
CX9CX10CX2H CXXXXXXXXXCXXXXXXXXXXCXXH

FIGURE 3

**Plant SYT-like polypeptide structure**

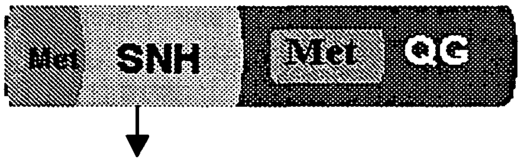


InterPro SSXT family
IPR007726

**Mammalian SYT-like polypeptide structure**

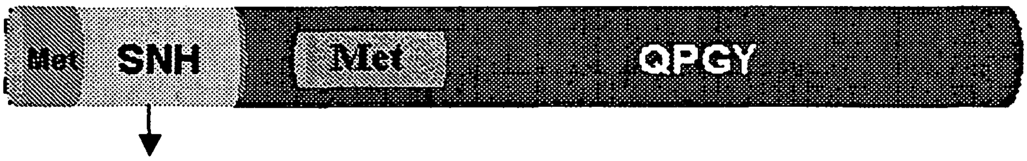


InterPro SSXT family
IPR007726

FIGURE 4

```
Allce_SYT2      ITTEQIQRYLDENKQLILAILDNQNLGRLNECAQYQAQLQKNLLYLAAIADAQP
Poptr_SYT1.2    ITTEQIQKYLEENKQLIMAILENQNKGNVSECASYQAQLQQNLMYLARIADAQP
Aqufo_SYT2      ITTEQIQKYLDENKTLILAILDNQNLGKLAECAQYQAQLQKNLMYLAAIADAQP
Vitvi_SYT2.2    ITTEQIQKYLDENKQLILAILENQTLGKLAECAQYQAQLQKNLIYLAAIADAQP
Citsi_SYT2      ITTEQIQKYLDENKKLILAILDNQNLGKLTECAHYQAQLQKNLMYLAAIADAQP
Vitvi_SYT2.1    ITTEQIQKYLDENKKLILAILDNQNLGKLAECAQYQAQLQKNLMYLAAIADAQP
Glyma_SYT2.1    ITTEQIQKYLDENKKLILAILDNQNLGKLAECAQYQAQLQKNLMYLAAIADAQP
Glyma_SYT2.2    ITTEQIQKYLDENKKLILAILDNQNLGKLAECAQYQAQLQKNLMYLAAIADAQP
Glyso_SYT2      ITTEQIQKYLDENKKLILAILDNQNLGKLAECAQYQAQLQKNLMYLAAIADAQP
Medtr_SYT2      ITTEQIQKYLDENKKLILAILDNQNLGKLAECAQYQAQLQKNLMYLAAIADAQP
Poptr_SYT2      ITTEQIQKYLDENKKLILAILDNQNLGKLAECAQYQAQLQKNLMYLAAIADAQP
Goshi_SYT2      ITTEQIQKYLDENKKLILAILDNQNLGKLAECAQYQAQLQKNLMYLAAIADAQP
Eupes_SYT2      ITTEQIQKYLDENKKLILAILDNQNLGKLAECAQYQALLQKNLMYLAAIADAQP
Frava_SYT2      ITTEQIQKCLDENKKLILAILDNQNLGKLAECAQYQTQLQKNLMYLAAIADAQP
Maldo_SYT2      ITTEQIQKYLDDNKKLILAILDNQNLGKLAECAQYQALLQKNLMYLAAIADAQP
Prupe_SYT2      ITTEQIQKYLDENKKLILAILDNQNLGKLAECAQYQAQLQKNLMYLAAIADAQP
Soltu_SYT3      ITTEQIQKYLDENKTLILAILDHQNLGKLAECAQYQAKLQKNLMYLAAIADAQP
Arath_SYT2      ITTEQIQKYLDENKKLIMAIMENQNLGKLAECAQYQALLQKNLMYLAAIADAQP
Brana_SYT2      ITTEQIQKYLEENKKLIMAIMENQNLGKLAECAQYQALLQKNLMYLAAIADAQP
Arath_SYT3      ITTEQIQKYLDENKKLIMAILENQNLGKLAECAQYQALLQKNLMYLAAIADAQP
Lacse_SYT2      ITTDQIQKFLDENKQLIIAIMSNLNLGKLAECAQYQALLQKNLMYLAAIADAQP
Tarof_SYT2      ITTDQIQKFLDENKQLILAIMNNQNLGKLAECAQYQALLQKNLMYLAAIADAQP
Betvu_SYT2      ITTEQIQKYLDENKQLILAIMESQNSGKMNECAQYQAQLQKNLMYLAAIADAQP
Tarof_SYT3      ITSDQIQKCLDDNENLIIAILENQNLGKFQECAQYQAILQKNLMYLAAIADAQP
Bradi_SYT3      ITTEQIQKYLDENKQLILAILENQNLGKLTECAQYQAQLQKNLLYLAAIADAQP
Triae_SYT3      ITTEQIQKYLDENKQLILAILENQNLGKLAECAQYQAQLQKNLLYLAAIADAQP
Triae_SYT3.2    ITTEQIQKYLDENKQLILAILENQNLGKLAECAQYQAQLQKNLLYLAAIADAQP
Orysa_SYT3      ITTEQIQKYLDENKQLILAILENQNLGKLAECAQYQAQLQKNLLYLAAIADAQP
Panvi_SYT3      ITTEQIQKYLDENKQLILAILENQNLGKLAECAQYQAQLQKNLLYLAAIADAQP
Sacof_SYT3      ITTEQIQKYLDENKQLILAILENQNLGKLAECAQYQAQLQKNLLYLAAIADAQP
Sorbi_SYT3      ITTEQIQKYLDENKQLILAILENQNLGKLAECAQYQAQLQKNLLYLAAIADAQP
Horvu_SYT2      LSTEQIQKYLDENKQLILAILENQNLGKLAECAQYQAQLQKNLLYLAAIADTQP
Triae_SYT2      LSTEQIQKYLDENKQLILAILENQNLGKLAECAQYQAQLQKNLLYLAAIADTQP
Orysa_SYT2      ITTEQIQKYLDENKQLILAILENQNLGKLAECAQYQAQLQKNLLYLAAIADTQP
Sacof_SYT2      ITTEQIQKYLDENKQLILAILENQNLGKLAECAQYQSQLQKNLLYLAAIADAQP
Sorbi_SYT2      ITTEQIQKYLDENKQLILAILENQNLGKLAECAQYQSQLQKNLLYLAAIADAQP
```

**FIGURE 5**

```
Zeama_SYT2     ISTEQIQKYLDENKQLILAILENQNLGKLAECAQYQSQLQKNLLYLAAIADAQP
Curlo_SYT2     ITTEQIQKYLDENKQLILAILENQNLGKLAECAQYQAQLQKNLLYLAAIADAQP
Cerri_SYT      VTTELVQKYLDENKQLILSILDNQNVGNLNECAVYQQKLQSNLMFLAAVADAQT
Aqufo_SYT1     VTTDHIQQYLDENKALILKILENQNSGKVSECAENQARLQRNLMYLAAIADAQT
Arath_SYT1     VTSDHIQQYLDENKSLILKIVESQNSGKLSECAENQARLQRNLMYLAAIADAQT
Brana_SYT1     VTSDHIQQYLDENKSLILKIVESQNSGKLSECAENQARLQRNLMYLAAIADAQT
Lyces_SYT1     VTTDHIQQYLDENKSLILKIVESQNSGKLSECAENQARLQRNLMYLAAIADAQT
Soltu_SYT1.1   VTTDHIQQYLDENKSLILKIVESQNSGKLSECAENQARLQRNLMYLAAIADAQT
Soltu_SYT1.2   VTTDHIQQFLDENKSLILKIVESQNSGKISECAESQAKLQRNLMYLAAIADAQT
Helan_SYT1     VTTDHIQQYLDENKSLILKIVESQNSGKMAECAEHQAKLQRNLMYLAAIADAQT
Citsi_SYT1     VTTDHIQQYLDENKSLILKIVESQNSGKLSECAENQARLQRNLMYLAAIADAQP
Goshi_SYT1     VTTDHIQQYLDENKSLILKIVESQNSGKLSECAENQARLQRNLMYLAAIADSQP
Poptr_SYT1     VTTDHIQQYLDENKSLILKIVESQNSGKLSECAENQARLQQNLMYLAAIADCQP
Vitvi_SYT1.1   VTTDHIQQYLDENKSLILKIVESQNSGKLTECAENQARLQRNLMYLAAIADSQP
Glyma_SYT1.1   VTTDHIQQYLDENKSLILKIVESQNSGKLSECAENQSRLQRNLMYLAAIADSQP
Medtr_SYT1     VTTDHIQQYLDENKSLILKIVESQNTGKLTECAENQSRLQRNLMYLAAIADSQP
Glyma_SYT1.2   VTTDHIQQYLDENKSLILKIVESQNSGKLSECAENQARLQRNLMYLAAIADSQP
Vitvi_SYT1.2   ITTDHIQQYLDENKSLILKILESQNSGKLSECAENQARLQRNLMYLAAIADCQP
Aspof_SYT1     VTTDIIQQYLDENKQLILAILENQNSGKADECAENQAKLQRNLMYLAAIADSQP
Orysa_SYT1     VTTDLIQQYLDENKQLILAILDNQNNGKVEECARNQAKLQHNLMYLAAIADSQP
Sacof_SYT1     VTTDLIQQYLDENKQLILAILDNQNNGKVEECERHQAKLQHNLMYLAAIADSQP
Sorbi_SYT1     VTTDLIQQYLDENKQLILAILDNQNNGKVEECERHQAKLQHNLMYLAAIADSQP
Zeama_SYT1     VTTDLIQQHLDENKQLILAILDNQNNGKAEECERHQAKLQHNLMYLAAIADSQP
Triae_SYT1     VTTDLIQQYLDENKQLILAILDNQNNGKVEECARNQAKLQQNLMYLAAIADSQP
Cryja_SYT1     ITTDHIQKYLDENKQLILAIMDNQNLGKLNECAQYQAKLQQNLMYLAAIADSQP
Picsi_SYT1     ITTDHIQKYLDENKQLILAILDNQNLGKLNECAQYQAKLQQNLMYLAAIADSQP
Pinta_SYT1     ITTDHIQKYLDENKQLILAILDNQNLGKLNECAQYQAKLQQNLMYLAAIADSQP
Welmi_SYT1     ITTDHIQKYLDENRQLILAILDNQNLGKLNECAQYQARLQQNLMYLAAIADSQP
Phypa_SYT1.1   ITTELIQKYLDENKQLILAILDNQNLGKLNECAMYQAKLQQNLMYLAAIADAQP
Phypa_SYT1.2   ITTELIQKYLDENKQLILAILDNQNLGKLNECATYQAKLQQNLMYLAAIADAQP
Phypa_SYT1.3   ITTELIQKYLDENKQLILAILDNQNLGKLNECATYQAKLQQNLMYLAAIADAQP
Phypa_SYT1.4   ITTELIQKYLDENKQLILAILDNQNLGKLNECATYQAKLQQNLMYLAAIADAQP
Homsa_SNH      ITPAAIQKMLDDNNHLIQCIMDSQNKGKTSECSQYQQMLHTNLVYLATIADSNQ
Chlre_SYT      MTTDKIQDMLEENFKFIKAIAEQQNLGRVQEVHQYQQKLQENLMLLAAVADTYS
Volca_SYT      MTTERIQEMLEENFKFIKALAEQQNLGRMQDVIQFQQKLQENLMLLAAVADTYS
Consensus      ITTEQIQKYLDENKQLILAILENQNLGKLAECAQYQA LQKNLMYLAAIADAQP
```

# FIGURE 5 (continued)

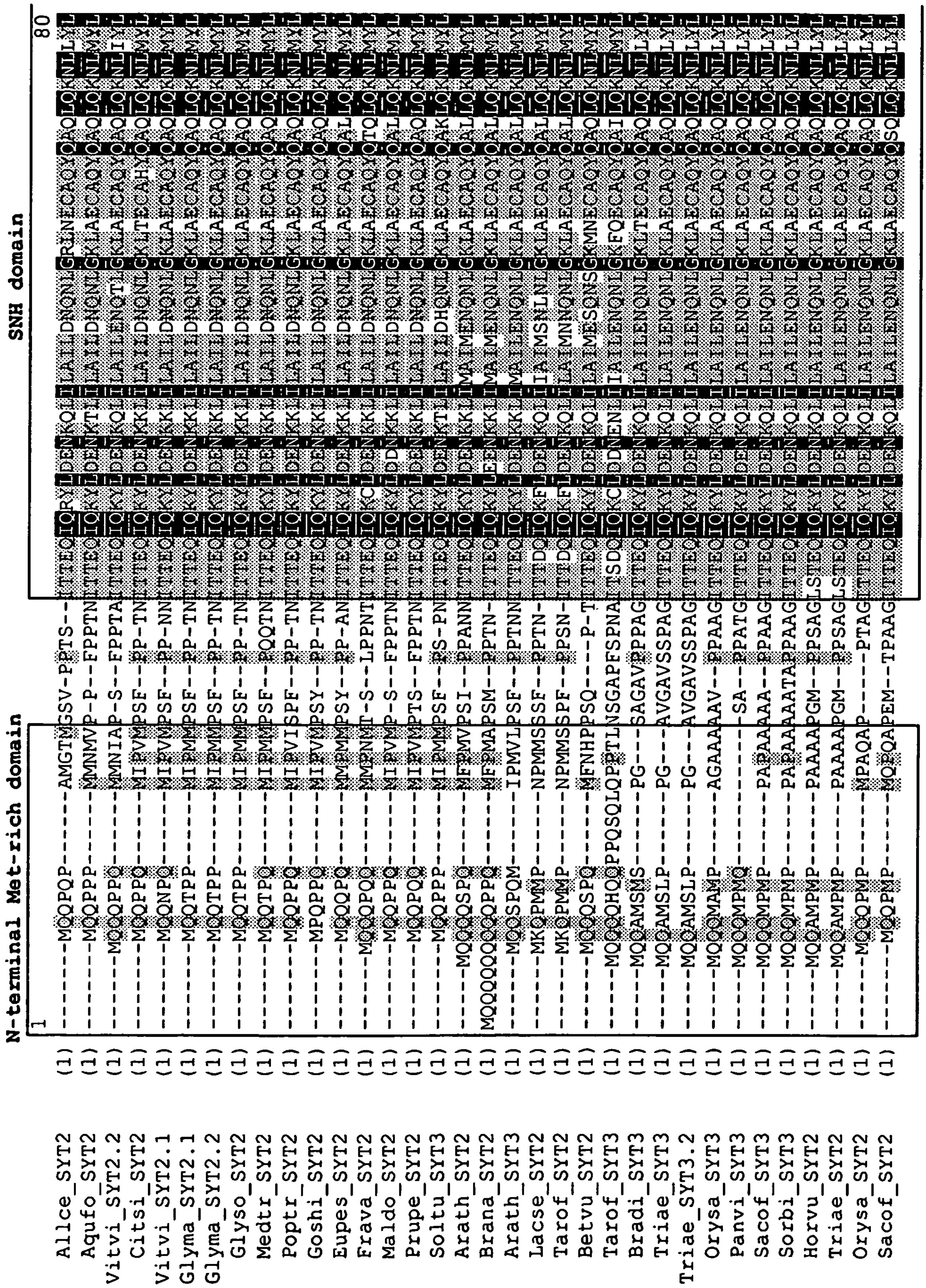


FIGURE 6

```
                              N-terminal Met-rich domain                 SNH domain

Sorbi_SYT2    (1) -------MQQPMP------MQPQAPAM--TPAAGITTEQIQKYLDENKQLILAILENQNLGKLAECAQYQSQLQKNLLYL
Zeama_SYT2    (1) -------MQQPMH------MQPQAPAI--TPAAGISTEQIQKYLDENKQLILAILENQNLGKLAECAQYQSQLQKNLLYL
Curlo_SYT2    (1) ------MQQSPHS------LAP---MS-AAPVANITTEQIQKYLDENKQLILAILENQNLGKLAECAQYQAQLQKNLLYL
Aqufo_SYT1    (1) --------MQHMQ------MQPMMPPY-SANS--VTTDHIQQYLDENKALILKILENQNSGKVSECAENQARLQRNLMYL
Arath_SYT1    (1) ------MQQHLMQ------MQPMMAGY-YPSN--VTSDHIQQYLDENKSLILKIVESQNSGKLSECAENQARLQRNLMYL
Brana_SYT1    (1) ------MQQHLMQ------MQPMMAGY-YPSN--VTSDHIQQYLDENKSLILKIVESQNSGKLSECAENQARLQRNLMYL
Lyces_SYT1    (1) ------MQQHLMQ------MQPMMAAY-YPTN--VTTDHIQQYLDENKSLILKIVESQNSGKLSECAENQARLQRNLMYL
Soltu_SYT1.1  (1) ------MQQHLMQ------MQPMMAAY-YPTN--VTTDHIQQYLDENKSLILKIVESQNSGKLSECAENQARLQRNLMYL
Soltu_SYT1.2  (1) -----MQQQHLMQ------MQPMMAAY-YPNN--VTTDHIQQFLDENKSLILKIVESQNSGKISECAESQAKLQRNLMYL
Helan_SYT1    (1) ------MQQHLMQ------MQPMMAAY-YPTNN-VTTDHIQQYLDENKSLILKIVESQNSGKMAECAEHQAKLQRNLMYL
Citsi_SYT1    (1) ------MQQHLMQ------MQPMMAAY-YPNN--VTTDHIQQYLDENKSLILKIVESQNSGKLSECAENQARLQRNLMYL
Goshi_SYT1    (1) ------MQQHLMQ------MQPMMAAY-YPNN--VTTDHIQQYLDENKSLILKIVESQNSGKLSECAENQARLQRNLMYL
Poptr_SYT1    (1) ------MQQHLMQ------MQPMMAAY-YPSN--VTTDHIQQYLDENKSLILKIVESQNSGKLSECAENQARLQQNLMYL
Vitvi_SYT1.1  (1) ------MQQHLMQ------MQPMMAAY-YPSN--VTTDHIQQYLDENKSLILKIVESQNSGKLTECAENQARLQRNLMYL
Glyma_SYT1.1  (1) ------MQQHLMQ------MQPMMAAY-YPNN--VTTDHIQQYLDENKSLILKIVESQNSGKLSECAENQSRLQRNLMYL
Medtr_SYT1    (1) ------MQQHLMQ------MQPMMAAY-YPNN--VTTDHIQQYLDENKSLILKIVESQNTGKLTECAENQSRLQRNLMYL
Glyma_SYT1.2  (1) ------MQQHLMQ------MQPMMAGY-YPNN--VTTDHIQQYLDENKSLILKIVESQNSGKLSECAENQARLQRNLMYL
Vitvi_SYT1.2  (1) ------MQQHLMQ------MQPMMAGS-HNLSS-ITTDHIQQYLDENKSLILKILESQNSGKLSECAENQARLQRNLMYL
Aspof_SYT1    (1) ------MQQHLMQ------MQP-MMAT-YGSPNQVTTDIIQQYLDENKQLILAILENQNSGKADECAENQAKLQRNLMYL
Orysa_SYT1    (1) -----MQQQHLMQ------MNQGMMGG-YASPTTVTTDLIQQYLDENKQLILAILDNQNNGKVEECARNQAKLQHNLMYL
Sacof_SYT1    (1) -----MQQQHLMQ------MNQNMIGG-YTSPAAVTTDLIQQYLDENKQLILAILDNQNNGKVEECERHQAKLQHNLMYL
Sorbi_SYT1    (1) -----MQQQHLMQ------MNQNMIGG-YTSPAAVTTDLIQQYLDENKQLILAILDNQNNGKVEECERHQAKLQHNLMYL
Zeama_SYT1    (1) -----MQQQHLMQ------MNQNMMGG-YTSPAAVTTDLIQQHLDENKQLILAILDNQNNGKAEECERHQAKLQHNLMYL
Triae_SYT1    (1) -----MQQQHLMQ------MNQSMMGG-YASSTTVTTDLIQQYLDENKQLILAILDNQNNGKVEECARNQAKLQQNLMYL
Cryja_SYT1    (1) ------MQQHLMQ------MQPMMAAA-YASNN-ITTDHIQKYLDENKQLILAIMDNQNLGKLNECAQYQAKLQQNLMYL
Picsi_SYT1    (1) ------MQQHLMQ------MQPMMAA--YASNN-ITTDHIQKYLDENKQLILAILDNQNLGKLNECAQYQAKLQQNLMYL
Pinta_SYT1    (1) ------MQQHLMQ------MQPMMAA--YASNN-ITTDHIQKYLDENKQLILAILDNQNLGKLNECAQYQAKLQQNLMYL
Welmi_SYT1    (1) -----------MQ------MQPMIGAG-YSSNS-ITTDHIQKYLDENRQLILAILDNQNLGKLNECAQYQARLQQNLMYL
Phypa_SYT1.1  (1) -------------------MQQMAAY-TGTS--ITTELIQKYLDENKQLILAILDNQNLGKLNECAMYQAKLQQNLMYL
Phypa_SYT1.2  (1) -------------------MQQMAPY-AGTS--ITTELIQKYLDENKQLILAILDNQNLGKLNECATYQAKLQQNLMYL
Phypa_SYT1.3  (1) ------------------MMQHMATY-ASSN--ITTELIQKYLDENKQLILAILDNQNLGKLNECATYQAKLQQNLMYL
Phypa_SYT1.4  (1) ------------------MMQHMTTY-ASSN--ITTELIQKYLDENKQLILAILDNQNLGKLNECATYQAKLQQNLMYL
Chlre_SYT     (1) ---------------------MAAAS---KPPPMTTDKIQDMLEENFKFIKAIAEQQNLGRVQEVHQYQQKLQENLMLL
Volca_SYT     (1) ---------------------MAAVSGQAKPAPMTTERIQEMLEENFKFIKALAEQQNLGRMQDVIQFQQKLQENLMLL
Consensus     (1)       QQ MQ        M P M      P    ITTEQIQKYLDENK LILAILENQNLGKL ECAQYQA LQKNLMYL
```

FIGURE 6 (continued)

Met-rich / QG-rich domain

```
                    81                                                                             160
   Allce_SYT2  (65) AAIADAQHQ--SPAV------RLQMMP-QGAAATPQAGN------------QFMQQQSPN--------------------
   Aqufo_SYT2  (65) AAIADAQH----QAP------AVPSQMPTHPAMQQGG--------------HYMQHPQAAMPQQ----------------
 Vitvi_SYT2.2  (66) AAIADAQH----PAP------TVPPQMPIHHAMQQG---------------HYMQHPQAAAAQQQ---------------
   Citsi_SYT2  (65) AAIADAQH----QAP------TMPPQMAPHPAMQASG--------------YYMQHPQAAAMAQ----------------
 Vitvi_SYT2.1  (65) AAIADAQH----QAPP-----TMPPQMAPHPAMQQGG--------------YYMQHPQAAAMAQ----------------
 Glyma_SYT2.1  (65) AAIADAQH----QTP------AMPPQMAPHPAMQPG---------------FYMQHPQAAAAAMAQQQ------------
 Glyma_SYT2.2  (65) AAIADAQH----QTP------AMPPQMAPHPAMQPG---------------FYMQHPQAAAAAMAQQQ------------
   Glyso_SYT2  (65) AAIADAQH----QTP------AMPPQMAPHPAMQPG---------------FYMQHPQAAAAAMAQQQ------------
   Medtr_SYT2  (66) AAIADAQH----QTP------ALPPQMAPHPAMQQG---------------FYMQHPQAAAMAQQQ--------------
   Poptr_SYT2  (65) AAIADAQH----QAP------AMPPQMAPHPAMQQGA--------------YYMQHPQAAAMAQ----------------
   Goshi_SYT2  (65) AAIADAQH----QSTP-----AMSPQMAPHPAMQPGG--------------YFMQHPQAAAMSQ----------------
   Eupes_SYT2  (65) AAIADAQH----QTP------PMPPQMSPHPAMQQGA--------------YYMQHPQAAAAAMAH--------------
   Frava_SYT2  (66) AAIADAQHQPQPQAPA-----MPAQQLAPHPAMQQAG--------------YYMQHPQAAAAMAQQQ-------------
   Maldo_SYT2  (65) AAIADAQH----QAPA-----APP-QMAPHPAMQQAG--------------YYMQHPQAAAMAQQQ--------------
   Prupe_SYT2  (66) AAIADAQH----QAPT-----VPA-QMAPHPAMQQAG--------------YYMQHPQAAAMAQQQ--------------
   Soltu_SYT3  (65) AAIADAQH----QSP------AIPTQMAPHPAMQQGG--------------FYMQHPQAAAMTQQQ--------------
   Arath_SYT2  (68) AAIADAQHP--PPTPGPSPSTAVAAQMATPHSGMQPP-S------------YFMQHPQAS--------------------
   Brana_SYT2  (72) AAIADAQHP--PSTAGATPPPAMASQMGAPHPGMQPP-S------------YFMQHPQASGMAQQAP-------------
   Arath_SYT3  (67) AAIADAQHQ--PPAATLTSGAMTPQAMAPNPSSMQPPPS------------YFMQQHQAVG-MAQQI-------------
   Lacse_SYT2  (66) AAIADAQHP--TPTP--TLNISYXMGPVPHPGMPQQGGF------------YMAQQHPQAAVMTAQP-------------
   Tarof_SYT2  (66) AAIADAQHP--TPTP--TPNISSQMGPVPHPGMPQQGGF------------YMG-QHPQAAVMAAQP-------------
   Betvu_SYT2  (64) AAIADAQHPAPTGPS------QSQPQNSQMPMQSTIPQG------------PFMPPPKP---------------------
   Tarof_SYT3  (76) AAIADAQHP--TQQP-------STPQMPPNSIPQQPN--------------NYMQQQHQITAPQQ---------------
   Bradi_SYT3  (67) AAIADAQH---PQNP------GSRPQMVQPGGMPGAG--------------HYMSQVPMFPPR-----------------
   Triae_SYT3  (67) AAIADAQH---PQNP------TSHPQMVQPGSMQGAG--------------HYMSQVPMFPPR-----------------
 Triae_SYT3.2  (67) AAIADAQH---PQNP------TSHPQMVQPGSMQGAG--------------HYMSQVPMFPPR-----------------
   Orysa_SYT3  (68) AAIADAQH---PQNP------GSRPQMMQPGATPGAG--------------HYMSQVPMFPPR-----------------
   Panvi_SYT3  (62) AAIADAQHQP-PQNP------ASRPQMMQPGMVPGAG--------------HYMSQVPMFPPR-----------------
   Sacof_SYT3  (68) AAIADAQHQP-PQNP------AGRPQMMQPGIVPGAG--------------HYMSQVPMFPPR-----------------
   Sorbi_SYT3  (70) AAIADAQHRP-PQNP------AGRPQMMQPGIVPGAG--------------HYMSQVPMFPPR-----------------
   Horvu_SYT2  (68) AAIADTQH----QTS------VSRPQMAPPAASPGAG--------------HYMSQVPMFPPR-----------------
   Triae_SYT2  (68) AAIADTQH----QTT------VSRPQMAPPSASPGAG--------------HYMSQVPMFPPR-----------------
   Orysa_SYT2  (64) AAIADTQH----QTT------ISRPQMVPHGASPGLGG-------------QYMSQVPMFPPR-----------------
   Sacof_SYT2  (66) AAIADAQH----QTA------VSRPQMAPPGALPGVG--------------QYMSQVPMFPPR-----------------
```

**FIGURE 6 (continued)**

Met-rich / QG-rich domain

```
Sorbi_SYT2    (66) AAIADAQF----QTA------VSRPQMAPPGALPGVG-------------QYMSQVPMFPPR----------------
Zeama_SYT2    (66) AAIADAQF----QTA------VSRPQMAPPGGSPGVG-------------QYMSQVPMFPPR----------------
Curlo_SYT2    (65) AAIADAQF----NAP------AVRPQQIMPHGTIPQGS------------PFMQQSPIFPRGP---------------
Aqufo_SYT1    (64) AAIADSQFQ--PPNM------HAQYSN--A-GIPPGA-------------HYLQHQQAQQ------------------
Arath_SYT1    (66) AAIADSQFQ--PPSV------HSQYGSAGGGMIQGEGGS-----------HYLQQQQATQQQ----------------
Brana_SYT1    (66) AAIADSQFQ--PPSV------HSQYGSAGGGLIQGEGAS-----------HYLQQQQATQQQ----------------
Lyces_SYT1    (66) AAIADSQFQ--PSSM------HSQFSS--GGMMQPGTHS-----------YLQQQQQQQQAQ----------------
Soltu_SYT1.1  (66) AAIADSQFQ--PSSM------HSQFSS--GGMMQPGTHS-----------YLQQQQQQQQAQ----------------
Soltu_SYT1.2  (67) AAIADSQFQ--PPSM------HSQLAS--GGMMQGGAHY-----------MQQQQ-----AQ----------------
Helan_SYT1    (67) AAIADSQFQ--APSL------HSQYP--QGGMMQQQAGS-----------HYMQQHQQAQ------------------
Citsi_SYT1    (66) AAIADAQFQ--PPSV------HAQFSS--GGIMQPGA-------------HYMQHQQSQP------------------
Goshi_SYT1    (66) AAIADSQFQ--PPTV------HAQFPS--GGIMQPGAG------------HYMQHQQAQQ------------------
Poptr_SYT1    (66) AAIADCQFQ--PPTM------HAQFPS--SGIMQPGA-------------HYMQHQQAQQ------------------
Vitvi_SYT1.1  (66) AAIADSQFQ--PPTM------HAQFPP--SGIVQPGA-------------HYMQHQQAQQ------------------
Glyma_SYT1.1  (66) AAIADSQFQ--PSPL------AGQYPS--SGLVQQGA-------------HYMQAQQAQQ------------------
Medtr_SYT1    (66) AAIADSQFQ--PPTM------PGQYPS--SGMMQQGG-------------HYMQAQQAQQ------------------
Glyma_SYT1.2  (66) AAIADSQFQ--PPTM------SGQYPP--SGMMQQGAQ------------YMQAQQQAQQ------------------
Vitvi_SYT1.2  (67) AAIADCQFQ--PPSL------QAQFSP--NMVMQPGVN-------------YMQHQQSQQ------------------
Aspof_SYT1    (67) AAIADSQF---QVPT------IAQYPPNAVAAMQSSA-------------RYMQQHQAAQ------------------
Orysa_SYT1    (69) AAIADSQFP--QTAA------MSQYPSN--LMMQSGA-------------RYMPQ-QSAQ------------------
Sacof_SYT1    (69) AAIADSQFP--QTAP------LSQYPSN--LMMQPGP-------------RYMPP-QSGQ------------------
Sorbi_SYT1    (69) AAIADSQFP--QTAP------LSQYPSN--LMMQPGP-------------RYMPP-QSGQ------------------
Zeama_SYT1    (69) AAIADSQFP--QTAP------LSQYPSN--LMMQPGP-------------RYMPP-QSGQ------------------
Triae_SYT1    (69) AAIADSQFP--QTAS------LSQYPSN--LMMQSGP-------------RYMQQ-QSAQ------------------
Cryja_SYT1    (67) AAIADSQFQ--VPAA------HAQIPP--NAVVQSGG-------------LFMQHQQAQQ------------------
Picsi_SYT1    (66) AAIADSQFQ--AQTA------HAQIPP--NAVMQSGG-------------HYMQHQQAQQ------------------
Pinta_SYT1    (66) AAIADSQFQ--AQTA------HAQIPP--NAVMQSGG-------------HYMQHQQAQQ------------------
Welmi_SYT1    (62) AAIADSQFQ--TPAA------HAQIAS--NAMLQAGG-------------HYMQHQQT--------------------
Phypa_SYT1.1  (58) AAIADAQFQ--VSQN-------STQVS-SGQSMQPSQ-------------QYIQQQQQQM------------------
Phypa_SYT1.2  (58) AAIADAQFQ--VP--------------AAQPMQPSQ-------------QYIQQQQQQ--------------------
Phypa_SYT1.3  (59) AAIADAQFQ--GPSS------QMPAPA-PAPTMQPAQ-------------QYMQQQQQLR------------------
Phypa_SYT1.4  (59) AAIADAQFQ--GPS--------TQMPA-PAPTMQPAQ-------------QYMQQQQQLR------------------
Chlre_SYT     (56) AAVADTYSNSAAAAQP--GGEAGAAAPAAATARPPTAPG-------APLGAPGAPPPAAP------------------
Volca_SYT     (59) AAVADTYSSASATGAA--AQATAAPGMAAQAARPPAAALPGTAVATAALPLPGLPQQQQPQQQQQPQQQPGQPSPLMMVM
Consensus     (81) AAIADAQF                           MQ                 YMQQ Q
```

FIGURE 6 (continued)

```
                                  Met-rich / QG-rich domain (continued)
                     161                                                                            240
   Allce_SYT2  (104) ----------------FPPKTGMQFT---PQQVQELQQQ------QLQHQPHMMP------PFQGQMGMRPMNG------
   Aqufo_SYT2  (105) --------------PSGFPPKSP------MQFNPQQMQEQQRL-QLQQQHQQ---------ALQGHMGIRPGVNNGLQ--
 Vitvi_SYT2.2  (106) --------------PGMFGAKLP------FQLSDQQQQ-QQHH-FLHLQQQQ---------PIQGLMGMRPIINNGMH--
   Citsi_SYT2  (105) -------------QQGIFPQKMP-LQ---FNNPHQLQDPQQ-----QLHQHQ---------AMQAQMGMRPGATNNGM--
 Vitvi_SYT2.1  (106) -------------QPGLFPPKMP-LQ---FGNPHQLQEQAQQL---QQLQQQ---------AMQGQMGMRPGGANNGM--
 Glyma_SYT2.1  (108) -------------Q-GMFPQKMP-LQ---FGNPHQMQEQQQQ------LHQQ---------AIQGQMGLRPGDINNGM--
 Glyma_SYT2.2  (108) -------------QQGMFPQKMP-LQ---FGNPHQMQEQQQQ------LHQQ---------AIQGQMGLRPGGINNGM--
   Glyso_SYT2  (108) -------------QQGMFPQKMP-LQ---FGNPHQMQEQQQQ------LHQQ---------AIQGQMGLRPGGINNGM--
   Medtr_SYT2  (107) ---------------GMFPQKMP-MQ---FGNPHQMQDQQHQQ-QQQQLHQQ---------AMQGQMGLRPGGINNGM--
   Poptr_SYT2  (105) -------------QPGVFPQKML-LQ---FNAGHQMQDPQQ------LH-QQ---------AMQGQIGIRPIGANNGM--
   Goshi_SYT2  (106) -------------QPGMYPQKVP-LQ---FNSPHQMQDPQHLL-Y--QQHQQ---------AMQGQMGIRPGGPNNSM--
   Eupes_SYT2  (107) -------------QSGIFPPKMSPLQ---FNNPHQIQDPQQ------LH-QA---------ALQGQMGMRPMGPNNGM--
   Frava_SYT2  (114) ---------------GLFPQKMQ-MQ---FNSPQQMHEMQQ------QLHQQ---------AMHGQMGMRPGGANGMP--
   Maldo_SYT2  (107) ---------------GIFSPKMP-MQ---FNNMHQMHDP--------QQHQQ---------AMQGQMGMRPGGPNGMP--
   Prupe_SYT2  (108) ---------------GIFPPKMP-LQ---FNNPHQMHDAAQQL-H--QQHQQ---------AMQGQMGMRAGGANGMP--
   Soltu_SYT3  (107) ---------------GMFTSKMP-LQ---FNNPQQLHDQQQLQ-H-QHQHQQ---------LQRQQQGMQLGGANSGM--
   Arath_SYT2  (113) -------------PAGIFAPRGP-LQ---FGSPLQFQDPQQQQ----QIHQQ---------AMQGHMGIRPMGMTNN---
   Brana_SYT2  (124) -------------PAGIFPPRGP-LQ---FGSPHQLQDPQQQ-----HMHQQ---------AMQGHMGMRPMGINNNN--
   Arath_SYT3  (119) -------------PPGIFPPRGP-LQ---FGSPHQFLDPQQQ------LHQQ---------AMQGHMGIRPMGLNNNN--
   Lacse_SYT2  (117) -------------PSGFPQPMPG-MQ---FNSP----Q-----------------------AIQGQMGGRSGGPPSS---
   Tarof_SYT2  (116) -------------PSGFPQPMPG-MQ---FNTP----Q-----------------------GIQGQMGGRSGGPPNS---
   Betvu_SYT2  (105) ---------------AVTPQQTGPRLPFALQSFDQ-QSP-----HMQMQYQQS---------MAGSMGMRMGGNN-----
   Tarof_SYT3  (118) --------------GGIGGGGGVPKLPFQLNALRTQDQQQQ---LLQFQQQQ---------QLQAQMGMRPSSQDGMLG-
   Bradi_SYT3  (107) --------------TPLTPQQMQEQQ---HQQ--LQQQQ---------AQAL---------AFPSQMVMRPGTVNGMQ--
   Triae_SYT3  (107) --------------TPLTPQQMQEQQ---HQQ--LQQQQ---------AQAL---------SFPAQVVMRPGTVNGMQ--
 Triae_SYT3.2  (107) --------------TPLTPQQMQEQQ---HQQ--LQQQQ---------AQAL---------SFPAQVVMRPGTVNGMQ--
   Orysa_SYT3  (108) --------------TPLTPQQMQEQQ---QQQ--LQQQQ---------AQAL---------AFPGQMLMRPGTVNGMQ--
   Panvi_SYT3  (104) --------------TPLTPQQMQEQQ---QQQQQLQQQQ---------AQAL---------AFPGQMVMRP-TINGMQ--
   Sacof_SYT3  (110) --------------TPLTPQQMQEQQ---QQQ--LQQQQ---------AQAL---------TFPGQMVMRPATINGIQ--
   Sorbi_SYT3  (112) --------------TPLTPQQMQEQQ---QQQ--LQQQQ---------AQAL---------AFPGQMVMRPATINGMQ--
   Horvu_SYT2  (107) --------------TPLTPQQMQEQQ---LQQ----QQ----------AQML---------PFAGQMVARPGAVNGIP--
   Triae_SYT2  (107) --------------TPLTPQQMQEQQ---LQQ----QQ----------AQML---------PFAGQMVARPGAVNGMP--
   Orysa_SYT2  (104) --------------TPLTPQQMQEQQ---LQQ----QQ----------AQLL---------SFGGQMVMRPGVVNGIP--
   Sacof_SYT2  (105) --------------TPLTPQQMQEQQ---LQQ----QQ----------AQLL---------NFSGLMVARPGMVNGMP--
```

FIGURE 6 (continued)

```
                                 Met-rich / QG-rich domain (continued)
Sorbi_SYT2    (105) --------------TPLTPQQMQEQQ---LQQ----QQ----------AQLL---------NFSGQMVARPGMVNGMP--
Zeama_SYT2    (105) --------------TPLTPQQMQEQQ---LQQ----QQ----------AQLL---------NFSGQMVARPGMVNGMA--
Curlo_SYT2    (106) --------------LPYNPQQMQGQL---HPQ---------------PPGM---------VFPGHMGIRPGAVNGLH--
Aqufo_SYT1    (100) ----------------MTQQSLMAAR---SNMLYAQPITG-------MQQQQ---------AMHSQLGMSSGGNSG----
Arath_SYT1    (109) ---------------QMTQQSLMAAR---SSMLYAQQQQQQ---QPYATLQHQ-------QLHHSQLGMSSSSGGG--GS
Brana_SYT1    (109) ---------------QMTQQSLMAAR---SSMMYQQQQ------QPYATLQHQ-------QLHHSQLGMSSSSGGG---S
Lyces_SYT1    (107) ---------------QMATQQLMAAR--SSSMLYGQQQQQ-----SQLSQYQQ-------GLHSSQLGMSSGSGGS---T
Soltu_SYT1.1  (107) ---------------QMATQQLMAAR--SSSMLYGQQQQQQQQ--SQLSQFQQ-------GLHSSQLGMSSGSGGS---T
Soltu_SYT1.2  (103) ---------------QLTTQSLMAAARSSSSMLYGQQQQQQQQQLSSLQQQQA-------AFHSQQLGMSSSGGGS---S
Helan_SYT1    (106) ---------------QMSPQALMAAR---SSMMYSQQQ---------YSSLQQ-------QAMHSHLGMSSGTGTS----
Citsi_SYT1    (103) ----------------MTPQSLMAAR---SSMVYS-QQQ-----FSVLQQQQ---------ALHGQLGMSSGGSSG----
Goshi_SYT1    (104) ----------------MTQQSLMAAR---SSMLYS-QQP-----FSALQQQQQ-------QALHSQLGMSSGGSTG----
Poptr_SYT1    (103) ----------------MTPQALMAAR---SSMLQYAQQP-----FSALQQQQ---------ALHSQLGMSSGGSAG----
Vitvi_SYT1.1  (103) ----------------MTPQSLLAAR---SSMLYT-QQP-----FSALQQQQ---------AIHSQLGMGSGGSAG----
Glyma_SYT1.1  (103) ----------------MSQQQLMASR---SSLLYS-QQP-----FSVLQQQQ---------GMHSQLGMSSSGSQG----
Medtr_SYT1    (103) ----------------MTQQQLMAAR---SSLMYA-QQ---------LQQQQ---------ALQSQLGMNSSGSQG----
Glyma_SYT1.2  (104) ----------------MTPQQLMAAR---SSLLYA-QQP-----YSALQQQQ---------AMHSALGSSSG--------
Vitvi_SYT1.2  (104) ----------------MMPQSLMAAR---APMLYAQQHP-----YLALQQQQ---------ALQSQLGMSSTGMGG----
Aspof_SYT1    (105) ---------------QMTPQSLMAAR---SSMLYS-QSP-----MSALQQQQQQ------AAMHSQLAMSSGGNNS-S-T
Orysa_SYT1    (105) ---------------MMAPQSLMAAR---SSMMYA-QPA-----LSPLQQQQQQ----QAAAAHGQLGMGSGG----T-T
Sacof_SYT1    (105) ---------------MMSPQSLMAAR---SSMMYA-HPS-----MSPLQQQQ---------AAHGQLGMASGGGGG-T-T
Sorbi_SYT1    (105) ---------------MMSPQSLMAAR---SSMMYA-HPS-----MSPLQQQQ---------AAHGQLGMASGGGGG-T-T
Zeama_SYT1    (105) ---------------MMNPQSLMAAR---SSMMYA-HPS-----LSPLQQQQ---------AAHGQLGMAPGGGGGGT-T
Triae_SYT1    (105) ---------------MMSPQSLMAAR---SSMMYA-QQA-----MSPLQQQQQQQ---HQAAAHGQLGMSSG-----A-T
Cryja_SYT1    (104) ---------------QVTPQSLMAAR---SSMLYTQQPMAA---LHQAQQQQ------QQQSLHSHLGISSGGSNG----
Picsi_SYT1    (103) ---------------QVTPQSLMAAR---SSMLYSQQPMAA---LHQAQQQQQQQHQQQQQSLHSQLGINSGGSSG----
Pinta_SYT1    (103) ---------------QVTPQSLMAAR---SSILYAQQ-------QQQQQHQQHQQQQQQQQSLHSQLGINSGGSSG----
Welmi_SYT1    (97)  ---------------VTPQSLLAAR---SSMLYSQQPMTA---FHQAQQQQ------QQQSLHGQLGINSGGNNG----
Phypa_SYT1.1  (96)  ----------------MMMNQRNSIP----QYMQQSQQGSPN--APSPQ-QQS-------YHSQQPQGMVPQRNSD----
Phypa_SYT1.2  (87)  ----------------MMMNQRN-------QYLQQNQQGVQN--APSPQSQQS-------YHNQQP-GMVSQGNSG----
Phypa_SYT1.3  (97)  ----------------MMSQQNSMIP----SYLQHSQQ----------ASQQN-------FYSQQS--LLSGGGGS----
Phypa_SYT1.4  (95)  ----------------MMSQQNAMIP----SYLQQSQQ----------VSQQN-------FYSQQS--LLTGGGSS----
Chlre_SYT     (107) ---------------ALTPQQIHAAVQQALAMKQQQQQQ---------QQQQ---------PQQPQQSAVAQYQQPPQ--
Volca_SYT     (137) PGSSGTQAPTQLGAMQLTQQQIQAAVQQALVRQQQQQQQ---------QQQQNPN------PFQGQQFQLPQSLQQPQS-
Consensus     (161)                   PQ                 QQ          QQQ         A  GQ GMRPGG NG
```

FIGURE 6 (continued)

```
                              Met-rich / QG-rich domain (continued)
                     241                                                                            320
    Allce_SYT2  (147) ------------------------------MQAAMHADSSLAYNTN-------------------NKQDAGNAAYEN--
    Aqufo_SYT2  (153) ---MHGDGNVGGSSSGGPSSTGNLPDFSRSGAGPGAGSSSLDAREG--------------------KQDGVEAG-----
  Vitvi_SYT2.2  (153) ---QAMQTGLG-----------------------ALSGFMDVRGS--------------------KPDGSEVG-----
    Citsi_SYT2  (152) ---HPMHAESSLGG--------------GSSGGPPSASGPGDIRGG--------------------NKQDASEAGTT---
  Vitvi_SYT2.1  (155) ---HPMHPEATLGG--------------GSSGGPPPSAGLSDARGG--------------------GKQDTSEAGAS---
  Glyma_SYT2.1  (153) ---HPMHSEAALGG--------------GNSGGPPSATGPNDARGG--------------------SKQDASEAGTA---
  Glyma_SYT2.2  (154) ---HPMHNE----G--------------GNSGGPPSATGPNDARGG--------------------SKQDASEAGTA---
    Glyso_SYT2  (154) ---HPMHNE----G--------------GNSGGPPSATGPNDARGG--------------------SKQDASEAGTA---
    Medtr_SYT2  (156) ---HPMHNEAALGG--------------SGSG------GPNDGRGGG-------------------SKQDASEAGTA---
    Poptr_SYT2  (150) ---HPMHAEIALG-----------------SSGPSASAGTNDVRGG--------------------SKQDASEAGTT---
    Goshi_SYT2  (155) ---HPMHSEASLGG--------------GSSGGPPQPSGPSDGRAG--------------------NKQEGSEAG-----
    Eupes_SYT2  (153) ---HPMHPEANLG-------------------------GSNDGRGG--------------------NKQDAPETGAS---
    Frava_SYT2  (158) ---SMHHTENTHG-------------------------------G--------------------SKQDNSEAG-A---
    Maldo_SYT2  (149) ---SMLHTEATHGG--------------GSGGPNS-AGDPNDGRGG--------------------SKQDASESG-A---
    Prupe_SYT2  (155) ---SMHHTEATLGG--------------GSGGPTSGGGGPNDGRGG--------------------KQQDYSEAG-T---
    Soltu_SYT3  (155) ---HSTLGSTS-----------------NVS--QLTTSGAGDARGG--------------------NKQDNSEAG-----
    Arath_SYT2  (160) ---GMQHAMQQ--P------------------ETGLGG---NVGLRG--------------------GK-------QD---
    Brana_SYT2  (171) ---GMQHQMQQQQP------------------ETSLGGSAANVGLRG--------------------GK-------QD---
    Arath_SYT3  (165) ---GLQHQMHHHET------------------ALAANNAGPNDASGG--------------------GKPDGTNMSQS---
    Lacse_SYT2  (150) -----AASDVWR-------------------------G--SMQDGG--------------------GG-AAADGGKD---
    Tarof_SYT2  (149) -----AGGDVWR-------------------------G--SMQDGG--------------------G--GGVDGGKD---
    Betvu_SYT2  (150) ----VLRPSIQTG-------------------YGAPTHFMDAR---------------------NRQDGSDAS-----
    Tarof_SYT3  (171) --MHQAMQSALAG---------------------NPGSLMDGRGN--------------------KQDGSEAAAS---
    Bradi_SYT3  (148) ---PMQADLQAAAAAPGLADSR------------GSKQ-DAAVAGAI-------------------SEPSGTESHKS---
    Triae_SYT3  (148) ------QPMQAAGDLQPAAAPG------------GSKQ-DAAVAGAS-------------------SEPSGTKSHKN---
  Triae_SYT3.2  (148) ------QPMQAAGDLQPAAAPG------------GSKQ-DAAVAGAS-------------------SEPSGTKSHKN---
    Orysa_SYT3  (149) ---SIPVADPARAADLQTAAPGSV--------DGRGNE----QD-AT-------------------SEPSGTESHKS---
    Panvi_SYT3  (146) ---PMQADPAAAAASLQQSAPGPT--------DGRGGK-QDATAGVS-------------------TEPSGTESHKST--
    Sacof_SYT3  (151) ---QPMQADPARAAELQQPPPIPA--------DGRVSKQQDTTAGVS-------------------SEPSANESHKTT--
    Sorbi_SYT3  (153) ---QPMQADPARAAELQQPASVPA--------DGRVSK-QDTAAGVS-------------------SEPSANESHKTT--
    Horvu_SYT2  (145) ---------QAPQVEQP---------------------AYAAGGAS-------------------SEPSGTESHRS---
    Triae_SYT2  (145) ---------QAPQVEP----------------------AYAAGGAS-------------------SEPSGTESHRS---
    Orysa_SYT2  (142) ---------QLLQGEMHR---G------------AD---HQNAGGAT-------------------SEPS--ESHRS---
    Sacof_SYT2  (143) ---------QSIQVQQAQPPPA------------GN---KQDAGGVA-------------------SEPSGIENHRS---
```

FIGURE 6 (continued)

**Met-rich / QG-rich domain (continued)**

```
   Sorbi_SYT2  (143) ---------QSIQAQQAQPSPA------------LN---KQDAGGVA------------------SEPSGTESHRS----
   Zeama_SYT2  (143) ---------QSMQAQLP---PG------------VN---KQDAGGVA------------------SEPSGTESHRS----
   Curlo_SYT2  (142) ---GSHTEPSHGGTANPLTTPSLSG----FPPTNSDGRGSKQEAGIA------------------MVPAVAESHRN----
   Aqufo_SYT1  (141) --LHMMHNEGS-------------------MGGSGALGSYSDYGRG-----SGGG-------VTIASKQDGGSGS-----
   Arath_SYT1  (159) SGLHILQGE--------------------------AGG-FHDFGRG---------------------KPEMGSGG-----
   Brana_SYT1  (155) SGLHILQGE--------------------------AGG-FHEFGRG---------------------KPEMGSG------
   Lyces_SYT1  (155) GLHHMLQSE----------------------SSPHGGGFSHDFGR--------------------ANKQDIGSSM-----
 Soltu_SYT1.1  (158) GLHHMLQSE----------------------SSPHGGGFSHDFGR--------------------ANKQDIGSSM-----
 Soltu_SYT1.2  (158) SGLHMLQSENT-------------------HSASTGGGGFPDFGRG----------------LGSGNKHEMGSSM-----
   Helan_SYT1  (148) -GLHMLQTDNNSAG---------------VSGTHLSGG-FPDFGR----------------------KQDIGPTG-----
   Citsi_SYT1  (145) --LHMLQSEGSTAG---------------GSGSLGGGG-FPDFGRG--SSGEGLHS------RGMGSKHDIGSSG-----
   Goshi_SYT1  (148) --LHMLQTESSTAG---------------GSGALGAGG-FPDFGRG--SSGEGIHGG---RPMAGGSKQDIGSAG-----
   Poptr_SYT1  (146) --LHMMQSEANTAG---------------GSGALGAGR-FPDFGMD--ASSRGIAS-------G--SKQDIRSAG-----
 Vitvi_SYT1.1  (145) --LHMLQSEGSNPG---------------GNGTLGTGG-FPDFSRG--TSGEGLQAAG--RGMAGGSKQDMGNAE-----
 Glyma_SYT1.1  (145) --LHMLQSEATNVG---------------GNATIGTGGGFPDFVR---------IGS---------GKQDIGISG-----
   Medtr_SYT1  (141) --LHMLHSEGANVG---------------GNSSLGAG--FPDFGRS--SAGDGLHGS---------GKQDIGST------
 Glyma_SYT1.2  (142) --LHMLQSE--------------------GSNVNVGGG-FPDFVRGGSSTGEGLHS-------G--GRGIIGSSK-----
 Vitvi_SYT1.2  (147) --IHMLQSEP--------------------NVGGNGTGAFSDLGRS--MTGEGLSAVS--RGLGSASKQDVGSVG-----
   Aspof_SYT1  (153) GGFTILHGEASIGG----------------NGSMNSGGVFGDFGRSSGG-------------------------------
   Orysa_SYT1  (152) SGFSILHGEASMGGGGGGG----G-----AGNSMMNAGVFSDFGRGGGGG----------------GKEGSTSLS-----
   Sacof_SYT1  (150) SGFNILHGEASMGG-AGGA----C-----AGNNMMNAGMFSGFGRSGSG-----------------AKEGSTSLS-----
   Sorbi_SYT1  (150) SGFSILHGEASMGG-AAGA----G-----TGNSMMNAGMFSGFGRSGSG-----------------AKEGSTSLS-----
   Zeama_SYT1  (151) SGFSILHGEASMGGGGAGA----G-----AGNNMMNAGMFSGFGRSGSG-----------------AKEGSTSLS-----
   Triae_SYT1  (152) TGFNLLHGEASMGGGGGG-----A-----SGNSMMNAGVFSDYRRGGSG-----------------AKEGSTSLS-----
   Cryja_SYT1  (153) --LHMLHGEANMG----------------GNGPLSSGG-FPDFSRGTGASGEGIQANRGMCIDRGANKHDGAGTENAHPG
   Picsi_SYT1  (158) --LHMLHGETNMG----------------CNGPLSSGG-FPEFGRGSATSAEGMQANRGFTIDRGSNKQDGVGSENAHPG
   Pinta_SYT1  (154) --LHMLHGETNMG----------------CNGPLSSGG-FPEFGRGSATSADGMQVNRGFAIDRGSNKQDGVGSENAHAG
   Welmi_SYT1  (145) --LHILHGETSMG----------------SNGPLTTGS-FPDFGRG-SVNCDLLQGNRSLTIDRGSSKIDGLGTENTHPV
 Phypa_SYT1.1  (142) --MHLVKNSTGG-------------------NGNQTGGSVSEYGKPEESR------------EGTPTSLSTRNDGPQAGA
 Phypa_SYT1.2  (130) --MHMVNSSMGG-------------------NGNQTGGNVSEYGKPEDSR------------EGTPTSLNTRNEGPQAGA
 Phypa_SYT1.3  (134) --IHMMSTDRGIGG-----------------NGSQASPGYSDGGRDQSQMG-----------LAMQGDMHGGNGTDLGCS
 Phypa_SYT1.4  (132) --IHMMSTDRGMGG-----------------NGSQASPGYSDGGRDQNQLG-----------MTMQGDMHGGNGTDLGCS
    Chlre_SYT  (152) -------------------------------------AGLPIPGGMAP--------------------------------
    Volca_SYT  (201) -----LGQQPVLSA------------------MGALGGPLAGQGTAA---------------------------------
    Consensus  (241)   H     E                                D G                       K
```

**FIGURE 6 (continued)**

```
                         Met-rich / QG-rich domain (continued)
                         321                                  358
  Allce_SYT2     (175)   TAANTDGSIQKKTANDDLDPSAANPRRSEDAKSS----
  Aqufo_SYT2     (204)   ---AGDGQG-------NSAARNNGSNGDT---------
Vitvi_SYT2.2     (180)   ---FGDGQG-------KFASGHGSGNRDS---------
  Citsi_SYT2     (192)   ---GADGQG-------SSAGGHGGDGEEAK--------
Vitvi_SYT2.1     (195)   ---GGDGQG-------SSAAGHGGDGESPYLKGSEDGK
Glyma_SYT2.1     (193)   ---GGDGQG-------SSAAAHNSGDG-EEAK------
Glyma_SYT2.2     (190)   ---GGDGQG-------SSAAAHNSGDG-EEAK------
  Glyso_SYT2     (190)   ---GGDGQG-------SSAAAHNSGDG-EEAK------
  Medtr_SYT2     (191)   ---GGDGQGT------SAAAAHNSGDASEEGK------
  Poptr_SYT2     (187)   ---GADGLG-------GSAAGHNGADGSEDAK------
  Goshi_SYT2     (193)   ----GNGQG-------STTGGHGGGDGADEAK------
  Eupes_SYT2     (182)   ---GGDGQG--------NSGGDGAEDGK----------
  Frava_SYT2     (179)   ---GGDGQG-------NSAGGHRSGDGEDK--------
  Maldo_SYT2     (187)   ---GGDGQG-------TSAGGRGTGDGEDGK-------
  Prupe_SYT2     (194)   ---GGDGQG-------SSAGGHGNGDGEDGK-------
  Soltu_SYT3     (188)   ----ADGQAS------SVTAQVSEERK-----------
  Arath_SYT2     (185)   ---GADGQG------------------KDDGK------
  Brana_SYT2     (201)   ---GADGQG------------------KDDGK------
  Arath_SYT3     (202)   ---GADGQGG-------SAARHGGGDAKTEGK------
  Lacse_SYT2     (174)   ---GHAGGGP------------------EEAK------
  Tarof_SYT2     (172)   ---GHAGGGP-------P----------EEGK------
  Betvu_SYT2     (179)   --LGDDHGK-----------------------------
  Tarof_SYT3     (203)   ---GGGGGNR-------ES-------------------
  Bradi_SYT3     (190)   --TGADHEAG------GDVAEQS---------------
  Triae_SYT3     (187)   --AGAEEVG-------ADVAEQS---------------
Triae_SYT3.2     (187)   --AGAEEVG-------ADVAEQS---------------
  Orysa_SYT3     (191)   --AGADNDAG------GDIAEKS---------------
  Panvi_SYT3     (193)   --TAADHDVG------TDVAEKS---------------
  Sacof_SYT3     (199)   --TGADSEAG------GDVAEKS---------------
  Sorbi_SYT3     (200)   --TGADSEAG------GDVAEKS---------------
  Horvu_SYT2     (172)   --TGADNDGG------SGLADQS---------------
  Triae_SYT2     (171)   --TGADNDGG------SGWADQS---------------
  Orysa_SYT2     (171)   --TGTENDGG------SDFGDQS---------------
  Sacof_SYT2     (177)   --TGGDNDGG------SD--------------------
```

**FIGURE 6 (continued)**

**Met-rich / QG-rich domain (continued)**

```
    Sorbi_SYT2  (177) --TGGDNDGG------SD--------------------
    Zeama_SYT2  (174) --TGGD-DGG------SD--------------------
    Curlo_SYT2  (193) --SGSEPVSG------DADQSHAKRPEDTKTP------
    Aqufo_SYT1  (183) G-EGRGGNSG--GQ-SADGGESLYLKNSDEGN------
    Arath_SYT1  (186) GGEGRGGSS-------GDGGETLYLKSSDDGN------
    Brana_SYT1  (181) --EGRGGSS-------GDGGETLYLKSSDDGN------
    Lyces_SYT1  (188) SAEGRGGSS------G---GENLYLKASED--------
  Soltu_SYT1.1  (191) SAEGRGGSS------GGDGGENLYLKASED--------
  Soltu_SYT1.2  (198) SDQGRGGSSS--GH-GGDGGENLYLKSSEDGN------
    Helan_SYT1  (184) --EGRGGGSS--GG--GDGGETLYLKSPDKGN------
    Citsi_SYT1  (194) SAEGRGGSSG--SQ---DGGETLYLKGADDGN------
    Goshi_SYT1  (200) SAEGRGGSSG--GQGGGDGGETLYLKAADDGN------
    Poptr_SYT1  (192) SSEGRGGSSG--GQGG-DGGETLYLKSADDGN------
  Vitvi_SYT1.1  (198) ---GRGGNSG--GQGG-DGGETLYLKAAEDGN------
  Glyma_SYT1.1  (185) --EGRGGNSS--GHSGDGGETLNYLKAAGDGN------
    Medtr_SYT1  (185) --DGRGGSSS--GHSGDGGETL-YLKSSGDGN------
  Glyma_SYT1.2  (185) --QEMGGSSE--GRG--EGGENLYLKVADDGN------
  Vitvi_SYT1.2  (196) SAEGRRGYLG--GQ-GADKGETLYFKSAEEKD------
    Aspof_SYT1  (186) --KQETG--S--EGHGTETPMYLKG-SEEEGN------
    Orysa_SYT1  (202) --VDVRG-AN--SGAQSGDGEYLKG-TEEEGS------
    Sacof_SYT1  (198) --VDVRGGTS--SGAQSGDGEYLKAGTEEEGS------
    Sorbi_SYT1  (198) --VDVRGGTS--SGAQSGDGEYLKAGTEEEGS------
    Zeama_SYT1  (200) --VDVRGGTS--SGAQSGDGEYLKVGTEEEGS------
    Triae_SYT1  (200) --ADARG-AN--SGAHSGDGEYLKG-TEEEGS------
    Cryja_SYT1  (214) PGDGRGSSTG--GQ-NTDGSEQSYLKASEE-GN-----
    Picsi_SYT1  (219) AGDGRGSSTG--GQ-NADESEPSYLKASEEEGN-----
    Pinta_SYT1  (215) AGDGRGSSTG--GQ-NADESEPSYLKASEEEGN-----
    Welmi_SYT1  (205) E--GRGGASV--GQ-NTEEGAPSYLKASEDEGS-----
  Phypa_SYT1.1  (189) SPLGQAREG---NGAAGEDSEASYLKSSD---------
  Phypa_SYT1.2  (177) SPLGQAREG---NGAPGEDSEASYLKSSE---------
  Phypa_SYT1.3  (184) SPLGHRGDGGNGHGQGTDDSEASYLKGSDGSSLN----
  Phypa_SYT1.4  (182) SPLGHRGDGGGGHGQGNDDSEASYLKGSDGSSLN----
     Chlre_SYT  (163) ---GQGAPPG----FTLPAPPPLNLAGQ----------
     Volca_SYT  (225) ---GQAGAGG----FQLPAAPPLNLGL-----------
     Consensus  (321)    G  G
```

FIGURE 6 (continued)

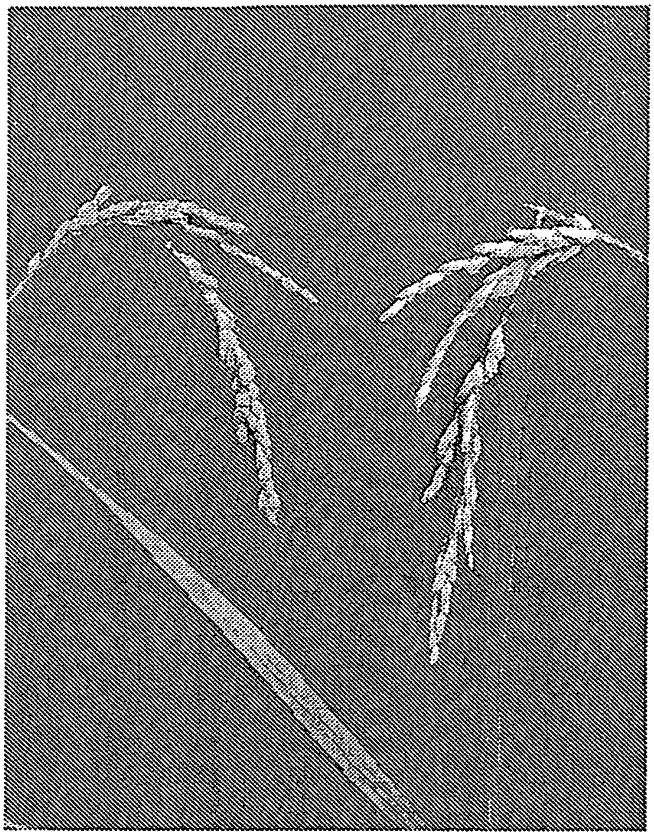

FIGURE 7

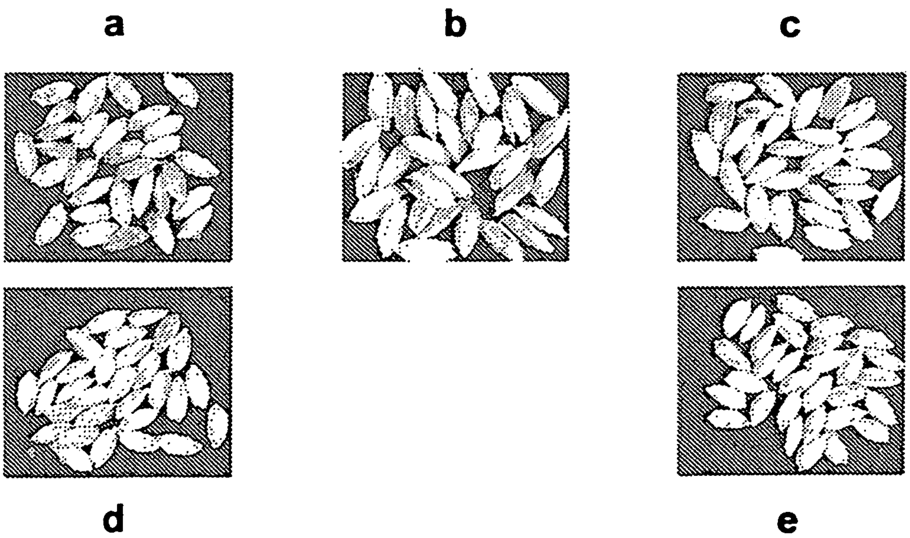


FIGURE 8

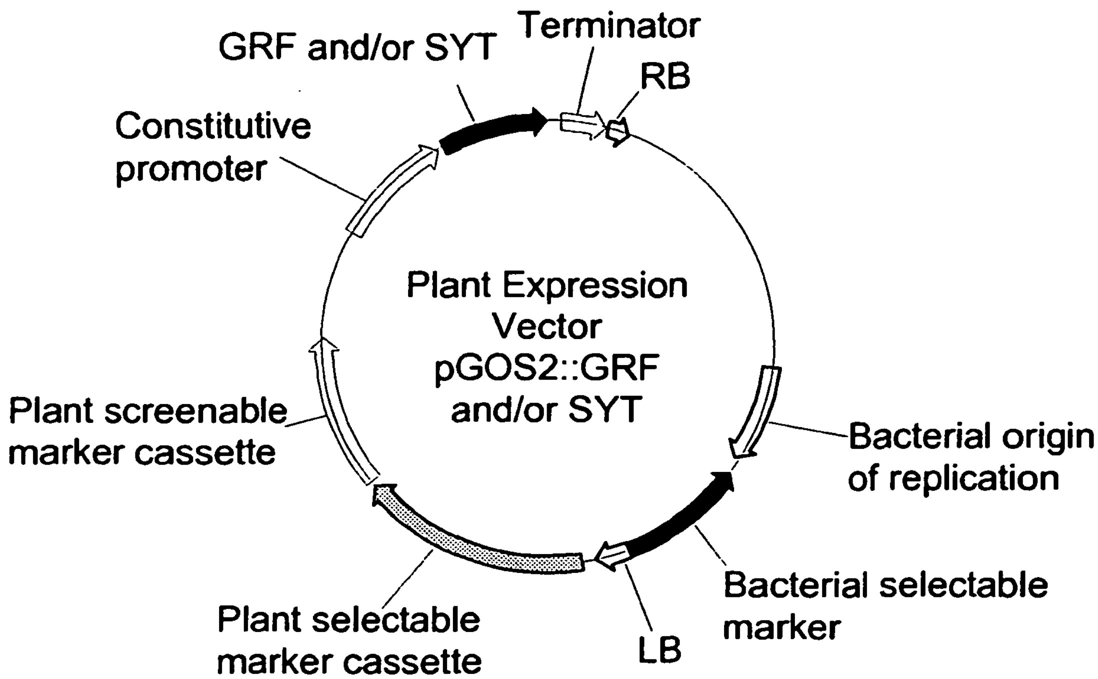
GRF and/or SYT
Terminator
RB
Constitutive promoter
Plant Expression Vector pGOS2::GRF and/or SYT
Plant screenable marker cassette
Bacterial origin of replication
Bacterial selectable marker
Plant selectable marker cassette
LB

**FIGURE 9**

**SEQ ID NO: 1 Arabidopsis thaliana Arath_GRF_AT3G13960.1 nucleic acid sequence**

ATGATGAGTCTAAGTGGAAGTAGCGGGAGAACAATAGGAAGGCCTCCATTTACACCAACACAATGG
GAAGAACTGGAACATCAAGCCCTAATCTACAAGTACATGGTCTCTGGTGTTCCTGTCCCACCTGAG
CTCATCTTCTCCATTAGAAGAAGCTTGGACACTTCCTTGGTCTCTAGACTCCTTCCTCACCAATCC
CTTGGATGGGGGTGTTACCAGATGGGATTTGGGAGAAAACCAGATCCAGAGCCAGGAAGATGCAGA
AGAACAGATGGTAAGAAATGGAGATGCTCAAGAGAAGCTTACCCAGATTCGAAGTACTGTGAAAAA
CACATGCACAGAGGAAGAAACCGTGCCAGAAAATCTCTTGATCAGAATCAGACAACAACAACTCCT
TTAACATCACCATCTCTCTCATTCACCAACAACAACAACCCAAGTCCCACCTTGTCTTCTTCTTCT
TCCTCTAATTCCTCTTCTACTACTTATTCTGCTTCTTCTTCTTCAATGGATGCCTACAGTAACAGT
AATAGGTTTGGGCTTGGTGGAAGTAGTAGTAACACTAGAGGTTATTTCAACAGCCATTCTCTTGAT
TATCCTTATCCTTCTACTTCACCCAAACAACAACAACAAACTCTTCATCATGCTTCCGCTTTGTCA
CTTCATCAAAATACTAATTCTACTTCTCAGTTCAATGTCTTAGCCTCTGCTACTGACCACAAAGAC
TTCAGGTACTTTCAAGGGATTGGGGAGAGAGTTGGAGGAGTTGGGGAGAGAACGTTCTTTCCAGAA
GCATCTAGAAGCTTTCAAGATTCTCCATACCATCATCACCAACAACCGTTAGCAACAGTGATGAAT
GATCCGTACCACCACTGTAGTACTGATCATAATAAGATTGATCATCATCACACATACTCATCCTCA
TCATCATCTCAACATCTTCATCATGATCATGATCATAGACAGCAACAGTGTTTTGTTTTGGGCGCC
GACATGTTCAACAAACCTACAAGAAGTGTCCTTGCAAACTCATCAAGACAAGATCAAAATCAAGAA
GAAGATGAGAAAGATTCATCAGAGTCGTCCAAGAAGTCTCTACATCACTTCTTTGGTGAGGACTGG
GCACAGAACAAGAACAGTTCAGATTCTTGGCTTGACCTTTCTTCCCACTCAAGACTCGACACTGGT
AGCTAA

**SEQ ID NO: 2 Arabidopsis thaliana Arath_GRF_AT3G13960.1 translated polypeptide sequence**

MMSLSGSSGRTIGRPPFTPTQWEELEHQALIYKYMVSGVPVPPELIFSIRRSLDTSLVSRLLPHQS
LGWGCYQMGFGRKPDPEPGRCRRTDGKKWRCSREAYPDSKYCEKHMHRGRNRARKSLDQNQTTTTP
LTSPSLSFTNNNNPSPTLSSSSSSNSSSTTYSASSSSMDAYSNSNRFGLGGSSSNTRGYFNSHSLD
YPYPSTSPKQQQQTLHHASALSLHQNTNSTSQFNVLASATDHKDFRYFQGIGERVGGVGERTFFPE
ASRSFQDSPYHHHQQPLATVMNDPYHHCSTDHNKIDHHHTYSSSSSSQHLHHDHDHRQQQCFVLGA
DMFNKPTRSVLANSSRQDQNQEEDEKDSSESSKKSLHHFFGEDWAQNKNSSDSWLDLSSHSRLDTG
S

**SEQ ID NO: 3 Arabidopsis thaliana Arath_GRF_At2G06200 nucleic acid sequence**

ATGGCTACAAGGATTCCATTCACAGAATCACAATGGGAAGAACTTGAAAACCAAGCTCTTGTGTTC
AAGTACTTAGCTGCAAATATGCCTGTTCCACCTCATCTTCTCTTCCTCATCAAAAGACCCTTTCTC
TTCTCTTCTTCTTCTTCTTCATCTTCTTCTTCAAGCTTCTTCTCTCCCACTCTTTCTCCACACTTT
GGGTGGAATGTGTATGAGATGGGAATGGGAAGAAAGATAGATGCAGAGCCAGGAAGATGTAGAAGA
ACTGATGGCAAGAAATGGAGATGCTCTAAAGAAGCTTACCCTGACTCTAAGTACTGTGAGAGACAT
ATGCATAGAGGCAAGAACCGTTCTTCCTCAAGAAAGCCTCCTCCTACTCAATTCACTCCAAATCTC
TTTCTCGACTCTTCTTCCAGAAGAAGAAGAAGTGGATACATGGATGATTTCTTCTCCATAGAACCT
TCCGGGTCAATCAAAAGCTGCTCTGGCTCAGCAATGGAAGATAATGATGATGGCTCATGTAGAGGC
ATCAACAACGAGGAGAAGCAGCCGGATCGACATTGCTTCATCCTTGGTACTGACTTGAGGACACGT
GAGAGGCCATTGATGTTAGAGGAGAAGCTGAAACAAAGAGATCATGATAATGAAGAAGAGCAAGGA
AGCAAGAGGTTTTATAGGTTTCTTGATGAATGGCCTTCTTCTAAATCTTCTGTTTCTACTTCACTC
TTCATTTGA

**FIGURE 10**

**SEQ ID NO: 4 Arabidopsis thaliana Arath_GRF_At2G06200 translated polypeptide sequence**

MATRIPFTESQWEELENQALVFKYLAANMPVPPHLLFLIKRPFLFSSSSSSSSSSFFSPTLSPHF
GWNVYEMGMGRKIDAEPGRCRRTDGKKWRCSKEAYPDSKYCERHMHRGKNRSSSRKPPPTQFTPNL
FLDSSSRRRRSGYMDDFFSIEPSGSIKSCSGSAMEDNDDGSCRGINNEEKQPDRHCFILGTDLRTR
ERPLMLEEKLKQRDHDNEEEQGSKRFYRFLDEWPSSKSSVSTSLFI

**SEQ ID NO: 5 Arabidopsis thaliana Arath_GRF_At2G22840 nucleic acid sequence**

ATGGATCTTGGAGTTCGTGTTTCTGGTCATGAAACCGTTTCTTCTCCGGGTCAAACTGAACTCGGA
TCTGGTTTCAGTAACAAGCAAGAAAGATCCGGTTTCGATGGTGAAGATTGCTGGAGAAGTTCAAAG
CTCTCACGAACATCAACTGATGGATTCTCTTCTTCCCCTGCCTCTGCTAAAACGCTGTCGTTTCAT
CAAGGCATCCCTTTACTGAGATCTACCACTATTAATGATCCTCGTAAAGGACAAGAACACATGCTT
AGCTTCTCTTCTGCTTCAGGCAAATCAGATGTCTCACCTTATCTTCAGTACTGTAGAAACTCAGGA
TATGGTTTAGGAGGAATGATGAACACAAGCAACATGCATGGAAACTTGTTGACAGGAGTAAAAGGA
CCTTTTTCATTGACTCAGTGGGCAGAGCTAGAGCAACAGGCGTTGATCTATAAGTATATCACAGCC
AATGTCCCTGTTCCATCTAGTTTACTTCTCTCTCTCAAGAAATCTTTTTTCCCTTATGGTTCCTTG
CCTCCTAATTCTTTTGGATGGGGCTCTTTTCATCTGGGCTTTTCCGGTGGTAACATGGATCCCGAG
CCAGGGAGATGTCGCCGGACAGATGGAAAGAAATGGCGGTGCTCGAGGGACGCTGTTCCCGATCAA
AAGTACTGTGAACGACATATTAACAGAGGCCGCCATCGTTCAAGAAAGCCTGTGGAAGGCCAAAAT
GGCCACAATACTAATGCTGCCGCCGCTGCTTCTGCTGCTGCCGCTTCTACCGCTGCTGCTGTGTCC
AAAGCGGCAGCGGGGACTTCAGCTGTTGCGATGCGTGGATCAGATAATAACAATAGCCTTGCCGCT
GCTGTTGGAACACAACATCATACCAATAATCAATCTACAGATTCTTTGGCTAACAGAGTTCAAAAT
TCTCGAGGGGCTTCGGTTTTTCCTGCCACGATGAACTTACAGTCGAAGGAAACTCATCCGAAACAA
AGCAATAATCCCTTTGAATTCGGACTCATCTCTTCTGATTCGTTACTTAATCCGTCGCATAAACAA
GCCTCGTATGCAACCTCTTCCAAAGGCTTTGGATCGTATCTTGACTTCGGCAACCAAGCCAAGCAC
GCGGGGAATCACAACAATGTCGATTCTTGGCCCGAAGAGCTGAAATCGGATTGGACTCAGCTCTCA
ATGTCAATCCCTATGGCTCCATCTTCCCCTGTTCAAGATAAACTTGCACTCTCACCTTTAAGGTTA
TCGCGTGAGTTTGACCCCGCGATCCACATGGGATTAGGCGTCAACACCGAGTTTCTTGACCCCGGG
AAAAAGACGAATAACTGGATACCAATCTCCTGGGGTAATAACAACTCCATGGGAGGTCCACTCGGC
GAGGTACTAAACAGCACGACCAATAGTCCCAAGTTTGGTTCCTCTCCAACAGGCGTCTTGCAAAAG
TCGACATTTGGTTCTCTTTCTAACAGCAGCTCGGCAAGCAGCACCATCATTGGCGATAACAACAAT
AAGAACGGTGATGGAAAAGATCCGCTTGGCCCGACCACGCTGATGAATACTTCTGCTACTGCTCCT
TCTCTGTGA

**SEQ ID NO: 6 Arabidopsis thaliana Arath_GRF_At2G22840 translated polypeptide sequence**

MDLGVRVSGHETVSSPGQTELGSGFSNKQERSGFDGEDCWRSSKLSRTSTDGFSSSPASAKTLSFH
QGIPLLRSTTINDPRKGQEHMLSFSSASGKSDVSPYLQYCRNSGYGLGGMMNTSNMHGNLLTGVKG
PFSLTQWAELEQQALIYKYITANVPVPSSLLLSLKKSFFPYGSLPPNSFGWGSFHLGFSGGNMDPE
PGRCRRTDGKKWRCSRDAVPDQKYCERHINRGRHRSRKPVEGQNGHNTNAAAAASAAAASTAAAVS
KAAAGTSAVAMRGSDNNNSLAAAVGTQHHTNNQSTDSLANRVQNSRGASVFPATMNLQSKETHPKQ
SNNPFEFGLISSDSLLNPSHKQASYATSSKGFGSYLDFGNQAKHAGNHNNVDSWPEELKSDWTQLS
MSIPMAPSSPVQDKLALSPLRLSREFDPAIHMGLGVNTEFLDPGKKTNNWIPISWGNNNSMGGPLG
EVLNSTTNSPKFGSSPTGVLQKSTFGSLSNSSSASSTIIGDNNNKNGDGKDPLGPTTLMNTSATAP
SL

**FIGURE 10 (continued)**

**SEQ ID NO: 7 Arabidopsis thaliana Arath_GRF_At2G36400 nucleic acid sequence**

ATGGATTTGCAACTGAAACAATGGAGAAGCCAGCAGCAGCAACAACATCAGACAGAGTCAGAAGAA
CAACCTTCTGCAGCTAAGATACCAAAACATGTCTTTGACCAGATTCATTCTCACACTGCAACTTCT
ACTGCTCTTCCTCTCTTTACCCCTGAGCCTACTTCTTCTAAACTCTCCTCTTTGTCTCCTGATTCT
TCCTCCAGGTTCCCCAAGATGGGGAGCTTCTTTAGCTGGGCACAGTGGCAAGAACTTGAACTACAA
GCTCTGATCTACAGGTACATGTTGGCTGGTGCTGCTGTTCCTCAGGAGCTCCTTTTACCAATCAAG
AAAAGCCTTCTCCATCTATCTCCTTCCTACTTTCTTCACCATCCTCTTCAACACCTACCTCATTAC
CAACCTGCTTGGTATTTGGGAAGGGCAGCGATGGATCCTGAGCCAGGCAGATGCAGGAGAACGGAT
GGTAAGAAGTGGAGATGTTCAAGAGACGTCTTCGCTGGCCACAAGTATTGCGAGCGCCACATGCAC
CGTGGCCGCAACCGTTCAAGAAAGCCTGTGGAAACTCCAACCACCGTCAATGCAACTGCCACGTCC
ATGGCTTCATCAGTAGCAGCCGCAGCCACCACTACAACAGCAACAACAACATCTACGTTTGCTTTT
GGTGGTGGTGGTGGTAGTGAGGAAGTGGTTGGTCAAGGAGGATCTTTCTTCTTCTCTGGCTCTTCT
AACTCTTCATCTGAACTTCTCCACCTTAGTCAAAGTTGTTCGGAGATGAAGCAAGAAAGCAACAAC
ATGAACAACAAGAGGCCATACGAGTCCCACATCGGATTCAGTAACAACAGATCAGATGGAGGACAC
ATCCTGAGGCCCTTCTTTGACGATTGGCCTCGTTCTTCGCTCCAAGAAGCTGACAATAGTTCAAGC
CCCATGAGCTCAGCCACTTGTCTCTCCATCTCCATGCCCGGGAACTCTTCCTCAGACGTCTCTCTG
AAGCTGTCCACAGGCAACGAAGAGGGAGCCCGGAGCAACAACAATGGGAGAGATCAGCAAAACATG
AGCTGGTGGAGCGGTGGAGGTTCCAACCACCATCATCACAACATGGGCGGACCATTGGCCGAAGCC
CTGAGATCTTCTTCCTCATCTTCCCCAACCAGTGTTCTCCATCAGCTTGGTGTCTCGACACAAGCC
TTTCATTGA

**SEQ ID NO: 8 Arabidopsis thaliana Arath_GRF_At2G36400 translated polypeptide sequence**

MDLQLKQWRSQQQQQHQTESEEQPSAAKIPKHVFDQIHSHTATSTALPLFTPEPTSSKLSSLSPDS
SSRFPKMGSFFSWAQWQELELQALIYRYMLAGAAVPQELLLPIKKSLLHLSPSYFLHHPLQHLPHY
QPAWYLGRAAMDPEPGRCRRTDGKKWRCSRDVFAGHKYCERHMHRGRNRSRKPVETPTTVNATATS
MASSVAAAATTTTATTSTFAFGGGGGSEEVVGQGGSFFFSGSSNSSSELLHLSQSCSEMKQESNN
MNNKRPYESHIGFSNNRSDGGHILRPFFDDWPRSSLQEADNSSSPMSSATCLSISMPGNSSSDVSL
KLSTGNEEGARSNNNGRDQQNMSWWSGGGSNHHHHNMGGPLAEALRSSSSSSPTSVLHQLGVSTQA
FH

**SEQ ID NO: 9 Arabidopsis thaliana Arath_GRF_At2G45480 nucleic acid sequence**

ATGCAGAGCCCTAAAATGGAGCAGGAGGAGGTTGAGGAGGAGAGGATGAGGAATAAGTGGCCGTGG
ATGAAGGCGGCGCAGTTAATGGAGTTTCGGATGCAAGCTTTGGTGTATAGATACATAGAGGCTGGT
CTCCGTGTGCCTCATCATCTCGTGGTGCCTATTTGGAACAGTCTTGCTCTCTCTTCTTCCTCCAAT
TACAACTATCACTCTTCTTCTCTGTTGAGTAACAAGGGAGTAACCCATATCGACACGTTGGAAACT
GAACCAACTAGGTGCAGGAGAACAGATGGGAAGAAATGGCGCTGTAGCAACACGGTCCTTCTATTC
GAGAAGTACTGTGAACGGCACATGCATAGAGGTCGTAAACGTTCAAGAAAGCTTGTGGAATCTTCT
TCTGAGGTTGCTTCATCATCAACCAAATACGACAACACTTATGGTTTGGATAGGTATAACGAGAGT
CAGAGTCATCTTCATGGGACAATCTCGGGTTCTAGTAATGCGCAGGTAGTTACCATTGCTTCACTG
CCTAGTGCCAGATCCTGTGAAAATGTCATTCGTCCGTCTTTAGTGATCTCTGAATTCACAAACAAA
AGTGTGAGTCACGGCAGAAAGAACATGGAGATGAGTTATGATGACTTTATTAATGAAAAAGAGGCG
AGTATGTGTGTTGGAGTTGTTCCTCTTCAAGGTGATGAGAGCAAACCTTCGGTTCAAAAGTTCTTC
CCTGAGGTATCTGATAAATGCTTAGAAGCTGCAAAATTCTCAAGCAACAGGAAGAATGATATAATT
GCAAGAAGCAGAGAATGGAAGAATATGAATGTTAATGGTGGTTTGTTTCATGGTATCCACTTTTCT
CCAGACACTGTTCTTCAAGAACGTGGTTGTTTTCGTTTACAAGGAGTTGAAACAGACAATGAACCA

## FIGURE 10 (continued)

GGAAGGTGCCGAAGAACAGATGGGAAGAAGTGGAGATGCAGCAAAGATGTTTTGTCTGGTCAGAAG
TACTGCGATAAGCACATGCATAGAGGTATGAAGAAGAAGCATCCAGTTGATACTACTAACTCACAT
GAGAATGCCGGGTTTAGCCCGTTAACCGTGGAAACAGCTGTTAGATCGGTTGTGCCTTGCAAAGAT
GGAGATGACCAGAAGCATTCTGTTTCAGTCATGGGAATTACACTGCCCCGAGTTTCTGATGAGAAG
AGCACTAGCAGTTGCAGTACCGACACTACCATTACTGACACAGCTTTAAGGGGTGAAGACGACGAT
GAGGAGTACTTGTCTTTGTTTTCACCAGGTGTTTAG

**SEQ ID NO: 10 Arabidopsis thaliana Arath_GRF_At2G45480 translated polypeptide sequence**

MQSPKMEQEEVEEERMRNKWPWMKAAQLMEFRMQALVYRYIEAGLRVPHHLVVPIWNSLALSSSSN
YNYHSSSLLSNKGVTHIDTLETEPTRCRRTDGKKWRCSNTVLLFEKYCERHMHRGRKRSRKLVESS
SEVASSSTKYDNTYGLDRYNESQSHLHGTISGSSNAQVVTIASLPSARSCENVIRPSLVISEFTNK
SVSHGRKNMEMSYDDFINEKEASMCVGVVPLQGDESKPSVQKFFPEVSDKCLEAAKFSSNRKNDII
ARSREWKNMNVNGGLFHGIHFSPDTVLQERGCFRLQGVETDNEPGRCRRTDGKKWRCSKDVLSGQK
YCDKHMHRGMKKKHPVDTTNSHENAGFSPLTVETAVRSVVPCKDGDDQKHSVSVMGITLPRVSDEK
STSSCSTDTTITDTALRGEDDDEEYLSLFSPGV

**SEQ ID NO: 11 Arabidopsis thaliana Arath_GRF_AT3G52910.1 nucleic acid sequence**

ATGGACTTGCAACTGAAACAATGGAGAAGTCAGCAGCAGAATGAGTCAGAAGAACAAGGCTCTGCT
GCAACTAAGATATCAAACTTTTTCTTTGATCAGATTCAGTCCCAAACTGCTACTTCTGCTGCTGCG
GCTCCTCTTCCTCTCTTTGTCCCTGAACCCACTTCTTCCTCTTCTTTCTCTTGCTTCTCTCCTGAC
TCTTCTAATTCTTCTTCTTCTTCCAGGTTCCTCAAGATGGGAAACTTCTTCAGCTGGGCACAGTGG
CAAGAACTTGAGCTACAAGCACTGATCTATAGATACATGTTGGCTGGTGCTTCTGTTCCTCAAGAG
CTTCTCTTACCTATTAAGAAAAGTCTCCTCCATCAATCTCCTATGCATTTCCTTCACCATCCTCTT
CAACATAGTTTTCCTCATCACCAACCTTCTTGGTATTGGGGAAGAGGAGCAATGGATCCTGAGCCA
GGGAGGTGTAAGAGAACTGACGGCAAGAAATGGAGATGTTCAAGGGATGTTGTAGCGGGCCACAAG
TATTGTGACCGCCACATTCACCGTGGAAGAAACCGTTCAAGAAAGCCTGTGGAAACCGCCACAACC
ACCATCACAACGACAGCCACAACAACCGCATCTTCTTTTGTCTTAGGTGAGGAGCTTGGTCATGGA
CCAAACAACAACCACTTCTTCTCCTCTGGTTCATCTCAACCTCTCCACCTTAGTCATCAACAAAGT
TGTTCTTCAGAGATGAAACAAGAAAGCAACAACAACAAGAGGCCATATGAAGCTAACAGTGGATTC
AGCAATGGAAGATCAGACGATGGTCACATCTTGAGGCATTTCTTTGACGATTGGCCACGATCATCA
GACTCTACCTCCAGTCCAATGAGCTCATCCACTTGTCATCTTTCAATCTCCATGCCCGGTAACAAC
ACGTCCTCAGATGTTTCTCTAAAACTTTCCACAGGCAATGAAGAAGAAGAAGAGAACATGAGAAAT
AACAACAATGAGAGGGAGCAAATGAATTGGTGGAGCAATGGAGGGAATCACCACAACAATATGGGA
GGACCATTAGCTGAGGCTTTGAGGTCAGCTTCTTCGACGTCAAGTGTTCTTCATCAGATGGGAATC
TCTACTCAAGAAATGAAGTATGTGAAGCCATTGAGCTTATTGGGTAATGCGCTGAAGACCAAAGTG
TCAGTCCCTGGTCGGTTTCTGGGTTTAGATGTTGGTGATAAGTATGTTGGATTAGCTATCTCAGAT
CCTTCAAATATGGTTGCTTCTCCATTGAGTGTTTTGCTCAGAAAGAAATCAAACATTGACCTGATG
GCTACAGATTTCCAGAACCTGGTCAAAGCATTTTCTGTGTCGGGATTAGTCGTTGGTTATCCATTT
GGCAAACTGAACAATGTAGAGGATGTTGTCACTGTGAATCTTTTCATTGAGGAACTTCGTAAGACC
GAAAAACTCAAGGATGTGAAATACACATATTGGGACGAGCGATTATCATCAAAGACCGTTGAACTG
ATGTTGAAGCCCTTGAATTTGCATCCTGTTCAAGAGAAGACAATGTTGGACAAGTTAGCCGCAGTA
GTTATACTTCAGGAGTATTTAGATTACGCGAACAGGTATGTAAACACTGAGCCAGCAGAGTAA

**FIGURE 10 (continued)**

**SEQ ID NO: 12 Arabidopsis thaliana Arath_GRF_AT3G52910 translated polypeptide sequence**

MDLQLKQWRSQQQNESEEQGSAATKISNFFFDQIQSQTATSAAAAPLPLFVPEPTSSSSFSCFSPD
SSNSSSSSRFLKMGNFFSWAQWQELELQALIYRYMLAGASVPQELLLPIKKSLLHQSPMHFLHHPL
QHSFPHHQPSWYWGRGAMDPEPGRCKRTDGKKWRCSRDVVAGHKYCDRHIHRGRNRSRKPVETATT
TITTTATTTASSFVLGEELGHGPNNNHFFSSGSSQPLHLSHQQSCSSEMKQESNNNKRPYEANSGF
SNGRSDDGHILRHFFDDWPRSSDSTSSPMSSSTCHLSISMPGNNTSSDVSLKLSTGNEEEEENMRN
NNNEREQMNWWSNGGNHHNNMGGPLAEALRSASSTSSVLHQMGISTQEMKYVKPLSLLGNALKTKV
SVPGRFLGLDVGDKYVGLAISDPSNMVASPLSVLLRKKSNIDLMATDFQNLVKAFSVSGLVVGYPF
GKLNNVEDVVTVNLFIEELRKTEKLKDVKYTYWDERLSSKTVELMLKPLNLHPVQEKTMLDKLAAV
VILQEYLDYANRYVNTEPAE

**SEQ ID NO: 13 Arabidopsis thaliana Arath_GRF_AT4G24150.1 nucleic acid sequence**

ATGAGGATGCTTCTTGGGATTCCTTACGTAGACAAGTCGGTTCTTTCCAACTCTGTTCTTGAGAGA
GGCAAGCAGGATAAAAGCAAACTATTGTTAGTCGACAAATGCCATTATGAGCTTGATGTTGAAGAA
CGCAAGGAAGATTTTGTTGGTGGGTTTGGATTTGGTGTTGTAGAAAATTCGCATAAAGACGTTATG
GTGCTACCTCATCATCACTATTATCCATCATATTCATCACCTTCCTCTTCTTCTTTGTGTTACTGT
TCTGCTGGTGTTAGCGATCCCATGTTCTCTGTTTCTAGCAATCAGGCTTACACTTCTTCTCACAGT
GGTATGTTCACACCCGCCGGTTCTGGTTCTGCTGCTGTGACTGTAGCAGATCCTTTTTTCTCCTTG
AGCTCTTCAGGGGAAATGAGAAGAAGTATGAACGAAGATGCTGGTGCAGCTTTCAGCGAAGCTCAA
TGGCATGAGCTTGAGAGGCAGAGGAATATATACAAGTACATGATGGCTTCTGTTCCTGTTCCTCCA
GAGCTTCTCACACCCCTTTCCCAAGAACCACCAATCAAACACTAACCCGGATGTGGATACATATAGG
AGTGGAATGTTTAGTATTTATGCTGATTACAAGAATCTGCCGTTGTCTATGTGGATGACAGTAACT
GTGGCAGTGGCGACAGGAGGCTCATTGCAGCTGGGGATTGCTTCAAGCGCAAGCAATAACACGGCT
GATCTGGAGCCATGGAGGTGCAAGAGAACAGATGGGAAGAAATGGAGGTGCTCTAGAAACGTGATT
CCTGATCAGAAATACTGTGAGAGACACACACACAAGAGCCGTCCTCGTTCAAGAAAGCATGTGGAA
TCATCTCACCAATCATCTCACCACAATGACATTCGTACGGCTAAGAATGATACTAGCCAGCTTGTG
AGAACTTATCCTCAGTTTTACGGACAACCTATAAGCCAGATCCCTGTGCTTTCTACTCTTCCGTCT
GCCTCCTCTCCATATGATCACCACAGAGGACTGAGGTGGTTTACGAAAGAAGATGATGCCATTGGA
ACCTTAAACCCGGAGACTCAAGAAGCTGTCCAGCTGAAAGTTGGATCAAGCAGAGAGCTCAAACGG
GGATTCGATTATGATCTGAATTTCAGGCAGAAAGAGCCAATAGTAGACCAGAGCTTTGGAGCATTG
CAGGGTCTATTAAGTCTAAACCAGACACCACAACATAACCAAGAAACAAGACAGTTTGTTGTAGAA
GGAAAGCAAGATGAAGCGATGGGAAGCTCTCTGACACTCTCAATGGCTGGAGGAGGCATGGAGGAA
ACAGAGGGAACAAACCAGCATCAGTGGGTTAGCCATGAAGGTCCATCATGGCTCTATTCAACAACA
CCAGGTGGACCATTGGCTGAAGCACTGTGTCTCGGTGTCTCCAACAACCCAAGTTCTAGTACTACT
ACTAGTAGCTGCAGCAGAAGCTCAAGCTAA

**SEQ ID NO: 14 Arabidopsis thaliana Arath_GRF_ AT4G24150.1 translated polypeptide sequence**

MRMLLGIPYVDKSVLSNSVLERGKQDKSKLLLVDKCHYELDVEERKEDFVGGFGFGVVENSHKDVM
VLPHHHYYPSYSSPSSSSLCYCSAGVSDPMFSVSSNQAYTSSHSGMFTPAGSGSAAVTVADPFFSL
SSSGEMRRSMNEDAGAAFSEAQWHELERQRNIYKYMMASVPVPPELLTPFPKNHQSNTNPDVDTYR
SGMFSIYADYKNLPLSMWMTVTVAVATGGSLQLGIASSASNNTADLEPWRCKRTDGKKWRCSRNVI
PDQKYCERHTHKSRPRSRKHVESSHQSSHHNDIRTAKNDTSQLVRTYPQFYGQPISQIPVLSTLPS
ASSPYDHHRGLRWFTKEDDAIGTLNPETQEAVQLKVGSSRELKRGFDYDLNFRQKEPIVDQSFGAL
QGLLSLNQTPQHNQETRQFVVEGKQDEAMGSSLTLSMAGGGMEETEGTNQHQWVSHEGPSWLYSTT
PGGPLAEALCLGVSNNPSSSTTTSSCSRSSS

## FIGURE 10 (continued)

**SEQ ID NO: 15 Arabidopsis thaliana Arath_GRF_AT4G37740.1 nucleic acid sequence**

ATGGATATTGGTGTTCATGTTCTTGGGTCGGTTACTAGTAATGAAAATGAGTCACTTGGTCTAAAA
GAGCTTATAGGAACTAAACAAGATAGATCCGGATTCATCGGTGAGGATTGCTTGCAACGAAGCTTG
AAGCTAGCAAGAACGACAACTAGAGCGGAAGAAGAAGAAAACTTGTCTTCTTCTGTTGCAGCTGCT
TATTGCAAAACGATGTCGTTTCACCAAGGCATTCCTCTCATGAGATCTGCTTCTCCTCTTTCCTCT
GATTCTCGCCGTCAAGAACAAATGCTTAGCTTCTCAGATAAACCAGACGCTCTTGATTTCAGTAAA
TATGTCGGTTTGGATAATAGCAGTAATAACAAGAACTCTCTCTCGCCGTTTCTTCACCAGATTCCT
CCACCTTCTTACTTTAGAAGCTCAGGAGGATATGGTTCTGGTGGAATGATGATGAACATGAGCATG
CAAGGGAACTTCACAGGTGTTAAAGGACCTTTTACATTGACTCAATGGGCTGAGTTAGAGCAACAG
GCGTTGATCTATAAGTACATCACAGCCAATGTCCCTGTTCCTTCTAGTTTGCTCATCTCTATCAAG
AAGTCTTTTTATCCTTACGGATCTTTGCCTCCTAGTTCCTTCGGATGGGGAACTTTCCATCTCGGT
TTCGCAGGCGGTAACATGGACCCTGAGCCAGGGAGATGCCGCAGAACAGATGGGAAGAAATGGCGG
TGCTCAAGAGACGCCGTTCCTGATCAGAAATACTGTGAAAGACACATCAACAGAGGCCGTCATCGT
TCAAGAAAGCCTGTGGAAGTCCAATCTGGCCAAAACCAAACCGCCGCTGCTGCATCCAAAGCGGTT
ACTACACCACAACAGCCTGTTGTCGCTGGTAATACTAACAGAAGCAATGCCCGTGCATCAAGCAAC
CGCAGCCTCGCCATTGGAAGTCAATATATCAATCCTTCTACAGAATCTTTACCTAACAACAGAGGA
GTTTCGATATATCCTTCCACCGTCAACTTACAACCCAAGGAATCTCCGGTTATTCATCAGAAACAC
AGAAACAACAACAACCCTTTTGAGTTTGGACACATATCCTCTGATTCGTTACTCAACCCGAATACC
GCAAAGACCTATGGATCATCGTTCTTGGATTTCAGCAGCAACCAAGAGAAGCATTCAGGGAATCAC
AATCACAATTCTTGGCCTGAAGAGCTGACATCAGATTGGACACAGCTCTCAATGTCAATTCCAATA
GCATCATCATCCCCTTCCTCCACACACAACAACAACAATGCTCAAGAAAAAACAACACTCTCGCCT
CTCAGGCTATCCCGCGAGCTTGACCTATCGATCCAAACCGATGAAACAACAATCGAGCCTACTGTG
AAAAAGGTGAATACTTGGATACCAATCTCATGGGGAAACTCCTTAGGAGGTCCTCTAGGTGAAGTA
CTAAACAGTACAACGAATAGTCCAACATTTGGATCTTCTCCTACAGGGGTTTTGCAAAAGTCCACA
TTTTGTTCACTCTCTAACAACAGCTCCGTGAGCAGCCCCATTGCAGAGAACAACAGACACAATGGC
GATTACTTTCATTACACAACCTGA

**SEQ ID NO: 16 Arabidopsis thaliana Arath_GRF_AT4G37740.1 translated polypeptide sequence**

MDIGVHVLGSVTSNENESLGLKELIGTKQDRSGFIGEDCLQRSLKLARTTTRAEEEENLSSSVAAA
YCKTMSFHQGIPLMRSASPLSSDSRRQEQMLSFSDKPDALDFSKYVGLDNSSNNKNSLSPFLHQIP
PPSYFRSSGGYGSGGMMMNMSMQGNFTGVKGPFTLTQWAELEQQALIYKYITANVPVPSSLLISIK
KSFYPYGSLPPSSFGWGTFHLGFAGGNMDPEPGRCRRTDGKKWRCSRDAVPDQKYCERHINRGRHR
SRKPVEVQSGQNQTAAAASKAVTTPQQPVVAGNTNRSNARASSNRSLAIGSQYINPSTESLPNNRG
VSIYPSTVNLQPKESPVIHQKHRNNNNPFEFGHISSDSLLNPNTAKTYGSSFLDFSSNQEKHSGNH
NHNSWPEELTSDWTQLSMSIPIASSSPSSTHNNNNAQEKTTLSPLRLSRELDLSIQTDETTIEPTV
KKVNTWIPISWGNSLGGPLGEVLNSTTNSPTFGSSPTGVLQKSTFCSLSNNSSVSSPIAENNRHNG
DYFHYTT

**SEQ ID NO: 17 Arabidopsis thaliana Arath_GRF_AT5G53660.1 nucleic acid sequence**

ATGGACTTTCTCAAAGTTTCAGACAAGACAACAATTCCATATAGAAGTGATTCTTTGTTTAGTTTG
AATCAGCAACAATACAAAGAGTCTTCTTTTGGATTCAGAGACATGGAGATTCATCCGCATCCTACT
CCATATGCAGGAAATGGACTTTTGGGTTGTTATTACTATTACCCTTTCACAAACGCACAATTGAAG
GAGCTTGAGAGACAAGCAATGATCTACAAGTACATGATCGCATCTATTCCTGTTCCTTTCGATCTA
CTTGTTTCTTCACCATCCTCTGCCTCTCCTTGTAACAATAAAAACATCGCCGGAGATTTAGAGCCG
GGAAGATGCCGGAGAACAGACGGAAAGAAATGGAGATGCGCGAAAGAAGTCGTCTCTAATCACAAA

**FIGURE 10 (continued)**

TACTGTGAGAAACACTTACACAGAGGTCGTCCTCGTTCAAGAAAGCATGTGGAACCTCCTTATTCT
CGCCCTAACAACAATGGTGGTTCTGTGAAAAACAGAGATCTCAAAAAGCTTCCTCAAAAGTTATCT
AGTAGTTCCATCAAAGACAAAACACTTGAGCCAATGGAGGTTTCATCATCAATCTCAAACTATAGA
GACTCCAGAGGAAGTGAGAAATTTACTGTATTGGCAACAACAGAGCAAGAGAACAAGTATCTGAAT
TTCATAGATGTATGGTCCGATGGAGTAAGATCATCTGAAAAACAGAGTACAACTTCAACACCTGTT
TCTTCTTCCAATGGCAATCTCTCTCTTTACTCGCTTGATCTCTCAATGGGAGGAAACAACTTAATG
GGCCAAGACGAAATGGGCCTGATACAAATGGGCTTAGGTGTAATCGGGTCGGGTAGTGAGGATCAT
CACGGGTATGGTCCTTATGGTGTGACTTCTTCACTAGAGGAGATGTCAAGCTGGCTTGCTCCGATG
TCTACCACACCTGGTGGACCATTAGCGGAGATACTGAGGCCGAGTACGAATTTGGCGATCTCTGGT
GATATCGAATCGTATAGCTTGATGGAGACTCCCACTCCAAGCTCGTCCCCGTCTAGAGTGATGAAG
AAGATGACTAGTTCAGTGTCCGACGAAAGCAGCCAGGTTTAG

**SEQ ID NO: 18 Arabidopsis thaliana Arath_GRF_AT5G53660.1 translated polypeptide sequence**

MDFLKVSDKTTIPYRSDSLFSLNQQQYKESSFGFRDMEIHPHPTPYAGNGLLGCYYYYPFTNAQLK
ELERQAMIYKYMIASIPVPFDLLVSSPSSASPCNNKNIAGDLEPGRCRRTDGKKWRCAKEVVSNHK
YCEKHLHRGRPRSRKHVEPPYSRPNNNGGSVKNRDLKKLPQKLSSSSIKDKTLEPMEVSSSISNYR
DSRGSEKFTVLATTEQENKYLNFIDVWSDGVRSSEKQSTTSTPVSSSNGNLSLYSLDLSMGGNNLM
GQDEMGLIQMGLGVIGSGSEDHHGYGPYGVTSSLEEMSSWLAPMSTTPGGPLAEILRPSTNLAISG
DIESYSLMETPTPSSSPSRVMKKMTSSVSDESSQV

**SEQ ID NO: 19 Aquilegia formosa x Aquilegia pubescens Aqufo_GRF nucleic acid sequence DT756681, DR946716**

ACTTAAAAGACCAGTCTTAGCTTTCTTCATTAATTCCTACTACTGTTCTCAGTGTTGCTCTTTGAG
TTTATAGATATTTTTCTTACAATGATGATGAGTGCTAGAAACAGAAATCCTTTCACTGTAACTCAA
TGGCAAGAACTTGAACATCAAGCTCTCATTTATAAGTATATGGCTTCAGGAATGCCTATACCACCT
GATCTCATCTTCCCTATTAAGAGAAGTCTTGATTCTTCAAGATTCTTTCCTCATCAACCAATGGAT
TGGGGTTGTTTTCAGATGGGTTATGGCAGGAAAGTTGATCCAGAACCTGGAAGGTGCAGAAGAACA
GATGGAAAGAAGTGGAGATGCTCAAAGGAAGCATACCCAGACTCAAAGTACTGTGAGAGACACATG
CACAGAGGCAGAAACCGTTCAAGAAAGCCTGTGGAAGTTAATACTACATCAAATTCCTCATTACCA
CTTTCATCTTTTACCTCTAGAACTCCTTCTAGTACCATTACTTCAAATACCAACCCTTCTTCTTAT
TCCCTTTCTTCATCTCTAACATCTGACAAATCTCAGCAAGAACATCATCACCCTTATCATAACACC
CCTCTTCATTCCTTTCTCAATCCTAGTAGAACTTCTTGTTCTTCTCCTAGAACTCATAATATTGAT
TTCTCACCTCATAGCAATAACAATGCCAATTTGGTATTAGACTCTGGGTCTTACTCTAACTCTTAT
GAAGATCACAGAAACAGGTATGTTCATGGTCTAAAAGAAGAGGTAGATGAAAGAGCTTTCTTTTCA
GAAGCATCAGGAACATTAAGAAGTGTACCAGAATCAACTTTGAAAGATCCATGGCGTTTAACACCA
TTAAGAATGAGTTCTTCAACTCATAACCAACCAAAAGATGGAAATTTTTCTGATTTACAAAGAGGG
TATTCTCAGTTTCAACTCCAACATAAACAACAACAACAGCAACAAGAAGAAGAACAGCATTGTTTT
ATTTTAGGTACTGATTTCAAATCTGACAGGTTTATGAAAACTAGTACTACTACTACTGAGAAAGAA
GAATCACAACAACCACTTCGCCATTTCTTTGATGAATGGCCACCTAAGAGTAAAGATTCTTGGTTG
GGTTTAGAAGAAGATAGATCAGATCAAGGTTCACATTCAACAACTCAACTTTCAATATCTATTCCT
ATGTCTTCTCATGAGTTCTCAGTTTCAAATTCCAGAACCTAACAATAAGATGATGGTTGATTTACT
TAAAGTGGGATTATTATGGAAAGATTAATGACAACAAGGAGTTGATTCAAGGTTGGGTTCAGTGTC
TTTGTACTTGTATTGTCTTTATTTAATTGATGATGAGAAGTTTAGGTAGAGAGTGCTATGTGTTAT
TTTTTTTTTTGTTATGTGTGTGGAGTGATTGAAAAGTGTGTCTTTAAACAGTAAGATTCCTGTCTT
GTGTTTTCTTGATAGCTGTTAGAACTTTGTTTGAATGACTGATGAACAAATATTTGGGATTTGGGG
ATTTGTTTGTATCAATATTAGGTGTTTTTTCTGTCTTTTTGGCTTCTTCCATGATTGCCAAAGACC
ATTTGTTCAACCTAAAAATGATAATGAAGGGGGGGCCAATTTGATATCATGAGCTTGGTTGTCAGT
TAGGAAAG

**FIGURE 10 (continued)**

**SEQ ID NO: 20 Aquilegia formosa x Aquilegia pubescens Aqufo_GRF translated polypeptide sequence**

MMMSARNRNPFTVTQWQELEHQALIYKYMASGMPIPPDLIFPIKRSLDSSRFFPHQPMDWGCFQMG
YGRKVDPEPGRCRRTDGKKWRCSKEAYPDSKYCERHMHRGRNRSRKPVEVNTTSNSSLPLSSFTSR
TPSSTITSNTNPSSYSLSSSLTSDKSQQEHHHPYHNTPLHSFLNPSRTSCSSPRTHNIDFSPHSNN
NANLVLDSGSYSNSYEDHRNRYVHGLKEEVDERAFFSEASGTLRSVPESTLKDPWRLTPLRMSSST
HNQPKDGNFSDLQRGYSQFQLQHKQQQQQEEEQHCFILGTDFKSDRFMKTSTTTEKEESQQPLR
HFFDEWPPKSKDSWLGLEEDRSDQGSHSTTQLSISIPMSSHEFSVSN

**SEQ ID NO: 21 Brassica napus Brana_GRF nucleic acid sequence contig of CN730217.1, ES922527**

GAAGAAAGATGATGGGTCTAAGTGGAAATGGTGGGAGAACAATAGAGAGGCCTCCATTTACACCAA
CACAATGGCAAGAACTGGAGAATCAAGCCCTAATTTACAAGTACATGGTCTCAGGAGTTCCTGTCC
CACCTGAGCTCATCTTCTCCATTAGAAGAAGCTTGGACTCTTCCTTGGTCTCTAGACTCCTCCCTC
ACCAATCCATTGGGTGGGGATGCTATCAGATGGGGTTTGGTAGAAAACCAGATCCAGAACCAGGAA
GGTGCAGAAGAACAGATGGTAAGAAATGGAGATGCTCAAGAGAAGCATACCCTGATTCAAAGTACT
GTGAAAAACACATGCACAGAGGAAGGAACCGTGCCAGAAAATCTATTGATCAGAATCAGACAACTG
CTCCTTTAACATCACCATCTCTCTCTTTCCCCAACAACAACAACCCAAGCCCTACCTTGTCTTCTT
CCTCCTCTACTTATTCAGCTGCTTCTTCATCTCCTTCCATTGATGCTTACAGTAATATCAATAGGC
TTGGTGTTGGTAGTAGTAACAGTAGAGGTTACTTCAACAACCATTCCCTTGACTATCCTTATCCTT
TGTCCTCACCTAAACAGCAACAACAACAGCAACAAACTCTTAGTCATGTTTCTGCTTTGTCACTTC
ATCAAAACACATCTACACCTCAGCTCAATGTCTTTGCCTCTGCAACTGACCACAAAGACTTCAGAT
ATTTTCAAGGGATTGGGGAGAGAGTTGGAGTTGGGGAAAGAACTTTTTTTCCAGAAGCTTCTAGAA
GCTTTCAAGATTCTCCATACCATCACCAACAACCGTTAGCAACGGTAGTGGATAATCCGTACGACT
GTACTACTGATCATAAGTTTGATCATCATCATACATACTCATCATCATCTCAACATCATCATCATG
ACCAAGATCATCGACAACAACAACAATGTTTTGTTTTGGGCGCCGACATGTTCAACAAACCCACAA
GAACTATCTTGGAAAACACATCGAGACAAGATTATCTTAATCAAGAAGAGGAAGAGAAAGATTCAT
CGGACACGAAGAAGTCCCTTCATCATTTCTTTGGTGAAGAGTGGACACAGAACAAGAACAGTTCAG
ATTCTTGGCTTGACCTTTCTTCCCAGTCAAGACTCGACACTGGTAGCTGATTGATGAGGCCAGATA
GCATCAGTGATGGGTCTGCACCAACACACACACAAACACGTTTGAAGGGTCACATTTCACATCTAT
TTCCGTGGAACATTGAGACAGACAAGACACTG

**SEQ ID NO: 22 Brassica napus Brana_GRF translated polypeptide sequence**

MMGLSGNGGRTIERPPFTPTQWQELENQALIYKYMVSGVPVPPELIFSIRRSLDSSLVSRLLPHQS
IGWGCYQMGFGRKPDPEPGRCRRTDGKKWRCSREAYPDSKYCEKHMHRGRNRARKSIDQNQTTAPL
TSPSLSFPNNNNPSPTLSSSSSTYSAASSSPSIDAYSNINRLGVGSSNSRGYFNNHSLDYPYPLSS
PKQQQQQQTLSHVSALSLHQNTSTPQLNVFASATDHKDFRYFQGIGERVGVGERTFFPEASRSFQ
DSPYHHQQPLATVVDNPYDCTTDHKFDHHHTYSSSSQHHHHDQDHRQQQQCFVLGADMFNKPTRTI
LENTSRQDYLNQEEEEKDSSDTKKSLHHFFGEEWTQNKNSSDSWLDLSSQSRLDTGS

**SEQ ID NO: 23 Hordeum vulgare Horvu_GRF nucleic acid sequence AK250947**

GGGCAGCCGCAGCCGCAGCCGCAGCAGAGGAGAGAGAGAGGGAGGGAGAAGCATATATGGCGATGC
CCTTTGCCTCCCTGTCGCCGGCAGCCGACCACCACCGCTCCTCCCCCATCTTCCCCTTCTGCCGCT
CCTCCCCTCTCTACTCGGTAGGGGAGGAGGCGGCGCATCAGCATCCTCATCCTCAGCAGCAGCAGC
AGCAGCACGCGATGAGCGGCGCGCGGTGGGCGGCGAGGCCGGCGCCCTTCACGGCGGCGCAGTACG
AGGAGCTGGAGCAGCAGGCGCTCATCTACAAGTACCTCGTCGCCGGCGTCCCCGTCCCGCAGGACC

**FIGURE 10 (continued)**

TCCTCCTCCCCATCCGCCGCGGCTTCGAGACCCTCGCCTCGCGCTTCTACCACCACCACGCCCTTG
GGTACGGGTCCTACTTCGGGAAGAAGCTGGATCCGGAGCCGGGGCGGTGCCGGCGGACGGACGGCA
AGAAGTGGCGGTGCTCCAAGGAGGCCGCTCAGGACTCCAAGTACTGCGAGCGCCACATGCACCGCG
GCCGCAACCGTTCAAGAAAGCCTGTGGAAACGCAGCTCGTCGCCAGCTCCCACTCCCAGTCCCAGC
AGCACGCCACCGCCGCCTTCCACAACCACTCGCCGTATCCGGCGATCGCCACTGGCGGTGGCTCCT
TCGCCCTGGGGTCTGCTCAGCTGCACATGGACACTGCTGCGCCTTACGCGACGACCGCCGGTGCTG
CCGGAAACAAAGATTTCAGGTATTCTGCCTATGGAGTGAGGACGTCGGCGATCGAGGAGCACAACC
AGTTCATCACCGCGGCCATGGACACCGCCATGGACAACTACTCGTGGCGCCTGATGCCGTCCCAGG
CCTCGGCATTCTCGCTCTCCAGCTACCCCATGCTGGGCACGCTGAGCGACCTGGACCAGAGCGCGA
TCTGCTCGCTGGCCAAGACTGAGAGGGAGCCACTGTCCTTCTTCGGCGGCGGCGGCGACTTCGACG
ACGACTCGGCTGCGGTGAAGCAGGAGAACCAGACGCTGCGGCCCTTCTTCGACGAGTGGCCCAAGG
ACAGGGACTCGTGGCCGGAGCTGCAAGACCACGACGCCAACAACAACAGCAACGCCTTCTCAGCCA
CCAAGCTGTCCATCTCCATGCCGGTCACCAGCTCCGACTTCTCTGGCACCACCGCCGGCTCCCGCT
CGCCCAACGGTATATACTCCCGGTGAACGGCGTCGGCCGGCCTGATCTCTGCTGATTTGCCGTGGT
CACGACGGGCGTCCTCAAATCATCACAGATGAGCGAACCGGCCGACCCGATCGAATGTGTCTGTGA
GCCGACTGCAGCTTGCTTGCTCATTTTGTATGGATCGTCGTGCAGCAGGAACGAAACACTACTCCT
TTAATTTCCTTTCTTTAATTTCACAACGTTTTTTCTGGGTTTTGCCGTGTATCGGCCGGAACTGTA
CTACCAAGTTTTCTATAGCCTCGATGGTCATGCACGACATCGTTGACTGTTTCCCGCGCACTTACT
GTTGAAATAATCTTCCATTTTTGGCAAAAAAAAAAAAAAAA

**SEQ ID NO: 24 Hordeum vulgare Horvu_GRF translated polypeptide sequence**

MAMPFASLSPAADHHRSSPIFPFCRSSPLYSVGEEAAHQHPHPQQQQQQHAMSGARWAARPAPFTA
AQYEELEQQALIYKYLVAGVPVPQDLLLPIRRGFETLASRFYHHHALGYGSYFGKKLDPEPGRCRR
TDGKKWRCSKEAAQDSKYCERHMHRGRNRSRKPVETQLVASSHSQSQQHATAAFHNHSPYPAIATG
GGSFALGSAQLHMDTAAPYATTAGAAGNKDFRYSAYGVRTSAIEEHNQFITAAMDTAMDNYSWRLM
PSQASAFSLSSYPMLGTLSDLDQSAICSLAKTEREPLSFFGGGGDFDDDSAAVKQENQTLRPFFDE
WPKDRDSWPELQDHDANNNSNAFSATKLSISMPVTSSDFSGTTAGSRSPNGIYSR

**SEQ ID NO: 25 Lycopersicon esculentum Lyces_GRF nucleic acid sequence BT013977**

GATGATAAGAAACACACAAATGACTTAACTTTGCAGGTTTCACCGCACTCGACACTGCAAAAAAGA
TACATATAAAAAAAAAGGTCCACTCAACTCTCTGCAAAAATAAAAAAAATTAAAAACTTTTGTCCA
AGACTTAACTTTCTCTTCAGAAATAAATTTGCCTTCACATTAATATTTTGTTGTTAGTAACAAAAA
TCATTCTCAATCGAAACATGGACTTCAATATGAAGCAATGGAGTAATCAACATGAGTCAGAAAATC
AAGAATCACCAACAAAGTTACCAAGACTTCTTCTTGACTTCCACTCTGTTTCTTCTGATTCTGCTT
CTGCTGCTGCTCTACCATTGTTTGTATCTGAACCAACAACATCAACAACAACTTGTACCAAATTAA
TGTCAGATTCAGCAACCACTGTCACCACCAAATTTCCAAGGATTGGAAGTGGTGGTGGTTACTTCA
GCTTGGCTCAATGGCAAGAACTTGAACTACACAGTTTGATTTTTAGGCATTTTGTAGCTGGTGCCC
CTGTTCCTTCTGAACTACTTCATCTTGTTAAGAAAAGTATTATTGCTTCTCCTCCTCCTCCTCCTT
CATATTACTTTGCTCATCCATATCAACAGTATCCTCATTATCAACAAGCTTTGATGCAGTCAGGGT
ACTGGGGTAGAGCCGCCATGGATCCAGAACCAGGAAGGTGTAGGAGGACTGATGGCAAGAAATGGA
GGTGCTCAAGGGATGTAGTGGCTGGCCAGAAATACTGCGAGCGCCACGTTCATCGTGGCCGCAGCC
GTTCAAGAAAGCCTGTGGAAATTCCCACACCTGCCAACAATGGCAGTAAAAACAACAACACTGTTT
CTCATCATCAAGCCTTTGGAAAAATGACTGGACATGCTCATGCTGGTGGTGGTGCTCCTCAGTTTT
CTCTTTCGGGACATTCACCTTCCACTAATGCGCCTTTTCATCTCAATCAAAGGCCAATTAAGGGTC
CACCACAAGAAGTACTTCAAAAAGATGTATCTATTGGTGATGGTAAATCATCTAGTGGCCAAATCC
TACGCCATTTCTTCGACGATTGGCCTAGACAACAACTTCAAGAAGGCGACAATGCTGCAACCAGCC

**FIGURE 10 (continued)**

TGTCCATTTCGATGCCCGGTGTAGGGGGTAACCCCTCGTCAGACTTCTCGTTGAAGCTTTCAACTG
GGAATTACTATGATTCAGGTACTCAAGTTAGTAATGTTGAACGGTCTACATGGGGGACGAGTCACC
ACCACGTAGCCTCAATGGGTGGTCCACTTGCCGAGGCCTTAAGGTCATCAACAACTAACTCGTCCC
CTACTAGCGTGTTGCATCAATTGGCACGAGGTAGCGCGTCCGAGGCCAGCTATATTAGCACTTGAT
TTCTGCAAGTGTTCTTGTTAAATGTTTTTTCTTTTGGACTTTATTGTTTTTAACTTGGTTGTGT
TGTTGTTCATTGTTCTTTATTGGTATTGATATACCTAACTGTCACCTGTACAAAAAAAAAAAAAAA
AAA

**SEQ ID NO: 26 Lycopersicon esculentum Lyces_GRF translated polypeptide sequence**
MDFNMKQWSNQHESENQESPTKLPRLLLDFHSVSSDSASAAALPLFVSEPTTSTTTCTKLMSDSAT
TVTTKFPRIGSGGGYFSLAQWQELELHSLIFRHFVAGAPVPSELLHLVKKSIIASPPPPPSYYFAH
PYQQYPHYQQALMQSGYWGRAAMDPEPGRCRRTDGKKWRCSRDVVAGQKYCERHVHRGRSRSRKPV
EIPTPANNGSKNNNTVSHHQAFGKMTGHAHAGGGAPQFSLSGHSPSTNAPFHLNQRPIKGPPQEVL
QKDVSIGDGKSSSGQILRHFFDDWPRQQLQEGDNAATSLSISMPGVGGNPSSDFSLKLSTGNYYDS
GTQVSNVERSTWGTSHHHVASMGGPLAEALRSSTTNSSPTSVLHQLARGSASEASYIST

**SEQ ID NO: 27 Medicago truncatula Medtr_GRF nucleic acid sequence AC144645.17**
ATGATGAGTGCAAGTTCAAGAAATAGGTCACTTTTCACACCAAATCAATGGCAAGAACTTGAACAA
CAAGCCCTAGTTTTTAAATACATGGTTACTGGAACACCTATTCCACCAGATCTCATATACTCTATT
AAGAGAAGTTTAGACACTTCAATATCTTCAAGAATCTTTCCTCATCCACCAATTGGGTGGGGATGT
TTTGAAATGGGATTTGGCAGAAAAGTAGACCCAGAGCCAGGGAGGTGCAGAAGAACAGATGGCAAG
AAATGGAGATGCTCAAAGGAAGCATATCCAGACTCAAAGTACTGTGAAAGACACATGCACAGAGGT
AGAAACCGTTCAAGAAAGCCTGTGGAACTAGTAGTTTCTTCTTCAACAACAACACCAACAAATAAC
ACAAACACAGCATCTTCTTACAGCAACAGAAACATCTCCTTGAACAACAACAGCAGCAGCATAAAC
TCACCTTCTTCTTTCCCTTTCTCTACTTCATCCATGGCTTGTCATGATCAGTCACAATCTTTTTCA
CAATCCTACCAAAACTCTTCTTTAAACCCTTACTATTACTCTCAATCAATTACCTCTACTAACCCA
CTTGATCATTCTCATTTTCAAACTCAAGATGCTACTACTCATCACCTCTTTTTGGACTCAACATCT
TATTCTCAGGATGACAAGGACTTTAGGTATGTACAAGTTCAAGGAATAAGAGATGGTACTGTGGAT
GAGAGAACTTTCTTTCCAGAAGCTACAGGTTCATCTAGGAGCTGTTATCATGATTCATATCAACAA
CAACTATCAATGAATCCCTTTAAGTCTTACTCAAGCTCACAGTTTCAGAATATCAATGATGATAAT
TCAAGACAACAACAAGAACAACACTGTTTTGTTTTAGGCACTGACATCAAGTCAACAAGAACAACA
AACAAGGACAAAGAAAGTGAGACAACTCAGAAACCACTTCATCATTTCTTTGGTGAGTGGACACCA
AAGAACACAGATTCCTGGCTAGATCTTGCTTCTAACTCCAGAATTCCAACAGGTTGATTATCATTT
ATCATCATTCCTATGTTTTTGTTTTTTTTTTGTTATTATTAATA

**SEQ ID NO: 28 Medicago truncatula Medtr_GRF translated polypeptide sequence**
MMSASSRNRSLFTPNQWQELEQQALVFKYMVTGTPIPPDLIYSIKRSLDTSISSRIFPHPPIGWGC
FEMGFGRKVDPEPGRCRRTDGKKWRCSKEAYPDSKYCERHMHRGRNRSRKPVELVVSSSTTTPTNN
TNTASSYSNRNISLNNNSSSINSPSSFPFSTSSMACHDQSQSFSQSYQNSSLNPYYYSQSITSTNP
LDHSHFQTQDATTHHLFLDSTSYSQDDKDFRYVQVQGIRDGTVDERTFFPEATGSSRSCYHDSYQQ
QLSMNPFKSYSSSQFQNINDDNSRQQQEQHCFVLGTDIKSTRTTNKDKESETTQKPLHHFFGEWTP
KNTDSWLDLASNSRIPTG

## FIGURE 10 (continued)

**SEQ ID NO: 29 Medicago truncatula Medtr_GRF_like nucleic acid sequence AC174350.4**

ATGCATATGTTGACAATGGAAGCTAAACCTCTTCAACTTGTTCCCTCTTCACACAACAGCACAACT
GGTGGTGGACCCCAGATGAAGATTGAGAATGGTGAAGTTGATGAAGAGAAAAGGGTTGTTGTTGGA
GTGAAGGAAGATATAGAAAACAAGCCTTTGATCACAGAAGCTCAAAGGCGTGAACTTGATCATCAA
GTTTTTATTTTTAATCATTTTGCTTATAATCTTCCTCTTCCTTATTACCTTTTGCAATTTCCAAGT
AATATGTCAGAGTACAGTCGTCGTGGGTCTGATTATGTGACTATGGTGGATCAAGAACCACATAGG
TGTAGAAGAACTGACGGAAAGAAATGGAGGTGCGGCAAGGACACAGTACCTAATCAGAAGTATTGT
GAACGTCACATGCACAGAGGTCGAAATCGTTCAAGAAAGCTTGTGGAAACATCTCAACTTAACTCT
CCTTTGAAAACAAATCCTAGTGGTGGTGGCAAGTCACATGCAAAACTAGTCCCAAACATTAAATCT
TCAGTTTCAAATCCAAACCCTTTGATTATTCATCACAATGGCACATTCTCATACAATCCGAGGACC
TTCTGCGTTGTAGATACTTCTTCTGTTTGTGATCGGTCGAGACATGTCATAGATTATGGTGCCACT
GCAGTGACAACTTCGGGAAGCACGACATCCGTTTCTTTGGATAACAGAGTTTGTCCTAACGTATGC
AAGCAAGATGAGCAGATCAAGAGGTGTATCACCGACAACGTGGGTATTAAAAGTGGTCGGAAAGGA
AGCATATCTTGTGAAAGTATTGGCATCTCTACTGGAATAGGCTTTTCCCCAAAGAGTGTTCTTCCA
GTTTCTGGTTGCAATGATTCATACCTCAACAACAGAAACAATATATTAGAACCTGAACCCGGTAGA
TGCCGAAGAACAGATGGTAAGAAGTGGCGATGCAAGAGTGCGGTTCTTCCAGGTCAGAAGTATTGT
GCAACACATATGCATAGAGGTGCTAAAAGGCGTTTTACAAACCTCGAATCTCCTCCTCCTGCCACC
ACTGTTATTCCTAAAACTACTGATATTAGTTCAGCTGTTACCATTGCTCAGTTGCCCGACCCTTCG
GCTCCAATCGACATCCAGAAAGCGAATTGTTGGTCTCCGAGCACTAAGCTTTCAATGTCGGTTCAA
GAAAGTGCGCCCTTTGTTGATTGTAATGAGAAAAGTGTTAGCAGCGGTGACACGGATGGTACTAGT
ACCACCATCACTGACACCATGAATGAGTGTAGCTATCTTTCTTTCTAA

**SEQ ID NO: 30 Medicago truncatula Medtr_GRF_like translated polypeptide sequence**

MHMLTMEAKPLQLVPSSHNSTTGGGPQMKIENGEVDEEKRVVVGVKEDIENKPLITEAQRRELDHQ
VFIFNHFAYNLPLPYYLLQFPSNMSEYSRRGSDYVTMVDQEPHRCRRTDGKKWRCGKDTVPNQKYC
ERHMHRGRNRSRKLVETSQLNSPLKTNPSGGGKSHAKLVPNIKSSVSNPNPLIIHHNGTFSYNPRT
FCVVDTSSVCDRSRHVIDYGATAVTTSGSTTSVSLDNRVCPNVCKQDEQIKRCITDNVGIKSGRKG
SISCESIGISTGIGFSPKSVLPVSGCNDSYLNNRNNILEPEPGRCRRTDGKKWRCKSAVLPGQKYC
ATHMHRGAKRRFTNLESPPPATTVIPKTTDISSAVTIAQLPDPSAPIDIQKANCWSPSTKLSMSVQ
ESAPFVDCNEKSVSSGDTDGTSTTITDTMNECSYLSF

**SEQ ID NO: 31 Oryza sativa Orysa_GRF_Os02g47280 nucleic acid sequence**

ATGGCGATGCCGTATGCCTCCCTGTCTCCGGCGGTGGCCGACCACCGCTCGTCCCCGGCAGCCGCG
ACCGCCTCCCTCCTCCCCTTCTGCCGCTCCACCCCGCTCTCCGCGGGCGGTGGTGGCGTCGCGATG
GGGGAGGACGCGCCGATGACCGCGAGGTGGCCGCCGGCGGCGGCGGCGAGGCTGCCGCCGTTCACC
GCGGCGCAGTACGAGGAGCTGGAGCAGCAGGCGCTCATATACAAGTACCTGGTGGCAGGCGTGCCC
GTCCCGCCGGATCTCGTGCTCCCCATCCGCCGCGGACTCGACTCCCTCGCCGCCCGCTTCTACAAC
CATCCCGCCCTTGGATATGGTCCGTACTTCGGCAAGAAGCTGGACCCAGAGCCAGGGCGGTGCCGG
CGTACGGACGGCAAGAAATGGCGGTGCTCGAAGGAGGCCGCGCCGGATTCCAAGTACTGCGAGCGC
CACATGCACCGCGGCCGCAACCGTTCAAGAAAGCCTGTGGAAACGCAGCTGGTCGCCCAGTCCCAA
CCGCCCTCATCTGTTGTCGGTTCTGCGGCGGCGCCCCTTGCTGCTGCCTCCAATGGCAGCAGCTTC
CAAAACCACTCTCTTTACCCTGCTATTGCCGGCAGCAATGGCGGGGGCGGGGGGAGGAACATGCCC
AGCTCATTTGGCTCGGCGTTGGGTTCTCAGCTGCACATGGATAATGCTGCCCCTTATGCAGCTGTT
GGTGGTGGAACAGGCAAAGATCTCAGGTATACTGCTTATGGCACAAGATCTTTGGCGGATGAGCAG
AGTCAACTCATTACTGAAGCTATCAACACATCTATTGAAAATCCATGGCGGCTGCTGCCATCTCAG

**FIGURE 10 (continued)**

AACTCGCCATTTCCCCTTTCAAGCTATTCTCAGCTGGGGGCACTAAGTGACCTTGGTCAGAACACC
CCCAGCTCACTTTCAAAGGTTCAGAGGCAGCCACTTTCGTTCTTTGGGAACGACTATGCGGCTGTC
GATTCTGTGAAGCAAGAGAACCAGACGCTGCGTCCCTTCTTTGATGAGTGGCCAAAGGGAAGGGAT
TCATGGTCAGACCTCGCTGATGAGAATGCTAATCTTTCGTCATTCTCAGGCACCCAACTGTCGATC
TCCATACCAATGGCATCCTCTGACTTCTCGGCGGCCAGTTCTCGATCAACTAATGGTGACTGA

**SEQ ID NO: 32 Oryza sativa Orysa_GRF_ Os02g47280.2 translated polypeptide sequence**

MAMPYASLSPAVADHRSSPAAATASLLPFCRSTPLSAGGGGVAMGEDAPMTARWPPAAAARLPPFT
AAQYEELEQQALIYKYLVAGVPVPPDLVLPIRRGLDSLAARFYNHPALGYGPYFGKKLDPEPGRCR
RTDGKKWRCSKEAAPDSKYCERHMHRGRNRSRKPVETQLVAQSQPPSSVVGSAAAPLAAASNGSSF
QNHSLYPAIAGSNGGGGGRNMPSSFGSALGSQLHMDNAAPYAAVGGGTGKDLRYTAYGTRSLADEQ
SQLITEAINTSIENPWRLLPSQNSPFPLSSYSQLGALSDLGQNTPSSLSKVQRQPLSFFGNDYAAV
DSVKQENQTLRPFFDEWPKGRDSWSDLADENANLSSFSGTQLSISIPMASSDFSAASSRSTNGD

**SEQ ID NO: 33 Oryza sativa Orysa_GRF_Os02g53690 nucleic acid sequence**

ATGATGATGATGAGCGGTCGCCCGAGCGGCGGCGCCGGCGGAGGTCGGTACCCGTTCACGGCGTCG
CAGTGGCAGGAGCTGGAGCACCAGGCGCTCATCTACAAGTACATGGCGTCCGGGACTCCCATCCCC
TCCGACCTCATCCTCCCCCTCCGCCGCAGCTTCCTCCTCGACTCCGCCCTCGCCACCTCCCCTTCC
CTCGCCTTCCCTCCCCAACCTTCACTGGGGTGGGGTTGCTTTGGCATGGGGTTTGGGCGGAAGGCG
GAGGACCCGGAGCCAGGGCGATGCCGGCGTACGGACGGCAAGAAGTGGCGGTGCTCCAAGGAGGCG
TACCCGGACTCCAAGTACTGCGAGAAGCACATGCACCGTGGCAAGAACCGTTCAAGAAAGCCTGTG
GAAATGTCCTTGGCCACGCCGCCGCCGCCGTCCTCCTCCGCCACCTCCGCCGCGTCGAACACCTCC
GCCGGCGTCGCCCCCACCACCACCACCACCTCCTCCCCGGCGCCCTCCTACAGCCGCCCGGCGCCG
CACGACGCGGCGCCGTACCAGGCGCTCTACGGCGGCCCTACGCCGCGGCCACCGCGCGCACCCCC
GCCGCCGCGGCGTACCACGCGCAGGTGAGCCCGTTCCACCTCCAGCTCGACACCACCCACCCGCAC
CCGCCGCCGTCCTACTACTCCATGGACCACAAGGAGTACGCGTACGGGCACGCCACCAAGGAGGTG
CACGGCGAGCACGCCTTCTTCTCCGATGGCACCGAGAGGGAGCACCACCACGCCGCCGCCGGGCAC
GGCCAGTGGCAGTTCAAGCAGCTCGGCATGGAGCCCAAGCAGAGCACCACGCCTCTCTTCCCGGGC
GCCGGCTACGGCCACACCGCGGCGTCGCCGTACGCCATTGATCTTTCAAAAGAGGACGACGATGAG
AAAGAGAGGCGGCAACAGCAGCAGCAGCAGCAGCAGCAGCACTGCTTCCTCCTGGGCGCCGACCTC
CGTCTGGAGAAGCCGGCGGGCCACGACCACGCGGCGGCGGCGCAGAAACCTCTCCGCCACTTCTTC
GACGAGTGGCCGCATGAGAAGAACAGCAAGGGCTCCTGGATGGGGCTCGAAGGCGAGACGCAGCTG
TCCATGTCCATCCCCATGGCCGCCAACGACCTCCCGATCACCACCACCTCCCGCTACCACAATGAT
GATTAA

**SEQ ID NO: 34 Oryza sativa Orysa_GRF_Os02g53690 translated polypeptide sequence**

MMMMSGRPSGGAGGGRYPFTASQWQELEHQALIYKYMASGTPIPSDLILPLRRSFLLDSALATSPS
LAFPPQPSLGWGCFGMGFGRKAEDPEPGRCRRTDGKKWRCSKEAYPDSKYCEKHMHRGKNRSRKPV
EMSLATPPPPSSSATSAASNTSAGVAPTTTTTSSPAPSYSRPAPHDAAPYQALYGGPYAAATARTP
AAAAYHAQVSPFHLQLDTTHPHPPPSYYSMDHKEYAYGHATKEVHGEHAFFSDGTEREHHHAAAGH
GQWQFKQLGMEPKQSTTPLFPGAGYGHTAASPYAIDLSKEDDDEKERRQQQQQQQQHCFLLGADL
RLEKPAGHDHAAAAQKPLRHFFDEWPHEKNSKGSWMGLEGETQLSMSIPMAANDLPITTTSRYHND
D

FIGURE 10 (continued)

**SEQ ID NO: 35 Oryza sativa Orysa_GRF_Os03g51970.1 nucleic acid sequence**

ATGCAGGGTGCAATGGCCAGGGTGAGGGGTCCCTTCACGCCGTCTCAGTGGATCGAGCTGGAGCAC
CAGGCGCTGATATACAAGTACTTGGCTGCGAATAGCCCTGTACCACACAGCCTCCTCATCCCCATC
AGGAGGAGCCTCACATCGCCCTACTCACCTGCCTACTTTGGCTCAAGCACATTGGGATGGGGATCT
TTCCAGCTGGGCTACTCCGGCAGCGCGGATCCGGAGCCCGGCCGGTGCCGCCGGACGGACGGCAAG
AAATGGCGGTGCTCGAGGGATGCGGTCGCCGACCAGAAGTACTGTGAGCGACACATGAACCGGGGA
CGCCACCGTTCAAGAAAGCATGTGGAAGGCCAGCCTGGCCATGCCGCGAAAGCGATGCCCGCGGCG
GTGGCAGCAGCCGCTGCCTCTGCTACCCAGCCTAGTGCTCCGGCCGCCCACAGTGGCGGAGCTGTT
GCTGGCCTCGCTATCAACCATCAGCACCAGCAAATGAAGAACTACGCTGCCAACACTGCCAATCCT
TGCTCTCTGCAATATAGCAGGGATCTGGCAAACAAGCATAATGAGAGTGAACAAGTGCAAGACTCA
GACAGTCTCTCGATGCTGACTTCCATTAGCACGAGAAATACGGGCAGCCTGTTTCCGTTCTCAAAA
CAACATAATCCTTTTGAAGTGTCCAACTCAAGGCCAGATTTTGGCCTAGTATCACCTGATTCACTG
ATGAGTTCTCCTCATAGCTCCTTGGAGAACGTCAATTTGCTCACTTCGCAGAGTCTGAATGAACAA
CAGAGTTCAGTTTCCCTTCAACACTTTGTGGACTGGCCAAGGACACCTGCACAAGGAGCTCTCGCA
TGGCCTGATGCTGAAGACATGCAAGCTCAGAGAAGCCAGCTCTCAATATCTGCTCCAATGGCGTCT
TCTGACCTGTCATCAGCCTCAACATCTCCCATCCATGAGAAGCTGATGTTGTCACCACTTAAACTG
AGCCGTGAATATAGTCCTATTGGTCTCGGTTTTGCAGCAAATAGAGATGAGGTTAACCAGGGAGAA
GCAAACTGGATGCCTATGTTCCGTGATTCTTTGATGGGCGGACCATTGGGAGAGGTTTTAACCAAG
AATAACAACATGGAAGCAAGGAATTGCCTATCGGAGTCTCTGAATCTTTTAAATGATGGCTGGGAT
TCAAGCTCAGGGTTTGATTCATCCCCAGTTGGTGTTCTGCAGAAGACCACCTTTGGATCAGTATCC
AGTAGCACCGGAAGCAGTCCTAGACTGGAGAATCATAGTGTTTATGATGGCAACAGTAACCTGCGG
GATGATCTCGGTTCAGTTGTTGTAAATCATCCGAGCATCCGCCTGGTGTGA

**SEQ ID NO: 36 Oryza sativa Orysa_GRF_Os03g51970.1 translated polypeptide sequence**

MQGAMARVRGPFTPSQWIELEHQALIYKYLAANSPVPHSLLIPIRRSLTSPYSPAYFGSSTLGWGS
FQLGYSGSADPEPGRCRRTDGKKWRCSRDAVADQKYCERHMNRGRHRSRKHVEGQPGHAAKAMPAA
VAAAASATQPSAPAAHSGGAVAGLAINHQHQQMKNYAANTANPCSLQYSRDLANKHNESEQVQDS
DSLSMLTSISTRNTGSLFPFSKQHNPFEVSNSRPDFGLVSPDSLMSSPHSSLENVNLLTSQSLNEQ
QSSVSLQHFVDWPRTPAQGALAWPDAEDMQAQRSQLSISAPMASSDLSSASTSPIHEKLMLSPLKL
SREYSPIGLGFAANRDEVNQGEANWMPMFRDSLMGGPLGEVLTKNNNMEARNCLSESLNLLNDGWD
SSSGFDSSPVGVLQKTTFGSVSSSTGSSPRLENHSVYDGNSNLRDDLGSVVVNHPSIRLV

**SEQ ID NO: 37 Oryza sativa Orysa_GRF_Os04g48510.1 nucleic acid sequence**

ATGTTGGCCGAGGGAAGGCAAGTCTACTTGCCGCCGCCGCCGCCGTCCAAGCTTCCTCGTCTCTCC
GGCACCGATCCAACCGACGGCGTGGTGACGATGGCAGCGCCGTCGCCGCTGGTTCTTGGGCTGGGT
CTCGGTCTGGGCGGCAGCGGCAGCGACAGCAGTGGGAGCGACGCGGAAGCGTCTGCGGCCACCGTG
CGGGAGGCGCGGCCGCCGTCGGCGCTGACGTTCATGCAGCGGCAGGAGCTGGAGCAGCAGGTGCTC
ATCTACCGCTACTTCGCCGCCGGCGCGCCTGTGCCGGTTCACCTCGTGCTGCCCATATGGAAGAGC
ATCGCCGCCGCCTCCTCGTTCGGCCCGCAAAGCTTTCCCTCCCTGACGGGCCTGGGGAGCCTGTGC
TTCGACTACAGGAGCAGCATGGAGCCGGAGCCGGGGCGGTGCCGGCGCACGGACGGCAAGAAGTGG
CGGTGCTCGCGCGACGTGGTGCCGGGGCACAAGTATTGCGAGCGGCACGTCCACCGTGGCCGCGGC
CGTTCAAGAAAGCCTATGGAAGCCTCTGCAGCAGTCGCTCCCACATATCTCCCGGTCCGGCCGGCA
CTCCACACCGTCGCCACCCTCGCCACCAGCGCGCCATCGCTGTCGCACCTCGGTTTCTCCTCCGCC
AGCAAAGTGCTCCTCGCCCACACCACCACCGGCACCACGCGCGCTACTTGA

**FIGURE 10 (continued)**

**SEQ ID NO: 38 Oryza sativa Orysa_GRF_Os04g48510.1 translated polypeptide sequence**

MLAEGRQVYLPPPPPSKLPRLSGTDPTDGVVTMAAPSPLVLGLGLGLGGSGSDSSGSDAEASAATV
REARPPSALTFMQRQELEQQVLIYRYFAAGAPVPVHLVLPIWKSIAAASSFGPQSFPSLTGLGSLC
FDYRSSMEPEPGRCRRTDGKKWRCSRDVVPGHKYCERHVHRGRGRSRKPMEASAAVAPTYLPVRPA
LHTVATLATSAPSLSHLGFSSASKVLLAHTTTGTTRAT

**SEQ ID NO: 39 Oryza sativa Orysa_GRF_Os04g51190.1 nucleic acid sequence**

ATGGCGATGCCCTTTGCCTCCCTGTCGCCGGCAGCCGACCACCGGCCCTCCTTCATCTTCCCCTTC
TGCCGCTCCTCCCCTCTCTCCGCGGTCGGGGAGGAGGCGCAGCAGCACATGATGGGCGCGAGGTGG
GCGGCGGCGGTGGCCAGGCCGCCGCCCTTCACGGCGGCGCAGTACGAGGAGCTGGAGCAGCAGGCG
CTCATATACAAGTACCTCGTCGCCGGCGTGCCCGTCCCGGCGGATCTCCTCCTCCCCATCCGCCGT
GGCCTCGACTCACTCGCCTCGCGCTTCTACCACCACCCTGTCCTTGGATACGGTTCCTACTTCGGC
AAGAAGCTGGACCCGGAGCCCGGACGGTGCCGGCGTACGGACGGCAAGAAGTGGCGGTGCTCCAAG
GAGGCCGCGCCGGACTCCAAGTACTGTGAGCGACACATGCACCGCGGCCGCAACCGTTCAAGAAAG
CCTGTGGAAGCGCAGCTCGTCGCCCCCCACTCGCAGCCCCCCGCCACGGCGCCGGCCGCCGCCGTC
ACCTCCACCGCCTTCCAGAACCACTCGCTGTACCCGGCGATTGCTAATGGCGGCGGCGCCAACGGA
GGCGGTGGTGGTGGTGGCGGTGGCGGCAGCGCGCCTGGCTCGTTCGCCTTGGGGTCTAATACTCAG
CTGCACATGGACAATGCTGCGTCTTACTCGACTGTTGCTGCTGGTGCCGGAAACAAAGATTTCAGG
TATTCTGCTTATGGAGTGAGACCATTGGCAGATGAGCACAGCCCACTCATCACTGGAGCTATGGAT
ACCTCTATTGACAATTCGTGGTGCTTGCTGCCTTCTCAGACCTCCACATTTTCAGTTTCGAGCTAC
CCTATGCTTGGAAATCTGAGTGAGCTGGACCAGAACACCATCTGCTCGCTGCCGAAGGTGGAGAGG
GAGCCATTGTCATTCTTCGGGAGCGACTATGTGACCGTCGACTCCGGGAAGCAGGAGAACCAGACG
CTGCGCCCCTTTTTCGACGAGTGGCCAAAGGCAAGGGACTCCTGGCCTGATCTAGCTGATGACAAC
AGCCTTGCCACCTTCTCTGCCACTCAGCTCTCGATCTCCATTCCAATGGCAACCTCTGACTTCTCG
ACCACCAGCTCACGATCACACAACGGTATATACTCCCGATGA

**SEQ ID NO: 40 Oryza sativa Orysa_GRF_Os04g51190.1 translated polypeptide sequence**

MAMPFASLSPAADHRPSFIFPFCRSSPLSAVGEEAQQHMMGARWAAAVARPPPFTAAQYEELEQQA
LIYKYLVAGVPVPADLLLPIRRGLDSLASRFYHHPVLGYGSYFGKKLDPEPGRCRRTDGKKWRCSK
EAAPDSKYCERHMHRGRNRSRKPVEAQLVAPHSQPPATAPAAAVTSTAFQNHSLYPAIANGGGANG
GGGGGGGGGSAPGSFALGSNTQLHMDNAASYSTVAAGAGNKDFRYSAYGVRPLADEHSPLITGAMD
TSIDNSWCLLPSQTSTFSVSSYPMLGNLSELDQNTICSLPKVEREPLSFFGSDYVTVDSGKQENQT
LRPFFDEWPKARDSWPDLADDNSLATFSATQLSISIPMATSDFSTTSSRSHNGIYSR

**SEQ ID NO: 41 Oryza sativa Orysa_GRF_LOC_Os06g02560.1 nucleic acid sequence**

ATGCTGAGCTCGTCGCCCTCGGCGGCGGCGCCGGGGATAGGAGGGTACCAGCCGCAGCGCGGGGCG
GCGGTCTTCACGGCGGCGCAGTGGGCGGAGCTGGAGCAGCAGGCGCTCATTTACAAGTACCTCGTC
GCCGGTGTCCCCGTCCCGGGCGATCTCCTCCTCCCAATCCGCCCCCACTCCTCCGCCGCCGCCACC
TACTCCTTCGCCAACCCCGCCGCCGCGCCCTTCTACCACCACCACCACCACCCCTCTCTGAGCTAT
TATGCCTACTATGGCAAGAAGCTTGACCCTGAGCCGTGGCGTTGCCGCCGCACCGACGGCAAGAAG
TGGCGGTGCTCCAAGGAGGCGCACCCCGACTCCAAGTACTGCGAGCGCCACATGCACCGTGGCCGC
AACCGTTCAAGAAAGCCTGTGGAATCCAAGACCGCTGCCCCTGCGCCCCAGTCGCAGCCCCAGCTG
TCCAATGTCACGACCGCGACTCACGACACCGATGCGCCTCTCCCGTCACTCACTGTGGGTGCTAAA
ACCCACGGTCTGTCCCTTGGTGGTGCTGGCTCGTCGCAGTTCCATGTCGACGCACCATCGTACGGC

**FIGURE 10 (continued)**

AGCAAGTACTCTCTTGGAGCTAAAGCTGATGTGGGTGAACTGAGCTTCTTCTCAGGAGCATCAGGA
AACACCAGGGGCTTCACCATTGATTCTCCAACAGATAGCTCATGGCATTCACTGCCTTCCAGTGTA
CCCCCATACCCGATGTCAAAGCCAAGGGACTCTGGCCTCCTACCAGGTGCCTACTCCTACTCCCAC
CTTGAACCTTCACAGGAACTTGGCCAGGTCACCATCGCCTCGCTGTCCCAAGAGCAGGAGCGCCGC
TCTTTTGGTGGTGGAGCGGGGGGGATGCTAGGAAATGTGAAGCACGAGAACCAGCCGCTGAGGCCT
TTCTTCGATGAGTGGCCTGGGAGGCGAGACTCGTGGTCGGAGATGGATGAGGAGAGGTCCAACCAG
ACCTCCTTCTCGACAACCCAGCTCTCGATCTCCATCCCGATGCCCAGATGTGGGTCCCCTATCGGT
CCGCGTCTACCTTGA

**SEQ ID NO: 42 Oryza sativa Orysa_GRF_LOC_Os06g02560.1 translated polypeptide sequence**

MLSSSPSAAAPGIGGYQPQRGAAVFTAAQWAELEQQALIYKYLVAGVPVPGDLLLPIRPHSSAAAT
YSFANPAAAPFYHHHHHPSLSYYAYYGKKLDPEPWRCRRTDGKKWRCSKEAHPDSKYCERHMHRGR
NRSRKPVESKTAAPAPQSQPQLSNVTTATHDTDAPLPSLTVGAKTHGLSLGGAGSSQFHVDAPSYG
SKYSLGAKADVGELSFFSGASGNTRGFTIDSPTDSSWHSLPSSVPPYPMSKPRDSGLLPGAYSYSH
LEPSQELGQVTIASLSQEQERRSFGGGAGGMLGNVKHENQPLRPFFDEWPGRRDSWSEMDEERSNQ
TSFSTTQLSISIPMPRCGSPIGPRLP

**SEQ ID NO: 43 Oryza sativa Orysa_GRF_Os11g35030.1 nucleic acid sequence**

ATGCTGAGCTCTTGTGGTGGCCATGGCCATGGAAATCCAAGAAGCTTGCAAGAAGAACACCATGGC
AGATGTGGTGAGCAGCAAGGTGGAGGAGGAGGAGGAGGGCAAGAGCAAGAGCAAGATGGGTTCTTG
GTGAGAGAGGCAAGGGCATCCCCACCATCTCCATCTTCTTCATCATTTCTTGGATCCACAAGCTCT
TCTTGTTCTGGAGGAGGAGGAGGAGGGCAGATGTTGAGCTTCTCCTCCCCCAATGGAACAGCAGGG
TTGGGCTTGAGCTCAGGAGGAAGCATGCAGGGGGTCTTGGCAAGGGTCAGGGGGCCGTTCACCCCA
ACACAGTGGATGGAGCTGGAGCACCAGGCACTGATCTACAAGCACATTGCTGCAAATGTTTCTGTC
CCTTCCAGCTTGCTCCTCCCCATCAGGAGAAGCCTCCATCCATGGGGATGGGGATCATTCCCTCCT
GGCTGTGCTGATGTAGAACCCAGAAGATGCCGCCGCACAGACGGCAAGAAGTGGCGGTGCTCCAGA
GATGCTGTTGGGGATCAGAAGTATTGTGAGCGACACATAAACCGTGGTCGCCATCGTTCAAGAAAG
CATGTGGAAGGCCGAAAGGCGACACTCACCATTGCAGAACCATCCACGGTTATTGCTGCTGGTGTA
TCATCTCGCGGCCACACTGTGGCTCGGCAGAAGCAGGTGAAAGGCTCAGCTGCTACTGTCTCTGAT
CCTTTCTCGAGACAATCCAACAGGAAATTTCTGGAGAAACAGAACGTTGTCGACCAATTGTCTCCC
ATGGATTCATTTGATTTCTCATCCACACAATCTTCTCCAAACTATGACAATGTAGCATTGTCACCA
CTGAAGTTGCACCATGATCATGATGAATCTTACATCGGGCATGGAGCAGGCAGTTCATCAGAAAAA
GGCAGTATGATGTACGAAAGTCGGTTAACAGTCTCTAAGGAAACACTTGATGATGGACCTTTAGGT
GAAGTTTTCAAAAGAAAGAATTGCCAATCAGCTTCTACAGAAATCTTAACTGAAAAATGGACTGAG
AACCCCAACTTACATTGCCCATCTGGAATCCTACAAATGGCTACTAAGTTCAATTCAATTTCCAGC
GGCAACACAGTAAATAGTGGTGGCACCGCAGTGGAGAATCTTATCACTGATAATGGATATCTTACT
GCAAGAATGATGAATCCTCATATTGTCCCAACACTTCTCTAA

**SEQ ID NO: 44 Oryza sativa Orysa_GRF_Os11g35030.1 translated polypeptide sequence**

MLSSCGGHGHGNPRSLQEEHHGRCGEQQGGGGGGGQEQEQDGFLVREARASPPSPSSSSFLGSTSS
SCSGGGGGGQMLSFSSPNGTAGLGLSSGGSMQGVLARVRGPFTPTQWMELEHQALIYKHIAANVSV
PSSLLLPIRRSLHPWGWGSFPPGCADVEPRRCRRTDGKKWRCSRDAVGDQKYCERHINRGRHRSRK
HVEGRKATLTIAEPSTVIAAGVSSRGHTVARQKQVKGSAATVSDPFSRQSNRKFLEKQNVVDQLSP
MDSFDFSSTQSSPNYDNVALSPLKLHHDHDESYIGHGAGSSSEKGSMMYESRLTVSKETLDDGPLG
EVFKRKNCQSASTEILTEKWTENPNLHCPSGILQMATKFNSISSGNTVNSGGTAVENLITDNGYLT
ARMMNPHIVPTLL

**FIGURE 10 (continued)**

**SEQ ID NO: 45 Oryza sativa Orysa_GRF_LOC_Os12g29980.1 nucleic acid sequence**

ATGGCAATGGCGACCCCTACGACCAACGGCAGCTTCCTTCTTGGATCAGGTGGCTATCCCGGTGCC
CAGATTCTAAGCTTCTCCTCCTCAGGTCACAGCGGCAATGGGTTGGATTGTGGAAGCTCAGATGTG
GCAAGAATGCAGGGGGTTTTAGCAAGGGTTAGGGGGCCATTCACACCAACACAATGGATGGAGCTG
GAGCACCAGGCTCTGATCTACAAGCACATTGTGGCGAATGCGCCGGTACCGGCCGGCTTGCTCCTC
CCCATCAGGAGAAGCCTCCATCCACCAGTGTTCCCACACTTCTCCTCTGGTGGCATTCTTGGCTCC
AGCTCCTTGGGATGGGGGTCATTTCAGCTGGGCTATTCTGGGAGTGCTGACTCCGAGCCCGGGAGA
TGCCGTCGAACCGATGGCAAGAAATGGCGGTGCTCGAGAGACGCAGTTGTCGACCAAAAGTACTGC
GAGCGGCACATAAACCGGGGTCGCCACCGTTCAAGAAAGCATGTGGAAGGCCAATCTAGCCATGCC
GCAAAAGCAACGGTTCCCGCCATAGCACAACCACCCATTGGTGCATCTAATGGCAAATTGTCAGGC
AGCCATGGTGTGTCAAATGAGCTCACGAAAACCTTGGCTACTAACAGGATGATGTTGGATAAAGCA
AATCTTATTGAACGCTCCCAGGACTACACTAATCAGCAACACAACATCCTACAGAACAACACAAAA
GGTGATAATTGGTCTGAAGAGATGTCCTCACAAGCAGACTATGCAGTAATCCCTGCTGGCTCTCTC
ATGAACACACCGCAATCGGCGAATTTAAATCCAATTCCCCAGCAACAACGCTGTAAGCAGTCACTC
TTTGGCAAAGGGATACAGCATGATGACATTCAGCTGTCGATATCCATTCCCGTGGATAACTCCGAC
TTACCCACTAACTACAACAAGGCTCAAATGGACCATGTAGTAGGCGGTTCATCGAATGGCGGAAAC
AACACGCGAGCAAGTTGGATACCGGGCTCCTGGGAAGCGTCCATAGGTGGACCTCTGGGTGAGTTC
TTCACCAACACCAGCAGCGCATCAGACGACAAAGGCAAAAGCCGCCACCCGCCATCTTTGAACCTC
TTAGCTGATGGACATACTACAAGTCCACAGCTGCAATCGCCCACCGGAGTCCTGCAGATGACTAGC
TTCAGTTCAGTGCCCAGCAGCACTGTTAGTAGTCCTGCAGGCAGCCTCTGCAATGGCTTGCTCACT
TCAGGCCTGGTGAATGCCCAGACTGTCCAAACACTGTGA

**SEQ ID NO: 46 Oryza sativa Orysa_GRF_LOC_Os12g29980.1 translated polypeptide sequence**

MAMATPTTNGSFLLGSGGYPGAQILSFSSSGHSGNGLDCGSSDVARMQGVLARVRGPFTPTQWMEL
EHQALIYKHIVANAPVPAGLLLPIRRSLHPPVFPHFSSGGILGSSSLGWGSFQLGYSGSADSEPGR
CRRTDGKKWRCSRDAVVDQKYCERHINRGRHRSRKHVEGQSSHAAKATVPAIAQPPIGASNGKLSG
SHGVSNELTKTLATNRMMLDKANLIERSQDYTNQQHNILQNNTKGDNWSEEMSSQADYAVIPAGSL
MNTPQSANLNPIPQQQRCKQSLFGKGIQHDDIQLSISIPVDNSDLPTNYNKAQMDHVVGGSSNGGN
NTRASWIPGSWEASIGGPLGEFFTNTSSASDDKGKSRHPPSLNLLADGHTTSPQLQSPTGVLQMTS
FSSVPSSTVSSPAGSLCNGLLTSGLVNAQTVQTL

**SEQ ID NO: 47 Oryza sativa Orysa_GRF_Os03g47140.1 nucleic acid sequence**

ATGTTTGCTGACTTCTCTGCTGCTGCCATGGAGCTTGGAGAGGTGTTGGGCTTGCAAGGACTCACA
GTGCCATCCACCAAGGAGGGTGATCTGAGCCTCATCAAGAGAGCTGCTGCTGGTAGCTTCACCCAG
GCTGCTGCTGCATCATACCCTTCCCCCTTTCTTGATGAACAGAAGATGCTCAGATTCGCCAAGGCT
GCTCACACATTGCCATCAGGTTTGGATTTTGGGAGGGAAAATGAGCAGAGGTTCTTGTTGTCTAGG
ACCAAGAGGCCTTTCACTCCCTCACAGTGGATGGAGCTGGAGCACCAGGCTCTCATTTACAAGTAT
CTCAATGCAAAGGCCCCTATACCTTCCAGCCTGCTCATTTCAATCAGCAAAAGCTTCAGATCATCA
GCTAACAGAATGAGCTGGAGGCCTCTCTATCAAGGCTTCCCAAATGCAGACTCTGACCCAGAACCT
GGAAGATGCCGTCGAACAGATGGCAAGAAATGGCGGTGTTCAAAGGAGGCCATGGCCGACCACAAG
TATTGTGAGAGGCACATCAACAGAAACCGCCACCGTTCAAGAAAGCCTGTGGAAAACCAAAGTAGA
AAGACTGTGAAAGAGACACCGTGTGCTGGCTCATTGCCATCTTCTGTCGGGCAGGGCAGCTTCAAG
AAGGCAAAAGTTAATGAAATGAAGCCACGCAGTATCAGCTATTGGACAGATAGTTTGAACAGGACA
ATGGCGAACAAAGAGAAAGGAAACAAAGCTGCTGAAGAAAACAATGGCCCACTGCTAAATTTAACG
AATCAACAGCCAACATTGTCCCTGTTCTCTCAGTTGAAGCAACAGAACAAACCGGAGAAGTTCAAT

**FIGURE 10 (continued)**

ACAGCAGGAGACAGTGAATCGATTTCTTCAAATACCATGTTGAAGCCTTGGGAGAGCAGCAACCAG
CAGAACAACAAAAGCATTCCTTTCACCAAGATGCATGATCGTGGATGCCTTCAGTCAGTCCTTCAG
AATTTCAGCTTGCCTAAGGACGAGAAAATGGAGTTTCAGAAAAGCAAAGATTCCAATGTCATGACA
GTTCCATCAACTTTCTATTCCTCGCCAGAGGACCCACGCGTCAGCTGCCATGCACCTAATATGGCA
CAAATGCAAGAGGATAGCATCTCAAGTTCTTGGGAGATGCCTCAAGGTGGACCTCTAGGTGAGATC
TTGACAAACTCCAAAAATCCTGACGATTCAATCATGAAACCAGAAGCAAGGCCATATGGTTGGTTA
CTGAACCTCGAGGATCATGCAATGTGA

**SEQ ID NO: 48 Oryza sativa Orysa_GRF_Os03g47140.1 translated polypeptide sequence**

MFADFSAAAMELGEVLGLQGLTVPSTKEGDLSLIKRAAAGSFTQAAAASYPSPFLDEQKMLRFAKA
AHTLPSGLDFGRENEQRFLLSRTKRPFTPSQWMELEHQALIYKYLNAKAPIPSSLLISISKSFRSS
ANRMSWRPLYQGFPNADSDPEPGRCRRTDGKKWRCSKEAMADHKYCERHINRNRHRSRKPVENQSR
KTVKETPCAGSLPSSVGQGSFKKAKVNEMKPRSISYWTDSLNRTMANKEKGNKAAEENNGPLLNLT
NQQPTLSLFSQLKQQNKPEKFNTAGDSESISSNTMLKPWESSNQQNNKSIPFTKMHDRGCLQSVLQ
NFSLPKDEKMEFQKSKDSNVMTVPSTFYSSPEDPRVSCHAPNMAQMQEDSISSSWEMPQGGPLGEI
LTNSKNPDDSIMKPEARPYGWLLNLEDHAM

**SEQ ID NO: 49 Oryza sativa Orysa GRF gi_115447910 ref NM_001054270.1 nucleic acid sequence**

GACAACTCACTGCCCCCCATCTTCTTTCTTCATTCCTCTTCCCACCAAGAACCCCAAACCTTACCT
CCATTGAGTTCGAAACCGAGGAGCGAGGAGTTACAAGCCGAGTTGTCAGAATGGATGAGGAGAAGG
AAGCCGACTCGCCGCAGCCACCGTCCAAGCTGCCTCGCCTCTCCGGCGCTGACCCGAATGCCGGAG
TGGTGACCATGGCAGCACCCCCGCCGCCGGTGGGTCTTGGGCTGGGGCTTGGACTCGGCGGCGACA
GCCGCGGCGAGCGTGACGTGGAAGCGTCGGCGGCGGCGGCGCACAAGGCGACGGCGCTGACGTTCA
TGCAGCAGCAGGAGCTGGAGCACCAGGTGCTCATCTACCGCTACTTCGCCGCGGGCGCGCCCGTGC
CGGTGCACCTCGTGCTCCCCATCTGGAAGAGCGTCGCGTCCTCCTCCTTCGGCCCGCACCGCTTCC
CTTCCCTGGCAGTGATGGGGTTGGGGAACCTGTGCTTCGACTACCGGAGCAGCATGGAGCCGGACC
CAGGGCGGTGCAGGCGCACGGACGGCAAGAAGTGGCGGTGCTCGCGCGACGTGGTGCCGGGGCACA
AGTACTGCGAGCGGCACGTCCACCGCGGACGCGGCCGTTCAAGAAAGCCTGTGGAAGCCTCCGCGG
CCGCCACCCCGGCGAACAACGGCGGCGGCGGTGGCATCGTCTTCTCCCCCACCAGCGTCCTCCTCG
CCCACGGCACCGCGCGCGCCACCTGACCAGTGACCAGACCGGCGCCCGTTTGTTTGTTTGTCTCGG
CGCATGGGAAAACCCAAATCCGCAGGGATTATGTCATGTCCTGTAACTCTTTTTTTCCTCGCAACT
TTTGAAGCCAAAACAATTCTCACCGTGTTCGATGGC

**SEQ ID NO: 50 Oryza sativa Orysa GRF gi_115447910 ref NM_001054270.1 translated polypeptide sequence**

MDEEKEADSPQPPSKLPRLSGADPNAGVVTMAAPPPPVGLGLGLGLGGDSRGERDVEASAAAAHKA
TALTFMQQQELEHQVLIYRYFAAGAPVPVHLVLPIWKSVASSSFGPHRFPSLAVMGLGNLCFDYRS
SMEPDPGRCRRTDGKKWRCSRDVVPGHKYCERHVHRGRGRSRKPVEASAAATPANNGGGGIVFSP
TSVLLAHGTARAT

**SEQ ID NO: 51 Oryza sativa Orysa GRF gi_115460325 ref NM_001060298.1 nucleic acid sequence**

ATGCCCTTTGCCTCCCTGTCGCCGGCAGCCGACCACCGGCCCTCCTTCATCTTCCCCTTCTGCCGC
TCCTCCCCTCTCTCCGCGGTCGGGGAGGAGGCGCAGCAGCACATGATGGGCGCGAGGTGGGCGGCG
GCGGTGGCCAGGCCGCCGCCCTTCACGGCGGCGCAGTACGAGGAGCTGGAGCAGCAGGCGCTCATA
TACAAGTACCTCGTCGCCGGCGTGCCCGTCCCGGCGGATCTCCTCCTCCCCATCCGCCGTGGCCTC

## FIGURE 10 (continued)

GACTCACTCGCCTCGCGCTTCTACCACCACCCTGTCCTTGGATACGGTTCCTACTTCGGCAAGAAG
CTGGACCCGGAGCCCGGACGGTGCCGGCGTACGGACGGCAAGAAGTGGCGGTGCTCCAAGGAGGCC
GCGCCGGACTCCAAGTACTGTGAGCGACACATGCACCGCGGCCGCAACCGTTCAAGAAAGCCTGTG
GAAGCGCAGCTCGTCGCCCCCCACTCGCAGCCCCCCGCCACGGCGCCGGCCGCCGCCGTCACCTCC
ACCGCCTTCCAGAACCACTCGCTGTACCCGGCGATTGCTAATGGCGGCGGCGCCAACGGAGGCGGT
GGTGGTGGTGGCGGTGGCGGCAGCGCGCCTGGCTCGTTCGCCTTGGGGTCTAATACTCAGCTGCAC
ATGGACAATGCTGCGTCTTACTCGACTGTTGCTGCTGGTGCCGGAAACAAAGATTTCAGGTATTCT
GCTTATGGAGTGAGACCATTGGCAGATGAGCACAGCCCACTCATCACTGGAGCTATGGATACCTCT
ATTGACAATTCGTGGTGCTTGCTGCCTTCTCAGACCTCCACATTTTCAGTTTCGAGCTACCCTATG
CTTGGAAATCTGAGTGAGCTGGACCAGAACACCATCTGCTCGCTGCCGAAGGTGGAGAGGGAGCCA
TTGTCATTCTTCGGGAGCGACTATGTGACCGTCGACTCCGGGAAGCAGGAGAACCAGACGCTGCGC
CCCTTTTTCGACGAGTGGCCAAAGGCAAGGGACTCCTGGCCTGATCTAGCTGATGACAACAGCCTT
GCCACCTTCTCTGCCACTCAGCTCTCGATCTCCATTCCAATGGCAACCTCTGACTTCTCGACCACC
AGCTCACGATCACACAACGATGAGTGA

**SEQ ID NO: 52 Oryza sativa Orysa GRF gi_115460325 ref NM_001060298.1 translated polypeptide sequence**

MPFASLSPAADHRPSFIFPFCRSSPLSAVGEEAQQHMMGARWAAAVARPPPFTAAQYEELEQQALI
YKYLVAGVPVPADLLLPIRRGLDSLASRFYHHPVLGYGSYFGKKLDPEPGRCRRTDGKKWRCSKEA
APDSKYCERHMHRGRNRSRKPVEAQLVAPHSQPPATAPAAAVTSTAFQNHSLYPAIANGGGANGGG
GGGGGGGSAPGSFALGSNTQLHMDNAASYSTVAAGAGNKDFRYSAYGVRPLADEHSPLITGAMDTS
IDNSWCLLPSQTSTFSVSSYPMLGNLSELDQNTICSLPKVEREPLSFFGSDYVTVDSGKQENQTLR
PFFDEWPKARDSWPDLADDNSLATFSATQLSISIPMATSDFSTTSSRSHNDE

**SEQ ID NO: 53 Oryza sativa Orysa GRF gi_115471984 ref NM_001066126.1 nucleic acid sequence**

GAGAGCTCCGTATCACCGGCCTCTTTCCCTTCCCTTCCCCTCCGATCCAATCCCCCCTTCTCCTCC
TCGCGGCGCTCGCTGAGCATGGCGGCGGAGGGGGAGGCCAAGAAGGACAGCGCCAGCAACCCTCCC
GGGGGAGGAGGCGGCGGAGGTGGAGGGGAGGAGGAGGAGGATAGCAGCCTGGCTGTCGGGGAGGCG
GCGGTCGGGGTGGGCGAGGCTGGTGGAGGAGGAGGAGGAGGGGAGAAGGCGGATCGAGAGGAGGAG
GAGGGGAAGGAGGATGTGGAGGAGGGCGGCGTGTGTAAGGATCTGGTGCTCGTCGAGGACGCCGTC
CCCGTCGAGGATCCGGAGGAAGCCGCAGCAACTGCAGCACTTCAGGAAGAAATGAAAGCGCTCGTT
GAATCCGTCCCAGTTGGTGCTGGGGCGGCATTCACCGCGATGCAACTACAGGAGCTTGAGCAGCAA
TCTCGTGTCTACCAGTATATGGCTGCCCGTGTGCCTGTGCCTACTCATCTCGTCTTCCCAATATGG
AAGAGTGTTACTGGTGCATCTTCTGAAGGCGCCCAGAAGTACCCGACATTGATGGGGTTGGCAACA
CTCTGCTTGGACTTTGGAAAGAACCCAGAACCAGAACCTGGGAGGTGCCGGCGAACTGATGGAAAG
AAGTGGCGGTGCTGGAGAAATGCAATTGCAAATGAGAAATATTGCGAACGCCATATGCACCGTGGC
CGCAAGCGTCCTGTACAGCTTGTTGTCGAGGATGACGAGCCTGATTCTACCTCAGGGTCGAAACCA
GCATCTGGCAAGGCCACCGAAGGTGGCAAGAAGACTGATGACAAGAGCTCAAGTAGCAAGAAGCTT
GCAGTGGCAGCACCAGCTGCTGTGGAGTCTACATGATTGATGCAGCATTTAGGAGCTGCATAAAGA
GCATAACTGTGCTGGCAATTAGAGTTCGCTTCTTATTGTAATCCTGAAAAGACTGTAGTCTGGTCT
AGCTATAACCTCATCAAGCAAGAAAAGTGTCTGTGGAAAGAAGCCACAAAAACTTTCATTTAGCTG
TCACTGAAATTTTCAGTTTAGGTGTATAGTTTGATTTAGCTTTGCCGTGCCCTCTGCCTTCAGGCA
GATGAGCGGCATTATTGGATAAATCCTCTCTGACTGACAATATCGCATTGTGACTCAAGAAGCCGA
TGGAAGGATCTGCGAGACTAGATACGAAGCTATTTGTTGTGTATCATTTTATATGGCCTGCACAAT
TGTGTGATTTTGTCAGTTGCATAACATGTGGAAGATCCATAATTTTATGCACTATGGAGATTCAAT
TACCTTCCTGAATGTCTGAGCTTCGACATGTTATTGGTTATTGTAACTTAAAAGCAACCTGAGATT
CAATGTGAAAGGGTTTAGATTCCAGCTTC

## FIGURE 10 (continued)

**SEQ ID NO: 54 Oryza sativa Orysa GRF gi_115471984 ref NM_001066126.1_ translated polypeptide sequence**
MAAEGEAKKDSASNPPGGGGGGGGGEEEEDSSLAVGEAAVGVGEAGGGGGGGEKADREEEEGKEDV
EEGGVCKDLVLVEDAVPVEDPEEAAATAALQEEMKALVESVPVGAGAAFTAMQLQELEQQSRVYQY
MAARVPVPTHLVFPIWKSVTGASSEGAQKYPTLMGLATLCLDFGKNPEPEPGRCRRTDGKKWRCWR
NAIANEKYCERHMHRGRKRPVQLVVEDDEPDSTSGSKPASGKATEGGKKTDDKSSSSKKLAVAAPA
AVEST

**SEQ ID NO: 55 Populus tremuloides Poptr_GRF_ lcl_scaff_XIV.39 nucleic acid sequence**
TAGTGAAGCTCCTTCTCATGTCTCACCTCCTGAGACCAAACCAAAGATTCTTGGATCTGTGTTAAG
TAAGCGAGAAAGATCAGCTTCGTCTGCTCAAGATGATTACTGGAGGACTTCAAAGATGCCAAAAAA
TGATGATTTTTCTGTCACCAAAACAATGTCGTTGCACCAACCCACTTCTTTACTGAGATCTAATTA
CATGCTTTCTGATGATTCTCGCCAACAAGAGCACATGATGAGCTTCTCTTCTCCAAGACCAGAAAC
GACTCCATTTCTAAGCAAAGATGGTGAGTTAGTGGAGAGAAGCACACAAAACCACACTGCCTTAAG
CTTTCGTTACCATCAGAACACAGCTTCTTCTTATATTAGAAGTGCAGGTTATGACACCGGAGGCTT
GAATGCAGGCATGCACGGGCCTCTTACTGGGGTTAGAGGACCATTTACTCCATCTCAGTGGATGGA
GCTTGAACATCAGGCCTTGATCTACAAATACATCACTGCTCGTGTGCCTGTGCCTTCTAATTTGAT
CATTCCTCTCAAGAAGTCTGTCTACCCTTATAGCTTACCTGGCTCCTCTACTGGATCCTTCCCTCA
CAATTCATTGGGATGGAGCGCTTTCCATCTTGGTTACCCTGGCAACAACACTGATCCGGAGCCTGG
AAGGTGTCGTCGGACTGATGGGAAGAAATGGCGGTGCTCAAGGGATGCTGTAGCTGACCAAAAGTA
TTGTGAAAGGCACATAAACAGAGGCCGCCATCGTTCAAGAAAGCCTGTGGAAGGCCAGACTGGCCA
TGCTGCCACTGGGACTGCCAGTTCAAAGGTGGTGCCAATGTCGAACTCGATGTCAAAATTGGCAAT
AACCAGTGGTGGTGCCTCCAACAGCATTGCGATGACCACGCAACAACAGTTCAAAATTTTGCAGCC
GGCTGCTGCCAACACTTCTGCAGATGTTGATGTCAACAGAGCACAAGATGCACAGAGCATTTCTAT
GATGTCTTCCACCATCAACCGGAAATCTGATGAGTCCTCTTTCTTTGTTCCTAAACAAGATATCTT
AATGGAGCAGTGCTCTCAAACAGAGTTTGGATTTGTCTCCTCTGACTCTCTCCTCAACCCATCGCA
GAAGAGCTCTTACATTAACTCTAAGCCCTACGAGTCTTTTCTAAACTTTAATGACGAAGAAAGCCA
AGATCAGCATCCCCTTCGTCAATTCATTGATGAGTGGCCGAAGGATCAATCTAATTGTTCTGTCAT
TAGCTGGCCAGAAGAGTTGAAATCTGACTGGACCCAGCTCTCCATGTCAATCCCAATGGCCTCATC
AGACTTCTCATCATCATCATCCTCACCCACACAAGAGAAACTTGCCCTCTCACCAATGAGTTTATC
TTGCGAGTTTGACCCTGTACAAATGGGTTTAAGGGTGAGCGTTGACCATAATGAATCAAGCCAAAA
GCAAACCAACTGGATACCTATCTCCTGGGGGACTTCAATTGGTGGCCCTTTAGGAGAGGTCTTGAC
CACCAGCACTAGCCATGCGGATTCCTGCAAGAGCTCATCAGCCCTTAGCCTTTTGAGAGAAGGTTG
TGATGGCAGCCCACAGTTGGGATCTTCTCCGACGGGAGTCTTGCAGAAATCAACTTTCTGTTCCCT
TTCCAATAGCAGTTCTGGGAGCAGCCCAAGAGCTGAGAGCAAGAAAAACAATGACACTGCTAGTCT
GTATGAGGATGTGGGTGGTTCGATAATTGCAAGTTCATCACCTATTCCACCCCTGTAATCAAGCGA
ACTGTAAGGATGAAACCTGTCAAGGAAATGTGAAGAAGCTTGGAGTTTCTATTTATCTGATAAATT
CCTGTA

**SEQ ID NO: 56 Populus tremuloides Poptr_GRF_ lcl_scaff_XIV.39 translated polypeptide sequence**
MDFGVLGLEGLVGPETSSEAPSHVSPPETKPKILGSVLSKRERSASSAQDDYWRTSKMPKNDDFSV
TKTMSLHQPTSLLRSNYMLSDDSRQQEHMMSFSSPRPETTPFLSKDGELVERSTQNHTALSFRYHQ
NTASSYIRSAGYDTGGLNAGMHGPLTGVRGPFTPSQWMELEHQALIYKYITARVPVPSNLIIPLKK
SVYPYSLPGSSTGSFPHNSLGWSAFHLGYPGNNTDPEPGRCRRTDGKKWRCSRDAVADQKYCERHI
NRGRHRSRKPVEGQTGHAATGTASSKVVPMSNSMSKLAITSGGASNSIAMTTQQQFKILQPAAANT
SADVDVNRAQDAQSISMMSSTINRKSDESSFFVPKQDILMEQCSQTEFGFVSSDSLLNPSQKSSYI

## FIGURE 10 (continued)

NSKPYESFLNFNDEESQDQHPLRQFIDEWPKDQSNCSVISWPEELKSDWTQLSMSIPMASSDFSSS
SSSPTQEKLALSPMSLSCEFDPVQMGLRVSVDHNESSQKQTNWIPISWGTSIGGPLGEVLTTSTSH
ADSCKSSSALSLLREGCDGSPQLGSSPTGVLQKSTFCSLSNSSSGSSPRAESKKNNDTASLYEDVG
GSIIASSSPIPPL

**SEQ ID NO: 57 Populus tremuloides Poptr_GRF_ lcl_scaff_II.1070 nucleic acid sequence**
AAGTAATAGTGGTTTCGCTTCTCTTGCTAGTTCAGATCCTGAAGCAAAGCAGAAGTACGGATCTGG
GTTCCTGAAGCAAGAGAGATCTGCCGCAGCCGATGACGATTGGAGGAACTCTAAATTGGCCAAAAC
CGAGTCAATGCTGCTTGACCAGAGAAACACTTTTCTTCTGAAATCTAGCAACAACTCTCTCTTCAC
TGATGGACAGCAGCAGCAGCAGATGCTCAGCTTCTCCTGTCCCAAATCAGCTTCTTCAGGGGAGAG
AAGCTCCCCAAATGCCATGTTGCCATACTTTCACCTCACATCTTCTGCTTGTAATAGAAATACAGG
CTACAACTCTGGAATCTTCAATGCTGCCAGCATGCATGGGGTTTTGACTGAGACTAGATGGCCATT
CACTCAATTACAATGGATGGAGCTTGAACATCAGGCCTTGATCTACAAATACATGACTGCAAATGT
GCCTATACCATCTAATCTGCTCATCCCCATTAGGAAAGCTCTTGATTCTGCTGGGTTTTCTAGCTT
TTCTGGTGGACTTTTCAAACCCAGTGCATTGCAATGGGGTACTTTCCATATGGGTTTCTCCAGCAA
CACTGATCCGGAGCCAGGACGGTGTCGAAGAACAGATGGGAAGAAATGGCGGTGCTCAAGAGACGC
AGTTGCTGATCAGAAGTATTGTGAGCGGCACATGAACAGGGGTCGCCATCGTTCAAGAAAGCCTGT
GGAAGGACAATCAGGCCATTCCGCTGCGGCCACCACCACTTTAAAGCCAATGGCCAATGGCACTTC
CTCTTTTGCATCAGCATCAGTGGTGGGGCTTCGCAGCGCTGTGTCCGACAGCCACACTATTGTGCA
TAATCAGCAGCAACCTGCCAGTTCTTCTAATCTTTCTGCCACCAATACGCTCAGCAGGGTGTTCCT
CGCTACAGAGAATGTAGGTGAGAGAATGCAAGATGCATCGGGCTTATCCATGCTACCATCCAGCAT
TGACCTGAAATCCAAAGAAACTCCATTCTTCATATCAAAACAACAGAACTCTTACGGTGAATCCCT
GCAAAATGAGTTTGCACTTGTCACCTCCGACTCCCTCCTCAACCATTCACAGAAAAGCTCGTCCTT
GATGAGTTGCAGAAATTTTGGTTCGTCTCAGGACCTTACTGACCAGGAATCTGTTTCACAGCACTC
CCTCCGCCAATTTATGGATGATTGTCCTAAAAGTCATTCTGATCGCTCTGCTGTTGCTTGGCCTGG
ACTTGATCTGCAATCTGAGAGAACCCAGCTATCAATTTCAATCCCCATGGCTCCTGCAGACTTTGT
GTCATCCACTTCATCTTCAAACAATGAAAAGATCTCTCTCTCCCCGCTGAGATTATCGCGTGAATT
TGATCCAATAAAGATGGGGCTGGGAGTGGGAGCCGGTAGTGTCGCCAATGAACCAAACCAAAGGCA
AGCGAATTGGATTCCCATTTCTTGGGAAACTTCAATGGGTGGTCCACTTGGGGAGGTTTTGCACAA
CACCAATAATAATGCAACAGCAGAATGCAAGAATGAATCATCGCTCAACCTAATGACAGAGAGATG
GGACAACAGTCCTCGGGTAGGCTCATCTCCTACCGGGGTCTTACAAAAGTCTGCCTTTGCTTCTCT
TTCAAATAGCAGTGCTGGAAGCAGCCCAAGAGCAGAGAACAAGACCATTGAAGGTGGCAATCTCTG
CAATGACCTTGGATCTACTATCGTGCATTCTTCATCATTGCCTGCCTTGTAACTCTCTGACCTGCC
ATTTAAGAAGTCTTCAGCTGATGCCAGATTATGAATAATTTGTTTTTAAAGTTCTCAATCAGTCT

**SEQ ID NO: 58 Populus tremuloides Poptr_GRF_ lcl_scaff_II.1070 translated polypeptide sequence**
MDFGVQVGLDGLVGSDTSNSGFASLASSDPEAKQKYGSGFLKQERSAAADDDWRNSKLAKTESMLL
DQRNTFLLKSSNNSLFTDGQQQQQMLSFSCPKSASSGERSSPNAMLPYFHLTSSACNRNTGYNSGI
FNAASMHGVLTETRWPFTQLQWMELEHQALIYKYMTANVPIPSNLLIPIRKALDSAGFSSFSGGLF
KPSALQWGTFHMGFSSNTDPEPGRCRRTDGKKWRCSRDAVADQKYCERHMNRGRHRSRKPVEGQSG
HSAAATTTLKPMANGTSSFASASVVGLRSAVSDSHTIVHNQQQPASSSNLSATNTLSRVFLATENV
GERMQDASGLSMLPSSIDLKSKETPFFISKQQNSYGESLQNEFALVTSDSLLNHSQKSSSLMSCRN
FGSSQDLTDQESVSQHSLRQFMDDCPKSHSDRSAVAWPGLDLQSERTQLSISIPMAPADFVSSTSS
SNNEKISLSPLRLSREFDPIKMGLGVGAGSVANEPNQRQANWIPISWETSMGGPLGEVLHNTNNNA
TAECKNESSLNLMTERWDNSPRVGSSPTGVLQKSAFASLSNSSAGSSPRAENKTIEGGNLCNDLGS
TIVHSSSLPAL

**FIGURE 10 (continued)**

**SEQ ID NO: 59 Populus tremuloides Poptr_GRF_ lcl_scaff_I.1018 nucleic acid sequence**
ATGGCAAGAGCTTGAACACCAAGCTCTCATTTACAAATACATGGTCTCTGGTGTTCCTGTCCCGCC
AGAACTCCTCTATTCTGTCAAAAGAAGCTTGGGATCTTCTTTGGCATCAAGACTCTTCCCTCACCA
ACCTATTGGGTGGGGTTGTTTTCAGGCGGGTTTTGGCAGAAAAGCAGACCCAGAGCCAGGAAGGTG
CAGAAGAACGGATGGAAAAAAATGGAGGTGCTCAAAGGAAGCATACCCAGACTCAAAATATTGTGA
GAGGCACATGCACAGAGGCAGAAGCCGTTCAAGAAAGCCTGTGGAACTTACTTCAAGTACTACTAC
AACAGCAACAACAATTCCTTTAACATCAATCAACAGAAACCTCTCTAACCCCACTATTTCACCCTC
CAGCTCCTCTTATTCTTTCTCTCACCCTTCATCTGCGGAATCTGAAGTTTATGCCCATCAAAACCC
TTCGCATGGAACCTTCCTTAACCCCTTCCTTTATCCTCATTCTTCATCTTCTGGACCTCCTGATTC
TGGTTTTTCACCTCTAAATAGCACCCCTCACAACCTGTTTTTGGAGTCTGGATCTTCTCCTCAAGT
TGACAAAGAGCACAGGTATTATCATGGAATGAGGGAGGATGTGGATGAGAGAGCTTTCTTTCCAGA
TGGTTTAGGGAGTGCAAGAGGTGTTCAAGATTCATATAACCAATTGACAATGAGTTCCTACAAAGG
TTACTCACTGTCACAGTTTCAAACCTTTGCTGATACTTCTAAAGAAGAGCAGCAACAACCAGGGCA
GCACTGCTTTGTTTTGGGCACTGATATTATCAAGTCATCAGCAACAAGGTCAATCAAGTTGGAGAA
AGAAACTGAAACCCTGAAGCCATTGCACCATTTCTTTGATGAATGGGAACCAAAGGACGCAGACTC
TTGGCTTGATCTTGCATCCAGTTCAAGACCTCACACTTCTGATGATTGAAGTCTCAATAATGGATC
TTTGTAATATGACGAAAACAGTACTTGTTCGTGGGTCATCAATGCCTTTCTTGCCTCAAATGA

**SEQ ID NO: 60 Populus tremuloides Poptr_GRF_ lcl_scaff_I.1018 translated polypeptide sequence**
MLNTTISRNRFPFTATQWQELEHQALIYKYMVSGVPVPPELLYSVKRSLGSSLASRLFPHQPIGWG
CFQAGFGRKADPEPGRCRRTDGKKWRCSKEAYPDSKYCERHMHRGRSRSRKPVELTSSTTTTATTI
PLTSINRNLSNPTISPSSSSYSFSHPSSAESEVYAHQNPSHGTFLNPFLYPHSSSSGPPDSGFSPL
NSTPHNLFLESGSSPQVDKEHRYYHGMREDVDERAFFPDGLGSARGVQDSYNQLTMSSYKGYSLSQ
FQTFADTSKEEQQQPGQHCFVLGTDIIKSSATRSIKLEKETETLKPLHHFFDEWEPKDADSWLDLA
SSSRPHTSDD

**SEQ ID NO: 61 Populus tremuloides Poptr_GRF_lcl_scaff_28.10 nucleic acid sequence**
TCTTCTTTTCTCTTCCAGGGTAATATGATAATGAGTGGAGGAAACAGGTTTCCCTTCACTGCATCC
CAGTGGCAAGAGCTTGAGCATCAAGCCCTAATCTACAAGTACATGGTTTCAGGCATCCCCATCCCT
CCCGATCTTCTTTTCACCATCAAAAGAAGTGGCTGCTTGGACTCTTCACTCTCTTCAAAGCTCTTT
CCTTGCCAACCTCCACATTTTTCCTGGGGCTGTTTTCAGATGGGTTTGGGAAGGAAAATAGATCCA
GAACCGGGGAGGTGCAGGAGAACTGATGGAAAGAAATGGAGATGCTCAAAAGAAGCATACCCAGAT
TCTAAGTACTGTGAGAAACATATGCATAGAGGGAAGAACCGTTCAAGAAAGCCTGTGGAAGTTGCA
ACACAATCAATAACAGCACCAACTGTCTCATCAATGACCAGAAACCACTCTAATAATTCACTACTA
ACAACATCCCCCACCTCTCTTTCGTTATTGTCACCTAAGACCCACCACCAGAATCACCTTCACTAT
CCTGCTCCTGCAGGTTATCATGCCCATCCAAATCATCAATTCTTGTCTTCTTCCAGACCCCTTGGG
ATTGGTCTGTCCCCTCATGAAAATCCTACTCACTTGCTTTTGGACTCTGGTGGTTCTTCTCTGGCC
AATACAGATTACAGAAGAAACAGGAATGTTTATGGGCTGAAAGAGGAGGTTGATGAGCATGCTTTC
TTCTCAGAACCTTCAGGTTCTATGAGAAGCTTGTCCGGTTCATCTTTGGATGATGCTTGGCAACTC
ACCCCACTCACAATGAACTCTTCTCCTTCTACCACCAACTCTTCAAAGCAAAGGAGCTTGTCTAGT
TTACACAACGAATATTCTTACTTGCAGCTTCAAAGCCTGAGTGATCCCGATACCCCAAAACAACAA
AAGCAGTGTCAACATAACTATCTTCTGGGAAGTAGTGATGTAGACAGTCTAGGGCCCATAAAAATG
GAGAAGGAAAAATCCCAAAAGACTGTTCACCGTTTCTTTGATGAATGGCCACCAAAGGATAAAGAT
TCATGGCTTGATTTGGATGACAAATCATCAAAAAGTGCATCAGTTTCAGCAACCGGACTCTCAATA
TCCATTCCCTCCTCTCATGACTTTCTTCCAATCTTCAGTTCAAGAACTAATAATGGTGGTTGATTT
TACTCTGGTGGGTTTCTGGCCCAAGATGTACTTGGTGGGAAGGGGGGGGTCACCGCCTTCTGTCAA
GAGGCCTCAGA

FIGURE 10 (continued)

**SEQ ID NO: 62 Populus tremuloides Poptr_GRF_lcl_scaff_28.10 translated polypeptide sequence**

MIMSGGNRFPFTASQWQELEHQALIYKYMVSGIPIPPDLLFTIKRSGCLDSSLSSKLFPCQPPHFS
WGCFQMGLGRKIDPEPGRCRRTDGKKWRCSKEAYPDSKYCEKHMHRGKNRSRKPVEVATQSITAPT
VSSMTRNHSNNSLLTTSPTSLSLLSPKTHHQNHLHYPAPAGYHAHPNHQFLSSSRPLGIGLSPHEN
PTHLLLDSGGSSLANTDYRRNRNVYGLKEEVDEHAFFSEPSGSMRSLSGSSLDDAWQLTPLTMNSS
PSTTNSSKQRSLSSLHNEYSYLQLQSLSDPDTPKQQKQCQHNYLLGSSDVDSLGPIKMEKEKSQKT
VHRFFDEWPPKDKDSWLDLDDKSSKSASVSATGLSISIPSSHDFLPIFSSRTNNGG

**SEQ ID NO: 63 Populus tremuloides Poptr_GRF_ lcl_scaff_I.995 nucleic acid sequence**

CTAGGGACTGGACTGCTAAACGGTTGGAACATGGAGGGAAATAGGCGCAATGGTCGGTCTCGGTCA
CCTTCAATTGGGCTAGGAGTTGAGCTTGGACGTGGTGGTTCTAGTCAAAGACCAATAACTGGCTGC
AAAAAACCTTATGGGTTCACTATTCTTCAACTGCATGAGCTAGAACTTCAGTCTCTTATCTACAAG
TATATCCAAGCTGGATTTCCTGTACCTTACCATCTTGTTTTACCTATATGGAAAAGTGTTACTGCT
TCCCTTGGTGGTCTCAGTTCAAGCTTGTACCAGCTCTACCCTAGCTTTATGGGGTGTAAGTGTAAC
CCATTATATTTGGAATATAAGAAAGGAATGGAACATGAGCCAGGGAGATGTAGGAGAACGGATGGA
AAGAAGTGGAGGTGTAGCAAAGAGGTTCTTCCAGATCAAAAGTACTGTGACAGGCACATACACAGA
GGACGCCAGCGTTCAAGAAAGCTTGTGGAAGCTGCTTCTCATAGTAATGCCAGCACCAACCTCTCC
ATTTCTCTCCCTGGAATCGGTAGTGCTAGCGCCTAGCAGTACTAATCTCTCTCCAATATGTTGTCT
CTCTCCAAAGAGTGTTCTCCACAAGAAATATGTAATAGGAGCAGCTGCTA

**SEQ ID NO: 64 Populus tremuloides Poptr_GRF_ lcl_scaff_I.995 translated polypeptide sequence**

MEGNRRNGRSRSPSIGLGVELGRGGSSQRPITGCKKPYGFTILQLHELELQSLIYKYIQAGFPVPY
HLVLPIWKSVTASLGGLSSSLYQLYPSFMGCKCNPLYLEYKKGMEHEPGRCRRTDGKKWRCSKEVL
PDQKYCDRHIHRGRQRSRKLVEAASHSNASTNLSISLPGIGSASA

**SEQ ID NO: 65 Populus tremuloides Poptr_GRF_ lcl_scaff_III.741 nucleic acid sequence**

CAAGTGAAGTTGAAGAGAGAAGTGAAAGAAATGAGCAACTCATCAGTCACAGTGGCGGGGGTGGGA
TCAAGATCACCACCAGGTTTCACGATGTCTCAGTGGCATGAGCTGGAGCATCAAGTTCTTATCTTT
AAGTGTTTAAATGCAGGGTTACCTGTCCCTCCTTCCCTTCTCCTTCCTATTCGTAAGAGTTTTCAG
CTTCTTTCCCCTGGTTTCTTGCACCCATCAAATTTGAGCTACTGTTCCTATTTTGGGAAGAAGATT
GACTCAGAGCCAGGGAGGTGTCGGAGGACAGATGGCAAGAAATGGAGGTGCTCCAAAGATGCTCAC
CCAGACTCCAAGTACTGTGAGCGGCATATGAATAGAAGCCGTAACCGTTCAAGAAAGCCTGTGGAA
TCACAAACTACCTCTCAGTCCTTGTCAACTGTGGCATCAGAAATTGCAACTGGGAGCAGCAGCATT
GGGAGCAGAGGGTATCCAACTAATCCTGGGACCTTAGGTTTGGGAAGTAATATGTCACGTTGGCAG
ATGGAGTCTATGCCTTATGGTGTTAATAGTAAAGACTACAGGTCTCTCCATGGACCGAAGCCTGAA
GCAGATGAGAAAACTTTCCTACCAGAAGCTTTGGGAAATACAAGAAGCTTTGGAATGAACTCTACT
GTGGACAGCACTTGGCATCTCACATCCCAAGTCCCTGCAAACCCTGTGCCAGAATCAAGAAATGGT
TCTCTTTTGCAAAACTACCCACAAGTACAGACACTGCAGGATTTTGAGCCCCTAACTGTTGATGCT
GCATCGCCAAAACAACAGCAGCAGCAGCATTATTTATTTGGAAGGGAGTTCAGTTCATCAGGATCT
ATGAGGCGGGAAAATCAGTCTCTTCAGCCTCTCTTTGACGAGTGGCCAAAATGCAGGGATATGGAT
TCCCATCTCACTGATCAAAGATCTAACAATAACTCGTCTGCTGTTCAGCTATCAATGGCCATTCCA
ATGGCTCCTAACCCTGCTGCGAGGAGTTATCATTCCCCAAATGGTGAGACAGGTTTATCTGGAACA
TACTTTTCCGCAACCTAGACGCCCCCAAGACTTGGTGGAAAACAAATAGATGAGTTCTAATCCTCT
CATGTTATAATGCAGATGCTTGAAAGGAGCAT

**FIGURE 10 (continued)**

**SEQ ID NO: 66 Populus tremuloides Poptr_GRF_ lcl_scaff_III.741 translated polypeptide sequence**

MSNSSVTVAGVGSRSPPGFTMSQWHELEHQVLIFKCLNAGLPVPPSLLLPIRKSFQLLSPGFLHPS
NLSYCSYFGKKIDSEPGRCRRTDGKKWRCSKDAHPDSKYCERHMNRSRNRSRKPVESQTTSQSLST
VASEIATGSSSIGSRGYPTNPGTLGLGSNMSRWQMESMPYGVNSKDYRSLHGPKPEADEKTFLPEA
LGNTRSFGMNSTVDSTWHLTSQVPANPVPESRNGSLLQNYPQVQTLQDFEPLTVDAASPKQQQQQH
YLFGREFSSSGSMRRENQSLQPLFDEWPKCRDMDSHLTDQRSNNNSSAVQLSMAIPMAPNPAARSY
HSPNGETGLSGTYFSAT

**SEQ ID NO: 67 Populus tremuloides Poptr_GRF_ lcl_scaff_VII.1274 nucleic acid sequence**

TAGTCATGCTCCTTCTCATGTCTCACTTCCTGAGACCAAACCAAAGATTCTTGGATCTGTGTTAAC
TAAGCAAGAAAGATCATCTTCATCTGCATCAGCTCAGGATGATTACTGGAGGGCTTCAAAGATGCC
AAAACTTGATGATTTCTCTTCCACCAAAACAATGCCACTGCACCAACCCGCTCCTTTGCTGAGACC
TAATTCTATGTTTTCTAATGATTCTCGCCAACAAGAGCACATGCTAAGCTTCTCTTCTCCAAAACC
AGAAGCTACTCCATTTCTCGTTAAAGATGCTGGCTTGGTTGAGAGAAACACACAAAACCACACTGC
CTTGAGTTTTCCTTACTACCAGCACGCACCTCTTTCCGCTAGCAGAAGTGCAGGTTATGGCACTGG
AAACTTGAATGCAAGCATGCAGGGGCCTTTTACTGGGGTTAGAGGACCATTTACTCCATCTCAGTG
GATGGAGCTTGAACACCAGGCCTTGATCTACAAATACATCACTGCACGTGTGCCTGTGCCTTCCAA
TTTAATCATTCCTCTCAAGAAATCTCTCAACCCTTATGGCTTACCTTTTTCCTCTGCTGGATCATT
CCCTCCCAGTTCATTGGGATGGGGCACTTTCCACCTTGGTTACCCTGGCAACAACACTGATCAGGA
GCCTGGAAGGTGTCGTCGGACTGATGGCAAGAAATGGCGGTGCTCAAGGGATGCTGTAGCTGACCA
AAAATATTGTGAAAGGCACATAAACAGAGGCCGCCATCGTTCAAGAAAGCCTGTGGAAGGCCAGAC
TGGCCATGCTGCTACTGGGACTGCCAGTTCAAAGGTGGTGCCAATGTCTAACTCCATGCCAACCTC
GATTACAACCAGTGGCGCTACCTCGAACAGCATTGTGATCACACAGCAACAGTTAAAAAATTTTCA
GCCGGCTGCTGCTTCCATCTCTTCTGCAGATGCTCGTGTCAACGGAGCACAAGATGCACGGAGGGT
TTCTATGATGTCTTCCACTATCAACCGGAAATCTGACGAGTCTACTTTCTGTATTCCTAGACAAGA
TATCCTATTTGAACAGTGCTCTCAAACAGAGTTTGGACTTGTCTCCTATGATTCTCTCCTCAACCC
ATCGCAGAAGAGCTCTTACTTTAACGCTAAACCCTACGAGTCTTTTCTAAACTTTAGTGATGAAGA
AAGCCATGATCAGCATCCCCTTCGTCAATTCATTGATGACTGGCCGAAGGACCAATCAAATCGTTC
TGTCATTAGCTGGCCAGAAGAGTTGAAATCTGACTGTACCCAGCTCTCAATGTCAATCTCAATGGT
CTCGTCAGACTTCTCGTCGTCATCATCCTCACTTCTGCGAGAGAAACTTGCCTTCTCACCATTGAG
GTTATCTCGCGAGTTTGACCCTATACAAATGGGTTTAAGGGTGAGCGGTGACCATAATGAATCAAG
CCAGAAGCAAGCCAACTGGATACCTATCTCTTGGGGAACTTCAATTGGCGGCCCTTTAGGAGAGGT
CTTGACCACCAGCGCCAGCCATGCGGATTCCTGCAAAAGCTCATCAGCTCTTAACCTTTTAAGAGA
GGGTTGGGATGGCAGCCCGCAGCTGGGATCTTCTCCAACAGGAGTCTTGCAGAAATCGACTTTTGG
TTCACTTTCAAATAGCAGTTCAGGTAGCAGCCCAAGAGCAGAGAGCAAGAAAAACAATGAAAGTGC
TAGTCTGTATGAGGATGTTGTTGGTTCGATAATTGCAAGTGATCCCCTATTCCATCCCTGTAATCA
AGAAAATGGTTAGGATGAAACTTGTGAAGAAGAAGCTTGGAGTTATTTATCTTATTAATTTCTGCA
GACTGTTTCTCCTTGTTGCTTGTTCCC

**SEQ ID NO: 68 Populus tremuloides Poptr_GRF_ lcl_scaff_VII.1274 translated polypeptide sequence**

MPKLDDFSSTKTMPLHQPAPLLRPNSMFSNDSRQQEHMLSFSSPKPEATPFLVKDAGLVERNTQNH
TALSFPYYQHAPLSASRSAGYGTGNLNASMQGPFTGVRGPFTPSQWMELEHQALIYKYITARVPVP
SNLIIPLKKSLNPYGLPFSSAGSFPPSSLGWGTFHLGYPGNNTDQEPGRCRRTDGKKWRCSRDAVA
DQKYCERHINRGRHRSRKPVEGQTGHAATGTASSKVVPMSNSMPTSITTSGATSNSIVITQQQLKN
FQPAAASISSADARVNGAQDARRVSMMSSTINRKSDESTFCIPRQDILFEQCSQTEFGLVSYDSLL

**FIGURE 10 (continued)**

NPSQKSSYFNAKPYESFLNFSDEESHDQHPLRQFIDDWPKDQSNRSVISWPEELKSDCTQLSMSIS
MVSSDFSSSSSSLLREKLAFSPLRLSREFDPIQMGLRVSGDHNESSQKQANWIPISWGTSIGGPLG
EVLTTSASHADSCKSSSALNLLREGWDGSPQLGSSPTGVLQKSTFGSLSNSSSGSSPRAESKKNNE
SASLYEDVVGSIIASDPLFHPCNQENG

**SEQ ID NO: 69 Populus tremuloides Poptr_GRF_ lcl_scaff_XII.277 nucleic acid sequence**
AAAAATTATTCTTCTTTATTTTTCATCATGATGACAACAGATGATGGCTTAAACGTTTCAAACAAG
GTAGCTAAGGAAATAAACACTACTAGTAGTATTAGTAATGTTGATTTTGGTGTGAAGCTACATCAA
CCTATTGATCATCATCAATCATTTCCTTCTAGTACTCCTATGATGGTTCCTCATGTTAATCACCAC
CGTCCAATGTTTGACAATGGTCCCACATCATCATGTGATAGAAACAAGTCTTTGATGAACTATATA
AGTGATCGTATATACCGTGTTGCTGCTGGTGGTGCTACCAGTGGCGGTGCAGTTGGGGTTAGGAAT
TTGCAGCCTTTTGACATTTCTGAAACAAGTATCTCTACAGCAGCTTCTGCCTTCAGATCCCCAGGA
GGCAACATGGCAGCGTCTTTGGGGTTTCCTTTTACAAATACACAGTGGAAAGAGCTTGAAAGACAA
GCCATGATATACAACTATATAACGGCCTCAGTCCCTGTGCCTCCTCAATTTCTAATTCCAACCCCA
ATGGGGAATGGATTGAATGTAAGGTTCTCAAATGGGGCAGATCTAGAACCAGGGAGGTGTAGGAGA
ACAGATGGGAAGAAATGGAGGTGCTCAAGAGATGTGGCACCTGATCAGAAATACTGTGAGCGTCAT
ATGCATAGAGGCAGACCCCGTTCAAGAAAGCATGTGGAACTCAATGCTAGCAACAATAACAACAAG
AAGAACCGCCATAATCCTGCTATTTGTCCAGAAGCTCCTGTTACCGTGGCCATTTCTAAACCCACA
ATCAACAACAGCAACAGTGGCTCTGCCTCTCACGATCAGTTTTTTGGGCCTATGCCTCAGCCATAT
ATCCAGACCCCAGTTTTTGTAAACAAAACCAGCGAGAAGACTTCAACTTATGATGTTAATGGAGCC
TATGGTTCCACATTCAAAGAACCCAGGAGCTTGGACTGGATGTTGAAAGGGGAAGCTGGTCCTATA
GCCAAAAATGATCAACAATGGCCACATCTAGTGCACAAAGAAATTGAACTAGCTACTGAAGGTTCC
TTTAACAGTGCTTCTGTTCTCAAACAGCATTACCAAGGAGAGTCTTTGAATTTGAACTCATTTGGA
AATTTTAATGCTAGAGAAGACCAACAAAGCAATCAATATAGTCTGTTTCTTGATGAGGCTCCAAGG
AGTTTTATTGATGCATGGTCTAATGATGCAATTTCTAGAAACACAAGTTCTGTTTCCTCAGATGGG
AAGCTCCATCTTTCCCCTCTCAGTCTATCAATGGGAAGCAATAGGTCTACTGATGATGAAATGGGT
CAGATCCAAATGGGTTTAGGCCTAATCAAATCAGATCGAAATGAAGAATGTGGTAACACTAGCAGC
GCCCCAGGTGGCCCCTTGGCAGAGGTGTTACAACTGAGGACAAGCAACACCACAGGAACCAATCAA
TCTTCTTCTATGATGGAAAATGGTGATTCTATTAGTCCTCCAGCTACTACAGTCTCTTCTCCATCT
GGGGTTTTGCAGAAAACACTTGCCTCATTTTCTGATAGCAGTGGTAATAGCAGTCCAACTCTTGCC
AGTTCAAGGACCAAACCTGAAATTGCCATGCTTTGGTTAAATCAAGGCTAAATGTGCCACTCTCTA
GTTAGTTAAGGGCACACAAGGCCATAAGGGCAATATAAATTTTTATAAGCTTGATATATTTTTAT

**SEQ ID NO: 70 Populus tremuloides Poptr_GRF_ lcl_scaff_XII.277 translated polypeptide sequence**
MMTTDDGLNVSNKVAKEINTTSSISNVDFGVKLHQPIDHHQSFPSSTPMMVPHVNHHRPMFDNGPT
SSCDRNKSLMNYISDRIYRVAAGGATSGGAVGVRNLQPFDISETSISTAASAFRSPGGNMAASLGF
PFTNTQWKELERQAMIYNYITASVPVPPQFLIPTPMGNGLNVRFSNGADLEPGRCRRTDGKKWRCS
RDVAPDQKYCERHMHRGRPRSRKHVELNASNNNNKKNRHNPAICPEAPVTVAISKPTINNSNSGSA
SHDQFFGPMPQPYIQTPVFVNKTSEKTSTYDVNGAYGSTFKEPRSLDWMLKGEAGPIAKNDQQWPH
LVHKEIELATEGSFNSASVLKQHYQGESLNLNSFGNFNAREDQQSNQYSLFLDEAPRSFIDAWSND
AISRNTSSVSSDGKLHLSPLSLSMGSNRSTDDEMGQIQMGLGLIKSDRNEECGNTSSAPGGPLAEV
LQLRTSNTTGTNQSSSMMENGDSISPPATTVSSPSGVLQKTLASFSDSSGNSSPTLASSRTKPEIA
MLWLNQG

## FIGURE 10 (continued)

**SEQ ID NO: 71 Populus tremuloides Poptr_GRF_ scaff_XIII.769 nucleic acid sequence**

AGCAGCGGGGATGGCAGCTGGGGGAATGGGGACAGCAGCGATGACAATGAGGTCACCATTTACAGT
GTCACAGTGGCAAGAACTGGAACATCAAGCTTTGATCTATAAGTACATGGTGGCAGGTCTGCCTGT
TCCACCTGATCTTGTGCTCCCTATTCAGAGGAGCTTTGAATCCATTTCTCATAGATTCTTCCACCA
TCCCACCATGAGCTATTGCACTTTCTATGGCAAGAAGGTGGATCCGGAACCAGGTCGATGCAGGAG
GACCGACGGCAAGAAGTGGAGGTGCTCCAAAGATGCCTACCCAGACTCCAAGTACTGTGAGCGCCA
CATGCACCGTGGCCGCAACCGTTCAAGAAAGCCTGTGGAATCACAAACCATGACACAGTCATCGTC
CACCGTGACATCACTGACTGTTACAGGAAGCAGCAGTGGAACTGGAAGCTTCCAGAACCTTCCATT
GCACACATATAGCAATCCCCAGGGCACTGCTTCTGGAACTAACCAATCATATTATCATATGAACTC
CATTCCCTACGGAATCCCAACCAAAGATTACAGGTATCTTCAAGAACTTACGCCTGAAGGTGGGGA
GCATAGCTTCTTGTCTGAAGCCTCAGGAAGCAACAAGGGGCTTCAGATAGACTCACAGCTGGACAA
TGCATGGTCTTTGATGCAATCCAGAGTCTCATCATTCCCCACAGAGAAATCAACTGAAAACTCGAT
GTTGCAAAGTAATCATCCCCAGCATTCATTTTTCAGTAGTGATTTCACCACCAGGGAATCTGTGAA
ACAGGACGGGCAGTCTCTTCGACCCTTCTTTGATGAGTGGCCTAAAAACCGAGATGCCTGGTCTGG
CCTCGAGAATGATAGTTCCAACCAGACCTCATTCTCTACAACGCAGCTGTCGATATCCATTCCAAT
GGCCTCATCTGACTTCTCCACAAGTTGTCGTTCTCCACGAGATAACTAAGAGGACACTAAGAATTC
ACAAAACAAAGCCAAAGGTGGTCCTGGCCAAAGATTAACACATACTTGTGTTAAATTTTTGTG

**SEQ ID NO: 72 Populus tremuloides Poptr_GRF_ scaff_XIII.769 translated polypeptide sequence**

MAAGGMGTAAMTMRSPFTVSQWQELEHQALIYKYMVAGLPVPPDLVLPIQRSFESISHRFFHHPTM
SYCTFYGKKVDPEPGRCRRTDGKKWRCSKDAYPDSKYCERHMHRGRNRSRKPVESQTMTQSSSTVT
SLTVTGSSSGTGSFQNLPLHTYSNPQGTASGTNQSYYHMNSIPYGIPTKDYRYLQELTPEGGEHSF
LSEASGSNKGLQIDSQLDNAWSLMQSRVSSFPTEKSTENSMLQSNHPQHSFFSSDFTTRESVKQDG
QSLRPFFDEWPKNRDAWSGLENDSSNQTSFSTTQLSISIPMASSDFSTSCRSPRDN

**SEQ ID NO: 73 Populus tremuloides Poptr_GRF_ lcl_scaff_XIV.174 nucleic acid sequence**

GTCTGTTCTTGTTTTTGTAGATACACGACAATGGAGAAAAGAGTATCTGAAGAATCAGCGCCGTCG
ATGAAACTGTCTCTTGGGATTGGTGCTGGTGATCATGGTGATGATGATCAAGATGATAGACACGTG
TTCCCACAGTTAACAGAGACTCAGTTGCATGAGCTTAAACAGCAAGCTTTGATATTCAAGTACATA
GTAGCTGGTCTTCGCGTGCCTCCTGATCTTGTAGTTCCCATTTGGCATAGTGTTGCTAGCAGCTCT
CTTGGTTCATTTAGTGGTGCTGATATTTATAGGCAATTCCCAAGCTTTGTGGGATTAAGTCCTCAG
GGATTTGATTATAGACAAATGATGGATCCAGAACCTGGGAGATGTAGAAGAACTGATGGGAAGAAA
TGGAGGTGTAGCAAGGATGTAGTTGCTGGTCAGAAGTATTGTGAACGCCATATGCATAGAGGCCGT
CAACGTTCAAGAAAGCTTGTGGAAGCTTCTCAAACTGCTGCTGCTTCTGAAAAACCATCACCTCAC
AATTCAAGCAAGAATTCAGACAATCCAACCACTCATTCTTCTAATTTAGCCAAAGTAAGTTCACAG
ATAAAAGCCCCACCTCTTAATAATACCCCCACCATTTTAACCACTTGCACCACAAGTTGCAATTCT
GACATTGAAATCACTGGCATGAGCTTGGCCACTACTGCTAATTCTGACTGCAAAAACCCCTTTACA
ACCATGACTACTAGCATTGTTACCGGCTACAAGAACACTGCAACAATGATCGCTAGTGCTGTTCAT
GCTGACATTACAGCCACTGGTAACGACTACAAGAGTAGCATCAACTTAAAGAGACACTACATTGAT
GACAGAAACAGTAATTGCAGCAACTCTGTTACTTACAAGGGTATAATCGACAGAAACTGCAGCAAC
AAAAAAATAAAAAATGCTGGTAGCAATGTATCTCAAGGATTGAACTTCTCCCCGAAGAGTGTTCTT
CAAGTTCAAGGTTGCGGCGCCTCACACATTTACATGAATGATGTGGAACTTGAACTGGGAAGGTGT
AGAAGAACAGATGGAAAGAAGTGGCGATGCCGCAGGGATGTTGTAGCTAATCAGAAGTATTGCGAG
ATGCACATGCACCGAGGTTCTAAGCAGCACTTGGAAGCGTCCAAACCTGCTGCAATTCCCGCTACA
ATCCCATTTGTCCCTGGGAATGTTCATTCATATCCTGCAACGAACTTGCCAAGTAAAGCAGATCGC

**FIGURE 10 (continued)**

AGAAGCTTAAACACCGATCTCTGTATTTCGATCCCAACAAGTCCTCAACTGATCATGACCAATGAT
GATACAAGAACTATCAGCAATAGCAGTGACACTACCATAAGCGACACCATGAGGGGCACCACACCA
GGACTGGTATCAACACATGCAGGTAGTGGGGATCCCAAAAGTCCTCCTGGTGGTGACAGAGCCAGG
TTATTAAAGAAAGCTGAAGTTATGGAGGCGGCGGTAGAGATTTACGAGGAAGTACAATCCGGCCGT
GGGGTCAGAAGTTTTATCCAAGGCAGAAGACTTCCAACTTTTCCCTATGTTTATATCACACCTCTG
CTGTTAATCTGCAATTAATTCTAGTCAAACCATGTGATTAAAGTTACAAGAGGTGTAAGAGAAGAA
TAACGTTTAATTCCATCCTATGCAGACAACTG

**SEQ ID NO: 74 Populus tremuloides Poptr_GRF_ lcl_scaff_XIV.174 translated polypeptide sequence**
MEKRVSEESAPSMKLSLGIGAGDHGDDDQDDRHVFPQLTETQLHELKQQALIFKYIVAGLRVPPDL
VVPIWHSVASSSLGSFSGADIYRQFPSFVGLSPQGFDYRQMMDPEPGRCRRTDGKKWRCSKDVVAG
QKYCERHMHRGRQRSRKLVEASQTAAASEKPSPHNSSKNSDNPTTHSSNLAKVSSQIKAPPLNNTP
TILTTCTTSCNSDIEITGMSLATTANSDCKNPFTTMTTSIVTGYKNTATMIASAVHADITATGNDY
KSSINLKRHYIDDRNSNCSNSVTYKGIIDRNCSNKKIKNAGSNVSQGLNFSPKSVLQVQGCGASHI
YMNDVELELGRCRRTDGKKWRCRRDVVANQKYCEMHMHRGSKQHLEASKPAAIPATIPFVPGNVHS
YPATNLPSKADRRSLNTDLCISIPTSPQLIMTNDDTRTISNSSDTTISDTMRGTTPGLVSTHAGSG
DPKSPPGGDRARLLKKAEVMEAAVEIYEEVQSGRGVRSFIQGRRLPTFPYVYITPLLLICN

**SEQ ID NO: 75 Populus tremuloides Poptr_GRF_ lcl_scaff_XIV.51 nucleic acid sequence**
AAGTAATAGTGGTTTTGCTTCTCTTGCTGGCTCAGATCCTGAAGCAAAGCAGAAGTTGTACGGATC
TGGGTTCTTGAAGCAAGAGAGACCTGGCAACATCGATGGTGATTGGAGGAGCTCTAAATTGTCGAA
AACTGAGTCAATGCTGCTTGAGCAGAGTAACACTTCACTTCTGAAATCTAGCTCCAACTTTCTCTT
CGCTGATGGACAGCAGCAGCAGCAGCAGATGCTCAGCTTCTCCTATCCCAGATCAGCTCCTTCAGC
GGAGAGAAGCTCCCAAAATGGCACATTGCCCTCTGGTATCTACAATGCTGCCAGCATGCATGGGGT
TTTGACCGAGACCAGATGGCCATTCACTCAATCACAATGGATGGAGCTTGAACATCAGGCCTTGAT
CTACAAGTATATAGCAGCAAATGTGCCTATACCATCTAATCTGCTCCTTCCCATTAGAAAAGCTCT
TGATTCTGCTGGGTTTCCTAGCTTTTCTGCTGGATTTTTCAGGCCCAATACATTGCCATGGGGTGC
TTTCCATATGGGTTTCTCCAGCAACACTGATCCGGAGCCAGGACGGTGTCGAAGGACAGATGGAAA
GAAATGGCGGTGCTCAAGAGATGCAGTTGCCGATCAGAAGTATTGTGAGCGGCATATGAACAGGGG
CCGCCATCGTTCAAGAAAGCCTGTGGAAGGCCAATCAGGCCATTCCGCTGCAGCCGCCACCACTGT
AAAGCCAGCCAATGGCACTTCGTCTTCTACATCATCATCAGTGGTGGGGCTTCGCAGCACTGTGTC
CGACAGCCTCACTATTGCTCATAATCAGCAGCAAGCAGCTAGTCCATCTAATCTTTCTGCCTCTAA
TACGCTCAGCAGGATGTTCCTTACTAAAGAGAATGTAGGTGAGAGAACGCAGGATGCGACAGCCTT
GTCCATGCTTCGATCCAACATGGATCTTAAATCTAAAGAAACTCCATTCTTCATATCAAAACAACA
AAACTCATATGGGGAATCCTTACGAAATGAGTTTGGACTTGTCACCTCCGACTCCCTCCTCAATCA
CTCACAGAAAAGCTCATCTTTAATGAGTTGCAGAAATTTTGGTTCGTCTCAGGACCTCACTGACCA
GGAATCTGTTTCACAGCACTCCCTCCGCCAATTCATGGATGATTGGCCTAAAAGTCAGTCTGATCG
TTCTGCTGTTTCTTGGCCTGAACTTGATCAGCAATCTGAGAGAACCCAGCTATCGATTTCAATCCC
CATGGCTCCTGCAGACTTCATGTCATCTACTTCCTCCCCAAACAATGAAAAAGTCACTCTCTCCCC
ATTGAGATTATCACGAGAATTTGATCCAATACAGATGGGACTGGGAGTGGGAGGTGGAGGTGGTGG
TATTGCCAACGAACCAAACCAAAGGCAAGCCAACTGGATTCCCATTTCTTGGGGAACTTCAATGGG
TGGTCCGCTCGGGGAGGTCTTGCACAACACCAATAACAATGCAGCAGCAGAGTGCAAGACCACGTC
ATCGCTGAACCTGATGACCTATAGATGGGACAACAGTCCTCGTATAGGTTCATCTCCAACTGGGGT
CTTACAAAAGTCAGCGTTTGCTTCCCTTTCAAATAGCAGTGCGGGAAGCAGCCCAAGAGCAGAGAA
CAAGACCAATGAAGGTGGCAGTCTCTGCAATGACCTCCTTGGATCCACTATTGTGCATTCTTCTTC
ATTGCCTGCCATGTAACTCTGTTGATCTGCTGCCATCCAAGAAGTCTCCTGTCATCGTAGCTGACA
AAACATGGAGCACTTTGTTTTTGAAGTTGT

**FIGURE 10 (continued)**

**SEQ ID NO: 76 Populus tremuloides Poptr_GRF_ lcl_scaff_XIV.51 translated polypeptide sequence**

MLLEQSNTSLLKSSSNFLFADGQQQQQQMLSFSYPRSAPSAERSSQNGTLPSGIYNAASMHGVLTE
TRWPFTQSQWMELEHQALIYKYIAANVPIPSNLLLPIRKALDSAGFPSFSAGFFRPNTLPWGAFHM
GFSSNTDPEPGRCRRTDGKKWRCSRDAVADQKYCERHMNRGRHRSRKPVEGQSGHSAAAATTVKPA
NGTSSSTSSSVVGLRSTVSDSLTIAHNQQQAASPSNLSASNTLSRMFLTKENVGERTQDATALSML
RSNMDLKSKETPFFISKQQNSYGESLRNEFGLVTSDSLLNHSQKSSSLMSCRNFGSSQDLTDQESV
SQHSLRQFMDDWPKSQSDRSAVSWPELDQQSERTQLSISIPMAPADFMSSTSSPNNEKVTLSPLRL
SREFDPIQMGLGVGGGGGGIANEPNQRQANWIPISWGTSMGGPLGEVLHNTNNNAAAECKTTSSLN
LMTYRWDNSPRIGSSPTGVLQKSAFASLSNSSAGSSPRAENKTNEGGSLCNDLLGSTIVHSSSLPA
M

**SEQ ID NO: 77 Populus tremuloides Poptr_GRF_ lcl_scaff_XIX.480 nucleic acid sequence**

AGTGATGACCATGAGGTCGCCATTTACAGTATCGCAATGGCAAGAACTGGAACATCAAGCTTTGAT
CTATAAGTACATGGTGGCAGGTCTGCCTGTTCCTCCTGATCTTGTGCTCCCTATTCAGAGGAGTTT
TGAGTCCATTTCTCATAGATTCTTCCACCATCCCGCCATGGGCTATTGCACTTTCTATGGGAAGAA
GGTGGATCCGGAGCCAGGTCAATGCAGGAGGACCGACGGCAAGAAGTGGAGGTGCTCCAAAGATGC
ATACCCGGGCTCCAAGTACTGTGAGCGCCACATGCACCGTGGCCGCAACCGTTCAAGAAAGGCTGT
GGAATCACAAACCATGACACAGTCATCGTCCACTGTGACATCACTGACTGTAACAGGAAGCAGCAG
TGGAACAGGGAGCTTCCAGAACCTTCCACTGCGCACATATGGTAATCCCCAGGGCACTGGTTCTGG
ACCTAACCAATCCCATTATCATATGAACTGCATTCCCCGTGGAATCCCAACTAAAGATTGCAGGTA
TCTTCAAGGACTGAAGACTGAGGGTGGCGAGCATAGCTTCTTGTCTGAACCTTCAGGATGCAAAAG
GGGTCTCCAGAAGGACTCACAGCTAGACAATGCCTGGTCTTTGATGCTATCCAGAGGCTCATCATT
CCCCACAGAGAAATCGACTGACGACTCGACGTTGAAGAATGATTATCCCCAGCATTCATTTTTCAG
TAGTGATTTCACCACCGGAGAACCCGTGAAACACGAAGGGCAGTCTCTTCGACCCTTCTTTGACGA
GTGGCCTGAGGACCAGGACATTTGGTCTGGCCTCAAAGATAATAGATCCAACTCCACCTCATTCTC
TACAACGAAGCTGTCGATGTCCATTCCAATTGCCTCATCTGGCTTCTCCACAAGTTCTCGTTCTCC
ACAAGAAAACTGAGAGGACAGAATGAGAATTTACAAAGCACGATCAAAGGTGATCCTCAACCAAAG
ATTGGTGTGTTAACTTGAAAACTCCTA

**SEQ ID NO: 78 Populus tremuloides Poptr_GRF_ lcl_scaff_XIX.480 translated polypeptide sequence**

MTMRSPFTVSQWQELEHQALIYKYMVAGLPVPPDLVLPIQRSFESISHRFFHHPAMGYCTFYGKKV
DPEPGQCRRTDGKKWRCSKDAYPGSKYCERHMHRGRNRSRKAVESQTMTQSSSTVTSLTVTGSSSG
TGSFQNLPLRTYGNPQGTGSGPNQSHYHMNCIPRGIPTKDCRYLQGLKTEGGEHSFLSEPSGCKRG
LQKDSQLDNAWSLMLSRGSSFPTEKSTDDSTLKNDYPQHSFFSSDFTTGEPVKHEGQSLRPFFDEW
PEDQDIWSGLKDNRSNSTSFSTTKLSMSIPIASSGFSTSSRSPQEN

**SEQ ID NO: 79 Populus tremuloides Poptr_GRF_ lcl_scaff_28.309 nucleic acid sequence**

ACAACCCTCTGCAAAGATGCCAAAACTCCTCATGGATCCCCATCAACCACAACAACATCCACACTC
ATCTGGGTCTGCTGCCTTCCCTTTGTTTCTACCCGAGCCCAGCTGCAAAAATAGTAACCTGTCAGC
ATTTCCTGATTCAAACACAGCTGCAAACACCAGACTTCCTAAGATCATGGGGAATTACTTTAGCCT
GGAACAGTGGCAAGAGCTAGAGCTGCAGGCTTTGATCTACAGATTCATGTTAGCCGGTGCAGCTAT
TCCTCCGGAGCTCCTCCAACCAATCAAGAAAACCCTTCTTCATTCTCACCCCCCTCCATATTTCCT
CCATCATCCTCTTCAATTACATTGCTCTTATTATCAGCCATCTTGGTATTGGGGAAGAGCAGCCAT
GGATCCGGAGCCAGGTCGGTGCCGGAGAACAGATGGGAAGAAATGGCGGTGCTCCAGAGACGTGGT

## FIGURE 10 (continued)

GGCAGGGCACAAGTATTGCGAGCGCCACTTGCACCGTGGCCGCAACCGTTCAAGAAAGCCTGTGGA
AAATCCCACACCTACAATATCCACTAACATCACTTGCATTGGTATTGGAGGTGCGGGTGGTACCGC
ATCAGCTGCTGCTTTCAATTGCAGCACCACACCAACCATATCAGAGGTGGTCAATGAGACTCATTT
TTCGCATACACTAGAATCCCCTTCCATTCATCTCAATCATAGCTCCAAAACTGAAAGCAAGGGCTT
AATTGGACCACCACCTCCAAATGAGGTTGGTAACAGGTCTGATGGCCACATTCTGTGGCATTTTTT
TGATGACTGGCCACGATCCGTTGATGAATCCGACAATATGAATGCTGGAAGCTCAATGAACTCTTT
AACCTGCCTCTCCGTTTCAATGCCTGGAAACTCACCAGCATCAGATGTGTCATTGAAATTGTCCAC
TGGGAATAATATTGCAGAGGAGGAGCCGGAGCCAGTCCCAGCCCCGATCCCTAGAGGCAATACAAG
CAATTGGGCTGCTGCAGGATGGGGCACAAAAATTACAAACCAGGTGGTGACTTCAATGGGGGGACC
TCTTGCTGAGGCGCTGAGGTCCTCCACTACCAAACTCATCTCCCACGAATGTTCTGCACCAGTTAT
GTCGCCCCACTGTTTCTGAAACTTGATCTATTTTAGGTTAGTTTGTGGTGTAGTAACACATGCATG
CATACACACACACACACACACACACACACACATCA

**SEQ ID NO: 80 Populus tremuloides Poptr_GRF_ lcl_scaff_28.309 translated polypeptide sequence**

MDFHLKQWRNQHEESGQQPSAKMPKLLMDPHQPQQHPHSSGSAAFPLFLPEPSCKNSNLSAFPDSN
TAANTRLPKIMGNYFSLEQWQELELQALIYRFMLAGAAIPPELLQPIKKTLLHSHPPPYFLHHPLQ
LHCSYYQPSWYWGRAAMDPEPGRCRRTDGKKWRCSRDVVAGHKYCERHLHRGRNRSRKPVENPTPT
ISTNITCIGIGELDQTTFSLFCFCFNLLAHPYCSSKTESKGLIGPPPPNEVGNRSDGHILWHFFDD
WPRSVDESDNMNAGSSMNSLTCLSVSMPGNSPASDVSLKLSTGNNIAEEEPEPVPAPIPRGNTSNW
AAAGWGTKITNQVVTSMGGPLAEALRSSTTKLISHECSAPVMSPHCF

**SEQ ID NO: 81 Populus tremuloides Poptr_GRF_ lcl_scaff_I.688 nucleic acid sequence**

GCGCTCGCTTGTGTTGTGGTGACAGGAGAAATGACACCATCTGTGAATGAGAGAGTGCTTTTTACA
GCTGCTCAGTGGCAAGAACTTGAAAGACAAACCACGATTTACAAGTACATGATGGCTTCTGTTCCT
GTCCCTCCTGAACTCCTTATACCCATCACCAAAAATCAATCAAATGTCCTTCCTCCACGGTCTAAC
AGTTCACTAGAACTGGGAATTCCTAGCCTGAACTCATCAGATGCAGAACCATGGAGATGCAAAAGA
ACTGATGGGAAAAAATGGAGGTGTTCAAGAGATGTGGCACCTGACCAGAAATACTGTGAGAGGCAC
TCTCATAAGAGCCGTCCCCGTTCAAGAAAGCCTGTGGAATTACACACTCATGACTCCCCGAGGACA
TTGACCAACAATAACACTAACACCAACAATAGCAATTACTCCACTAATCCACACCTGTTTAATCAA
AAACCTTACTTTCCAAGCCATTTATTTATGTTTCCTAGTGCCATGGCCCCTTCTGCCAGCTCATAT
GATCAACCCAGGAGCTTGGAATGGCTCTTGAAAGGCGAGATTTTACCCGTTGCCAGTAATTACAGC
CAAGAATGGCAGCATTTGAAGAGAGACAGCATCAAGGGTAATGGCAAAGTGTACAACGTTTATGGA
GAAGAGCAGCCGCTTTGCTCAAATACATATAGAGGTGGCCATTCATTACAAGCTCAGAGGCTAAAT
GATCATTGCAGCGTGTTATCAAGTCCCAAATCAACTACTTTGGAAAGGGCTTTAAGTCCTAGCCTG
ACCCAAGAACAAGAGACAAGGCACTTCATTGATGCTTGGTCAACTAATTCAGGGAGAGACGACATT
GGTGGGATTGGTAAAAAAAGTTACGTTTCTTCAAGTGAGAAGTTAGTATTGCCACATTCAGCTCTT
ACATTGTCAATGTCACCTGGCACTGGAAGTGAAACTAATAATGAAGGAAATGGGAGTGCTCAACTG
AGTAGTTTTGGGATCATGGGATTATCAGATAGAGATCATCAGAGTGCGAGTGGCTTGAGACCTCAG
TGGATGATGAGTCATGGTGGTTCATGGATAGTATCACCACCTGGTGGACCATTAGCTGAAGCCTTG
TGTCTTGGCATTTCCAGCAATGCAAAAACTGCTTCCAATTTACCATCCCCTTGCAGCAGTAGCTGT
GGCCCCAATTAATGTCAACAAAAACCAACCGCTATAGTTTGTTGTAAATTCTGGCTGGGTAGAGGC
CAGAGGGTAGAAGCTAATGTGGCCCA

FIGURE 10 (continued)

**SEQ ID NO: 82 Populus tremuloides Poptr_GRF_ lcl_scaff_I.688 translated polypeptide sequence**

MRSSWSRTRSGVFVDDIGLGLRMQDNLESCSGSSKRSVTAMSCDHEPAAHELSSSSCSGGGGGSGP
LFYSTSNHVTCLGDIKDVVASVSASGTGTPDAIAESKSLQYPYFISDSSPFTFNSSGEMTPSVNER
VLFTAAQWQELERQTTIYKYMMASVPVPPELLIPITKNQSNVLPPRSNSSLELGIPSLNSSDAEPW
RCKRTDGKKWRCSRDVAPDQKYCERHSHKSRPRSRKPVELHTHDSPRTLTNNNTNTNNSNYSTNPH
LFNQKPYFPSHLFMFPSAMAPSASSYDQPRSLEWLLKGEILPVASNYSQEWQHLKRDSIKGNGKVY
NVYGEEQPLCSNTYRGGHSLQAQRLNDHCSVLSSPKSTTLERALSPSLTQEQETRHFIDAWSTNSG
RDDIGGIGKKSYVSSSEKLVLPHSALTLSMSPGTGSETNNEGNGSAQLSSFGIMGLSDRDHQSASG
LRPQWMMSHGGSWIVSPPGGPLAEALCLGISSNAKTASNLPSPCSSSCGPN

**SEQ ID NO: 83 Saccharum officinarum Sacof_GRF nucleic acid sequence contig of CA084837.1, CA238919.1,CA122516.1**

CCAGCATCCACTCTCTCATCAGCAGCCTCTTCTTCTTCTCCCCCAAATGAGTGCTGAGTTTTGTGC
TGCTGCGGGCATGGAGCTCGGAGTCGGGGATGTGATGGGGCTGCAGCAAGGCATCGCCATCACCGC
GCCATCGCCCAGGGGCAGCGGCGACCTGGGTCTTCTCAAGCGAGCAGCCCTCACCCAGGCAGCAGC
TGGCCCCTACCCCTCCCCCTTCCTCGACGAACAGAAGATGCTCAGGTTCTCCAAGGCGGCTCACAC
ATTGCCCTCAGGGCTAGGCTTGGATTTTGGAGGCCCAAGCGAGCAGGCTTTCCTGCTGTCCAGGAC
CAAGAGGCCATTTACTCCCTCGCAGTGGATGGAGCTGGAGCACCAGGCTCTGATATACAAGTATCT
CAATGCAAAGGCCCCCATACCTTCCAGCCTGCTCATTTCAATCAGCAAGAGCTTCAGATCATCCAA
TAGAGTGAGCTGGAGGCCTCTCTATCAAGGCTACACAAATGCAGACTCTGACCCAGAGCCTGGAAG
ATGCCGACGAACAGATGGAAAGAAGTGGCGATGCTCCAAGGAGGCAATGGCTGATCACAAGTACTG
TGAGCGGCACATCAACAGAAACCGTCACCGTTCAAGAAAGCCTGTGGAAAACCAACCCAAAAAGAC
CACCAAGGAGGTGCCTGCTGCTGCTAGCTCATTGCCATGTGCTGGGCCACAAGGTTGCTTGAAGAA
GGCAAAAGTTAATGACTCCAAGCCAGGCACTGTCAGCTGTTGGACAGATAGTTTAAACAGGACAAT
GTTGAGCAGAGAGAAAGCAAACAAACCGACGGAGGACAACTCTTTGCTGCTTAATTCTACGAATAG
CCAGCCCACCTTGTCCCTGCTCTCTCAACTGAAGCAGCAGAACAAACCAGATAAGTTAGGTCCCAC
ACTGGAAAATGAGTCAAACTCAGACACAATACTGAAAGCCTGGGGTGGCAACCAGCCTAGCCACAA
GAGCATTTCCTCCACACAGCACCATGATGCTGAATCCCTCCAATCAGTCCTTCAAAATTTCAGCCT
AGCCCAGAATGAGAAGATGGAGTCAGAAAAGAACAAATATTCTGATTCCATGCTAGTTTCATCGAC
TTTCTATTCTGCAGACGGTCCACGATCTACCTGCCTTACACCTAACATGACACAAGTGCAGCAGGA
TTGCATATCAAGCTCTTGGGAGATGCCTCAAGGTGGACCTCTAGGCGAGATCTTAACCAACTCCAA
GAATAGTGAGGACTTAAGCAAGTGTGAATCAAGGTCATATGGTTGGTTATTGAATCTTGACCATGC
ACCATGATTCCTCAATCCATGGAGAGCTTGACATAGATGTCTCACCATGGAAGCAAACAATGGTCA
GAAAAAGAAGGTTCAAATGACCACATTGTTTGCCCCATGCATGCTCGCTATCTACATTTGTATTTC
TGTTTTGTAGCATTTAGCTAGTTGAATTATCAGTTCTTCTGAATCTGGCTGTATTTTAAACAAATT
CTAGTTTGTGTCAGATGATATCTTGCTTGCTAGATGTTTCATGTCTAACTTTCAACAGGAACTTCA
GAGATCCATTTTGATCAACAGAAAACTGTTTGAAGAACC

**SEQ ID NO: 84 Saccharum officinarum Sacof_GRF translated polypeptide sequence**

MSAEFCAAAGMELGVGDVMGLQQGIAITAPSPRGSGDLGLLKRAALTQAAAGPYPSPFLDEQKMLR
FSKAAHTLPSGLGLDFGGPSEQAFLLSRTKRPFTPSQWMELEHQALIYKYLNAKAPIPSSLLISIS
KSFRSSNRVSWRPLYQGYTNADSDPEPGRCRRTDGKKWRCSKEAMADHKYCERHINRNRHRSRKPV
ENQPKKTTKEVPAAASSLPCAGPQGCLKKAKVNDSKPGTVSCWTDSLNRTMLSREKANKPTEDNSL
LLNSTNSQPTLSLLSQLKQQNKPDKLGPTLENESNSDTILKAWGGNQPSHKSISSTQHHDAESLQS
VLQNFSLAQNEKMESEKNKYSDSMLVSSTFYSADGPRSTCLTPNMTQVQQDCISSSWEMPQGGPLG
EILTNSKNSEDLSKCESRSYGWLLNLDHAP

## FIGURE 10 (continued)

**SEQ ID NO: 85 Vitis vinifera Vitvi_GRF nucleic acid sequence AM468035**

ATGATGATGAGTGCAAGAAACAGGTCTCCTTTCACAGCATCACAGTGGCAAGAGCTTGAACATCAA
GCTCTTATCTTCAAATATATAGTGTCAGGAGTACCAATCCCAGCTGATCTCATCTGCACTGTCAAA
AGAAGCTTGGACTCTTCATTGTCTTCAAGGCTATTTCCTCACCAACCCATTGGGTGGGGTTGTTTT
CAGATGGGGTTTGGCAGGAAAGCAGACCCAGAGCCAGGGAGGTGCAGAAGAACTGATGGCAAGAAA
TGGAGGTGCTCCAAAGAAGCATACCCAGATTCAAAATACTGTGAGAGACACATGCACAGAGGCAAA
AACCGTTCAAGAAAGCCTGTGGAAGTTATTTCAGCTACAAACCCTTCACCAACCATCTCATCAATC
AACTCAAATCCTTCCTCCACCACCACCAATTCTTACTCTCTCTCCCTCTCTCTCCCTCTCTCTTCT
TCAATGACTTCTGAAACCTCCCATCCCCATCACCATTCCTACCACAACACCTCTCTTTATCCCTTC
CTCTACCCTCACCCCTCCTCTTCTAGACCTCCTGGTTCTTGCCTATCACCTCAAGCCACCAGCAGT
TACAGCACCCATCATCTGTTTTTGGACTCTGGGTCTTATTCCCAGGCTGATAGGGATTACAGGGGT
GTGGATGAGAGAGCTTTCTTCCCAGAAGCTTCAGGGACTGTAAGGGGCCTACATGATTCATATACT
CCATTAACAATGAGTTCCTCCAAGGGATACTCTCACTTTCAGTATCAAAGCCCCGCTGATAATCCC
AAACAGCAGCAAGAACAGCAAGAGCAGCAGCACTGCTTTGTCTTGGGCACTGATTTCAAATCGTCA
AGGCCAATTAAAGTAGAGAGAGATGATGAAGCCCAGAAGCCTCTCCACCATTTCTTTGGAGAGTGG
CCTCTAAAGAACAGAGACTCCTGGCTTGACCTTGAGGAGGATCCACCAACCCATGCATCATTCTCC
ACCACCCAGCTCTCAATTTCAATCCCAATGTCCTCACACAAGCTTCTTGCATCGGATTCCAGAATC
CAAACTGGTACTTAGACTTCACTTCAGATTTGGCCTTCTTGCCCTTTATTTCCCTTTTCTGCTAA
GTCTCATCTTCTACTTCATTTTTCCCCCTTGTGCAGATGGATGAGTTCTCAATACTGGTTCTTCTG
ATCATGGCCAAAAAAGTACTTGTACTGGTGGGTTCAATGCTTTTCTTGTTGATTTTGTGACTGAAG
GAAGTTTCTTTTGCCAAATGTGCGGATGAATAATGTAGGGCCTATAGGAGGATTCTTGTCTTTGTG
CTTTCTGAGTTGCTAATTTTCATTCTTATCTTTAAAGAAACATGTTTGAATTTGTAGACTTGTTTG
TTTGACAGGAAGCATCTTGATATGGTTTTTGAGTTTGAGTTTGAGTTGTTCTCTAATCTCTATGTT
GTTTTGTGAGTTGTGGCCACCTTTTCTTTTCCCTTTGGCTTCTGCTTATTGTACTTCAGAAAG

**SEQ ID NO: 86 Vitis vinifera Vitvi_GRF translated polypeptide sequence**

MMMSARNRSPFTASQWQELEHQALIFKYIVSGVPIPADLICTVKRSLDSSLSSRLFPHQPIGWGCF
QMGFGRKADPEPGRCRRTDGKKWRCSKEAYPDSKYCERHMHRGKNRSRKPVEVISATNPSPTISSI
NSNPSSTTTNSYSLSPLSPLSSSMTSETSHPHHHSYHNTSLYPFLYPHPSSSRPPGSCLSPQATSS
YSTHHLFLDSGSYSQADRDYRGVDERAFFPEASGTVRGLHDSYTPLTMSSSKGYSHFQYQSPADNP
KQQQEQQEQQHCFVLGTDFKSSRPIKVERDDEAQKPLHHFFGEWPLKNRDSWLDLEEDPPTHASFS
TTQLSISIPMSSHKLLASDSRIQTGT

**SEQ ID NO: 87 Zea mays Zeama_GRF10_gi_146008494_gb_EF515849.1 nucleic acid sequence**

AGAGCGCCGTATCACCTGTCTCTCCGTCCACCGCCGTCTCGATCCGCGCCAAAGATACCTTTCCCC
CACCCCTTCCTCGCGCCGCCGTTTGGTGCGACCATGACGGCGGAGGGGGAGGCCAAGAACCCGTCG
GCCGGTGGCGGAGGGGATAACCCCCAGCACCAGCAGGCTGCGCCGGCGCCGGCGCCGGCACAGGGG
GAAGTGGCGCAGGAGGCTGCAGTGCAGGGGACGGGACAAGAGCAGGAGCGGGACAAGGCGGATCGA
GAGGTGCAGGGCGGCGCGGGGGAGAAGGACGACGGCGCGTGCAGAGATCTGGTCCTGGTCGAGGAT
CCGGAGGTCCTCGCCGTCGAGGATCCGGAGGAAGCTGCAGCAACCGCAGCACTCCAGGAAGAAATG
AAAGCGCTTGTGGCATCGATCCCTGATGGTGCTGGAGCAGCATTCACAGCCATGCAGCTTCAGGAG
CTAGAGCAGCAGTCCCGGGTGTACCAGTACATGGCTGCCCGAGTACCTGTGCCTACTCACCTCGTC
TTCCCGGTATGGAAGAGTGTGACCGGTGCATCCTCTGAAGGCGCCCAGAAGTACCCTACTTTGATG
GGCTTAGCAACGCTCTGCTTGGACTTTGGGAAGAACCCGGAACCAGAACCAGGGAGGTGTCGGCGA
ACAGATGGTAAGAAATGGCGATGTTGGAGAAACACTATCCCAAACGAGAAATACTGCGAACGTCAC

**FIGURE 10 (continued)**

ATGCATCGTGGCCGCAAGCGTCCTGTACAGGTTTTCCTGGAGGACGACGAGCCCGATTCTGCTTCA
GGGTCAAAACCCGCCGCTCCTGGCAAGGCTACCGAAGGTGCCAAGAAGGCCGATGACAAGAGCCCA
AGCAGCAAGAAGCTTGCAGTGGCAGCGCCTGCCGCTGTGCAGTCTACATAGTCAATTGCAGCTTTA
GTAGCCCGCAGAAAGAGCATA

**SEQ ID NO: 88 Zea mays Zeama_GRF10 translated polypeptide sequence**
MTAEGEAKNPSAGGGGDNPQHQQAAPAPAPAQGEVAQEAAVQGTGQEQERDKADREVQGGAGEKDD
GACRDLVLVEDPEVLAVEDPEEAAATAALQEEMKALVASIPDGAGAAFTAMQLQELEQQSRVYQYM
AARVPVPTHLVFPVWKSVTGASSEGAQKYPTLMGLATLCLDFGKNPEPEPGRCRRTDGKKWRCWRN
TIPNEKYCERHMHRGRKRPVQVFLEDDEPDSASGSKPAAPGKATEGAKKADDKSPSSKKLAVAAPA
AVQST

**SEQ ID NO: 89 Zea mays Zeama_GRF11 gi_146008515_gb_EF515850.1 nucleic acid sequence**
GCCTCTGACACCAGCACAAACCTGGAGACTACTACTAGTATTGGAGTCCCCTCCACTTCCACCTCC
CTTGCCACTGAAGCGAGAGCTCTCGGAGCCGTCGTCCTCTGTCTCTCATCCTTCTTCGTTGTTGAG
CAAAGCGGGCTCGAGGAGGAGATGATGCTGAGCGGGCACGGCGGCGGGAGGCGCCTGTTCACGGCG
TCGCAGTGGCAGGAGCTGGAGCACCAGGCGCTCATCTTCAAATACATGGCCTCCGGCGCGCCCGTG
CCGCACGACCTCGTCCTGCCGCTCCGCCTCGCCACCGGCGTCGACACCGCGCCCTCCCTCGCCTTC
CCGCCCCAGCCTTCGCCGTCGCTGGCGTACTGGGGCTGCTATGGCGCGGGGGCGCCGTTCGGCCGC
AAGGCGGAGGACCCGGAGCCCGGGCGGTGCCGGCGGACGGACGGCAAGAAGTGGCGATGCTCCAGG
GAGGCCCACGGAGACTCCAAGTACTGCGAGAAGCACATCCACCGCGGGAAGAGCCGTTCAAGAAAG
CCTGTGGAAGTGACCTCCCCCGCCGCCTACCGCCCGTCCGCGTTCTCCATCTCGCCGCCTCGCGCG
GCCGACGCGCCGCCGCCGCCGCCGGGCCTCGGCCACCCGCAGCAGCAGCATCTCCGCCACGGCGCT
CTCTCTCCAGCAGGCCGCGCCCACGCCGCTGGCGCTCTCCAGCTCCACCTCGACTCGAGCCTGCAC
GCGGCGTCGCCGCCGCCGTCCTACCACAGGTACGCCCACTCCCACGCTCACTACACGCCGCCGCCG
CCGCCGTCGCTCTACGACTACGGGCAGTCCAAGGAGCTTCGGGAGGCGGCGGAGCTCAGGCGGCGG
CACTTCCACGCGCTCGGGGCCGACCTGAGCCTCGACAAGCCGCTGGCCGACGCCGGGGCCGCGGAG
AAGCCCCTGCGGCGTTTCTTCGACGAGTGGCCGCGGGAGAGAGGCGACACGAGGCCGTCGTGGGCG
GGGGCGGAGGACGCGACGCAGCTCTCCATCTCCATCCCCGCGGCTTCGCCCTCCTCTGACCACGCT
GCCTCTGCCGCCGCGCGATGCCACAACGATGGGAGTGATCGGTGCATCTCCTAGCTGCAACTGCAA
TGCAAGCCTGCAACCGCGTGGATTGTTGTTGATTGGTGTAGTTTCCTAGCTGCAATTCAAGCCTGC
AACAGCGAGCAGTGAGCAGCAAATGCGTGGGGAGGGCACGCAGCTCAGGCTGATGCGCAAAATCCG
AAGCGAGTCAAGCAGCAATAGGACTCTAGGTCTATGATTTGATCTTCCTTTGTAGCAGTACGTTAC
CAAAATGTTAGCTCGTTGTTGTTCGGTGTGACATTTTCGTTCAGGTTGCTCC

**SEQ ID NO: 90 Zea mays Zeama_GRF11 translated polypeptide sequence**
MMLSGHGGGRRLFTASQWQELEHQALIFKYMASGAPVPHDLVLPLRLATGVDTAPSLAFPPQPSPS
LAYWGCYGAGAPFGRKAEDPEPGRCRRTDGKKWRCSREAHGDSKYCEKHIHRGKSRSRKPVEVTSP
AAYRPSAFSISPPRAADAPPPPPGLGHPQQQHLRHGALSPAGRAHAAGALQLHLDSSLHAASPPPS
YHRYAHSHAHYTPPPPPSLYDYGQSKELREAAELRRRHFHALGADLSLDKPLADAGAAEKPLRRFF
DEWPRERGDTRPSWAGAEDATQLSISIPAASPSSDHAASAAARCHNDGSDRCIS

**SEQ ID NO: 91 Zea mays Zeama_GRF12 gi_146008534_gb_EF515851.1 nucleic acid sequence**
CGCATCCGTTCTCTATCGAAAGGGAGGAGGAGGAGCGCGCGGGAGTGGGCTGGGGGCCCACCGATG
CTGAGCTCGGCGTCCTCGGCCGGGGCGGCCATGGGGATGGGCGGCGGGTACCAACACCAGCCGCTG
CCACTGCCGCAGCGCGGGGCGGCGGCCGCGGTCTTCACCGCCGCGCAGTGGGCGGAGCTGGAGCAG

FIGURE 10 (continued)

CAGGCGCTCATCTACAAGTACCTCATGGCCGGCGTCCCCGTCCCGCCCGATCTCCTCCGCCCCGCC
CCCCACGCCGCCGCCTTCTCCTTCGCCAGCCCCGCCGCGTCGCCCTTCTACCATCACCACCACCAC
CACCCGTCCCTGAGTTACTACGCCTACTACGGGAAGAAGCTGGACCCGGAGCCGTGGCGGTGCCGC
CGCACCGACGGCAAGAAGTGGCGGTGCTCCAAGGAGGCGCACCCCGACTCCAAGTACTGCGAGCGC
CACATGCACCGTGGCCGCAACCGTTCAAGAAAGCCTGTGGAATCCAAGACCGCCTCCTCGCCGCCC
CAGCTGTCCACCGTCGTCACCACCACCACCACCCGGGAGGCCGCCGCCGCGACGCCCCTCGAGTCC
CTCGCGGGGGCGGGGGGTAAGGCTCACGGCCTGTCCCTCGGCGGCGGGGCTGGCTCGTCGCACCTC
AGCGTCGACGCTTCGAACACTCACTTTCGCTATGGCAGCAAGTACCCTCTTGGAGCTAAATCCGAT
GCTGGCGAGCTGAGCTTCTTCTCAGGAGCACCAGGGAACTCCAGGGGCTTCACCATTGATTCTCCA
GCAGATAACTCTTGGCACTCCCTGCCATCCAACGTGCCCCCGTTTACACTGTCCAAGGGCAGAGAT
TCTGGCCTCCTGCCTGGAGCGCCACCAGTCGTCGTTCAGCAGCAGCGGGGCCGGCGCTGGTGGGTT
GCTGGGGAGCGTGAAGCAGGAGAACCAGCCGCTGAGGCCCTTCTTCGACGAGTGGCCTGGGACGCG
GGACTCGTGGTCGGAGATGGACGACGCGAGGTCCAGTAGGACCTCCTTCTCGACGACCCAGCTCTC
CATCTCCATTCCGATGCCCAGATGTGATTGAGAACGAAGCTCG

**SEQ ID NO: 92 Zea mays Zeama_GRF12 translated polypeptide sequence**
MLSSASSAGAAMGMGGGYQHQPLPLPQRGAAAAVFTAAQWAELEQQALIYKYLMAGVPVPPDLLRP
APHAAAFSFASPAASPFYHHHHHHPSLSYYAYYGKKLDPEPWRCRRTDGKKWRCSKEAHPDSKYCE
RHMHRGRNRSRKPVESKTASSPPQLSTVVTTTTREAAAATPLESLAGAGGKAHGLSLGGGAGSSH
LSVDASNTHFRYGSKYPLGAKSDAGELSFFSGAPGNSRGFTIDSPADNSWHSLPSNVPPFTLSKGR
DSGLLPGAPPVVVQQQRGRRWWVAGEREAGEPAAEALLRRVAWDAGLVVGDGRREVQ

**SEQ ID NO: 93 Zea mays Zeama_GRF13 gi_146008539_gb_EF515852.1 nucleic acid sequence**
CCTCCCGTCAGCCTCTTCTTCTCCCCCTGATGAGCGCTGAGTTCTGTGCTGCCGCCGCTGGTGCTG
TGGCCATGGAGCTCGGAGTCGGGGATGTGATGGGGCTGCAGCAAGGCATCGCCGCCGCCACCGGGC
CATCGTCCGGAGACAGCGACCTGGGTCTTCTCAAGCGAGCAGGCCTCGCCCAGGCAGCCACCTCCT
ACCCCTCCCCTTTCCTCGACCAACAGAAGATGCTCAGGTTCTCCAAGGCGGCGGCGGCTCACACGT
CGCCCTCAGGCCTAGATTTCGGAGGAGGCCCAAGCGAGCAGGCTTTCCTGCTGTCCAGGACCAAGC
GGCCGTTCACCCCGTCGCAGTGGATGGAGCTGGAGCACCAGGCTCTCATATACAAGTATCTCAATG
CCAAGGCCCCCATACCTTCCAGCCTGCTCGTTTCCATCAGCAAGAGCTTCAGGTCATCCAACAGAG
TGAGCTGGAGGCCTCTTTACCAAGGCTACGCAAACGCAGACTCCGACCCAGAACCTGGGAGGTGCC
GGCGGACAGACGGAAAGAAGTGGCGGTGCTCTAAGGAGGCGATGCCTGATCACAAGTACTGCGAGC
GCCACATCAATAGGAACCGCCACCGTTCAAGAAAGCCTGTGGAAAACCAACCTAGAAAGACCAGCA
AGGAGGTGCCTACCGCTGCTGCTGGCTCGTTGCCGTGTGCCGGGCCACAAGGTAGCTTGAAGAAGG
CAAAAGTTAATGACTCCAAGCCAGGCACTGGCAGCTATTGGACAGATAGCTTAAACAGGACAATGC
TGAGCAGGGAGAAGGCAAACAAACCGACGGAAGACGAGTCTTTGCTGCTTAGTTCTACGAAGAACA
GCCAGCCCACCTTGTCCCTGCTCACTCAACTGAAGCAGCAGAACAAACCAGATAAGTTAGGTCCCA
CACCGGAAAATGAGCCGAACTCGGACACAATGTTGAAAGCCTGGGGTGGCAGCCACCACAAGAACA
TTTCCTCCACACAGCGCCATGACGCTGAATCCCTCCAATCAGTCCTCCAAAATTTCAGCCTAGCCC
AGAATGACAGGTTGGAGTCAGAAAAGAACAGATATTCTGATTCCGTGCTAGTCTCATCGGCTTTCT
ATTCTGCAGACGGTCCACAAACTACCTGCCTTACACCTAACATGACACAAGTGCAGCAGGACTGCA
TATCAAGCTCCTGGGAGATGCCTCAAGGTGGACCTCTAGGCGAGATCTTAACGAACTCCAAGATTA
GTGAGGACTCAAGCAAGTGTGGATCTAGGTCATATGGTTGGCTATTGAATCTTGACCATGCACCAT
GATTCCTC

**FIGURE 10 (continued)**

**SEQ ID NO: 94 Zea mays Zeama_GRF13 translated polypeptide sequence**
MSAEFCAAAAGAVAMELGVGDVMGLQQGIAAATGPSSGDSDLGLLKRAGLAQAATSYPSPFLDQQK
MLRFSKAAAAHTSPSGLDFGGGPSEQAFLLSRTKRPFTPSQWMELEHQALIYKYLNAKAPIPSSLL
VSISKSFRSSNRVSWRPLYQGYANADSDPEPGRCRRTDGKKWRCSKEAMPDHKYCERHINRNRHRS
RKPVENQPRKTSKEVPTAAAGSLPCAGPQGSLKKAKVNDSKPGTGSYWTDSLNRTMLSREKANKPT
EDESLLLSSTKNSQPTLSLLTQLKQQNKPDKLGPTPENEPNSDTMLKAWGGSHHKNISSTQRHDAE
SLQSVLQNFSLAQNDRLESEKNRYSDSVLVSSAFYSADGPQTTCLTPNMTQVQQDCISSSWEMPQG
GPLGEILTNSKISEDSSKCGSRSYGWLLNLDHAP

**SEQ ID NO: 95 Zea mays Zeama_GRF14 gi_146008560_gb_EF515853.1 nucleic acid sequence**
GCCACCAAGAGCCCTCCAACACACACCTGACCTCCCCTTCCCCCCTCTCTCCGCCGCCCGTTCCCC
GCGCCTCCGCCCGTACGTCCCGTTCCCGGTCGGCCGGCCGGTCCAAAGGGAGGGGAGGAGGAGGGG
CGCGGGAGTCGGGGCCCGCACCGATGCTGAGCTCGGCATCCTCGGCCGCGGGGGCGGCCATGGGGA
TGGGCGGCGGCGGGTACGCGCACCAGCCCCCGCCACAGCGCGCGGTCTTCACCGCCGCGCAGTGGG
CGGAGCTGGAGCAGCAGGCGCTCATCTACAAGTACCTCATGGCCGGCGTCCCCGTCCCGCCCGACC
TCCTCCTCCCCGTCCGCCCCGGCCCCGCCGCCGCCTTCTCCTTCGCCGGCCCCGCCGCCGCGTCGC
CCTTCTACCACCAACACCACCCGTCCCTGAGCTACTACGCCTACTACGGCAAGAAGCTGGACCCGG
AGCCGTGGCGGTGCCGCCGCACCGACGGCAAGAAGTGGCGGTGCTCCAAGGAGGCGCACCCCGACT
CCAAGTACTGCGAGCGCCACATGCACCGTGGCCGCAACCGTTCAAGAAAGCCTGTGGAATCCAAGA
CCGCCTCGTCGTCGTCGCCCGCGCACCCGTCGCCGCCCCAGCTGTCCACCGTCACCACCACCGCGC
CTCTCGAGCCCCTTGCAGCGGCGGGGGGCAAGGTCCACGGCCTGTCCCTCGGCGGCGGCGCTGCTG
GCTCGTCGCACCTCGGCGTCGATGCTTCGAATGCTCACTATCGTTATGGTAGCAACAGGTACCCTC
TCGGAGCTAAACCGGACGGCGGCGAGTTGAGCTTCTTCTCAGGAGCGTCATCGGGGAACAACTCGA
GGGGTGGCTTCACCATCGACTCTCCATCAGATAACAACTCGTGGCACTCCGCCCTGGCGTCCAGCG
TGCCCCCGTTCACGCTGTCGACGAAGAGCGGGGACTCCGGCCTCCTGCCCGGCGCCTACGCCTCCT
ACTCCCAGTCCCACTCCCACATGGAGCCGCCGCGGGAGCTCGGGCAGGTCACCATCGCCTCGCTGG
CGCAGGAGCAGGAGCGCCAGCAGCCGTTCAGTGGTGGGATGCTCGGGAACGTGAAGCAGGAGAACC
AGAACCAGCCGCTGCGGCCCTTCTTCGACGAGTGGCCCGGGACGCGGGCGGACTCGTGGCCGCCGG
AGATGGACGGCGCGCCGCGGGCCGGCAGGACCTCCTTCTCCTCCTCCACCACCCAGCTCTCCATCT
CCATCCCGATGCCCAGATGTGAGCTGCATCTCAGAAACCAGAACTCTTAATTCTGTTCGCTGCCCG
AATCATGCTTGACCGAAACTTGTTTCTGCAGGCGACTGACGAGGAACCGTCGATCGGGCGGCCAC
TAGACGGTGGACGCTCACGCTCACTAGTGCGCTGTCGCCTGGAGTGGAGATCGA

**SEQ ID NO: 96 Zea mays Zeama_GRF14 translated polypeptide sequence**
MLSSASSAAGAAMGMGGGGYAHQPPPQRAVFTAAQWAELEQQALIYKYLMAGVPVPPDLLLPVRPG
PAAAFSFAGPAAASPFYHQHHPSLSYYAYYGKKLDPEPWRCRRTDGKKWRCSKEAHPDSKYCERHM
HRGRNRSRKPVESKTASSSSPAHPSPPQLSTVTTTAPLEPLAAAGGKVHGLSLGGGAAGSSHLGVD
ASNAHYRYGSNRYPLGAKPDGGELSFFSGASSGNNSRGGFTIDSPSDNNSWHSALASSVPPFTLST
KSGDSGLLPGAYASYSQSHSHMEPPRELGQVTIASLAQEQERQQPFSGGMLGNVKQENQNQPLRPF
FDEWPGTRADSWPPEMDGAPRAGRTSFSSSTTQLSISIPMPRCELHLRNQNS

**SEQ ID NO: 97 Zea mays Zeama_GRF1 gi_146008330_gb_EF515840.1 nucleic acid sequence**
GACAGGTTGAGATGGCGATGCCGTATGCCTCTCTTTCCCCGGCAGGCGCCGCCGACCACCGCTCCT
CCACAGCCACGGCGTCCCTCGTCCCCTTCTGCCGCTCCACCCCGCTCTCCGCGGGCGGCGGGCTGG
GGGAGGAGGACGCCCAGGCGAGCGCGAGGTGGCCGGCCGCGAGGCCGGTGGTGCCGTTCACGCCGG
CGCAGTACCAGGAGCTGGAGCAGCAGGCGCTCATATACAAGTACCTGGTGGCCGGCGTGCCCGTTC

**FIGURE 10 (continued)**

CGCCGGATCTCGTGGTTCCAATCCGCCGCGGCCTCGACTCCCTCGCTACCCGCTTCTACGGCCAAC
CCACACTCGGGTACGGACCGTACCTGGGGAGGAAACTGGATCCGGAGCCCGGCCGGTGCCGGCGAA
CGGACGGCAAGAAGTGGCGGTGCTCCAAGGAAGCCGCCCCGGACTCCAAGTACTGCGAGCGCCACA
TGCACCGCGGCCGCAACCGTTCAAGAAAGCCTGTGGAAACGCAGCTCGCGCCCCAGTCCCAACCGC
CCGCCGCCGCGGCCGTCTCCGCCGCTCCGCCCCTGGCAGCCGCCGCCGCCGCCGCCACCAACGGCA
GCGGCTTCCAGAACCACTCTCTCTACCCGGCCATCGCCGGCAGCACTGGTGGTGGAGGAGGAGTTG
GCGGGTCCGGCAATATCTCCTCCCCGTTCTCCTCGTCGATGGGGGGATCGTCTCAGCTGCACATGG
ACAGTGTTGCCAGCTACTCCTACGCAGCTCTTGGTGGTGGAACTGCAAAGGATCTCAGGTACAACG
CTTACGGAATAAGATCTCTGGCGGACGAGCACAACCAGCTGATCGCAGAAGCCATCGACTCGTCGA
TAGAGAGCCAGAGGCGCCTCCCCAGCTCGTCGTTCCCGCTCTCGAGCTACCCACATCTCGGGGCGC
TGGGCGACCTGGGCGGCCAGAACAGCACGGTGAGCTCGCTGCCGAAGATGGAGAAGCAGCAGCCGC
CCTCGTCCTTCCTAGGGAACGACACCGGGGCCGGCATGGCCATGGGCTCCGCCTCCGCGAAGCAGG
AGGGCCAGACGCTGCGGCACTTCTTCGACGAGTGGCCCAAGGCGCGGGACTCCTGGCCGGGCCTCT
CCGACGAGACCGCCAGCCTCGCCTCGTCCCCCCCGGCGACCCAGCTGTCGATGTCCATACCCATGG
CGTCCTCCGACTTCTCCGTGGCCAGCTCCCAGTCGCCCAACGATGACTAATGGTGCGTGGATCGTC
GCGTTCTGGCCCTTTGTCTATCTCCCCTCCAGTCCTCCACCCACCGCGCAGTAGTAGCTGCGGAAA
CAGCCCATGCTCCTGTATATTTGTCGGTCATTTTCCGTGTCAGATCTGTGTACCAAACCAAGCGGC
GG

**SEQ ID NO: 98 Zea mays Zeama_GRF1 translated polypeptide sequence**
MAMPYASLSPAGAADHRSSTATASLVPFCRSTPLSAGGGLGEEDAQASARWPAARPVVPFTPAQYQ
ELEQQALIYKYLVAGVPVPPDLVVPIRRGLDSLATRFYGQPTLGYGPYLGRKLDPEPGRCRRTDGK
KWRCSKEAAPDSKYCERHMHRGRNRSRKPVETQLAPQSQPPAAAAVSAAPPLAAAAAAATNGSGFQ
NHSLYPAIAGSTGGGGGVGGSGNISSPFSSSMGGSSQLHMDSVASYSYAALGGGTAKDLRYNAYGI
RSLADEHNQLIAEAIDSSIESQRRLPSSSFPLSSYPHLGALGDLGGQNSTVSSLPKMEKQQPPSSF
LGNDTGAGMAMGSASAKQEGQTLRHFFDEWPKARDSWPGLSDETASLASSPPATQLSMSIPMASSD
FSVASSQSPNDD

**SEQ ID NO: 99 Zea mays Zeama_GRF2 gi_146008352_gb_EF515841.1 nucleic acid sequence**
CCATCTGGCCATCTCCCCTTCCCCTGCTCCCCCGAAGCAGCAAGCCAGCCTGCCCACCCGCAGCCA
TCACCTCCGCCGCTCTCCACCATGAATCCCATCCACCAGCACGACATCGTACCCAATCCTTCGTGA
CTGTTGCCTCCGCGCATCTCCGGGAGCAATGGAAGGAGGCCGAGATGTGTTCTTAGGTGCGGCGGC
AAGGGCGCCGCCGCCGCCGCCGTCTTGCCCGTTTCACGGATCCGCTACCGCCACCCGCTCCGGTGG
AGCGCAGATGCTCAGCTTCTCCTCCAATGGCGTAGCAGGGTTGGGTCTGTGCTCAGGTGCCAGCAA
GATGCAGGGTGTGTTGTCGAGGGTGAGGAGGCCCTTCACTCCGACGCAGTGGATGGAGCTGGAGCA
CCAGGCCCTGATCTACAAGCACTTCGCTGTGAATGCCCCTGTGCCGTCCAGCTTGCTCCTCCCTAT
CAAAAGAAGCCTCAATCCATGGAGCAGCCTTGGCTCCAGCTCATTGGGATGGGCACCATTTCGTTC
CGGCTCTGCTGATGCAGAACCAGGAAGATGCCGCCGCACAGATGGCAAGAAGTGGCGGTGCTCTAG
AGATGCTGTCGGGGACCAAAAATACTGTGAGCGATACATAAAACGTGGTTGCCACCGTTCAAGAAA
GCATGTGGAAGGCCGAAAGGCAACACCGACCACTGCAGATCCAACCATGGCTGTTTCTGGTGGTTC
ATTGTTGCACAGCCATGCTGTTGCTTGGCAGCAGCAGGGCAAAAGCTCAGCTGCTAATGTGACTGA
TCCATTCTCACTAGGGTCCAACAGGAATTTGCTGGATAAGCAGAATCTAGGTGACCAGTTCTCTGT
ATCCACTTCCATGGACTCCTTTGACTTCTCATCATCACATTCTTCCCCAAACCAAGCCAAAGTTGC
ATTTTCACCGGTGGCCATGCAGCACGAACATGATCAGCTGTATCTTGTGCATGGAGCCGGCAGCTC
AGCAGAAAACGTTAACAAGTCTCAGGATGGTCAGCTGCTAGTCTCGAGGGAAACAATTGACGACGG
ACCTCTGGGCGAGGTGTTCAAGGGCAAGAGTTGCCAGTCAGCATCCGCAGACATCTTAACTGACCA
TTGGACTTCGACTCGTGACTTGCGTCCTCCAACCGGAGTCCTACAAATGTCTAGCAGCAACACAGT

**FIGURE 10 (continued)**

GCCAGCAGAGAATCACACGAGTAACAGTAGCTATCTCATGGCGAGGATGGCGAATTCTCAGACCGT
CCCAACACTCCACTGAGTGTTCATCAGGCTGGTCTTTGTTGGGACCACAAAATAACTGAAGCCATG
TTGATGTCCTGAGTTTGCTGATACAGTGATACTAGGTTTTCAGTCGAGTCTTGTAACTCCTGTTTT
AGAGTTGTTATATGTTCACGTCATGTTGCCTTTCATTTTCGGTTTCATTCAGATGGGTGTACTAAT
AATTTCTTTCCTTCTTACCTGTGAAGGATTTGAGTTCCAATCTGAGACGTGGGT

**SEQ ID NO: 100 Zea mays Zeama_GRF2 translated polypeptide sequence**
MEGGRDVFLGAAARAPPPPPSCPFHGSATATRSGGAQMLSFSSNGVAGLGLCSGASKMQGVLSRVR
RPFTPTQWMELEHQALIYKHFAVNAPVPSSLLLPIKRSLNPWSSLGSSSLGWAPFRSGSADAEPGR
CRRTDGKKWRCSRDAVGDQKYCERYIKRGCHRSRKHVEGRKATPTTADPTMAVSGGSLLHSHAVAW
QQQGKSSAANVTDPFSLGSNRNLLDKQNLGDQFSVSTSMDSFDFSSSHSSPNQAKVAFSPVAMQHE
HDQLYLVHGAGSSAENVNKSQDGQLLVSRETIDDGPLGEVFKGKSCQSASADILTDHWTSTRDLRP
PTGVLQMSSSNTVPAENHTSNSSYLMARMANSQTVPTLH

**SEQ ID NO: 101 Zea mays Zeama_GRF3 gi_146008368_gb_EF515842.1 nucleic acid sequence**
TAGCCGTGCTCCGCTCACCTTCTCTCGCGCTACAGTCTCAAGGGGTAGCTAGCCAAGCTACCAAGC
TCGTCAGGAACGAGAGAAAGAGGCCGGCGGTGCGCGGGGATGATGATGATGAGCAGCGGCCGGGCG
GGCGGCGGGGCCACCGCGGGGCGGTACCCGTTCACGGCGTCGCAGTGGCAGGAGCTGGAGCACCAG
GCGCTCATCTACAAGTGCCTGGCGTCCGGCAAGCCCATCCCTTCCTACCTCATGCCGCCGCTCCGC
CGCATCCTCGACTCCGCCCTCGCCACGTCGCCGTCCCTCGCCTACCCGCCGCAACCCTCGCTGGGC
TGGGGCTGCTTCGGGATGGGCTTCACCCGGAAGGCCGACGAGGACCCGGAGCCCGGGCGGTGCCGG
CGCACGGACGGCAAGAAGTGGCGCTGCTCCAAGGAGGCGTACCCGGACTCCAAGTACTGCGAGAAG
CACATGCACCGGGGCAAGAACCGTTCAAGAAAGCCTGTGGAAATGTCCTTGGCCACGCCGGCCCCG
GCGCCGGCCCCCGCCGCCGCCACAACCGCCACCGCCACCTCATCCCCGGCGCCGTCCTACCACCGC
CCGGCCCACGACGCCACGCCGTCTCCGTACCACGCGCTGTATGGAGGCGGCGGCGGCGGCGGCGGT
AGCCCTTACTCGGCGTCGGCACGCCCAGGAGCAACCGGAGGCGGCGGCGCGTACCACCACGCGCAG
CATGTGAGCCCCTTCCACCTCCACCTCGAGACCACCCACCCGCACCCGCCGCCGCCCTACAACTAC
TCCGCCGACCAGAGGGACTACGCGTACGGGCACGCGGCCGCCAAGGAGGTCGGCGAGCACGCCTTC
TTCTCGGACGGCGCGGGCGAGCGGGTCGACCGCCAGGCCGCGGCGGGGCAGTGGCAGTTCAGGCAG
CTCGGGGTGGAGACGAAGCCGGGCCCCACGCCGCTGTTCCCCGTCGCCGGGTACGGGCACGGCGCG
GCGTCGCCGTACGGCGTCGAGCTGGGCAAGGACGACGACGAGCAGGAGGAGAGGCGCCGCCAGCAC
TGCTTCGTTCTTGGAGCCGACCTGCGGCTGGAGCGGCCGTCGTCGGGCCATGGCCATGGCCATGGC
CATGACCATGACGACGCCGCCGCCGCGCAGAAGCCGCTCCGGCCCTTCTTCGACGAGTGGCCGCAC
CAGAAGGGGGACAAGGCCGGGTCGTGGATGGGGCTCGACGGCGAGACGCAGCTCTCCATGTCCATC
CCCATGGCCGCTACCGACCTCCCCGTCACCTCCCGCTTCCGTAACGACGAGTGATGCCACATCAAA
CCTGGCGCTGGAAACTCGGAACGTATGGTG

**SEQ ID NO: 102 Zea mays Zeama_GRF3 translated polypeptide sequence**
MMMMSSGRAGGGATAGRYPFTASQWQELEHQALIYKCLASGKPIPSYLMPPLRRILDSALATSPSL
AYPPQPSLGWGCFGMGFTRKADEDPEPGRCRRTDGKKWRCSKEAYPDSKYCEKHMHRGKNRSRKPV
EMSLATPAPAPAPAAATTATATSSPAPSYHRPAHDATPSPYHALYGGGGGGGSPYSASARPGATG
GGGAYHHAQHVSPFHLHLETTHPHPPPPYNYSADQRDYAYGHAAAKEVGEHAFFSDGAGERVDRQA
AAGQWQFRQLGVETKPGPTPLFPVAGYGHGAASPYGVELGKDDDEQEERRRQHCFVLGADLRLERP
SSGHGHGHGHDHDDAAAAQKPLRPFFDEWPHQKGDKAGSWMGLDGETQLSMSIPMAATDLPVTSRF
RNDE

**FIGURE 10 (continued)**

SEQ ID NO: 103 Zea mays Zeama_GRF4 gi_146008393_gb_EF515843.1 nucleic acid sequence
tcccttcaccgctgcctcgacccgcgccgaaagatacctttccccccttcctctcgcgccgccgt
tttggtgcgaccatggcggcggagggggaggccaagaacccgtccggcggtggcgaagggggtaac
ccccagcaccagcaggcagtgcaggctgcgccggcggagccgccaatggcacaggggaagcggtg
caggaggctggagcgcaggcgacgggacaagagccggagggggagaaggcgaatcgagatggggag
ggaagcgcgggggagaaggacgacggcgcgtgcagagatctggttctggttgaggatccggaggtg
ctcgccgtcgaggacccggaggaagctgcagcaaccgcagcactccaggaagaaatgaaagcgctc
gtggcatccgtccctgacggtgctggggcagcattcacagccatgcagcttcaggagctagagcag
cagtcccgggtttatcagtacatggctgcccgagtacctgtgcctactcacctcgtcttccccgta
tggaagagtgtaaccggtgcatcctctgaaggcgcccagaagtaccctactttgttgggcttagca
acactctgcttggacttcgggaagaaccctgaaccagaaccagggaggtgccggcgaacggatggc
aaaaaatggcgatgttggagaaacactattccaaacgagaagtactgcgaacgccgcatgcatcgc
ggtcgcaagcgtcctgtacaggtcgtcgaggaagccgagcctgactctgcttcaggctcaaaatct
gctcccggcaaggccaccgaaggcgccaagaaggttggcgacaagagcccaggtagcaagaagctt
gccgtggcggcggcagctgcagctgctgcgcagtctacgtaattgatgcagcattttagtagtcgc
aggaagagcatggcggcgctggcaactagcgccttcttttcattgcatgtgatctttagctataac
ctcatttagcacactcccagtggtgtccgtgggaggag

SEQ ID NO: 104 Zea mays Zeama_GRF4 translated polypeptide sequence
MAAEGEAKNPSGGGEGGNPQHQQAVQAAPAEPPMAQGEAVQEAGAQATGQEPEGEKANRDGEGSAG
EKDDGACRDLVLVEDPEVLAVEDPEEAAATAALQEEMKALVASVPDGAGAAFTAMQLQELEQQSRV
YQYMAARVPVPTHLVFPVWKSVTGASSEGAQKYPTLLGLATLCLDFGKNPEPEPGRCRRTDGKKWR
CWRNTIPNEKYCERRMHRGRKRPVQVVEEAEPDSASGSKSAPGKATEGAKKVGDKSPGSKKLAVAA
AAAAAQST

SEQ ID NO: 105 Zea mays Zeama_GRF5 gi_146008412_gb_EF515844.1 nucleic acid sequence
cagccaggtaaggcaaaagagagagggcggaagcagcggcagagcggagagggagagagaagagca
tatatgggcatggcgatgccctttgcctccccgtctccggcagccgaccaccgcccctcctccctc
ctccccttctgccgcgccgcccctctctccgcggcgggagaggacgccgcgcagcagcacgcgatg
agcggcaggtgggccgcgaggccggcgctcttcacggcggcgcagtacgaggagctggagcaccag
gcgctcatatacaagtacctcgtcgccggcgtgcccgtcccgccggacctcctcctcccccctgcgc
cgaggcttcgtcttccaccagccacccgcccttgggtacggcccctacttcggcaagaaggtggac
ccggagcccgggcggtgccggcgtacggacggcaagaagtggcggtgctccaaggaggccgccccg
gactccaagtactgcgagcgccacatgcaccgcggccgcaaccgttcaagaaagcctgtggaagcg
cagctcgcgcccccgccgcacgcccagccgccgcagcagcagcaggcccccgcgcccgctgctggc
ttccagaaccactcgctgtacccgtcgatcctcaacggcaacggcggcggcgggttaggtgctggt
gctggtggtggcacgttcggcctggggcccacctctcagctgcacatggacagtgccgctgcctac
gcgactgctgccggtggagggagcaaatatctcaggtactctgcatacggggtgaaatctctgtcg
gacgagcacagcacgctcttgtcgggcggcatggatccgtcgatgatggacaactcgtggcgcctt
ctgccatcccaaaacaacacattccaagccacaagctaccctgtgttcggcacgctgagtgggcta
gacgagagcaccatcgcgtcgctgccgaagacccagagggagcccctctctttcttcgggagcgac
ttcgtgaccgccgccaagcaggagaaccagacgctgcgccctttcttcgacgagtggcccaagtcg
agggactcgtggccggagctgggcgaggacggcagcctcggcttctcggccacccagctctccatc
tccattcccatggcgacctccgacttctccaacaccagctccagatcgccgggtggaataccgtcg
agatgaacgagtaccgtgcatgtggatcccagcgtcttagggttgacgactcttcggtgctggcct
catcgtatcatgctcctaaatttcgaacgatatatgccttatgtaacgctatttctctcattgtt
acaacaccctttacccgtttggaattgtgttgaagtggatggtctgcgttgctc

**FIGURE 10 (continued)**

**SEQ ID NO: 106 Zea mays Zeama_GRF5 translated polypeptide sequence**
MGMAMPFASPSPAADHRPSSLLPFCRAAPLSAAGEDAAQQHAMSGRWAARPALFTAAQYEELEHQA
LIYKYLVAGVPVPPDLLLPLRRGFVFHQPPALGYGPYFGKKVDPEPGRCRRTDGKKWRCSKEAAPD
SKYCERHMHRGRNRSRKPVEAQLAPPHAQPPQQQAPAPAAGFQNHSLYPSILNGNGGGLGAGA
GGGTFGLGPTSQLHMDSAAAYATAAGGGSKYLRYSAYGVKSLSDEHSTLLSGGMDPSMMDNSWRLL
PSQNNTFQATSYPVFGTLSGLDESTIASLPKTQREPLSFFGSDFVTAAKQENQTLRPFFDEWPKSR
DSWPELGEDGSLGFSATQLSISIPMATSDFSNTSSRSPGGIPSR

**SEQ ID NO: 107 Zea mays Zeama_GRF6 gi_146008429_gb_EF515845.1 nucleic acid sequence**
GATATATGGCGATGCCCTTTGCCTCCCTGTCTCCGGCAGCCGACCACCGCCCCTCCTCCCTCCTCC
CCTACTGCCGCGCCGCCCCTCTCTCCGCGGTGGGAGAGGACGCCGCCGCGCAGGCGCAGCAGCAGC
AGCAGCAGCACGCTATGAGCGGCAGGTGGGCAGCGAGGCCGCCGGCGCTCTTCACAGCGGCGCAGT
ACGAGGAGCTGGAGCACCAGGCGCTCATATACAAGTACCTCGTCGCCGGCGTGCCCGTCCCGCCGG
ACCTCCTCCTCCCCCTACGCCGAGGCTTCGTCTACCACCAACCCGCCCTTGGGTACGGGCCCTACT
TCGGCAAGAAGGTGGACCCGGAGCCCGGGCGGTGCCGGCGTACGGACGGCAAGAAGTGGCGGTGCT
CCAAGGAGGCCGCCCCGGACTCCAAGTACTGCGAGCGCCACATGCACCGCGGCCGCAACCGTTCAA
GAAAGCCTGTGGAAGCGCAGCTCGTGCCCCCGCCGCACGCCCAGCCGCAGCAGCAGGCCCCCGCGC
CCACCGCTGGCTTCCAGAGCCACCCCATGTACCCATCCATCCTCGCCGGCAACGGCGGCGGCGGCG
GCGGGGTAGGTGGCGGTGCTGGCGGTGGCACGTTCGGCCTGGGCCCCACCTCTCAGCTGCGCATGG
ACAGTGCCGCTGCTTACGCGACTGCTGCTGATGGAGGGAGCAAAGATCTCAGGTACTCTGCCTACG
GGGTGAAGTCACTGTCGGACGAGCACAGCCAGCTCTTGCCCGGCGGCGGCGGCGGCATGGACGCGT
CAATGGACAACTCGTGGCGCCTGTTGCCGTCCCAAACCGCCGCCACGTTCCAAGCCACAAGCTACC
CTCTGTTCGGCGCGCTGAGCGGTCTGGACGAGAGCACCATCGCCTCGCTGCCCAAGACGCAGAGGG
AGCCCCTCTCCTTCTTCGGGAGCGACTTCGTGACCCCGAAGCAGGAGAACCAGACGCTGCGCCCCT
TCTTCGACGAGTGGCCCAAGTCGAGGGACTCGTGGCCGGAGCTGAACGAGGACAACAGCCTCGGCT
CCTCGGCCACCCAGCTCTCCACCTCCATCCCCATGGCGCCCTCCGACTTCAACACCAGCTCCAGAT
CGCCGAATGGAATACCGTCAAGATGAACCTGAGTAACCATGCGGACCCCA

**SEQ ID NO: 108 Zea mays Zeama_GRF6 translated polypeptide sequence**
MAMPFASLSPAADHRPSSLLPYCRAAPLSAVGEDAAAQAQQQQQHAMSGRWAARPPALFTAAQYE
ELEHQALIYKYLVAGVPVPPDLLLPLRRGFVYHQPALGYGPYFGKKVDPEPGRCRRTDGKKWRCSK
EAAPDSKYCERHMHRGRNRSRKPVEAQLVPPPHAQPQQQAPAPTAGFQSHPMYPSILAGNGGGGG
VGGGAGGGTFGLGPTSQLRMDSAAAYATAADGGSKDLRYSAYGVKSLSDEHSQLLPGGGGGMDASM
DNSWRLLPSQTAATFQATSYPLFGALSGLDESTIASLPKTQREPLSFFGSDFVTPKQENQTLRPFF
DEWPKSRDSWPELNEDNSLGSSATQLSTSIPMAPSDFNTSSRSPNGIPSR

**SEQ ID NO: 109 Zea mays Zeama_GRF7 gi_146008440_gb_EF515846.1 nucleic acid sequence**
AGCGTGCATTGTTGAGCGAGTGCGGCCAAGCAACGCGGGCTCGAGGAGATGATGCTGAGCGGGCAC
GGCGGCGGGAGGCGCCTGTTCACGGCGTCGCAGTGGCAGGAGCTCGAGCACCAGGCGCTCATCTTC
AAGTACATGGCCTCGGGCGCGCCCGTGCCGCACGACCTCGTCCTACCGCTCCGCCTCGCCACCGGC
GTCGACACCGCGCCCTCCCTCGCCTTCCCGCCCCAGCCTTCGCCGTCGCTGGCGTACTGGGGCTGC
TACGGCGCGGGGGCGCCGTTCGTCGGCCGCAAGGCGGCGGAGGACACGGAGCCGGGGCGGTGCCGG
CGGACGGACGGCAAGAAGTGGCGGTGCTCCAGGGAGGCCCACGGCGACTCCAAGTACTGCGAGAAG
CACATTCACCGCGGGAAGAGCCGTTCAAGAAAGCCTGTGGAAGTGACCTCCTCCCCCGCCGCCGGC
GCCGCTGCGGCGTACCGACCGTCCGCGATCTCCACCATCTCGCCGCCCCGCGCGGCCGACGCGCCG
CCGCCGAGCCTCGCCTACCCGCAGCAGCATCTCCTCCACGGCGCCTCCTCCTCCGCAGCAGCCCGC

**FIGURE 10 (continued)**

GCCCCCGCTGGCGCTCTCCAGCTCCACCTCGACGCGAGCCTGCACGCGGCGGCGGCGTCGCCATCG
CCGCCGCCGTCCTACCACAGGTACGCCCACTACACACCGCCAGCGTCGTCGCTCTTCCCGGGCGGC
GGCTACGGCTACGACTACGACTACGGGCAGTCCAAGGAGCTCAGGCGACGGCACTTCCACGCGCTC
GGGGCCGACCTGAGCCTCGACAAGCCGCTGCCCGAGCCCGACACCGGCTCCGACGAGAAGCAGCCC
CTGCGGCGTTTCTTCGACGAGTGGCCGCGGGAGAGCGGCGACATGGCGGCGGACGACGCGACGCAG
CTTTCCATCTCCATCCCCGCGGCTTCGCCCTCCGACCTCGCTGCTACCTCCGCCTCCGCCGCCGCC
GCGCGATTCCACAACGGGGAGTGATCGGTCCATCTCCTAGCTGCAGCCCTGCAACAGCGTGGATTG
ACCGCTGCATTTCCTGGCTGCAATGCAAGCCTGCAACAGCGAGCAGTAAGCCAGTGACGTGGATGC
ATCTCGTAGCGGCAAACCCTGCTTCTGCCTCT

**SEQ ID NO: 110 Zea mays Zeama_GRF7 translated polypeptide sequence**
MMLSGHGGGRRLFTASQWQELEHQALIFKYMASGAPVPHDLVLPLRLATGVDTAPSLAFPPQPSPS
LAYWGCYGAGAPFVGRKAAEDTEPGRCRRTDGKKWRCSREAHGDSKYCEKHIHRGKSRSRKPVEVT
SSPAAGAAAAYRPSAISTISPPRAADAPPPSLAYPQQHLLHGASSSAAARAPAGALQLHLDASLHA
AAASPSPPPSYHRYAHYTPPASSLFPGGGYGYDYDYGQSKELRRRHFHALGADLSLDKPLPEPDTG
SDEKQPLRRFFDEWPRESGDMAADDATQLSISIPAASPSDLAATSASAAAARFHNGE

**SEQ ID NO: 111 Zea mays Zeama_GRF8 gi_146008461_gb_EF515847.1 nucleic acid sequence**
TTCGGCACGACCCAACAATGCACACCAACATCCACTCCCTCGTCAGGCTCCTCTCCCCCAAATGAG
CGCTGAGTTCTGCGCTGCTGCGGGTGTCGTGGCCATGGAGCTCGGGGTCGGAGATGCGCTGGGGCT
GCAGCAAGGCATCGCAATCACCGCGCCATCGCCCAGGGACAGCGACCTGGGTCTTCTCAAGCGAGC
AGGCCTCACCCAGGCTGCGGCTGCTGCCCCCTACCCCTCCCCCTTCCTTGACGGGGAGAAGATGCT
CAGGTTCTCCAAGGCGGCTCACACATCGCACTCAGGCTTGGATTTTGGAGGCCCAGGTGAGCAGGC
TTTCCTGCTGTCCAGGACCAAGATGCCATTTACTCCCTCGCAGTGGATGGAGCTGGGGCACCAGGC
TCTGATATACAAGTACCTCAATGCAAAGGCCCCCATACCTTCCAGCCTGCTCATTTCAATCAGCAA
GAGCTTCAGATCATCCAATAGAGTGAGCTGGAGGCCTCTGTATCAAGGCTACACAAATGCAGACTC
TGACCCAGAACCTGGGAGATGCCGACGAACGGATGGAAAGAAGTGGCGGTGCTCCAAGGAAGCAAT
GGCTGATCACAAGTACTGTGAGCGGCACATCAACAGAAACCGTCACCGTTCAAGAAAGCCTGTGGA
AAATCAACCTAAGAAGACCACCAAGGAGGTGCCTGCTGCTGCTGGCTCATTACCATGTGCTGGGCC
ACAAGGTAGCTTGAAGAAGGCAAAAGTTAATGACTCCAAGCCAGGCACTGTCAGCTATTGGGCAGA
TAGTTTAAACAGGACAATGTTGAGCAGAGAGAAAGCAAACAAACCGACGGAAGATAGCTCTTTGCT
GCTTACTTCTACGAACAGCCAACCCACCTGGTCCCTGCTCTCTCAGCTGAAGCAGCAAAACAAACC
AGATAAGTTAGGCCCCACACTGGAAAATGAGTCAAACCCAGACACAATATTGAAAGCCTGGGGTGG
CAACCAGCCTAGCCACAAGAGCATTTCCTCTACAGAGCGCCATGATGCTGAATCCCTCCAATCAGT
CCTTCAAAATCTCAGCCTAGCCCAGAATGAGAAGATGGAGTCAGAAAAGGACAAATATTCTGATTC
CGTGCTAGTTTCGTCGACTTTCTATTCTGCAGGCGGTCCAAGAGCTACCTGCCTTACACCTAACAT
GACACAGGTGAAGCAGGATTGCATATCAAGCTCTTGGGAGATGCCTCAAGGTGGACCTCTAGGCGA
AATCTTAACGAACTCCAAGAATAGCAAGGACTTAAGCAAGTGCAAACCAAGGTCATATGGTTGGTT
GTTGAATCTTGACCATGCACCATGATTCCTCAATCCATGAAGAGCTTGACATAGATGTCCCATCAT
GTAGGCAAACAATGGTCAGAAAAAGGTTATGACCACATTGCTTGCCCCATGCATGCTTGCTATCTA
CATTTGTATTTCTGTTGCGTAGCATTTAGCTAGTTGAATTATCAGTTCTTCTGGATACGGCTGT

**SEQ ID NO: 112 Zea mays Zeama_GRF8 translated polypeptide sequence**
MSAEFCAAAGVVAMELGVGDALGLQQGIAITAPSPRDSDLGLLKRAGLTQAAAAPYPSPFLDGEK
MLRFSKAAHTSHSGLDFGGPGEQAFLLSRTKMPFTPSQWMELGHQALIYKYLNAKAPIPSSLLISI
SKSFRSSNRVSWRPLYQGYTNADSDPEPGRCRRTDGKKWRCSKEAMADHKYCERHINRNRHRSRKP

**FIGURE 10 (continued)**

VENQPKKTTKEVPAAAGSLPCAGPQGSLKKAKVNDSKPGTVSYWADSLNRTMLSREKANKPTEDSS
LLLTSTNSQPTWSLLSQLKQQNKPDKLGPTLENESNPDTILKAWGGNQPSHKSISSTERHDAESLQ
SVLQNLSLAQNEKMESEKDKYSDSVLVSSTFYSAGGPRATCLTPNMTQVKQDCISSSWEMPQGGPL
GEILTNSKNSKDLSKCKPRSYGWLLNLDHAP

**SEQ ID NO:113 Zea mays Zeama_GRF9 gi_146008475_gb_EF515848.1 nucleic acid sequence**
GTAGGTCGTTCGCAGGTAGGTAACCGTAACCTAGCTAGCTCGTCGGGATGATGATGATGAGCGGTC
GAGCGGCCACCGCGGGGCGGTACCCGTTCACGGCGTCGCAGTGGCAGGAGCTGGAGCACCAGGCGC
TCATCTACAAGTGCCTGGCGTCCGGCAAGCCCATCCCGTCCTACCTCATGCCACCGCTCCGCCGCA
TCCTCGACTCCGCCCTCGCCACGTCGCCGTCGCTCGCCGCCTTCCAGCCGCAACCCTCGCTGGGGT
GGGGGGGCTGCTTCGGGATGGGCTTCAGCAGGAAGCCCGCCGACGAGGACCCGGAGCCCGGGCGGT
GCCGGCGCACGGACGGCAAGAAGTGGCGCTGCTCCAAGGAGGCGTACCCGGACTCCAAGTACTGCG
AGAAGCACATGCACCGGGGCAAGAACCGTTCAAGAAAGCCTGTGGAAATGTCCTTGGCCACGCCGG
CGCCGCCGGCCTCCTCCGCTGCCACCACCTCGACGTCCCCGGCGCCGTCCTACCACCGCCCGGCCC
CCGCCGCGCACGACGCCGTGCCGTACCACGCGCCCTACGGCGCCGCGTACCATCACACGCAGACGC
AGGTGATGAGCCCCTTCCACCTCCACCTCGAGACCACCCACCCGCACCCGCCGCCGCCGCCGCCCT
ACTACTACGCGGACCAGAGGGACTACGCCTACGGCAAGGAGGTCGGCGAGCGCGCCTTCTTCTCCG
ACGGCGCGGGGGAGAGGGACCGCCAGCAGCAGGCCGCGGGGCAGTGGCAGTTCAAGCAGCTCGGGA
CGATGGAGGCGACGAAGCCGTGCCCCACCCCCACGCCGCTGCTCCCCGCCGCCGGGTACGGCGTCG
GTCAGGCCAAGGAAGACGAGGAGGAGGAAACGCGGCGGCAGCAGCAGCAGCACTGCTTCGTTCTTG
GCGCCGACCTGCGGCTGGCGGAGCGGCCGTCGGGGGCACATGACGACGCCGCGCAGAAGCCGCTCC
GGCATTTCTTCGACGAGTGGCCGCACGAGAAAGGGAGCAAGGCGGGGTGGTGGATTGGGGGACTCG
ACGGCGAGACGACGCAGCTCTCCATGTCCATCCCGATGGCGGCCGCTGCCGACCTCCCCGTCACCT
CCCGCTACCGTACGTGA

**SEQ ID NO: 114 Zea mays Zeama_GRF9 translated polypeptide sequence**
MMMMSGRAATAGRYPFTASQWQELEHQALIYKCLASGKPIPSYLMPPLRRILDSALATSPSLAAFQ
PQPSLGWGGCFGMGFSRKPADEDPEPGRCRRTDGKKWRCSKEAYPDSKYCEKHMHRGKNRSRKPVE
MSLATPAPPASSAATTSTSPAPSYHRPAPAAHDAVPYHAPYGAAYHHTQTQVMSPFHLHLETTHPH
PPPPPPYYYADQRDYAYGKEVGERAFFSDGAGERDRQQQAAGQWQFKQLGTMEATKPCPTPTPLLP
AAGYGVGQAKEDEEETRRQQQQHCFVLGADLRLAERPSGAHDDAAQKPLRHFFDEWPHEKGSKAG
WWIGGLDGETTQLSMSIPMAAAADLPVTSRYRT

**SEQ ID NO: 115 QLQ domain**
RPPFTPTQWEELEHQALIYKYMVSGVPVPPELIFSIRRS

**SEQ ID NO: 116 WRC domain**
DPEPGRCRRTDGKKWRCSREAYPDSKYCEKHMHRGRNRARKSLD

**SEQ ID NO: 117 Oryza sativa GOS2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA

**FIGURE 10 (continued)**

CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

**SEQ ID NO: 118 prm10010**
GGGGACAAGTTTGTACAAAAAAGCAGGCTTAAACAATGATGAGTCTAAGTGGAAGTAG

**SEQ ID NO: 119 prm10011**
**ggggaccactttgtacaagaaagctgggtagctctacttaattagctaccag**

**SEQ ID NO: 120 Arabidopsis thaliana Arath_SYT1 nucleic acid sequence (AY102639)**
ATGCAACAGCACCTGATGCAGATGCAGCCCATGATGGCTGGTTACTACCCCAGCAATGTTACCTCT
GATCATATCCAACAGTACTTGGACGAAAACAAATCGTTGATTCTGAAGATTGTTGAGTCTCAAAAC
TCTGGAAAGCTTAGCGAATGCGCCGAGAATCAAGCAAGGCTTCAACGCAACCTAATGTACCTAGCT
GCAATAGCAGATTCTCAGCCTCAGCCACCAAGTGTGCATAGCCAGTATGGATCTGCTGGTGGTGGG
ATGATTCAGGGAGAAGGAGGGTCACACTATTTGCAGCAGCAACAAGCGACTCAACAGCAACAGATG
ACTCAGCAGTCTCTAATGGCGGCTCGATCTTCAATGTTGTATGCTCAGCAACAGCGGCAGCAGCAG
CCTTACGCGACGCTTCAGCATCAGCAATCGCACCATAGCCAGCTTGGAATGAGCTCGAGCAGCGGA
GGAGGAGGAAGCAGTGGTCTCCATATCCTTCAGGGAGAGGCTGGTGGGTTTCATGATTTTGGCCGT
GGGAAGCCGGAAATGGGAAGTGGTGGTGGCGGTGAAGGCAGAGGAGGAAGTTCAGGGGATGGTGGA
GAAACCCTTTACTTGAAATCATCAGATGATGGGAATTGA

## FIGURE 10 (continued)

**SEQ ID NO: 121 Arabidopsis thaliana Arath_SYT1 translated polypeptide polypeptide sequence**
MQQHLMQMQPMMAGYYPSNVTSDHIQQYLDENKSLILKIVESQNSGKLSECAENQARLQRNLMYLA
AIADSQPQPPSVHSQYGSAGGGMIQGEGGSHYLQQQATQQQQMTQQSLMAARSSMLYAQQQRQQQ
PYATLQHQQSHHSQLGMSSSSGGGGSSGLHILQGEAGGFHDFGRGKPEMGSGGGGEGRGGSSGDGG
ETLYLKSSDDGN

**SEQ ID NO: 122 Arabidopsis thaliana Arath_SYT2 nucleic acid sequence (AY102640)**
ATGCAGCAGCAGCAGTCTCCGCAAATGTTTCCGATGGTTCCGTCGATTCCCCCTGCTAACAACATC
ACTACCGAACAGATCCAAAAGTACCTTGATGAGAACAAGAAGCTGATTATGGCCATCATGGAAAAC
CAGAATCTCGGTAAACTTGCTGAGTGCGCCCAGTACCAAGCTCTTCTCCAGAAGAACTTGATGTAT
CTTGCTGCAATTGCTGATGCTCAACCCCCACCACCTACGCCAGGACCTTCACCATCTACAGCTGTC
GCTGCCCAGATGGCAACACCGCATTCTGGGATGCAACCACCTAGCTACTTCATGCAACACCCACAA
GCATCCCCTGCAGGGATTTTCGCTCCAAGGGGTCCTTTACAGTTTGGTAGCCCACTCCAGTTTCAG
GATCCGCAACAGCAGCAGCAGATACATCAGCAAGCTATGCAAGGACACATGGGGATTAGACCAATG
GGTATGACCAACAACGGGATGCAGCATGCGATGCAACAACCAGAAACCGGTCTTGGAGGAAACGTG
GGGCTTAGAGGAGGAAAGCAAGATGGAGCAGATGGACAAGGAAAAGATGATGGCAAGTGA

**SEQ ID NO: 123 Arabidopsis thaliana Arath_SYT2 translated polypeptide sequence**
MQQQQSPQMFPMVPSIPPANNITTEQIQKYLDENKKLIMAIMENQNLGKLAECAQYQALLQKNLMY
LAAIADAQPPPPTPGPSPSTAVAAQMATPHSGMQPPSYFMQHPQASPAGIFAPRGPLQFGSPLQFQ
DPQQQQQIHQQAMQGHMGIRPMGMTNNGMQHAMQQPETGLGGNVGLRGGKQDGADGQGKDDGK

**SEQ ID NO: 124 Arabidopsis thaliana Arath_SYT3 nucleic acid sequence (AY102641)**
ATGCAGCAATCTCCACAGATGATTCCGATGGTTCTTCCTTCATTTCCGCCCACCAATAATATCACC
ACCGAACAGATCCAAAAGTATCTTGATGAGAACAAGAAGCTGATAATGGCGATCTTGGAAAATCAG
AACCTCGGTAAACTTGCAGAATGTGCTCAGTATCAAGCTCTTCTCCAGAAGAATTTGATGTATCTC
GCTGCAATTGCGGATGCTCAACCTCAGCCACCAGCAGCTACACTAACATCAGGAGCCATGACTCCC
CAAGCAATGGCTCCTAATCCGTCATCAATGCAGCCACCACCAAGCTACTTCATGCAGCAACATCAA
GCTGTGGGAATGGCTCAACAAATACCTCCTGGGATTTTCCCTCCTAGAGGTCCATTGCAATTTGGT
AGCCCGCATCAGTTTCTGGATCCGCAGCAACAGTTACATCAACAAGCTATGCAAGGGCACATGGGG
ATTAGACCAATGGGTTTGAATAATAACAACGGACTGCAACATCAAATGCACCACCATGAAACTGCT
CTTGCCGCAAACAATGCGGGTCCTAACGATGCTAGTGGAGGAGGTAAACCGGATGGGACCAATATG
AGCCAGAGTGGAGCTGATGGGCAAGGTGGCTCAGCCGCTAGACATGGCGGTGGTGATGCAAAAACT
GAAGGAAAATGA

**SEQ ID NO: 125 Arabidopsis thaliana Arath_SYT3 translated polypeptide sequence**
MQQSPQMIPMVLPSFPPTNNITTEQIQKYLDENKKLIMAILENQNLGKLAECAQYQALLQKNLMYL
AAIADAQPQPPAATLTSGAMTPQAMAPNPSSMQPPPSYFMQQHQAVGMAQQIPPGIFPPRGPLQFG
SPHQFLDPQQQLHQQAMQGHMGIRPMGLNNNNGLQHQMHHHETALAANNAGPNDASGGGKPDGTNM
SQSGADGQGGSAARHGGGDAKTEGK

**FIGURE 10 (continued)**

**SEQ ID NO: 126 Allium cepa Allce_SYT2 nucleic acid sequence CF437485**

ATGCAGCAGCCGCAGCCAGCGATGGGAACCATGGGCTCGGTGCCACCTACTAGCATCACCACCGAA
CAGATTCAAAGGTACTTGGATGAGAACAAACAGTTAATATTGGCAATTTTGGATAATCAAAATTTA
GGAAGACTGAATGAGTGTGCTCAATATCAAGCTCAGCTTCAAAAGAATCTGCTTTACCTGGCAGCA
ATAGCTGATGCTCAGCCTCAGTCTCCTGCGGTGCGTCTGCAGATGATGCCTCAAGGTGCAGCTGCC
ACGCCTCAAGCTGGAAACCAATTTATGCAGCAGCAGAGCCCTAATTTCCCTCCCAAAACAGGAATG
CAATTTACTCCTCAACAAGTACAAGAATTGCAGCAGCAACAGCTACAACATCAGCCACATATGATG
CCTCCATTTCAAGGTCAAATGGGTATGAGACCTATGAATGGAATGCAGGCAGCAATGCATGCAGAT
TCATCTCTTGCTTATAACACTAACAATAAGCAAGATGCAGGAAACGCAGCTTATGAAAATACTGCT
GCCAACACAGATGGTTCCATTCAAAAGAAAACAGCAAATGATGATTTAGACCCTTCTGCAGCAAAC
CCTAGAAGGTCTGAAGATGCCAAATCATCATGA

**SEQ ID NO: 127 Allium cepa Allce_SYT2 translated polypeptide sequence**

MQQPQPAMGTMGSVPPTSITTEQIQRYLDENKQLILAILDNQNLGRLNECAQYQAQLQKNLLYLAA
IADAQPQSPAVRLQMMPQGAAATPQAGNQFMQQQSPNFPPKTGMQFTPQQVQELQQQLQHQPHMM
PPFQGQMGMRPMNGMQAAMHADSSLAYNTNNKQDAGNAAYENTAANTDGSIQKKTANDDLDPSAAN
PRRSEDAKSS

**SEQ ID NO: 128 Aquilegia formosa x Aquilegia pubescens Aqufo_SYT1 nucleic acid sequence DT758802.1**

ATGCAACACATGCAGATGCAGCCCATGATGCCACCTTATAGTGCCAACAGCGTCACTACTGATCAT
ATCCAACAGTACTTGGATGAAAATAAGGCGTTGATTCTGAAGATACTTGAGAACCAAAATTCGGGA
AAAGTTAGTGAATGTGCAGAGAACCAAGCAAGACTTCAACGAAATCTTATGTATCTGGCTGCAATT
GCTGATTCTCAACCACAGCCTCCCAATATGCATGCTCAGTACTCTAATGCGGGTATACCACCTGGT
GCACATTACCTACAACACCAACAGGCCCAACAGATGACACAACAGTCGCTCATGGCTGCTCGATCA
AATATGCTGTATGCTCAGCCAATCACAGGAATGCAGCAACAGCAAGCAATGCATAGCCAGCTTGGC
ATGAGCTCTGGTGGTAACAGTGGACTCCACATGATGCACAATGAGGGCAGCATGGGAGGTAGTGGG
GCACTTGGAAGCTATTCTGATTATGGCCGTGGCAGTGGTGGTGGAGTAACTATCGCTAGCAAACAA
GATGGTGGAAGTGGTTCTGGTGAAGGACGAGGTGGAAACTCTGGAGGCCAAAGTGCAGATGGAGGT
GAATCTCTTTACCTGAAAAACAGTGACGAAGGGAACTAA

**SEQ ID NO: 129 Aquilegia formosa x Aquilegia pubescens Aqufo_SYT1 translated polypeptide sequence**

MQHMQMQPMMPPYSANSVTTDHIQQYLDENKALILKILENQNSGKVSECAENQARLQRNLMYLAAI
ADSQPQPPNMHAQYSNAGIPPGAHYLQHQQAQQMTQQSLMAARSNMLYAQPITGMQQQQAMHSQLG
MSSGGNSGLHMMHNEGSMGGSGALGSYSDYGRGSGGGVTIASKQDGGSGSGEGRGGNSGGQSADGG
ESLYLKNSDEGN

**SEQ ID NO: 130 Aquilegia formosa x Aquilegia pubescens Aqufo_SYT2 nucleic acid sequence DT758802.1**

ATGCAGCAACCTCCGCCAATGATGAACATGGTCCCACCATTTCCTCCTACTAACATTACAACTGAA
CAGATTCAAAAGTATCTGGATGAGAACAAAACACTGATTTTGGCAATATTAGACAATCAGAATCTT
GGAAAATTAGCCGAATGTGCTCAGTACCAAGCTCAGCTTCAGAAGAATCTGATGTATCTTGCTGCA
ATTGCTGATGCCCAACCGCAAGCTCCTGCAGTTCCCTCTCAGATGCCAACACATCCGGCAATGCAA
CAGGGAGGACATTATATGCAACATCCGCAAGCAGCTATGCCTCAACAGCCCAGTGGTTTTCCACCC
AAGTCCCCCATGCAATTTAACCCTCAGCAAATGCAAGAGCAGCAACGGCTGCAGCTACAACAGCAA

**FIGURE 10 (continued)**

CACCAACAGGCACTTCAAGGTCATATGGGCATTCGACCTGGAGTCAACAATGGTTTGCAAATGCAT
GGGGATGGTAATGTTGGAGGCAGCAGCAGCGGTGGCCCATCATCAACCGGTAACTTACCTGATTTC
TCACGCAGTGGTGCCGGTCCTGGTGCTGGTTCTAGTTCATTGGATGCACGGGAAGGTAAGCAAGAT
GGAGTGGAAGCGGGTGCTGGTGATGGTCAAGGCAATTCAGCAGCCAGAAATAATGGTTCAAATGGG
GATACATG

**SEQ ID NO: 131 Aquilegia formosa x Aquilegia pubescens Aqufo_SYT2 translated polypeptide sequence**
MQQPPPMMNMVPPFPPTNITTEQIQKYLDENKTLILAILDNQNLGKLAECAQYQAQLQKNLMYLAA
IADAQPQAPAVPSQMPTHPAMQQGGHYMQHPQAAMPQQPSGFPPKSPMQFNPQQMQEQQRLQLQQQ
HQQALQGHMGIRPGVNNGLQMHGDGNVGGSSSGGPSSTGNLPDFSRSGAGPGAGSSSLDAREGKQD
GVEAGAGDGQGNSAARNNGSNGDT

**SEQ ID NO: 132 Aspergillus officinalis Aspof_SYT1 nucleic acid sequence (CV287542)**
ATGCAGCAGCACCTGATGCAGATGCAGCCCATGATGGCAACCTACGGTTCACCGAATCAGGTCACC
ACCGATATCATTCAGCAGTATCTGGACGAGAACAAGCAGTTGATTCTGGCTATTCTTGAAAACCAA
AATTCAGGAAAAGCTGATGAATGTGCTGAGAATCAGGCTAAGCTTCAGAGGAATCTGATGTATCTT
GCAGCCATTGCGGATAGCCAGCCCCAAGTTCCTACCATTGCTCAGTATCCTCCCAACGCTGTTGCT
GCTATGCAATCGAGTGCTCGCTACATGCAACAACACCAAGCAGCTCAACAGATGACCCCTCAATCT
CTCATGGCTGCTCGCTCCTCAATGCTCTACTCACAGTCCCCAATGTCTGCACTCCAGCAGCAACAG
CAGCAAGCAGCAATGCATAGCCAGCTCGCCATGAGCTCCGGAGGCAACAACAGCAGCACCGGAGGA
TTCACCATTCTTCATGGTGAAGCTAGCATAGGAGGCAATGGCTCAATGAATTCTGGTGGAGTCTTT
GGAGATTTTGGACGGAGCAGCGGTGGGAAGCAAGAGACTGGGAGCGAAGGGCACGGGACAGAGACT
CCTATGTACCTGAAAGGCTCTGAAGAAGAAGGAAACTGA

**SEQ ID NO: 133 Aspergillus officinalis Aspof_SYT1 polypeptide**
MQQHLMQMQPMMATYGSPNQVTTDIIQQYLDENKQLILAILENQNSGKADECAENQAKLQRNLMYL
AAIADSQPQVPTIAQYPPNAVAAMQSSARYMQQHQAAQQMTPQSLMAARSSMLYSQSPMSALQQQQ
QQAAMHSQLAMSSGGNNSSTGGFTILHGEASIGGNGSMNSGGVFGDFGRSSGGKQETGSEGHGTET
PMYLKGSEEEGN

**SEQ ID NO: 134 Beta vulgaris Betvu_SYT2 nucleic acid sequence contig of BQ594749.1, BQ594658.1**
Atgcagcaacaatcacctcaaatgttcaaccacccaccttcacaacccacaattactaccgaacaa
attcaaaagtatcttgatgagaacaagcagttgattttggcaattatggaaagtcaaaactctgga
aaaatgaatgaatgtgcccagtatcaagctcagctgcagaaaaacttgatgtacttggctgcaatt
gctgatgctcagccaccagcacctacagggccctctcagtctcagccgcagaattctcagatgcct
atgcaatcgaccattccacaaggcccttttatgccgcctcctaaacctgcagttaccccacagcaa
acaggtcccagattgccctttgctctacagtcgtttgatcagcagtcaccccatatgcaaatgcaa
taccaacagtccatggcaggctccatgggtatgagaatgggtgggaataatgttttacgcccttcc
atccagaccggatatggagctccaacacatttatggatgcacggaacagacaagatggttctgac
gcaagtctcggtgatgatcatggaaagtaa

**SEQ ID NO: 135 Beta vulgaris Betvu_SYT2 translated polypeptide sequence**
MQQQSPQMFNHPPSQPTITTEQIQKYLDENKQLILAIMESQNSGKMNECAQYQAQLQKNLMYLAAI
ADAQPPAPTGPSQSQPQNSQMPMQSTIPQGPFMPPPKPAVTPQQTGPRLPFALQSFDQQSPHMQMQ
YQQSMAGSMGMRMGGNNVLRPSIQTGYGAPTHFMDARNRQDGSDASLGDDHGK

**FIGURE 10 (continued)**

**SEQ ID NO: 136 Brachypodium distachyon SYT3 nucleic acid sequence DV480064.1**

ATGCAGCAGGCGATGTCCATGTCCCCGGGGTCGGCCGGCGCGGTGCCGCCTCCGGCCGGCATCACC
ACAGAGCAGATCCAAAAGTATTTGGATGAAAATAAGCAACTTATTTGGCCATCCTGGAAAATCAG
AACCTAGGAAAGTTGACTGAATGTGCTCAGTATCAAGCTCAACTTCAGAAGAATCTCTTGTATCTG
GCTGCCATTGCGGATGCCCAACCACCACAGAACCCTGGAAGTCGCCCCCAGATGGTGCAGCCTGGT
GGTATGCCAGGTGCAGGGCATTACATGTCGCAAGTACCAATGTTCCCTCCAAGAACCCCTTTAACC
CCACAACAGATGCAAGAGCAACAGCACCAGCAGCTTCAGCAGCAGCAAGCACAGGCTCTTGCTTTC
CCCAGCCAGATGGTCATGAGACCAGGTACTGTGAACGGCATGCAGCCTATGCAAGCTGATCTCCAA
GCAGCAGCAGCAGCACCTGGCCTGGCAGACAGCCGAGGAAGTAAGCAGGACGCAGCGGTAGCTGGG
GCCATCTCGGAACCTTCTGGCACCGAGAGTCACAAGAGTACAGGAGCGGATCATGAGGCAGGTGGC
GATGTAGCTGAGCAATCCTAA

**SEQ ID NO: 137 Brachypodium distachyon SYT3 translated polypeptide sequence polypeptide**

MQQAMSMSPGSAGAVPPPAGITTEQIQKYLDENKQLILAILENQNLGKLTECAQYQAQLQKNLLYL
AAIADAQPPQNPGSRPQMVQPGGMPGAGHYMSQVPMFPPRTPLTPQQMQEQQHQQLQQQQAQALAF
PSQMVMRPGTVNGMQPMQADLQAAAAAPGLADSRGSKQDAAVAGAISEPSGTESHKSTGADHEAGG
DVAEQS

**SEQ ID NO: 138 Brassica napus SYT1 nucleic acid sequence (CD823592)**

ATGCAGCCCATGATGGCTGGTTACTACCCCAGCAATGTCACCTCTGATCATATCCAGCAGTACTTG
GATGAGAACAAGTCTTTGATTCTGAAGATAGTTGAGTCTCAAAACTCAGGAAAGCTCAGCGAGTGT
GCCGAGAATCAGGCAAGGCTTCAACGCAACCTCATGTACTTGGCTGCAATAGCAGATTCTCAGCCT
CAACCTCCAAGCGTGCATAGCCAGTATGGATCTGCTGGTGGTGGGTTGATTCAGGGAGAAGGAGCG
TCACACTATTTGCAGCAGCAACAGGCGACTCAACAGCAGCAGATGACTCAGCAGTCTCTTATGGCA
GCTCGTTCTTCAATGATGTATCAGCAGCAGCAACAGCCTTATGCAACGCTTCAGCATCAGCAGTTG
CACCATAGCCAGCTTGGGATGAGCTCTAGCAGCGGAGGAGGAAGCAGTGGTCTCCATATCCTTCAG
GGAGAGGCTGGTGGGTTTCATGAATTTGGCCGTGGGAAGCCGGAGATGGGAAGTGGTGAAGGCAGG
GGTGGAAGCTCAGGGGATGGTGGAGAAACACTCTACTTGAAGTCATCAGATGATGGGAACTGA

**SEQ ID NO: 139 Brassica napus SYT1 translated polypeptide sequence**

MQQHLMQMQPMMAGYYPSNVTSDHIQQYLDENKSLILKIVESQNSGKLSECAENQARLQRNLMYLA
AIADSQPQPPSVHSQYGSAGGGLIQGEGASHYLQQQQATQQQQMTQQSLMAARSSMMYQQQQQPYA
TLQHQQLHHSQLGMSSSSGGGSSGLHILQGEAGGFHEFGRGKPEMGSGEGRGGSSGDGGETLYLKS
SDDGN

**SEQ ID NO: 140 Brassica napus SYT2 cDNA CN732814**

ATGCAGCAGCAGCAGCAGCAGCAGCAGCAGCCTCCGCAAATGTTTCCGATGGCTCCTTCGATGCCG
CCAACTAACATCACCACCGAACAGATCCAAAAGTACCTTGAGGAGAACAAGAAGCTGATAATGGCA
ATCATGGAAAATCAGAATCTTGGCAAGCTTGCAGAGTGTGCACAGTACCAAGCTCTTCTCCAGAAG
AACTTAATGTACCTCGCTGCTATTGCTGATGCTCAACCTCCTCCATCTACCGCTGGAGCTACACCA
CCACCAGCTATGGCTTCCCAGATGGGGGCACCGCATCCTGGGATGCAACCGCCGAGCTACTTTATG
CAACACCCACAAGCTTCAGGGATGGCTCAACAAGCACCACCCGCTGGTATCTTCCCTCCGAGAGGT
CCTTTGCAGTTTGGTAGCCCACACCAGCTTCAGGATCCGCAACAGCAGCATATGCATCAACAGGCT
ATGCAAGGACACATGGGGATGCGACCAATGGGTATCAACAACAACAATGGGATGCAGCATCAGATG
CAGCAACAACAACCAGAAACCTCTCTTGGAGGAAGCGCTGCAAACGTGGGGCTTAGAGGTGGAAAG
CAAGATGGAGCAGATGGACAAGGAAAAGATGATGGCAAATGA

**FIGURE 10 (continued)**

**SEQ ID NO : 141 Brassica napus SYT2 polypeptide**
MQQHLMQMQPMMAGYYPSNVTSDHIQQYLDENKSLILKIVESQNSGKLSECAENQARLQRNLMYLA
AIADSQPQPPSVHSQYGSAGGGLIQGEGASHYLQQQATQQQMTQQSLMAARSSMMYQQQQQPYA
TLQHQQLHHSQLGMSSSSGGGSSGLHILQGEAGGFHEFGRGKPEMGSGEGRGGSSGDGGETLYLKS
SDDGN

**SEQ ID NO: 142 Chlamydomonas reinhardtii Chlre_SYT nucleic acid sequence contig of BQ814858, jgi_Chlre3_194013 estExt fgenesh2_pg.C_510025**
ATGGCGGCAGCCTCAAAACCTCCACCGATGACGACCGACAAAATACAAGACATGCTGGAGGAGAAT
TTCAAGTTCATTAAAGCCATTGCCGAGCAGCAAAACTTGGGCCGGGTTCAAGAAGTGCACCAGTAC
CAGCAGAAGCTGCAGGAGAACCTGATGCTGCTGGCCGCAGTGGCAGACACCTACTCCAACTCAGCA
GCAGCAGCACAGCCGGGGGGAGAAGCTGGCGCAGCCGCACCCGCGGCGGCCACGGCACGACCACCC
ACCGCGCCTGGAGCGCCGCTGGGGGCACCGGGAGCACCGCCGCCCGCGGCTCCGGCTCTCACACCA
CAGCAGATCCACGCGGCCGTGCAGCAGGCACTGGCCATGAAGCAGCAGCAGCAGCAGCAGCAGCAG
CAACAGCCGCAGCAGCCGCAGCAGTCGGCGGTGGCGCAGTACCAGCAACCGCCTCAAGCGGGGCTG
CCCATACCGGGCGGGATGGCGCCCGGGCAAGGCGCGCCGCCAGGCTTCACGCTACCGGCGCCACCG
CCCCTGAACCTAGCCGGGCAGTAG

**SEQ ID NO: 143 Chlamydomonas reinhardtii Chlre_SYT translated polypeptide sequence**
MAAASKPPPMTTDKIQDMLEENFKFIKAIAEQQNLGRVQEVHQYQQKLQENLMLLAAVADTYSNSA
AAAQPGGEAGAAAPAAATARPPTAPGAPLGAPGAPPPAAPALTPQQIHAAVQQALAMKQQQQQQQ
QQPQQPQQSAVAQYQQPPQAGLPIPGGMAPGQGAPPGFTLPAPPPLNLAGQ

**SEQ ID NO : 144 Citrus sinensis Citsi_SYT1 nucleic acid sequence (CB290588)**
ATGCAACAGCACCTGATGCAGATGCAGCCCATGATGGCAGCTTATTATCCCAACAACGTCACTACT
GACCACATTCAACAGTATCTAGATGAGAACAAATCATTGATTTTGAAGATTGTTGAGAGCCAGAAT
TCAGGGAAACTGAGCGAGTGTGCAGAGAACCAGGCAAGATTGCAGCGGAATCTCATGTACCTGGCT
GCTATTGCTGATGCTCAACCCCAACCACCTAGCGTTCATGCCCAGTTCTCTTCTGGTGGCATTATG
CAGCCAGGAGCTCACTATATGCAACACCAGCAATCTCAGCCAATGACACCACAGTCACTTATGGCT
GCACGCTCATCCATGGTGTACTCTCAACAGCAATTTTCAGTGCTTCAGCAACAGCAAGCCTTGCAT
GGTCAGCTTGGCATGAGCTCTGGTGGTAGCTCAGGACTTCACATGCTGCAAAGTGAGGGTAGTACT
GCAGGAGGTAGTGGTTCACTTGGGGGTGGGGGATTCCCTGATTTTGGCCGTGGCTCATCTGGTGAA
GGCTTGCACTCAAGGGGAATGGGGAGCAAGCATGATATAGGCAGTTCTGGATCTGCTGAAGGACGA
GGAGGGAGCTCAGGAAGCCAAGATGGAGGCGAAACTCTCTACTTGAAAGGGGCTGATGATGGAAAT
TAA

**SEQ ID NO : 145 Citrus sinensis Citsi_SYT1 translated polypeptide sequence**
MQQHLMQMQPMMAAYYPNNVTTDHIQQYLDENKSLILKIVESQNSGKLSECAENQARLQRNLMYLA
AIADAQPQPPSVHAQFSSGGIMQPGAHYMQHQQSQPMTPQSLMAARSSMVYSQQQFSVLQQQQALH
GQLGMSSGGSSGLHMLQSEGSTAGGSGSLGGGGFPDFGRGSSGEGLHSRGMGSKHDIGSSGSAEGR
GGSSGSQDGGETLYLKGADDG

**FIGURE 10 (continued)**

**SEQ ID NO : 146 Citrus sinensis Citsi_SYT2 nucleic acid sequence CV717501**

ATGCAGCAGCCACCGCAAATGATCCCTGTTATGCCTTCATTTCCACCCACCAACATCACCACAGAG
CAGATTCAAAAGTACCTTGATGAGAACAAAAAGTTGATTTTGGCAATTTTGGACAATCAAAATCTT
GGAAAGCTTACAGAATGTGCCCACTATCAAGCTCAGCTTCAAAAGAATTTAATGTATTTAGCTGCA
ATTGCTGATGCACAACCACAAGCACCAACAATGCCTCCTCAGATGGCTCCACATCCTGCAATGCAA
GCTAGTGGGTATTACATGCAACATCCTCAGGCGGCAGCAATGGCTCAGCAACAAGGAATCTTTCCC
CAAAAGATGCCATTACAATTCAATAACCCTCATCAACTACAGGATCCTCAACAGCAGCTACACCAA
CATCAAGCCATGCAAGCACAAATGGGAATGAGACCGGGTGCCACTAACAATGGTATGCATCCCATG
CATGCTGAAAGCTCTCTTGGAGGTGGCAGCAGTGGAGGACCCCCTTCAGCATCAGGCCCAGGTGAC
ATACGTGGTGGAAATAAGCAAGATGCCTCGGAGGCTGGGACTACTGGTGCTGATGGCCAGGGCAGT
TCGGCTGGTGGGCATGGTGGGGATGGAGAGGAGGCAAAGTGA

**SEQ ID NO : 147 Citrus sinensis Citsi_SYT2 translated polypeptide sequence**

MQQPPQMIPVMPSFPPTNITTEQIQKYLDENKKLILAILDNQNLGKLTECAHYQAQLQKNLMYLAA
IADAQPQAPTMPPQMAPHPAMQASGYYMQHPQAAAMAQQQGIFPQKMPLQFNNPHQLQDPQQQLHQ
HQAMQAQMGMRPGATNNGMHPMHAESSLGGGSSGGPPSASGPGDIRGGNKQDASEAGTTGADGQGS
SAGGHGGDGEEAK

**SEQ ID NO: 148 Cryptomeria japonica Cryja_SYT1 nucleic acid sequence TA3001_3369 _2**

atgcagcagcatctcatgcaaatgcagccgatgatggcagcagcttacgcttctaacaacattacc
actgatcacattcaaaagtacttggatgagaacaagcagttgatattagcaattatggacaatcaa
aatctgggaaagcttaatgaatgtgcacagtaccaagcaaaacttcaacagaacttgatgtatcta
gctgctattgctgattctcagcctcaagttccggctgcacatgctcagattcctcctaatgcggtt
gtgcagtctggtgggcttttcatgcagcaccagcaagcacagcagcaagttactcctcagtctctt
atggcagctagatcttccatgttgtatacccagcagccgatggctgctttgcatcaagcccagcag
cagcagcaacaacaatctcttcacagccatcttggtataagttctggaggaagcaatggcttgcac
atgttgcatggtgaagcaaacatgggaggtaacgggcctctctcatctggaggcttccctgacttt
tcacgtggaactggggcctctggtgaaggcattcaggccaataggggcatgtgtatagatcgtggt
gcaaataagcatgatggcgctggaacggagaatgctcatccaggcccaggggatgggcgagggagt
tcgactggaggccagaatacagatgggtcagaacaatcatacctgaaagcctcagaagaggggaac
tag

**SEQ ID NO: 149 Cryptomeria japonica Cryja_SYT1 translated polypeptide sequence**

MQQHLMQMQPMMAAAYASNNITTDHIQKYLDENKQLILAIMDNQNLGKLNECAQYQAKLQQNLMYL
AAIADSQPQVPAAHAQIPPNAVVQSGGLFMQHQQAQQQVTPQSLMAARSSMLYTQQPMAALHQAQQ
QQQQQSLHSHLGISSGGSNGLHMLHGEANMGGNGPLSSGGFPDFSRGTGASGEGIQANRGMCIDRG
ANKHDGAGTENAHPGPGDGRGSSTGGQNTDGSEQSYLKASEEGN

**SEQ ID NO: 150 Curcuma longa Curlo_SYT2 nucleic acid sequence TA2676_136217**

ATGCAGCAATCTCCACATTCGCTAGCCCCCATGTCAGCAGCCCCTGTTGCGAATATTACAACAGAA
CAAATTCAAAAGTACTTGGATGAGAATAAGCAGCTCATTTTGGCAATATTGGAAAATCAGAACCTT
GGGAAATTGGCTGAATGTGCTCAGTACCAAGCGCAGCTTCAGAAAAATCTACTTTATCTTGCTGCA
ATTGCTGATGCTCAACCTAATGCACCTGCAGTTCGTCCCCAGCAGATCATGCCACACGGTACGATA

**FIGURE 10 (continued)**

CCACAGGGAAGCCCTTTCATGCAACAATCACCCATCTTCCCTCGAGGTCCTCTTCCATATAATCCT
CAACAAATGCAAGGGCAGCTACATCCCCAACCCCCAGGAATGGTGTTCCCAGGCCATATGGGCATT
AGGCCCGGCGCTGTCAACGGCTTACATGGCTCGCATACTGAACCATCTCATGGTGGCACTGCTAAT
CCCCTCACAACTCCAAGCTTGTCTGGATTCCCACCAACCAACTCAGATGGACGTGGGAGTAAGCAA
GAAGCCGGCATCGCCATGGTACCTGCCGTAGCTGAGAGCCACAGGAACTCAGGAAGTGAGCCTGTC
AGTGGGGATGCTGATCAATCACATGCTAAAAGACCAGAGGATACAAAGACACCATGA

**SEQ ID NO: 151 Curcuma longa Curlo_SYT2 translated polypeptide sequence**

MQQSPHSLAPMSAAPVANITTEQIQKYLDENKQLILAILENQNLGKLAECAQYQAQLQKNLLYLAA
IADAQPNAPAVRPQQIMPHGTIPQGSPFMQQSPIFPRGPLPYNPQQMQGQLHPQPPGMVFPGHMGI
RPGAVNGLHGSHTEPSHGGTANPLTTPSLSGFPPTNSDGRGSKQEAGIAMVPAVAESHRNSGSEPV
SGDADQSHAKRPEDTKTP

**SEQ ID NO: 152 Euphorbia esula Eupes_SYT2 nucleic acid sequence DV144834**

ATGCAGCAGCAACCGCAGATGATGCCTATGATGCCTTCATATCCACCAGCAAACATTACCACGGAG
CAAATCCAAAAGTATCTTGATGAAAATAAAAAATTGATTTTGGCGATCTTGGATAATCAAAATCTT
GGAAAACTCGCTGAGTGTGCACAGTATCAAGCCCTGCTGCAAAAAAATCTGATGTATTTAGCCGCA
ATTGCTGATGCACAACCCCAGACCCCACCCATGCCACCTCAGATGTCCCCACATCCGGCTATGCAA
CAAGGAGCATATTACATGCAACATCCTCAGGCTGCAGCAGCAGCAATGGCTCATCAGTCGGGTATT
TTCCCACCAAAGATGTCTCCGTTACAATTCAATAATCCTCATCAAATACAGGACCCCCAGCAGTTA
CATCAAGCAGCCCTCCAAGGGCAAATGGGAATGAGGCCCATGGGGCCCAATAACGGGATGCATCCG
ATGCACCCCGAGGCAAATCTTGGAGGATCTAATGATGGTCGTGGAGGAAACAAACAGGATGCTCCG
GAGACGGGAGCATCGGGAGGTGATGGCAAGGCAATTCTGGTGGTGATGGGGCTGAAGATGGGAAA
TGA

**SEQ ID NO: 153 Euphorbia esula Eupes_SYT2 translated polypeptide sequence**

MQQQPQMMPMMPSYPPANITTEQIQKYLDENKKLILAILDNQNLGKLAECAQYQALLQKNLMYLAA
IADAQPQTPPMPPQMSPHPAMQQGAYYMQHPQAAAAMAHQSGIFPPKMSPLQFNNPHQIQDPQQL
HQAALQGQMGMRPMGPNNGMHPMHPEANLGGSNDGRGGNKQDAPETGASGGDGQGNSGGDGAEDGK

**SEQ ID NO: 154 Fragaria vesca Frave_SYT2 nucleic acid sequence DY668312**

ATGCAGCAGCAGCCACAGCAGATGATGCCCAACATGACTTCGCTTCCTCCCAATACCATCACCACC
GAGCAAATTCAGAAGTGCCTTGATGAGAACAAAAAGTTGATTCTAGCAATATTGGACAATCAAAAC
CTTGGAAAACTTGCTGAGTGTGCCCAGTACCAAACTCAGCTTCAAAAGAATCTCATGTATTTAGCA
GCAATTGCTGATGCACAACCACAACCACAACCACAAGCACCAGCAATGCCGGCCCAGCAGCTGGCC
CCGCATCCTGCGATGCAACAAGCTGGATATTACATGCAGCATCCTCAGGCTGCAGCAGCAATGGCT
CAGCAACAGGGTCTTTTCCCTCAAAAGATGCAAATGCAGTTTAATAGCCCACAACAAATGCACGAG
ATGCAGCAGCAGTTACACCAACAGGCCATGCATGGCCAGATGGGGATGAGACCTGGAGGGGCCAAT
GGGATGCCTTCAATGCATCATACTGAGAACACCCATGGAGGAAGCAAGCAAGACAACTCAGAGGCT
GGGGCAGGTGGTGATGGCCAGGGGAACTCAGCCGGTGGCCACAGAAGTGGCGACGGAGAGGACAAG
TGA

FIGURE 10 (continued)

**SEQ ID NO: 155 Fragaria vesca Frave_SYT2 translated polypeptide sequence**
MQQQPQQMMPNMTSLPPNTITTEQIQKCLDENKKLILAILDNQNLGKLAECAQYQTQLQKNLMYLA
AIADAQPQPQPQAPAMPAQQLAPHPAMQQAGYYMQHPQAAAAMAQQQGLFPQKMQMQFNSPQQMHE
MQQQLHQQAMHGQMGMRPGGANGMPSMHHTENTHGGSKQDNSEAGAGGDGQGNSAGGHRSGDGEDK

**SEQ ID NO: 156 Glycine max Glyma_SYT1.1 nucleic acid sequence TA55102_3847**
ATGCAGCAGCACCTGATGCAGATGCAGCCCATGATGGCTGCCTACTACCCCAACAACGTCACCACT
GATCACATTCAACAGTACCTGGATGAGAACAAGTCCTTGATTCTGAAGATTGTTGAAAGCCAGAAT
TCTGGCAAGCTGAGCGAGTGTGCCGAGAACCAATCAAGGCTGCAGAGAAATCTCATGTACCTAGCT
GCAATAGCTGATTCTCAACCACAACCATCTCCATTGGCTGGTCAGTATCCTTCTAGTGGACTTGTG
CAGCAGGGAGCACACTACATGCAGGCTCAACAGGCTCAGCAGATGTCACAACAACAGCTAATGGCT
TCGCGCTCCTCGCTCCTGTACTCCCAACAGCCTTTCTCAGTGCTTCAACAGCAGCAAGGCATGCAC
AGCCAACTTGGCATGAGCTCCAGTGGAAGTCAAGGCCTCCACATGCTGCAAAGTGAAGCCACTAAT
GTTGGAGGCAATGCAACCATAGGAACCGGAGGAGGGTTTCCGGACTTTGTACGCATTGGTAGTGGC
AAGCAAGATATTGGAATCTCTGGTGAAGGCAGAGGAGGAAACTCTAGTGGCCACTCTGGTGATGGT
GGTGAGACACTTAATTACCTGAAAGCTGCTGGTGATGGAAACTGA

**SEQ ID NO: 157 Glycine max Glyma_SYT1.1 translated polypeptide sequence**
MQQHLMQMQPMMAAYYPNNVTTDHIQQYLDENKSLILKIVESQNSGKLSECAENQSRLQRNLMYLA
AIADSQPQPSPLAGQYPSSGLVQQGAHYMQAQQAQQMSQQQLMASRSSLLYSQQPFSVLQQQQGMH
SQLGMSSSGSQGLHMLQSEATNVGGNATIGTGGGFPDFVRIGSGKQDIGISGEGRGGNSSGHSGDG
GETLNYLKAAGDGN

**SEQ ID NO: 158 Glycine max Glyma_SYT1.2 nucleic acid sequence TA51451_3847**
ATGCAGCAGCACCTGATGCAGATGCAGCCCATGATGGCAGGCTACTACCCCAACAACGTCACCACT
GATCACATTCAGCAGTATCTGGATGAGAACAAGTCCTTAATTCTGAAGATTGTTGAAAGCCAGAAT
TCTGGCAAGCTGAGCGAGTGTGCCGAGAACCAAGCAAGGCTTCAGAGAAATCTCATGTACTTAGCT
GCAATAGCTGATTCTCAACCCCAACCACCCACCATGTCTGGTCAGTACCCTCCGAGTGGGATGATG
CAGCAGGGAGCACAGTACATGCAGGCTCAACAACAGGCACAGCAGATGACACCACAACAACTAATG
GCAGCACGCTCATCTCTTTTGTACGCACAGCAGCCGTACTCAGCACTTCAACAGCAGCAAGCCATG
CACAGTGCACTGGGGTCGAGTTCGGGGCTCCACATGCTGCAAAGTGAAGGCAGCAATGTGAATGTG
GGAGGAGGGTTTCCTGACTTTGTGCGTGGCGGCAGCTCCACAGGGGAGGGTTTGCACAGTGGTGGA
AGGGGTATCATTGGAAGTAGCAAGCAGGAAATGGGGGGTTCAAGTGAAGGCCGCGGTGAAGGGGGT
GAAAACCTCTACCTCAAAGTTGCTGATGATGGAAACTAG

**SEQ ID NO: 159 Glycine max Glyma_SYT1.2 translated polypeptide sequence**
MQQHLMQMQPMMAGYYPNNVTTDHIQQYLDENKSLILKIVESQNSGKLSECAENQARLQRNLMYLA
AIADSQPQPPTMSGQYPPSGMMQQGAQYMQAQQQAQQMTPQQLMAARSSLLYAQQPYSALQQQQAM
HSALGSSSGLHMLQSEGSNVNVGGGFPDFVRGGSSTGEGLHSGGRGIIGSSKQEMGGSSEGRGEGG
ENLYLKVADDGN

## FIGURE 10 (continued)

**SEQ ID NO: 160 Glycine max Glyma_SYT2.1 nucleic acid sequence BQ612648**

ATGCAGCAGACACCGCCAATGATTCCTATGATGCCTTCTTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACCCCGGCCATGCCTCCGCAGATGGCACCGCACCCTGCCATGCAA
CCAGGATTCTATATGCAACATCCTCAGGCTGCTGCAGCAGCAATGGCTCAGCAGCAGCAAGGAATG
TTCCCCCAGAAAATGCCATTGCAATTTGGCAATCCACATCAAATGCAGGAACAACAACAGCAGCTA
CACCAGCAGGCCATCCAAGGTCAAATGGGACTTAGACCTGGAGATATAAATAATGGCATGCATCCA
ATGCACAGTGAGGCTGCTCTTGGAGGTGGAAACAGCGGTGGTCCACCTTCGGCTACTGGTCCAAAC
GATGCACGTGGTGGAAGCAAGCAAGATGCCTCTGAGGCTGGAACAGCTGGTGGAGACGGCCAAGGC
AGCTCCGCGGCTGCTCATAACAGTGGAGATGGTGAAGAGGCAAAGTGA

**SEQ ID NO: 161 Glycine max Glyma_SYT2.1 translated polypeptide sequence**

MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQGMFPQKMPLQFGNPHQMQEQQQQL
HQQAIQGQMGLRPGDINNGMHPMHSEAALGGGNSGGPPSATGPNDARGGSKQDASEAGTAGGDGQG
SSAAAHNSGDGEEAK

**SEQ ID NO: 162 Glycine max Glyma_SYT2.2 nucleic acid sequence TA48452_3847**

ATGCAGCAGACACCGCCTATGATTCCTATGATGCCTTCGTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACACCAGCCATGCCTCCACAGATGGCACCACACCCTGCCATGCAA
CCAGGATTCTATATGCAACATCCTCAGGCTGCAGCAGCAGCAATGGCTCAGCAGCAGCAGCAAGGA
ATGTTCCCCCAGAAAATGCCATTGCAATTTGGCAATCCACATCAAATGCAGGAACAACAGCAGCAG
CTACACCAGCAAGCCATCCAAGGTCAAATGGGACTGAGACCTGGAGGAATAAATAATGGCATGCAT
CCAATGCACAATGAGGGCGGCAACAGCGGTGGTCCACCCTCGGCTACCGGTCCGAACGACGCACGT
GGTGGAAGCAAGCAAGATGCTTCTGAGGCTGGAACAGCTGGTGGAGATGGCCAAGGCAGCTCTGCA
GCTGCTCATAACAGTGGAGATGGTGAAGAGGCAAAGTGA

**SEQ ID NO: 163 Glycine max Glyma_SYT2.2 translated polypeptide sequence**

MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQQGMFPQKMPLQFGNPHQMQEQQQQ
LHQQAIQGQMGLRPGGINNGMHPMHNEGGNSGGPPSATGPNDARGGSKQDASEAGTAGGDGQGSSA
AAHNSGDGEEAK

**SEQ ID NO: 164 Glycine soya Glyso_SYT2 nucleic acid sequence CA799921**

ATGCAGCAGACACCGCCTATGATTCCTATGATGCCTTCGTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACACCAGCCATGCCTCCACAGATGGCACCACACCCTGCCATGCAA
CCAGGATTCTATATGCAACATCCTCAGGCTGCAGCAGCAGCAATGGCTCAGCAGCAGCAGCAAGGA
ATGTTCCCCCAGAAAATGCCATTGCAATTTGGCAATCCACATCAAATGCAGGAACAACAGCAGCAG

**FIGURE 10 (continued)**

CTACACCAGCAAGCCATCCAAGGTCAAATGGGACTGAGACCTGGAGGAATAAATAATGGCATGCAT
CCAATGCACAATGAGGGCGGCAACAGCGGTGGTCCACCCTCGGCTACCGGTCCGAACGACGCACGT
GGTGGAAGCAAGCAAGATGCTTCTGAGGCTGGAACAGCTGGTGGAGATGGCCAAGGCAGCTCTGCA
GCTGCTCATAACAGTGGAGATGGTGAAGAGGCAAAGTGA

**SEQ ID NO: 165 Glycine soya Glyso_SYT2 translated polypeptide sequence**
MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQQGMFPQKMPLQFGNPHQMQEQQQQ
LHQQAIQGQMGLRPGGINNGMHPMHNEGGNSGGPPSATGPNDARGGSKQDASEAGTAGGDGQGSSA
AAHNSGDGEEAK

**SEQ ID NO: 166 Gossypium arboreum Gosar_SYT nucleic acid sequence BM359324**
ATGCAGCAGCACCTGATGCAGATGCAGCCCATGATGGCAGCTTATTATCCCAACAACGTCACTACT
GATCATATTCAACAGTATCTCGATGAGAACAAGTCATTGATCTTAAAGATTGTTGAGAGCCAGAAT
TCTGGGAAATTGAGTGAATGTGCTGAGAACCAAGCAAGGCTGCAGCGAAACCTCATGTACCTGGCT
GCCATTGCGGATTCTCAACCCCAACCACCCACCGTGCATGCACAGTTTCCATCTGGTGGTATCATG
CAGCAAGGAGCTGGGCACTACATGCAGCACCAACAAGCTCAACANATGACACAACAGTCGCTTATG
GCTGCTCGGTCCTCAATGTTGTATTCTCAGCAACCATTTTCTGCACTGCAACAACAACAACAACAA
GGCTTTGCACAGTCAGCTTGGCATGAGCTCTGGCGGGAGCACAGGCCTTTCATATGCTGCAAACTG
AATCTAGTACTGCAGGGGGCAGTGAGACACCTTGGGCCCGAGGGTTGTCCTGATTTGGACGGGGGT
CTTTTGGAGAGGCATCCCTGGTGGCAGGCCAATGGCCGGGGGAACAACCAAAAATCCGGGGAGGCC
GGCTCACCTAAGGGCCGGGAGGAGCCCTTGGGGCAGGGGGGGGTGATGGGGGGAACCTCTTCTTAA

**SEQ ID NO: 167 Gossypium arboreum Gosar_SYT translated polypeptide sequence**
MQQHLMQMQPMMAAYYPNNVTTDHIQQYLDENKSLILKIVESQNSGKLSECAENQARLQRNLMYLA
AIADSQPQPPTVHAQFPSGGIMQQGAGHYMQHQQAQXMTQQSLMAARSSMLYSQQPFSALQQQQQQ
GFAQSAWHELWREHRPFICCKLNLVLQGAVRHLGPEGCPDLDGGLLERHPWWQANGRGNNQKSGEA
GSPKGREEPLGQGGVMGGTSS

**SEQ ID NO: 168 Gossypium hirsutum Goshi_SYT1 nucleic acid sequence DT558852**
ATGCAGCAGCACCTGATGCAGATGCAGCCCATGATGGCAGCTTATTATCCCAACAACGTCACTACT
GATCATATTCAACAGTATCTCGATGAGAACAAGTCATTGATCTTAAAGATTGTTGAGAGCCAGAAT
TCTGGGAAATTGAGTGAATGTGCTGAGAACCAAGCAAGGCTGCAGCGAAACCTCATGTACCTGGCT
GCCATTGCGGATTCTCAACCCCAACCACCCACCGTGCATGCACAGTTTCCATCTGGTGGTATCATG
CAGCCAGGAGCTGGGCACTACATGCAGCACCAACAAGCTCAACAAATGACACAACAGTCGCTTATG
GCTGCTCGGTCCTCAATGTTGTATTCTCAGCAACCATTTTCTGCACTGCAACAACAACAGCAGCAA
GCTTTGCACAGTCAGCTTGGCATGAGCTCTGGCGGAAGCACAGGCCTTCATATGCTGCAAACTGAA
TCTAGTACTGCAGGTGGCAGTGGAGCACTTGGGGCCGGAGGGTTTCCTGATTTTGGACGTGGTTCT
TCTGGAGAAGGCATCCATGGTGGCAGGCCAATGGCAGGTGGAAGCAAGCAAGATATCGGGAGTGCC
GGCTCAGCTGAAGGTCGTGGAGGAAGCTCTGGTGGTCAGGGTGGTGGTGATGGGGGTGAAACCCTT
TACTTAAAAGCAGCCGATGATGGGAACTGA

**FIGURE 10 (continued)**

**SEQ ID NO: 169 Gossypium hirsutum Goshi_SYT1 translated polypeptide sequence**

MQQHLMQMQPMMAAYYPNNVTTDHIQQYLDENKSLILKIVESQNSGKLSECAENQARLQRNLMYLA
AIADSQPQPPTVHAQFPSGGIMQPGAGHYMQHQQAQQMTQQSLMAARSSMLYSQQPFSALQQQQQQ
ALHSQLGMSSGGSTGLHMLQTESSTAGGSGALGAGGFPDFGRGSSGEGIHGGRPMAGGSKQDIGSA
GSAEGRGGSSGGQGGGDGGETLYLKAADDGN

**SEQ ID NO: 170 Gossypium hirsutum Goshi_SYT2 nucleic acid sequence DT563805**

ATGCCGCAGCCACCGCAAATGATTCCTGTGATGCCTTCATATCCACCTACTAATATCACTACTGAA
CAGATTCAGAAGTACCTTGATGAGAATAAGAAGTTGATTTTGGCAATTTTGGACAATCAGAATCTT
GGAAAACTCGCTGAATGCGCCCAGTATCAAGCTCAGCTGCAAAAGAATTTGATGTATTTAGCTGCA
ATTGCGGATGCTCAACCTCAATCAACGCCAGCAATGTCGCCTCAGATGGCACCGCATCCAGCAATG
CAACCCGGAGGATATTTTATGCAACATCCTCAAGCTGCTGCAATGTCACAGCAACCTGGCATGTAC
CCTCAAAAGGTGCCATTGCAATTCAATAGTCCGCATCAAATGCAGGACCCTCAGCACCTCCTATAT
CAGCAGCATCAACAAGCAATGCAAGGTCAAATGGGAATCAGGCCTGGGGGACCCAATAATAGCATG
CATCCCATGCATTCAGAGGCTAGCCTTGGAGGCGGCAGCAGTGGTGGTCCCCCTCAACCTTCAGGC
CCAAGTGATGGACGTGCTGGAAACAAGCAAGAGGGCTCCGAAGCTGGTGGTAATGGGCAGGGCAGC
ACAACTGGTGGGCATGGTGGCGGTGATGGAGCGGATGAGGCAAAGTGA

**SEQ ID NO: 171 Gossypium hirsutum Goshi_SYT2 transalted polypeptide sequence**

MPQPPQMIPVMPSYPPTNITTEQIQKYLDENKKLILAILDNQNLGKLAECAQYQAQLQKNLMYLAA
IADAQPQSTPAMSPQMAPHPAMQPGGYFMQHPQAAAMSQQPGMYPQKVPLQFNSPHQMQDPQHLLY
QQHQQAMQGQMGIRPGGPNNSMHPMHSEASLGGGSSGGPPQPSGPSDGRAGNKQEGSEAGGNGQGS
TTGGHGGGDGADEAK

**SEQ ID NO: 172 Helianthus annuus Helan_SYT1 nucleic acid sequence TA12738_4232**

ATGCAGCAGCACCTGATGCAGATGCAGCCCATGATGGCAGCCTATTATCCCACCAACAACGTCACT
ACTGATCATATTCAACAGTACTTGGATGAAAACAAGTCTCTGATCTTGAAGATTGTTGAGAGCCAA
AACTCTGGGAAAATGGCTGAATGTGCAGAACATCAGGCCAAGCTTCAGAGAAACCTTATGTACCTT
GCTGCAATTGCTGATTCTCAACCTCAAGCACCTAGTCTTCACTCTCAGTATCCTCAAGGTGGGATG
ATGCAGCAGCAGGCTGGAAGTCACTACATGCAGCAGCACCAACAGGCACAACAGATGTCACCACAA
GCACTCATGGCTGCACGCTCATCCATGATGTACAGTCAGCAGCAGTACTCTTCACTACAGCAGCAA
GCAATGCATAGCCATCTGGGCATGAGTTCTGGAACTGGAACCAGTGGACTTCACATGCTGCAGACC
GACAATAATAGCGCGGGTGTCAGTGGGACCCACCTAAGTGGTGGGTTCCCCGACTTTGGTCGTAAG
CAAGATATTGGTCCCACCGGTGAGGGGCGGGGTGGTGGTAGCTCTGGCGGTGGAGACGGTGGCGAG
ACGCTCTACTTGAAGTCGCCTGATAAAGGTAACTGA

**SEQ ID NO: 173 Helianthus annuus Helan_SYT1 translated polypeptide sequence**

MQQHLMQMQPMMAAYYPTNNVTTDHIQQYLDENKSLILKIVESQNSGKMAECAEHQAKLQRNLMYL
AAIADSQPQAPSLHSQYPQGGMMQQQAGSHYMQQHQQAQQMSPQALMAARSSMMYSQQQYSSLQQQ
AMHSHLGMSSGTGTSGLHMLQTDNNSAGVSGTHLSGGFPDFGRKQDIGPTGEGRGGGSSGGGDGGE
TLYLKSPDKGN

FIGURE 10 (continued)

**SEQ ID NO: 174 Hordeum vulgare Horvu_SYT2 nucleic acid sequence CA032350**

ATGCAGCAAGCGATGCCCATGCCGCCGGCGGCGGCGGCGCCTGGGATGCCTCCTTCTGCCGGCCTC
AGCACCGAGCAGATCCAAAAGTACCTGGATGAAAATAAACAACTAATTTTGGCTATCTTGGAAAAT
CAGAACCTGGGAAAGTTGGCGGAATGTGCTCAGTATCAAGCTCAGCTTCAGAAGAATCTTTTGTAT
TTGGCTGCGATTGCTGATACTCAGCCACAGACCTCTGTAAGCCGTCCTCAGATGGCACCACCTGCT
GCATCCCCAGGGGCAGGGCATTACATGTCACAGGTGCCAATGTTCCCTCCGAGGACCCCTCTAACG
CCTCAGCAGATGCAGGAGCAGCAACTACAGCAACAACAGGCTCAGATGCTTCCGTTTGCTGGTCAA
ATGGTTGCGAGACCCGGGGCTGTCAATGGCATTCCCCAGGCCCCTCAAGTTGAACAACCAGCCTAT
GCAGCAGGTGGGGCCAGTTCCGAGCCTTCTGGCACCGAGAGCCACAGGAGCACTGGCGCCGATAAC
GATGGTGGGAGCGGCTTGGCTGACCAGTCCTAA

**SEQ ID NO: 175 Hordeum vulgare Horvu_SYT2 translated polypeptide sequence**

MQQAMPMPPAAAAPGMPPSAGLSTEQIQKYLDENKQLILAILENQNLGKLAECAQYQAQLQKNLLY
LAAIADTQPQTSVSRPQMAPPAASPGAGHYMSQVPMFPPRTPLTPQQMQEQQLQQQQAQMLPFAGQ
MVARPGAVNGIPQAPQVEQPAYAAGGASSEPSGTESHRSTGADNDGGSGLADQS

**SEQ ID NO: 176 Lactuca serriola Lacse_SYT1 nucleic acid sequence DW110765**

ATGAAGCAGCCGATGATGCCGAATCCAATGATGTCTTCTTCGTTTCCTCCTACAAACATCACCACC
GATCAGATCCAAAAGTTCCTTGATGAAAACAAGCAACTAATTATAGCAATAATGAGCAACCTAAAT
CTTGGAAAGCTTGCTGAATGTGCCCAGTACCAAGCTCTACTCCAAAAAAATTTGATGTATCTAGCA
GCCATTGCAGATGCTCAACCACCTACACCTACACCAACACTAAATATCTCTTATNAGATGGGCCCG
GTTCCACATCCAGGGATGCCACAGCAAGGTGGATTTTACATGGCGCAGCAGCACCCTCAGGCGGCT
GTAATGACGGCTCAGCCACCTTCTGGTTTTCCACAACCGATGCCTGGTATGCAATTTAACAGCCCA
CAGGCTATTCAAGGGCAGATGGGCGGGAGGTCCGGTGGGCCGCCAAGCTCAGCCGCTAGTGATGTC
TGGAGAGGAAGCATGCAAGATGGTGGTGGTGGTGCTGCTGCTGATGGTGGTAAGGATGGTCATGCT
GGCGGTGGACCTGAGGAAGCAAAGTAA

**SEQ ID NO: 177 Lactuca serriola Lacse_SYT1 translated polypeptide sequence polypeptide**

MKQPMMPNPMMSSSFPPTNITTDQIQKFLDENKQLIIAIMSNLNLGKLAECAQYQALLQKNLMYLA
AIADAQPPTPTPTLNISYXMGPVPHPGMPQQGGFYMAQQHPQAAVMTAQPPSGFPQPMPGMQFNSP
QAIQGQMGGRSGGPPSSAASDVWRGSMQDGGGAAADGGKDGHAGGGPEEAK

**SEQ ID NO: 178 Lycopersicon esculentum Lyces_SYT1 nucleic acid sequence contig of AW934450.1 BP893155.1**

ATGCAGCAGCACCTGATGCAGATGCAGCCCATGATGGCAGCTTACTATCCAACGAACGTCACTACT
GACCATATTCAACAGTATTTGGATGAAAACAAATCACTCATTCTGAAGATTGTTGAGAGCCAGAAC
TCTGGGAAACTCAGTGAATGTGCGGAGAACCAAGCTAGGCTTCAGAGGAATCTGATGTACCTTGCT
GCGATTGCTGATTCACAACCTCAACCTTCTAGCATGCATTCTCAGTTCTCTTCTGGTGGGATGATG
CAGCCAGGGACACACAGTTACTTGCAGCAGCAGCAGCAGCAACAACAAGCGCAACAAATGGCAACA
CAACAACTCATGGCTGCAAGATCCTCGTCGATGCTCTATGGACAACAGCAGCAGCAATCTCAGTTA
TCGCAATATCAACAAGGCTTGCATAGTAGCCAACTCGGCATGAGTTCTGGCAGTGGCGGAAGCACT
GGACTTCATCACATGCTTCAAAGTGAATCATCACCTCATGGTGGTGGTTTCTCTCATGACTTCGGC
CGCGCAAATAAGCAAGACATTGGGAGTAGTATGTCTGCTGAAGGGCGCGGCGGAAGTTCAGGTGGT
GAGAATCTTTATCTGAAAGCTTCTGAGGATTGA

## FIGURE 10 (continued)

**SEQ ID NO: 179 Lycopersicon esculentum Lyces_SYT1 translated polypeptide sequence**

MQQHLMQMQPMMAAYYPTNVTTDHIQQYLDENKSLILKIVESQNSGKLSECAENQARLQRNLMYLA
AIADSQPQPSSMHSQFSSGGMMQPGTHSYLQQQQQQQAQQMATQQLMAARSSSMLYGQQQQQSQL
SQYQQGLHSSQLGMSSGSGGSTGLHHMLQSESSPHGGGFSHDFGRANKQDIGSSMSAEGRGGSSGG
ENLYLKASED

**SEQ ID NO: 180 Malus domestica Maldo_SYT2 nucleic acid sequence contig of CV084230 DR997566**

ATGCAGCAGCCACCACAAATGATCCCCGTCATGCCTTCATTTCCTCCCACCAACATCACCACCGAA
CAAATTCAGAAGTACCTTGATGACAACAAAAAGTTGATTCTGGCAATATTGGATAATCAAAATCTT
GGAAAACTTGCTGAGTGTGCTCAGTACCAGGCTCTGCTTCAAAAGAATCTGATGTATTTAGCAGCA
ATTGCCGATGCGCAACCACAGGCACCAGCTGCCCCTCCCCAGATGGCCCCACATCCTGCTATGCAA
CAGGCAGGATATTACATGCAACATCCTCAGGCAGCAGCAATGGCTCAGCAACAGGGTATTTTCTCC
CCAAAGATGCCGATGCAATTCAATAACATGCATCAAATGCACGATCCACAGCAGCACCAACAAGCC
ATGCAAGGGCAAATGGGAATGAGACCTGGAGGGCCTAACGGCATGCCTTCCATGCTTCATACTGAG
GCCACACATGGTGGTGGTAGTGGCGGCCCAAATTCAGCTGGAGACCCAAATGATGGGCGTGGAGGA
AGCAAGCAAGACGCCTCTGAGTCTGGGGCAGGTGGTGATGGCCAGGGGACCTCAGCCGGCGGGCGT
GGAACTGGTGATGGAGAGGACGGCAAGTGA

**SEQ ID NO: 181 Malus domestica Maldo_SYT2 translated polypeptide sequence**

MQQPPQMIPVMPSFPPTNITTEQIQKYLDDNKKLILAILDNQNLGKLAECAQYQALLQKNLMYLAA
IADAQPQAPAAPPQMAPHPAMQQAGYYMQHPQAAAMAQQQGIFSPKMPMQFNNMHQMHDPQQHQQA
MQGQMGMRPGGPNGMPSMLHTEATHGGGSGGPNSAGDPNDGRGGSKQDASESGAGGDGQGTSAGGR
GTGDGEDGK

**SEQ ID NO: 182 Medicago truncatula Medtr_SYT1 nucleic acid sequence CA858507**

ATGCAGCAGCACCTGATGCAGATGCAGCCCATGATGGCAGCTTACTATCCTAACAACGTCACTACT
GATCATATTCAACAGTATCTTGATGAGAACAAGTCCTTGATTCTCAAGATTGTTGAAAGCCAGAAC
ACTGGCAAGCTCACCGAGTGTGCTGAGAACCAATCAAGGCTTCAGAGAAATCTCATGTACCTAGCT
GCAATAGCTGATTCTCAACCCCAACCACCTACTATGCCTGGCCAGTACCCTTCAAGTGGAATGATG
CAGCAGGGAGGACACTACATGCAGGCTCAACAAGCTCAGCAGATGACACAACAACAATTAATGGCT
GCACGTTCCTCTCTTATGTATGCTCAACAGCTTCAACAGCAGCAAGCCTTGCAAAGCCAACTTGGT
ATGAATTCCAGTGGAAGTCAAGGCCTTCACATGTTGCATAGTGAAGGGGCTAATGTTGGAGGCAAT
TCATCTCTAGGGGCTGGTTTTCCTGATTTTGGCCGTAGCTCAGCCGGTGATGGTTTGCACGGCAGT
GGTAAGCAAGACATTGGAAGCACTGATGGCCGCGGTGGAAGCTCTAGTGGTCACTCTGGTGATGGC
GGCGAAACACTTTACCTGAAATCTTCTGGTGATGGGAATTAG

**SEQ ID NO: 183 Medicago truncatula Medtr_SYT1 translated polypeptide sequence**

MQQHLMQMQPMMAAYYPNNVTTDHIQQYLDENKSLILKIVESQNTGKLTECAENQSRLQRNLMYLA
AIADSQPQPPTMPGQYPSSGMMQQGGHYMQAQQAQQMTQQQLMAARSSLMYAQQLQQQQALQSQLG
MNSSGSQGLHMLHSEGANVGGNSSLGAGFPDFGRSSAGDGLHGSGKQDIGSTDGRGGSSSGHSGDG
GETLYLKSSGDGN

FIGURE 10 (continued)

**SEQ ID NO: 184 Medicago trunculata Medtr_SYT2 nucleic acid sequence contig of CA858743 BI310799.1 AL382135.1**
ATGCAGCAGACACCTCAAATGATTCCTATGATGCCTTCATTCCCACAACAAACAAACATAACCACT
GAGCAGATTCAAAAATATCTTGATGAGAACAAGAAGCTGATCCTGGCAATATTGGACAATCAAAAT
CTTGGAAAACTTGCAGAATGTGCCCAGTACCAAGCTCAGCTTCAGAAGAATTTGATGTATTTAGCT
GCAATTGCTGACGCGCAGCCACAAACACCGGCCTTGCCTCCACAGATGGCCCCGCACCCTGCGATG
CAACAAGGATTCTATATGCAACATCCTCAGGCTGCAGCAATGGCTCAGCAACAAGGAATGTTCCCC
CAAAAAATGCCAATGCAGTTCGGTAATCCGCATCAAATGCAGGATCAGCAGCATCAGCAGCAACAA
CAGCAGCTACATCAGCAAGCTATGCAAGGTCAAATGGGACTTAGACCTGGAGGGATAAATAACGGC
ATGCATCCAATGCACAACGAGGCTGCTCTCGGAGGTAGCGGCAGTGGTGGTCAAATGACGGGCGTG
GTGGTGGAGCAAGCAAGATGCTTCGGAGCTGGGACAGCCGGCGGTGATGGTCAAGGAACCTCTGCC
GCAGCTGCGCACAACAGTGGAGATGCTTCAGAAGAAGGAAAGTAA

**SEQ ID NO: 185 Medicago truncula Medtr_SYT2 translated polypeptide sequence**
MQQTPQMIPMMPSFPQQTNITTEQIQKYLDENKKLILAILDNQNLGKLAECAQYQAQLQKNLMYLA
AIADAQPQTPALPPQMAPHPAMQQGFYMQHPQAAAMAQQQGMFPQKMPMQFGNPHQMQDQQHQQQQ
QQLHQQAMQGQMGLRPGGINNGMHPMHNEAALGGSGSGGPNDGRGGGSKQDASEAGTAGGDGQGTS
AAAAHNSGDASEEGK

**SEQ ID NO: 186 Oryza sativa Orysa_SYT1 nucleic acid sequence (AK058575)**
ATGCAGCAGCAACACCTGATGCAGATGAACCAGGGCATGATGGGGGGATATGCTTCCCCTACCACC
GTCACCACTGATCTCATTCAGCAGTATCTGGATGAGAACAAGCAGCTGATCCTGGCCATCCTTGAC
AACCAGAACAATGGGAAGGTGGAAGAGTGCGCTCGGAACCAAGCTAAGCTCCAGCACAATCTCATG
TACCTCGCCGCCATCGCCGACAGCCAGCCGCCGCAGACGGCCGCCATGTCCCAGTATCCGTCGAAC
CTGATGATGCAGTCCGGGGCGAGGTACATGCCGCAGCAGTCGGCGCAGATGATGGCGCCGCAGTCG
CTGATGGCGGCGAGGTCTTCGATGATGTACGCGCAGCCGGCGCTGTCGCCGCTCCAGCAGCAGCAG
CAGCAGCAGGCGGCGGCGGCGCACGGGCAGCTGGGCATGGGCTCGGGGGGCACCACCAGCGGGTTC
AGCATCCTCCACGGCGAGGCCAGCATGGGCGGCGGCGGCGGCGGCGGTGGCGCCGGTAACAGCATG
ATGAACGCCGGCGTGTTCTCCGACTTCGGACGCGGCGGCGGCGGCGGCGGCAAGGAGGGGTCCACC
TCGCTGTCCGTCGACGTCCGGGGCGCCAACTCCGGCGCCCAGAGCGGCGACGGGGAGTACCTCAAG
GGCACCGAGGAGGAAGGCAGCTAG

**SEQ ID NO: 187 Oryza sativa Orysa_SYT1 translated polypeptide sequence**
MQQQHLMQMNQGMMGGYASPTTVTTDLIQQYLDENKQLILAILDNQNNGKVEECARNQAKLQHNLM
YLAAIADSQPPQTAAMSQYPSNLMMQSGARYMPQQSAQMMAPQSLMAARSSMMYAQPALSPLQQQQ
QQQAAAAHGQLGMGSGGTTSGFSILHGEASMGGGGGGGAGNSMMNAGVFSDFGRGGGGGGKEGST
SLSVDVRGANSGAQSGDGEYLKGTEEEGS

**SEQ ID NO: 188 Oryza sativa Oyrsa_SYT2 nucleic acid sequence AK105366**
ATGCAGCAGCAGCCGATGCCGATGCCCGCGCAGGCGCCGCCGACGGCCGGAATCACCACCGAGCAG
ATCCAAAAGTATCTGGATGAAAACAAGCAGCTTATTTTGGCTATTTTGGAAAATCAGAATCTGGGA
AAGTTGGCAGAATGTGCTCAGTATCAAGCGCAGCTTCAGAAGAATCTCTTGTACTTGGCTGCAATT
GCTGATACTCAACCGCAGACCACTATAAGCCGTCCCCAGATGGTGCCGCATGGTGCATCGCCGGGG
TTAGGGGGGCAATACATGTCGCAGGTGCCAATGTTCCCCCCCAGGACCCCTCTAACGCCCCAGCAG

**FIGURE 10 (continued)**

ATGCAGGAGCAGCAGCTGCAGCAACAGCAAGCCCAGCTGCTCTCGTTCGGCGGTCAGATGGTTATG
AGGCCTGGCGTTGTGAATGGCATTCCTCAGCTTCTGCAAGGCGAAATGCACCGCGGAGCAGATCAC
CAGAACGCTGGCGGGGCCACCTCGGAGCCTTCCGAGAGCCACAGGAGCACCGGCACCGAAAATGAC
GGTGGAAGCGACTTCGGCGATCAATCCTAA

**SEQ ID NO: 189 Oryza sativa Orysa_SYT2 translated polypeptide sequence**

MQQQPMPMPAQAPPTAGITTEQIQKYLDENKQLILAILENQNLGKLAECAQYQAQLQKNLLYLAAI
ADTQPQTTISRPQMVPHGASPGLGGQYMSQVPMFPPRTPLTPQQMQEQQLQQQQAQLLSFGGQMVM
RPGVVNGIPQLLQGEMHRGADHQNAGGATSEPSESHRSTGTENDGGSDFGDQS

**SEQ ID NO: 190 Oryza sativa Orysa_SYT3 nucleic acid sequence BP185008**

ATGCAGCAGCAGATGGCCATGCCGGCGGGGGCCGCCGCCGCCGCGGTGCCGCCGGCGGCCGGCATC
ACCACCGAGCAGATCCAAAAGTATTTGGATGAAAATAAACAGCTAATTTTGGCCATCCTGGAAAAT
CAAAACCTAGGGAAGTTGGCTGAATGTGCTCAGTACCAAGCTCAGCTTCAAAAGAATCTCTTGTAT
CTGGCTGCCATTGCAGATGCCCAACCACCTCAGAATCCAGGAAGTCGCCCTCAGATGATGCAGCCT
GGTGCTACCCCAGGTGCTGGGCATTACATGTCCCAAGTACCGATGTTCCCTCCAAGAACTCCCTTA
ACCCCACAACAGATGCAAGAGCAGCAGCAGCAGCAACTCCAGCAACAGCAAGCTCAGGCTCTAGCC
TTCCCCGGCCAGATGCTAATGAGACCAGGTACTGTCAATGGCATGCAATCTATCCCAGTTGCTGAC
CCTGCTCGCGCAGCCGATCTTCAGACGGCAGCACCGGGCTCGGTAGATGGCCGAGGAAACAAGCAG
GATGCAACCTCGGAGCCTTCCGGGACCGAGAGCCACAAGAGTGCGGGAGCAGATAACGACGCAGGC
GGTGACATAGCGGAGAAGTCCTGA

**SEQ ID NO: 191 Oryza sativa Oyrsa_SYT3 translated polypeptide sequence**

MQQQMAMPAGAAAAAVPPAAGITTEQIQKYLDENKQLILAILENQNLGKLAECAQYQAQLQKNLLY
LAAIADAQPPQNPGSRPQMMQPGATPGAGHYMSQVPMFPPRTPLTPQQMQEQQQQQLQQQQAQALA
FPGQMLMRPGTVNGMQSIPVADPARAADLQTAAPGSVDGRGNKQDATSEPSGTESHKSAGADNDAG
GDIAEKS

**SEQ ID NO: 192 Panicum virgatum Panvi_SYT3 nucleic acid sequence DN152517**

ATGCAGCAGCAGATGCCCATGCAGTCGGCGCCCCCGGCGACCGGCATCACCACCGAGCAGATCCAA
AAGTATTTGGATGAAAATAAGCAGCTTATTTTGGCCATCCTGGAAAATCAGAACTTAGGAAAGTTG
GCTGAATGTGCTCAGTATCAAGCTCAGCTTCAAAAGAATCTCTTGTACCTGGCTGCGATTGCAGAT
GCCCAACCCCAACCACCACAGAACCCTGCAAGTCGCCCACAGATGATGCAACCTGGCATGGTACCA
GGTGCAGGGCATTACATGTCCCAAGTACCAATGTTCCCGCCAAGAACACCATTAACCCCGCAACAG
ATGCAAGAACAGCAGCAGCAGCAGCAGCAGCTTCAACAGCAGCAAGCACAGGCTCTTGCTTTCCCG
GGACAGATGGTCATGAGACCTACCATTAATGGCATGCAGCCTATGCAAGCCGACCCTGCTGCCGCC
GCCGCCAGCCTACAGCAGTCAGCACCTGGCCCTACTGATGGGCGAGGAGGCAAGCAAGATGCAACT
GCTGGGGTGAGCACAGAGCCTTCTGGCACCGAGAGCCACAAGAGCACAACCGCAGCAGATCACGAT
GTGGGCACTGATGTCGCGGAGAAATCCTAA

**SEQ ID NO: 193 Panicum virgatum Panvi_SYT3 translated polypeptide sequence**

MQQQMPMQSAPPATGITTEQIQKYLDENKQLILAILENQNLGKLAECAQYQAQLQKNLLYLAAIAD
AQPQPPQNPASRPQMMQPGMVPGAGHYMSQVPMFPPRTPLTPQQMQEQQQQQQLQQQQAQALAFP
GQMVMRPTINGMQPMQADPAAAAASLQQSAPGPTDGRGGKQDATAGVSTEPSGTESHKSTTAADHD
VGTDVAEKS

**FIGURE 10 (continued)**

**SEQ ID NO: 194 Physcomitrella patens Phypa_SYT1.1 nucleic acid sequence TA28566_3218**
ATGCAGCAAATGGCGGCGTATACGGGGACGTCTATTACCACGGAGCTAATCCAGAAGTATCTGGAC
GAGAACAAGCAGCTTATCCTCGCGATTCTTGACAATCAAAACCTTGGAAAGCTGAACGAATGCGCT
ATGTATCAAGCAAAGTTGCAGCAGAATCTTATGTACCTCGCAGCCATTGCGGATGCACAACCTCAA
GTGAGCCAAAACTCAACTCAGGTCTCATCAGGACAATCTATGCAGCCCTCTCAGCAATATATTCAA
CAGCAGCAGCAGCAGCAGATGATGATGATGAATCAGCGAAACTCAATCCCACAATACATGCAACAA
AGCCAACAGGGGTCACCAAACGCACCATCACCGCAGCAGCAGTCCTACCACAGCCAGCAGCCGCAA
GGTATGGTTCCCCAGAGAAACTCAGACATGCACTTGGTGAAAAACTCTACAGGAGGCAACGGTAAT
CAGACAGGAGGTAGTGTATCCGAGTATGGAAAGCCTGAGGAATCCCGGGAGGGGACCCCAACAAGC
TTAAGCACAAGAAACGATGGTCCACAGGCGGGGGCTTCTCCGTTGGGACAAGCGAGAGAAGGCAAT
GGAGCTGCTGGAGAGGACTCTGAGGCTTCTTACTTGAAAAGCTCCGACTAA

**SEQ ID NO: 195 Physcomitrella patens Phypa_SYT1.1 translated polypeptide sequence**
MQQMAAYTGTSITTELIQKYLDENKQLILAILDNQNLGKLNECAMYQAKLQQNLMYLAAIADAQPQ
VSQNSTQVSSGQSMQPSQQYIQQQQQQMMMMNQRNSIPQYMQQSQQGSPNAPSPQQQSYHSQQPQ
GMVPQRNSDMHLVKNSTGGNGNQTGGSVSEYGKPEESREGTPTSLSTRNDGPQAGASPLGQAREGN
GAAGEDSEASYLKSSD

**SEQ ID NO: 196 Physcomitrella patens Phypa_SYT1.2 nucleic acid sequence TA21282_3218**
ATGCAGCAAATGGCGCCGTATGCGGGGACATCTATCACTACTGAGCTCATCCAGAAGTACCTGGAC
GAGAACAAGCAGCTGATTCTCGCAATTCTCGATAATCAAAACCTTGGAAAGCTGAACGAATGTGCT
ACGTATCAAGCAAAGTTGCAGCAGAATCTTATGTACCTCGCAGCTATTGCAGACGCACAACCCCAA
GTTCCAGCAGCACAACCAATGCAACCATCACAGCAATATATTCAGCAGCAGCAGCAGCAGATGATG
ATGAATCAACGAAATCAGTACTTGCAGCAAAATCAACAAGGAGTACAAAACGCACCATCACCGCAG
TCGCAGCAGTCGTATCACAACCAGCAGCCAGGCATGGTCTCCCAGGGAAACTCGGGGATGCACATG
GTGAATAGTTCCATGGGTGGTAATGGCAACCAGACAGGAGGAAATGTTTCCGAGTATGGGAAACCA
GAAGATTCCCGGGAGGGGACTCCAACAAGCTTGAATACAAGGAATGAAGGTCCACAAGCGGGGGCT
TCCCCACTGGGACAAGCAAGAGAAGGGAATGGTGCGCCAGGAGAGGATTCAGAGGCTTCATACTTA
AAAAGCTCCGAGTGA

**SEQ ID NO: 197 Physcomitrella patens Phypa_SYT1.2 translated polypeptide sequence**
MQQMAPYAGTSITTELIQKYLDENKQLILAILDNQNLGKLNECATYQAKLQQNLMYLAAIADAQPQ
VPAAQPMQPSQQYIQQQQQQMMMNQRNQYLQQNQQGVQNAPSPQSQQSYHNQQPGMVSQGNSGMHM
VNSSMGGNGNQTGGNVSEYGKPEDSREGTPTSLNTRNEGPQAGASPLGQAREGNGAPGEDSEASYL
KSSE

**SEQ ID NO: 198 Physcomitrella patens Phypa_SYT1.3 nucleic acid sequence TA20922_3218**
ATGATGCAGCACATGGCGACGTATGCCAGCTCCAACATCACAACGGAGCTCATTCAGAAGTACTTG
GACGAGAATAAGCAGTTGATTCTCGCTATCCTCGACAACCAAAACCTCGGCAAGCTCAATGAGTGT
GCAACGTATCAAGCGAAGTTGCAGCAGAATCTCATGTATTTGGCTGCCATAGCTGATGCTCAGCCA
CAAGGCCCATCTTCGCAGATGCCAGCACCTGCGCCGGCGCCAACCATGCAACCAGCTCAGCAGTAC
ATGCAACAGCAGCAACAACTCCGGATGATGAGCCAACAGAACAGTATGATCCCTTCCTACCTTCAG
CACAGCCAACAAGCATCGCAGCAGAACTTCTACAGTCAACAAAGCCTGCTCTCCGGGGGAGGGGGC

**FIGURE 10 (continued)**

TCAATACACATGATGTCCACGGACCGCGGCATCGGAGGCAATGGATCCCAGGCTTCGCCAGGATAT
TCTGATGGAGGGCGGGATCAGAGCCAAATGGGTTTGGCAATGCAGGGCGACATGCATGGTGGAAAC
GGGACGGACCTGGGGTGCTCCTCCCCGCTTGGCCACCGAGGAGACGGTGGTAACGGCCACGGCCAG
GGGACCGATGACTCTGAAGCTTCCTACTTGAAGGGATCTGATGGGAGCAGTCTGAATTAA

**SEQ ID NO: 199 Physcomitrella patens Phypa_SYT1.3 translated polypeptide sequence**
MMQHMATYASSNITTELIQKYLDENKQLILAILDNQNLGKLNECATYQAKLQQNLMYLAAIADAQP
QGPSSQMPAPAPAPTMQPAQQYMQQQQQLRMMSQQNSMIPSYLQHSQQASQQNFYSQQSLLSGGGG
SIHMMSTDRGIGGNGSQASPGYSDGGRDQSQMGLAMQGDMHGGNGTDLGCSSPLGHRGDGGNGHGQ
GTDDSEASYLKGSDGSSLN

**SEQ ID NO: 200 Physcomitrella patens Phypa_SYT1.4 nucleic acid sequence TA29452_3218**
ATGATGCAGCACATGACGACCTATGCCAGCTCCAACATCACCACGGAGCTCATTCAGAAGTACTTG
GACGAGAATAAACAGCTGATTCTCGCCATTCTCGACAACCAAAACCTCGGCAAGCTCAATGAGTGT
GCGACGTATCAAGCGAAGCTGCAGCAGAACCTCATGTATCTGGCCGCTATAGCTGATGCCCAGCCA
CAAGGCCCATCTACGCAGATGCCAGCGCCCGCGCCAACTATGCAACCAGCTCAACAATACATGCAA
CAGCAGCAACAGCTCCGCATGATGAGCCAACAAAATGCCATGATCCCCTCCTACCTGCAGCAAAGC
CAACAAGTTTCCCAGCAGAACTTCTACAGCCAACAGAGCCTGCTTACCGGCGGTGGCAGCTCCATC
CACATGATGTCCACTGATCGCGGCATGGGAGGCAATGGGTCACAAGCCTCACCTGGATATTCTGAC
GGAGGGCGAGATCAGAACCAATTGGGTATGACGATGCAGGGCGACATGCATGGTGGAAACGGCACT
GACTTGGGCTGCTCCTCACCTCTCGGCCACCGGGGAGATGGCGGTGGCGGCCACGGCCAGGGCAAC
GACGACTCTGAAGCTTCTTACTTGAAGGGTTCCGATGGCAGCAGTCTGAACTAG

**SEQ ID NO: 201 Physcomitrella patens Phypa_SYT1.4 translated polypeptide sequence**
MMQHMTTYASSNITTELIQKYLDENKQLILAILDNQNLGKLNECATYQAKLQQNLMYLAAIADAQP
QGPSTQMPAPAPTMQPAQQYMQQQQQLRMMSQQNAMIPSYLQQSQQVSQQNFYSQQSLLTGGGSSI
HMMSTDRGMGGNGSQASPGYSDGGRDQNQLGMTMQGDMHGGNGTDLGCSSPLGHRGDGGGGHGQGN
DDSEASYLKGSDGSSLN

**SEQ ID NO: 202 Picea sitchensis Picsi_SYT1 nucleic acid sequence DR484100 DR478464.1**
ATGCAGCAGCATCTCATGCAAATGCAGCCCATGATGGCGGCATACGCCTCCAACAACATCACCACT
GATCACATCCAGAAGTACCTGGATGAGAACAAGCAGTTGATTCTGGCAATTCTGGACAACCAAAAT
CTTGGAAAGCTCAATGAGTGTGCTCAGTACCAAGCAAAACTTCAGCAGAATTTGATGTATCTGGCT
GCGATTGCTGATTCTCAACCACAAGCACAAACTGCACATGCTCAGATTCCTCCTAATGCAGTGATG
CAGTCTGGTGGGCATTACATGCAGCACCAGCAGGCACAGCAACAAGTGACTCCTCAGTCTCTGATG
GCAGCTAGATCTTCCATGCTGTATTCTCAGCAGCCGATGGCTGCTTTGCATCAAGCTCAGCAACAA
CAGCAGCAGCAGCATCAGCAGCAACAACAATCTCTTCACAGCCAGCTTGGCATAAATTCTGGAGGA
AGCAGTGGATTGCATATGTTGCATGGTGAGACAAACATGGGATGTAATGGGCCTCTCTCATCTGGG
GGCTTCCCTGAATTTGGGCGTGGGTCTGCTACCTCTGCTGAAGGTATGCAGGCCAACAGGGGCTTC
ACTATAGATCGTGGTTCAAATAAGCAGGATGGAGTAGGATCAGAGAATGCCCATCCAGGTGCTGGT
GATGGAAGAGGGAGTTCAACTGGAGGGCAGAATGCAGATGAGTCAGAACCATCATACCTGAAAGCC
TCCGAAGAAGAAGGAAACTAG

FIGURE 10 (continued)

**SEQ ID NO: 203 Picea sitchensis Picsi_SYT1 translated polypeptide sequence**
MQQHLMQMQPMMAAYASNNITTDHIQKYLDENKQLILAILDNQNLGKLNECAQYQAKLQQNLMYLA
AIADSQPQAQTAHAQIPPNAVMQSGGHYMQHQQAQQQVTPQSLMAARSSMLYSQQPMAALHQAQQQ
QQQQHQQQQQSLHSQLGINSGGSSGLHMLHGETNMGCNGPLSSGGFPEFGRGSATSAEGMQANRGF
TIDRGSNKQDGVGSENAHPGAGDGRGSSTGGQNADESEPSYLKASEEEGN

**SEQ ID NO: 204 Pinus taeda Pinta_SYT1 nucleic acid sequence DT625916**
ATGCAGCAGCACCTCATGCAAATGCAGCCCATGATGGCGGCCTACGCCTCCAACAATATCACCACT
GATCACATCCAGAAGTACCTGGATGAGAACAAGCAGTTGATTCTGGCAATTTTGGACAACCAAAAT
CTCGGAAAGCTCAATGAGTGTGCTCAATACCAAGCAAAACTTCAGCAGAATTTGATGTATCTGGCT
GCTATTGCTGATTCTCAACCTCAAGCACAAACTGCACATGCTCAGATTCCTCCAAATGCGGTGATG
CAGTCTGGTGGGCATTACATGCAGCATCAACAGGCACAGCAACAAGTTACTCCTCAGTCTCTGATG
GCAGCTAGATCTTCCATACTGTATGCTCAGCAACAACAGCAGCAGCAGCATCAGCAGCATCAGCAG
CAACAGCAGCAACAACAGTCTCTTCACAGCCAGCTTGGCATAAATTCTGGAGGAAGCAGCGGTTTG
CATATGTTGCATGGTGAGACAAACATGGGATGTAATGGGCCTCTGTCATCTGGGGGATTCCCTGAA
TTTGGGCGTGGGTCTGCTACCTCTGCTGATGGTATGCAGGTGAACAGGGGCTTTGCTATAGATCGT
GGTTCAAACAAGCAGGATGGAGTTGGATCAGAGAATGCCCATGCTGGTGCTGGTGATGGAAGAGGG
AGTTCAACTGGAGGGCAGAATGCAGATGAGTCAGAACCATCATACCTGAAGGCCTCCGAGGAAGAA
GGAAACTAG

**SEQ ID NO: 205 Pinus taeda Pinta_SYT1 translated polypeptide sequence**
MQQHLMQMQPMMAAYASNNITTDHIQKYLDENKQLILAILDNQNLGKLNECAQYQAKLQQNLMYLA
AIADSQPQAQTAHAQIPPNAVMQSGGHYMQHQQAQQQVTPQSLMAARSSILYAQQQQQQHQQHQQ
QQQQQQSLHSQLGINSGGSSGLHMLHGETNMGCNGPLSSGGFPEFGRGSATSADGMQVNRGFAIDR
GSNKQDGVGSENAHAGAGDGRGSSTGGQNADESEPSYLKASEEEGN

**SEQ ID NO: 206 Populus trichocarpa Poptr_SYT1 nucleic acid sequence DT476906**
ATGCAACAGCACCTGATGCAGATGCAGCCCATGATGGCAGCCTATTACCCCAGCAACGTCACTACT
GATCATATTCAACAGTATCTGGACGAAAACAAGTCATTGATTTTGAAGATTGTTGAGAGCCAGAAT
TCAGGGAAACTCAGTGAGTGTGCAGAGAACCAAGCAAGACTGCAACAAAATCTCATGTACTTGGCT
GCAATTGCTGATTGTCAGCCCCAACCACCTACCATGCATGCCCAGTTCCCTTCCAGCGGCATTATG
CAGCCAGGAGCACATTACATGCAGCATCAACAAGCTCAACAGATGACACCACAAGCCCTTATGGCT
GCACGCTCTTCTATGCTGCAGTATGCTCAACAGCCATTCTCAGCGCTTCAACAACAGCAAGCCTTA
CACAGCCAGCTCGGCATGAGCTCTGGTGGAAGCGCAGGACTTCATATGATGCAAAGCGAGGCTAAC
ACTGCAGGAGGCAGTGGAGCTCTTGGTGCTGGACGATTTCCTGATTTTGGCATGGATGCCTCCAGT
AGAGGAATCGCAAGTGGGAGCAAGCAAGATATTCGGAGTGCAGGGTCTAGTGAAGGGCGAGGAGGA
AGCTCTGGAGGCCAGGGTGGTGATGGAGGTGAAACCCTTTACTTGAAATCTGCTGATGATGGGAAC
TGA

**SEQ ID NO: 207 Populus trichocarpa Poptr_SYT1 translated polypeptide sequence**
MQQHLMQMQPMMAAYYPSNVTTDHIQQYLDENKSLILKIVESQNSGKLSECAENQARLQQNLMYLA
AIADCQPQPPTMHAQFPSSGIMQPGAHYMQHQQAQQMTPQALMAARSSMLQYAQQPFSALQQQQAL
HSQLGMSSGGSAGLHMMQSEANTAGGSGALGAGRFPDFGMDASSRGIASGSKQDIRSAGSSEGRGG
SSGGQGGDGGETLYLKSADDGN

**FIGURE 10 (continued)**

**SEQ ID NO: 208 Populus trichocarpa Poptr_SYT2 nucleic acid sequence scaff_XIV.493**

ATGCAGCAGCCACCGCAAATGATTCCTGTCATTTCTCCATTTCCACCAACAAACATCACCACTGAG
CAGATCCAAAAGTACCTTGACGAAAACAAAAAGTTGATTTTGGCTATATTGGACAACCAAAACCTT
GGAAAACTTGCTGAATGTGCCCAGTATCAAGCCCAGCTGCAGAAGAATTTGATGTATTTGGCTGCA
ATTGCTGATGCCCAACCACAGGCACCAGCAATGCCTCCCCAGATGGCCCCGCATCCTGCAATGCAA
CAAGGGGCATATTACATGCAACATCCTCAGGCAGCAGCAATGGCTCAGCAGCCAGGTGTTTTCCCC
CAAAAGATGCTATTACAATTCAATGCTGGACATCAAATGCAGGATCCTCAGCAGTTACACCAACAA
GCCATGCAAGGGCAAATAGGAATTAGACCTATAGGGGCTAACAATGGCATGCATCCCATGCACGCT
GAGATTGCTCTTGGAAGCAGTGGCCCTTCAGCAAGTGCTGGCACAAATGATGTACGTGGGGGAAGC
AAACAGGATGCCTCTGAGGCTGGCACAACCGGTGCTGATGGCCTAGGGGGCTCTGCTGCTGGGCAT
AATGGTGCTGACGGTTCCGAGGATGCAAAATGA

**SEQ ID NO: 209 Populus trichocarpa Poptr_SYT2 translated polypeptide sequence**

MQQPPQMIPVISPFPPTNITTEQIQKYLDENKKLILAILDNQNLGKLAECAQYQAQLQKNLMYLAA
IADAQPQAPAMPPQMAPHPAMQQGAYYMQHPQAAAMAQQPGVFPQKMLLQFNAGHQMQDPQQLHQQ
AMQGQIGIRPIGANNGMHPMHAEIALGSSGPSASAGTNDVRGGSKQDASEAGTTGADGLGGSAAGH
NGADGSEDAK

**SEQ ID NO: 210 Populus trichocarpa Poptr_SYT1.2 nucleic acid sequence CV257942.1**

Atgcagcagtcaccgcaacaaatgttgagcatcaccactgagcagattcaaaagtacttagaagag
aacaagcagctgattatggctatactggagaatcagaacaagggaaacgtttctgaatgtgcttcg
tatcaagcccagttacagcagaacctgatgtacctagcaagaattgctgatgcccaaccacaagga
accacaatgccttctcagatgccccctcagcagcccgcagtgaagcaagagcagtacatgcagcca
tctcaagttgctatgactcagcaaccaatttcttcaatcagaagctccctttccaaacgaacttt
cagcatgagcagcagcaacagctgccaccacacctccaacagcaacacttcacccaaggacagatg
agaatgagacccggtgtcactgatcaagattctgatgcctaa

**SEQ ID NO: 211 Populus trichocarpa Poptr_SYT1.2 translated polypeptide sequence**

MQQSPQQMLSITTEQIQKYLEENKQLIMAILENQNKGNVSECASYQAQLQQNLMYLARIADAQPQG
TTMPSQMPPQQPAVKQEQYMQPSQVAMTQQPIFFNQKLPFQTNFQHEQQQQLPPHLQQQHFTQGQM
RMRPGVTDQDSDA

**SEQ ID NO: 212 Prunus persica Prupe_SYT2 nucleic acid sequence contig of DT454880.1, DT455286.1**

ATGCAGCAGCCACAGCAAATGATCCCTGTGATGCCTACTTCATTTCCACCCACTAACATCACCACC
GAGCAAATTCAGAAGTACCTTGACGAGAACAAAAAATTGATTCTGGCAATATTGGATAATCAAAAC
CTTGGAAAACTTGCTGAGTGTGCCCAGTACCAAGCTCAGCTTCAAAAGAATCTGATGTATTTAGCA
GCTATTGCTGATGCACAACCACAGGCACCAACAGTGCCTGCTCAGATGGCCCCACATCCTGCTATG
CAACAAGCAGGATATTACATGCAACATCCTCAGGCAGCAGCAATGGCTCAGCAACAGGGTATTTTC
CCCCCAAAGATGCCATTGCAGTTCAATAACCCGCACCAAATGCATGATGCAGCACAGCAGCTACAC
CAGCAGCACCAACAAGCCATGCAAGGGCAAATGGGAATGAGAGCTGGAGGGGCCAATGGCATGCCT
TCCATGCATCATACTGAAGCCACACTTGGTGGTGGTAGTGGTGGCCCCACTTCAGGTGGAGGGGGT
CCAAACGATGGGCGTGGAGGAAAGCAGCAAGACTACTCAGAGGCTGGGACAGGTGGTGATGGCCAG
GGGAGCTCAGCCGGCGGGCATGGCAATGGTGATGGAGAAGATGGAAAGTGA

**FIGURE 10 (continued)**

**SEQ ID NO: 213 Prunus persica Prupe_SYT2 translated polypeptide sequence**

MQQPQQMIPVMPTSFPPTNITTEQIQKYLDENKKLILAILDNQNLGKLAECAQYQAQLQKNLMYLA
AIADAQPQAPTVPAQMAPHPAMQQAGYYMQHPQAAAMAQQQGIFPPKMPLQFNNPHQMHDAAQQLH
QQHQQAMQGQMGMRAGGANGMPSMHHTEATLGGGSGGPTSGGGGPNDGRGGKQQDYSEAGTGGDGQ
GSSAGGHGNGDGEDGK

**SEQ ID NO: 214 Saccharum officinarum Sacof_SYT1 nucleic acid sequence contig of CA078249.1 CA078630 CA082679 CA234526 CA239244 CA083312**

ATGCAGCAGCAACACCTGATGCAGATGAACCAGAACATGATTGGGGGCTACACCTCTCCTGCCGCT
GTGACAACCGATCTCATCCAGCAGTACCTGGATGAGAACAAGCAGCTGATCCTGGCCATCCTCGAC
AACCAGAACAATGGCAAGGTGGAGGAGTGCGAACGGCACCAAGCTAAGCTCCAGCACAACCTCATG
TACCTGGCCGCCATCGCCGACAGCCAGCCACCACAGACTGCACCACTATCACAATACCCGTCCAAC
CTGATGATGCAGCCGGGCCCTCGGTACATGCCACCGCAGTCCGGGCAGATGATGAGCCCGCAGTCG
CTAATGGCGGCGCGGTCCTCCATGATGTACGCGCACCCGTCCATGTCACCACTCCAGCAGCAGCAG
GCAGCGCACGGGCAGCTGGGCATGGCTTCAGGGGGCGGCGGTGGCACGACCAGTGGGTTCAACATC
CTCCATGGCGAGGCCAGTATGGGCGGTGCTGGTGGCGCTTGTGCCGGCAACAACATGATGAACGCC
GGCATGTTCTCAGGCTTTGGCCGCAGCGGCAGTGGCGCCAAGGAGGGATCGACCTCGCTGTCGGTT
GACGTCCGTGGTGGCACCAGCTCCGGCGCGCAAAGCGGGGACGGCGAGTACCTGAAAGCAGGCACC
GAGGAAGAAGGCAGTTAA

**SEQ ID NO: 215 Saccharum officinarum Sacof_SYT1 translated polypeptide sequence**

MQQQHLMQMNQNMIGGYTSPAAVTTDLIQQYLDENKQLILAILDNQNNGKVEECERHQAKLQHNLM
YLAAIADSQPPQTAPLSQYPSNLMMQPGPRYMPPQSGQMMSPQSLMAARSSMMYAHPSMSPLQQQQ
AAHGQLGMASGGGGGTTSGFNILHGEASMGGAGGACAGNNMMNAGMFSGFGRSGSGAKEGSTSLSV
DVRGGTSSGAQSGDGEYLKAGTEEEGS

**SEQ ID NO: 216 Saccharum officinarum Sacof_SYT2 nucleic acid sequence CA110367**

ATGCAGCAGCCGATGCCCATGCAGCCGCAGGCGCCGGAGATGACCCCGGCCGCCGGAATCACCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCCCAACCACAGACTGCTGTAAGCCGCCCTCAGATGGCGCCGCCTGGTGCATTG
CCTGGAGTAGGGCAGTACATGTCACAGGTGCCTATGTTCCCACCGAGGACACCTCTAACACCCCAG
CAGATGCAGGAGCAGCAACTTCAGCAGCAGCAGGCTCAGCTGCTAAATTTCAGTGGCCTAATGGTT
GCTAGACCTGGCATGGTCAACGGCATGCCTCAGTCCATTCAAGTTCAGCAAGCTCAGCCACCACCA
GCAGGGAACAAACAGGATGCTGGTGGGGTCGCCTCGGAGCCCTCGGGCATTGAGAACCACAGGAGC
ACTGGTGGTGATAATGATGGTGGAAGCGACTAG

**SEQ ID NO: 217 Saccharum officinarum Sacof_SYT2 translated polypeptide sequence**

MQQPMPMQPQAPEMTPAAGITTEQIQKYLDENKQLILAILENQNLGKLAECAQYQSQLQKNLLYLA
AIADAQPQTAVSRPQMAPPGALPGVGQYMSQVPMFPPRTPLTPQQMQEQQLQQQQAQLLNFSGLMV
ARPGMVNGMPQSIQVQQAQPPPAGNKQDAGGVASEPSGIENHRSTGGDNDGGSD

## FIGURE 10 (continued)

**SEQ ID NO: 218 Saccharum officinarum Sacof_SYT3 nucleic acid sequence contig of CA161933.1 CA265085**
ATGCAGCAGCAGATGCCCATGCCGCCGGCGCCCGCTGCGGCGGCGCCCCCGGCGGCCGGCATC
ACCACCGAGCAGATCCAAAAGTATTTGGACGAAAATAAGCAACTTATTTGGCCATCCTGGAAAAT
CAGAACTTAGGAAAGTTGGCTGAATGTGCTCAGTATCAAGCTCAACTTCAAAAGAACCTCTTGTAC
CTGGCTGCGATTGCTGATGCCCAACCCCAGCCACCACAAAACCCTGCAGGTCGCCCTCAGATGATG
CAACCTGGTATAGTGCCAGGTGCGGGCATTACATGTCACAAGTACCAATGTTCCCTCCAAGAACT
CCATTAACCCCACAGCAGATGCAAGAGCAGCAGCAGCAACAGCTTCAGCAGCAGCAAGCGCAGGCT
CTTACATTCCCTGGACAGATGGTCATGAGACCAGCTACCATCAACGGCATACAGCAGCCTATGCAA
GCTGACCCTGCCCGGGCAGCGGAGCTGCAACAACCACCACCTATCCCAGCTGACGGGCGAGTAAGC
AAGCAGCAGGACACAACGGCTGGCGTGAGCTCAGAGCCTTCTGCCAATGAGAGCCACAAGACCACA
ACTGGAGCAGATAGTGAGGCAGGTGGTGACGTGGCGGAGAAATCCTAA

**SEQ ID NO: 219 Saccharum officinarum Sacof_SYT3 translated polypeptide sequence**
MQQQMPMPPAPAAAAAPPAAGITTEQIQKYLDENKQLILAILENQNLGKLAECAQYQAQLQKNLLY
LAAIADAQPQPPQNPAGRPQMMQPGIVPGAGHYMSQVPMFPPRTPLTPQQMQEQQQQLQQQQAQA
LTFPGQMVMRPATINGIQQPMQADPARAAELQQPPPIPADGRVSKQQDTTAGVSSEPSANESHKTT
TGADSEAGGDVAEKS

**SEQ ID NO: 220 Solanum tuberosum Soltu_SYT1.1 nucleic acid sequence CK265597**
ATGCAGCAGCACCTGATGCAGATGCAGCCCATGATGGCAGCTTACTATCCAACGAACGTCACTACT
GACCATATTCAACAGTATTTGGATGAGAACAAATCACTCATTCTGAAAATTGTTGAGAGCCAAAAC
TCGGGAAAACTCAGTGAATGTGCAGAGAACCAAGCTAGGCTTCAGAGGAATCTGATGTACCTTGCT
GCTATTGCTGATTCACAACCTCAGCCTTCTAGCATGCATTCTCAGTTCTCTTCTGGTGGGATGATG
CAGCCAGGGACACACAGTTACCTGCAGCAGCAGCAGCAGCAACAACAAGCGCAACAAATGGCAACA
CAACAACTCATGGCTGCAAGATCCTCATCAATGCTCTATGGACAACAACAGCAGCAGCAGCAGCAG
TCTCAGTTATCACAATTTCAACAAGGCTTGCATAGTAGCCAACTTGGCATGAGTTCTGGCAGTGGT
GGAAGCACTGGACTTCATCACATGCTTCAAAGTGAATCATCACCTCATGGTGGTGGTTTCTCTCAT
GACTTCGGCCGTGCAAATAAGCAAGACATTGGGAGTAGTATGTCTGCTGAAGGGCGCGGCGGAAGC
TCAGGTGGTGATGGTGGTGAGAATCTTTATCTGAAAGCTTCTGAGGATTGA

**SEQ ID NO: 221 Solanum tuberosum Soltu_SYT1.1 translated polypeptide sequence**
MQQHLMQMQPMMAAYYPTNVTTDHIQQYLDENKSLILKIVESQNSGKLSECAENQARLQRNLMYLA
AIADSQPQPSSMHSQFSSGGMMQPGTHSYLQQQQQQQAQQMATQQLMAARSSSMLYGQQQQQQQ
SQLSQFQQGLHSSQLGMSSGSGGSTGLHHMLQSESSPHGGGFSHDFGRANKQDIGSSMSAEGRGGS
SGGDGGENLYLKASED

**SEQ ID NO: 222 Solanum tuberosum Soltu_SYT1.2 nucleic acid sequence BG590990**
ATGCAGCAGCAGCACCTGATGCAGATGCAGCCCATGATGGCAGCCTATTATCCCAACAATGTCACT
ACTGATCATATTCAACAGTTCCTGGATGAGAACAAATCACTTATTCTGAAGATTGTTGAGAGCCAG
AACTCTGGGAAAATAAGTGAATGTGCAGAGTCCCAAGCTAAACTTCAGAGAAATCTTATGTACCTT
GCAGCTATTGCTGATTCACAGCCCCAGCCTCCTAGTATGCATTCACAGTTAGCTTCTGGTGGGATG
ATGCAGGGAGGGGCACATTATATGCAGCAACAACAAGCTCAACAACTCACAACGCAATCGCTTATG
GCTGCAGCAAGATCCTCCTCCTCAATGCTCTATGGACAACAACAACAACAACAACAACAACAACTA

**FIGURE 10 (continued)**

TCATCATTGCAACAACAGCAAGCAGCCTTTCATAGCCAGCAACTCGGAATGAGCAGCTCTGGTGGA
GGAAGCAGTAGTGGACTTCACATGCTACAAAGCGAAAACACTCATAGTGCTAGCACTGGTGGTGGT
GGTTTCCCTGACTTTGGCAGAGGATTAGGCAGTGGAAACAAGCATGAAATGGGAAGTTCTATGTCT
GATCAAGGACGGGGCGGAAGCTCGAGTGGTCATGGTGGTGATGGAGGTGAGAATCTTTACTTGAAA
TCTTCTGAAGATGGGAATTAG

**SEQ ID NO: 223 Solanum tuberosum Soltu_SYT1.2 translated polypeptide sequence**
MQQQHLMQMQPMMAAYYPNNVTTDHIQQFLDENKSLILKIVESQNSGKISECAESQAKLQRNLMYL
AAIADSQPQPPSMHSQLASGGMMQGGAHYMQQQQAQQLTTQSLMAAARSSSSMLYGQQQQQQQQL
SSLQQQQAAFHSQQLGMSSSGGGSSSGLHMLQSENTHSASTGGGGFPDFGRGLGSGNKHEMGSSMS
DQGRGGSSSGHGGDGGENLYLKSSEDGN

**SEQ ID NO: 224 Solanum tuberosum Soltu_SYT3 nucleic acid sequence CK272804**
ATGCAGCAACCACCACCCATGATTCCAATGATGCCCTCTTTTCCTTCTCCTAATATCACTACTGAG
CAGATTCAAAAGTACCTGGACGAGAACAAGACATTGATTTTGGCCATATTGGACCATCAAAATCTT
GGGAAACTAGCTGAATGTGCACAGTACCAGGCTAAACTTCAGAAGAACTTGATGTACTTGGCCGCT
ATTGCTGATGCTCAACCACAATCACCAGCTATTCCAACGCAAATGGCTCCTCATCCTGCAATGCAA
CAAGGAGGATTTTACATGCAGCACCCTCAGGCTGCAGCCATGACTCAACAACAAGGTATGTTTACT
TCAAAGATGCCACTGCAGTTCAACAACCCACAGCAACTACACGATCAGCAGCAGCTTCAACATCAA
CATCAACATCAACAACTACAGCGACAGCAGCAAGGTATGCAACTTGGAGGTGCCAACAGTGGAATG
CACTCCACTCTTGGTAGTACAAGTAATGTTAGCCAGCTTACAACTTCAGGTGCTGGTGATGCACGC
GGAGGAAACAAACAAGACAACTCTGAAGCGGGTGCTGATGGTCAGGCTAGCTCAGTGACTGCCCAA
GTCTCGGAAGAACGCAAGTGA

**SEQ ID NO: 225 Solanum tuberosum Soltu_SYT3 translated polypeptide sequence**
MQQPPPMIPMMPSFPSPNITTEQIQKYLDENKTLILAILDHQNLGKLAECAQYQAKLQKNLMYLAA
IADAQPQSPAIPTQMAPHPAMQQGGFYMQHPQAAAMTQQQGMFTSKMPLQFNNPQQLHDQQQLQHQ
HQHQQLQRQQQGMQLGGANSGMHSTLGSTSNVSQLTTSGAGDARGGNKQDNSEAGADGQASSVTAQ
VSEERK

**SEQ ID NO: 226 Sorghum bicolor Sorbi_SYT1 nucleic acid sequence TA40712_4558**
ATGCAGCAGCAACACCTGATGCAGATGAACCAGAACATGATTGGGGGCTACACCTCTCCTGCCGCT
GTGACCACCGATCTCATCCAGCAGTACCTGGATGAGAACAAGCAGCTGATCCTGGCCATCCTCGAC
AACCAGAACAATGGAAAGGTGGAGGAGTGCGAACGGCACCAAGCTAAGCTCCAGCACAACCTCATG
TACCTGGCGGCCATCGCTGACAGCCAGCCACCACAGACTGCACCACTATCACAGTACCCGTCCAAC
CTGATGATGCAGCCAGGCCCTCGGTACATGCCACCGCAGTCCGGGCAGATGATGAGCCCGCAGTCG
CTAATGGCGGCGCGGTCCTCCATGATGTACGCGCACCCGTCCATGTCGCCACTCCAGCAGCAGCAG
GCAGCGCACGGCCAGCTGGGCATGGCTTCAGGGGGCGGCGGTGGCACGACCAGTGGGTTCAGCATC
CTCCACGGCGAGGCCAGCATGGGCGGTGCTGCTGGCGCAGGCACCGGCAACAGCATGATGAACGCC
GGCATGTTCTCAGGCTTTGGCCGCAGCGGCAGTGGCGCCAAGGAGGGATCGACCTCGCTGTCTGTT
GACGTCCGTGGTGGCACCAGCTCCGGCGCGCAGAGCGGGGACGGCGAGTACCTGAAAGCAGGCACC
GAGGAAGAAGGCAGTTAA

FIGURE 10 (continued)

**SEQ ID NO: 227 Sorghum bicolor Sorbi_SYT1 translated polypeptide sequence**
MQQQHLMQMNQNMIGGYTSPAAVTTDLIQQYLDENKQLILAILDNQNNGKVEECERHQAKLQHNLM
YLAAIADSQPPQTAPLSQYPSNLMMQPGPRYMPPQSGQMMSPQSLMAARSSMMYAHPSMSPLQQQQ
AAHGQLGMASGGGGGTTSGFSILHGEASMGGAAGAGTGNSMMNAGMFSGFGRSGSGAKEGSTSLSV
DVRGGTSSGAQSGDGEYLKAGTEEEGS

**SEQ ID NO: 228 Sorghum bicolor Sorbi_SYT2 nucleic acid sequence contig of CF482417 CW376917**
ATGCAGCAGCCGATGCCCATGCAGCCGCAGGCGCCGGCGATGACCCCGGCCGCCGGAATCACCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCCCAACCACAGACTGCTGTAAGCCGCCCTCAGATGGCGCCGCCTGGTGCATTG
CCTGGAGTAGGGCAGTACATGTCACAGGTGCCTATGTTCCCACCAAGGACACCTCTCACACCCCAA
CAGATGCAGGAGCAGCAACTTCAGCAGCAGCAGGCTCAGTTGCTAAATTTCAGTGGCCAGATGGTT
GCTAGACCTGGCATGGTCAACGGCATGCCTCAGTCCATTCAAGCTCAACAAGCTCAGCCATCACCA
GCATTGAACAAACAGGATGCTGGTGGAGTCGCCTCAGAGCCCTCGGGCACTGAGAGCCACAGGAGC
ACTGGTGGTGATAATGATGGTGGAAGCGACTAG

**SEQ ID NO: 229 Sorghum bicolor Sorbi_SYT2 translated polypeptide sequence**
MQQPMPMQPQAPAMTPAAGITTEQIQKYLDENKQLILAILENQNLGKLAECAQYQSQLQKNLLYLA
AIADAQPQTAVSRPQMAPPGALPGVGQYMSQVPMFPPRTPLTPQQMQEQQLQQQQAQLLNFSGQMV
ARPGMVNGMPQSIQAQQAQPSPALNKQDAGGVASEPSGTESHRSTGGDNDGGSD

**SEQ ID NO : 230 Sorghum bicolor Sorbi_SYT3 nucleic acid sequence CX611128**
ATGCAGCAGCAGATGCCCATGCCGCCGGCGCCCGCTGCGGCGGCGGCGACGGCGCCCCCGGCGGCC
GGCATCACCACCGAGCAGATCCAGAAGTATTTGGACGAAAATAAGCAACTTATTTTGGCCATCCTA
GAAAATCAGAACTTAGGAAAGTTGGCTGAATGTGCTCAGTATCAAGCTCAACTTCAAAAGAACCTC
TTGTACCTGGCTGCGATTGCTGATGCCCAACCCCGACCACCGCAAAACCCTGCAGGTCGCCCTCAG
ATGATGCAACCTGGTATAGTGCCAGGTGCAGGGCATTACATGTCACAAGTACCAATGTTCCCTCCA
AGAACTCCATTAACCCCACAGCAAATGCAAGAGCAGCAGCAGCAACAGCTTCAGCAGCAGCAAGCG
CAGGCTCTTGCATTCCCTGGGCAGATGGTCATGAGACCAGCTACCATCAACGGCATGCAGCAGCCT
ATGCAGGCTGACCCTGCCCGGGCAGCGGAGCTGCAACAGCCAGCATCTGTCCCAGCCGACGGGCGA
GTAAGCAAGCAGGACACAGCGGCTGGGGTGAGCTCAGAGCCTTCTGCCAATGAGAGCCACAAGACC
ACAACCGGAGCAGATAGTGAGGCAGGTGGAGACGTGGCGGAGAAATCCTAA

**SEQ ID NO: 231 Sorghum bicolor Sorbi_SYT3 translated polypeptide sequence**
MQQQMPMPPAPAAAAATAPPAAGITTEQIQKYLDENKQLILAILENQNLGKLAECAQYQAQLQKNL
LYLAAIADAQPRPPQNPAGRPQMMQPGIVPGAGHYMSQVPMFPPRTPLTPQQMQEQQQQLQQQQA
QALAFPGQMVMRPATINGMQQPMQADPARAAELQQPASVPADGRVSKQDTAAGVSSEPSANESHKT
TTGADSEAGGDVAEKS

FIGURE 10 (continued)

**SEQ ID NO: 232 Taraxacum officinale Tarof_SYT2 nucleic acid sequence TA1299_50225**

ATGAAGCAGCCGATGATGCCTAATCCAATGATGTCTTCTCCGTTTCCTCCTTCCAACATCACCACC
GATCAGATCCAAAAGTTCCTAGACGAAAACAAGCAACTGATATTAGCAATAATGAACAACCAAAAC
CTAGGAAAGCTTGCTGAATGTGCCCAGTATCAAGCTCTACTCCAAAAGAATTTAATGTATCTAGCA
GCCATTGCAGATGCTCAACCACCAACACCAACACCAACACCAAATATCTCATCTCAGATGGGCCCG
GTTCCACATCCAGGGATGCCACAACAAGGTGGATTCTACATGGGGCAGCACCCTCAAGCGGCAGTA
ATGGCGGCTCAGCCACCTTCTGGTTTCCCACAACCAATGCCAGGCATGCAGTTTAATACCCCACAG
GGTATCCAAGGTCAGATGGGCGGGAGGTCCGGTGGGCCACCAAACTCAGCTGGCGGCGATGTTTGG
AGAGGAAGCATGCAAGATGGTGGTGGTGGCGGTGTTGATGGTGGTAAGGATGGTCATGCTGGCGGT
GGTCCACCGGAGGAAGGAAAGTGA

**SEQ ID NO: 233 Taraxacum officinale Tarof_SYT2 translated polypeptide sequence**

MKQPMMPNPMMSSPFPPSNITTDQIQKFLDENKQLILAIMNNQNLGKLAECAQYQALLQKNLMYLA
AIADAQPPTPTPTPNISSQMGPVPHPGMPQQGGFYMGQHPQAAVMAAQPPSGFPQPMPGMQFNTPQ
GIQGQMGGRSGGPPNSAGGDVWRGSMQDGGGGGVDGGKDGHAGGGPPEEGK

**SEQ ID NO: 234 Taraxacum officinale Tarof_SYT3 nucleic acid sequence TA5000_50225**

ATGCAGCAGCAACAGCATCAACAACCACCGCAATCGCAACTGCAACCGCCCACCTTAAACTCCGGT
GCTCCATTTTCTCCCAATGCGATCACCTCCGACCAGATTCAAAAGTGTCTGGATGACAACGAGAAC
CTGATTATAGCAATATTGGAAAATCAAAATCTTGGGAAATTTCAGGAGTGTGCTCAGTATCAAGCC
ATTCTCCAAAAGAACTTAATGTATTTAGCTGCAATTGCTGATGCTCAACCACCAACACAACAACCA
TCAACTCCTCAAATGCCACCAAATTCCATTCCTCAACAACCAAACAATTACATGCAACAACAACAC
CAAATCACCGCCCCTCAACAAGGCGGCATAGGTGGTGGTGGCGGTGTTCCAAAACTACCCTTTCAA
CTTAATGCCCTTCGTACACAAGATCAACAACAACAATTACTCCAATTTCAACAACAACAACAACTT
CAAGCACAAATGGGGATGAGACCTAGTTCCCAAGATGGGATGCTTGGAATGCATCAAGCTATGCAG
TCTGCACTCGCGGGCAATCCGGGCAGTTTGATGGATGGTAGAGGGAACAAGCAAGATGGATCAGAG
GCGGCGGCTTCTGGTGGTGGTGGTGGTAATAGAGAATCATGA

**SEQ ID NO: 235 Taraxacum officinale Tarof_SYT3 translated polypeptide sequence**

MQQQQHQQPPQSQLQPPTLNSGAPFSPNAITSDQIQKCLDDNENLIIAILENQNLGKFQECAQYQA
ILQKNLMYLAAIADAQPPTQQPSTPQMPPNSIPQQPNNYMQQQHQITAPQQGGIGGGGGVPKLPFQ
LNALRTQDQQQQLLQFQQQQQLQAQMGMRPSSQDGMLGMHQAMQSALAGNPGSLMDGRGNKQDGSE
AAASGGGGGNRES

**SEQ ID NO: 236 Triticum aestivum Triae_SYT1 nucleic acid sequence TA105893_4565**

ATGCAGCAGCAACACCTGATGCAGATGAACCAGAGCATGATGGGGGGCTACGCTTCCTCTACCACT
GTCACCACTGATCTCATTCAGCAGTACCTGGATGAGAACAAGCAGCTGATCCTGGCCATCCTCGAC
AACCAGAACAACGGCAAGGTGGAGGAGTGCGCACGGAACCAAGCTAAGCTCCAGCAGAACCTCATG
TACCTCGCCGCCATCGCCGACAGCCAGCCTCCGCAGACGGCATCGCTGTCTCAGTACCCGTCCAAC
CTGATGATGCAGTCCGGGCCGCGGTACATGCAGCAGCAGTCGGCGCAGATGATGTCGCCGCAGTCG
CTGATGGCGGCGCGGTCGTCGATGATGTACGCGCAGCAGGCCATGTCGCCGCTCCAGCAGCAGCAG
CAGCAGCAGCACCAGGCGGCCGCGCACGGCCAGCTGGGGATGTCCTCCGGCGCGACCACCGGGTTC

FIGURE 10 (continued)

AACCTCCTCCACGGCGAGGCCAGCATGGGCGGCGGCGGCGGCGGCGCCAGTGGCAACAGCATGATG
AACGCCGGCGTCTTCTCGGACTACCGCCGCGGCGGCAGCGGCGCCAAGGAGGGGTCGACCTCGCTG
TCGGCCGACGCTCGCGGCGCCAACTCTGGCGCGCACAGCGGCGACGGGGAGTACCTCAAGGGCACC
GAGGAGGAAGGAAGCTAG

**SEQ ID NO: 237 Triticum aestivum Triae_SYT1 translated polypeptide sequence**
MQQQHLMQMNQSMMGGYASSTTVTTDLIQQYLDENKQLILAILDNQNNGKVEECARNQAKLQQNLM
YLAAIADSQPPQTASLSQYPSNLMMQSGPRYMQQQSAQMMSPQSLMAARSSMMYAQQAMSPLQQQQ
QQQHQAAAHGQLGMSSGATTGFNLLHGEASMGGGGGGASGNSMMNAGVFSDYRRGGSGAKEGSTSL
SADARGANSGAHSGDGEYLKGTEEEGS

**SEQ ID NO: 238 Triticum aestivum Triae_SYT2 nucleic acid sequence CD901951**
ATGCAGCAAGCGATGCCCATGCCGCCGGCGGCGGCGGCGCCGGGGATGCCTCCGTCTGCTGGCCTC
AGCACCGAGCAGATCCAAAAGTACCTGGATGAAAATAAGCAACTAATTTTGGCTATCTTGGAAAAT
CAGAACCTGGGAAAGTTGGCGGAATGTGCTCAGTATCAAGCTCAGCTTCAGAAGAATCTTTTGTAT
TTGGCTGCAATCGCTGATACTCAGCCACAGACCACTGTAAGCCGTCCTCAGATGGCACCACCTAGT
GCATCCCCAGGGGCAGGGCATTACATGTCACAGGTGCCAATGTTCCCTCCGAGGACCCCTCTAACG
CCTCAGCAGATGCAGGAGCAGCAACTACAGCAGCAACAGGCTCAGATGCTTCCGTTTGCTGGTCAA
ATGGTTGCGAGACCTGGGGCTGTCAATGGCATGCCTCAGGCCCCTCAAGTTGAACCAGCCTATGCA
GCAGGTGGGGCCAGTTCTGAGCCTTCTGGCACTGAGAGCCACAGGAGCACTGGTGCCGATAATGAC
GGGGGGAGCGGCTGGGCTGATCAGTCCTAA

**SEQ ID NO: 239 Triticum aestivum Triae_SYT2 translated polypeptide sequence**
MQQAMPMPPAAAAPGMPPSAGLSTEQIQKYLDENKQLILAILENQNLGKLAECAQYQAQLQKNLLY
LAAIADTQPQTTVSRPQMAPPSASPGAGHYMSQVPMFPPRTPLTPQQMQEQQLQQQQAQMLPFAGQ
MVARPGAVNGMPQAPQVEPAYAAGGASSEPSGTESHRSTGADNDGGSGWADQS

**SEQ ID NO: 240 Triticum aestivum Triae_SYT3 nucleic acid sequence contig of BJ246754 BJ252709**
ATGCAGCAGGCGATGTCCTTGCCCCCGGGAGCGGTCGGCGCGGTGTCCTCGCCGGCCGGCATCACC
ACCGAGCAGATCCAAAAGTATTTGGATGAAAATAAGCAACTTATTTTGGCCATCCTTGAAAATCAG
AACCTAGGAAAGTTGGCTGAATGTGCTCAGTATCAAGCTCAACTCCAAAAGAATCTCTTGTATCTA
GCTGCTATCGCGGATGCCCAACCACCACAGAACCCTACAAGTCACCCTCAGATGGTGCAGCCTGGT
AGTATGCAAGGTGCAGGGCATTACATGTCACAAGTACCAATGTTCCCTCCAAGAACGCCTTTAACC
CCACAGCAGATGCAAGAGCAGCAGCACCAGCAGCTTCAGCAGCAGCAAGCCCAGGCCCTTTCTTTC
CCCGCCCAGGTGGTCATGAGACCAGGCACCGTCAACGGCATGCAGCAGCCTATGCAAGCAGCCGGC
GACCTCCAGCCAGCAGCAGCACCTGGAGGGAGCAAGCAGGACGCCGCAGTGGCTGGGGCCAGCTCG
GAACCATCTGGCACCAAGAGCCACAAGAACGCGGGAGCAGAGGAGGTGGGCGCTGATGTAGCAGAA
CAATCCTAA

**SEQ ID NO: 241 Triticum aestivum Triae_SYT3 translated polypeptide sequence**
MQQAMSLPPGAVGAVSSPAGITTEQIQKYLDENKQLILAILENQNLGKLAECAQYQAQLQKNLLYL
AAIADAQPPQNPTSHPQMVQPGSMQGAGHYMSQVPMFPPRTPLTPQQMQEQQHQQLQQQQAQALSF
PAQVVMRPGTVNGMQQPMQAAGDLQPAAAPGGSKQDAAVAGASSEPSGTKSHKNAGAEEVGADVAE
QS

**FIGURE 10 (continued)**

**SEQ ID NO: 242 Vitis vinifera Vitvi_SYT1.1 nucleic acid sequence DV219834**

ATGCAGCAGCACCTGATGCAGATGCAGCCCATGATGGCAGCCTATTACCCCAGCAACGTCACCACT
GATCACATTCAGCAGTATCTTGATGAAAACAAGTCATTGATTCTGAAGATTGTTGAGAGCCAGAAT
TCAGGAAAATTGACTGAATGTGCAGAGAACCAGGCAAGACTACAGAGAAACCTCATGTACCTGGCT
GCAATTGCTGATTCTCAACCCCAACCACCCACCATGCATGCTCAGTTCCCTCCTAGTGGCATTGTT
CAGCCAGGAGCTCACTACATGCAACACCAACAAGCTCAACAAATGACACCACAGTCGCTCCTGGCT
GCACGCTCCTCCATGCTGTACACCCAACAACCATTTTCGGCCCTGCAACAACAACAAGCCATCCAT
AGCCAGCTTGGCATGGGCTCTGGTGGAAGTGCAGGACTTCACATGCTGCAAAGCGAGGGGAGTAAT
CCAGGAGGCAATGGAACACTGGGGACTGGTGGGTTTCCTGATTTCAGCCGTGGAACTTCTGGAGAA
GGCCTGCAGGCTGCAGGCAGGGGAATGGCTGGTGGGAGCAAGCAAGATATGGGAAATGCAGAAGGG
CGAGGAGGGAACTCAGGAGGTCAGGGTGGGGATGGAGGTGAGACTCTTTACTTGAAAGCTGCTGAA
GATGGGAATTGA

**SEQ ID NO: 243 Vitis vinifera Vitvi_SYT1.1 translated polypeptide sequence**

MQQHLMQMQPMMAAYYPSNVTTDHIQQYLDENKSLILKIVESQNSGKLTECAENQARLQRNLMYLA
AIADSQPQPPTMHAQFPPSGIVQPGAHYMQHQQAQQMTPQSLLAARSSMLYTQQPFSALQQQQAIH
SQLGMGSGGSAGLHMLQSEGSNPGGNGTLGTGGFPDFSRGTSGEGLQAAGRGMAGGSKQDMGNAEG
RGGNSGGQGGDGGETLYLKAAEDGN

**SEQ ID NO: 244 Vitis vinifera Vitvi_SYT1.2 nucleic acid sequence EE108079**

ATGCAGCAACACCTTATGCAGATGCAGCCTATGATGGCAGGAAGCCATAACCTCAGCAGCATCACT
ACTGATCACATCCAACAGTACCTAGATGAGAACAAGTCTTTGATTTTGAAAATTCTTGAGAGCCAA
AATTCAGGGAAACTCAGTGAATGTGCGGAGAACCAAGCAAGACTTCAGCGAAACCTTATGTACCTT
GCTGCAATTGCTGATTGCCAACCACAACCACCATCCCTGCAGGCTCAGTTTTCCCCCAATATGGTC
ATGCAACCAGGAGTCAACTACATGCAGCACCAACAATCCCAACAGATGATGCCACAGTCACTAATG
GCAGCCCGAGCACCCATGTTGTATGCTCAGCAGCATCCATATTTGGCATTGCAGCAACAACAAGCT
CTACAAAGCCAGCTTGGCATGAGCTCCACTGGAATGGGTGGAATCCACATGCTACAAAGTGAACCT
AATGTTGGAGGGAATGGGACTGGAGCCTTTTCCGATCTTGGTCGCAGCATGACTGGGGAGGGCTTG
TCGGCTGTGAGCAGGGGACTGGGTAGTGCAAGCAAGCAAGATGTGGGGAGTGTAGGCTCTGCGGAA
GGTCGACGTGGCTACTTGGGAGGGCAAGGTGCAGATAAAGGAGAAACTCTTTACTTTAAAAGTGCT
GAAGAAAAGGACTGA

**SEQ ID NO: 245 Vitis vinifera Vitvi_SYT1.2 translated polypeptide sequence**

MQQHLMQMQPMMAGSHNLSSITTDHIQQYLDENKSLILKILESQNSGKLSECAENQARLQRNLMYL
AAIADCQPQPPSLQAQFSPNMVMQPGVNYMQHQQSQQMMPQSLMAARAPMLYAQQHPYLALQQQQA
LQSQLGMSSTGMGGIHMLQSEPNVGGNGTGAFSDLGRSMTGEGLSAVSRGLGSASKQDVGSVGSAE
GRRGYLGGQGADKGETLYFKSAEEKD

**SEQ ID NO: 246 Vitis vinifera Vitvi_SYT2.1 nucleic acid sequence EC939550**

ATGCAGCAGAACCCCCAGATGATACCTGTTATGCCTTCTTTTCCACCCAACAACATCACTACCGAG
CAGATTCAGAAGTATCTCGATGAGAATAAAAAATTGATTCTGGCAATATTGGACAATCAAAACCTT
GGAAAGCTTGCTGAGTGTGCACAGTACCAAGCTCAGCTTCAAAAGAATTTGATGTATCTAGCTGCA
ATTGCTGATGCTCAGCCACAGGCACCACCGACAATGCCTCCCCAGATGGCCCCACACCCTGCAATG

**FIGURE 10 (continued)**

CAGCAGGGAGGGTACTACATGCAGCATCCCCAGGCGGCAGCAATGGCTCAGCAACCTGGTCTTTTC
CCTCCCAAGATGCCCTTACAATTTGGTAACCCACATCAACTTCAGGAGCAAGCACAGCAGCTGCAG
CAGCTACAGCAACAAGCCATGCAAGGGCAGATGGGCATGAGACCTGGAGGGGCCAACAACGGCATG
CATCCCATGCATCCTGAGGCCACTCTTGGTGGTGGCAGCAGTGGTGGCCCTCCACCATCTGCCGGC
CTCAGTGATGCACGCGGAGGTGGCAAGCAAGACACTTCCGAAGCAGGGGCTTCTGGTGGTGATGGT
CAGGGGAGCTCAGCTGCTGGGCATGGCGGCGATGGCGAATCACCCTACTTGAAGGGGTCAGAGGAT
GGAAAGTGA

**SEQ ID NO: 247 Vitis vinifera Vitvi_SYT2.1 translated polypeptide sequence**
MQQNPQMIPVMPSFPPNNITTEQIQKYLDENKKLILAILDNQNLGKLAECAQYQAQLQKNLMYLAA
IADAQPQAPPTMPPQMAPHPAMQQGGYYMQHPQAAAMAQQPGLFPPKMPLQFGNPHQLQEQAQQLQ
QLQQQAMQGQMGMRPGGANNGMHPMHPEATLGGGSSGGPPPSAGLSDARGGGKQDTSEAGASGGDG
QGSSAAGHGGDGESPYLKGSEDGK

**SEQ ID NO: 248 Vitis vinifera Vitvi_SYT2.2 nucleic acid sequence**
ATGCAGCAGCAACCACCACAGATGATGAACATTGCGCCTTCATTTCCTCCCACCGCCATCACCACT
GAGCAGATTCAGAAGTATCTGGATGAGAACAAACAATTGATTCTGGCAATTCTGGAAAACCAGACC
CTTGGAAAACTCGCCGAGTGTGCCCAATATCAAGCCCAGCTTCAGAAGAACTTGATATATCTAGCT
GCAATTGCTGATGCCCAACCACCAGCACCAACAGTGCCTCCTCAGATGCCTATACATCATGCCATG
CAACAAGGGCATTACATGCAACACCCTCAGGCTGCTGCAGCTCAGCAACAACCAGGCATGTTTGGT
GCAAAGTTGCCTTTCCAGCTTAGCGATCAGCAACAGCAGCAGCAGCATCATTTTTTACACCTCCAA
CAACAGCAACCCATCCAAGGGCTCATGGGCATGAGGCCTATCATCAACAATGGCATGCATCAGGCC
ATGCAAACTGGGCTTGGTGCTTTGAGCGGTTTCATGGATGTACGTGGAAGCAAGCCAGATGGCTCA
GAGGTTGGTTTTGGTGATGGCCAAGGGAAGTTTGCTTCTGGACATGGCAGTGGAAATAGAGATTCC
TAA

**SEQ ID NO: 249 Vitis vinifera Vitvi_SYT2.2 translated polypeptide sequence**
MQQQPPQMMNIAPSFPPTAITTEQIQKYLDENKQLILAILENQTLGKLAECAQYQAQLQKNLIYLA
AIADAQPPAPTVPPQMPIHHAMQQGHYMQHPQAAAAQQQPGMFGAKLPFQLSDQQQQQHHFLHLQ
QQQPIQGLMGMRPIINNGMHQAMQTGLGALSGFMDVRGSKPDGSEVGFGDGQGKFASGHGSGNRDS

**SEQ ID NO: 250 Volvox carteri Volca_SYT nucleic acid sequence contig of JGI_CBHO11121.fwd, JGI_CBHO11121.rev**
ATGGCGGCAGTGTCAGGTCAGGCCAAGCCAGCCCCAATGACAACGGAGCGAATCCAAGAAATGCTT
GAAGAAAACTTCAAGTTTATCAAGGCACTCGCCGAACAGCAAAACCTTGGTCGAATGCAGGACGTC
ATCCAGTTCCAGCAGAAGCTTCAAGAAAACCTTATGCTACTAGCAGCTGTGGCGGACACCTACTCA
TCAGCGTCTGCAACGGGAGCTGCTGCCCAGGCGACTGCAGCTCCCGGCATGGCGGCACAGGCGGCC
CGGCCGCCCGCTGCTGCTCTCCCTGGAACAGCTGTTGCTACGGCGGCGCTCCCGCTCCCTGGCTTG
CCGCAACAACAGCAACCGCAGCAGCAGCAGCAGCCGCAGCAGCAGCCAGGGCAGCCCAGCCCGCTA
ATGATGGTCATGCCGGGCTCTTCCGGGACGCAGGCGCCTACGCAACTGGGGGCCATGCAACTCACG
CAGCAGCAGATACAAGCTGCGGTACAGCAAGCGCTAGTCCGACAGCAGCAGCAGCAACAACAACAG
CAGCAGCAAAATCCAAATCCGTTTCAGGGGCAGCAATTCCAGCTGCCACAGTCACTTCAGCAGCCG
CAGTCCCTGGGACAGCAGCCAGTTTTAAGTGCGATGGGGGCCCTGGGAGGGCCCTTGGCTGGTCAG
GGGACGGCGGCAGGGCAGGCGGGAGCAGGTGGTTTCCAGCTGCCCGCGGCGCCTCCTTTGAACCTT
GGGCTTTGA

## FIGURE 10 (continued)

**SEQ ID NO: 251 Volvox carteri Volca_SYT translated polypeptide sequence**
MAAVSGQAKPAPMTTERIQEMLEENFKFIKALAEQQNLGRMQDVIQFQQKLQENLMLLAAVADTYS
SASATGAAAQATAAPGMAAQAARPPAAALPGTAVATAALPLPGLPQQQPQQQQPQQQPGQPSPL
MMVMPGSSGTQAPTQLGAMQLTQQQIQAAVQQALVRQQQQQQQQQQNPNPFQGQQFQLPQSLQQP
QSLGQQPVLSAMGALGGPLAGQGTAAGQAGAGGFQLPAAPPLNLGL

**SEQ ID NO: 252 Welwitschia mirabilis Welmi_SYT nucleic acid sequence DT598761**
ATGCAAATGCAGCCTATGATAGGGGCAGGATACTCCTCCAATAGCATCACCACTGATCACATTCAG
AAGTATTTGGATGAGAATAGGCAATTGATTCTGGCAATTCTTGACAACCAAAATCTTGGAAAGTTG
AATGAATGTGCACAATACCAAGCCAGGCTTCAACAAAATTTAATGTATTTGGCTGCTATTGCTGAT
TCCCAACCGCAAACACCTGCTGCACATGCCCAGATCGCATCCAATGCAATGCTTCAAGCAGGTGGT
CATTATATGCAGCACCAGCAGACAGTAACTCCCCAGTCACTTCTTGCTGCAAGGTCATCTATGCTT
TACAGTCAGCAACCTATGACTGCATTCCATCAAGCACAGCAGCAGCAGCAACAACAATCTCTCCAT
GGCCAGTTGGGGATAAATTCAGGAGGAAACAATGGATTACATATTCTTCATGGTGAAACAAGCATG
GGTAGTAATGGACCTCTCACGACAGGAAGCTTTCCTGATTTTGGGCGAGGTTCAGTAAATTGTGAT
CTATTGCAGGGCAATAGGAGCTTGACTATTGACCGTGGCTCTAGCAAGATTGATGGGCTTGGAACA
GAGAATACGCACCCTGTAGAAGGGCGAGGAGGAGCAAGTGTGGGCCAAAACACAGAAGAAGGGGCA
CCATCTTATTTGAAAGCTTCTGAGGATGAGGGAAGCTAG

**SEQ ID NO: 253 Welwitschia mirabilis Welmi_SYT translated polypeptide sequence**
MQMQPMIGAGYSSNSITTDHIQKYLDENRQLILAILDNQNLGKLNECAQYQARLQQNLMYLAAIAD
SQPQTPAAHAQIASNAMLQAGGHYMQHQQTVTPQSLLAARSSMLYSQQPMTAFHQAQQQQQQSLH
GQLGINSGGNNGLHILHGETSMGSNGPLTTGSFPDFGRGSVNCDLLQGNRSLTIDRGSSKIDGLGT
ENTHPVEGRGGASVGQNTEEGAPSYLKASEDEGS

**SEQ ID NO: 254 Zea mays Zeama_SYT1 nucleic acid sequence contig of BG874129.1, CA409022.1**
ATGCAGCAGCAACACCTGATGCAGATGAACCAGAACATGATGGGGGGCTACACCTCTCCTGCCGCC
GTGACCACCGATCTCATCCAGCAGCACCTGGACGAGAACAAGCAGCTGATCCTGGCCATCCTCGAC
AACCAGAACAATGGCAAGGCGGAGGAGTGCGAACGGCACCAAGCTAAGCTCCAGCACAACCTCATG
TACCTGGCCGCCATCGCTGACAGCCAGCCGCCACAGACCGCGCCACTATCACAGTACCCGTCCAAC
CTGATGATGCAGCCGGGCCCTCGGTACATGCCACCGCAGTCCGGGCAGATGATGAACCCGCAGTCG
CTGATGGCGGCGCGGTCCTCCATGATGTACGCGCACCCGTCCCTGTCGCCACTCCAGCAGCAGCAG
GCGGCGCACGGACAGCTGGGTATGGCTCCAGGGGGCGGCGGTGGCGGCACGACCAGCGGGTTCAGC
ATCCTCCACGGCGAGGCCAGCATGGGCGGTGGTGGTGCTGGCGCAGGCGCCGGCAACAACATGATG
AACGCCGGCATGTTCTCGGGCTTTGGCCGCAGCGGCAGTGGCGCCAAGGAAGGGTCGACCTCTCTG
TCGGTTGACGTCCGGGGTGGAACCAGCTCCGGCGCGCAGAGCGGGGACGGCGAGTACCTCAAAGTC
GGCACCGAGGAAGAAGGCAGTTAG

**SEQ ID NO: 255 Zea mays Zeama_SYT1 translated polypeptide sequence**
MQQQHLMQMNQNMMGGYTSPAAVTTDLIQQHLDENKQLILAILDNQNNGKAEECERHQAKLQHNLM
YLAAIADSQPPQTAPLSQYPSNLMMQPGPRYMPPQSGQMMNPQSLMAARSSMMYAHPSLSPLQQQQ
AAHGQLGMAPGGGGGGTTSGFSILHGEASMGGGGAGAGAGNNMMNAGMFSGFGRSGSGAKEGSTSL
SVDVRGGTSSGAQSGDGEYLKVGTEEEGS

## FIGURE 10 (continued)

**SEQ ID NO: 256 Zea mays Zeama_SYT2 nucleic acid sequence AY106697**
ATGCAGCAGCCGATGCACATGCAGCCACAGGCGCCGGCGATAACCCCAGCTGCCGGAATCAGCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCTCAACCGCAGACTGCTGTAAGCCGCCCTCAGATGGCGCCGCCTGGTGGATCG
CCTGGAGTAGGGCAGTACATGTCACAGGTGCCTATGTTCCCACCGAGGACACCTCTTACACCCCAG
CAGATGCAGGAGCAGCAGCTTCAGCAGCAGCAGGCTCAGTTGCTAAACTTCAGTGGCCAAATGGTT
GCTAGACCAGGCATGGTCAACGGCATGGCTCAGTCCATGCAAGCTCAGCTACCACCGGGTGTGAAC
AAGCAGGATGCTGGTGGGGTCGCCTCTGAGCCCTCGGGCACCGAGAGCCACAGGAGCACTGGTGGT
GACGATGGTGGAAGCGACTAG

**SEQ ID NO: 257 Zea mays Zeama_SYT2 translated polypeptide sequence**
MQQPMHMQPQAPAITPAAGISTEQIQKYLDENKQLILAILENQNLGKLAECAQYQSQLQKNLLYLA
AIADAQPQTAVSRPQMAPPGGSPGVGQYMSQVPMFPPRTPLTPQQMQEQQLQQQQAQLLNFSGQMV
ARPGMVNGMAQSMQAQLPPGVNKQDAGGVASEPSGTESHRSTGGDDGGSD

**SEQ ID NO: 258 Zea mays Zeama_SYT3 nucleic acid sequence CO468901**
ATGCAGCAGCAGATGCCCATGCCGCCGGCGCCCGCTGCCGCCGCGGCGGCGGCGCCCCCGGCGGCA
GGCATCACTACCGAGCAGATCCAGAAGTATTTGGACGAAAATAAGCAACTTATTTTGGCCATCCTG
GAAAATCAGAACTTAGGGAAGTTGGCTGAATGTGCTCAGTATCAAGCTCAACTTCAAAAGAACCTC
TTGTACCTGGCTGCGATTGCTGATGCCCAACCCCAGCCTCCGCAAAACCCTGCAGGTCGCCCTCAG
ATGATGCAGCCTGGTATAGTGCCAGGTGCGGGGCATTACATGTCACAAGTACCAATGTTCCCTCCA
AGAACCCCATTAACCCCACAGCAGATGCAGGAGCAGCAGCAACAACAACAGTTTCAGCAGCAGCAG
CAGCAAGTGCAGGCTCTTACATTTCCTGGACAGATGGTCATGAGACCAGGCACCATCAACGGCATG
CAGCAGCAGCAGCCTATGCAGGCTGACCCTGCCCGGGCAGCAGCGGAGCTGCAGCAGGCAGCACCT
ATCCCAGCTGACGGGCGAGGAAGCAAGCAGGACACCGCGGGTGGGGCGAGCTCAGAGCCTTCTGCC
AATGAGAGCCACAAGAGCGCCACCGGAGCAGATACCGAGGCAGGTGGCGACGTGGCCGAGAAATCC
TAA

**SEQ ID NO: 259 Zea mays Zeama_SYT3 translated polypeptide sequence**
MQQQMPMPPAPAAAAAAAPPAAGITTEQIQKYLDENKQLILAILENQNLGKLAECAQYQAQLQKNL
LYLAAIADAQPQPPQNPAGRPQMMQPGIVPGAGHYMSQVPMFPPRTPLTPQQMQEQQQQQQFQQQQ
QQVQALTFPGQMVMRPGTINGMQQQQPMQADPARAAAELQQAAPIPADGRGSKQDTAGGASSEPSA
NESHKSATGADTEAGGDVAEKS

**SEQ ID NO: 260 Homo sapiens SYT nucleic acid sequence CR542103**
ATGGGCGGCAACATGTCTGTGGCTTTCGCGGCCCCGAGGCAGCGAGGCAAGGGGGAGATCACTCCC
GCTGCGATTCAGAAGATGTTGGATGACAATAACCATCTTATTCAGTGTATAATGGACTCTCAGAAT
AAAGGAAAGACCTCAGAGTGTTCTCAGTATCAGCAGATGTTGCACACAAACTTGGTATACCTTGCT
ACAATAGCAGATTCTAATCAAAATATGCAGTCTCTTTTACCAGCACCACCCACACAGAATATGCCT
ATGGGTCCTGGAGGGATGAATCAGAGCGGCCCTCCCCCACCTCCACGCTCTCACAACATGCCTTCA
GATGGAATGGTAGGTGGGGGTCCTCCTGCACCGCACATGCAGAACCAGATGAACGGCCAGATGCCT
GGGCCTAACCATATGCCTATGCAGGGACCTGGACCCAATCAACTCAATATGACAAACAGTTCCATG
AATATGCCTTCAAGTAGCCATGGATCCATGGGAGGTTACAACCATTCTGTGCCATCATCACAGAGC
ATGCCAGTACAGAATCAGATGACAATGAGTCAGGGACAACCAATGGGAAACTATGGTCCCAGACCA
AATATGAGTATGCAGCCAAACCAAGGTCCAATGATGCATCAGCAGCCTCCTTCTCAGCAATACAAT
ATGCCACAGGGAGGCGGACAGCATTACCAAGGACAGCAGCCACCTATGGGAATGATGGGTCAAGTT
AACCAAGGCAATCATATGATGGGTCAGAGACAGATTCCTCCCTATAGACCTCCTCAACAGGGCCCA

## FIGURE 10 (continued)

CCACAGCAGTACTCAGGCCAGGAAGACTATTACGGGGACCAATACAGTCATGGTGGACAAGGTCCT
CCAGAAGGCATGAACCAGCAATATTACCCTGATGGAAATTCACAGTATGGCCAACAGCAAGATGCA
TACCAGGGACCACCTCCACAACAGGGATATCCACCCCAGCAGCAGCAGTACCCAGGGCAGCAAGGT
TACCCAGGACAGCAGCAGGGCTACGGTCCTTCACAGGGTGGTCCAGGTCCTCAGTATCCTAACTAC
CCACAGGGACAAGGTCAGCAGTATGGAGGATATAGACCAACACAGCCTGGACCACCACAGCCACCC
CAGCAGAGGCCTTATGGATATGACCAGGGACAGTATGGAAATTACCAGCAG

**SEQ ID NO: 261 Homo sapiens SYT translated polypeptide sequence CAG46900.1**

MGGNMSVAFAAPRQRGKGEITPAAIQKMLDDNNHLIQCIMDSQNKGKTSECSQYQQMLHTNLVYLA
TIADSNQNMQSLLPAPPTQNMPMGPGGMNQSGPPPPPRSHNMPSDGMVGGGPPAPHMQNQMNGQMP
GPNHMPMQGPGPNQLNMTNSSMNMPSSSHGSMGGYNHSVPSSQSMPVQNQMTMSQGQPMGNYGPRP
NMSMQPNQGPMMHQQPPSQQYNMPQGGGQHYQGQQPPMGMMGQVNQGNHMMGQRQIPPYRPPQQGP
PQQYSGQEDYYGDQYSHGGQGPPEGMNQQYYPDGNSQYGQQQDAYQGPPPQQGYPPQQQQYPGQQG
YPGQQQGYGPSQGGPGPQYPNYPQGQGQQYGGYRPTQPGPPQPPQQRPYGYDQGQYGNYQQ

**SEQ ID NO: 262 SNH domain from Arath_SYT1 (comprised in SEQ ID NO: 121)**

VTSDHIQQYLDENKSLILKIVESQNSGKLSECAENQARLQRNLMYLAAIADAQT

**SEQ ID NO: 263 most conserved residues comprised in the SNH domain**

QXXLXXNXXXIXXXXXXXXXGXXXXXXXXQXXLXXNLXXLAXXAD

**SEQ ID NO: 264 SSXT domain InterPro007726 (PFam05030) comprised in SEQ ID NO: 121**

QMQPMMAGYYPSNVTSDHIQQYLDENKSLILKIVESQNSGKLSECAENQARLQRNLMYLAAIADSQ
PQPPSVHS

**SEQ ID NO : 265 Prm06681**

GGGGACAAGTTTGTACAAAAAAGCAGGCTTAAACAATGCAACAGCACCTGATG

**SEQ ID NO : 266 Prm06682**

GGGGACCACTTTGTACAAGAAAGCTGGGTCATCATTAAGATTCCTTGTGC

**SEQ ID NO: 267 SEQ ID NO: 1 + SEQ ID NO: 120**

ATGATGAGTCTAAGTGGAAGTAGCGGGAGAACAATAGGAAGGCCTCCATTTACACCAACACAATGG
GAAGAACTGGAACATCAAGCCCTAATCTACAAGTACATGGTCTCTGGTGTTCCTGTCCCACCTGAG
CTCATCTTCTCCATTAGAAGAAGCTTGGACACTTCCTTGGTCTCTAGACTCCTTCCTCACCAATCC
CTTGGATGGGGGTGTTACCAGATGGGATTTGGGAGAAAACCAGATCCAGAGCCAGGAAGATGCAGA
AGAACAGATGGTAAGAAATGGAGATGCTCAAGAGAAGCTTACCCAGATTCGAAGTACTGTGAAAAA
CACATGCACAGAGGAAGAAACCGTGCCAGAAAATCTCTTGATCAGAATCAGACAACAACAACTCCT
TTAACATCACCATCTCTCTCATTCACCAACAACAACAACCCAAGTCCCACCTTGTCTTCTTCTTCT
TCCTCTAATTCCTCTTCTACTACTTATTCTGCTTCTTCTTCTTCAATGGATGCCTACAGTAACAGT
AATAGGTTTGGGCTTGGTGGAAGTAGTAGTAACACTAGAGGTTATTTCAACAGCCATTCTCTTGAT
TATCCTTATCCTTCTACTTCACCCAAACAACAACAACAAACTCTTCATCATGCTTCCGCTTTGTCA
CTTCATCAAAATACTAATTCTACTTCTCAGTTCAATGTCTTAGCCTCTGCTACTGACCACAAAGAC
TTCAGGTACTTTCAAGGGATTGGGGAGAGAGTTGGAGGAGTTGGGGAGAGAACGTTCTTTCCAGAA
GCATCTAGAAGCTTTCAAGATTCTCCATACCATCATCACCAACAACCGTTAGCAACAGTGATGAAT

**FIGURE 10 (continued)**

GATCCGTACCACCACTGTAGTACTGATCATAATAAGATTGATCATCATCACACATACTCATCCTCA
TCATCATCTCAACATCTTCATCATGATCATGATCATAGACAGCAACAGTGTTTTGTTTTGGGCGCC
GACATGTTCAACAAACCTACAAGAAGTGTCCTTGCAAACTCATCAAGACAAGATCAAAATCAAGAA
GAAGATGAGAAAGATTCATCAGAGTCGTCCAAGAAGTCTCTACATCACTTCTTTGGTGAGGACTGG
GCACAGAACAAGAACAGTTCAGATTCTTGGCTTGACCTTTCTTCCCACTCAAGACTCGACACTGGT
AGCTAANATGCAACAGCACCTGATGCAGATGCAGCCCATGATGGCTGGTTACTACCCCAGCAATG
TTACCTCTGATCATATCCAACAGTACTTGGACGAAAACAAATCGTTGATTCTGAAGATTGTTGAGT
CTCAAAACTCTGGAAAGCTTAGCGAATGCGCCGAGAATCAAGCAAGGCTTCAACGCAACCTAATGT
ACCTAGCTGCAATAGCAGATTCTCAGCCTCAGCCACCAAGTGTGCATAGCCAGTATGGATCTGCTG
GTGGTGGGATGATTCAGGGAGAAGGAGGGTCACACTATTTGCAGCAGCAACAAGCGACTCAACAGC
AACAGATGACTCAGCAGTCTCTAATGGCGGCTCGATCTTCAATGTTGTATGCTCAGCAACAGCGGC
AGCAGCAGCCTTACGCGACGCTTCAGCATCAGCAATCGCACCATAGCCAGCTTGGAATGAGCTCGA
GCAGCGGAGGAGGAGGAAGCAGTGGTCTCCATATCCTTCAGGGAGAGGCTGGTGGGTTTCATGATT
TTGGCCGTGGGAAGCCGGAAATGGGAAGTGGTGGTGGCGGTGAAGGCAGAGGAGGAAGTTCAGGGG
ATGGTGGAGAAACCCTTTACTTGAAATCATCAGATGATGGGAATTGA

Where N can be no nucleotide to any number of nucleotides

**SEQ ID NO: 268 SEQ ID NO: 120 + SEQ ID NO: 1**

ATGCAACAGCACCTGATGCAGATGCAGCCCATGATGGCTGGTTACTACCCCAGCAATGTTACCTCT
GATCATATCCAACAGTACTTGGACGAAAACAAATCGTTGATTCTGAAGATTGTTGAGTCTCAAAAC
TCTGGAAAGCTTAGCGAATGCGCCGAGAATCAAGCAAGGCTTCAACGCAACCTAATGTACCTAGCT
GCAATAGCAGATTCTCAGCCTCAGCCACCAAGTGTGCATAGCCAGTATGGATCTGCTGGTGGTGGG
ATGATTCAGGGAGAAGGAGGGTCACACTATTTGCAGCAGCAACAAGCGACTCAACAGCAACAGATG
ACTCAGCAGTCTCTAATGGCGGCTCGATCTTCAATGTTGTATGCTCAGCAACAGCGGCAGCAGCAG
CCTTACGCGACGCTTCAGCATCAGCAATCGCACCATAGCCAGCTTGGAATGAGCTCGAGCAGCGGA
GGAGGAGGAAGCAGTGGTCTCCATATCCTTCAGGGAGAGGCTGGTGGGTTTCATGATTTTGGCCGT
GGGAAGCCGGAAATGGGAAGTGGTGGTGGCGGTGAAGGCAGAGGAGGAAGTTCAGGGGATGGTGGA
GAAACCCTTTACTTGAAATCATCAGATGATGGGAATTGANATGATGAGTCTAAGTGGAAGTAGCG
GGAGAACAATAGGAAGGCCTCCATTTACACCAACACAATGGGAAGAACTGGAACATCAAGCCCTAA
TCTACAAGTACATGGTCTCTGGTGTTCCTGTCCCACCTGAGCTCATCTTCTCCATTAGAAGAAGCT
TGGACACTTCCTTGGTCTCTAGACTCCTTCCTCACCAATCCCTTGGATGGGGGTGTTACCAGATGG
GATTTGGGAGAAAACCAGATCCAGAGCCAGGAAGATGCAGAAGAACAGATGGTAAGAAATGGAGAT
GCTCAAGAGAAGCTTACCCAGATTCGAAGTACTGTGAAAAACACATGCACAGAGGAAGAAACCGTG
CCAGAAAATCTCTTGATCAGAATCAGACAACAACAACTCCTTTAACATCACCATCTCTCTCATTCA
CCAACAACAACAACCCAAGTCCCACCTTGTCTTCTTCTTCTTCCTCTAATTCCTCTTCTACTACTT
ATTCTGCTTCTTCTTCTTCAATGGATGCCTACAGTAACAGTAATAGGTTTGGGCTTGGTGGAAGTA
GTAGTAACACTAGAGGTTATTTCAACAGCCATTCTCTTGATTATCCTTATCCTTCTACTTCACCCA
AACAACAACAACAAACTCTTCATCATGCTTCCGCTTTGTCACTTCATCAAAATACTAATTCTACTT
CTCAGTTCAATGTCTTAGCCTCTGCTACTGACCACAAAGACTTCAGGTACTTTCAAGGGATTGGGG
AGAGAGTTGGAGGAGTTGGGGAGAGAACGTTCTTTCCAGAAGCATCTAGAAGCTTTCAAGATTCTC
CATACCATCATCACCAACAACCGTTAGCAACAGTGATGAATGATCCGTACCACCACTGTAGTACTG
ATCATAATAAGATTGATCATCATCACACATACTCATCCTCATCATCATCTCAACATCTTCATCATG
ATCATGATCATAGACAGCAACAGTGTTTTGTTTTGGGCGCCGACATGTTCAACAAACCTACAAGAA
GTGTCCTTGCAAACTCATCAAGACAAGATCAAAATCAAGAAGAAGATGAGAAAGATTCATCAGAGT
CGTCCAAGAAGTCTCTACATCACTTCTTTGGTGAGGACTGGGCACAGAACAAGAACAGTTCAGATT
CTTGGCTTGACCTTTCTTCCCACTCAAGACTCGACACTGGTAGCTAA

Where N can be no nucleotide to any number of nucleotides

**FIGURE 10 (continued)**

**SEQ ID NO: 269 SEQ ID NO: 2 + SEQ ID NO: 121**

MMSLSGSSGRTIGRPPFTPTQWEELEHQALIYKYMVSGVPVPPELIFSIRRSLDTSLVSRLLPHQS
LGWGCYQMGFGRKPDPEPGRCRRTDGKKWRCSREAYPDSKYCEKHMHRGRNRARKSLDQNQTTTTP
LTSPSLSFTNNNNPSPTLSSSSSSNSSSTTYSASSSSMDAYSNSNRFGLGGSSSNTRGYFNSHSLD
YPYPSTSPKQQQQTLHHASALSLHQNTNSTSQFNVLASATDHKDFRYFQGIGERVGGVGERTFFPE
ASRSFQDSPYHHHQQPLATVMNDPYHHCSTDHNKIDHHHTYSSSSSSQHLHHDHDHRQQQCFVLGA
DMFNKPTRSVLANSSRQDQNQEEDEKDSSESSKKSLHHFFGEDWAQNKNSSDSWLDLSSHSRLDTG
SXMQQHLMQMQPMMAGYYPSNVTSDHIQQYLDENKSLILKIVESQNSGKLSECAENQARLQRNLM
YLAAIADSQPQPPSVHSQYGSAGGMIQGEGGSHYLQQQQATQQQQMTQQSLMAARSSMLYAQQQR
QQQPYATLQHQQSHHSQLGMSSSSGGGGSSGLHILQGEAGGFHDFGRGKPEMGSGGGGEGRGGSSG
DGGETLYLKSSDDGN

Where X can be no amino acid to any number of amino acids

**SEQ ID NO: 270 SEQ ID NO: 121 + SEQ ID NO: 2**

MQQHLMQMQPMMAGYYPSNVTSDHIQQYLDENKSLILKIVESQNSGKLSECAENQARLQRNLMYLA
AIADSQPQPPSVHSQYGSAGGMIQGEGGSHYLQQQQATQQQQMTQQSLMAARSSMLYAQQQRQQQ
PYATLQHQQSHHSQLGMSSSSGGGGSSGLHILQGEAGGFHDFGRGKPEMGSGGGGEGRGGSSGDGG
ETLYLKSSDDGNXMMSLSGSSGRTIGRPPFTPTQWEELEHQALIYKYMVSGVPVPPELIFSIRRS
LDTSLVSRLLPHQSLGWGCYQMGFGRKPDPEPGRCRRTDGKKWRCSREAYPDSKYCEKHMHRGRNR
ARKSLDQNQTTTTPLTSPSLSFTNNNNPSPTLSSSSSSNSSSTTYSASSSSMDAYSNSNRFGLGGS
SSNTRGYFNSHSLDYPYPSTSPKQQQQTLHHASALSLHQNTNSTSQFNVLASATDHKDFRYFQGIG
ERVGGVGERTFFPEASRSFQDSPYHHHQQPLATVMNDPYHHCSTDHNKIDHHHTYSSSSSSQHLHH
DHDHRQQQCFVLGADMFNKPTRSVLANSSRQDQNQEEDEKDSSESSKKSLHHFFGEDWAQNKNSSD
SWLDLSSHSRLDTGS

Where X can be no amino acid to any number of amino acids

**FIGURE 10 (continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060048240 A
- US 20070022495 A
- JP 2004350553 A
- US 5811238 A
- US 6395547 A
- WO 2004070039 A
- WO 2004065596 A
- US 4962028 A
- WO 0114572 A
- WO 9412015 A
- EP 99106056 A
- US 5565350 A, Kmiec
- WO 9322443 A, Zarling
- WO 9836083 A
- WO 9915682 A
- WO 0015815 A
- EP 1198985 A1
- US 5164310 A
- EP 07116988 A
- US 60975882 B


### Non-patent literature cited in the description


- **TITTONELL et al.** *Agric Ecosys & Environ,* 2005, vol. 105, 213
- **FASOULA ; TOLLENAAR.** *Maydica,* 2005, vol. 50, 39
- **STEEGE et al.** *Plant Physiology,* 2005, vol. 139, 1078
- **HITTALMANI et al.** *Theoretical Applied Genetics,* 2003, vol. 107, 679
- **REBETZKE et al.** *Crop Science,* 2002, vol. 42, 739
- **GARDENER et al.** Physiology of Crop Plants. Iowa State University Press, 1985, 68-73
- **WANG et al.** *Planta,* 2003, vol. 218, 1-14
- **RIECHMANN et al.** *Science,* 2000, vol. 290, 2105-2109
- **GAO et al.** *Bioinformatics,* 2006, vol. 22 (10), 1286-7
- **KIM et al.** *Plant J,* 2003, vol. 36, 94-104
- **CHOI et al.** *Plant Cell Physiol,* 2004, vol. 45 (7), 897-904
- **ELLERSTROM et al.** *Plant Molec Biol,* 2005, vol. 59, 663-681
- **KIM ; KENDE.** *Proc Natl Acad Sci,* 2004, vol. 101, 13374-13379
- **DE VETTEN & FERL.** *Plant Physiol,* 1994, vol. 106, 1593-1604
- **VAN DER KNAPP et al.** *Plant Physiol,* 2000, vol. 122, 695-704
- **KIM HJ ; KENDE H.** *Proc Nat Acad Sc,* 2004, vol. 101, 13374-9
- **HORIGUCHI et al.** *Plant J,* 2005, vol. 43, 68-78
- **VAN DER KNAAP E et al.** *Plant Phys,* 2000, vol. 122, 695-704
- **NÄÄR AM et al.** *Annu Rev Biochem,* 2001, vol. 70, 475-501
- **THAETE et al.** *Hum Molec Genet,* 1999, vol. 8, 585-591
- **CREIGHTON.** Proteins. W.H. Freeman and Company, 1984
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **LEISNER.** *Proc Natl Acad Sci USA,* 1988, vol. 85 (5), 2553
- **JAIN et al.** *Crop Science,* 1999, vol. 39 (6), 1696

- **GATZ.** *Annu. Rev. Plant Physiol. Plant Mol. Biol.,* 1997, vol. 48, 89-108
- **XU et al.** *Plant Mol Biol,* 1995, vol. 27 (2), 237-48
- **NITZ et al.** *Plant Sci,* 2001, vol. 161 (2), 337-346
- **CONKLING et al.** *Plant Phys,* 1990, vol. 93 (3), 1203-1211
- **LERNER ; RAIKHEL.** *Plant Phys,* 1989, vol. 91, 124-129
- **KELLER ; LAMB.** *Genes & Dev,* 1989, vol. 3, 1639-1646
- **BLILOU et al.** *Genes & Dev,* 2002, vol. 16, 2566-2575
- **QING ; QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **SATO et al.** *Proc. Natl. Acad. Sci.,* 1996, (93), 8117-8122
- **WAGNER ; KOHORN.** *Plant Cell,* 2001, vol. 13 (2), 303-318
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **ANGELL ; BAULCOMBE.** *Plant J,* 1999, vol. 20 (3), 357-62
- **SCHWAB et al.** *Dev Cell,* 2005, vol. 8 (4), 517-27
- **SCHWAB et al.** *Plant Cell,* 2006, vol. 18 (5), 1121-33
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143

- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. Singapore, World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **TERADA.** *Curr Opin Biotech,* 2004, vol. 15 (2), 132-8
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 2003, vol. 10, 29
- **AUSUBEL et al.** Current Protocols in Molecular Biology. Current Protocols, 1994, vol. 1, 2
- **KIM ; KENDE.** *Proc Natl Acad Sci,* 2004, vol. 101 (36), 13374-13379
- Current Protocols in Molecular Biology. Wiley
- **RABBANI et al.** *Plant Physiol,* 2003, vol. 133, 1755-1767
- **FRINK et al.** *Proc Natl Acad Sci USA,* 1999, vol. 96 (4), 1175-1180
- **HALPIN.** *Plant Biotech J,* 2005, 141-155
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BEMATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic acid sequence Res.,* 1990, vol. 18, 3671
- **WALTER et al.** *Nat. Genet.,* 1997, vol. 7, 22-28
- **DEAR ; COOK.** *Nucleic acid sequence Res.,* 1989, vol. 17, 6795-6807

- **SAMBROOK.** Molecular Cloning: a laboratory manual. Cold Spring Harbor Laboratory Press, 2001
- **R.D.D. CROY.** Plant Molecular Biology Labfax. 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- *BMC Bioinformatics.,* 2003, vol. 4, 29
- **GASTEIGER E et al.** ExPASy: the proteomics server for in-depth protein knowledge and analysis. *Nucleic Acids Res,* 2003, vol. 31, 3784-3788
- **VAN DER KNAPP et al.** *Plant Phys,* 2000, vol. 122, 695-704
- **AUSUBEL et al.** Current Protocols in Molecular Biology. Current Protocols, vol. 1, 2
- **R.D.D. CROY.** Plant Molecular Biology Labfax. BIOS Scientific Publications Ltd, 1993
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659